(19) Europäisches Patentamt
European Patent Office
Office européen des brevets

(11) EP 4 703 354 A1

(12) **EUROPEAN PATENT APPLICATION**
published in accordance with Art. 153(4) EPC

(43) Date of publication:
**04.03.2026 Bulletin 2026/10**

(21) Application number: **24796238.4**

(22) Date of filing: **26.04.2024**

(51) International Patent Classification (IPC):
***C07D 213/81*** *(2006.01)* ***C07D 213/02*** *(2006.01)*
***C07D 401/12*** *(2006.01)* ***C07D 405/14*** *(2006.01)*
***C07C 237/30*** *(2006.01)* ***A61K 31/435*** *(2006.01)*
***A61K 31/4025*** *(2006.01)* ***A61P 37/00*** *(2006.01)*
***A61P 1/00*** *(2006.01)* ***A61P 19/02*** *(2006.01)*

(52) Cooperative Patent Classification (CPC):
**A61K 31/4025; A61K 31/435; A61P 1/00; A61P 19/02; A61P 37/00; C07C 237/30; C07D 213/02; C07D 213/81; C07D 401/12; C07D 405/14**

(86) International application number:
**PCT/CN2024/090046**

(87) International publication number:
**WO 2024/222865 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**28.04.2023 CN 202310481783**
**07.02.2024 CN 202410172844**
**18.04.2024 CN 202410467079**
**22.04.2024 CN 202410479410**

(71) Applicant: **Wuhan Createrna Science and Technology Co.,Ltd.**
**Wuhan, Hubei 430075 (CN)**

(72) Inventors:
- **LOU, Jun**
  **Wuhan, Hubei 430075 (CN)**
- **LU, Xiaoqin**
  **Wuhan, Hubei 430075 (CN)**
- **WU, Jingkang**
  **Wuhan, Hubei 430075 (CN)**
- **DONG, Xiaolong**
  **Wuhan, Hubei 430075 (CN)**
- **ZHANG, Yihan**
  **Wuhan, Hubei 430075 (CN)**

(74) Representative: **Murgitroyd & Company**
**165-169 Scotland Street**
**Glasgow G5 8PL (GB)**

(54) **COMPOUND AS CCR6 ANTAGONIST, AND PHARMACEUTICAL COMPOSITION AND USE THEREOF**

(57) A compound represented by formula (I), or a racemate, stereoisomer, tautomer, nitrogen oxide, or pharmaceutically acceptable salt thereof. As a CCR6 antagonist, the compound has good CCR6 antagonistic activity, and excellent pharmacokinetic and pharmacodynamic properties.

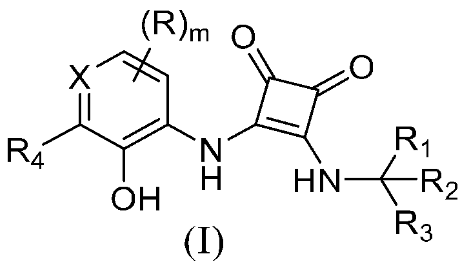

(I)

EP 4 703 354 A1

## Description

The present application claims the priority rights of the applicant's prior application:

the priority right of the prior application filed with the China National Intellectual Property Administration on April 28, 2023, with the patent application number 202310481783.9, entitled "Compound as CCR6 Antagonist, and Pharmaceutical Composition and Use Thereof";
the priority right of the prior application filed with the China National Intellectual Property Administration on February 07, 2024, with the patent application number 202410172844.8, entitled "Compound as CCR6 Antagonist, and Pharmaceutical Composition and Use Thereof";
the priority right of the prior application filed with the China National Intellectual Property Administration on April 18, 2024, with the patent application number 202410467079.2, entitled "Compound as CCR6 Antagonist, and Pharmaceutical Composition and Use Thereof";
the priority right of the prior application filed with the China National Intellectual Property Administration on April 22, 2024, with the patent application number 202410479410.2, entitled "Compound as CCR6 Antagonist, and Pharmaceutical Composition and Use Thereof".

The contents of the prior applications are incorporated herein by reference in their entireties.

TECHNICAL FIELD

The present disclosure relates to the field of pharmaceuticals, specifically to a compound as a CCR6 antagonist, a pharmaceutical composition thereof, and a use thereof.

BACKGROUND

Chemokines are a class of cytokines that control the directional migration of cells, and their functions are mediated by chemokine receptors. Through gene knockout, antibody blockade, and transgenic technology, it has been demonstrated that the chemokine system plays an important role in pathogen clearance, inflammatory responses, pathogen infection, cell and organ development, wound repair, tumor formation and metastasis, transplant immune rejection, *etc.* All chemokines exert their biological effects by interacting with G protein-coupled transmembrane receptors (*i.e.,* chemokine receptors). Chemokine receptors, as the natural ligands of chemokines, are a subfamily of seven-transmembrane proteins on the cell surface. Based on the types of chemokines they bind to, chemokine receptors are classified into four families: CCR, which binds to CC chemokines; CXCR, which binds to CXC chemokines; CX3CR1, which binds to the sole CX3C chemokine (CX3CL1); and XCR1, which binds to the two XC chemokines (XCL1 and XCL2).
CC chemokine receptor 6 (CCR6) is expressed on various key immune cells, including immature dendritic cells, B cells, memory T cells (encompassing all Th17 cells), neutrophils, and a subset of Treg cells. CCR6 is the sole known receptor for the chemokine CCL20, which is also referred to as macrophage inflammatory protein 3a (MIP-3a) or liver and activation-regulated chemokine (LARC). CCL20 is produced by synovial cells, colonic epithelial cells, various skin cells (such as keratinocytes and dermal fibroblasts), and alveolar epithelial cells. The ligand-receptor pair CCL20-CCR6 is responsible for the migration of immature dendritic cells and effector/memory T cells to the skin and mucosal surfaces under both homeostatic and inflammatory conditions, as well as in autoimmune diseases such as psoriasis and inflammatory bowel disease.
CCL20 is an inducible chemokine that is highly upregulated in the formation of inflammatory lesions in various inflammatory diseases, including allergic pulmonary inflammation, psoriasis, contact hypersensitivity, inflammatory bowel disease, Sjögren's syndrome, juvenile idiopathic arthritis, rheumatoid arthritis, and multiple sclerosis, and various autoimmune diseases. Studies have shown that CCL20 and CCR6 play important roles in regulating the physiological functions of the colonic mucosa. In normal colonic mucosa, CCL20 is weakly expressed, and CCL20 is markedly upregulated when stimulated by inflammation. Compared with normal mucosal tissues, the expression of CCL20 and CCR6 is markedly upregulated in colorectal cancer (CRC) liver metastases and shows a positive correlation with liver or lung metastases.
Inflammatory bowel disease (IBD) is an idiopathic, chronic, and recurrent inflammatory disease of the intestine. Its pathogenesis is highly complex and influenced by the interaction of genetic, environmental, and microbial factors. Clinical manifestations include diarrhea, abdominal pain, and even bloody stools, accompanied by symptoms such as anemia, malnutrition, and fatigue, significantly impairing patients' quality of life. Currently, the main drugs on the market for IBD are glucocorticoids, monoclonal antibodies such as Remicade, Humira, and Vedolizumab, as well as the small molecule inhibitor Imuran. CCL20/CCR6 can serve as a new therapeutic target for IBD patients.
Currently, there are no small molecule antagonists for CCR6 on the market, so the development of small

molecule antagonists with good biological activity has positive significance for the treatment of the above diseases.

SUMMARY

The present disclosure provides a compound represented by formula (I), a racemate thereof, a stereoisomer thereof, a tautomer thereof, an N-oxide thereof, or a pharmaceutically acceptable salt of any of the foregoing:

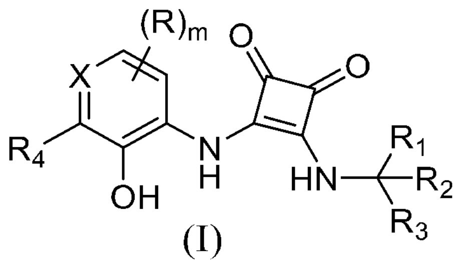

(I)

,

wherein

$R_1$ is H or $C_{1-6}$ alkyl;

$R_2$ is $C_{4-8}$ cycloalkyl or 5- to 6-membered heterocycloalkyl, wherein the $C_{4-8}$ cycloalkyl and 5- to 6-membered heterocycloalkyl are optionally substituted by 1, 2, 3, or 4 $R_{2-1}$;

each $R_{2-1}$ is the same or different and is independently halogen, deuterium, $C_{1-6}$ alkyl, or $C_{1-6}$ haloalkyl;

$R_3$ is 6- to 9-membered heteroaryl, $-(CR_fR_g)_n$-5- to 9-membered heteroaryl, CN, $C_{3-6}$ alkyl, $C_{2-6}$ alkynyl, $C_{3-6}$ cycloalkyl, $-C(=O)R_a$, $-C(=O)NR_bR_c$, $-NR_dC(=O)R_e$, 4- to 10-membered heterocyclyl, - $(CR_hR_i)_p$-4- to 6-membered heterocycloalkyl, $C_{6-10}$ aryl, or $-(CR_jR_k)_q-C_{6-10}$ aryl, wherein the 6- to 9-membered heteroaryl, $-(CR_fR_g)_{n-}$5- to 9-membered heteroaryl, $C_{3-6}$ alkyl, $C_{3-6}$ cycloalkyl, $-C(=O)R_a$, - $C(=O)NR_bR_c$, $-NR_dC(=O)R_e$, 4- to 10-membered heterocyclyl, $-(CR_hR_i)_p$-4- to 6-membered heterocycloalkyl, $C_{6-10}$ aryl, and $-(CR_jR_k)_q-C_{6-10}$ aryl are optionally substituted by 1, 2, 3, or 4 $R_{3-1}$, wherein the $C_{2-6}$ alkynyl is optionally substituted by 1, 2, 3, or 4 $R_{3-2}$;

each $R_{3-1}$ is the same or different and is independently H, CN, OH, oxo (=O), halogen, $C_{3-6}$ cycloalkyl, or $C_{1-6}$ alkyl, wherein the $C_{3-6}$ cycloalkyl and $C_{1-6}$ alkyl are optionally substituted by 1, 2, 3, or 4 substituents independently selected from the group consisting of halogen and deuterium;

each $R_{3-2}$ is the same or different and is independently H, OH, halogen, $C_{1-6}$ alkyl, $C_{3-6}$ cycloalkyl, 4- to 6-membered heterocycloalkyl, or 4- to 9-membered heteroaryl, wherein the $C_{1-6}$ alkyl, $C_{3-6}$ cycloalkyl, 4- to 6-membered heterocycloalkyl, and 4- to 9-membered heteroaryl are optionally substituted by 1, 2, 3, or 4 substituents independently selected from the group consisting of halogen and $C_{1-6}$ alkyl;

$R_a$ is $C_{1-6}$ alkyl or $C_{3-6}$ cycloalkyl;

$R_b$, $R_c$, $R_d$, and $R_e$ are each independently H or $C_{1-6}$ alkyl;

$R_f$, $R_g$, $R_h$, $R_i$, $R_j$, and $R_k$ are each independently H, halogen, or $C_{1-6}$ alkyl;

n, p, and q are each independently 1, 2, or 3;

m is 0, 1, or 2;

each R is the same or different and is independently H, CN, halogen, $C_{1-6}$ haloalkyl, or $C_{1-6}$ alkyl;

X is CRs or N;

$R_5$ is H, CN, halogen, $C_{1-6}$ haloalkyl, or $C_{1-6}$ alkyl;

$R_4$ is $-C(=O)NR_mR_n$, $-NR_xC(=O)R_y$, $-S(=O)(=NR_v)R_w$, or $-C(=O)R_z$;

$R_m$ and $R_n$ are each independently H, $C_{1-6}$ alkyl, or $C_{1-6}$ haloalkyl, wherein the $C_{1-6}$ alkyl is optionally substituted by 1, 2, or more deuterium atoms;

$R_x$ is H or $C_{1-6}$ alkyl;

$R_k$ is $C_{1-6}$ alkyl;

$R_z$ is $C_{1-6}$ alkyl, $C_{1-6}$ haloalkyl, or 4- to 10-membered heterocycloalkyl, wherein the 4- to 10-membered heterocycloalkyl is optionally substituted by 1, 2, 3, or 4 $R_{z-1}$;

each $R_{z-1}$ is the same or different and is independently halogen, oxo (=O), or $C_{1-6}$ alkyl;

$R_v$ and $R_w$ are each independently H or $C_{1-6}$ alkyl.

According to an embodiment of the present disclosure, $R_1$ is H or methyl.

According to an embodiment of the present disclosure, $R_2$ is $C_{4-6}$ cycloalkyl or 5- to 6-membered heterocycloalkyl, wherein the $C_{4-6}$ cycloalkyl and 5- to 6-membered heterocycloalkyl may be optionally substituted by 1, 2, 3, or 4 substituents selected from the group consisting of halogen, deuterium, and $C_{1-3}$ alkyl.

According to an embodiment of the present disclosure, $R_2$ is $C_{5-6}$ cycloalkyl or 5- to 6-membered heterocycloalkyl, wherein the $C_{5-6}$ cycloalkyl and 5- to 6-membered heterocycloalkyl may be optionally substituted by 1, 2, 3, or 4 substituents selected from the group consisting of halogen and $C_{1-3}$ alkyl.

According to an embodiment of the present disclosure, $R_2$ is cyclobutyl, cyclopentyl, spiro[2.2]pentyl, bicyclo[1.1.1]pentyl, bicyclo[3.1.0]hexyl, spiro[2.3]hexyl, bicyclo[2.1.1]hexyl, cyclohexyl, or tetrahydrofuranyl, wherein the cyclobutyl, cyclopentyl, spiro[2.2]pentyl, bicyclo[1.1.1]pentyl, bicyclo[3.1.0]hexyl, spiro[2.3]hexyl, bicyclo[2.1.1]hexyl, cyclohexyl, and tetrahydrofuranyl may be optionally substituted by 1, 2, 3, or 4 substituents selected from the group consisting of F, Cl, deuterium, $-CH_2F$, $-CHF_2$, $-CF_3$, and methyl.

According to an embodiment of the present disclosure, $R_2$ is

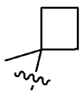 , 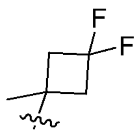 ,

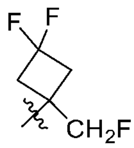 , 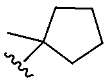 , 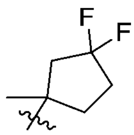 , 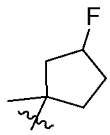 , 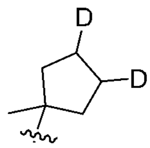 , 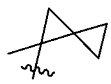 , 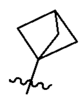 , 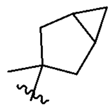 ,

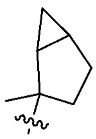 , 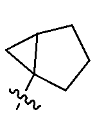 , 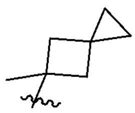 , 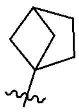 , 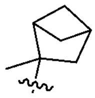 , 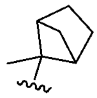 , 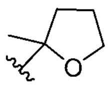

(such as

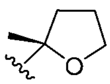

or

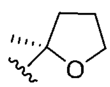

),

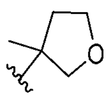 , 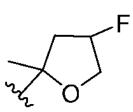 , or 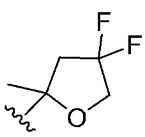

(such as

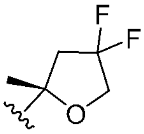 or 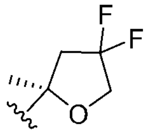

).

According to an embodiment of the present disclosure, $R_3$ is 6-membered heteroaryl, - $(CR_fR_g)_n$-5- to 6-membered heteroaryl, CN, $C_{3-6}$ alkyl, $C_{2-4}$ alkynyl, $C_{3-5}$ cycloalkyl, $-C(=O)R_a$, - $C(=O)NR_bR_c$, $-NR_dC(=O)R_e$, 4- to 8-

membered heterocyclyl, $-(CR_hR_i)_p$-4- to 6-membered heterocycloalkyl, phenyl, or $-(CR_jR_k)_q$-phenyl, wherein the 6-membered heteroaryl, $-(CR_fR_g)_n$-5- to 6-membered heteroaryl, $C_{3-6}$ alkyl, $C_{3-5}$ cycloalkyl, $-C(=O)R_a$, $-C(=O)NR_bR_c$, $-NR_dC(=O)R_e$, 4- to 8-membered heterocyclyl, $-(CR_hR_i)_p$-4- to 6-membered heterocycloalkyl, phenyl, and $-(CR_jR_k)_q$-phenyl are optionally substituted by 1, 2, 3, or 4 $R_{3-1}$, wherein the $C_{2-4}$ alkynyl is optionally substituted by 1, 2, 3, or 4 $R_{3-2}$, wherein $R_a$, $R_b$, $R_c$, $R_d$, $R_e$, $R_f$, $R_g$, $R_h$, $R_i$, $R_j$, $R_k$, $R_{3-1}$, n, p, and q each independently have the definition as described in any one embodiment of the present disclosure.

According to an embodiment of the present disclosure, $R_a$ is methyl, ethyl, *n*-propyl, isopropyl, *n*-butyl, isobutyl, *sec*-butyl, *tert*-butyl, cyclopropyl, or cyclobutyl.

According to an embodiment of the present disclosure, $R_b$, $R_c$, $R_d$, and $R_e$ are each independently H, methyl, ethyl, *n*-propyl, isopropyl, *n*-butyl, isobutyl, *sec*-butyl, or *tert*-butyl.

According to an embodiment of the present disclosure, $R_f$, $R_g$, $R_h$, $R_i$, $R_j$, and $R_k$ are each independently H, F, or methyl.

According to an embodiment of the present disclosure, $R_3$ is pyridinyl, pyrimidinyl, pyrazinyl, pyridazinyl, $-CH(CH_3)$-pyridinyl, $-CH(CH_3)$-oxazolyl, CN, *n*-propyl, isopropyl, *n*-butyl, *tert*-butyl, ethynyl, 1-propynyl, 2-propynyl, 2-butynyl, 3-butynyl, cyclopropyl, cyclobutyl, $-C(=O)C_{1-4}$ alkyl, - $C(=O)C_{3-4}$ cycloalkyl, $-C(=O)N(C_{1-3}$ alkyl$)(C_{1-4}$ alkyl$)$, $-NHC(=O)C_{1-3}$ alkyl, oxetanyl, azetidinyl, pyrrolidinyl, tetrahydrofuranyl,

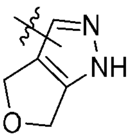, 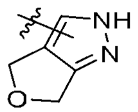, 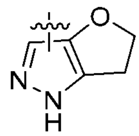, 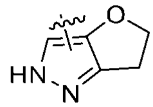, 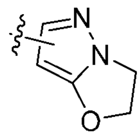,

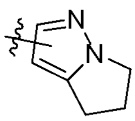, 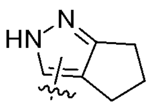, 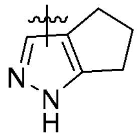, 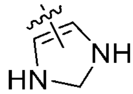,

$-CH(CH_3)$-azetidinyl, phenyl, or $-CH_2$-phenyl, wherein the pyridinyl, pyrimidinyl, pyrazinyl, pyridazinyl, $-CH(CH_3)$-pyridinyl, $-CH(CH_3)$-oxazolyl, *n*-propyl, isopropyl, *n*-butyl, *tert*-butyl, cyclopropyl, cyclobutyl, $-C(=O)C_{1-4}$ alkyl, $-C(=O)C_{3-4}$ cycloalkyl, $-C(=O)N(C_{1-3}$ alkyl$)(C_{1-4}$ alkyl$)$, $-NHC(=O)C_{1-3}$ alkyl, oxetanyl, azetidinyl, pyrrolidinyl, tetrahydrofuranyl,

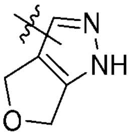, 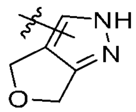, 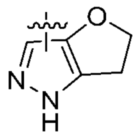, 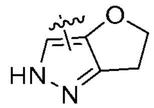, 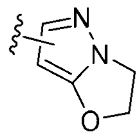, 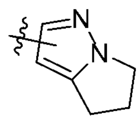,

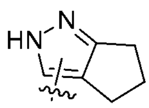, 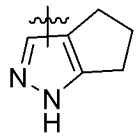, 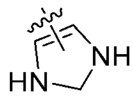,

$-CH(CH_3)$-azetidinyl, phenyl, and $-CH_2$-phenyl are optionally substituted by 1, 2, 3, or 4 $R_{3-1}$, wherein the ethynyl, 1-propynyl, 2-propynyl, 2-butynyl, and 3-butynyl are optionally substituted by 1, 2, 3, or 4 $R_{3-2}$, wherein $R_{3-1}$ and $R_{3-2}$ have the definition as described in any one embodiment of the present disclosure.

According to an embodiment of the present disclosure, each $R_{3-1}$ is the same or different and is independently H, CN, OH, oxo (=O), F, Cl, Br, methyl, ethyl, cyclopropyl, $-CH_2F$, $-CHF_2$, $-CF_3$, or - $CD_3$.

According to an embodiment of the present disclosure, each $R_{3-2}$ is the same or different and is independently H, OH, halogen, $C_{1-6}$ alkyl, $C_{3-6}$ cycloalkyl, 4- to 6-membered heterocycloalkyl, or 5-to 9-membered heteroaryl, wherein the $C_{1-6}$ alkyl, $C_{3-6}$ cycloalkyl, 4- to 6-membered heterocycloalkyl, and 5- to 9-membered heteroaryl are optionally substituted by 1, 2, 3, or 4 substituents independently selected from the group consisting of halogen and $C_{1-6}$ alkyl.

According to an embodiment of the present disclosure, each $R_{3-2}$ is the same or different and is independently $C_{3-5}$ cycloalkyl, 4- to 5-membered heterocycloalkyl, or 5- to 6-membered heteroaryl, wherein the $C_{3-5}$ cycloalkyl, 4- to 5-membered heterocycloalkyl, and 5- to 6-membered heteroaryl are optionally substituted by 1, 2, 3, or 4 substituents independently selected from the group consisting of halogen and $C_{1-3}$ alkyl.

According to an embodiment of the present disclosure, each $R_{3-2}$ is the same or different and is independently cyclopropyl, cyclobutyl, oxetanyl, azetidinyl, pyridyl, pyrazolyl, or bicyclo[1.1.1]pentyl, wherein the cyclopropyl, cyclobutyl, oxetanyl, azetidinyl, pyridyl, pyrazolyl, and bicyclo[1.1.1]pentyl are optionally substituted by 1, 2, 3, or 4 substituents independently selected from the group consisting of F and methyl.

According to an embodiment of the present disclosure, each $R_{3-2}$ is the same or different and is independently

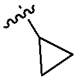, 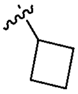, 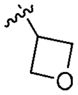, 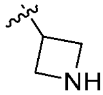, 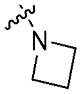, 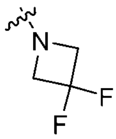, 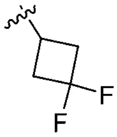, 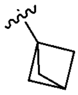,

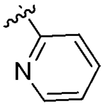, or 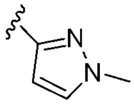.

According to an embodiment of the present disclosure, $R_3$ is

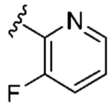, 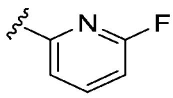,

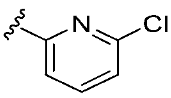, 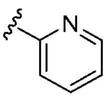, 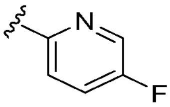, 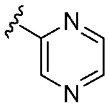, 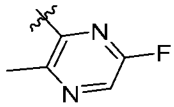, 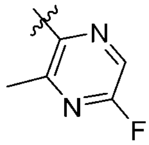, 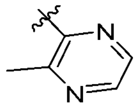,

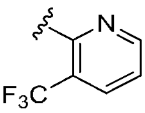, 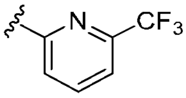, 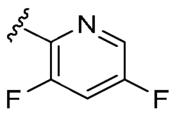, 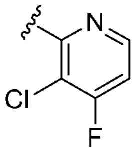, 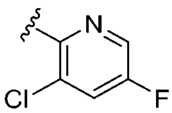, 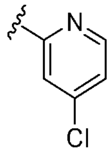,

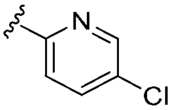, 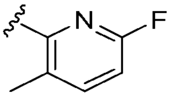, 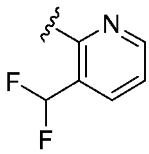, 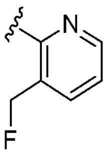, 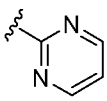, 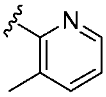, 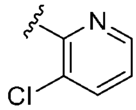,

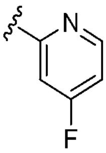, 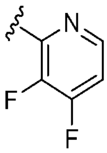, 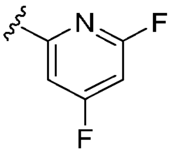, 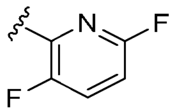, 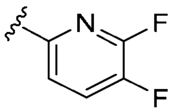, 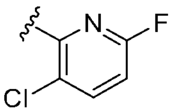,

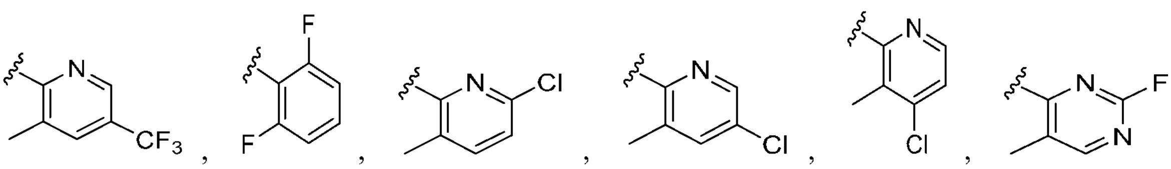
, , , , , ,

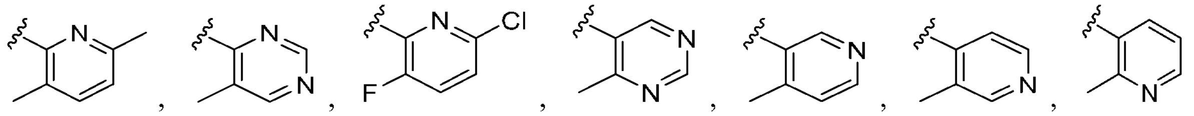
, , , , , , ,

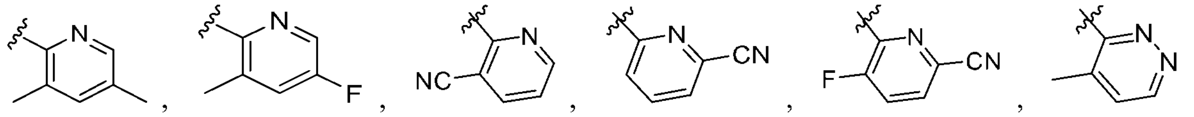
, , , , , ,

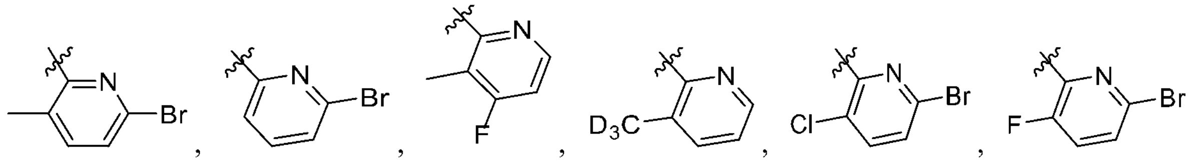
, , , , , ,

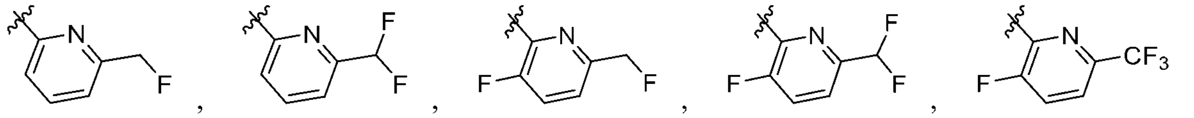
, , , , ,

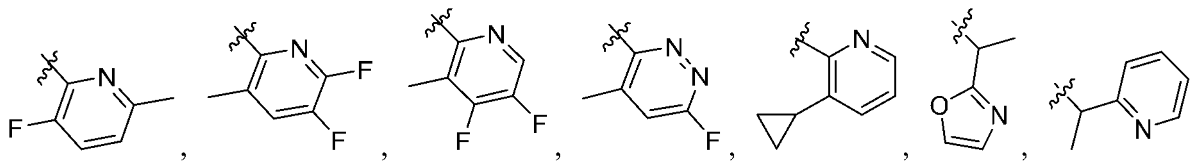
, , , , , , ,

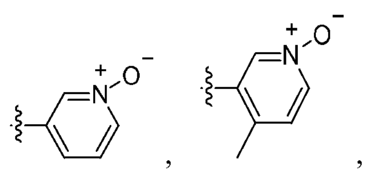
, ,

CN, isopropyl, *tert*-butyl, cyclopropyl,

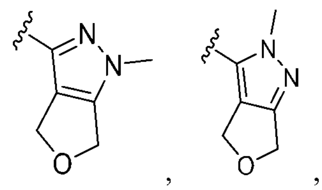
, ,

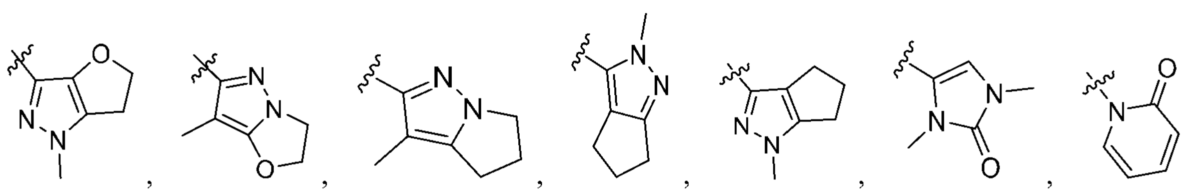
, , , , , , ,

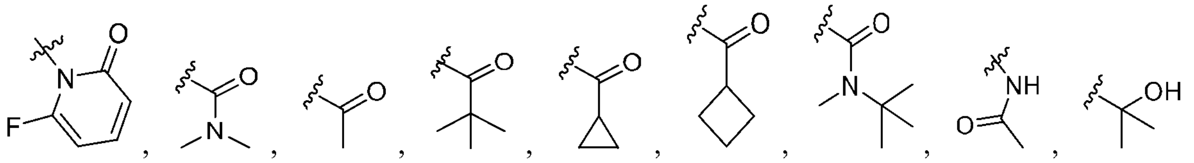
, , , , , , , , ,

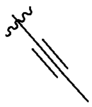, 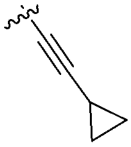, 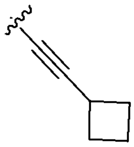, 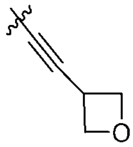, 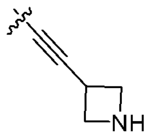, 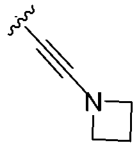, 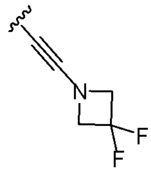,

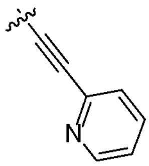, 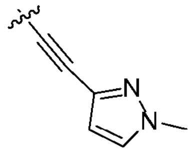, 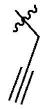, 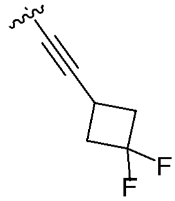, 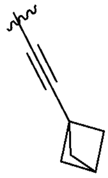, 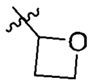, 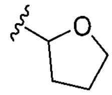, 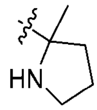,

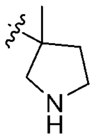, 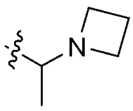, 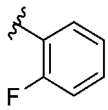,

phenyl, or benzyl; preferably, the

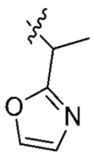, 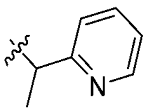, 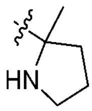,

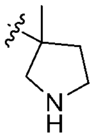, or 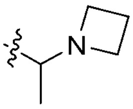

is

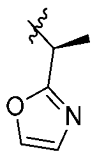, 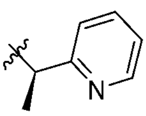, 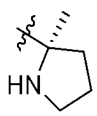, 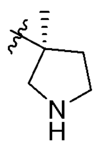, or 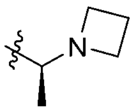.

According to an embodiment of the present disclosure, $R_m$ and $R_n$ are each independently H, methyl, ethyl, $CD_3$, *n*-propyl, isopropyl, $-CH_2CH_2F$, $-CH_2CHF_2$, or $-CH_2CF_3$.

According to an embodiment of the present disclosure, $R_x$ is H or methyl; $R_y$ is methyl or ethyl.

According to an embodiment of the present disclosure, $R_v$ is H, methyl, or ethyl; $R_w$ is methyl or ethyl.

According to an embodiment of the present disclosure, $R_z$ is $C_{1-3}$ alkyl, $C_{1-3}$ haloalkyl, or 5- to 8-membered heterocycloalkyl, wherein the 5- to 8-membered heterocycloalkyl is optionally substituted by 1, 2, 3, or 4 $R_{z-1}$, wherein $R_{z-1}$ has the definition as described in any one embodiment of the present disclosure; preferably, the 5- to 8-membered heterocycloalkyl contains 1, 2, or 3 N and 0, 1, or 2 O or S.

According to an embodiment of the present disclosure, $R_z$ is

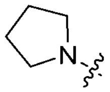, 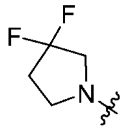,

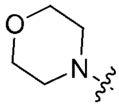, 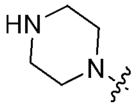, 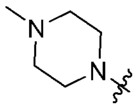, 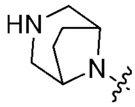, 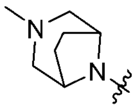, 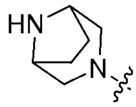, or 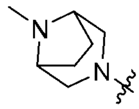.

According to an embodiment of the present disclosure, each $R_{z-1}$ is the same or different and is independently F, Cl, oxo (=O), methyl, or ethyl.

According to an embodiment of the present disclosure, $R_4$ is

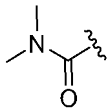, 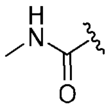, 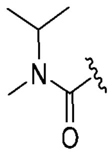,

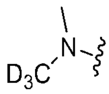, 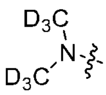, 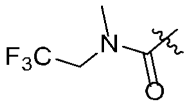, 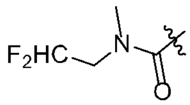, 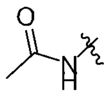, 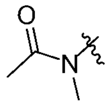, 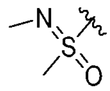, 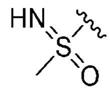,

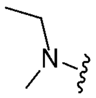, 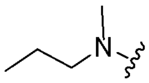, 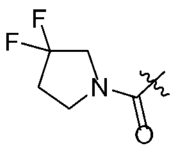, 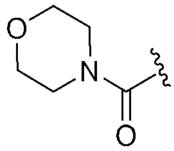, 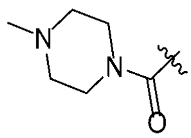, 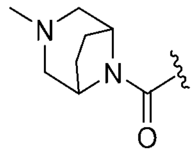,

or

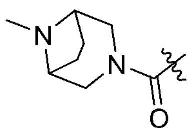.

According to an embodiment of the present disclosure, m is 0 or 1.

According to an embodiment of the present disclosure, each R is the same or different and is independently H, CN, F, Cl, $-CH_3$, or $-CH_2F$.

According to an embodiment of the present disclosure, $R_5$ is H, CN, F, Cl, $-CH_3$, or $-CH_2F$.

According to an embodiment of the present disclosure, X is CH, C(CN), CF, C(Cl), $C(CH_3)$, $C(CH_2F)$, or N.

According to an embodiment of the present disclosure, when $R_2$ is 5- to 6-membered heterocycloalkyl, $R_3$ is not $C_{6-10}$ aryl.

According to an embodiment of the present disclosure, when $R_2$ is 5- to 6-membered heterocycloalkyl, $R_3$ is not phenyl.

According to an embodiment of the present disclosure, when $R_2$ is tetrahydrofuranyl, $R_3$ is not $C_{6-10}$ aryl.

According to an embodiment of the present disclosure, when $R_2$ is tetrahydrofuranyl, $R_3$ is not phenyl.

According to an embodiment of the present disclosure, the compound represented by formula (I) has a structure represented by the following formula (I-a) or (I-b):

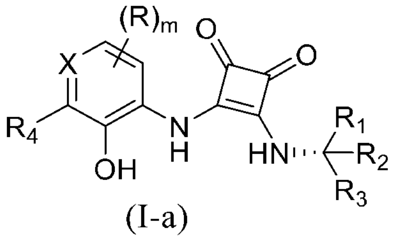
(I-a)
or
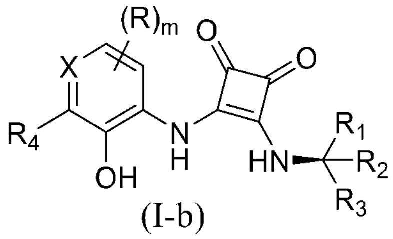
(I-b)
,

wherein X, m, R, $R_1$, $R_2$, $R_3$, and $R_4$ each independently have the definition as described in any one embodiment of the present disclosure.

According to an embodiment of the present disclosure, the compound represented by formula (I) has a structure represented by any one of the following formulas (I-1) to (I-3):

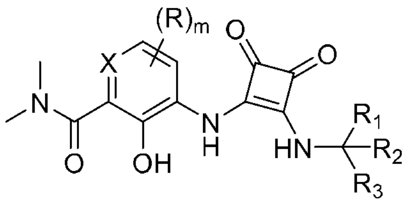
(I-1)
,
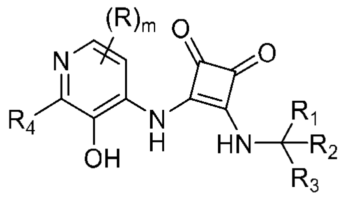
(I-2)
, or
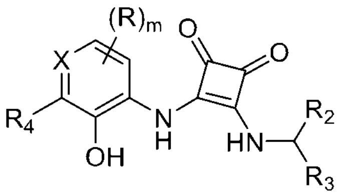
(I-3)
,

wherein X, m, R, $R_1$, $R_2$, $R_3$, and $R_4$ each independently have the definition as described in any one embodiment of the present disclosure.

According to an embodiment of the present disclosure, the compound represented by formula (I) has a structure represented by the following formula (II-1):

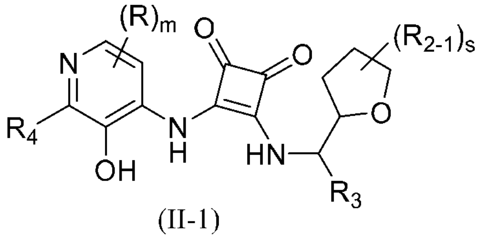
(II-1)
,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-2):

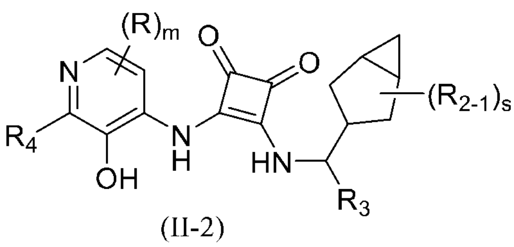
(II-2)
,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-3):

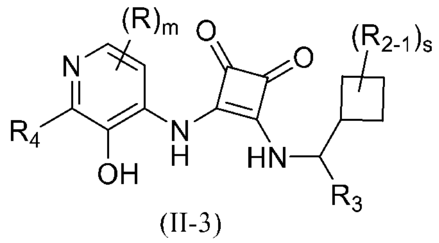

(II-3)

,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II):

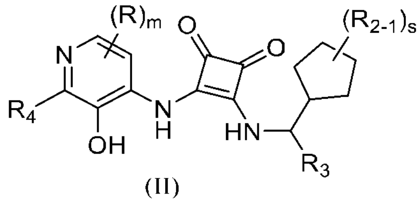

(II)

,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-1a) or formula (II-1b):

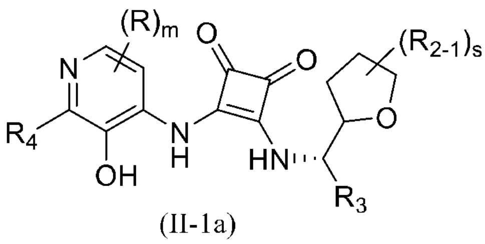

(II-1a)

or

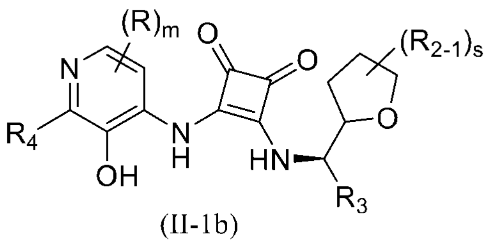

(II-1b)

,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-2a) or formula (II-2b):

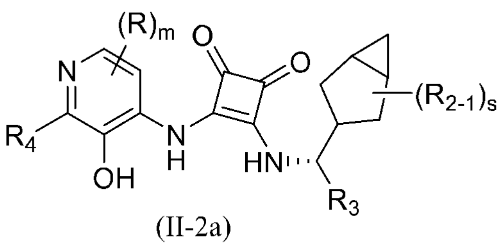

(II-2a)

or

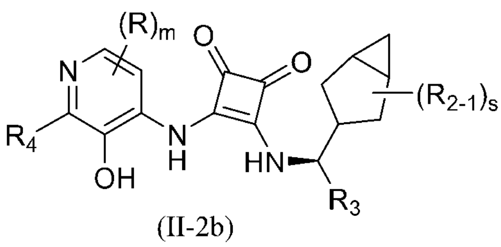

(II-2b)

,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-3a) or formula (II-3b):

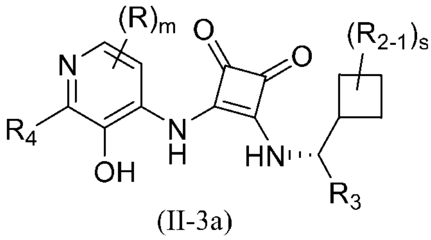

(II-3a) or

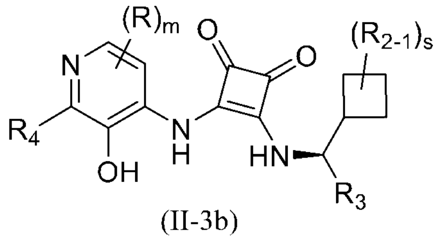

(II-3b) ,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (II-a) or formula (II-b):

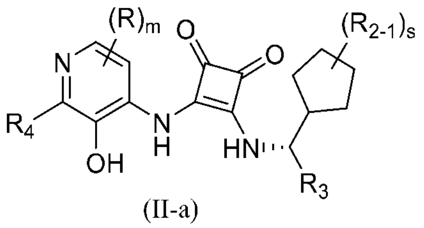

(II-a) or

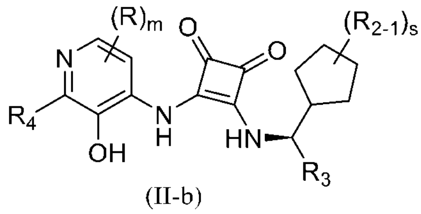

(II-b) ,

wherein m, R, $R_{2-1}$, $R_3$, and $R_4$ each independently have the definition as described in any one embodiment of the present disclosure, and s is 0, 1, 2, or 3.

According to an embodiment of the present disclosure, the compound represented by formula (I) has a structure represented by the following formula (III-1):

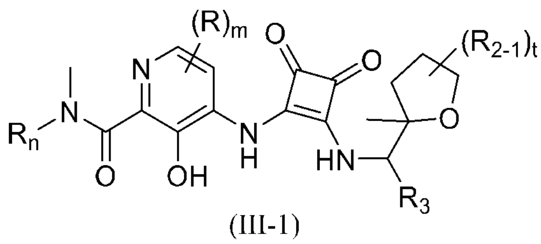

(III-1) ,

wherein t is 0, 1, or 2; m, $R_{2-1}$, $R_n$, R, and $R_3$ each independently have the definition as described in any one embodiment of the present disclosure.

According to an embodiment of the present disclosure, the compound represented by formula (I) has a structure represented by the following formula (III-2):

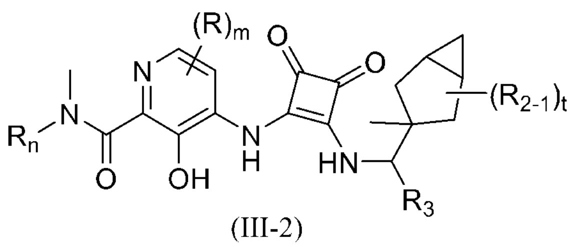

(III-2) ,

wherein t is 0, 1, or 2; m, $R_{2-1}$, $R_n$, R, and $R_3$ each independently have the definition as described in any one embodiment of the present disclosure.

According to an embodiment of the present disclosure, the compound represented by formula (I) has a structure represented by the following formula (III-3):

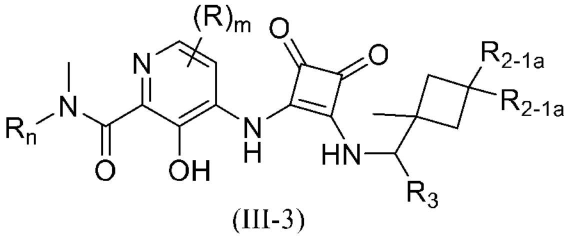

(III-3)

,

wherein t is 0, 1, or 2; each $R_{2\text{-}1a}$ is the same or different and is independently $R_{2\text{-}1}$, or two $R_{2\text{-}1a}$ together with the carbon atom to which they are attached form a cyclopropyl or a cyclobutyl; m, $R_{2\text{-}1}$, $R_n$, R, and $R_3$ each independently have the definition as described in any one embodiment of the present disclosure.

According to an embodiment of the present disclosure, the compound represented by formula (I) has a structure represented by the following formula (III):

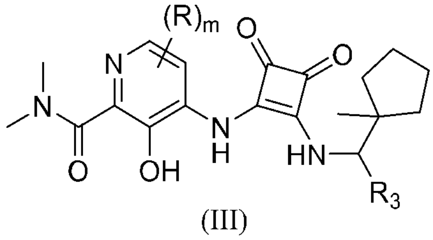

(III)

,

wherein m, R, and $R_3$ each independently have the definition as described in any one embodiment of the present disclosure.

According to an embodiment of the present disclosure, the compound represented by formula (I) has a structure represented by the following formula (III-1a) or formula (III-1b):

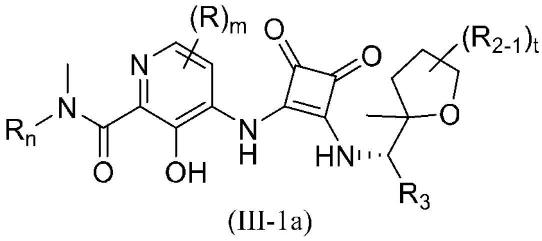

(III-1a)

or

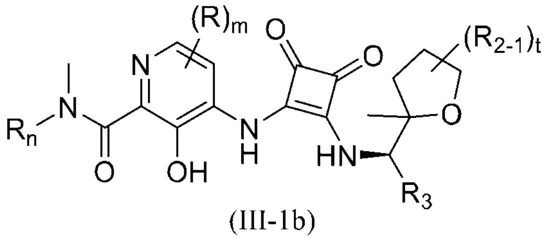

(III-1b)

,

wherein t is 0, 1, or 2; m, $R_{2\text{-}1}$, $R_n$, R, and $R_3$ each independently have the definition as described in any one embodiment of the present disclosure.

According to an embodiment of the present disclosure, the compound represented by formula (I) has a structure represented by the following formula (III-2a) or formula (III-2b):

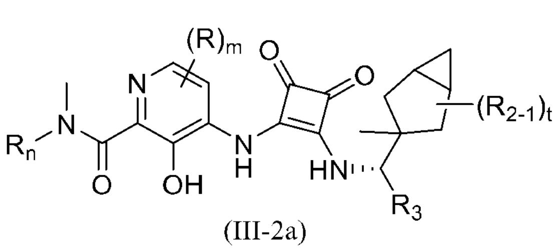

(III-2a)

or

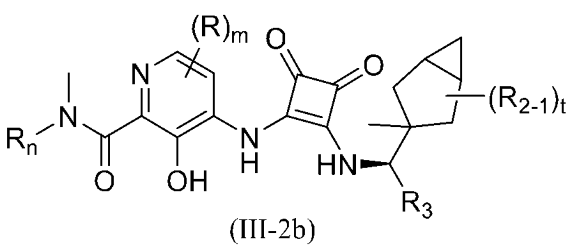

(III-2b)

,

wherein t is 0, 1, or 2; m, $R_{2\text{-}1}$, $R_n$, R, and $R_3$ each independently have the definition as described in any one embodiment of the present disclosure.

According to an embodiment of the present disclosure, the compound represented by formula (I) has a structure represented by the following formula (III-3a) or formula (III-3b):

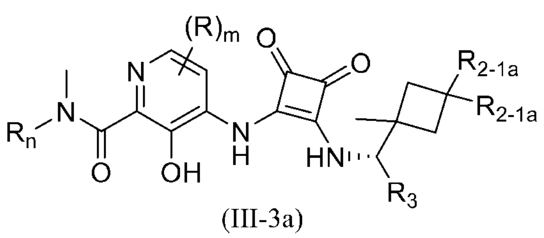

(III-3a)

or

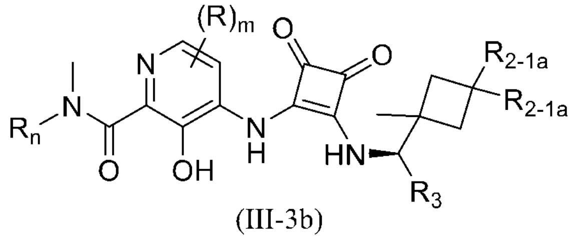

(III-3b)

,

wherein t is 0, 1, or 2; each $R_{2\text{-}1a}$ is the same or different and is independently $R_{2\text{-}1}$, or two $R_{2\text{-}1a}$ together with the carbon atom to which they are attached form a cyclopropyl or a cyclobutyl; m, $R_{2\text{-}1}$, $R_n$, R, and $R_3$ each independently have the definition as described in any one embodiment of the present disclosure.

According to an embodiment of the present disclosure, the compound represented by formula (I) has a structure represented by the following formula (III-a) or formula (III-b):

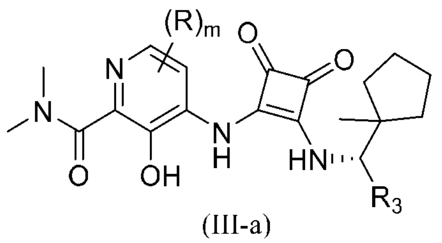

(III-a)

or

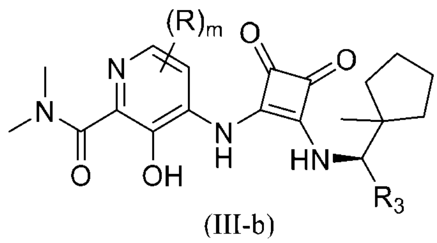

(III-b)

,

wherein m, R, and $R_3$ each independently have the definition as described in any one embodiment of the present disclosure.

According to an embodiment of the present disclosure, the compound represented by formula (I) has the structure shown below:

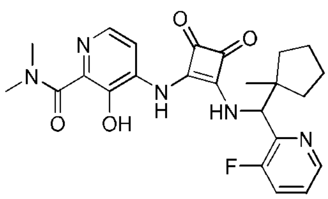

,

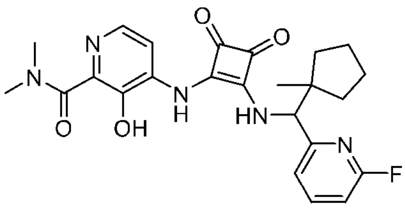

,

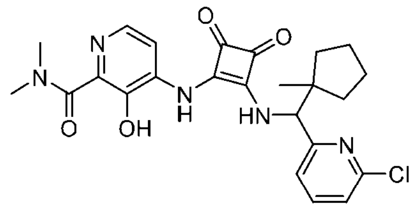

,

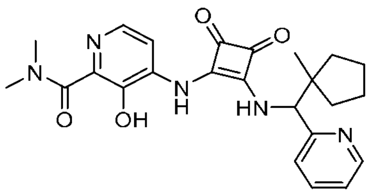

,

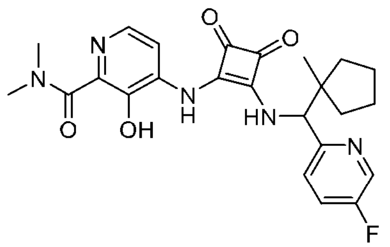

,

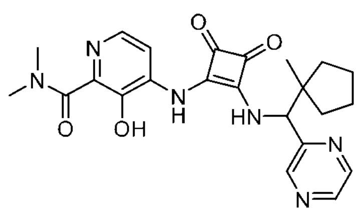

,

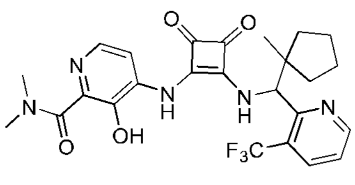

,

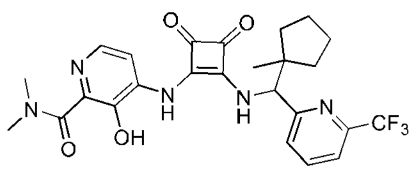

,

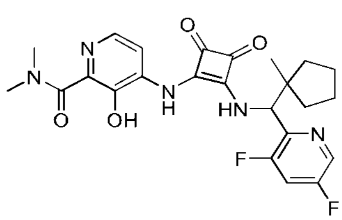

,

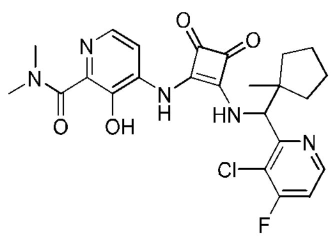 , 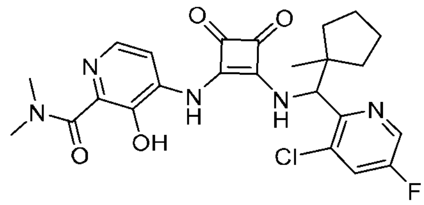 , 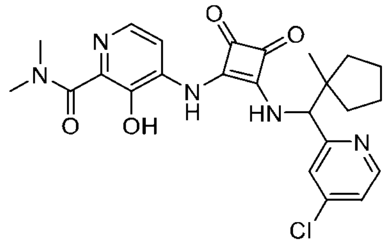 ,

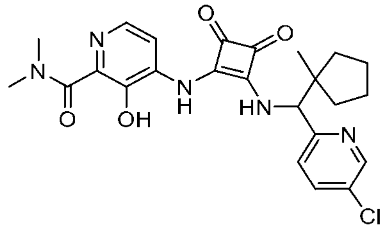 , 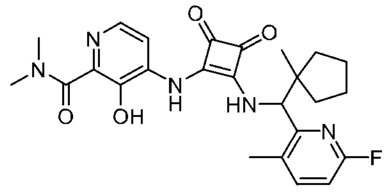 , 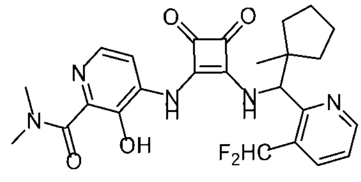 ,

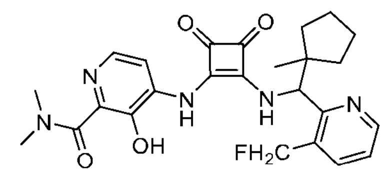 , 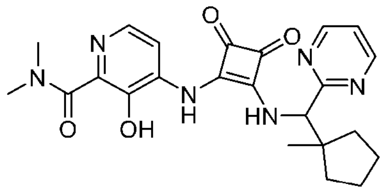 , 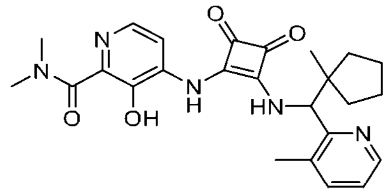 ,

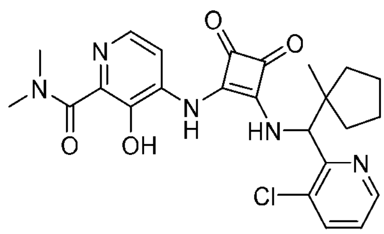 , 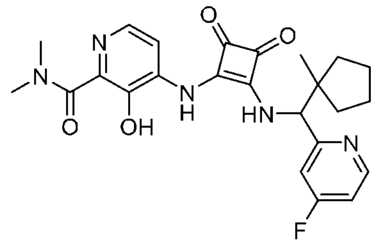 , 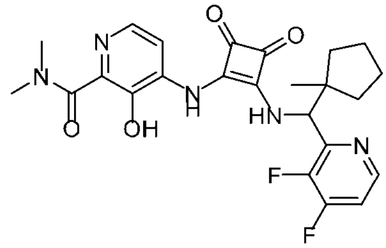 ,

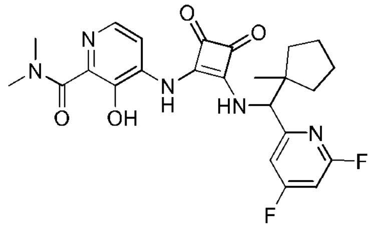 , 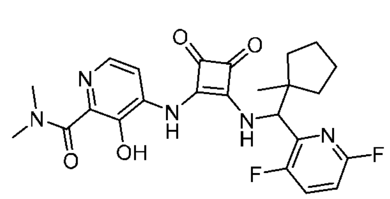 , 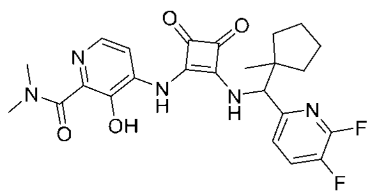 ,

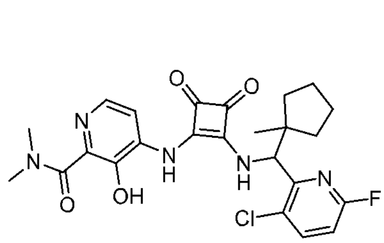 , 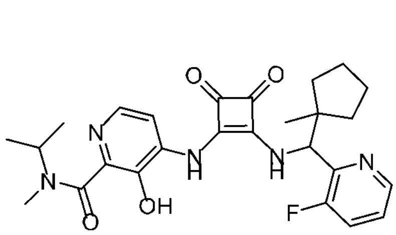 , 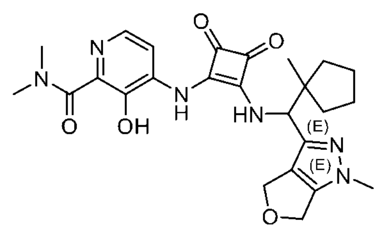 ,

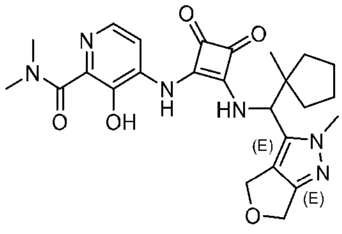, 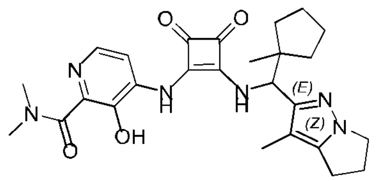, 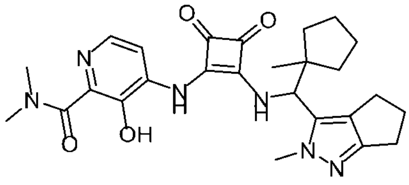,

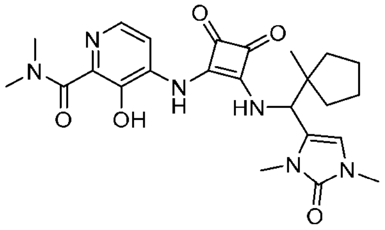, 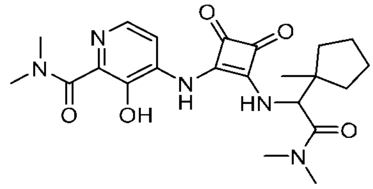, 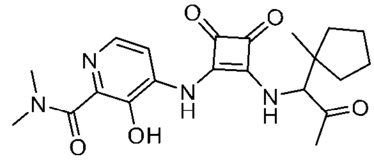,

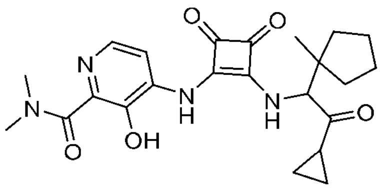, 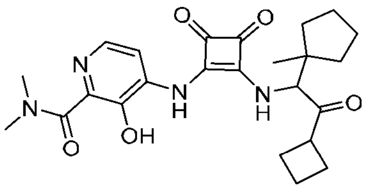, 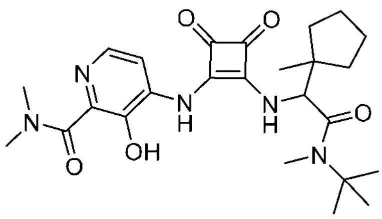,

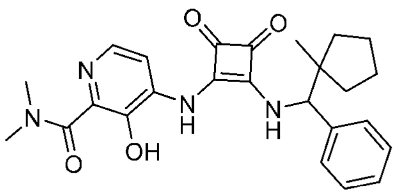, 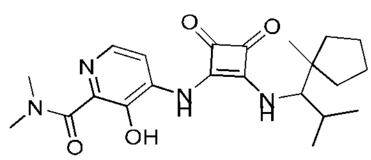, 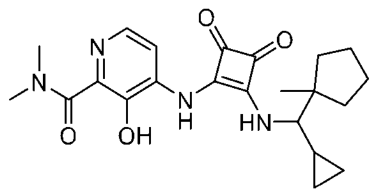,

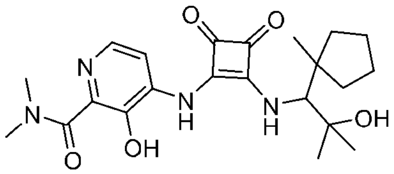, 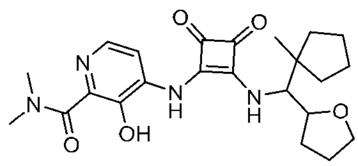, 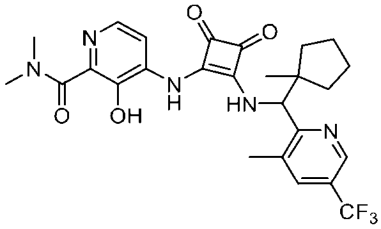,

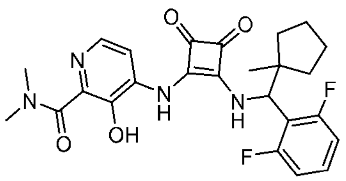, 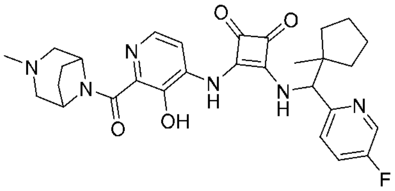, 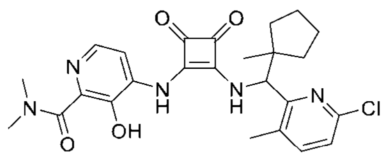,

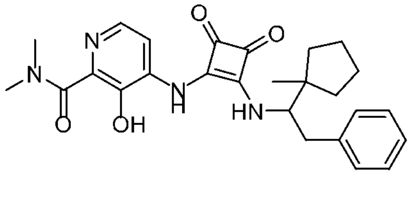, 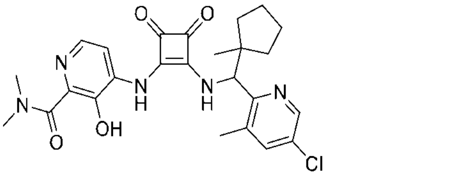,

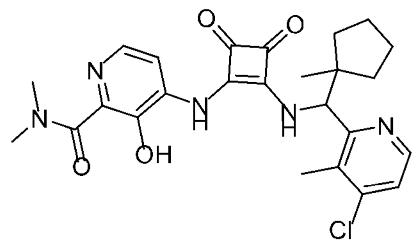

,

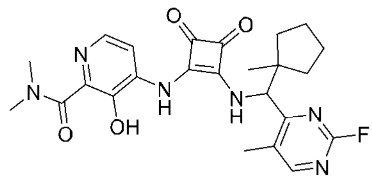

,

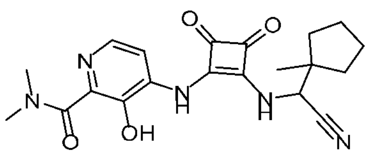

,

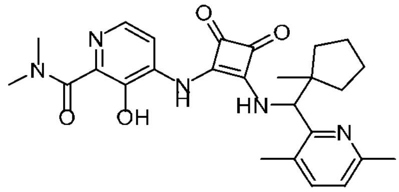

,

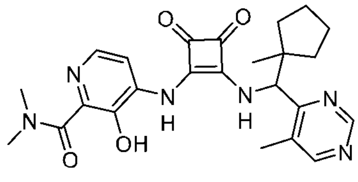

,

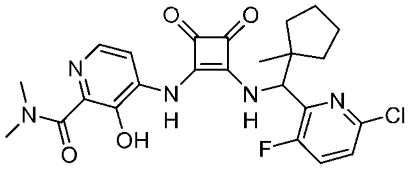

,

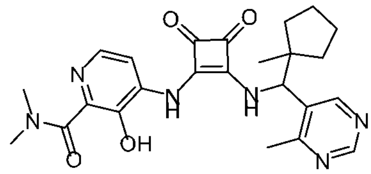

,

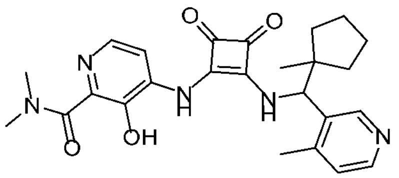

,

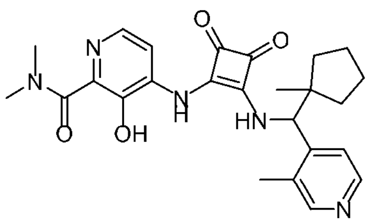

,

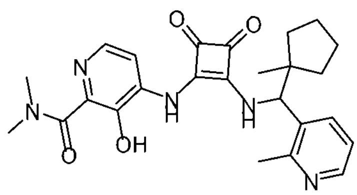

,

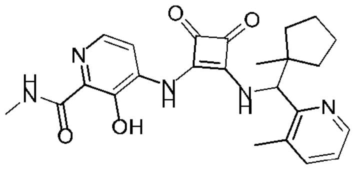

,

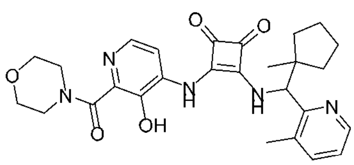

,

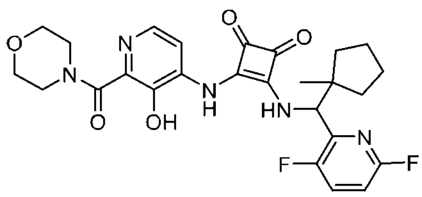

,

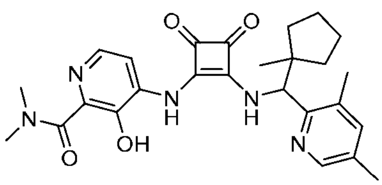

,

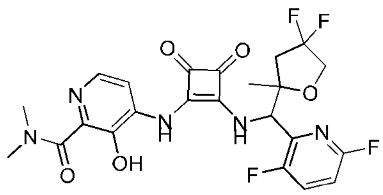

,

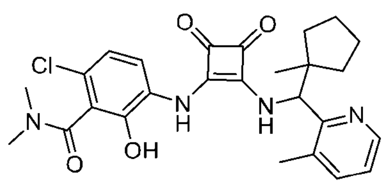

,

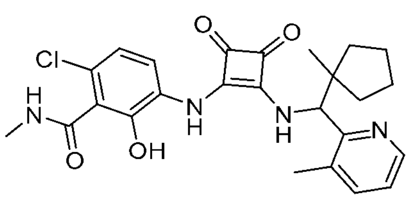

,

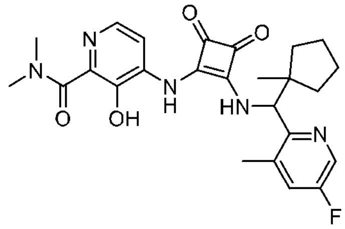

,

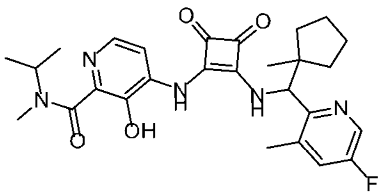

,

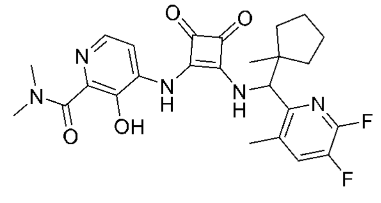, 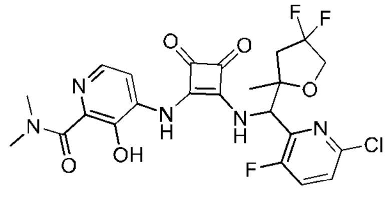, 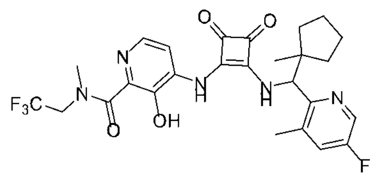,

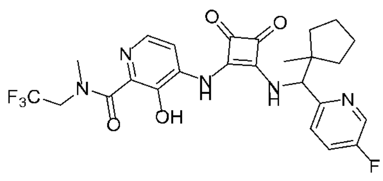, 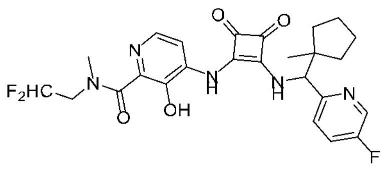, 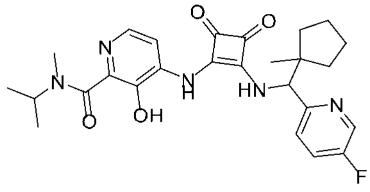,

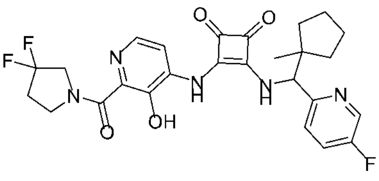, 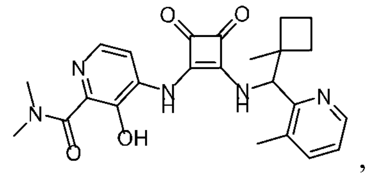, 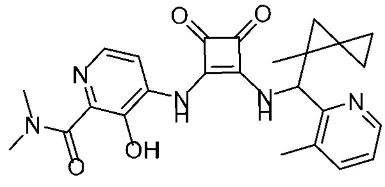,

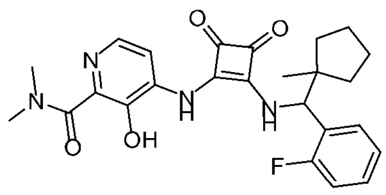, 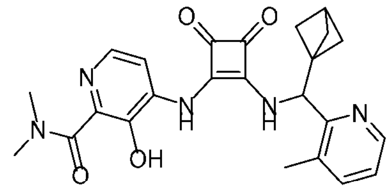, 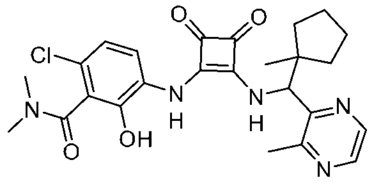,

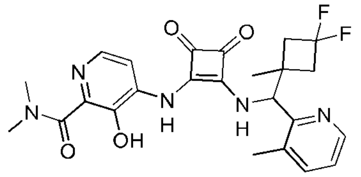, 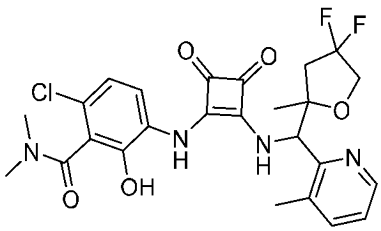, 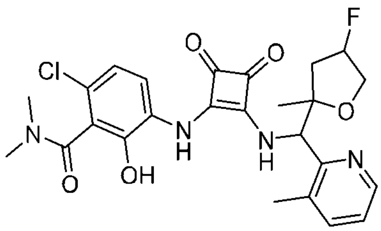,

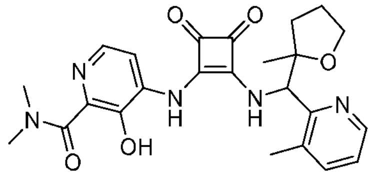, 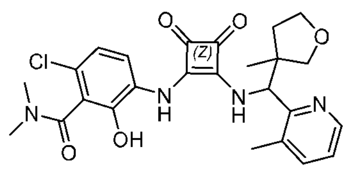, 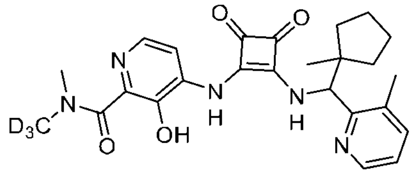,

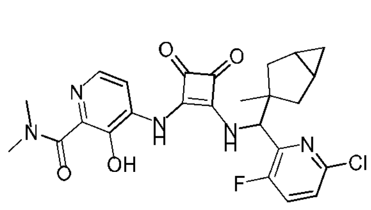, 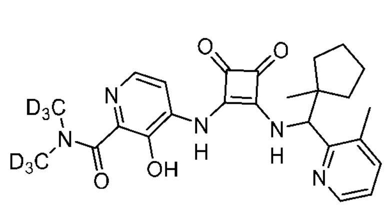, 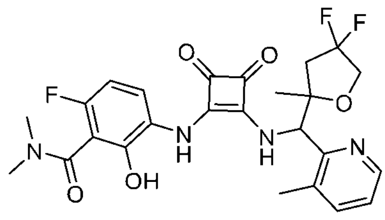,

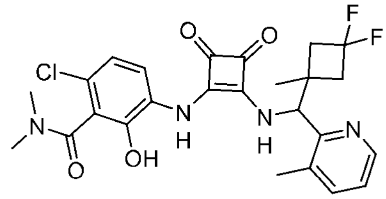, 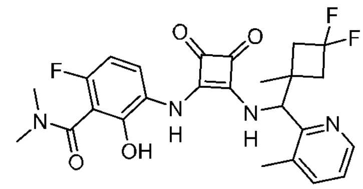, 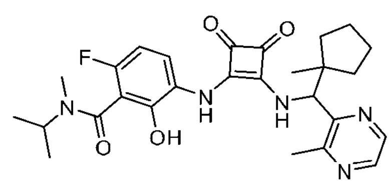,

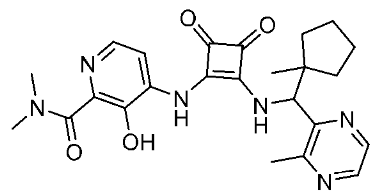, 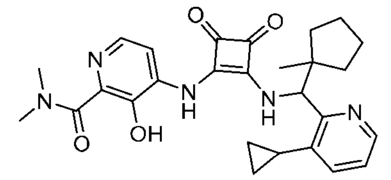, 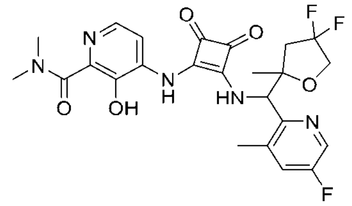,

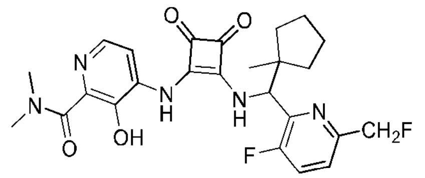, 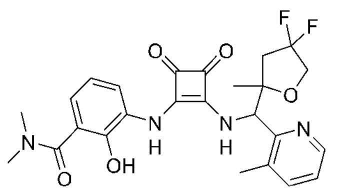, 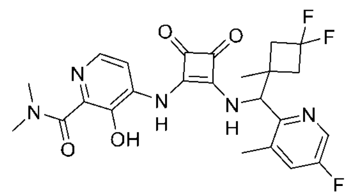,

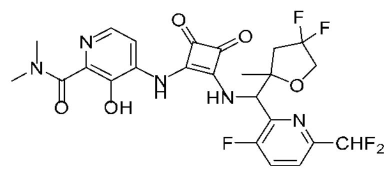, 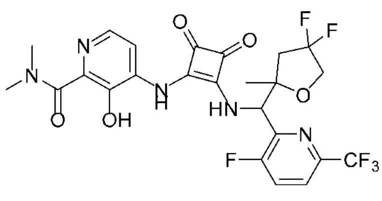, 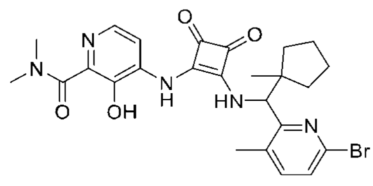,

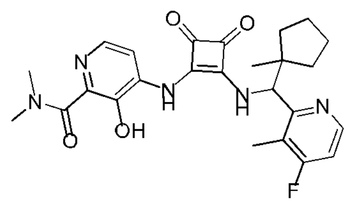, 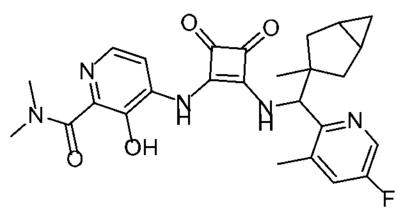, 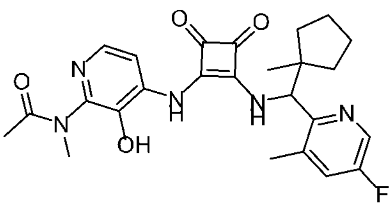,

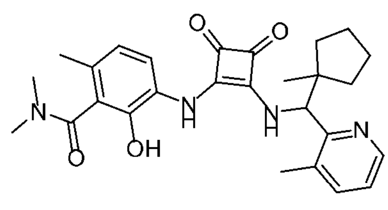, 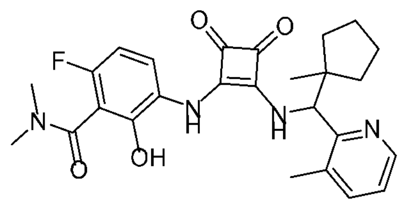, 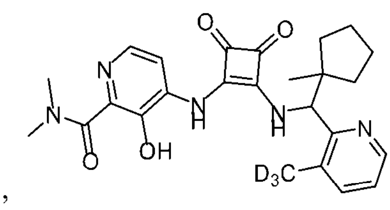,

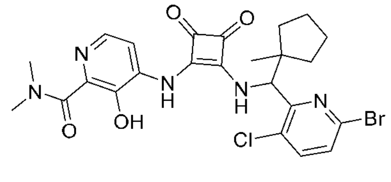, 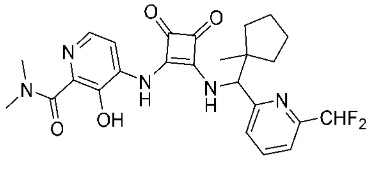, 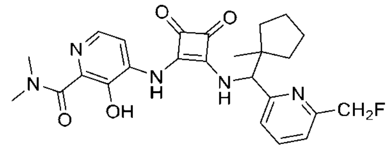,

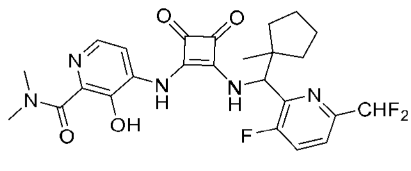,
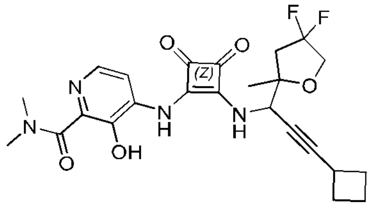,
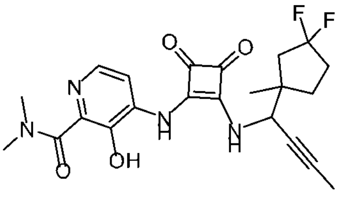,

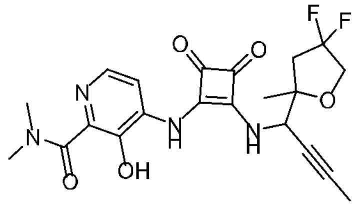,
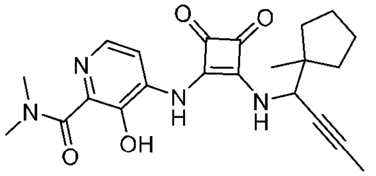,
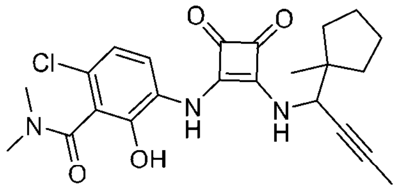,

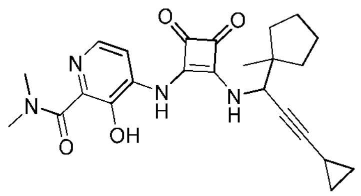,
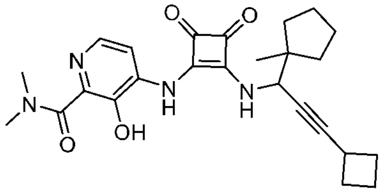,
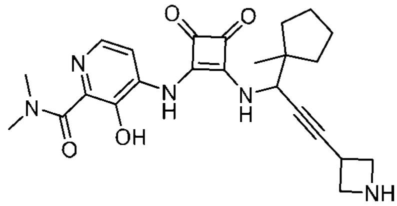,

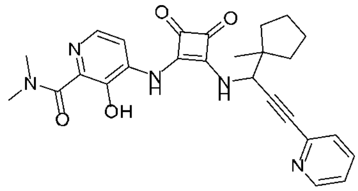,
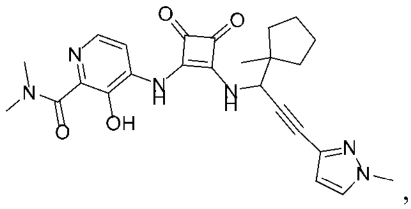,
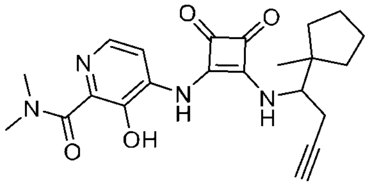,

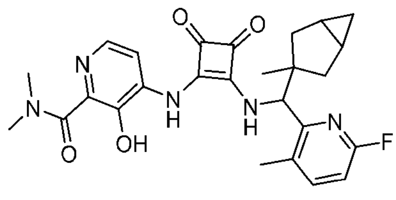,
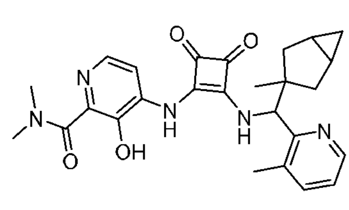,
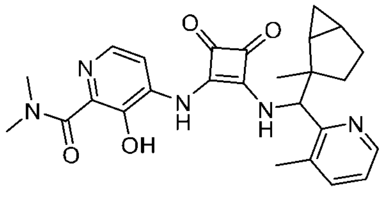,

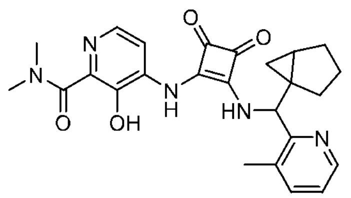,
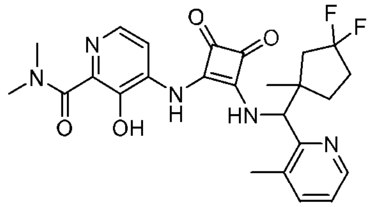,
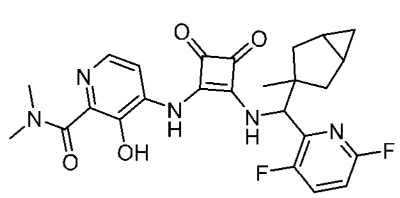,

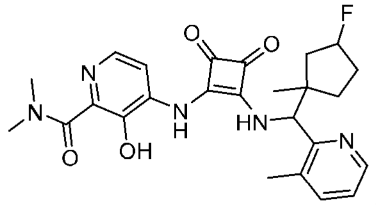,
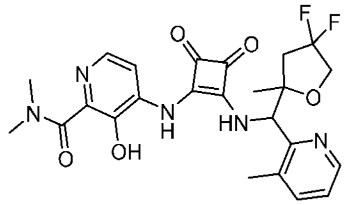,
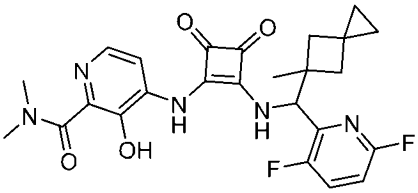,

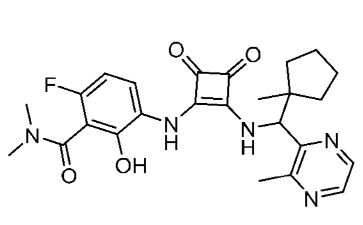 , 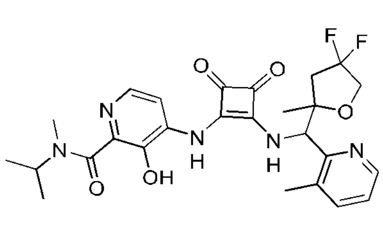 , 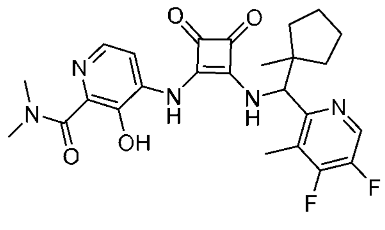 ,

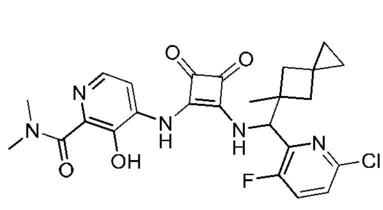 , 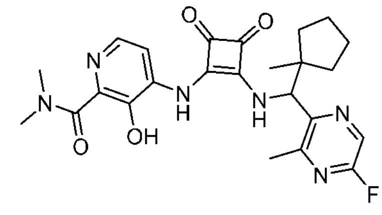 , 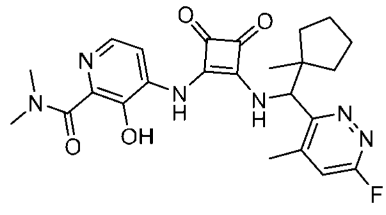 ,

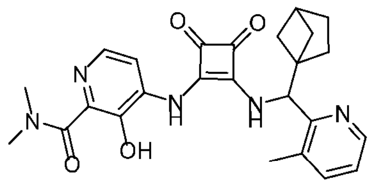 , 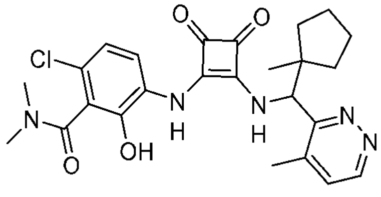 , 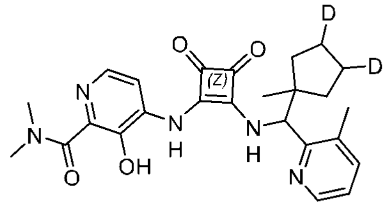 ,

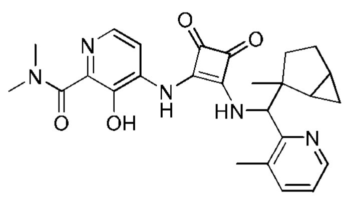 , 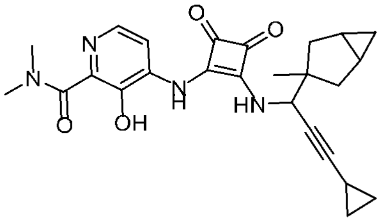 , 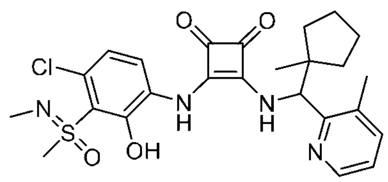 ,

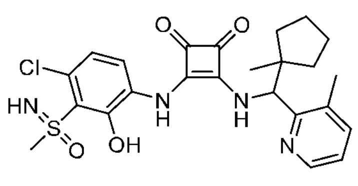 , 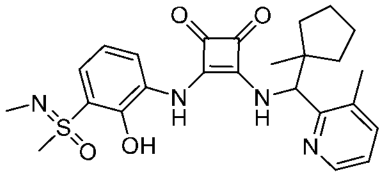 , 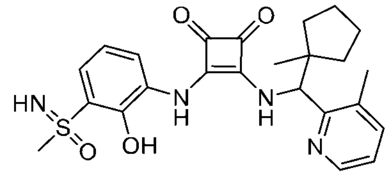 ,

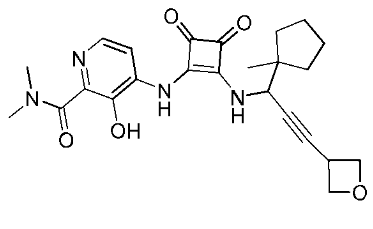 , 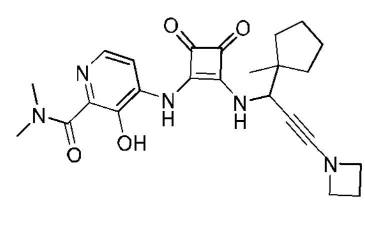 , 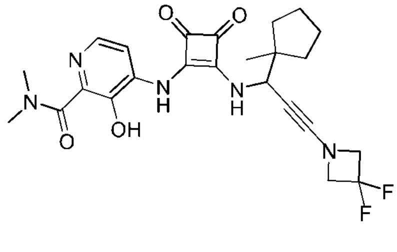 ,

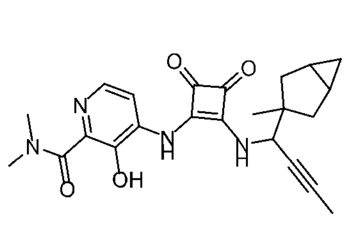 , 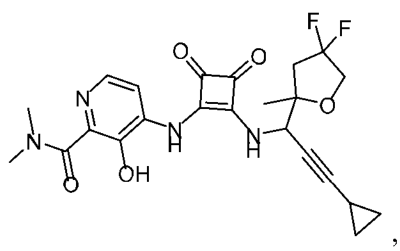 , 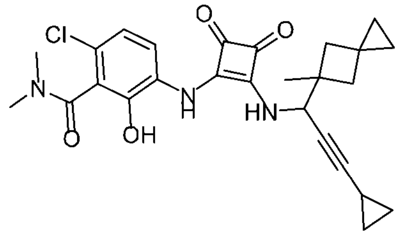 ,

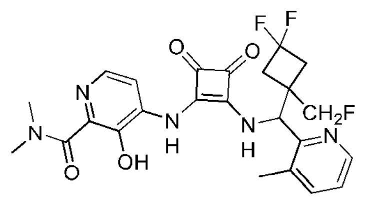,
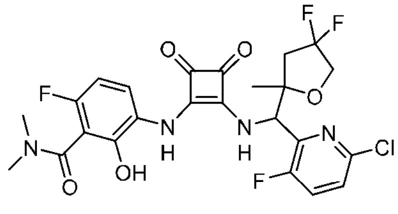,
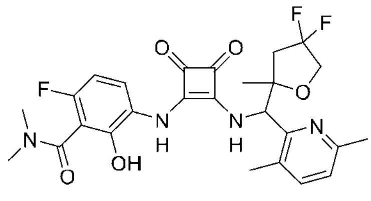,

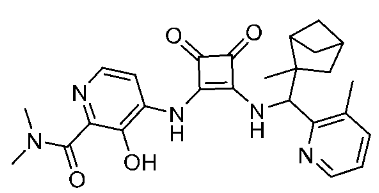,
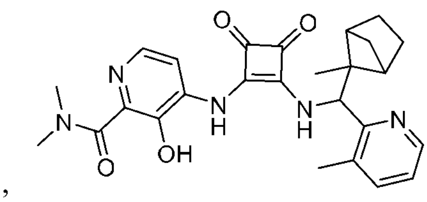,
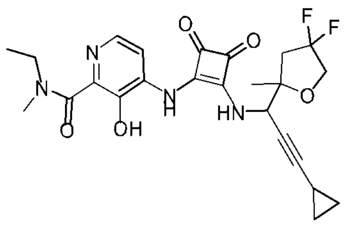,

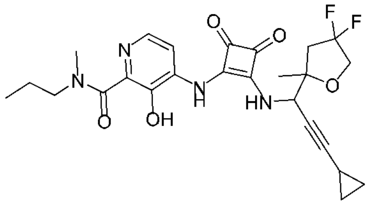,
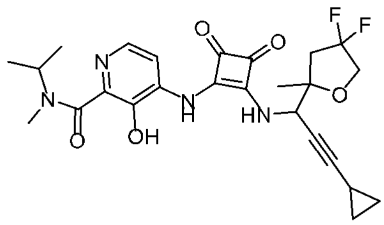,
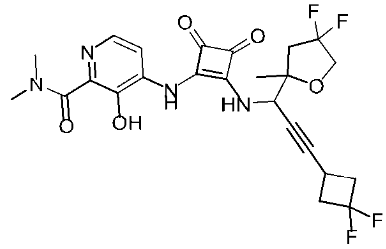,

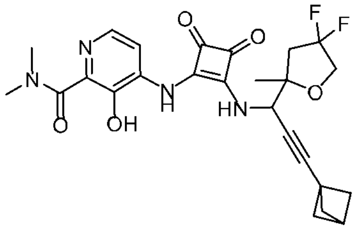,
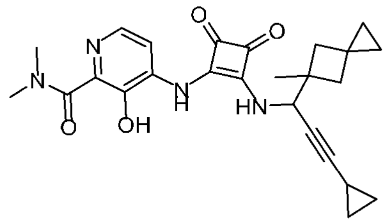,
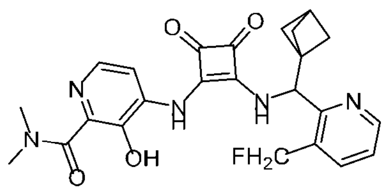,

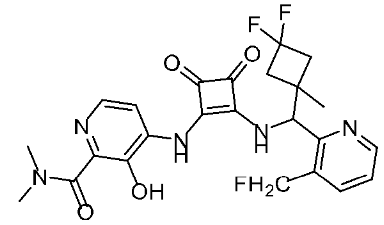,
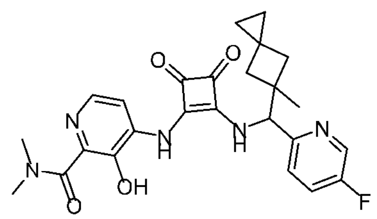,
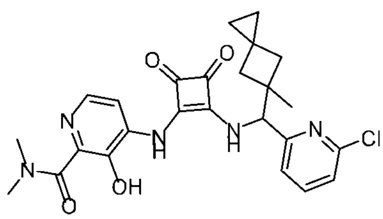,

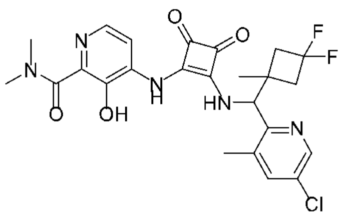,
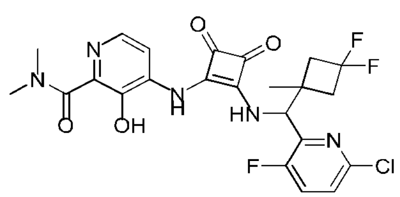,
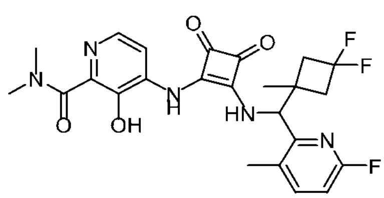,

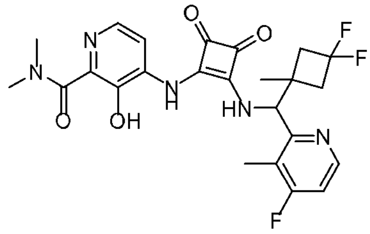,
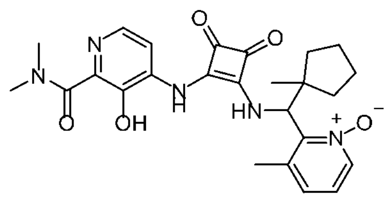,
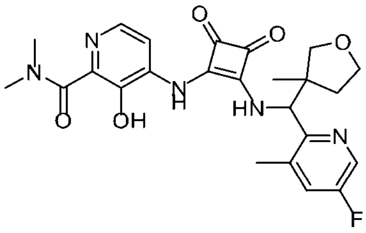,

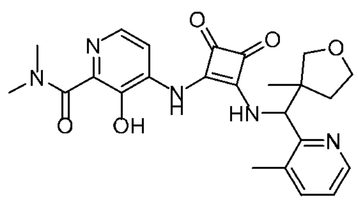,
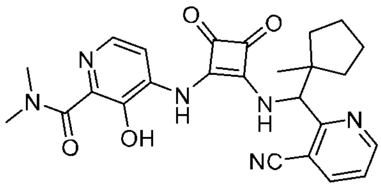,
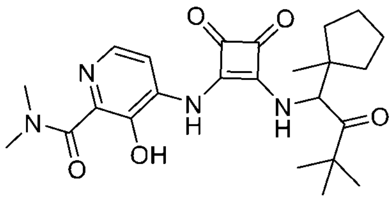,

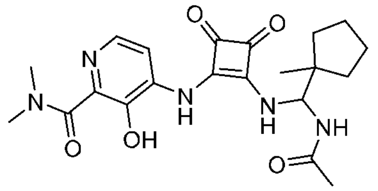,
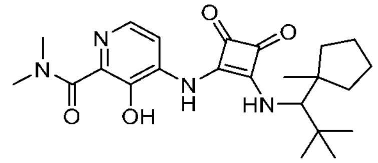,
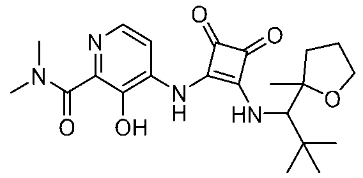,

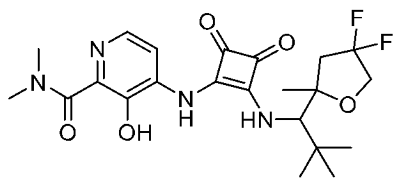,
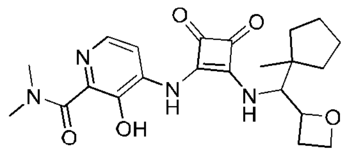,
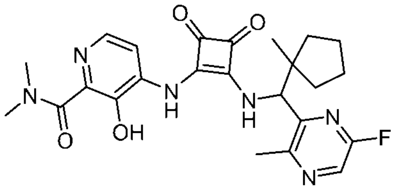,

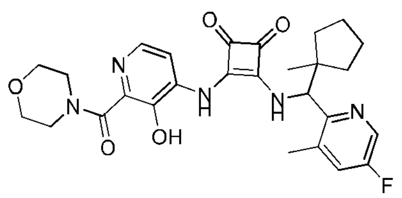,
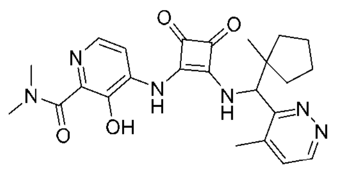,
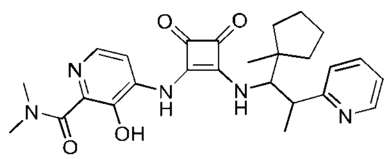,

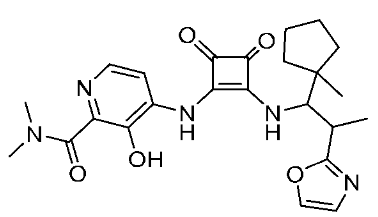,
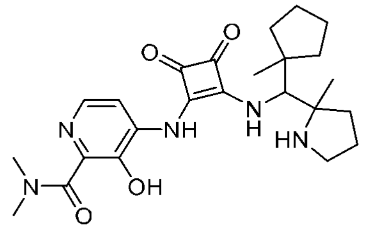,
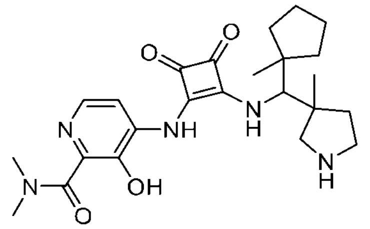,

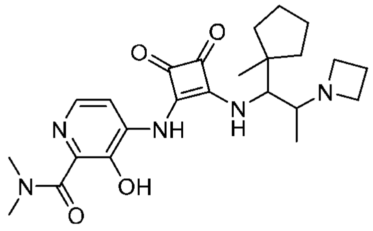,
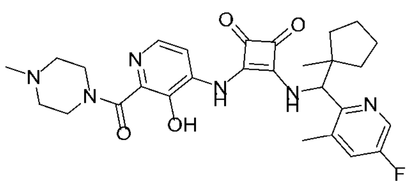,
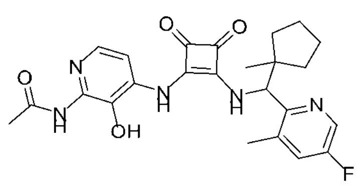,

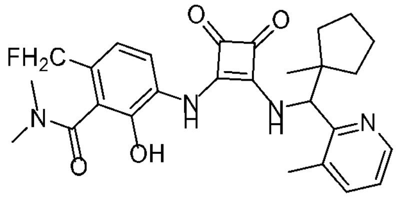, 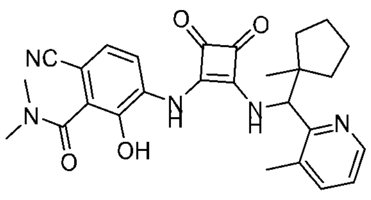, 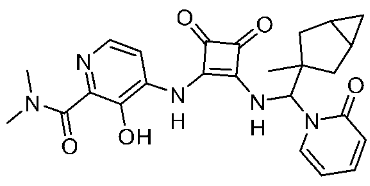,

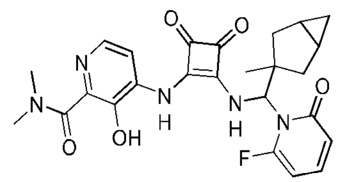, 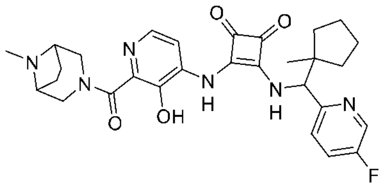, 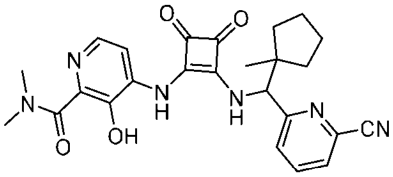,

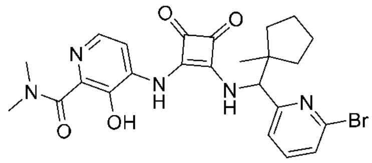, 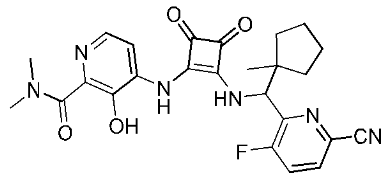, 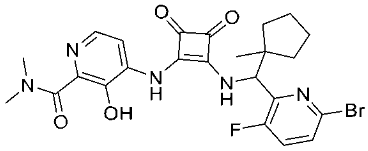,

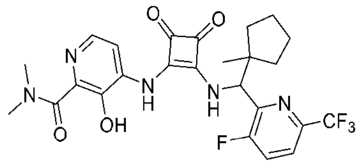, 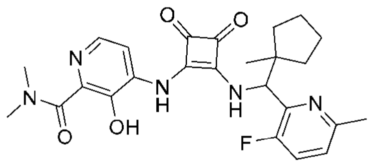, 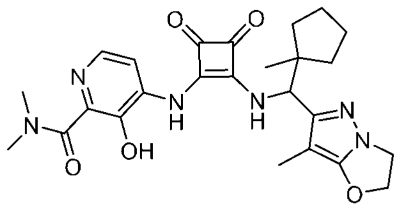,

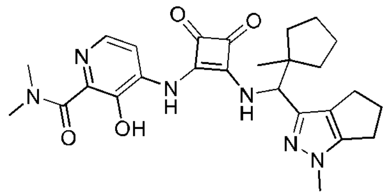, or 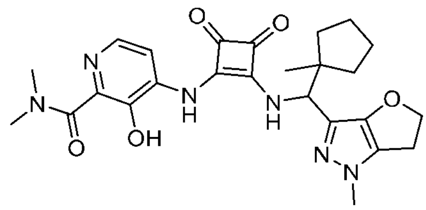.

According to an embodiment of the present disclosure, the compound represented by formula (I) has the structure shown below:

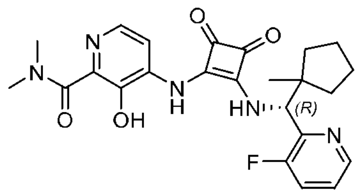, 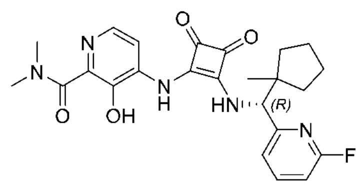, 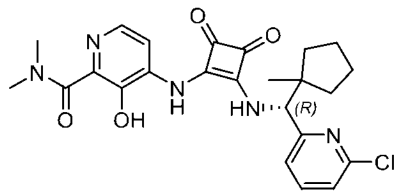,

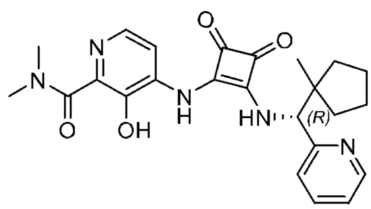, 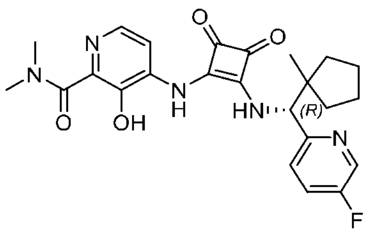, 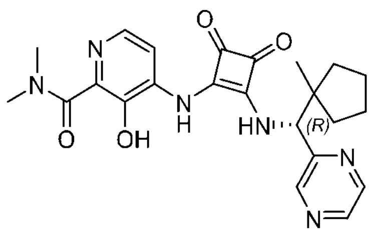,

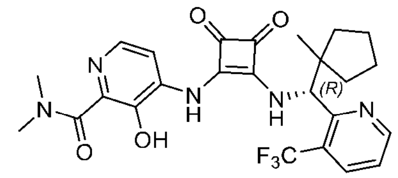, 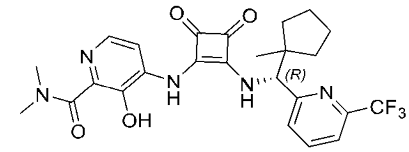, 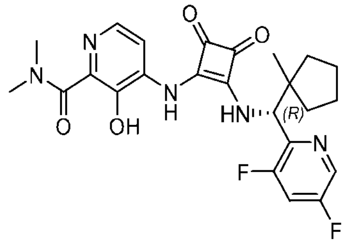,

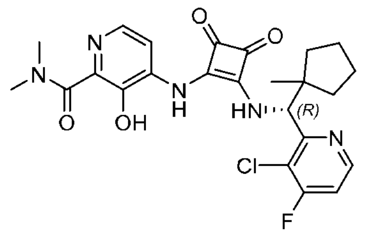, 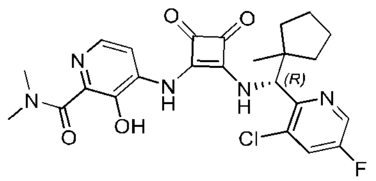, 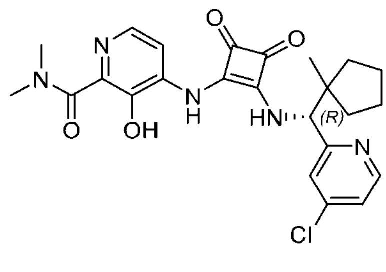,

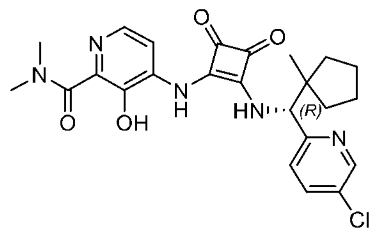, 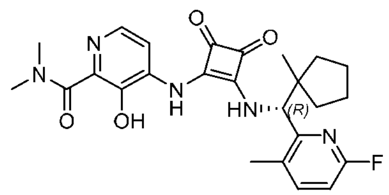, 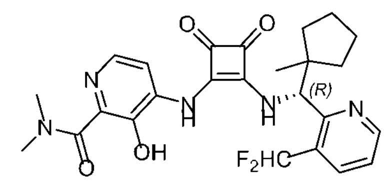,

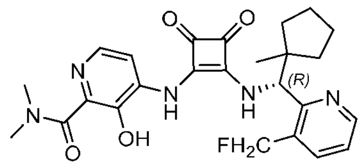, 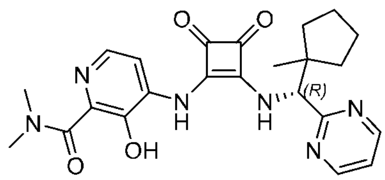, 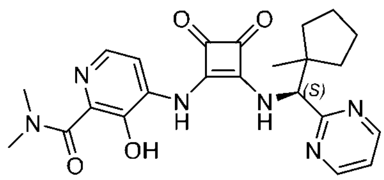,

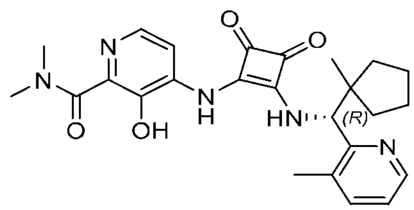, 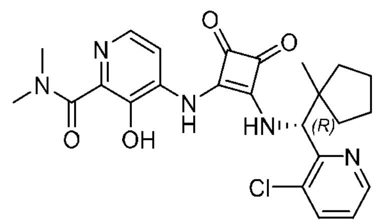, 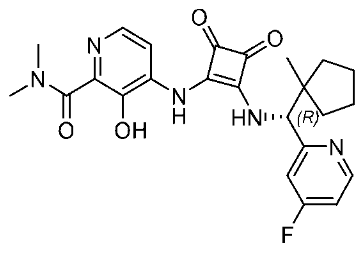,

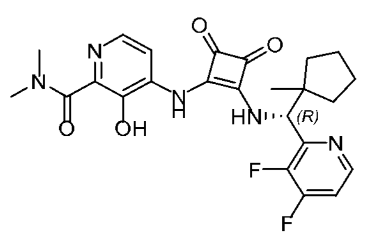, 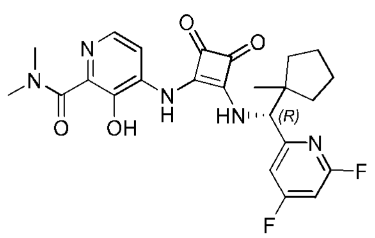, 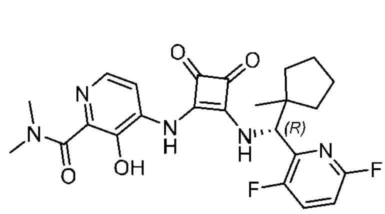,

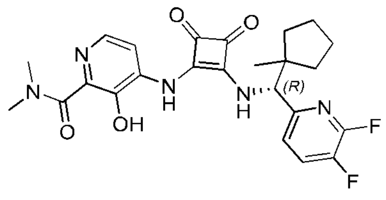 , 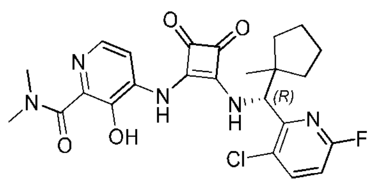 , 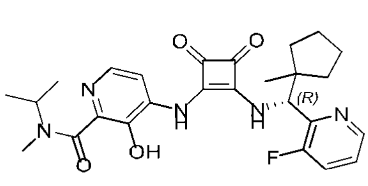 ,

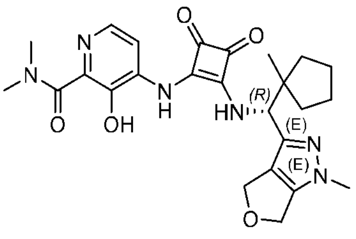 , 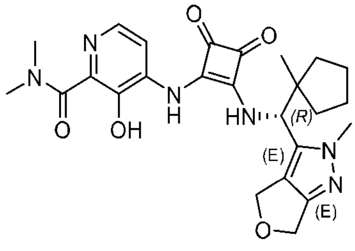 , 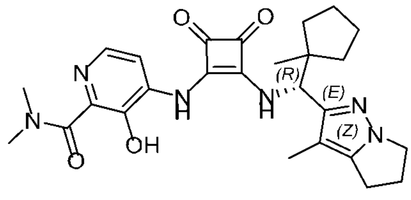 ,

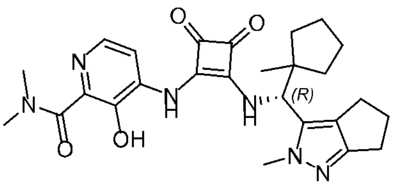 , 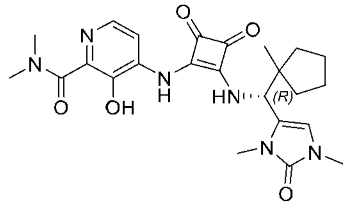 , 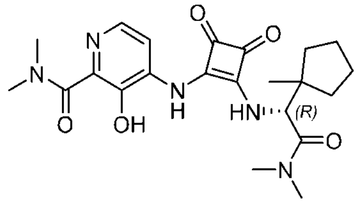 ,

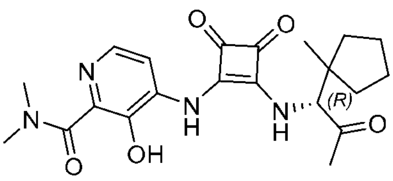 , 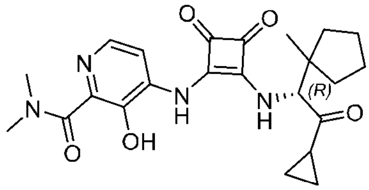 , 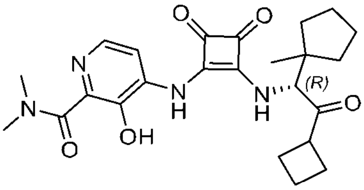 ,

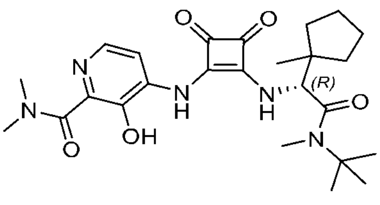 , 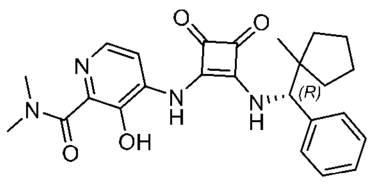 , 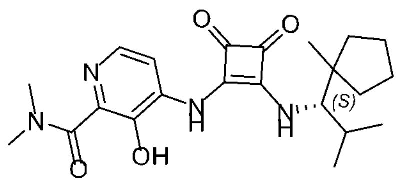 ,

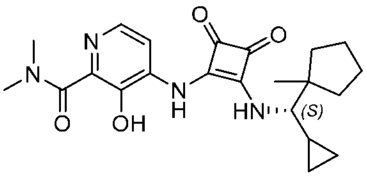 , 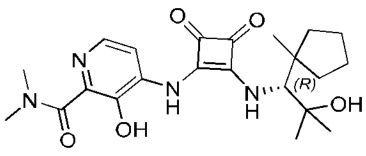 , 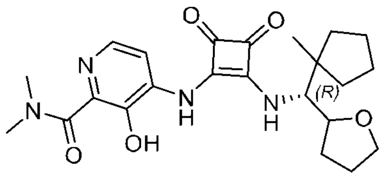 ,

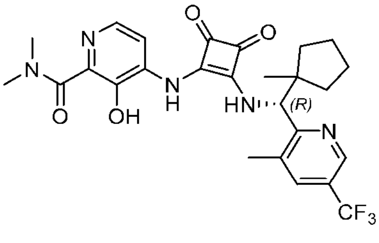 , 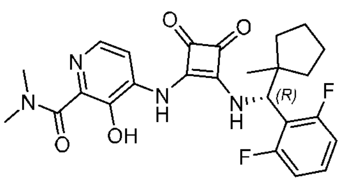 , 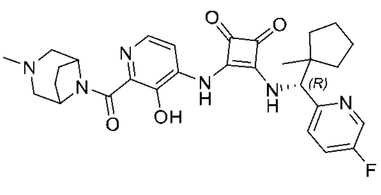 ,

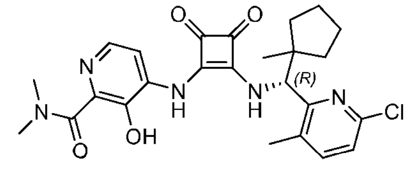, 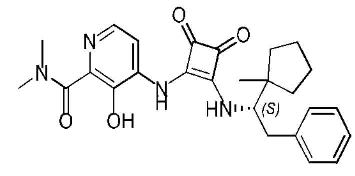, 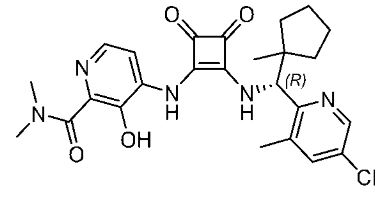,

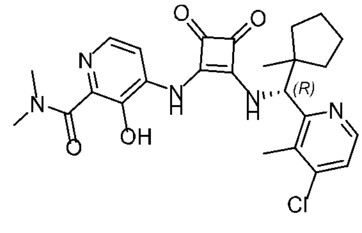, 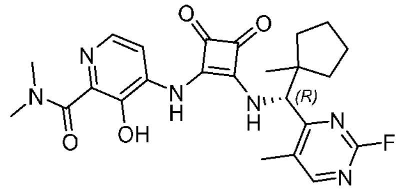, 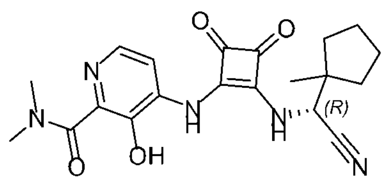,

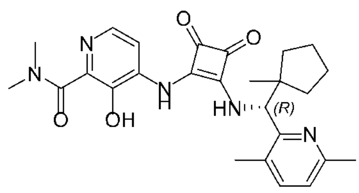, 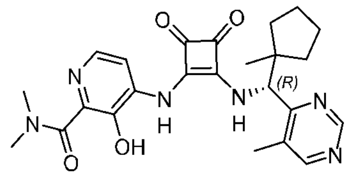, 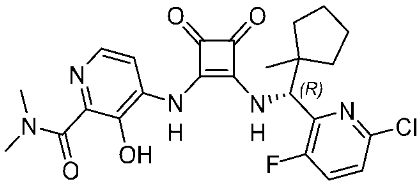,

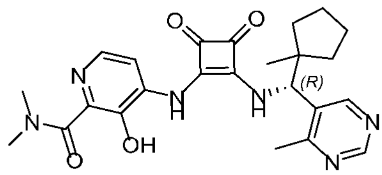, 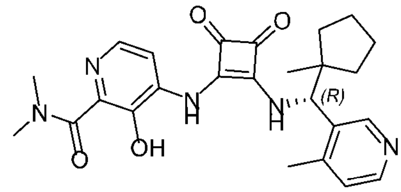, 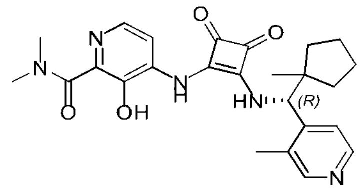,

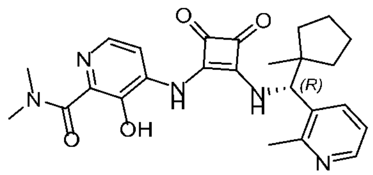, 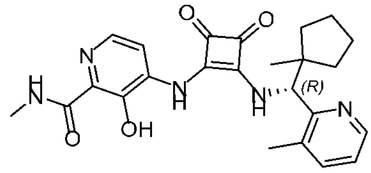, 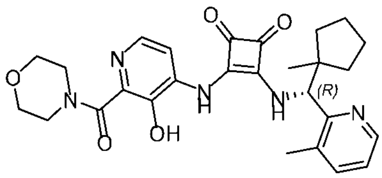,

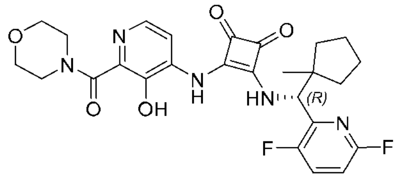, 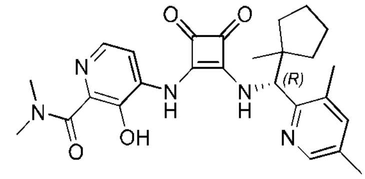, 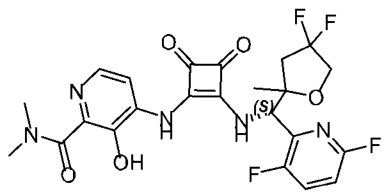,

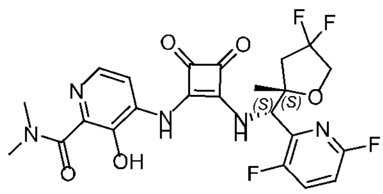, 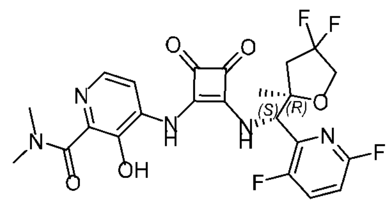, 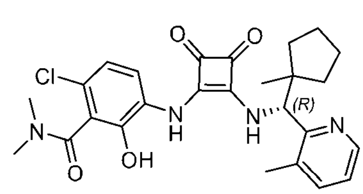,

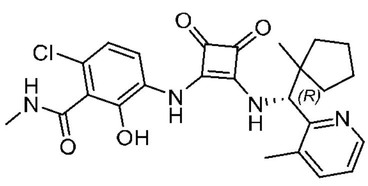,
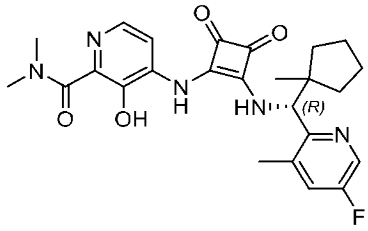,
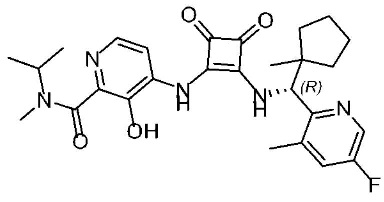,
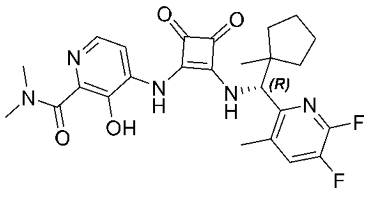,
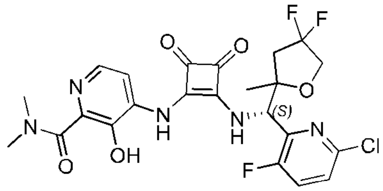,
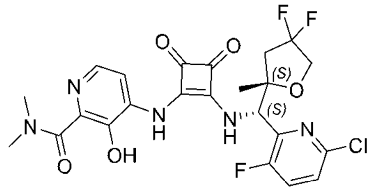,
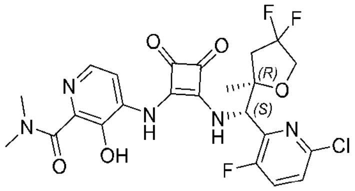,
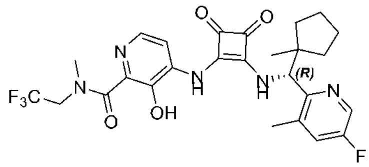,
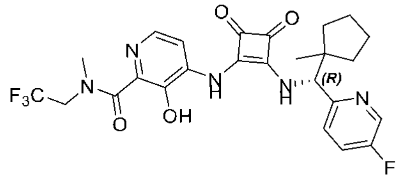,
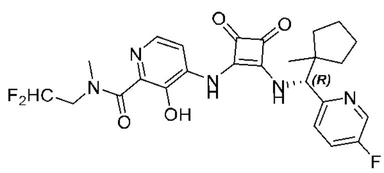,
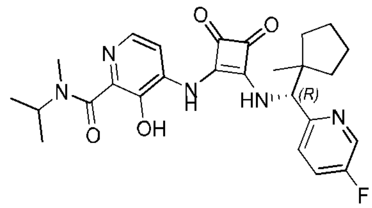,
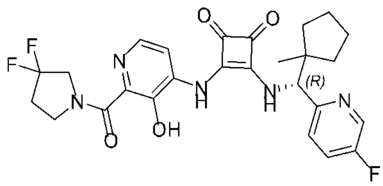,
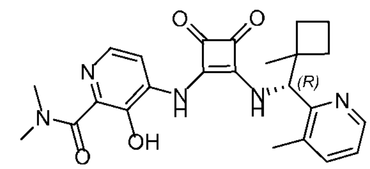,
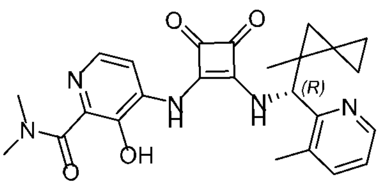,
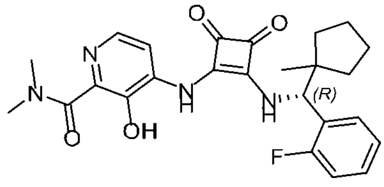,
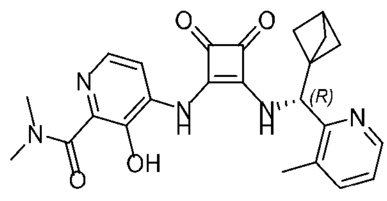,
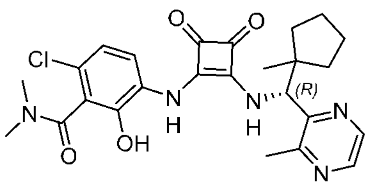,
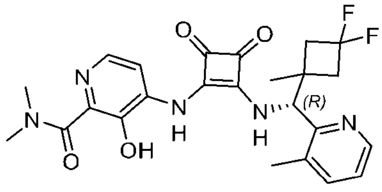,
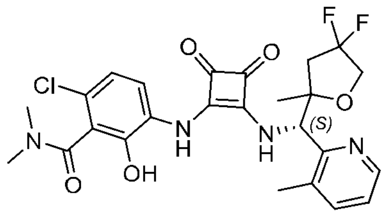,
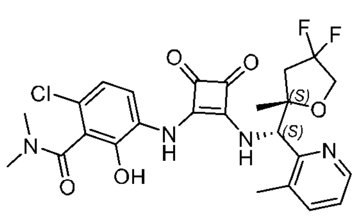,
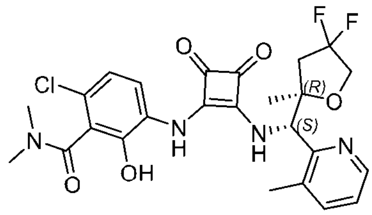,

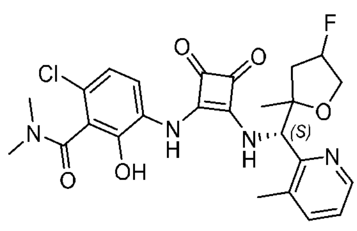, 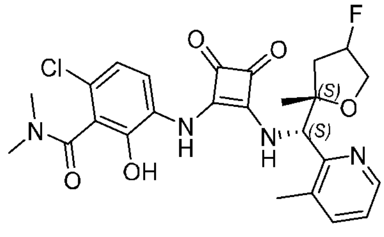, 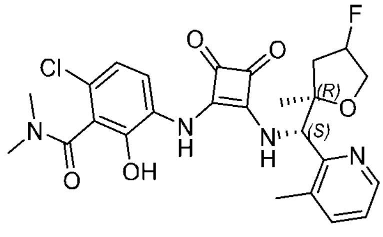,

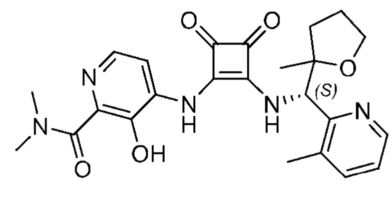, 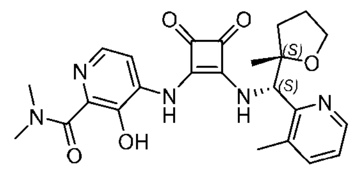, 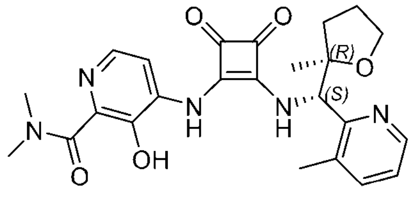,

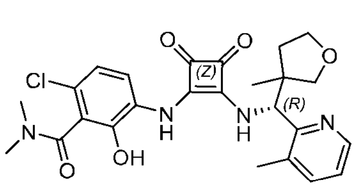, 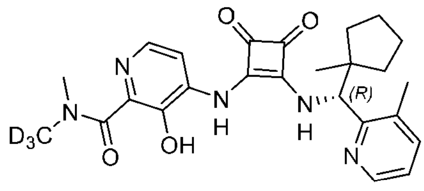, 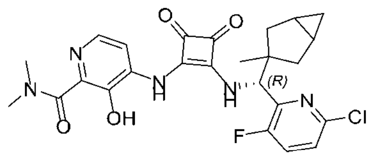,

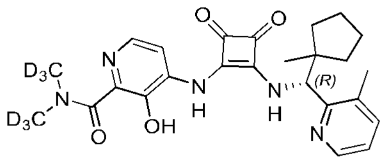, 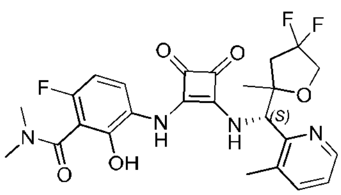, 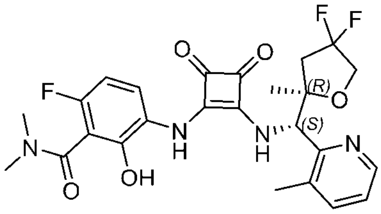,

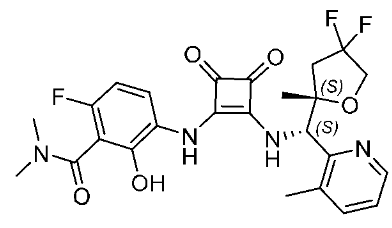, 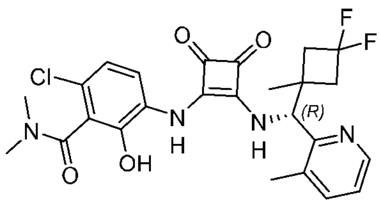, 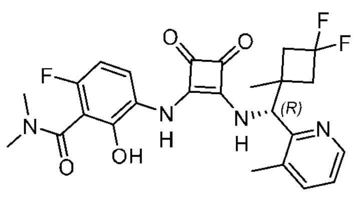,

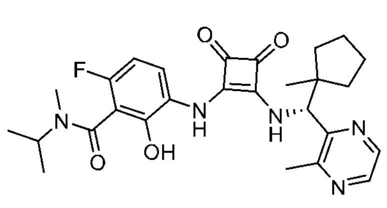, 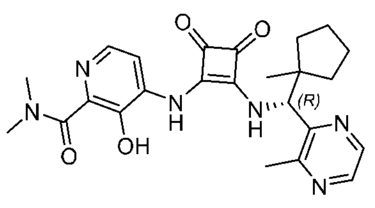, 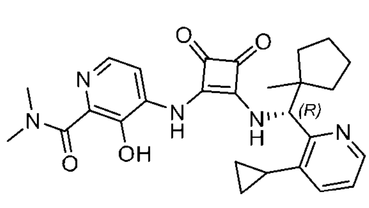,

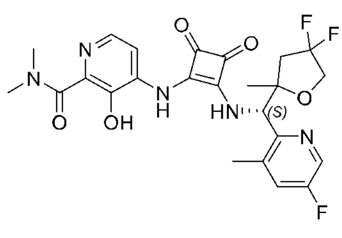, 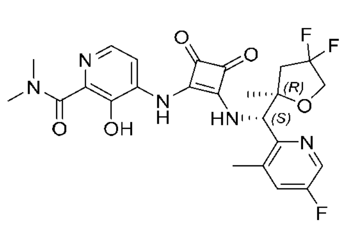, 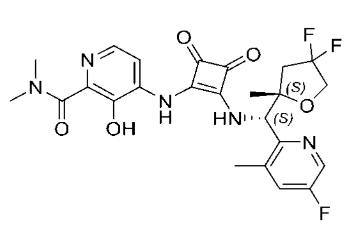,

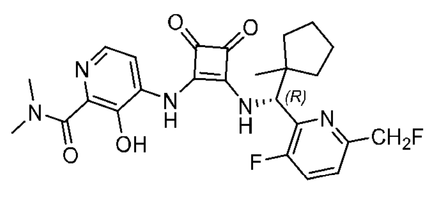 , 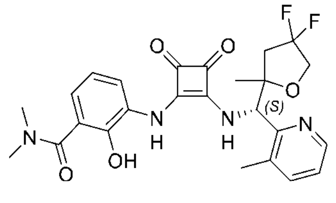 , 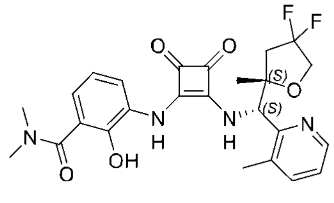 ,

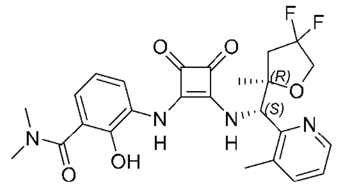 , 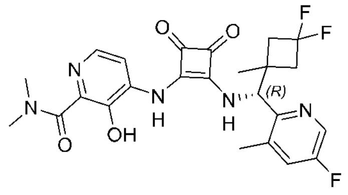 , 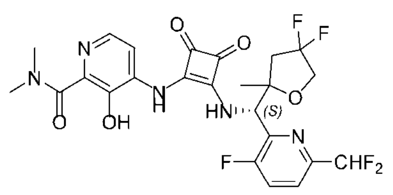 ,

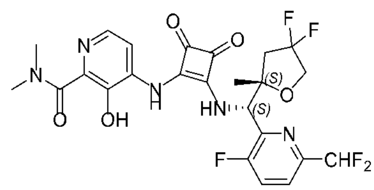 , 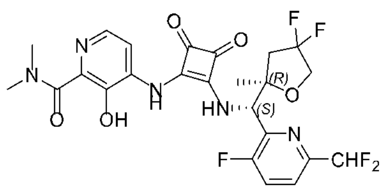 , 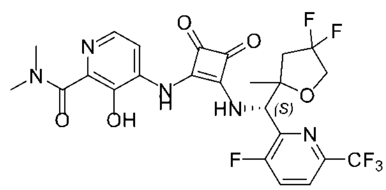 ,

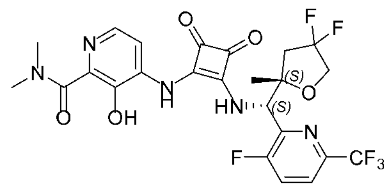 , 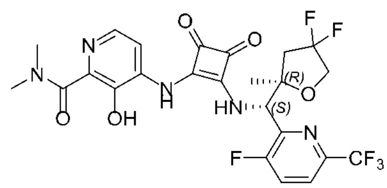 , 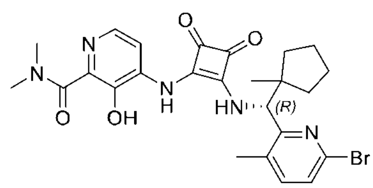 ,

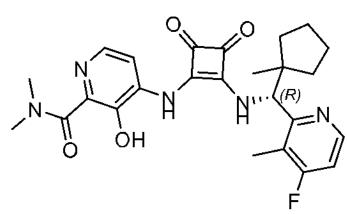 , 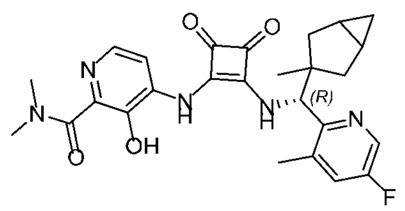 , 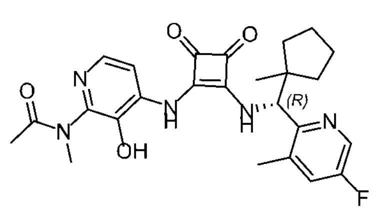 ,

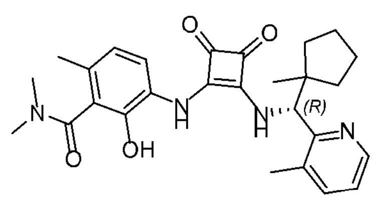 , 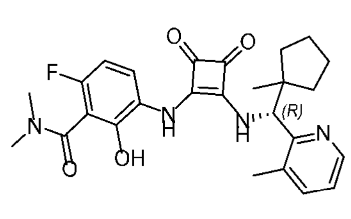 , 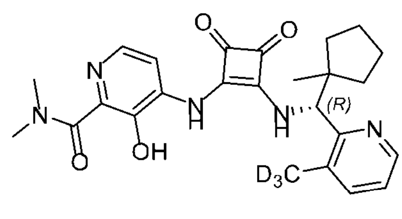 ,

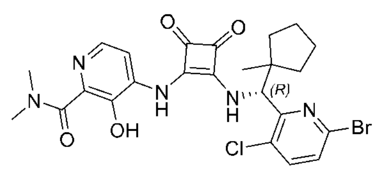 , 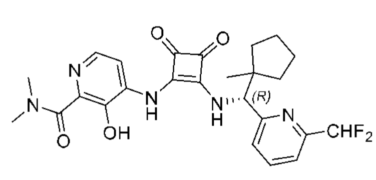 , 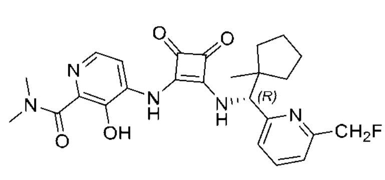 ,

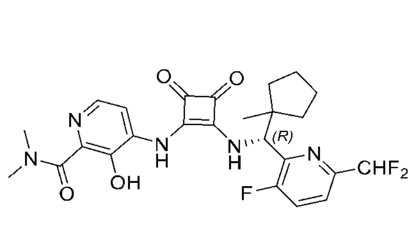,
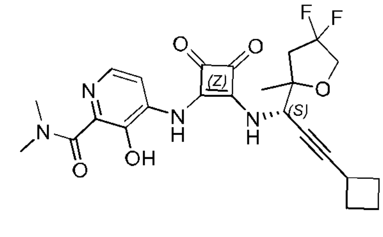,
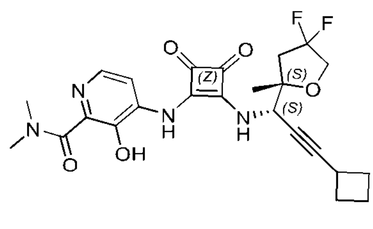,
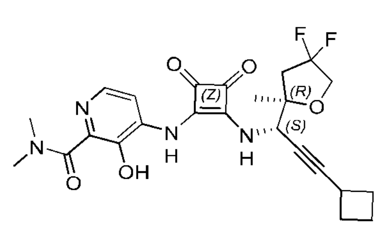,
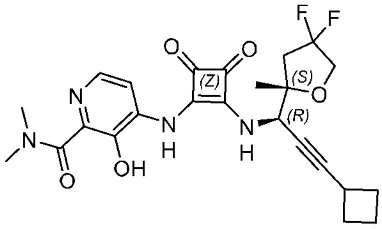,
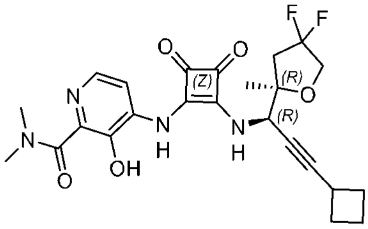,
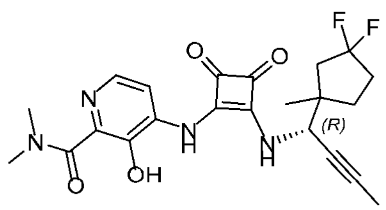,
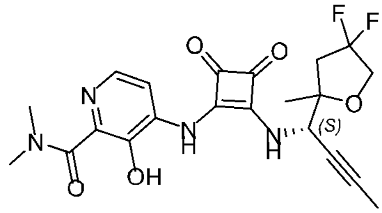,
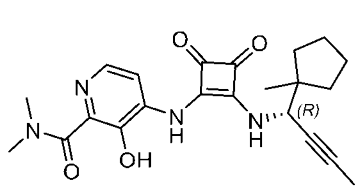,
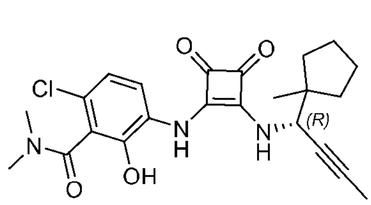,
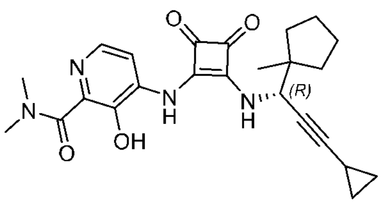,
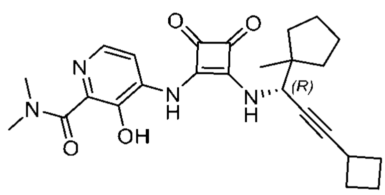,
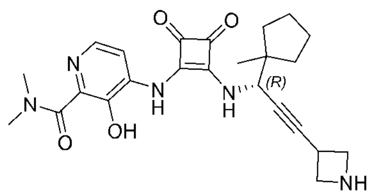,
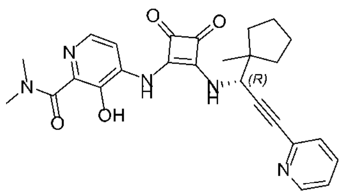,
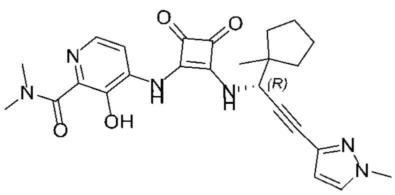,
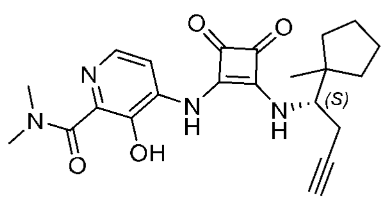,
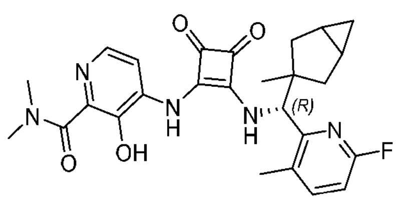,
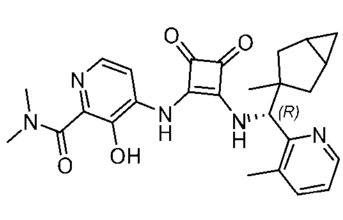,
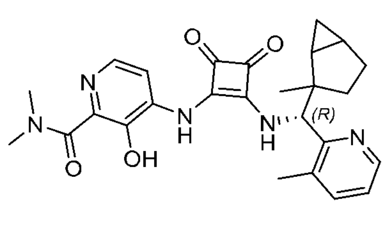,
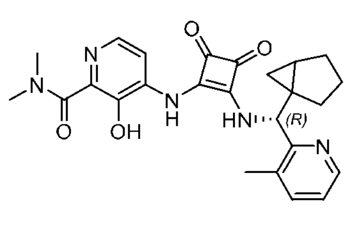,
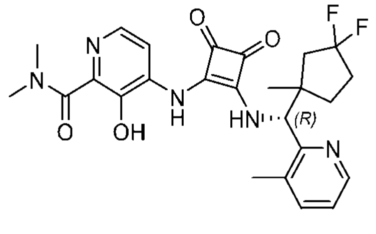,

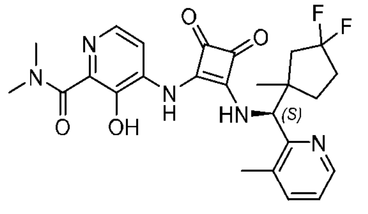,
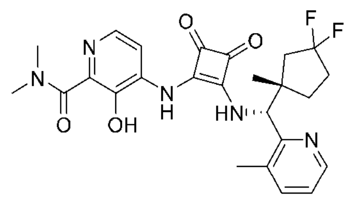,
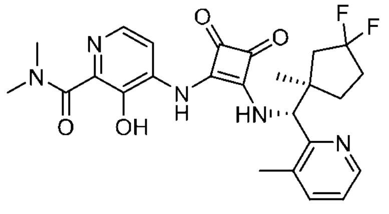,

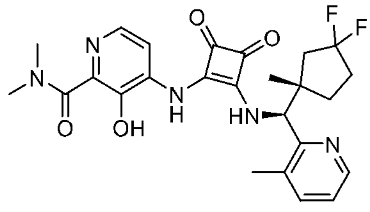,
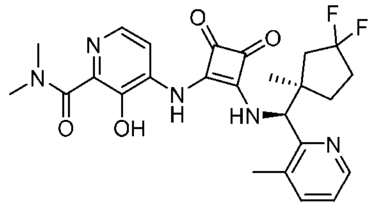,
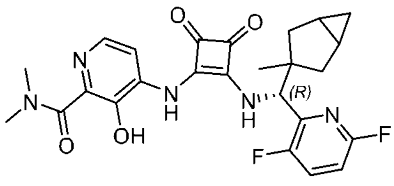,

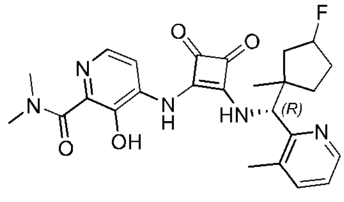,
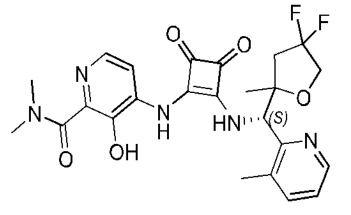,
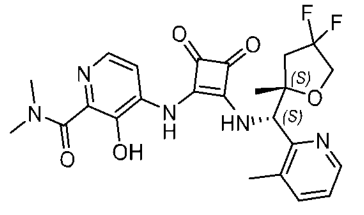,

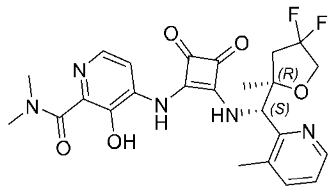,
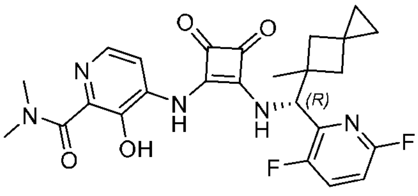,
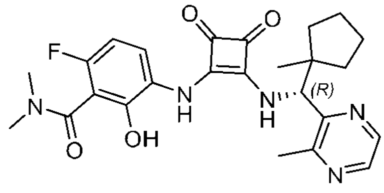,

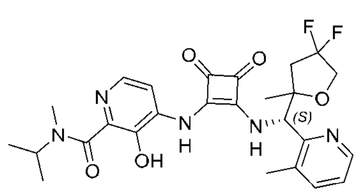,
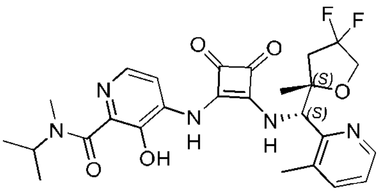,
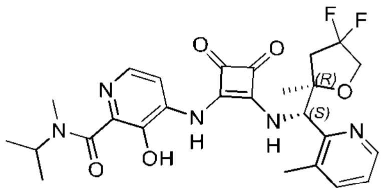,

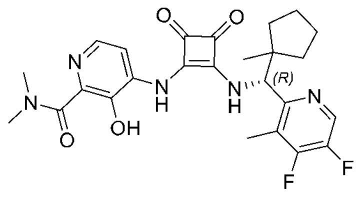,
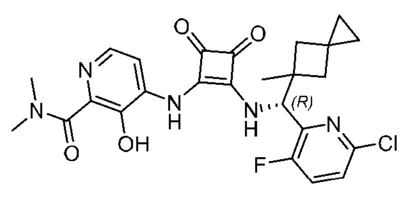,
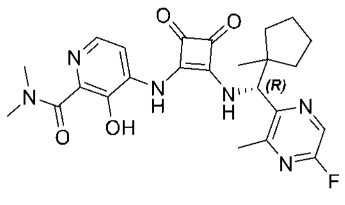,

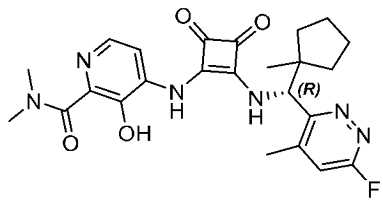,
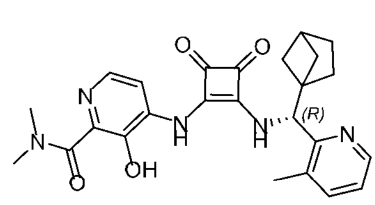,
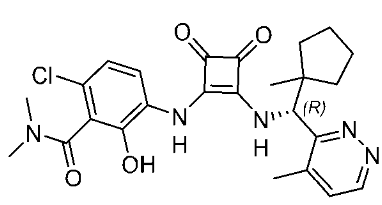,

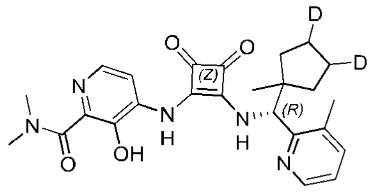, 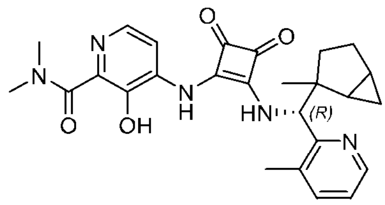, 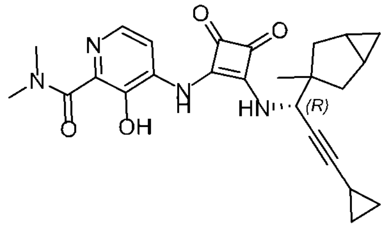,

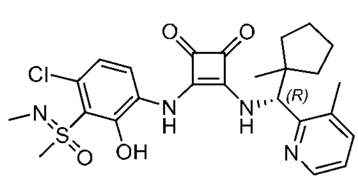, 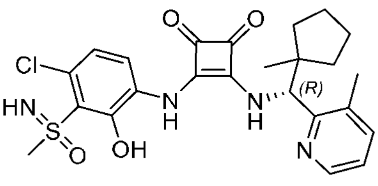, 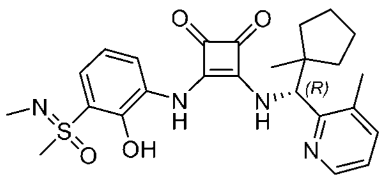,

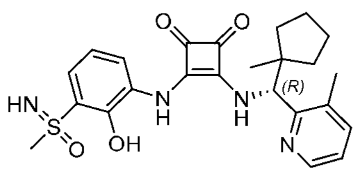, 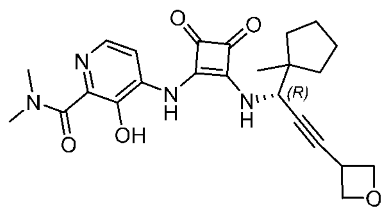, 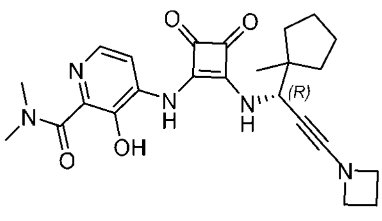,

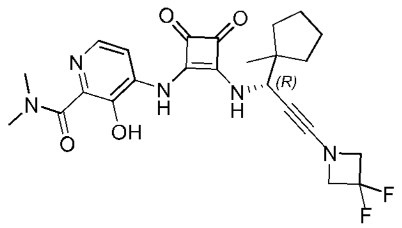, 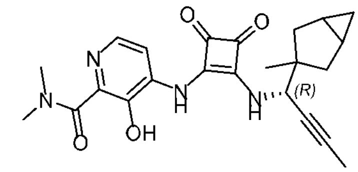, 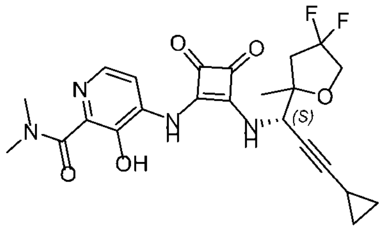,

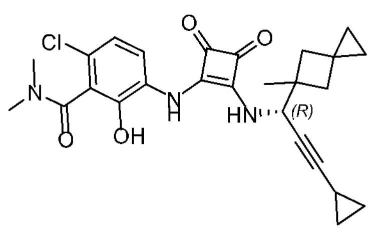, 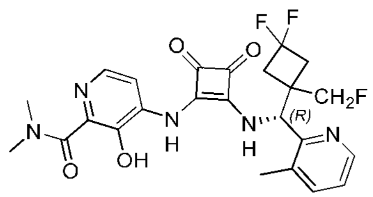, 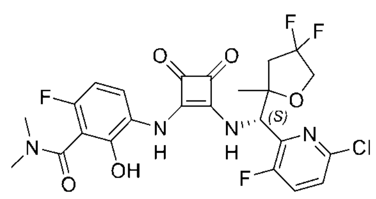,

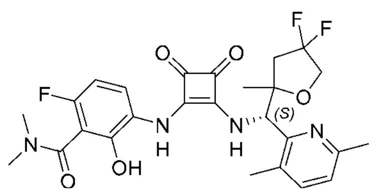, 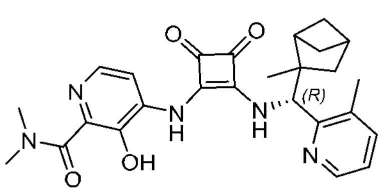, 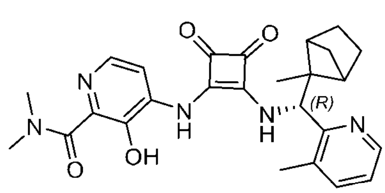,

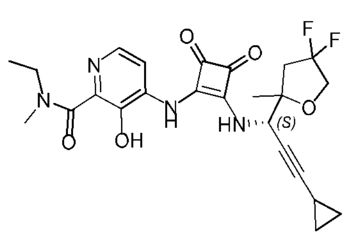, 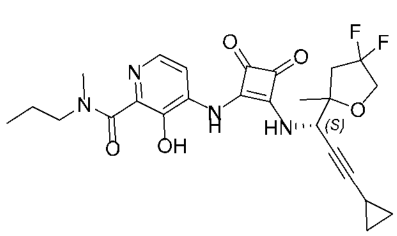, 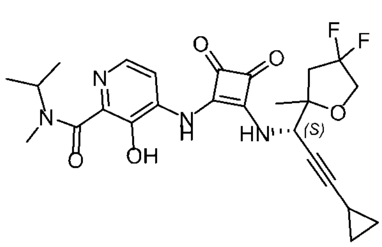,

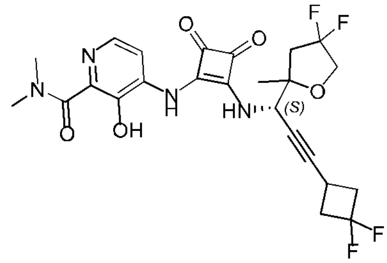, 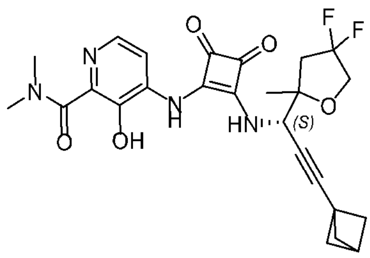, 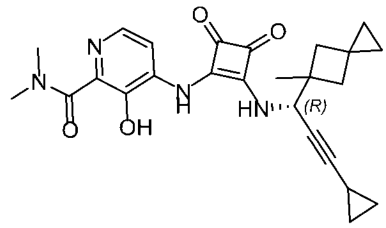,

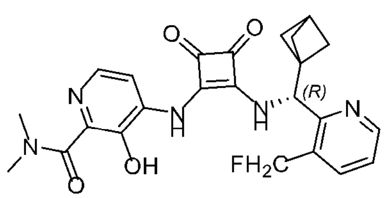, 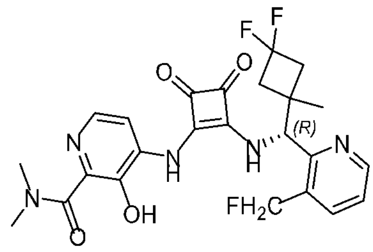, 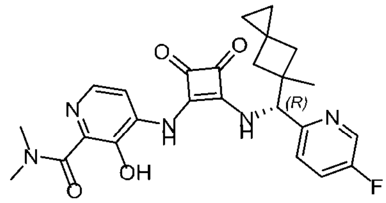,

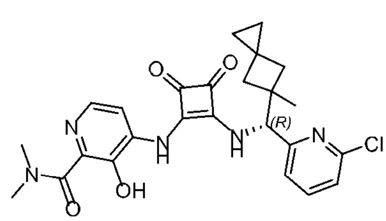, 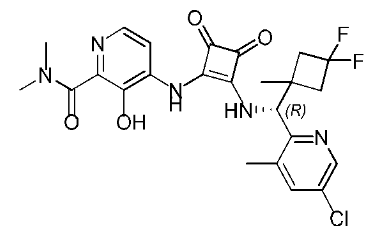, 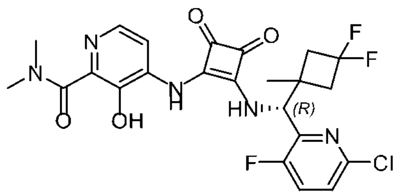,

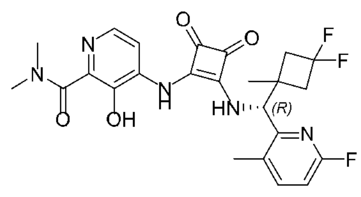, 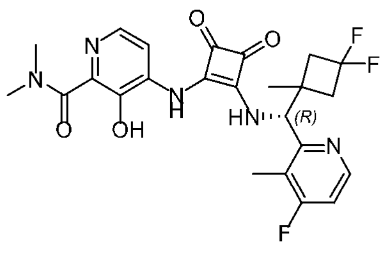, 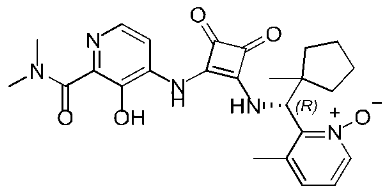,

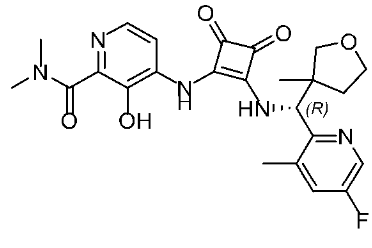, 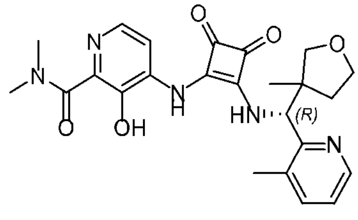, 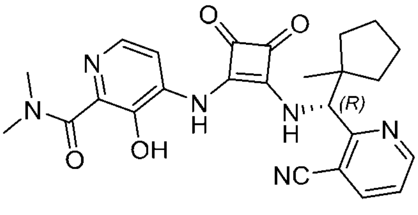,

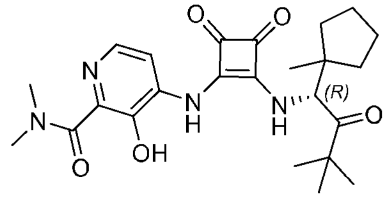, 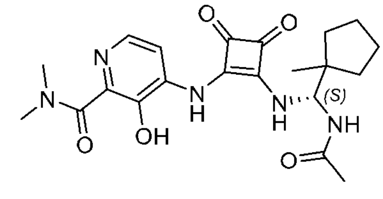, 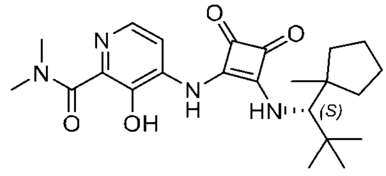,

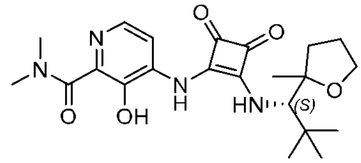,
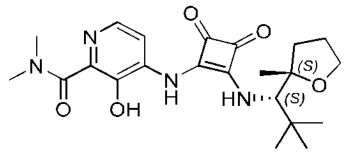,
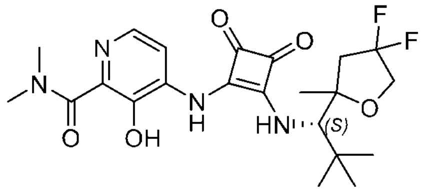,
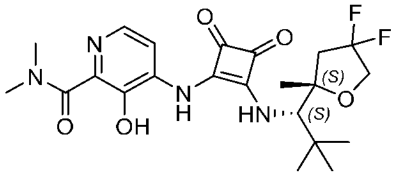,
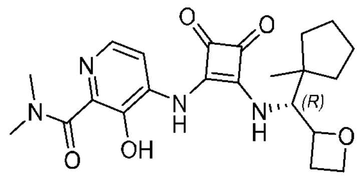,
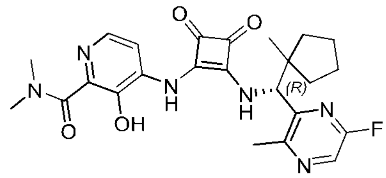,
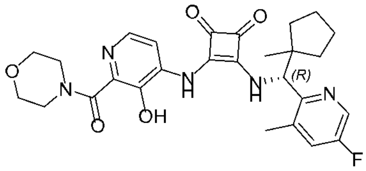,
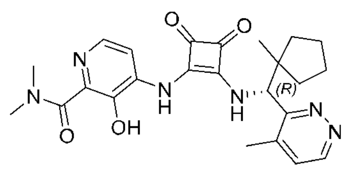,
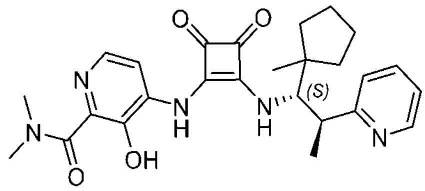,
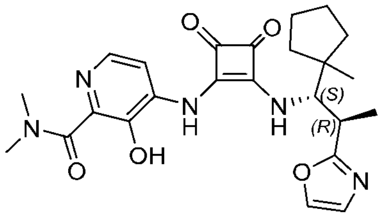,
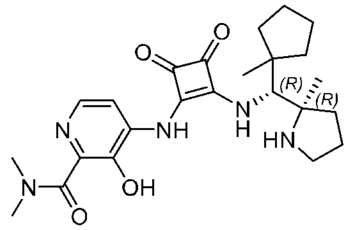,
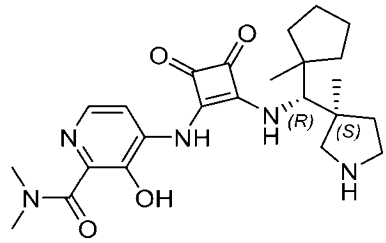,
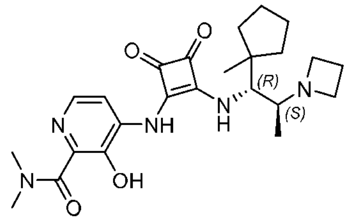,
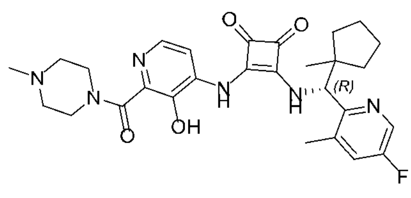,
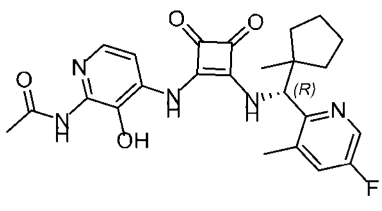,
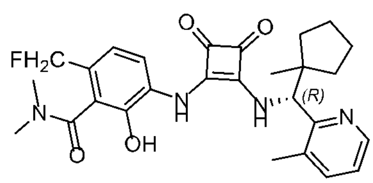,
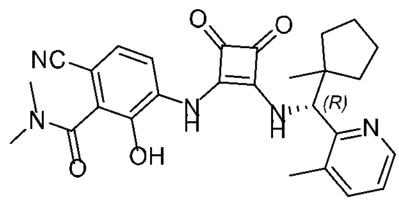,
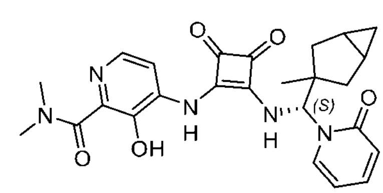,
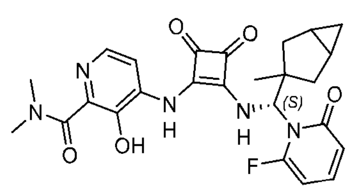,
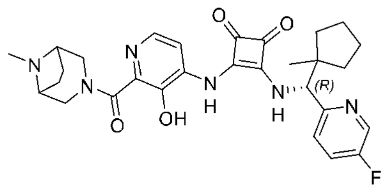,
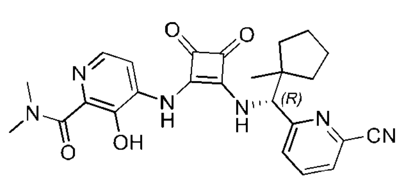,

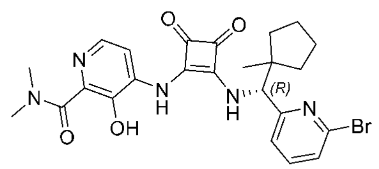, 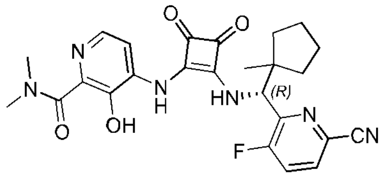, 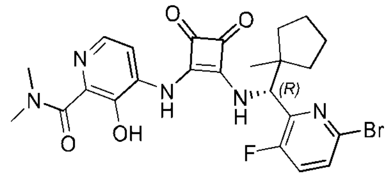,

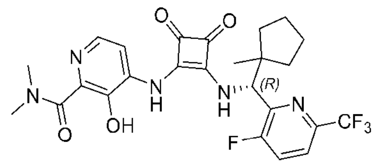, 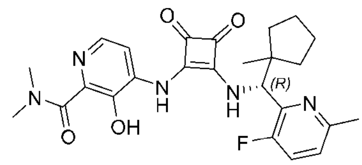, 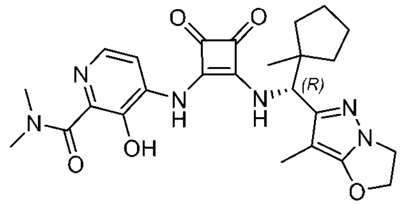,

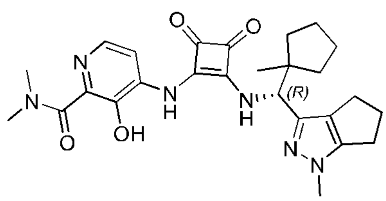, or 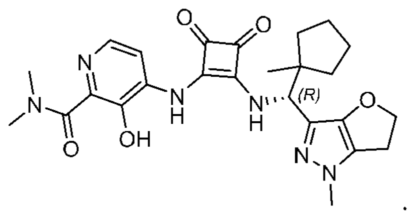.

The present disclosure also provides a method for preparing the compound represented by formula (I), comprising the following step:

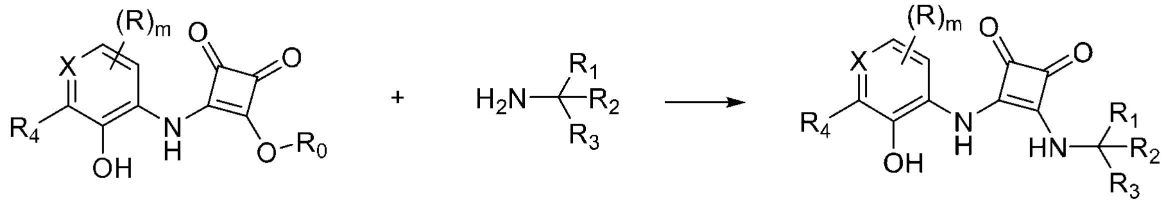

1 2 (I) ,

reacting compound 1 with compound 2 to obtain the compound represented by formula (I);
wherein $R_1$, $R_2$, $R_3$, $R_4$, R, X, and m each independently have the definition as described in any one embodiment of the present disclosure, and $R_0$ is $C_{1-6}$ alkyl.

The present disclosure also provides a compound represented by formula 1 or formula 2:

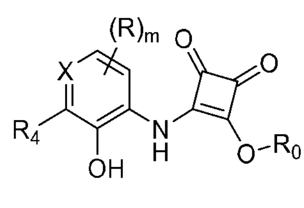 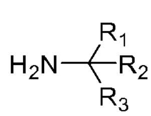

1 or 2 ,

wherein $R_0$, $R_1$, $R_2$, $R_3$, $R_4$, R, X, and m each independently have the definition as described in any one embodiment of the present disclosure.

According to an embodiment of the present disclosure, the compound represented by formula 2 is selected from the group consisting of the following compounds:

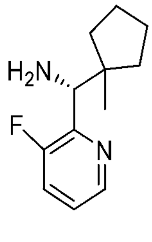
001-10 ,

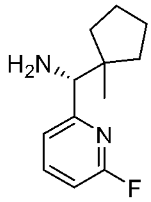
002-2 ,

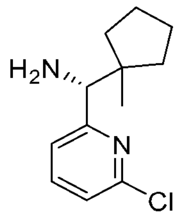
003-2 ,

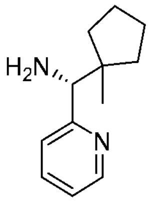
004-2 ,

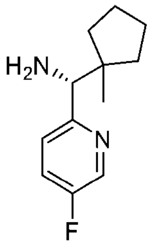
005-2 ,

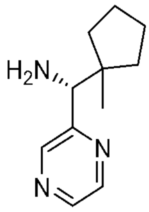
006-2 ,

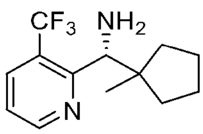
007-2 ,

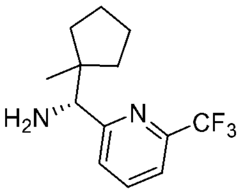
008-2 ,

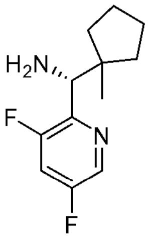
009-4 ,

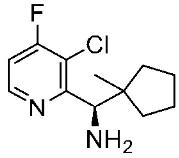
010-3 ,

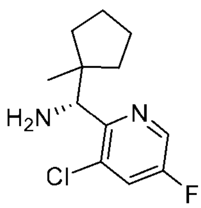
011-2 ,

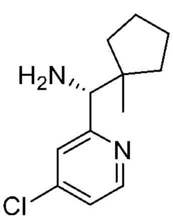
012-2 ,

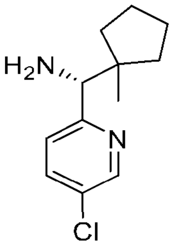
013-2 ,

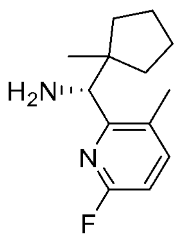
014-3 ,

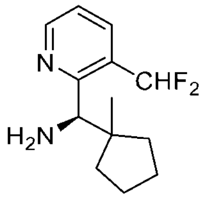
015-3 ,

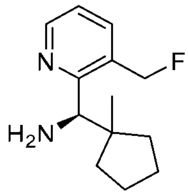
016-6 ,

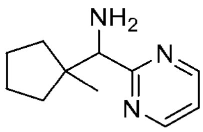
017-2 ,

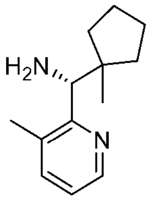
018-2 ,

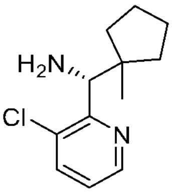
019-2 ,

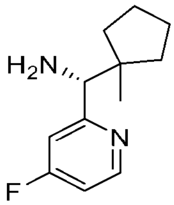
020-2 ,

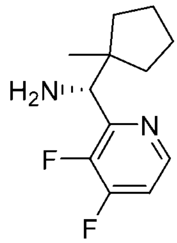
021-8 ,

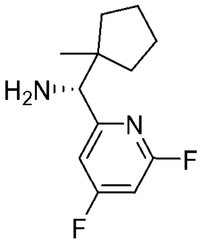
022-6 ,

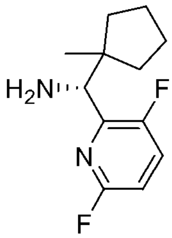
023-4 ,

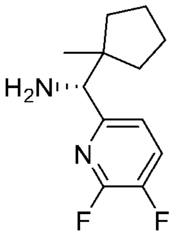
024-4 ,

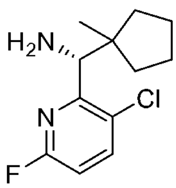
025-3 ,

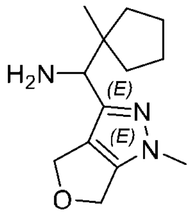
027-7 ,

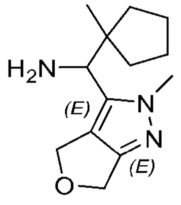
028-4 ,
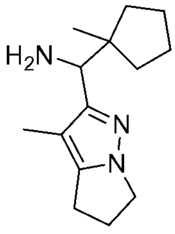
029-9 ,
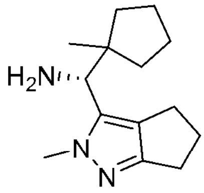
030-4 ,
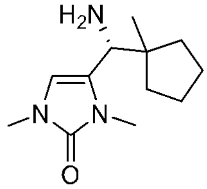
031-8 ,
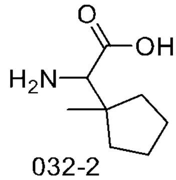
032-2 ,

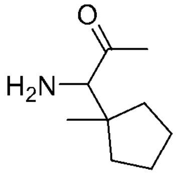
033-3 ,
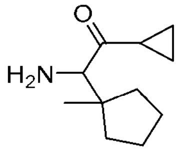
034-4 ,
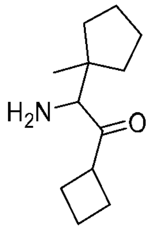
035-2 ,
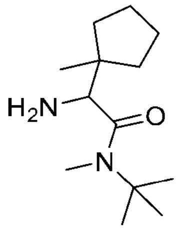
036-2 ,
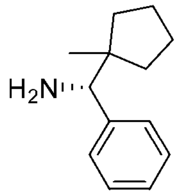
037-2 ,
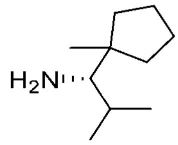
038-2 ,

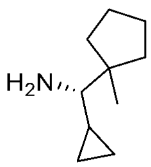
039-2 ,
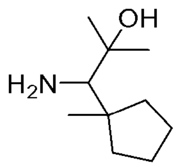
040-3 ,
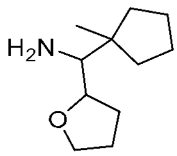
041-5 ,
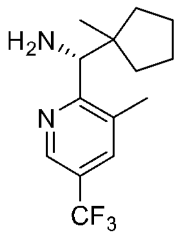
042-5 ,
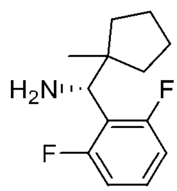
043-2 ,

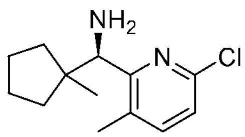
045-3 ,
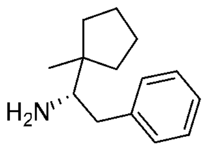
046-2 ,
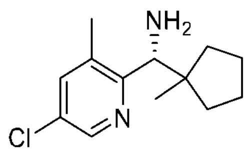
047-2 ,
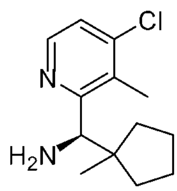
048-2 ,

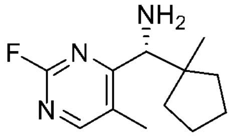
049-4 ,
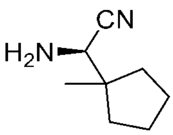
050-2 ,
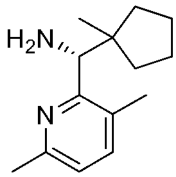
051-3 ,
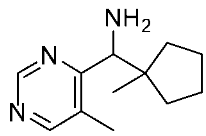
052-6 ,
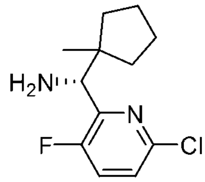
053-5 ,

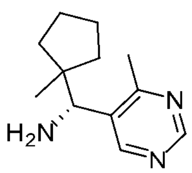
054-2 ,
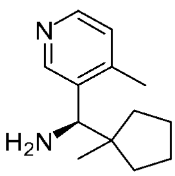
055-2 ,
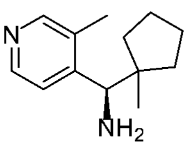
056-3 ,
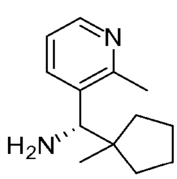
057-2 ,
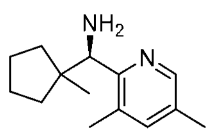
061-2 ,

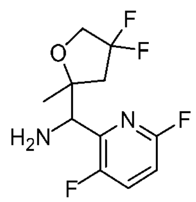
062-8 ,
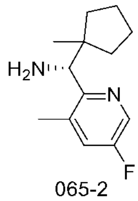
065-2 ,
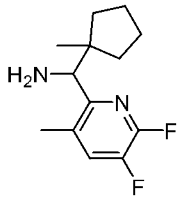
067-8 ,
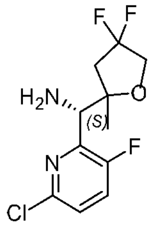
068-8 ,
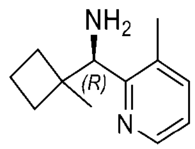
074-5 ,
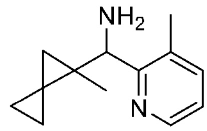
075-11 ,

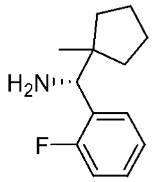
076-2 ,
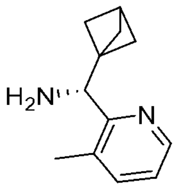
077-5 ,
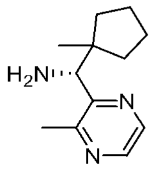
078-2 ,
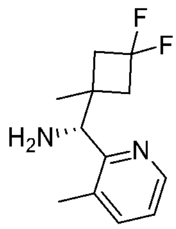
079-8 ,
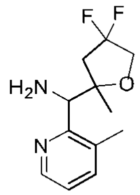
080-2 ,
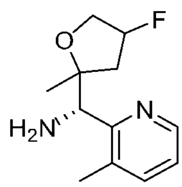
081-6 ,

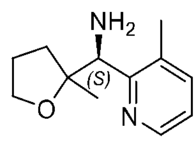
082-5 ,
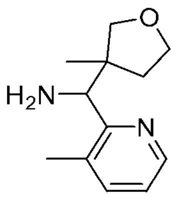
083-5 ,
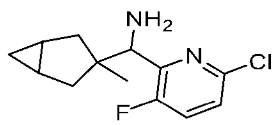
085-6 ,
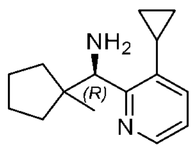
092-4 ,
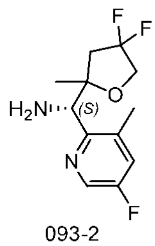
093-2 ,

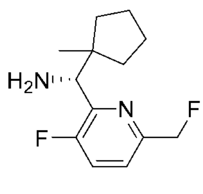
094-4 ,
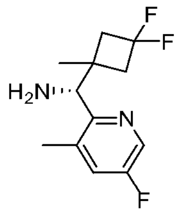
096-2 ,
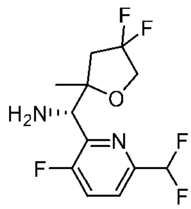
097-7 ,
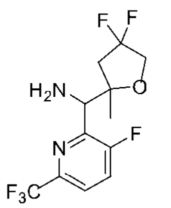
098-2 ,
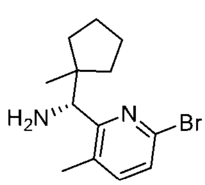
099-3 ,
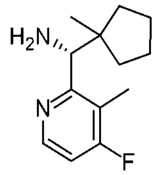
100-3 ,

or

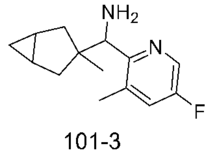
101-3 .

The present disclosure further provides a pharmaceutical composition comprising a therapeutically effective amount of the compound represented by formula (I), the racemate thereof, the stereoisomer thereof, the tautomer thereof, the N-oxide thereof, or the pharmaceutically acceptable salt of any of the foregoing as described herein.

In some embodiments, the pharmaceutical composition of the present disclosure further comprises a therapeutically effective amount of the compound represented by formula (I), the racemate thereof, the stereoisomer thereof, the tautomer thereof, the N-oxide thereof, or the pharmaceutically acceptable salt of any of the foregoing as described herein, and a pharmaceutically acceptable carrier.

The carrier in the pharmaceutical composition is "pharmaceutically acceptable", meaning it is compatible with the active ingredient of the composition (and preferably, capable of stabilizing the active ingredient) and is not harmful to the subject being treated. One or more pharmaceutical excipients may be used for delivering the active compound.

The present disclosure further provides a use of the compound represented by formula (I), the racemate thereof, the stereoisomer thereof, the tautomer thereof, the N-oxide thereof, or the pharmaceutically acceptable salt of any of the foregoing, or the pharmaceutical composition in the manufacture of a medicament.
According to some embodiments, the medicament is for the diagnosis, prevention, and/or treatment of a CCR6 receptor-mediated disease or disorder.
According to some embodiments, the medicament is a CCR6 antagonist.
According to some embodiments, the disease or disorder is an allergic disease, psoriasis, contact hypersensitivity, Sjögren's syndrome, dry eye disease, or multiple sclerosis.
According to some embodiments, the disease or disorder is rheumatoid arthritis, juvenile arthritis, juvenile rheumatoid arthritis, systemic-onset rheumatoid arthritis, pauciarticular rheumatoid arthritis, pauciarticular juvenile rheumatoid arthritis, polyarticular rheumatoid arthritis, enteropathic arthritis, juvenile Reiter's syndrome, ankylosing spondylitis, juvenile ankylosing spondylitis, SEA syndrome, reactive arthritis (reactive arthropathy), psoriatic arthropathy, juvenile enteropathic arthritis, polymyalgia rheumatica, enteropathic spondylitis, juvenile idiopathic arthritis (JIA), juvenile psoriatic arthritis, juvenile rheumatoid arthritis, systemic-onset juvenile rheumatoid arthritis, giant cell arteritis, or secondary osteoarthritis caused by an inflammatory disease.
According to some embodiments, the disease or disorder is inflammatory bowel disease (IBD), Crohn's disease, or ulcerative colitis.
The present disclosure also provides a method for diagnosing, preventing, and/or treating a CCR6 receptor-mediated disease or disorder, comprising administering to a patient in need thereof a therapeutically effective amount of at least one compound of the present disclosure alone, or optionally in combination with another compound of the present disclosure and/or at least one other type of therapeutic agent.
According to the present disclosure, the disease or disorder is an allergic disease, psoriasis, contact hypersensitivity, Sjögren's syndrome, dry eye disease, or multiple sclerosis.
According to the present disclosure, the disease or disorder is rheumatoid arthritis, juvenile arthritis, juvenile rheumatoid arthritis, systemic-onset rheumatoid arthritis, pauciarticular rheumatoid arthritis, pauciarticular juvenile rheumatoid arthritis, polyarticular rheumatoid arthritis, enteropathic arthritis, juvenile Reiter's syndrome, ankylosing spondylitis, juvenile ankylosing spondylitis, SEA syndrome, reactive arthritis (reactive arthropathy), psoriatic arthropathy, juvenile enteropathic arthritis, polymyalgia rheumatica, enteropathic spondylitis, juvenile idiopathic arthritis (JIA), juvenile psoriatic arthritis, juvenile rheumatoid arthritis, systemic-onset juvenile rheumatoid arthritis, giant cell arteritis, or secondary osteoarthritis caused by an inflammatory disease.
According to the present disclosure, the disease or disorder is inflammatory bowel disease (IBD), Crohn's disease, or ulcerative colitis.
The compounds of the present disclosure may be used in combination with an additional therapeutic agent.

## Beneficial effects

The present disclosure provides a class of CCR6 antagonist compounds represented by formula (I), which exhibit good CCR6 antagonistic activity as well as excellent pharmacokinetic and pharmacodynamic properties.

## Definition and explanation of terms

Unless otherwise specified, the definitions of groups and terms described in the specification and claims include definitions thereof as examples, exemplary definitions, preferred definitions, definitions recorded in tables, and definitions of specific compounds in the examples, *etc.,* which can be arbitrarily combined and integrated with each other. Such combinations and combined group definitions and compound structures should be understood to be within the scope of the specification and/or claims of the present disclosure.
The term "optional" (or "optionally", "option") in the general formula definitions of the present disclosure means the case of being substituted by 0, 1, or more substituents, for example, "optionally substituted by 1, 2, or more R" means that it may not be substituted by R (unsubstituted) or may be optionally substituted by 1, 2, or more R.
The terms "$C_{n-m}$" and "$C_n$-$C_m$", where n and m are integers, indicate a group containing n to m carbon atoms. Examples include $C_{1-6}$, $C_{1-3}$, and the like. This term is intended to explicitly disclose each member within this range, namely $C_n$, $C_{n+1}$, $C_{n+2}$, ..., $C_{m-2}$, $C_{m-1}$, $C_m$. For example, $C_{1-6}$ is intended to disclose $C_1$, $C_2$, $C_3$, $C_4$, $C_5$, and $C_6$. "$C_{n-m}$" means the same as "$C_n$-$C_m$".
The term "n-membered," where n is an integer, generally describes a ring wherein the number of ring-forming atoms is n.
The term "n- to m-membered", where n and m are integers, describes a range where the number of ring-forming atoms is from n to m. For example, piperidinyl is an example of a 6-membered heterocycloalkyl ring, pyrazolyl is an example of a 5-membered heteroaryl ring, and pyridinyl is an example of a 6-membered heteroaryl ring.

Unless otherwise specified, the numerical ranges recited in the specification and claims are equivalent to specifically reciting each and every individual integer value therein. For example, the numerical range "1-10" is equivalent to the disclosure of every integer value within the range "1-10", namely 1, 2, 3, 4, 5, 6, 7, 8, 9, and 10.

Unless otherwise specified, "more" refers to three or more, such as 3, 4, 5, 6, 7, 8, or 9.

The term "$C_{1-6}$ alkyl" refers to a straight or branched chain saturated hydrocarbon group having 1, 2, 3, 4, 5, or 6 carbon atoms. The $C_{1-6}$ alkyl includes $C_{1-3}$ alkyl, $C_{1-4}$ alkyl, $C_{3-4}$ alkyl, $C_{4-6}$ alkyl, and the like. Examples of the alkyl include, but are not limited to, methyl, ethyl, n-propyl, *n*-butyl, n-pentyl, n-hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, and the like, or isomers thereof.

The term "$C_{2-6}$ alkynyl" refers to a straight or branched hydrocarbon group having 2 to 6 carbon atoms and containing at least one carbon-carbon triple bond. The alkynyl includes, but is not limited to, ethynyl, 1-propynyl, 2-propynyl, 1-butynyl, 2-butynyl, 3-butynyl, or 2-pentynyl.

The term "$C_{3-8}$ cycloalkyl" refers to a monovalent or polyvalent cyclic alkane having 3-8 carbon atoms, which includes monocyclic, bicyclic, or tricyclic alkanes, wherein the bicyclic and tricyclic alkanes include fused rings, bridged rings, or spiro rings. The $C_{3-8}$ cycloalkyl includes $C_{4-8}$ cycloalkyl, $C_{3-5}$ cycloalkyl, $C_{3-6}$ cycloalkyl, $C_{4-6}$ cycloalkyl, $C_{3-4}$ cycloalkyl, $C_{5-6}$ cycloalkyl, and the like. The term "$C_{3-6}$ cycloalkyl" refers to a monovalent or polyvalent cyclic alkane having 3-6 carbon atoms, which includes monocyclic, bicyclic, or tricyclic alkanes, wherein the bicyclic and tricyclic alkanes include fused rings, bridged rings, or spiro rings. Examples of the $C_{3-8}$ cycloalkyl include, but are not limited to, cyclopropyl, cyclobutyl, cyclopentyl, cyclohexyl, cycloheptyl, cyclooctyl, spiro[2.2]pentyl, spiro[2.3]hexyl, bicyclo[1.1.1]pentyl, bicyclo[3.1.0]hexyl, bicyclo[2.1.1]hexyl, and the like.

The term "$C_{6-10}$ aryl" refers to a monovalent or polyvalent aromatic monocyclic, bicyclic, or tricyclic hydrocarbon ring having 6, 7, 8, 9, or 10 carbon atoms, wherein the bicyclic and tricyclic hydrocarbon rings may be fused rings. Where the ring has 6 carbon atoms ("$C_6$ aryl"), such as phenyl; or the ring has 9 carbon atoms ("$C_9$ aryl"), such as indenyl; or the ring has 10 carbon atoms ("$C_{10}$ aryl"), such as naphthyl. When the $C_{6-10}$ aryl is substituted, it may be monosubstituted or polysubstituted. Moreover, there is no restriction on the substitution site, which may be, for example, ortho, para, or meta substitution.

The term "5- to 10-membered heteroaryl" refers to a monovalent or polyvalent monocyclic, bicyclic, or tricyclic aromatic ring system having 5 to 10 ring atoms, wherein the ring atoms include 1 to 5 heteroatoms independently selected from the group consisting of N, O, and S, and the bicyclic and tricyclic aromatic ring systems may be fused rings. The 5- to 10-membered heteroaryl preferably has 1 to 3 heteroatoms. Additionally, in each case, the 5- to 10-membered heteroaryl may be benzo-fused. The 5- to 10-membered heteroaryl includes 5- to 9-membered heteroaryl, 5- to 9-membered heteroaryl, 6- to 9-membered heteroaryl, 5- to 6-membered heteroaryl, 6-membered heteroaryl, and the like. Examples of the heteroaryl include, but are not limited to, oxazolyl, pyrazolyl, thienyl, pyridinyl, pyrimidinyl, pyrazinyl, pyridazinyl, and the like. When the 5- to 10-membered heteroaryl is substituted, it may be monosubstituted or polysubstituted. Moreover, there is no restriction on the substitution site, for example, a hydrogen atom connected to a carbon atom on a heteroaryl ring may be substituted, or a hydrogen atom connected to a heteroatom on a heteroaryl ring may be substituted.

The term "4- to 10-membered heterocyclyl" refers to a saturated or unsaturated non-aromatic ring or ring system having 4 to 10 ring atoms, wherein the ring atoms contain 1, 2, 3, 4, 5, or more heteroatoms selected from the group consisting of O, S, and N, wherein N and S may optionally be oxidized to various oxidation states to form N-oxides, -S(=O)-, or $-S(=O)_2-$ states. The 4- to 10-membered heterocyclyl may be a 4-, 5-, 6-, or 7-membered monocyclic ring system, a 7-, 8-, 9-, or 10-membered bicyclic ring system, or a 10-membered tricyclic ring system, wherein the bicyclic and tricyclic ring systems include fused rings, bridged rings, or spiro rings. The 4- to 10-membered heterocyclyl includes 4- to 8-membered heterocyclyl, 4- to 6-membered heterocyclyl, 4- to 5-membered heterocyclyl, and the like. The heterocyclyl may be attached to the remainder of the molecule via any carbon atom or nitrogen atom (if present) on its ring. Examples of the heterocyclyl include, but are not limited to: 4-membered rings, such as azetidinyl, oxetanyl; 5-membered rings, such as tetrahydrofuranyl, dioxolyl, 2,3-dihydro-1*H*-imidazolyl, pyrrolidinyl, imidazolidinyl, pyrazolidinyl, pyrrolinyl; or 6-membered rings, such as dihydrofuranyl, dihydropyranyl, tetrahydropyranyl, dihydropyridinyl, piperidinyl, morpholinyl, dithianyl, thiomorpholinyl, piperazinyl, or trithianyl. Optionally, the heterocyclyl may be benzo-fused. The heterocyclyl may be bicyclic, for example but not limited to 5,5-membered rings, such as 4,6-dihydro-1*H*-furo[3,4-*c*]pyrazolyl, 2,6-dihydro-4*H*-furo[3,4-*c*]pyrazolyl, 5,6-dihydro-1*H*-furo[3,2-*c*]pyrazolyl, 5,6-dihydro-2*H*-furo[3,2-*c*]pyrazolyl, 2,3-dihydropyrazolo[5,1-*b*]oxazolyl, 5,6-dihydro-4*H*-pyrrolo[1,2-*b*]pyrazolyl, 2,4,5,6-tetracyclopenta[*c*]pyrazolyl, and the like.

The term "4- to 10-membered heterocycloalkyl" refers to a saturated ring or ring system having 4 to 10 ring atoms, wherein the ring atoms include 1, 2, 3, 4, or 5 heteroatoms selected from the group consisting of O, S, and N, and wherein N and S may optionally be oxidized to various oxidation states to form N-oxides, -S(=O)-, or $-S(=O)_2-$ states. The 4- to 10-membered heterocycloalkyl may be a 4-, 5-, 6-, or 7-membered monocyclic ring system, a 7-, 8-, 9-, or 10-membered bicyclic ring system, or a 10-membered tricyclic ring system, wherein the bicyclic and tricyclic systems include fused rings, bridged rings, or spiro rings. The 4- to 10-membered heterocycloalkyl includes 5- to 8-membered heterocycloalkyl, 4- to 6-membered heterocycloalkyl, 4- to 5-membered heterocycloalkyl, 5- to 6-membered heterocycloalkyl, and the like, wherein the 5- to 8-membered heterocycloalkyl contains one, two, or three N and zero, one, or two O or S. Examples of the heterocycloalkyl include, but are not limited to: azetidinyl, oxetanyl, tetrahydrofuranyl, pyrrolidinyl, imidazolidinyl, pyrazolidinyl, tetrahydropyranyl, morpholinyl, piperidinyl, piperazinyl, 3,8-diazabicyclo[3.2.1]octyl, and the like.

The term "spiro ring" refers to a ring system in which two rings share one ring-forming atom.

The term "fused ring" refers to a ring system in which two rings share two ring-forming atoms.

The term "bridged ring" refers to a ring system in which two rings share three or more ring atoms.

The term "halogen" refers to fluorine, chlorine, bromine, and iodine.

"Halo" refers to substitution by one or more halogens.

The term "$C_{1-6}$ haloalkyl" refers to an alkyl group as defined above, which is substituted by one or more halogens as defined above. The haloalkyl includes, but is not limited to, fluoromethyl, difluoromethyl, trifluoromethyl, trichloromethyl, 2,2,2-trifluoroethyl, 2,2-difluoroethyl, 1-fluoromethyl-2-fluoroethyl, 3-bromo-2-fluoropropyl, 1-bromomethyl-2-bromoethyl, and the like.

A person skilled in the art will appreciate that the compound represented by formula (I) may exist in the form of various pharmaceutically acceptable salts. If these compounds have a basic center, they can form acid addition salts; if these compounds have an acidic center, they can form base addition salts; if these compounds contain both an acidic center (*e.g.,* a carboxyl group) and a basic center (*e.g.,* an amino group), they can also form inner salts.

The compounds of the present disclosure may exist in the form of solvates (*e.g.*, hydrates), wherein the compounds of the present disclosure include polar solvents, particularly such as water, methanol, or ethanol, as structural elements of the crystal lattice of the compounds. The amount of polar solvent, particularly water, may be present in a stoichiometric or non-stoichiometric ratio.

Based on their molecular structures, the compounds of the present disclosure may be chiral (having one or more stereocenters), and thus may exist in various enantiomeric forms. Thus, these compounds may exist in racemic form or in optically active form. The compounds of the present disclosure encompass isomers in which each chiral carbon is in R or S configuration, or mixtures and racemates thereof. The compounds of the present disclosure or intermediates thereof may be separated into enantiomeric compounds by chemical or physical methods well known to those skilled in the art, or used in this form for synthesis. In the case of racemic amines, diastereomers are prepared from the mixture by reaction with an optically active resolving agent. Examples of suitable resolving agents are optically active acids such as tartaric acid, diacetyl tartaric acid, dibenzoyl tartaric acid, mandelic acid, malic acid, lactic acid, suitable N-protected amino acids (*e.g.*, N-benzoyl proline or N-benzenesulfonyl proline), or various optically active camphorsulfonic acids in R and S configurations. With the aid of optically active resolving agents (such as dinitrobenzoyl phenylglycine immobilized on silica gel, cellulose triacetate, or other carbohydrate derivatives or chirally derivatized isobutenoic ester polymers), chromatographic enantiomer resolution can also be advantageously performed. A suitable eluent for this purpose is an aqueous or alcoholic solvent mixture, such as hexane/isopropanol/acetonitrile.

In some embodiments, the compounds of the present disclosure have the (*R*)-configuration. In other embodiments, the compound has the (*S*)-configuration. In compounds having more than one chiral center, unless otherwise specified, each chiral center in the compound may independently be (*R*) or*(S*).

The compounds described herein may also include tautomeric forms. Tautomeric forms result from the exchange of a single bond with an adjacent double bond and the concomitant migration of a proton. Tautomeric forms include prototropic tautomers, which are isomeric protonation states with the same empirical formula and total charge. Examples of prototropic tautomers include ketoenol pairs, amide-imidic acid pairs, lactam-lactim pairs, enamine-imine pairs, and cyclic forms where a proton may occupy two or more positions of a heterocyclic system, such as 1*H*- and 3*H*-imidazole, *1H-, 2H-,* and 4*H*-1,2,4-triazole, 1*H*- and 2*H*-isoindole, and 1*H*- and 2*H*-pyrazole. Tautomeric forms may be in equilibrium or spatially locked into one form by appropriate substitution. The present disclosure is intended to encompass all such tautomers of the compounds.

The compounds described herein may also include all isotopes of atoms present in intermediates or final compounds. Isotopes include those atoms that have the same atomic number but different mass numbers. For example, isotopes of hydrogen include tritium and deuterium.

The corresponding stable isomers can be separated according to known methods, such as by extraction, filtration, or column chromatography.

The term "patient" refers to any animal, including mammals, preferably mice, rats, other rodents, rabbits, dogs, cats, pigs, cattle, sheep, horses, or primates, and most preferably humans.

The term "therapeutically effective amount" refers to the amount of an active compound or drug that a researcher, veterinarian, physician, or other clinician is seeking in a tissue, system, animal, individual, or human to elicit a biological or medical response, which includes one or more of the following: (1) preventing a disease: for example, preventing a disease, disorder, or condition in an individual who is susceptible to the disease, disorder, or condition but has not yet experienced or exhibited the pathology or symptoms of the disease. (2) Inhibiting the disease: for example, inhibiting the disease, disorder, or condition in an individual experiencing or exhibiting the pathology or symptoms of the disease,

disorder, or condition (*i.e.*, preventing further development of the pathology and/or symptoms). (3) Alleviating the disease: for example, alleviating the disease, disorder, or condition in an individual experiencing or exhibiting the pathology or symptoms of the disease, disorder, or condition (*i.e.,* reversing the pathology and/or symptoms).

DETAILED DESCRIPTION OF THE PREFERRED EMBODIMENT

Hereinafter, the compounds of the general formula of the present disclosure, their preparation methods, and uses will be further described in detail in conjunction with specific examples. The following examples are only used to illustrate and explain the present disclosure, and should not be construed as a limitation on the scope of protection of the present disclosure. All techniques realized based on the above contents of the present disclosure are within the scope of protection intended by the present disclosure.

Unless otherwise stated, the raw materials and reagents used in the following examples are commercially available or can be prepared by known methods.

**Method summary:**

The chiral purity of the compound of the present disclosure was tested using the following HPLC method: HPLC method A: CHIRALPAK AD-H, 4.6 * 250 mm 5 μm, 30% isopropanol/n-hexane, 0.8 mL/min, 30°C.

HPLC method B: CHIRALPAK AD-H, 4.6 * 250 mm 5 μm, 30% ethanol/n-hexane, 0.8 mL/min, 30°C.

HPLC method C: CHIRALPAK AD-H, 4.6 * 250 mm 5 μm, 20% ethanol/0.05% diethylamine/n-hexane, 0.8 mL/min, 30°C.

HPLC method D: CHIRALPAK AD-H, 4.6 * 250 mm 5 μm, 15% ethanol/n-hexane, 0.8 mL/min, 30°C.

HPLC method E: CHIRALPAK AD-H, 4.6 * 250 mm 5 μm, 15% ethanol/0.05% diethylamine/n-hexane, 0.8 mL/min, 30°C.

HPLC method F: CHIRALPAK AD-H, 4.6 * 250 mm 5 μm, 20% isopropanol/0.1% diethylamine/n-hexane, 0.8 mL/min, 30°C.

HPLC method G: CHIRALPAK AD-H, 4.6 * 250 mm 5 μm, 10% ethanol/n-hexane, 0.8 mL/min, 30°C.

HPLC method H: CHIRALPAK AD-H, 4.6 * 250 mm 5 μm, 20% ethanol/n-hexane, 0.8 mL/min, 30°C.

HPLC method I: CHIRALPAK AD-H, 4.6 * 250 mm 5 μm, 20% ethanol/0.1% trifluoroacetic acid/n-hexane, 0.8 mL/min, 30°C.

HPLC method J: CHIRALPAK AD-H, 4.6 * 250 mm 5 μm, 25% ethanol/n-hexane, 0.8 mL/min, 30°C.

HPLC method K: CHIRALPAK AD-H, 4.6 * 250 mm 5 μm, 35% isopropanol/0.05% diethylamine/*n*-hexane, 0.8 mL/min, 30°C.

HPLC method L: CHIRALPAK AD-H, 4.6 * 250 mm 5 μm, 25% ethanol/0.1% diethylamine/*n*-hexane, 0.8 mL/min, 30°C.

HPLC method M: CHIRALPAK AD-H, 4.6 * 250 mm 5 μm, 30% ethanol/0.1% diethylamine/*n*-hexane, 0.8 mL/min, 30°C.

HPLC method N: CHIRALPAK AD-H, 4.6 * 250 mm 5 μm, 25% isopropanol/0.1% diethylamine/*n*-hexane, 0.8 mL/min, 30°C.

HPLC method O: CHIRALPAKAD-H, 4.6 * 250 mm 5 μm, 20% isopropanol/*n*-hexane, 0.8 mL/min, 30°C.

HPLC method P: CHIRALPAKAD-H, 4.6 * 250 mm 5 μm, 10% isopropanol/10% ethanol/n-hexane, 0.8 mL/min, 30°C.

HPLC method Q: CHIRALPAK IC, 4.6 * 250 mm 5 μm, 10% isopropanol/20% ethanol/n-hexane, 0.8 mL/min, 30°C.

HPLC method R: CHIRALPAK AD-H, 4.6 * 250 mm 5 μm, 20% isopropanol/0.1% diethylamine/n-hexane, 0.8 mL/min, 30°C.

**Terms and representative reagents**

Unless otherwise specified, the nouns used in the following specific experimental descriptions represent the following reagents, respectively:

i-PrMgCl·LiCl: isopropylmagnesium chloride-lithium chloride complex
$(Boc)_2O$: di-tert-butyl dicarbonate; $B_2Pin_2$: bis(pinacolato)diboron; DIAD: diisopropyl azodicarboxylate; DMF: *N,N*-dimethylformamide; DCM: dichloromethane; DIEA: *N,N*-diisopropylethylamine; $Et_3N$: triethylamine; EA: ethyl acetate; KOAc: potassium acetate; MeOH: methanol; MeCN: acetonitrile; $PPh_3$: triphenylphosphine; $Pd_2(dba)_3$: tris(dibenzylideneacetone)dipalladium; $Pd(dppf)Cl_2$: [1,1'-bis(diphenylphosphino)ferrocene]dichloropalladium(II); $Pd(PPh_3)_4$: tetrakis(triphenylphosphine)palladium; TEA: triethylamine; TFA: trifluoroacetic acid; THF: tetrahydrofuran; X-Phos: 2-dicyclohexylphosphino-2',4',6'-triisopropylbiphenyl.

**Preparation example**

Example 1. Synthesis of (*R*)-4-((2-(((3-fluoropyridin-2-yl)(1-methylcyclopentyl)methyl)amino)-3,4-dioxocyclobut-1-en-1-yl)amino)-3-hydroxy-*N*,*N*-dimethylpicolinamide (compound 001)

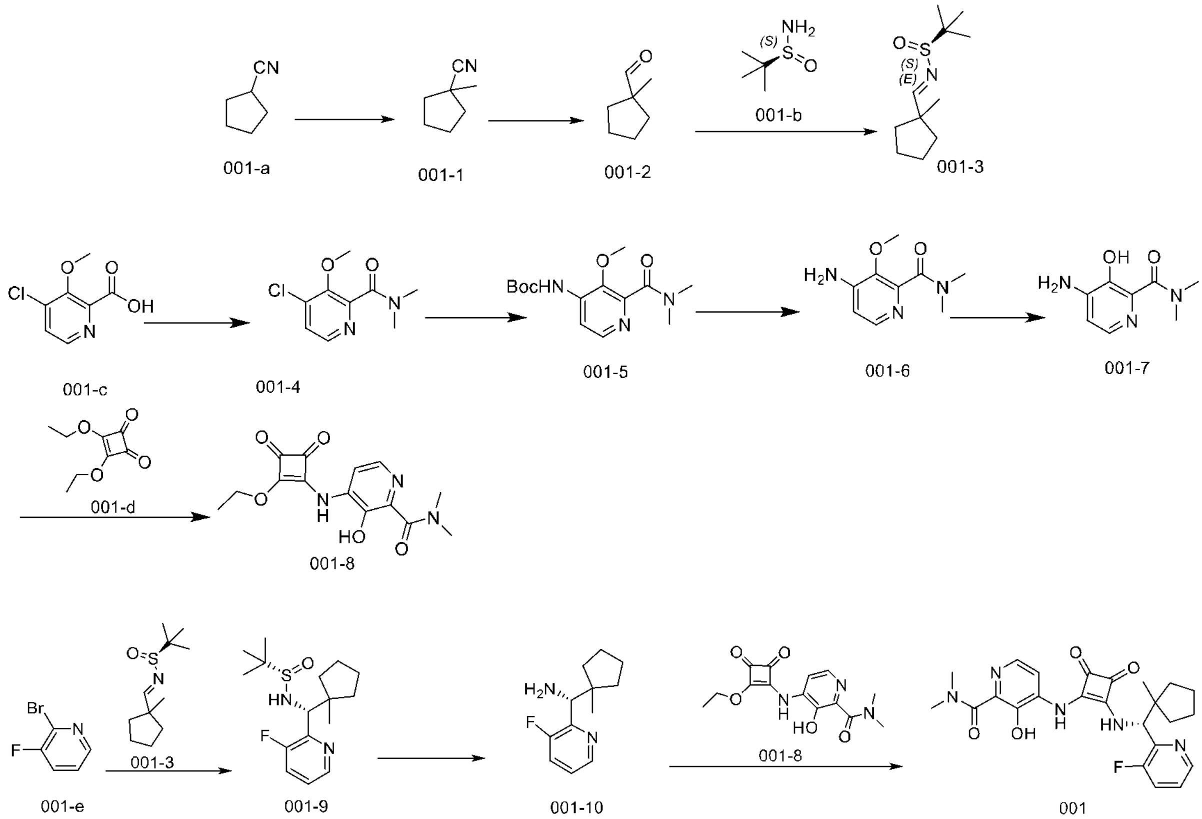

001-a 001-1 001-2 001-3
001-c 001-4 001-5 001-6 001-7
001-8
001-e 001-9 001-10 001

1.1 Synthesis of compound 001-1

At room temperature, 001-a (15.0 g, 157 mmol) was dissolved in tetrahydrofuran (150 mL). Under a nitrogen atmosphere at -78°C, lithium hexamethyldisilazide (173 mL, 173 mmol, 1 M) was added, and the mixture was stirred at this temperature for 1 hour. Then, iodomethane (29.1 g, 205 mmol) was added at -78°C. After the addition was completed, the reaction mixture was slowly warmed to room temperature and stirred for 2 hours. The reaction mixture was quenched with saturated aqueous ammonium chloride solution (100 mL) and extracted with ethyl acetate (50 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 001-1 (14.2 g, yield: 83%).

1.2 Synthesis of compound 001-2

At room temperature, 001-1 (17.0 g, 155.0 mmol) was dissolved in dichloromethane (60 mL). Under a nitrogen atmosphere at -78°C, diisobutylaluminum hydride (389 mL, 389.0 mmol, 1 M) was added, and the reaction was carried out at -78°C for 2 hours. A 20% aqueous ammonium chloride solution (50 mL) was added to quench the reaction. The mixture was extracted with dichloromethane (50 mL × 3). The organic phases were combined, washed with saturated brine (50 mL), dried over anhydrous sodium sulfate, filtered, and the filtrate was concentrated under reduced pressure to obtain compound 001-2 (15.6 g, crude product).

1.3 Synthesis of compound 001-3

At room temperature, 001-2 (15.6 g, crude product) was dissolved in dichloromethane (50 mL). Tetraethyl titanate (105.6 g, 372 mmol) and (*S*)-*tert*-butylsulfinamide (18.7 g, 155.0 mmol) were added, and the reaction was carried out at room temperature for 12 hours. The reaction mixture was quenched with water (100 mL) and extracted with ethyl acetate (50 mL × 3). The combined organic phases were directly concentrated under reduced pressure. The residue was purified by silica gel column chromatography to obtain compound 001-3 (17.0 g, yield over two steps 1.2 and 1.3: 51.5%).

1.4 Preparation of compound 001-4

001-c (10 g, 53.3 mmol) was weighed and added to dichloromethane (100 mL). At 0°C, oxalyl chloride (9 mL, 106.6 mmol) and two drops of *N,N*-dimethylformamide were added dropwise. The temperature was raised to ambient temperature, and stirring was continued for 2 hours. The reaction mixture was concentrated under reduced pressure, and the residue was dissolved in dichloromethane (150 mL). At 0°C, triethylamine (22.3 mL, 160 mmol) was added, followed by dropwise addition of a solution of dimethylamine (32 mL, 64 mmol, 2 N) in tetrahydrofuran, maintaining the internal temperature below 5°C during the addition. After the addition was completed, the reaction mixture was warmed to room temperature, and stirring was continued for 12 hours. The reaction mixture was quenched with water (200 mL) and extracted with dichloromethane (200 mL × 2). The combined organic phases were washed with saturated brine (200 mL), dried over anhydrous sodium sulfate, filtered, and the filtrate was concentrated. The residue was purified by silica gel column chromatography (petroleum ether/ethyl acetate = 1/1) to obtain compound 001-4 (11 g).

1.5 Preparation of compound 001-5

001-4 (11 g, 51.25 mmol) was dissolved in 1,4-dioxane (300 mL). *tert*-Butyl carbamate (12 g, 102.5 mmol), cesium carbonate (33.4 g, 102.5 mmol), palladium acetate (1.15 g, 5.12 mmol), and 2-dicyclohexylphosphino-2',4',6'-triisopropylbiphenyl (2.2 g, 5.12 mmol) were added. Under a nitrogen atmosphere, the reaction mixture was stirred at 95°C for 16 hours. The reaction mixture was cooled to 20°C, filtered through a pad of diatomite, and the filtrate was concentrated. The residue was purified by silica gel column chromatography (petroleum ether/ethyl acetate = 1/1) to obtain compound 001-5 (10 g).

1.6 Preparation of compound 001-6

001-5 (10 g, 34 mmol) was dissolved in dichloromethane (400 mL), and a solution of hydrogen chloride in 1,4-dioxane (52 mL, 4 M) was added. The reaction mixture was stirred at 20°C for 4 hours. The reaction mixture was directly concentrated to obtain compound 001-6 (10 g, crude product), which was used directly in the next step.

1.7 Preparation of compound 001-7

001-6 (10 g, 51.2 mmol) was dissolved in dichloromethane (50 mL). Tetrabutylammonium iodide (11.4 g, 30.7 mmol) was added. Under a nitrogen atmosphere, boron tribromide (154 mL, 154 mmol) was slowly added dropwise at 0°C. After the dropwise addition was completed, the reaction mixture was warmed to room temperature and stirred overnight. A saturated aqueous sodium bicarbonate solution (200 mL) was slowly added dropwise to quench the reaction. The resulting mixture was extracted with dichloromethane (200 mL × 2). The organic phases were discarded. The aqueous phase was concentrated to obtain compound 001-7 (35 g, crude product), which was directly used in the next step.

1.8 Preparation of compound 001-8

001-7 (35 g, 193.2 mmol) was weighed and dissolved in ethanol (150 mL). 001-d (6.78 g, 38.64 mmol) and *N*,*N*-diisopropylethylamine (21.6 g, 165.5 mmol) were added. The reaction mixture was stirred at 55°C overnight and then directly concentrated under reduced pressure. The residue was purified by silica gel column chromatography to obtain compound 001-8 (4.2 g).

1.9 Synthesis of compound 001-9

001-e (300 mg, 1.71 mmol) was dissolved in tetrahydrofuran (3 mL). The system was purged with nitrogen three times. At 0°C, isopropylmagnesium chloride-lithium chloride complex (2.70 mL, 3.42 mmol) was added dropwise to the reaction mixture. After the addition was completed, the reaction mixture was warmed to room temperature and stirred for 1 hour. The system temperature was again lowered to 0°C. A solution of 001-3 (235.2 mg, 1.38 mmol) in tetrahydrofuran (0.3

mL) was added dropwise to the reaction mixture. After the addition was completed, the temperature was returned to room temperature, and the reaction was stirred for 1 hour. The reaction mixture was poured into saturated aqueous ammonium chloride solution (10 mL) to quench, then extracted with ethyl acetate (15 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 001-9 (250 mg, yield: 58.00%). LC-MS: $[M+H]^+$ = 313.00.

1.10 Synthesis of compound 001-10

At room temperature, 001-9 (230 mg, 0.73 mmol) was added to a solution of hydrogen chloride in ethanol (6 mL, 1 M) and stirred at room temperature for 0.2 hours. The reaction mixture was directly concentrated under reduced pressure to obtain compound 001-10 (247 mg, crude product). LC-MS: $[M+H]^+$ = 209.10.

1.11 Synthesis of compound 001

001-10 (100 mg, 0.48 mmol) was dissolved in ethanol (2 mL). 001-8 (145 mg, 0.48 mmol) and *N,N*-diisopropylethylamine (323 mg, 2.40 mmol) were added, and the reaction mixture was stirred at room temperature for 1 hour. Then, the reaction mixture was purified by preparative HPLC (high-performance liquid chromatography, basic conditions) and lyophilized to obtain compound 001 (97.4 mg, chiral purity: 100% (HPLC method A)). LC-MS: $[M+H]^+$ = 467.95.

$^1$H NMR (400 MHz, DMSO-$d_6$): δ 11.56 (s, 1H), 10.16 (s, 1H), 9.44 (d, *J* = 9.8 Hz, 1H), 8.52 (d, *J* = 4.6 Hz, 1H), 7.97 (d, *J* = 5.2 Hz, 1H), 7.93 (s, 1H), 7.77 (t, *J* = 9.2 Hz, 1H), 7.48 (dt, *J* = 8.4, 4.4 Hz, 1H), 5.78 (d, *J* = 9.8 Hz, 1H), 3.16 (s, 3H), 3.04 (s, 3H), 1.80-1.63 (m, 2H), 1.60 (t, *J* = 13.0 Hz, 4H), 1.35-1.25 (m, 1H), 1.13 (dd, *J* = 27.0, 6.2 Hz, 1H), 0.97 (s, 3H).

Example 2. Synthesis of (*R*)-4-((2-(((6-fluoropyridin-2-yl)(1-methylcyclopentyl)methyl)amino)-3,4-dioxocyclobut-1-en-1-yl)amino)-3-hydroxy-*N*,*N*-dimethylpicolinamide (compound 002)

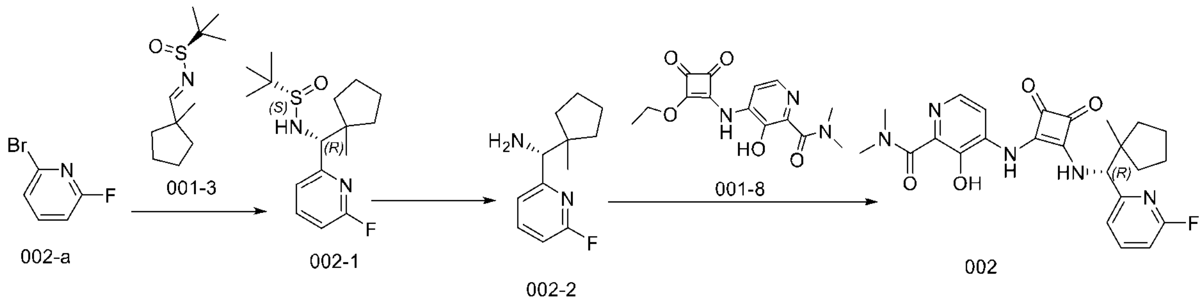


The synthesis of compound 002 was carried out with reference to the synthesis method for compound 001 in Example 1, except that the starting material 001-e was replaced with 002-a, to obtain compound 002 (123.1 mg, chiral purity: 99.6% (HPLC method C), yield: 42%).

$^1$H NMR (400 MHz, DMSO-$d_6$): δ 9.29 (d, *J* = 12.0 Hz, 1H), 8.10-7.80 (m, 3H), 7.34 (d, *J* = 8.0 Hz, 1H), 7.12 (d, *J* = 8.0 Hz, 1H), 5.33 (d, *J*= 8.0 Hz, 1H), 3.16 (s, 3H), 3.03 (s, 3H), 1.76-1.66 (m, 2H), 1.58 (s, 4H), 1.35-1.25 (m, 1H), 1.16-1.07 (m, 1H), 0.92 (s, 3H); LC-MS: $[M+H]^+$ = 467.95.

Example 3. Synthesis of (*R*)-4-((2-(((6-chloropyridin-2-yl)(1-methylcyclopentyl)methyl)amino)-3,4-dioxocyclobut-1-en-1-yl)amino)-3-hydroxy-*N,N*-dimethylpicolinamide (compound 003)

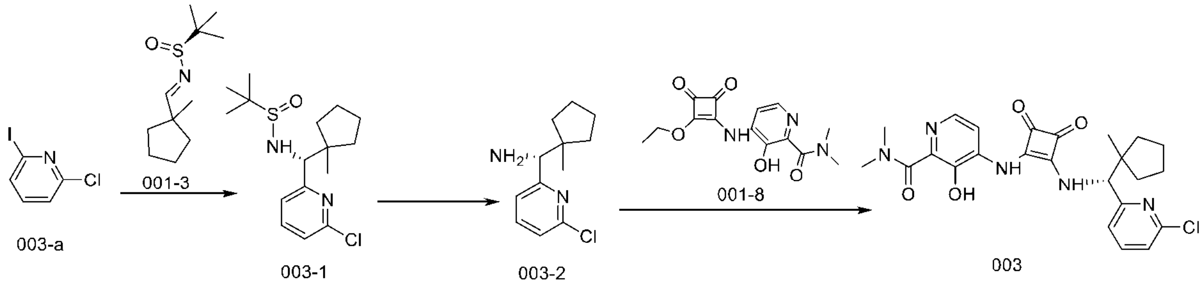


003-a 003-1 003-2 003

The synthesis of compound 003 was carried out with reference to the synthesis method for compound 001 in Example 1, except that the starting material 001-e was replaced with 003-a, to obtain compound 003 (68.4 mg, chiral purity: 100% (HPLC method E)).

$^1$H NMR (400 MHz, DMSO-$d_6$): δ 10.07 (s, 1H), 9.25 (s, 1H), 7.98 (s, 2H), 7.88 (t, *J* = 8.0 Hz, 1H), 7.48 (d, *J* = 8.0 Hz, 1H), 7.42 (d, *J* = 7.6 Hz, 1H), 5.40-5.32 (m, 1H), 3.17 (s, 3H), 3.05 (s, 3H), 1.76-1.71 (m, 1H), 1.61 (s, 4H), 1.37-1.29 (m, 1H), 1.22-1.09 (m, 2H), 0.96 (s, 3H); LC-MS: $[M+H]^+$ = 484.00.

Example 4. Synthesis of (*R*)-3-hydroxy-*N,N*-dimethyl-4-((2-(((1-methylcyclopentyl)(pyridin-2-yl)methyl)amino)-3,4-dioxocyclobut-1-en-1-yl)amino)picolinamide (compound 004)

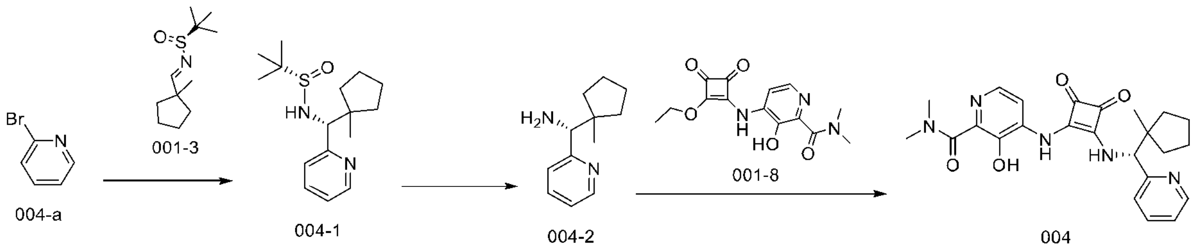


004-a 004-1 004-2 004

The synthesis of compound 004 was carried out with reference to the synthesis method for compound 001 in Example 1, except that the starting material 001-e was replaced with 004-a, to obtain compound 004 (26.0 mg, chiral purity: 97.4% (HPLC method H)).

$^1$H NMR (400 MHz, DMSO-$d_6$): δ 11.69 (s, 1H), 10.05 (s, 1H), 9.40 (d, *J* = 9.8 Hz, 1H), 8.63 (d, *J* = 4.4 Hz, 1H), 7.98 (d, *J* = 7.2 Hz, 2H), 7.80 (td, *J* = 7.8, 1.7 Hz, 1H), 7.43-7.32 (m, 2H), 5.38-5.30 (m, 1H), 3.12 (d, *J* = 57.0 Hz, 6H), 1.75 (t, *J* = 10.0 Hz, 2H), 1.59 (s, 4H), 1.35-1.25 (m, 1H), 1.17-1.06 (m, 1H), 0.93 (s, 3H); LC-MS: $[M+H]^+$ = 450.00.

Example 5. Synthesis of (*R*)-4-((2-(((5-fluoropyridin-2-yl)(1-methylcyclopentyl)methyl)amino)-3,4-dioxocyclobut-1-en-1-yl)amino)-3-hydroxy-*N*,*N*-dimethylpicolinamide (compound 005)

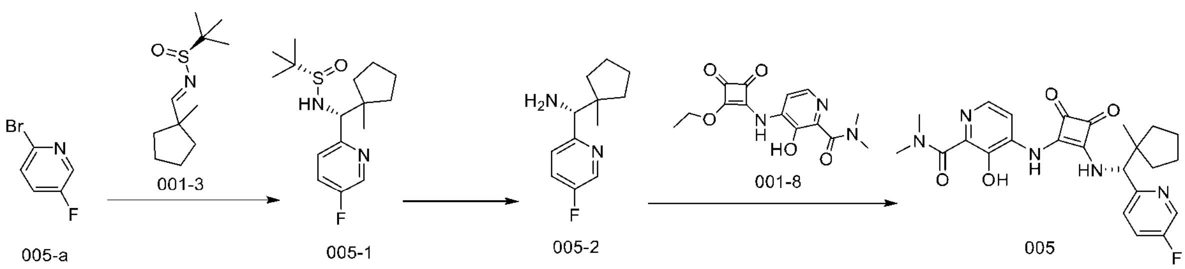


005-a 005-1 005-2 005

The synthesis of compound 005 was carried out with reference to the synthesis method for compound 001 in Example 1, except that the starting material 001-e was replaced with 005-a, to obtain compound 005 (59.5 mg, chiral purity: 100% (HPLC method D)).

$^1$H NMR (400 MHz, DMSO-$d_6$): δ 9.39 (s, 1H), 8.63 (d, *J* = 4.0 Hz, 1H), 7.98 (d, *J* = 4.0 Hz, 2H), 7.76-7.71 (m, 1H), 7.50 (dd, *J* = 4.0, 4.0 Hz, 1H), 5.38 (d, *J* = 8.0 Hz, 1H), 3.17 (s, 3H), 3.05 (s, 3H), 1.77-1.69 (m, 2H), 1.60 (s, 4H), 1.34-1.28

(m, 1H), 1.14-1.08 (m, 1H), 0.93 (s, 3H); LC-MS: $[M+H]^+$ = 468.00.

Example 6. Synthesis of (*R*)-3-hydroxy-*N*,*N*-dimethyl-4-((2-(((1-methylcyclopentyl)(pyrazin-2-yl)methyl)amino)-3,4-dioxocyclobut-1-en-1-yl)amino)picolinamide (compound 006)

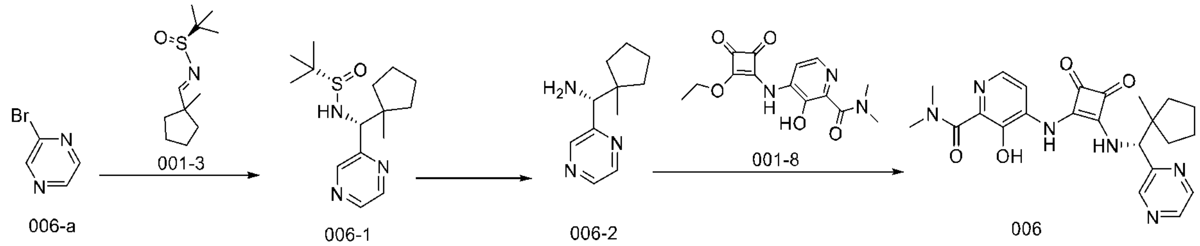


The synthesis of compound 006 was carried out with reference to the synthesis method for compound 001 in Example 1, except that the starting material 001-e was replaced with 006-a, to obtain compound 006 (31.4 mg, yield: 29%, chiral purity: 98.0% (HPLC method H)).

$^1$H NMR (600 MHz, DMSO-$d_6$): δ 11.70 (s, 1H), 10.03 (s, 1H), 9.38 (s, 1H), 8.75-8.70 (m, 2H), 8.63 (s, 1H), 7.98 (s, 1H), 5.44 (d, *J* = 8.0 Hz, 1H), 3.17 (s, 3H), 3.05 (s, 3H), 1.79-1.72 (m, 2H), 1.62-1.57 (m, 4H), 1.36-1.31 (m, 1H), 1.14-1.11 (m, 1H), 0.94 (s, 3H); LC-MS: $[M+H]^+$ = 451.00.

Example 7. Synthesis of (*R*)-3-hydroxy-*N*,*N*-dimethyl-4-((2-(((1-methylcyclopentyl)(3-(trifluoromethyl)pyridin-2-yl)methyl)amino)-3,4-dioxocyclobut-1-en-1-yl)amino)picolinamide (compound 007)

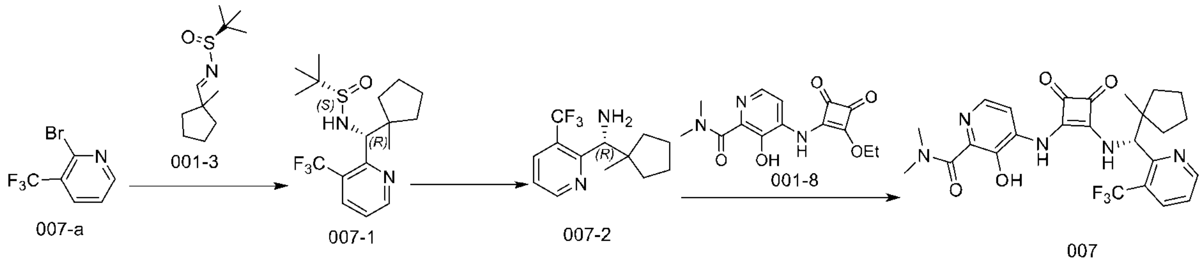


The synthesis of compound 007 was carried out with reference to the synthesis method for compound 001 in Example 1, except that the starting material 001-e was replaced with 007-a, to obtain compound 007 (56.0 mg, chiral purity: 100% (HPLC method O)).

$^1$H NMR (400 MHz, DMSO-$d_6$): δ 9.23 (s, 1H), 8.76 (d, *J*= 3.4 Hz, 1H), 8.12 (d, *J*= 8.0 Hz, 1H), 7.71 (d, *J* = 5.0 Hz, 1H), 7.48 (dd, *J* = 7.8, 4.8 Hz, 1H), 7.40 (s, 1H), 5.77 (s, 1H), 2.83 (s, 3H), 2.72 (s, 3H), 1.83 - 1.72 (m, 2H), 1.58 - 1.41 (m, 4H), 1.35 - 1.28 (m, 1H), 1.19 (dd, *J*= 10.6, 6.4 Hz, 1H), 0.88 (s, 3H); LC-MS: $[M+H]^+$ = 518.00.

Example 8. Synthesis of (*R*)-3-hydroxy-N,N-dimethyl-4-((2-(((1-methylcyclopentyl)(6-(trifluoromethyl)pyridin-2-yl)methyl)amino)-3,4-dioxocyclobut-1-en-1-yl)amino)picolinamide (compound 008)

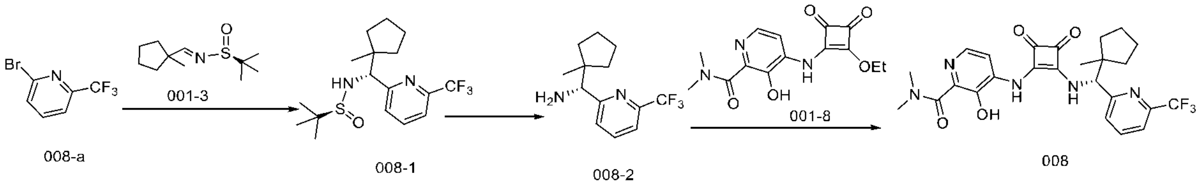


The synthesis of compound 008 was carried out with reference to the synthesis method for compound 001 in

Example 1, except that the starting material 001-e was replaced with 008-a, to obtain compound 008 (22.0 mg, yield: 21.9%, chiral purity: 99.8% (HPLC method I)).

$^1$H NMR (400 MHz, DMSO-$d_6$): δ 9.17 (s, 1H), 8.03 (t, *J* = 8.0 Hz, 1H), 7.92-7.82 (m, 3H), 7.69 (d, *J* = 8.0 Hz, 1H), 5.45 (d, *J* = 8.0 Hz, 1H), 3.13 (s, 3H), 3.02 (s, 3H), 1.76-1.70 (m, 2H), 1.60-1.52 (m, 4H), 1.36-1.33 (m, 1H), 1.14-1.11 (m, 1H), 0.94 (s, 3H); LC-MS: $[M+H]^+$ = 518.1.

Example 9. Synthesis of (*R*)-4-((2-(((3,5-difluoropyridin-2-yl)(1-methylcyclopentyl)methyl)amino)-3,4-dioxocyclobut-1-en-1-yl)amino)-3-hydroxy-*N*,*N*-dimethylpicolinamide (compound 009)

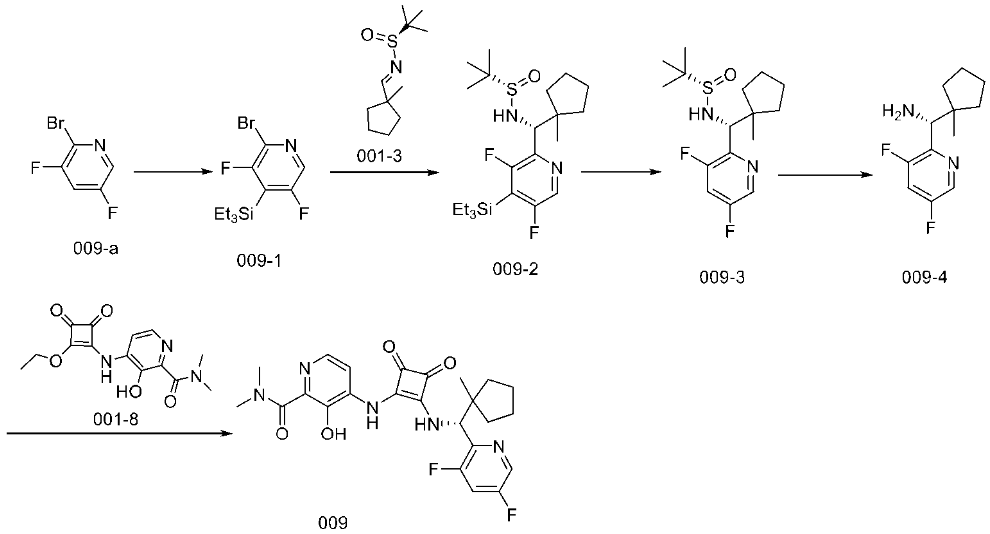


9.1 Synthesis of compound 009-1

009-a (260 mg, 1.24 mmol) was dissolved in tetrahydrofuran (3 mL). The system was purged with nitrogen three times, and the reaction mixture was cooled to -78°C. *n*-Butyllithium (0.84 mL, 1.34 mmol, 1.6 M) was added dropwise to the reaction mixture, and the reaction was carried out at this temperature for 0.5 hours. Subsequently, a solution of triethylchlorosilane (236 mg, 1.10 mmol) in tetrahydrofuran (0.3 mL) was added dropwise to the above reaction mixture, and the reaction was carried out at -78°C for 0.5 hours. The reaction mixture was poured into saturated aqueous ammonium chloride solution (10 mL) to quench, then extracted with ethyl acetate (15 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 009-1 (144 mg, yield: 38.15%). LC-MS: $[M+H]^+$ = 309.90.

9.2 Synthesis of compound 009-2

The synthesis of compound 009-2 was carried out with reference to the synthesis method for compound 001-9 in Example 1, except that the intermediate 001-e was replaced with 009-1, to obtain compound 009-2 (75 mg, yield: 51.19%). LC-MS: $[M+H]^+$ = 445.05.

9.3 Synthesis of compound 009-3

At room temperature, compound 009-2 (65 mg, 0.15 mmol) was dissolved in tetrahydrofuran (0.5 mL). Tetrabutylammonium fluoride (1 mL, 1 M) was added, and the reaction mixture was stirred at room temperature for 1 hour. The reaction mixture was poured into water (5 mL) to quench and extracted with ethyl acetate (10 mL × 3). The organic phases were combined, washed once with saturated brine (10 mL), dried over anhydrous sodium sulfate, filtered, and the filtrate was concentrated under reduced pressure to obtain compound 009-3 (40 mg, crude product). LC-MS: $[M+H]^+$ = 331.00.

9.4 Synthesis of compound 009-4

The synthesis of compound 009-4 was carried out with reference to the synthesis method for compound 001-10 in Example 1, except that the intermediate 001-9 was replaced with 009-3, to obtain compound 009-4 (40 mg, crude product). LC-MS: $[M+H]^+$ = 227.10.

9.5 Synthesis of compound 009

The synthesis of compound 009 was carried out with reference to the synthesis method for compound 001 in Example 1, except that the intermediate 001-10 was replaced with 009-4, to obtain compound 009 (31.2 mg, chiral purity: 99.8% (HPLC method B)).

$^1$H NMR (400 MHz, DMSO-$d_6$): δ 9.32 (s, 2H), 8.55 (s, 1H), 7.93 (t, *J* = 8.8 Hz, 1H), 7.59 (d, *J* = *4.8* Hz, 1H), 7.12 (s, 1H), 5.73 (s, 1H), 2.89 (s, 3H), 2.81 (s, 3H), 1.74 (d, *J*=7.2 Hz, 1H), 1.70 - 1.50 (m, 5H), 1.27 (d, *J* = 12.4 Hz, 1H), 1.16 (d, *J* = 9.6 Hz, 1H), 0.95 (s, 3H); LC-MS: $[M+H]^+$ = 485.95.

Example 10. Synthesis of (*R*)-4-((2-(((3-chloro-4-fluoropyridin-2-yl)(1-methylcyclopentyl)methyl)amino)-3,4-dioxocyclobut-1-en-1-yl)amino)-3-hydroxy-*N,N*-dimethylpicolinamide (compound 010)

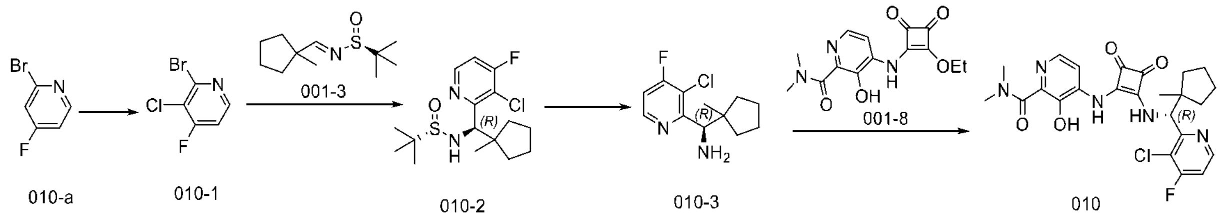


010-a 010-1 010-2 010-3 010

10.1 Synthesis of compound 010-1

Anhydrous tetrahydrofuran (15 mL) and diisopropylamine (2.6 g, 25.49 mmol) were placed in a three-necked flask. The system was purged with nitrogen three times. At -78°C, n-butyllithium solution (10.2 mL, 25.50 mmol, 2.5 M) was added dropwise slowly. The reaction mixture was slowly warmed to room temperature and stirred for 1 hour. *n*-Hexane solution (10 mL) was added. The temperature was lowered to -78°C, and 010-a (2.0 g, 17.05 mmol) was added. The reaction was carried out at this temperature for 1 hour. Then, at -78°C, 1,1,2-trifluorotrichloroethane (3.10 mL, 25.49 mmol) was added. The reaction mixture was slowly warmed to room temperature and stirred for 3 hours. The reaction was quenched with saturated aqueous ammonium chloride solution (30 mL). The resulting mixture was extracted with ethyl acetate (20 mL × 3). The organic phases were combined, washed with saturated aqueous sodium chloride solution (20 mL × 3), dried over anhydrous sodium sulfate, and filtered. The resulting filtrate was concentrated under reduced pressure. The residue was purified by silica gel column chromatography (petroleum ether/ethyl acetate = 20/1) and further purified by preparative HPLC to obtain compound 010-1 (280.0 mg). LC-MS: $[M+H]^+$ = 211.95.

10.2 Synthesis of compound 010

The synthesis of compound 010 was carried out with reference to the synthesis method for compound 001 in Example 1, except that the starting material 001-e was replaced with 010-1, to obtain compound 010 (37.6 mg, chiral purity: 99.4% (HPLC method H)).

$^1$H NMR (400 MHz, DMSO-$d_6$): δ 9.06 (s, 2H), 8.69 (d, *J* = 8.8 Hz, 1H), 8.55 (d, *J* = 8.4 Hz, 1H), 7.89 (s, 2H), 5.52 (s, 1H), 3.12 (d, *J* = 21.4 Hz, 3H), 3.01 (s, 3H), 1.64 (s, 4H), 1.44 (s, 1H), 1.18 (d, *J* = 5.8 Hz, 1H), 1.09 (d, *J* = 6.4 Hz, 2H), 0.97 (s, 3H); LC-MS: $[M+H]^+$ = 501.95.

Example 11. Synthesis of (*R*)-4-((2-(((3-chloro-5-fluoropyridin-2-yl)(1-methylcyclopentyl)methyl)amino)-3,4-dioxocyclobut-1-en-1-yl)amino)-3-hydroxy-*N,N*-dimethylpicolinamide (compound 011)

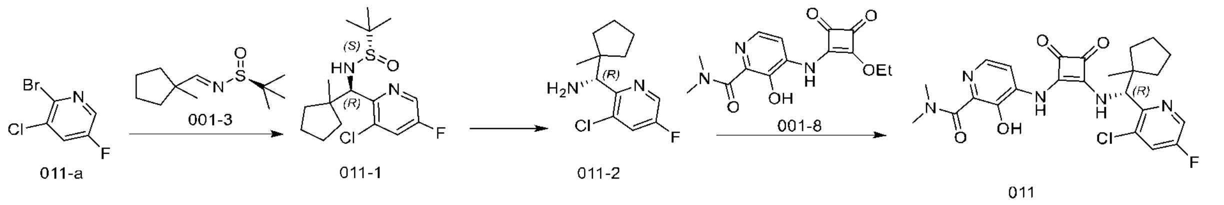


The synthesis of compound 011 was carried out with reference to the synthesis method for compound 001 in Example 1, except that the starting material 001-e was replaced with 011-a, to obtain compound 011 (17.7 mg, chiral purity: 99.7% (HPLC method H)).

$^1$H NMR (400 MHz, DMSO-$d_6$): δ 9.34 (s, 1H), 8.72 (d, $J$ = 2.4 Hz, 1H), 8.14 (dd, $J$ = 2.4, 2.4 Hz, 1H), 7.96 (s, 2H), 6.02 (d, $J$ = 9.6 Hz, 1H), 3.16 (s, 3H), 3.04 (s, 3H), 1.75 (s, 2H), 1.58 (s, 4H), 1.35 - 1.29 (m, 1H), 1.23 (s, 1H), 1.00 (s, 3H); LC-MS: [M+H]$^+$ = 501.95.

Example 12. Synthesis of (*R*)-4-((2-(((4-chloropyridin-2-yl)(1-methylcyclopentyl)methyl)amino)-3,4-dioxocyclobut-1-en-1-yl)amino)-3-hydroxy-*N,N*-dimethylpicolinamide (compound 012)

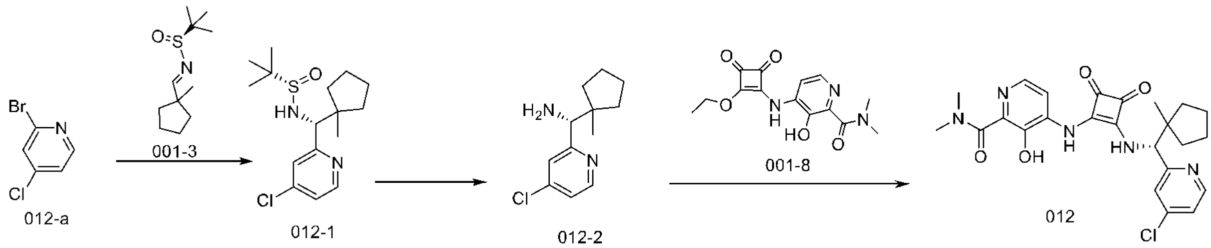


The synthesis of compound 012 was carried out with reference to the synthesis method for compound 001 in Example 1, except that the starting material 001-e was replaced with 012-a, to obtain compound 012 (16.1 mg, chiral purity: 99.6% (HPLC method G)).

$^1$H NMR (400 MHz, DMSO-$d_6$): δ 9.40 (s, 1H), 8.56 (d, $J$ = 5.2 Hz, 1H), 7.92 (d, $J$ = 5.2 Hz, 1H), 7.81 (s, 1H), 7.59 (d, $J$ = 1.6 Hz, 1H), 7.48 (dd, $J$ = 5.2, 2.0 Hz, 1H), 5.33 (s, 1H), 3.07 (s, 3H), 3.00 (s, 3H), 1.81 - 1.68 (m, 2H), 1.58 (s, 4H), 1.37 - 1.27 (m, 1H), 1.16 - 1.06 (m, 1H), 0.91 (s, 3H); LC-MS: [M+H]$^+$ = 483.90.

Example 13. Synthesis of (*R*)-4-((2-(((5-chloropyridin-2-yl)(1-methylcyclopentyl)methyl)amino)-3,4-dioxocyclobut-1-en-1-yl)amino)-3-hydroxy-*N,N*-dimethylpicolinamide (compound 013)

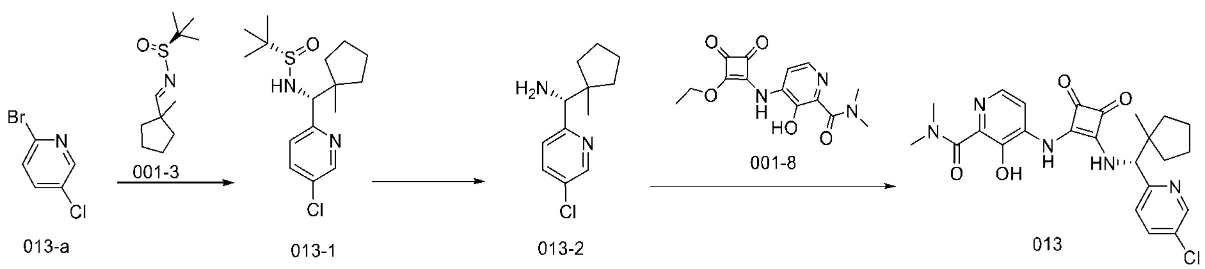


The synthesis of compound 013 was carried out with reference to the synthesis method for compound 001 in Example 1, except that the starting material 001-e was replaced with 013-a, to obtain compound 013 (29.0 mg, chiral purity: 99.9% (HPLC method H)).

$^1$H NMR (400 MHz, DMSO-$d_6$): δ 11.69 (s, 1H), 10.01 (s, 1H), 9.33 (d, $J$ = 9.8 Hz, 1H), 8.67 (d, $J$ = 1.8 Hz, 1H), 8.04 - 7.88 (m, 3H), 7.45 (d, $J$ = 8.4 Hz, 1H), 5.34 (d, $J$ = 9.8 Hz, 1H), 3.11 (d, $J$ = 56.0 Hz, 6H), 1.71 (d, $J$ = 7.2 Hz, 2H), 1.59 (s, 4H), 1.29 (d, $J$ = 12.0 Hz, 1H), 1.14 - 1.07 (m, 1H), 0.91 (s, 3H); LC-MS: [M+H]$^+$ = 483.95.

Example 14. Synthesis of (*R*)-4-((2-(((6-fluoro-3-methylpyridin-2-yl)(1-methylcyclopentyl)methyl)amino)-3,4-dioxocyclobut-1-en-1-yl)amino)-3-hydroxy-*N,N*-dimethylpicolinamide (compound 014)

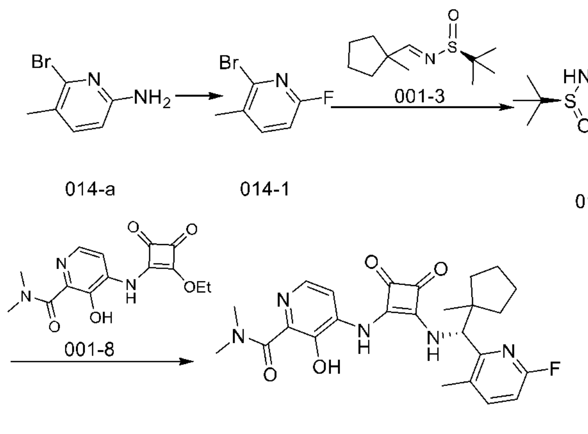


014-a 014-1 014-2 014-3

014

14.1 Synthesis of compound 014-1

014-a (0.5 g, 2.67 mmol) was weighed into a reaction flask. At 0°C, pyridine hydrofluoride solution (4 mL) was added, and then sodium nitrite (0.37 g, 5.35 mmol) was added. The reaction mixture was stirred at room temperature for 2 hours. To the reaction mixture was added saturated aqueous sodium bicarbonate solution (10 mL) to adjust the pH to 7-8, and the mixture was extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 014-1 (0.4 g, yield: 79%). LC-MS: $[M+H]^+$ = 189.95.

14.2 Synthesis of compound 014

The synthesis of compound 014 was carried out with reference to the synthesis method for compound 001 in Example 1, except that the starting material 001-e was replaced with 014-1, to obtain compound 014 (47 mg, yield: 22%, chiral purity: 97.8% (HPLC method I)).

$^1H$ NMR (400 MHz, DMSO-$d_6$): δ 11.70 (s, 1H), 10.06 (s, 1H), 9.19 (s, 1H), 7.99 (s, 2H), 7.84 (t, *J* = 12.0 Hz, 1H), 7.07-7.04 (m, 1H), 5.70 (d, *J* = 12.0 Hz, 1H), 3.18 (s, 3H), 3.05 (s, 3H), 2.39 (s, 3H), 1.74 - 1.53 (m, 6H), 1.38 - 1.31 (m, 1H), 1.26-1.22 (m, 1H), 1.00 (s, 3H); LC-MS: $[M+H]^+$ = 481.95.

Example 15. Synthesis of (*R*)-4-((2-(((3-(difluoromethyl)pyridin-2-yl)(1-methylcyclopentyl)methyl)amino)-3,4-dioxocyclobut-1-en-1-yl)amino)-3-hydroxy-*N,N*-dimethylpicolinamide (compound 015)

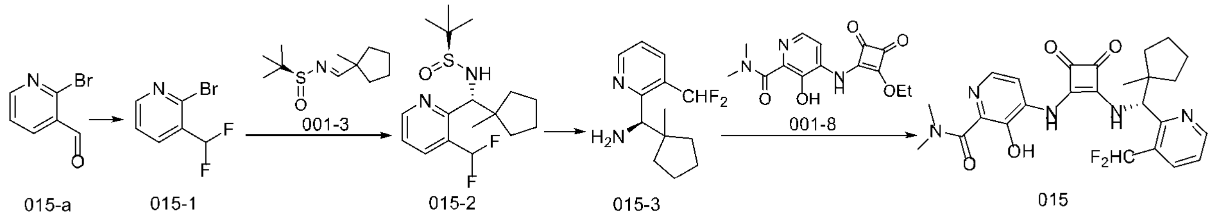


015-a 015-1 015-2 015-3 015

15.1 Synthesis of compound 015-1

015-a (500 mg, 2.69 mmol) was weighed into a reaction flask and dissolved in dichloromethane (10 mL). At 0°C, diethylaminosulfur trifluoride (1.30 g, 8.06 mmol) was added, and the reaction was carried out at room temperature for 4 hours. Water (5 mL) was added to the reaction mixture for dilution, and the mixture was stirred until homogeneous. The pH

was adjusted to 8-9 with saturated aqueous sodium bicarbonate solution, and the mixture was extracted with dichloromethane (15 mL × 3). The organic phase was dried over anhydrous sodium sulfate, filtered, and the filtrate was concentrated under reduced pressure. The residue was purified by silica gel column chromatography (petroleum ether/ethyl acetate = 9/1) to obtain compound 015-1 (500 mg, yield: 89%). LC-MS: $[M+H]^+$ = 209.90.

15.2 Synthesis of compound 015

The synthesis of compound 015 was carried out with reference to the synthesis method for compound 001 in Example 1, except that the starting material 001-e was replaced with 015-1, to obtain compound 015 (15.2 mg, chiral purity: 98.3% (HPLC method H)).

$^1H$ NMR (400 MHz, DMSO-$d_6$): δ 11.63 (s, 1H), 9.99 (s, 1H), 9.29 (s, 1H), 8.81 (d, *J* = 4.4 Hz, 1H), 8.05 (d, *J* = 7.8 Hz, 1H), 7.96 (s, 2H), 7.54 (dd, *J* = 7.8, 4.6 Hz, 1H), 7.37 (d, *J* = 54.4 Hz, 1H), 5.69 (d, *J* = 9.8 Hz, 1H), 3.18 (d, *J* = 22.4 Hz, 3H), 3.02 (s, 3H), 1.76 (dd, *J* = 13.8, 6.8 Hz, 2H), 1.64 - 1.46 (m, 4H), 1.40 - 1.29 (m, 1H), 1.26 - 1.14 (m, 1H), 0.95 (s, 3H); LC-MS: $[M+H]^+$ = 499.95.

Example 16. Synthesis of (*R*)-4-((2-(((3-(fluoromethyl)pyridin-2-yl)(1-methylcyclopentyl)methyl)amino)-3,4-dioxocyclobut-1-en-1-yl)amino)-3-hydroxy-*N,N*-dimethylpicolinamide (compound 016)

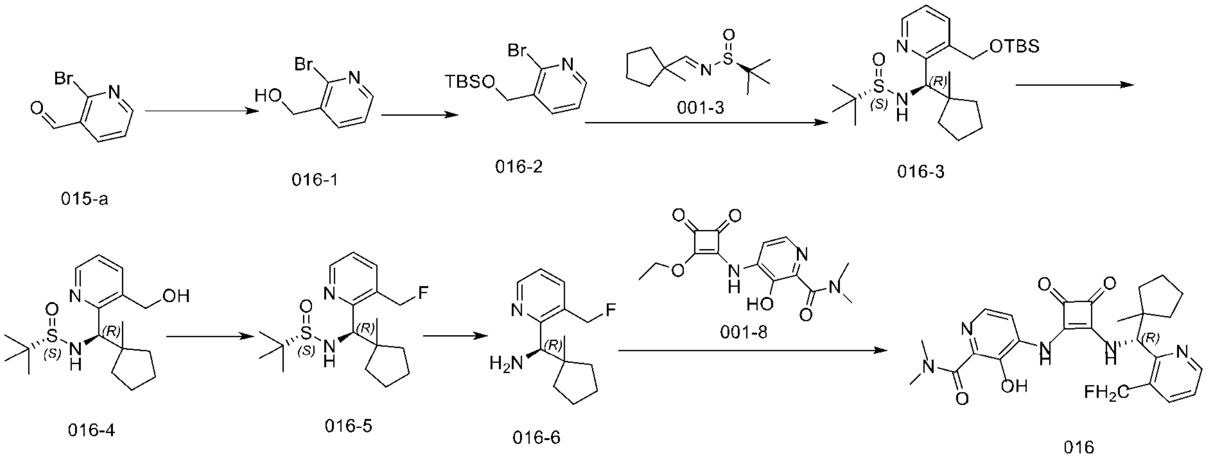

015-a 016-1 016-2 001-3 016-3 016-4 016-5 016-6 001-8 016

16.1 Synthesis of compound 016-1

015-a (10.0 g, 53.8 mmol) was weighed and dissolved in methanol (100 mL). At 0°C, sodium borohydride (2.4 g, 64.5 mmol) was added, and the reaction was carried out at this temperature for 1 hour. The reaction mixture was quenched with water (80 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 016-1 (9.6 g, yield: 95.0%).

16.2 Synthesis of compound 016-2

016-1 (9.6 g, 1.63 mmol) was weighed and dissolved in N,N-dimethylformamide (50 mL). Imidazole (6.9 g, 102.1 mmol) and tert-butyldimethylchlorosilane (9.2 g, 61.2 mmol) were added sequentially. The reaction mixture was reacted at 25°C for 4 hours, then quenched with water (100 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 016-2 (12.1 g, yield: 78.3%).

16.3 Synthesis of compound 016-3

The synthesis of compound 016-3 was carried out with reference to the synthesis method for compound 001-9 in Example 1, except that the starting material 001-e was replaced with 016-2, to obtain compound 016-3 (2.0 g, yield: 14.8%).

16.4 Synthesis of compound 016-4

016-3 (300 mg, 0.68 mmol) was weighed into a reaction flask. Tetrahydrofuran (5 mL) and tetrabutylammonium fluoride (1.38 mL, 1.38 mmol, 1 M) were added. The resulting reaction mixture was reacted at 25°C for 2 hours. The reaction mixture was quenched with water (20 mL) and extracted with ethyl acetate (30 mL × 3). The organic phase was dried over anhydrous sodium sulfate, filtered, and the filtrate was concentrated under reduced pressure. The residue was purified by silica gel column chromatography to obtain compound 016-4 (90 mg, yield: 40.7%), LC-MS: $[M+H]^+$ = 325.1.

16.5 Compound 016-5

016-4 (90 mg, 0.277 mmol) was dissolved in dichloromethane (1 mL). Diethylaminosulfur trifluoride (89 mg, 0.55 mmol) was added, and the reaction mixture was reacted at room temperature for 1 hour. The reaction mixture was quenched with water (15 mL) and extracted with ethyl acetate (30 mL × 3). The organic phase was dried over anhydrous sodium sulfate, filtered, and the filtrate was concentrated under reduced pressure. The residue was purified by silica gel column chromatography to obtain compound 016-5 (30 mg, yield: 44.1%). LC-MS: $[M+H]^+$ = 327.1.

16.6 Synthesis of compound 016

The synthesis of compound 016 was carried out with reference to the synthesis method for compound 001 in Example 1, except that the starting material 001-9 was replaced with 016-5, to obtain compound 016 (3.5 mg, yield: 8.1%, chiral purity: 78.1% (HPLC method H)).

$^1$H NMR (400 MHz, DMSO-$d_6$): δ 11.62 (s, 1H), 10.00 (s, 1H), 9.30 (s, 1H), 8.569 (d, *J* = 4.0 Hz, 1H), 7.95-7.88 (m,3H), 7.45-7.42 (m,1H), 5.71-7.47 (m,3H), 3.15 (s, 3H), 3.02 (s, 3H), 1.76-1.71 (m, 2H), 1.59-1.54 (m, 4H), 1.35-1.32 (m, 1H), 1.21-1.16 (m, 1H), 0.96 (s, 3H); LC-MS: $[M+H]^+$ = 482.1.

Example 17. Synthesis of (*R*)-3-hydroxy-*N*,*N*-dimethyl-4-((2-(((1-methylcyclopentyl)(pyrimidin-2-yl)methyl)amino)-3,4-dioxocyclobuten-1-yl)amino)picolinamide (compound 017A or 017B) and (*S*)-3-hydroxy-*N*,*N*-dimethyl-4-((2-(((1-methylcyclopentyl)(pyrimidin-2-yl)methyl)amino)-3,4-dioxocyclobuten-1-yl)amino)picolinamide (compound 017B or 017A)

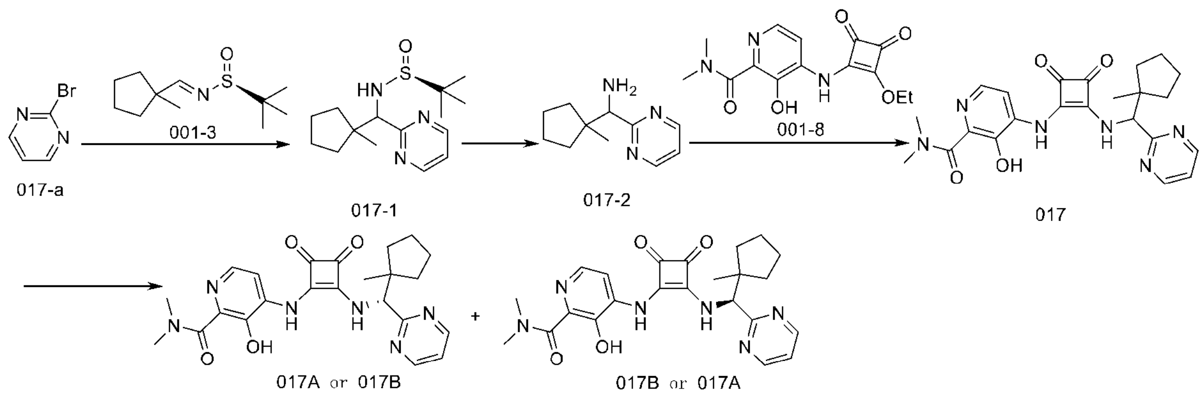


17.1 Synthesis of compound 017-1

017-a (477 mg, 3.01 mmol) was dissolved in toluene (6 mL). The system was purged with nitrogen three times. At -78°C, a solution of n-butyllithium in hexane (1.28 mL, 3.20 mmol, 2.5 M) was added. After stirring for 30 minutes, 001-3 (323 mg, 1.50 mmol) was added. The reaction mixture was stirred at -78°C for another 30 minutes, then warmed to room temperature and stirred for 2 hours. The reaction mixture was quenched with saturated aqueous ammonium chloride solution (10 mL), diluted with water (10 mL), and extracted with ethyl acetate (10 mL × 3). The organic phases were combined, washed with saturated aqueous sodium chloride solution (10 mL), dried over anhydrous sodium sulfate, filtered, and the filtrate was concentrated under reduced pressure. The residue was purified by silica gel column chromatography (dichloromethane/methanol = 10/1) to obtain compound 017-1 (144 mg, crude product).

17.2 Synthesis of compound 017-2 hydrochloride

017-1 (110 mg, crude product) was dissolved in ethanol (1.2 mL). A solution of hydrogen chloride in ethanol (0.4 mL, 4 M) was added, and the reaction mixture was stirred at room temperature for 1 hour. The reaction mixture was directly concentrated under reduced pressure to obtain compound 017-2 hydrochloride (100 mg, crude product), which was used directly in the next step.

17.3 Synthesis of compounds 017-A and 017-B

017-2 hydrochloride (100 mg, crude product), ethanol (1.6 mL), N, N-diisopropylethylamine (260 μL, 1.50 mmol), and 001-8 (114 mg, 0.37 mmol) were sequentially added to a reaction flask and stirred at room temperature for 2 hours. The reaction mixture was concentrated under reduced pressure. The residue was purified by preparative HPLC to obtain compound 017 (61.4 mg), which was then subjected to chiral resolution to obtain compound 017-A (24.6 mg) and 017-B (22.6 mg).

Characterization data for 017-A are as follows:

LCMS: $[M+H]^+$ = 451.00;
Chiral purity: 99.3% (HPLC method H); $T_R$ = 10.149 min.

$^1H$ NMR (400 MHz, DMSO-$d_6$): δ 9.44 (d, *J* = 10.4 Hz, 1H), 8.83 (d, *J* = 4.8 Hz, 2H), 7.93 (d, *J* = 5.2 Hz, 1H), 7.82 (d, *J* = 4.0 Hz, 1H), 7.46 (t, *J* = 4.8 Hz, 1H), 5.47 (d, *J* = 10.0 Hz, 1H), 3.07 (s, 3H), 3.00 (s, 3H), 1.86-1.75 (m, 2H), 1.63-1.51 (m, 4H), 1.32-1.28 (m, 1H), 1.15-1.09 (m, 1H), 0.93 (s, 3H).

Characterization data for 017-B are as follows:

LCMS: $[M+H]^+$ = 451.00;
Chiral purity: 96.5% (HPLC method H); $T_R$ = 12.511 min.

$^1H$ NMR (400 MHz, DMSO-$d_6$): δ 9.44 (d, *J* = 10.0 Hz, 1H), 8.82 (d, *J* = 4.8 Hz, 2H), 7.92 (d, *J* = 5.2 Hz, 1H), 7.79 (d, *J* = 4.0 Hz, 1H), 7.46 (t, *J* = 4.8 Hz, 1H), 5.47 (d, *J* = 10.0 Hz, 1H), 3.05 (s, 3H), 2.99 (s, 3H), 1.86-1.77 (m, 2H), 1.61-1.51 (m, 4H), 1.32-1.28 (m, 1H), 1.14-1.09 (m, 1H), 0.93 (s, 3H).

Example 18. Synthesis of (*R*)-3-hydroxy-*N*,*N*-dimethyl-4-((2-(((1-methylcyclopentyl)(3-methylpyridin-2-yl)methyl)amino)-3,4-dioxocyclobut-1-en-1-yl)amino)picolinamide (compound 018)

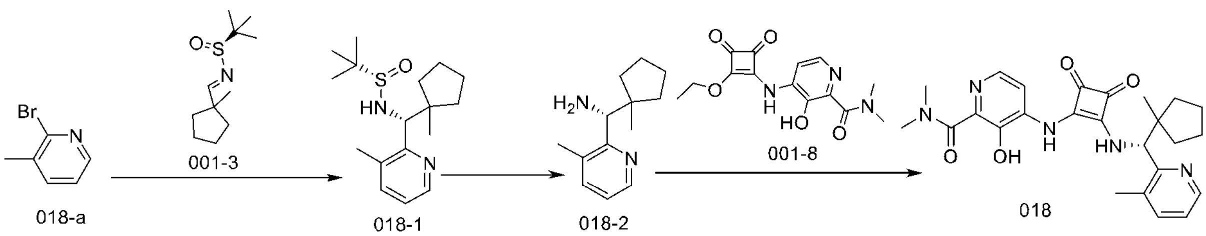


The synthesis of compound 018 was carried out with reference to the synthesis method for compound 001 in Example 1, except that the starting material 001-e was replaced with 018-a, to obtain compound 018 (65.1 mg, chiral purity: 64.1% (HPLC method E)).

$^1H$ NMR (400 MHz, DMSO-$d_6$): δ 9.36 (s, 1H), 8.48 (d, *J* =4.0 Hz, 1H), 7.98 (d, *J* = 4.0 Hz, 2H), 7.64 (d, *J* = 8.0 Hz, 1H), 7.26 (dd, *J* =4.0, 4.0Hz, 1H), 5.74 (d, *J* =12.0 Hz, 1H), 3.17 (s, 3H), 3.04 (s, 3H), 2.40 (s, 3H), 1.81 - 1.68 (m, 2H), 1.64 - 1.51 (m, 4H), 1.37 - 1.29 (m, 1H), 1.23 (s, 1H), 1.00 (s, 3H); LC-MS: $[M+H]^+$ = 464.05.

Example 19. Synthesis of (*R*)-4-((2-(((3-chloropyridin-2-yl)(1-methylcyclopentyl)methyl)amino)-3,4-dioxocyclobut-1-en-1-yl)amino)-3-hydroxy-*N*,*N*-dimethylpicolinamide (compound 019)

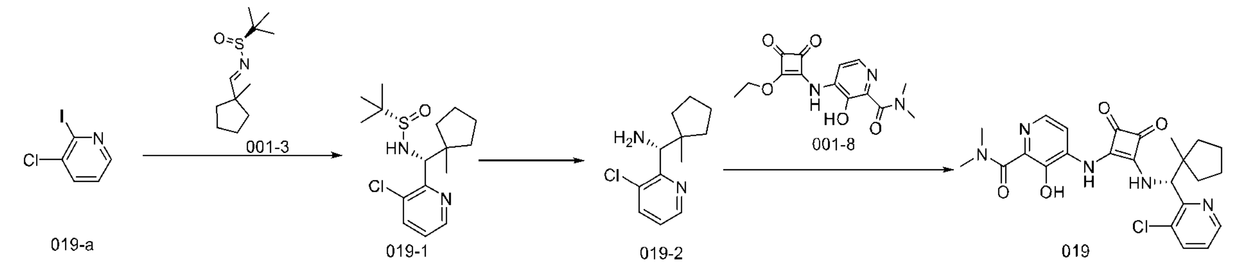


19.1 Synthesis of compound 019-1

019-a (200 mg, 0.84 mmol) was added to tetrahydrofuran (4 mL). At -78°C, a solution of*n*-butyllithium in n-hexane (680 μL, 1.10 mmol, 1.6 M) was added dropwise. After the dropwise addition was completed, the mixture was stirred at -78°C for 10 minutes. Then, a solution of 001-3 (180 mg, 0.84 mmol) in tetrahydrofuran (0.5 mL) was added dropwise at -78°C. After the addition was completed, the reaction mixture was stirred at -78°C for 0.5 hours. Saturated aqueous ammonium chloride solution (10 mL) was added to the reaction mixture to quench the reaction. Water (10 mL) was added for dilution, and the mixture was extracted with ethyl acetate (10 mL × 3). The organic phase was washed once with saturated brine (10 mL), then dried over anhydrous sodium sulfate, filtered, and the filtrate was concentrated under reduced pressure. The residue was purified by silica gel column chromatography (dichloromethane/methanol = 50/1 to 20/1) to obtain compound 019-1 (180 mg, yield: 65.1%). LC-MS: $[M+H]^+$ = 329.00.

19.2 Synthesis of compound 019

The synthesis of compound 019 was carried out with reference to the synthesis method for compound 001 in Example 1, except that the starting material 001-9 was replaced with 019-1, to obtain compound 019 (75.2 mg, yield: 65.1%, chiral purity: 100% (HPLC method K)).

$^1$H NMR (400 MHz, DMSO-*d*$_6$): δ 11.65 (s, 1H), 10.05 (s, 1H), 9.35 (s, 1H), 8.64 (dd, *J* = 4.4, 1.6 Hz, 1H), 7.98 (dd, *J* = 8.0, 1.2 Hz, 2H), 7.43 (dd, *J* = 8.0, 4.4 Hz, 1H), 6.10 - 5.99 (m, 1H), 3.13 (s, 3H), 3.04 (s, 3H), 1.84 - 1.68 (m, 2H), 1.58 (dd, *J* = 12.8, 8.0 Hz, 4H), 1.36 - 1.28 (m, 1H), 1.19 -1.13 (m, 1H), 1.01 (s, 3H); LC-MS: $[M+H]^+$ = 484.00.

Example 20. Synthesis of (*R*)-4-((2-(((4-fluoropyridin-2-yl)(1-methylcyclopentyl)methyl)amino)-3,4-dioxocyclobut-1-en-1-yl)amino)-3-hydroxy-*N,N*-dimethylpicolinamide (compound 020)

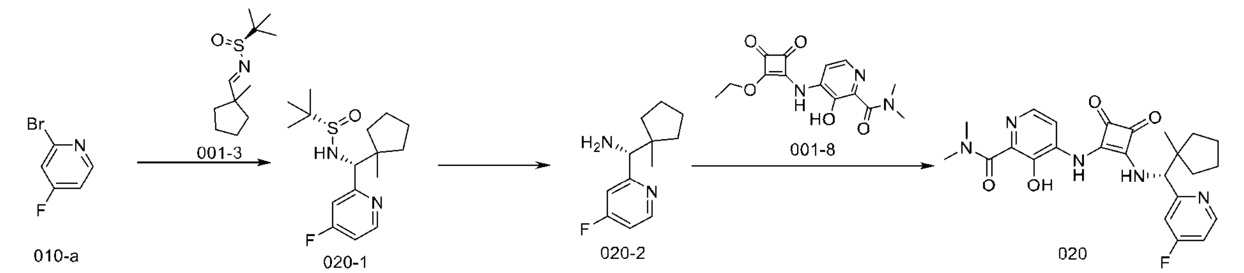


The synthesis of compound 020 was carried out with reference to the synthesis method for compound 001 in Example 1, except that the starting material 001-e was replaced with 010-a, to obtain compound 020 (45.4 mg, yield: 38.8%, chiral purity: 100% (HPLC method C)).

$^1$H NMR (400 MHz, DMSO-*d*$_6$): δ 11.80 (s, 1H), 9.77 (s, 1H), 8.95 (s, 1H), 8.60 (d, *J* = 10.4 Hz, 1H), 8.54 (dd, *J* = 8.0, 5.6 Hz, 1H), 7.98 - 7.95 (m, 2H), 7.36 (dd, *J* = 10.4, 5.6 Hz, 1H), 5.67 - 5.39 (m, 1H), 3.10 (s, 3H), 3.04 (s, 3H), 1.71 - 1.57 (m, 6H), 1.49 - 1.40 (m, 1H), 1.19 (d, *J* = 6.8 Hz, 1H), 1.00 (s, 3H); LC-MS: $[M+H]^+$ = 467.95.

Example 21. Synthesis of (*R*)-4-((2-(((3,4-difluoropyridin-2-yl)(1-methylcyclopentyl)methyl)amino)-3,4-dioxocyclobut-1-en-1-yl)amino)-3-hydroxy-*N,N*-dimethylpicolinamide (compound 021)

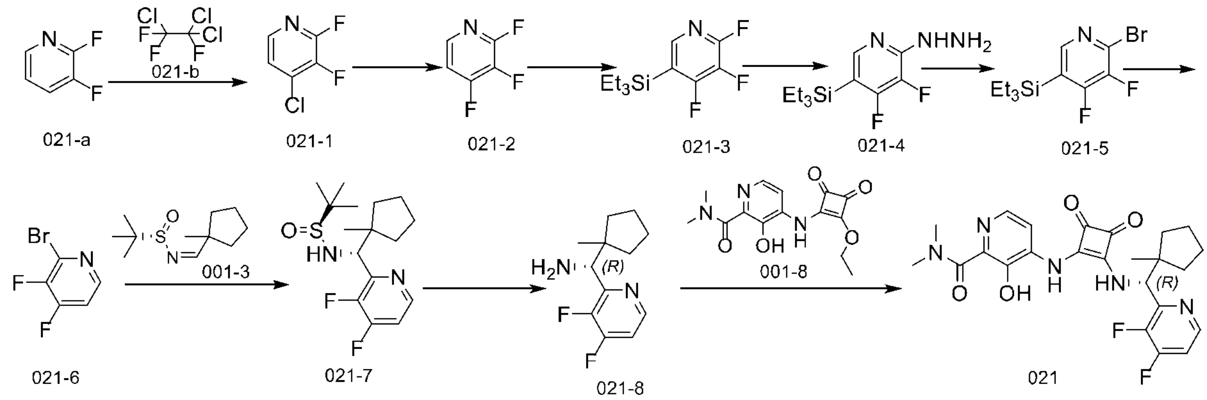


21.1 Synthesis of compound 021-1

021-a (5.0 g, 43.5 mmol) was added to anhydrous tetrahydrofuran (50 mL) and *n*-hexane (30 mL). At -78°C, a solution of *n*-butyllithium in *n*-hexane (32 mL, 52.2 mmol, 1.6 M) was slowly added. After the addition was completed, the reaction mixture was stirred at -78°C for 0.5 hours. Then, 021-b (9.8 g, 52.2 mmol) was slowly added dropwise to the reaction mixture. After the dropwise addition was completed, the reaction was stirred at -78°C for another 0.5 hours. To the reaction mixture, a saturated aqueous sodium thiosulfate solution (20 mL) was added to quench the reaction. The organic phase and the aqueous phase were then separated. The organic phase was dried over anhydrous sodium sulfate, filtered, and the filtrate was distilled at atmospheric pressure (80°C) to remove the solvent. After the distillation was completed, compound 021-1 (6.0 g, crude product) was obtained. LC-MS: $[M+H]^+$ = 150.05.

21.2 Synthesis of compound 021-2

At room temperature, 021-1 (5.0 g, crude product), tetrabutylammonium chloride (1.9 g, 6.7 mmol), and potassium fluoride (3.9 g, 67.2 mmol) were added to dimethyl sulfoxide (50 mL). The reaction mixture was stirred at 150°C for 2 hours. After the reaction mixture was cooled to room temperature, water (300 mL) was added for dilution. The mixture was extracted with *n*-hexane (100 mL × 3). The organic phase was dried over anhydrous sodium sulfate, filtered, and the filtrate was distilled at atmospheric pressure (70°C) to remove the solvent. After the distillation was completed, compound 021-2 (1.8 g, crude product) was obtained. LC-MS: $[M+H]^+$ = 134.20.

21.3 Synthesis of compound 021-3

At room temperature, 021-2 (1.8 g, crude product) and diisopropylamine (1.4 g, 13.5 mmol) were added to anhydrous tetrahydrofuran (90 mL). At -78°C, a solution of *n*-butyllithium in n-hexane (8.4 mL, 13.5 mmol) was slowly added. After the addition was completed, the reaction was stirred at -78°C for 0.5 hours. Then, triethylchlorosilane (2.0 g, 13.5 mmol) was slowly added dropwise to the reaction mixture. After the addition was completed, the reaction was stirred at -78°C for another 0.5 hours. Saturated aqueous ammonium chloride solution (50 mL) was added to the reaction mixture, and the mixture was extracted with ethyl acetate (50 mL × 3). The organic phase was washed with saturated brine (20 mL), dried over anhydrous sodium sulfate, filtered, and the filtrate was concentrated under reduced pressure. The residue was purified by silica gel column chromatography (petroleum ether/ethyl acetate = 1/0 to 20/1) to obtain compound 021-3 (2.0 g, yield: 60%). LC-MS: $[M+H]^+$ = 248.05.

21.4 Synthesis of compound 021-4

At room temperature, 021-3 (2.0 g, 8.1 mmol) and 98% hydrazine hydrate (0.81 g, 16.2 mmol) were added to tetrahydrofuran (20 mL). The reaction was stirred at 50°C for 1 hour. Water (30 mL) was added to the reaction mixture for dilution, and the mixture was extracted with ethyl acetate (30 mL × 3). The organic phase was washed with saturated brine (20 mL), then dried over anhydrous sodium sulfate, filtered, and the filtrate was concentrated under reduced pressure to obtain a crude product. The crude product was purified by silica gel column chromatography (petroleum ether/ethyl acetate = 1/1 to 1/3) to obtain compound 021-4 (1.1 g, yield: 51.9%), LC-MS: $[M+H]^+$ = 260.05.

21.5 Synthesis of compound 021-5

At room temperature, 021-4 (500 mg, 1.9 mmol) and bromine (608 mg, 3.8 mmol) were added to chloroform (5

mL), and the reaction mixture was stirred at 60°C for 1 hour. The reaction mixture was quenched with saturated aqueous sodium bicarbonate solution (10 mL), diluted with water (10 mL), and extracted with dichloromethane (20 mL × 3). The organic phase was washed with saturated brine (20 mL), dried over anhydrous sodium sulfate, filtered, and the filtrate was concentrated under reduced pressure (concentration temperature below 30°C) to obtain compound 021-5 (440 mg, yield: 73.7%). LC-MS: $[M+H]^+$ = 307.90.

21.6 Synthesis of compound 021-6

At room temperature, 021-5 (440 mg, 1.4 mmol) and 85% tetrabutylammonium fluoride (1.3 g, 4.2 mol) were added to tetrahydrofuran (10 mL) and stirred at room temperature for 1 hour. Water (10 mL) was added to the reaction mixture for dilution, and the mixture was extracted with ethyl acetate (20 mL × 3). The organic phase was washed with saturated brine (10 mL), dried over anhydrous sodium sulfate, and filtered. The filtrate was concentrated under reduced pressure (concentration temperature below 30°C) to obtain a crude product. The crude product was purified by silica gel column chromatography (petroleum ether/ethyl acetate = 5/1 to 3/1) to obtain compound 021-6 (190 mg, yield: 71.4%). LC-MS: $[M+H]^+$ = 193.90.

21.7 Synthesis of compound 021

The synthesis of compound 021 was carried out with reference to the synthesis method for compound 001 in Example 1, except that the starting material 001-e was replaced with 021-6, to obtain compound 021 (36.1 mg, yield: 37.1%, chiral purity: 100% (HPLC method J)).

$^1H$ NMR (400 MHz, DMSO-$d_6$): δ 9.36 (s, 1H), 8.56 - 8.41 (m, 1H), 7.67 - 7.44 (m, 2H), 7.06 (d, *J* = 4.8 Hz, 1H), 5.78 (s, 1H), 2.89 (s, 3H), 2.81 (s, 3H), 1.79 (d, *J* = 12.4 Hz, 1H), 1.69 (s, 1H), 1.59 (s, 4H), 1.30 - 1.27 (m, 1H), 1.18 (s, 1H), 0.97 (s, 3H); LC-MS: $[M+H]^+$ = 486.00.

Example 22. Synthesis of (*R*)-4-((2-(((4,6-difluoropyridin-2-yl)(1-methylcyclopentyl)methyl)amino)-3,4-dioxocyclobut-1-en-1-yl)amino)-3-hydroxy-*N*,*N*-dimethylpicolinamide (compound 022)

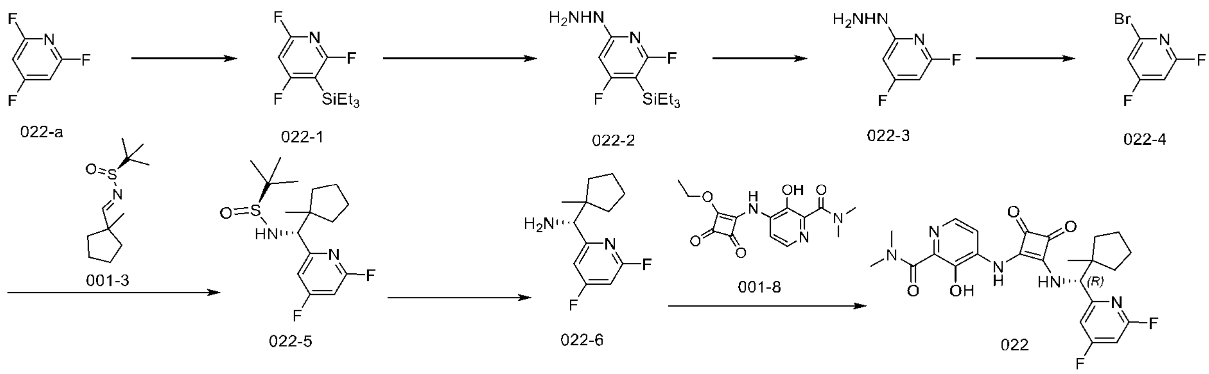

022-a 022-1 022-2 022-3 022-4 022-5 022-6 022

22.1 Synthesis of compound 022-1

022-a (1.0 g, 7.5 mmol) was dissolved in anhydrous tetrahydrofuran (20 mL). The system was purged with nitrogen three times. At -78°C, a solution of *n*-butyllithium in *n*-hexane (5.6 mL, 9.0 mmol, 1.6 M) was added dropwise slowly. After the dropwise addition was completed, the mixture was stirred at -78°C for half an hour. Triethylchlorosilane (1.3 g, 8.3 mmol) was added, and after the addition was completed, the reaction mixture was stirred at -78°C for another half an hour. The reaction mixture was quenched with saturated aqueous ammonium chloride solution (20 mL), diluted with water (20 mL), and extracted with ethyl acetate (20 mL × 3). The organic phase was washed with saturated brine (20 mL), dried over anhydrous sodium sulfate, filtered, and the filtrate was concentrated to obtain compound 022-1 (1.85 g, crude product). LC-MS: $[M+H]^+$ = 248.05.

22.2 Synthesis of compound 022-2

At room temperature, 022-1 (1.9 g, 7.5 mmol) and 85% hydrazine hydrate (882 mg, 15.0 mmol) were added to tetrahydrofuran (30 mL). The reaction mixture was stirred at 50°C for 1 hour. The reaction mixture was directly

concentrated to dryness under reduced pressure to obtain a crude product. The crude product was purified by silica gel column chromatography (petroleum ether/ethyl acetate = 10/1 to 5/1) to obtain compound 022-2 (1.3 g, yield: 66.7%). LC-MS: $[M+H]^+$ = 260.05.

22.3 Synthesis of compound 022-3

At room temperature, 022-2 (1.3 g, 5.6 mmol) and 85% tetrabutylammonium fluoride (1.9 g, 6.0 mol) were added to tetrahydrofuran (10 mL) and stirred at room temperature for 2 hours. Water (20 mL) was added to the reaction mixture for dilution, and the mixture was extracted with ethyl acetate (20 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 022-3 (480 mg, yield: 58.9%). LC-MS: $[M+H]^+$ = 146.10.

22.4 Synthesis of compound 022-4

At room temperature, 022-3 (200 mg, 1.38 mmol) and bromine (440 mg, 2.76 mmol) were added to chloroform (3 mL), and the reaction mixture was stirred at 65°C for 2 hours. The reaction mixture was quenched with saturated aqueous sodium bicarbonate solution (10 mL), diluted with water (10 mL), and extracted with dichloromethane (20 mL × 3). The organic phase was washed with saturated brine (20 mL), dried over anhydrous sodium sulfate, filtered, and the filtrate was concentrated under reduced pressure (concentration temperature below 30°C) to obtain compound 022-4 (200 mg, crude product). LC-MS: $[M+H]^+$ = 193.90.

22.5 Synthesis of compound 022

The synthesis of compound 022 was carried out with reference to the synthesis method for compound 001 in Example 1, except that the starting material 001-e was replaced with 022-4, to obtain compound 022 (31.8 mg, yield: 36.4%, chiral purity: 100% (HPLC method E)).

$^1$H NMR (400 MHz, DMSO-$d_6$): δ 11.70 (s, 1H), 10.03 (s, 1H), 9.24 (s, 1H), 8.30 - 7.55 (m, 2H), 7.45 (d, *J* = 7.2 Hz, 1H), 7.24 (d, *J* = 8.8 Hz, 1H), 5.32 (d, *J* = 10.0 Hz, 1H), 3.17 (s, 3H), 3.04 (s, 3H), 1.78 - 1.67 (m, 2H), 1.67 - 1.55 (m, 4H), 1.35 - 1.27 (m, 1H), 1.19 - 1.11 (m, 1H), 0.94 (s, 3H); LC-MS: $[M+H]^+$ = 486.00.

Example 23. Synthesis of (*R*)-4-((2-(((3,6-difluoropyridin-2-yl)(1-methylcyclopentyl)methyl)amino)-3,4-dioxocyclobut-1-en-1-yl)amino)-3-hydroxy-*N,N*-dimethylpicolinamide (compound 023)

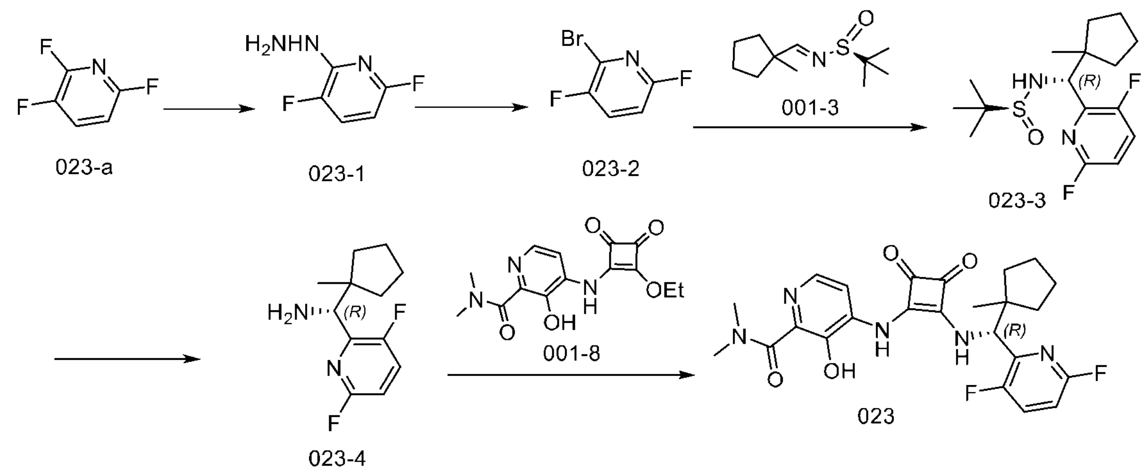


23.1 Synthesis of compound 023-1

At room temperature, 023-a (3.0 g, 22.44 mmol) and ethanol (60 mL) were added to a reaction flask. Hydrazine hydrate (98%, 2.2 g, 44.88 mmol) was added, and the reaction mixture was heated to 50°C and stirred for 1 hour. The reaction mixture was cooled to room temperature and concentrated under reduced pressure. The residue was diluted with water (50 mL), and the resulting mixture was extracted with ethyl acetate (30 mL × 3). The organic phases were combined,

washed with saturated brine (40 mL), dried over anhydrous sodium sulfate, filtered, and the filtrate was concentrated under reduced pressure. The residue was purified by silica gel column chromatography (ethyl acetate/petroleum ether = 1/6) to obtain compound 023-1 (1.8 g). LC-MS: $[M+H]^+$ = 146.10.

23.2 Synthesis of compound 023-2

At room temperature, 023-1 (500 mg, 3.45 mmol) and chloroform (5 mL) were added to a reaction flask. Bromine (1.1 g, 6.90 mmol) was added at room temperature, and the reaction was carried out at 60°C for 30 minutes. The reaction mixture was slowly added dropwise to a saturated sodium bicarbonate solution (15 mL) and extracted with n-hexane (10 mL × 3). The organic phases were combined, washed with saturated brine (15 mL), and dried over anhydrous sodium sulfate. The organic phase was directly purified by silica gel column chromatography (tetrahydrofuran/n-hexane = 1/30). The collected fractions were dried again over anhydrous sodium sulfate and concentrated under reduced pressure at low temperature to obtain compound 023-2 (100 mg). LC-MS: $[M+H]^+$ = 194.15.

23.3 Synthesis of compound 023

The synthesis of compound 023 was carried out with reference to the synthesis method for compound 001 in Example 1, except that the starting material 001-e was replaced with 023-2, to obtain compound 023 (76.0 mg, chiral purity: 90.7% (HPLC method H)).

$^1H$ NMR (400 MHz, DMSO-$d_6$): δ 11.68 (s, 1H), 10.06 (s, 1H), 9.28 (s, 1H), 8.07 - 7.95 (m, 3H), 7.28 (m, 1H), 5.73 (d, *J* = 9.6 Hz, 1H), 3.17 (s, 3H), 3.05 (s, 3H), 1.73 (dd, *J* = 12.0, 6.8 Hz, 1H), 1.62 (s, 5H), 1.33 (d, *J* = 12.4 Hz, 1H), 1.22 (d, *J* = 12.4 Hz, 1H), 1.00 (s, 3H); LC-MS: $[M+H]^+$ = 485.95.

Example 24. Synthesis of (*R*)-4-((2-(((5,6-difluoropyridin-2-yl)(1-methylcyclopentyl)methyl)amino)-3,4-dioxocyclobut-1-en-1-yl)amino)-3-hydroxy-*N,N*-dimethylpicolinamide (compound 024)

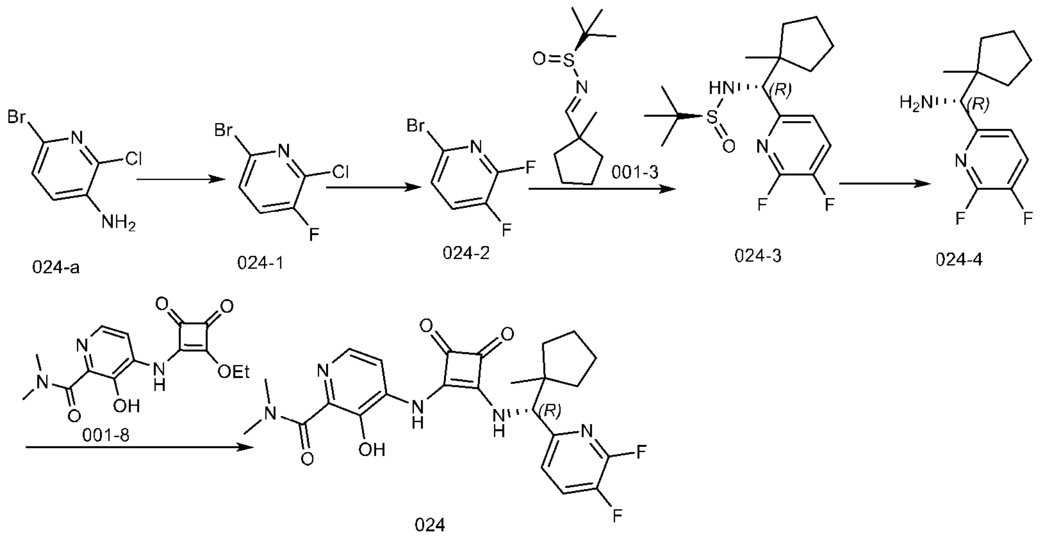


24.1 Synthesis of compound 024-1

At room temperature, 024-a (2.0 g, 9.7 mmol) was added to ethylene glycol dimethyl ether (20 mL). At -10°C, *tert*-butyl nitrite (1.2 g, 11.6 mmol) and boron trifluoride diethyl etherate (2.1 mg, 14.6 mmol) were slowly added. After the addition was completed, the reaction was stirred at -10°C for 0.5 hours. The reaction mixture was directly filtered, and the filter cake was rinsed with *n*-hexane (10 mL). After vacuum drying, the filter cake was transferred to a single-necked flask, heated and stirred at 85°C for 0.5 hours, and then stirred at 130°C for 1 hour. Water (20 mL) was added to the reaction system for dilution, and the mixture was extracted with ethyl acetate (20 mL × 3). The organic phase was washed with saturated brine (10 mL), dried over anhydrous sodium sulfate, filtered, and the filtrate was concentrated under reduced pressure. The residue was purified by silica gel column chromatography (petroleum ether/ethyl acetate = 20/1 to 5/1) to obtain compound 024-1 (1.0 g, yield: 49.5%).

24.2 Synthesis of compound 024-2

At room temperature, tetramethylammonium fluoride hydrate (1.1 g, 9.6 mmol) was added to *tert*-amyl alcohol (20 mL), and the reaction mixture was stirred at room temperature for 1 hour. The reaction mixture was concentrated to dryness under reduced pressure, and the system was replaced twice with *tert*-amyl alcohol (10 mL × 2) to obtain the *tert*-amyl alcohol complex of tetramethylammonium fluoride. The complex and 024-1 (1.0 g, 4.8 mmol) were added to dimethyl sulfoxide (10 mL) and stirred at 80°C for 1 hour. Water (30 mL) was added to the reaction mixture for dilution, and the mixture was extracted with *n*-hexane (20 mL × 3). The organic phase was washed with saturated brine (10 mL), dried over anhydrous sodium sulfate, and purified by silica gel column chromatography (*n*-hexane/tetrahydrofuran = 30/1 to 20/1) to obtain a solution of compound 024-2 in n-hexane and tetrahydrofuran. The solution was dried over anhydrous sodium sulfate and directly used in the next step. LC-MS: $[M+H]^+$ = 192.00.

24.3 Synthesis of compound 024

The synthesis of compound 024 was carried out with reference to the synthesis method for compound 001 in Example 1, except that the starting material 001-e was replaced with 024-2, to obtain compound 024 (21.4 mg, yield: 65.1%, chiral purity: 96.2% (HPLC method D)).

$^1$H NMR (400 MHz, DMSO-$d_6$): δ 11.73 (s, 1H), 10.01 (s, 1H), 9.20 (d, $J$ = 9.6 Hz, 1H), 8.08 - 7.96 (m, 2H), 7.44 (dd, $J$ = 8.4, 2.8 Hz, 1H), 5.34 (d, $J$ = 9.6 Hz, 1H), 3.20 (s, 3H), 3.02 (s, 3H), 1.82 - 1.51 (m, 6H), 1.39 - 1.30 (m, 1H), 1.22 - 1.11 (m, 1H), 0.95 (s, 3H); LC-MS: $[M+H]^+$ = 486.00.

Example 25. Synthesis of (*R*)-4-((2-(((3-chloro-6-fluoropyridin-2-yl)(1-methylcyclopentyl)methyl)amino)-3,4-dioxocyclobut-1-en-1-yl)amino)-3-hydroxy-*N,N*-dimethylpicolinamide (compound 025)

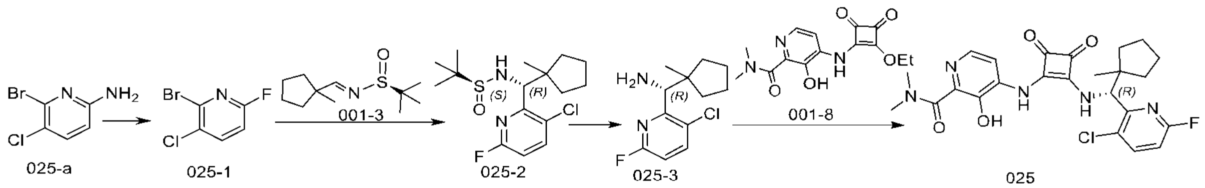


The synthesis of compound 025 was carried out with reference to the synthesis method for compound 014 in Example 14, except that the starting material 014-a was replaced with 025-a, to obtain compound 025 (13.2 mg, yield: 13.2%, chiral purity: 80.6% (HPLC method H)).

$^1$H NMR (400 MHz, DMSO-$d_6$): δ 11.69 (s, 1H), 10.03 (s, 1H), 9.20 (s, 1H), 8.16 (dd, $J$ = 8.8, 7.2 Hz, 1H), 8.05 - 7.87 (m, 2H), 7.26 (dd, $J$ = 8.8, 3.2 Hz, 1H), 6.04 - 5.91 (m, 1H), 3.15 (s, 3H), 3.03 (s, 3H), 1.82 - 1.67 (m, 2H), 1.66 - 1.51 (m, 4H), 1.34 - 1.21 (m, 2H), 1.00 (s, 3H); LC-MS: $[M+H]^+$ = 502.00.

Example 26. Synthesis of (*R*)-4-((2-(((3-fluoropyridin-2-yl)(1-methylcyclopentyl)methyl)amino)-3,4-dioxocyclobut-1-en-1-yl)amino)-3-hydroxy-N-isopropyl-N-methylpicolinamide (compound 026)

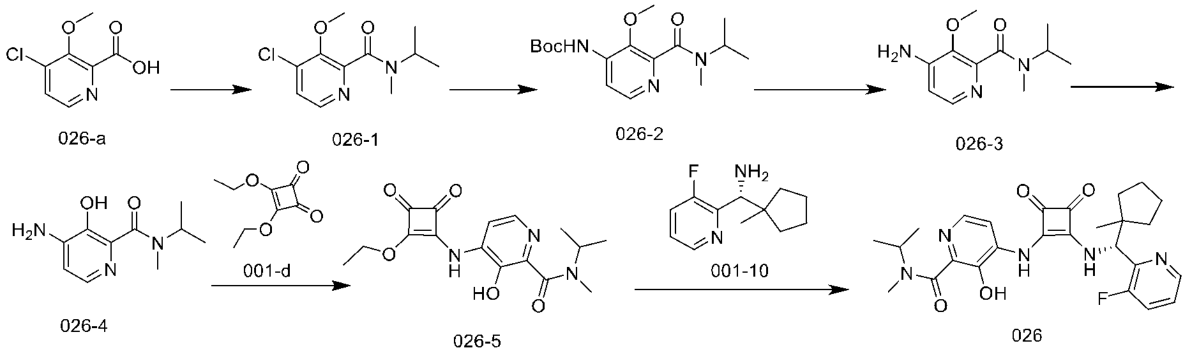

26.1 Synthesis of compound 026-1

026-a (200 mg, 1.07 mmol) was dissolved in dichloromethane (4 mL). At 0°C, *N,N*-dimethylformamide (0.1 mL) and oxalyl chloride (272 mg, 2.14 mmol) were sequentially added dropwise to the reaction mixture. After the dropwise addition was completed, the reaction was stirred at room temperature for 1 hour. The reaction mixture was concentrated under reduced pressure. Dichloromethane (4 mL) was added to the residue, followed by the sequential addition of *N*-isopropylmethylamine (94 mg, 1.28 mmol) and triethylamine (325 mg, 3.21 mmol). The reaction mixture was stirred at room temperature for 16 hours. The reaction mixture was poured into water (10 mL) to quench the reaction, then extracted with dichloromethane (20 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 026-1 (189 mg, yield: 73.00%).

26.2 Synthesis of compound 026-2

026-1 (189 mg, 0.78 mmol) was dissolved in 1,4-dioxane (2 mL). *tert*-Butyl carbamate (182 mg, 1.56 mmol), 2-dicyclohexylphosphino-2',4',6'-triisopropylbiphenyl (37 mg, 0.07 mmol), palladium acetate (17 mg, 0.07 mmol), and cesium carbonate (507 mg, 1.56 mmol) were added. The reaction was carried out at 95°C under a nitrogen atmosphere for 16 hours. The reaction mixture was poured into water (10 mL) for dilution, extracted with ethyl acetate (10 mL × 3), and the organic phases were combined, washed with saturated brine (10 mL), dried over anhydrous sodium sulfate, filtered, and the filtrate was concentrated under reduced pressure. The residue was purified by silica gel column chromatography to obtain compound 026-2 (190 mg, yield: 75.45%). LC-MS: $[M+H]^+$ = 324.05.

26.3 Synthesis of compound 026-3

026-2 (180 mg, 0.56 mmol) was dissolved in dichloromethane (6 mL). A solution of hydrogen chloride in 1,4-dioxane (3 mL, 4 M) was added, and the reaction mixture was stirred at room temperature for 2 hours. The reaction mixture was directly concentrated under reduced pressure to obtain compound 026-3 (131 mg, crude product). LC-MS: $[M+H]^+$ = 224.10.

26.4 Synthesis of compound 026-4

026-3 (131 mg, 0.59 mmol) and tetrabutylammonium iodide (129 mg, 0.35 mmol) were dissolved in dichloromethane (2 mL). At 0°C, boron tribromide (3.52 mL, 3.52 mmol) was added dropwise to the reaction mixture. After the dropwise addition was completed, the reaction was carried out at room temperature for 16 hours. The reaction mixture was poured into saturated aqueous sodium bicarbonate solution (8 mL) to quench the reaction, then extracted with dichloromethane (10 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 026-4 (35 mg, yield: 25.66%). LC-MS: $[M+H]^+$ = 210.10.

26.5 Synthesis of compound 026-5

026-4 (35 mg, 0.17 mmol) was dissolved in ethanol (1 mL). 001-d (34 mg, 0.20 mmol) and N,N-diisopropylethylamine (109 mg, 0.85 mmol) were added, and the reaction mixture was stirred at 55°C for 16 hours. The reaction mixture was directly concentrated under reduced pressure. The residue was purified by silica gel column chromatography to obtain compound 026-5 (40 mg, yield: 67.44%). LC-MS: $[M+H]^+$ = 334.00.

26.6 Synthesis of compound 026

026-5 (35 mg, 0.11 mmol) was dissolved in ethanol (1 mL). 001-10 (22 mg, 0.11 mmol) and N,N-diisopropylethylamine (71 mg, 0.55 mmol) were added, and the reaction mixture was stirred at room temperature for 1 hour. The reaction mixture was purified by preparative HPLC (basic conditions) and lyophilized to obtain compound 026 (10.1 mg, chiral purity: 99.7% (HPLC method)).

$^1$H NMR (400 MHz, DMSO-$d_6$): δ 8.46 (d, *J* = 3.8 Hz, 1H), 8.17 (d, *J* = 5.4 Hz, 1H), 7.89 (d, *J* = 5.0 Hz, 1H), 7.58 (t, *J* = 9.0 Hz, 1H), 7.38 (dt, *J* = 8.4, 4.0 Hz, 1H), 5.85 (s, 1H), 4.30 (s, 1H), 2.99 (s, 3H), 1.86 (s, 2H), 1.75 - 1.59 (m, 4H), 1.45 - 1.30 (m, 2H), 1.24 (d, *J= 4.8* Hz, 6H), 1.07 (s, 3H). LC-MS: $[M+H]^+$ = 496.05.

Example 27. Synthesis of 3-hydroxy-N,N-dimethyl-4-((2-(((1-methyl-4,6-dihydro-1H-furo[3,4-c]pyrazol-3-yl)(1-methylcyclopentyl)methyl)amino)-3,4-dioxocyclobut-1-en-1-yl)amino)picolinamide (compound 027)

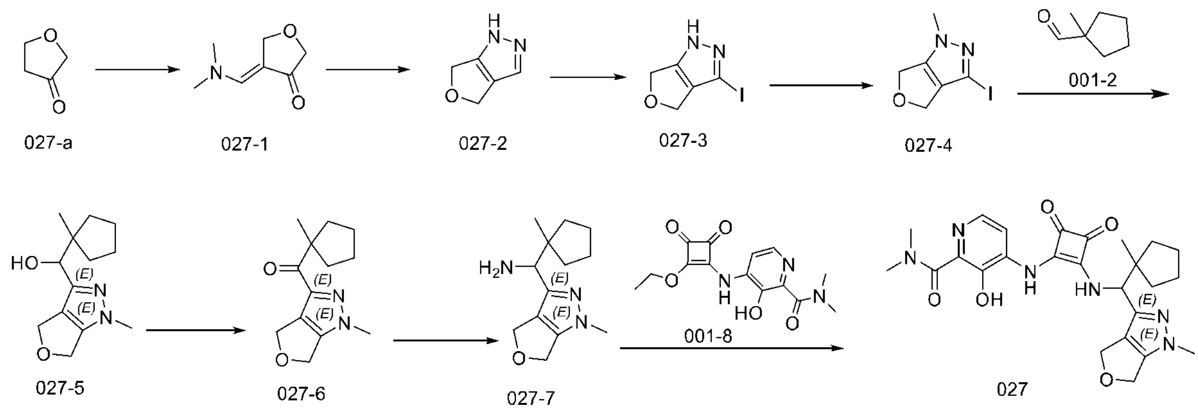

027-a 027-1 027-2 027-3 027-4
027-5 027-6 027-7 027

27.1 Synthesis of compound 027-1

At room temperature, 027-a (9.0 g, 31.4 mmol) was dissolved in toluene (90 mL), and *tert*-butoxybis(dimethylamino)methane (19.2 g, 32.9 mmol) was added. The reaction mixture was stirred at room temperature for 4 hours. The reaction mixture was concentrated under reduced pressure. The residue was added to methyl tert-butyl ether (30 mL) and stirred for 10 minutes, filtered, and the filter cake was dried under vacuum to obtain compound 027-1 (11.1 g).

27.2 Synthesis of compound 027-2

At room temperature, 027-1 (7.0 g, 49.6 mmol), hydrazine hydrochloride (7.0 g, 99.3 mmol), and acetic acid (9 mL, 148.8 mmol) were dissolved in ethanol (80 mL). The reaction mixture was stirred at 80°C for 2 hours. The reaction mixture was concentrated under reduced pressure. The residue was diluted with water (50 mL) and extracted with ethyl acetate (30 mL × 3). The organic phase was directly concentrated under reduced pressure, and the residue was purified by silica gel column chromatography to obtain compound 027-2 (3.7 g).

27.3 Synthesis of compound 027-3

At room temperature, 027-2 (3.7 g, 29.2 mmol) and potassium carbonate (10.4 g, 68.5 mmol) were dissolved in N,N-dimethylformamide (100 mL). At 0°C, iodine (18.4 g, 67.2 mmol) was added, and the reaction mixture was stirred at room temperature for 24 hours. The reaction mixture was quenched with saturated sodium sulfite solution (100 mL) and extracted with ethyl acetate (50 mL × 3). The organic phase was directly concentrated under reduced pressure, and the residue was purified by silica gel column chromatography to obtain compound 027-3 (1.54 g).

27.4 Synthesis of compound 027-4

At room temperature, sodium hydride (0.86 g, 21.35 mmol) was dissolved in *N,N*-dimethylformamide (20 mL). At 0°C, 027-3 (1.68 g, 7.12 mmol) was added, and the mixture was stirred at room temperature for 0.5 hours. Iodomethane (1.52 g, 10.68 mmol) was added, and the reaction mixture was stirred at room temperature for 1 hour. The reaction mixture was quenched with water (30 mL) and extracted with ethyl acetate (20 mL × 3). The organic phase was directly concentrated under reduced pressure, and the residue was purified by silica gel column chromatography to obtain compound 027-4 (0.53 g). LC-MS: $[M+H]^+ = 250.09$.

27.5 Synthesis of compound 027-5

At room temperature, 027-4 (400 mg, 1.6 mmol) was dissolved in tetrahydrofuran (8 mL). Under a nitrogen atmosphere at -78°C, isopropylmagnesium chloride-lithium chloride complex (1.8 mL, 1.8 mmol) was added dropwise. After stirring at -78°C for 0.5 hours, 001-2 (220 mg, 1.92 mmol) was added, and the reaction mixture was stirred at room temperature for 1 hour. The reaction mixture was quenched with aqueous ammonium chloride solution (20 mL) and extracted with ethyl acetate (20 mL × 3). The organic phase was directly concentrated under reduced pressure, and the

residue was purified by silica gel column chromatography to obtain compound 027-5 (88 mg). LC-MS: $[M+H]^+$ = 237.10.

27.6 Synthesis of compound 027-6

At room temperature, 027-5 (30 mg, 0.13 mmol) was dissolved in dichloromethane (2 mL). Dess-Martin periodinane (160 mg, 0.64 mmol) was added, and the reaction mixture was stirred at room temperature for 1 hour. The reaction mixture was diluted with water (10 mL) and extracted with dichloromethane (10 mL × 3). The organic phase was directly concentrated to dryness under reduced pressure to obtain compound 027-6 (30 mg). LC-MS: $[M+H]^+$ = 235.10.

27.7 Synthesis of compound 027-7

At room temperature, 027-6 (30 mg, 0.13 mmol), hydroxylamine hydrochloride (125 mg, 0.77 mmol), and sodium acetate (250 mg, 0.77 mmol) were dissolved in ethanol (3 mL). The reaction mixture was stirred at 45°C for 2 hours, then diluted with water (10 mL) and extracted with ethyl acetate (10 mL × 3). The organic phase was washed with saturated brine (20 mL) and then concentrated under reduced pressure. The residue was dissolved in methanol (2 mL), and zinc powder (80 mg, 1.3 mmol) was added. Then, 6 M hydrochloric acid (0.5 mL) was slowly added at 0°C, and the reaction mixture was stirred at room temperature for 1 hour. The reaction mixture was filtered, and the filtrate was concentrated. The residue was diluted with water (10 mL), and the pH was adjusted to 7-8 with saturated sodium carbonate solution. The mixture was extracted with ethyl acetate (10 mL × 3). The combined organic phases were directly concentrated under reduced pressure. The residue was purified by silica gel column chromatography to obtain compound 027-7 (10 mg). LC-MS: $[M+H]^+$ = 219.10.

27.8 Synthesis of compound 027

At room temperature, 027-7 (10 mg, 42.3 μmol), 001-8 (10 mg, 50.8 μmol), and *N,N*-diisopropylethylamine (15 mg, 211.5 μmol) were dissolved in ethanol (1 mL), and the reaction mixture was stirred at room temperature for 0.5 hours. The reaction mixture was purified by preparative HPLC (acidic conditions) and lyophilized to obtain the salt of compound 027. Then, an aqueous sodium bicarbonate solution was added for neutralization. The resulting mixture was extracted with ethyl acetate, and the combined organic phases were concentrated under reduced pressure and lyophilized to obtain compound 027 (4.7 mg).

$^1H$ NMR (400 MHz, $CD_3CN$): δ 12.05 (s, 1H), 8.84 (d, *J* = 12.0Hz, 1H), 8.60 (d, *J* = 4.0 Hz, 1H), 8.04 (d, *J* = 4.0 Hz, 1H), 5.22 (d, *J* = 8.0 Hz, 1H), 5.06 - 4.97 (m, 1H), 4.87 (dd, *J* = 4.0, 4.0 Hz, 1H), 4.79 (s, 2H), 3.73 (s, 3H), 3.02 (s, 6H), 1.77 - 1.60 (m, 6H), 1.37 - 1.25 (m, 2H), 1.07 (s, 3H); LC-MS: $[M+H]^+$ = 494.95.

Example 28. Synthesis of 3-hydroxy-N,N-dimethyl-4-((2-(((2-methyl-2,6-dihydro-4H-furo[3,4-c]pyrazol-3-yl))(1-methylcyclopentyl)methyl)amino)-3,4-dioxocyclobut-1-en-1-yl)amino)picolinamide (compound 028)

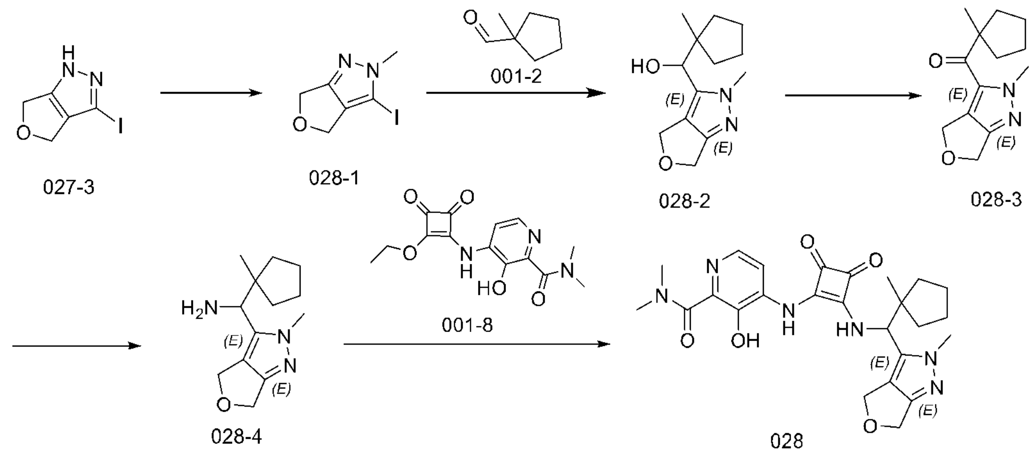

027-3
028-1
028-2
028-3
028-4
028

The synthesis of compound 028 was carried out with reference to the synthesis method for compound 027 in Example 27, except that the starting material 027-4 was replaced with 028-1, to obtain compound 028 (34.8 mg).

$^1H$ NMR (400 MHz, DMSO-$d_6$): δ 11.74 (s, 1H), 9.70 (s, 1H), 8.60 (d, *J* = 8.0 Hz, 1H), 8.01 (s, 1H), 7.93 (s, 1H), 5.42 (d, *J* = 8.0 Hz, 1H), 4.82 (s, 2H), 4.68-4.66 (m, 2H), 3.85 (s, 3H), 3.18 (s, 3H), 3.05 (s, 3H), 1.72-1.53 (m, 6H), 1.44-1.35 (m, 2H), 1.02 (s, 3H); LC-MS: $[M+H]^+$ = 495.05.

Example 29. Synthesis of 3-hydroxy-*N*,*N*-dimethyl-4-((2-(((3-methyl-5,6-dihydro-4*H*-pyrrolo[1,2-*b*]pyrazol-2-yl)(1-methylcyclopentyl)methyl)amino)-3,4-dioxocyclobut-1-en-1-yl)amino)picolinamide (compound 029)

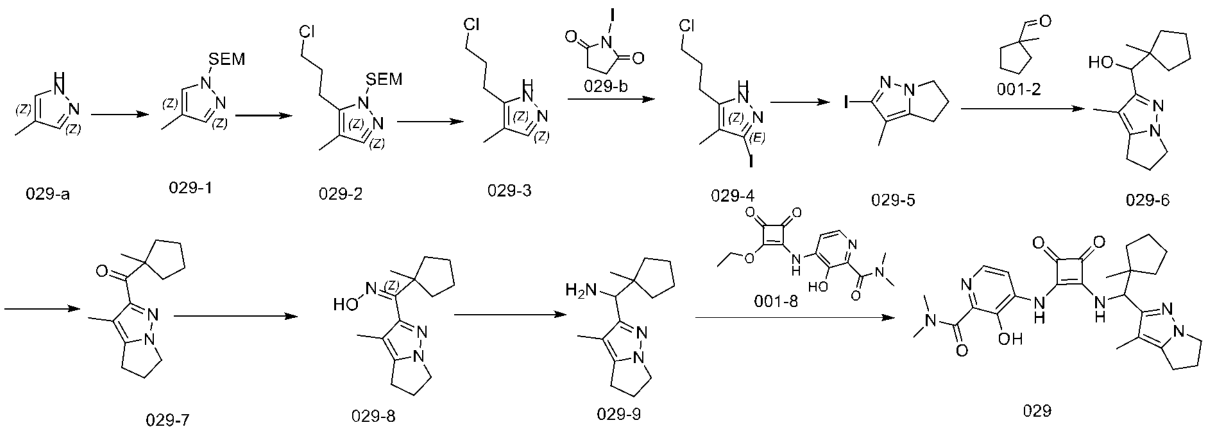


29.1 Synthesis of compound 029-1

At room temperature, 029-a (10.0 g, 121.79 mmol) and sodium hydroxide (9.7 g, 243.59 mmol) were dissolved in tetrahydrofuran (100 mL). At 0°C, 2-(trimethylsilyl)ethoxymethyl chloride (24.4 g, 146.15 mmol) was added dropwise to the reaction mixture. After the dropwise addition was completed, the reaction was carried out at room temperature for 2 hours. The reaction mixture was quenched with water (100 mL) and extracted with ethyl acetate (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 029-1 (19 g). LC-MS: $[M+H]^+$ = 213.10.

29.2 Synthesis of compound 029-2

029-1 (12.0 g, 56.51 mmol) was dissolved in tetrahydrofuran (100 mL). After purging with nitrogen, a solution of n-butyllithium in n-hexane (39 mL, 67.81 mmol, 1.6 M) was added dropwise at -78°C. After the dropwise addition was completed, the reaction was maintained at -78°C and carried out for 1 hour. Then, 1-bromo-3-chloropropane (13.3 g, 84.76 mmol) was slowly added dropwise to the above system. After the addition was completed, the reaction was carried out at -78°C for another 1 hour. The reaction mixture was quenched with water (100 mL) and extracted with ethyl acetate (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 029-2 (18 g). LC-MS: $[M+H]^+$ = 289.00.

29.3 Synthesis of compound 029-3

029-2 (18.0 g, 62.31 mmol) was dissolved in a solution of hydrogen chloride in ethyl acetate (100 mL, 4 M). The reaction was carried out at 60°C for 2 hours. The reaction mixture was concentrated under reduced pressure. The residue was adjusted to pH = 8 with a saturated sodium carbonate solution and extracted with ethyl acetate (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 029-3 (3.9 g). LC-MS: $[M+H]^+$ = 159.10.

29.4 Synthesis of compound 029-4

029-3 (3.9 g, 24.59 mmol) was dissolved in dichloromethane (60 mL). 029-b (27.7 g, 122.93 mmol) was slowly added, and the reaction was carried out at room temperature for 1 hour. The reaction mixture was quenched with water

(100 mL) and extracted with dichloromethane (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 029-4 (10 g). LC-MS: $[M+H]^+$ = 284.85.

29.5 Synthesis of compound 029-5

Compound 029-4 (10.0 g, 3.51 mmol) was dissolved in N,N-dimethylformamide (100 mL). The mixture was cooled to 0°C with an ice-water bath, and sodium hydride (110 mg, 4.22 mmol, 60% purity) was slowly added. After the addition was completed, the reaction was carried out at room temperature for 24 hours. The reaction mixture was quenched with water (300 mL) and extracted with ethyl acetate (100 mL × 3). The organic phases were combined, washed once with saturated brine (100 mL), dried over anhydrous sodium sulfate, filtered, and the filtrate was concentrated under reduced pressure. The residue was purified by silica gel column chromatography to obtain compound 029-5 (170 mg). LC-MS: $[M+H]^+$= 248.95.

29.6 Synthesis of compound 029-6

The synthesis of compound 029-6 was carried out with reference to the synthesis method for compound 027-5 in Example 27, except that the starting material 027-4 was replaced with 029-5, to obtain compound 029-6 (40 mg).

29.7 Synthesis of compound 029-7

The synthesis of compound 029-7 was carried out with reference to the synthesis method for compound 027-6 in Example 27, except that the starting material 027-5 was replaced with 029-6, to obtain compound 029-7 (40 mg, crude product).

29.8 Synthesis of compound 029-8

029-7 (40 mg, 0.17 mmol), hydroxylamine hydrochloride (71 mg, 1.03 mmol), and sodium acetate (84 mg, 1.03 mmol) were dissolved in ethanol (1 mL). The reaction was carried out at 80°C for 2 hours. The reaction mixture was diluted with water (5 mL) and extracted with ethyl acetate (10 mL × 3). The organic phases were combined, washed once with saturated brine (10 mL), dried over anhydrous sodium sulfate, filtered, and concentrated under reduced pressure to obtain compound 029-8 (50 mg, crude product). LC-MS: $[M+H]^+$ = 248.10.

29.9 Synthesis of compound 029-9

029-8 (50 mg, 0.20 mmol) and zinc powder (132 mg, 2.0 mmol) were dissolved in hydrochloric acid (1 mL, 2 M), and the reaction was carried out at room temperature for 1 hour. The pH was adjusted to 8 with saturated aqueous sodium bicarbonate solution, and then the mixture was extracted with ethyl acetate (10 mL × 3). The organic phases were combined, washed with saturated brine (10 mL), dried over anhydrous sodium sulfate, filtered, and the filtrate was concentrated under reduced pressure to obtain compound 029-9 (30 mg, crude product). LC-MS: $[M+H]^+$ = 217.10.

29.10 Synthesis of compound 029

The synthesis of compound 029 was carried out with reference to the synthesis method for compound 027 in Example 27, except that the starting material 027-7 was replaced with 029-9, to obtain compound 029 (10.4 mg).

$^1$H NMR (400 MHz, DMSO-$d_6$): δ 11.65 (s, 1H), 9.90 (s, 1H), 9.13 *(d, J*= 10.0 Hz, 1H), 8.00 (s, 2H), 5.32 - 5.28 (m, 1H), 4.09 - 3.96 (m, 2H), 3.29 (s, 2H), 3.19 (s, 3H), 3.05 (s, 3H), 2.74 (dd, *J*= 12.0, 6.8 Hz, 2H), 1.93 (s, 3H), 1.71 - 1.66 (m, 1H), 1.65 - 1.55 (m, 5H), 1.36 - 1.30 (m, 1H), 1.22 - 1.16 (m, 1H), 1.08 (s, 3H); LC-MS: $[M+H]^+$ = 493.05.

Example 30. Synthesis of (*R*)-3-hydroxy-*N,N*-dimethyl-4-((2-(((2-methyl-2,4,5,6-tetrahydrocyclopenta[*c*]pyrazol-3-yl)(1-methylcyclopentyl)methyl)amino)-3,4-dioxocyclobut-1-en-1-yl)amino)picolinamide (compound 030)

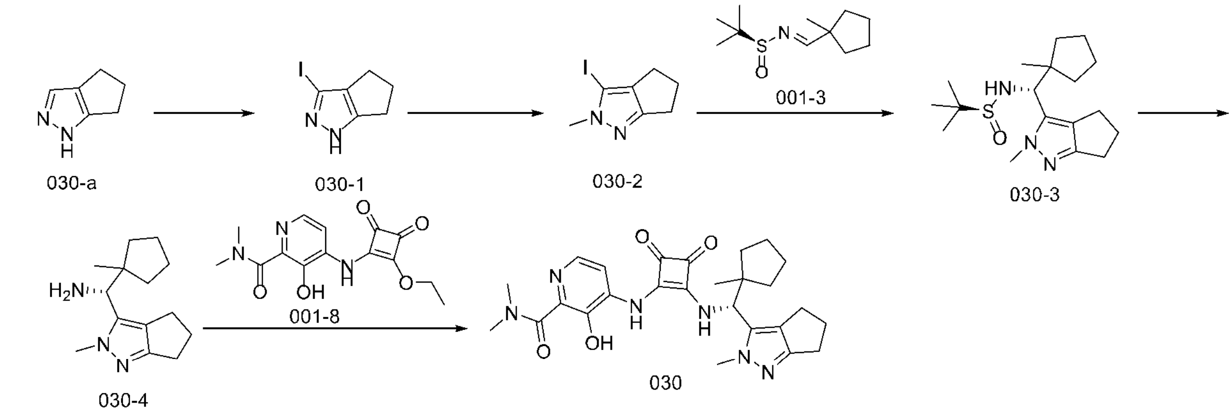


30.1 Synthesis of compound 030-2

The synthesis of compound 030-2 was carried out with reference to the synthesis method for compound 027-4 in Example 27, except that the starting material 027-2 was replaced with 030-a, to obtain compound 030-2 (231 mg, yield: 39.40%).

$^1$H NMR (400 MHz, $CDCl_3$): δ 3.84 (s, 3H), 2.74 (t, *J* = 7.4 Hz, 2H), 2.54 (t, *J* = 7.0 Hz, 2H), 2.41 - 2.31 (m, 2H). LC-MS: $[M+H]^+$ = 248.95.

30.2 Synthesis of compound 030

The synthesis of compound 030 was carried out with reference to the synthesis method for compound 019 in Example 19, except that the starting material 019-a was replaced with 030-2, to obtain compound 030 (47.4 mg, chiral purity: 69.5% (HPLC method O)).

$^1$H NMR (400 MHz, DMSO-$d_6$): δ 11.92 (s, 1H), 9.89 (s, 1H), 8.73 (d, *J* = 6.6 Hz, 1H), 7.92 (s, 2H), 5.39 (d, *J* = 9.0 Hz, 1H), 3.78 (s, 3H), 3.14 (s, 3H), 3.04 (s, 3H), 2.59 (ddd, *J* = 22.8, 15.4, 7.2 Hz, 4H), 2.29 (dt, *J* = 15.4, 7.6 Hz, 2H), 1.61 (d, *J* = 8.2 Hz, 6H), 1.43 (s, 1H), 1.33 (d, *J* = 9.2 Hz, 1H), 1.03 (s, 3H); LC-MS: $[M+H]^+$ = 493.10.

Example 31. Synthesis of (*R*)-4-((2-(((1,3-dimethyl-2-oxo-2,3-dihydro-1*H*-imidazol-4-yl)(1-methylcyclopentyl)methyl) amino)-3,4-dioxocyclobut-1-en-1-yl)amino)-3-hydroxy-*N*,*N*-dimethylpicolinamide (compound 031)

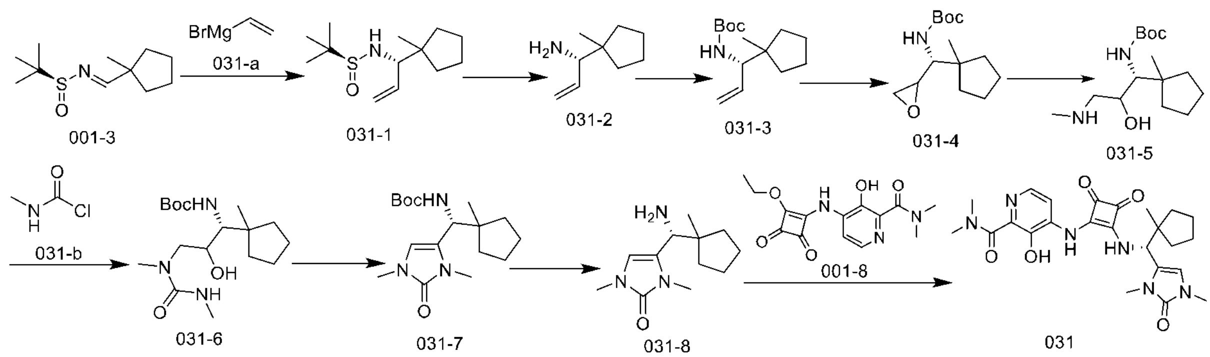


31.1 Synthesis of compound 031-1

At room temperature, 001-3 (2.0 g, 9.3 mmol) was added to anhydrous tetrahydrofuran (20 mL). Under a nitrogen atmosphere at -78°C, 031-a (18.6 mL, 18.6 mmol) was slowly added. After the addition was completed, the reaction was stirred at room temperature for 0.5 hours. The reaction mixture was quenched with saturated aqueous ammonium chloride solution (10 mL) and diluted with water (30 mL). The mixture was extracted with ethyl acetate (30 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 1/0 to 10/1) to obtain compound 031-1 (2.0 g, yield: 88.2%). LC-MS:

$[M+H]^+$ = 244.15.

31.2 Synthesis of compound 031-2 hydrochloride

At room temperature, 031-1 (2.0 g, 8.2 mmol) was added to dichloromethane (10 mL). A solution of hydrogen chloride in 1,4-dioxane (10 mL) was slowly added, and the reaction mixture was stirred at room temperature for 1 hour. The reaction mixture was directly concentrated to dryness under reduced pressure to obtain compound 031-2 hydrochloride (1.0 g, crude product). LC-MS: $[M+H]^+$ = 140.20.

31.3 Synthesis of compound 031-3

At room temperature, 031-2 hydrochloride (1.0 g, 5.6 mmol), di-tert-butyl dicarbonate (1.5 g, 6.7 mol), and triethylamine (1.7 g, 16.8 mmol) were added to dichloromethane (20 mL), and the reaction mixture was stirred at room temperature for 1 hour. Water (20 mL) was added to the reaction mixture for dilution, and the mixture was extracted with dichloromethane (20 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 10/1 to 5/1) to obtain compound 031-3 (1.4 g, yield: 100%). LC-MS: $[M+H-t-Bu]^+$ = 184.10.

31.4 Synthesis of compound 031-4

At room temperature, 031-3 (1.0 g, 4.65 mmol) and 85% m-chloroperoxybenzoic acid (1.9 g, 9.2 mmol) were added to dichloromethane (20 mL), and the reaction mixture was stirred at room temperature for 2 hours. The reaction mixture was quenched with saturated aqueous sodium sulfite solution (20 mL) and diluted with water (30 mL). The mixture was extracted with ethyl acetate (30 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 031-4 (1.0 g, yield: 84.3%). LC-MS: $[M+H-t-Bu]^+$ = 200.10.

31.5 Synthesis of compound 031-5

At room temperature, 031-4 (370 mg, 1.45 mmol) was added to a solution of methylamine in ethanol (4 mL, 33%). The reaction mixture was stirred at 50°C for 1 hour. The reaction mixture was directly concentrated to dryness under reduced pressure to obtain compound 031-5 (350 mg, crude product). LC-MS: $[M+H]^+$ = 287.10.

31.6 Synthesis of compound 031-6

At room temperature, 031-5 (350 mg, 1.2 mmol), 031-b (135 mg, 1.4 mmol), and triethylamine (245 mg, 2.4 mmol) were added to dichloromethane (5 mL). The reaction was stirred at room temperature for 0.5 hours. Water (10 mL) was added to the reaction mixture for dilution, and the mixture was extracted with dichloromethane (10 mL × 3). The organic phase was washed with saturated brine (10 mL), dried over anhydrous sodium sulfate, filtered, and the filtrate was concentrated under reduced pressure to obtain a crude product. The crude product was purified by silica gel column chromatography (dichloromethane/methanol = 30/1 to 10/1) to obtain compound 031-6 (300 mg, yield: 72.5%). LC-MS: $[M+H]^+$ = 344.10.

31.7 Synthesis of compound 031-7

Oxalyl chloride (308 mg, 1.74 mmol) was dissolved in anhydrous dichloromethane (6 mL). Under a nitrogen atmosphere at -78°C, dimethyl sulfoxide (204 mg, 2.61 mmol) was added dropwise to the reaction mixture. After the dropwise addition was completed, the mixture was stirred at -78°C for 15 minutes. At -78°C, 031-6 (300 mg, 0.87 mmol) was added, and the mixture was stirred at - 78°C for another 15 minutes. Then, triethylamine (532 mg, 5.22 mmol) was added, and the reaction mixture was warmed to room temperature and stirred for 15 minutes. Water (10 mL) was added to the reaction mixture for dilution, and the mixture was extracted with dichloromethane (10 mL × 3). The organic phase was washed with saturated brine (10 mL), dried over anhydrous sodium sulfate, filtered, and the filtrate was concentrated under reduced pressure to obtain a crude product. The crude product was purified by silica gel column chromatography (dichloromethane/methanol = 30/1 to 10/1) to obtain compound 031-7 (290 mg, yield: 100%). LC-MS: $[M+H]^+$ = 324.10.

31.8 Synthesis of compound 031-8 hydrochloride

At room temperature, 031-7 (100 mg, 0.31 mmol) was dissolved in dichloromethane (1 mL). A solution of hydrogen chloride in 1,4-dioxane (1 mL, 4 M) was slowly added, and the reaction mixture was stirred at room temperature for 1 hour. The reaction mixture was directly concentrated to dryness under reduced pressure to obtain compound 031-8 hydrochloride (78 mg, crude product). LC-MS: $[M-NH_2]^+$ = 207.10.

31.9 Synthesis of compound 031

At room temperature, 031-8 hydrochloride (78 mg, 0.3 mmol), 001-8 (92 mg, 0.3 mol), and N,N-diisopropylethylamine (154 mg, 1.2 mmol) were added to ethanol (3 mL) and stirred at room temperature for 1 hour. The reaction mixture was purified by preparative HPLC (basic conditions) and lyophilized to obtain compound 031 (63.5 mg, yield: 65.1%, chiral purity: 100% (HPLC method C)).

$^1H$ NMR (400 MHz, DMSO-$d_6$): δ 11.67 (s, 1H), 9.66 (s, 1H), 8.69 (s, 1H), 8.13-7.80 (m, 2H), 6.59 (s, 1H), 5.11-5.06 (m, 1H), 3.14 (s, 6H), 3.10-3.05 (m, 6H), 1.65-1.60 (m, 6H), 1.48-1.35 (m, 2H), 1.11 (s, 3H). LC-MS: $[M+H]^+$ = 483.05.

Example 32. Synthesis of 4-((2-((2-(dimethylamino)-1-(1-methylcyclopentyl)-2-oxoethyl)amino)-3,4-dioxocyclobut-1-en-1-yl)amino)-3-hydroxy-*N*,*N*-dimethylpicolinamide (compound 032)

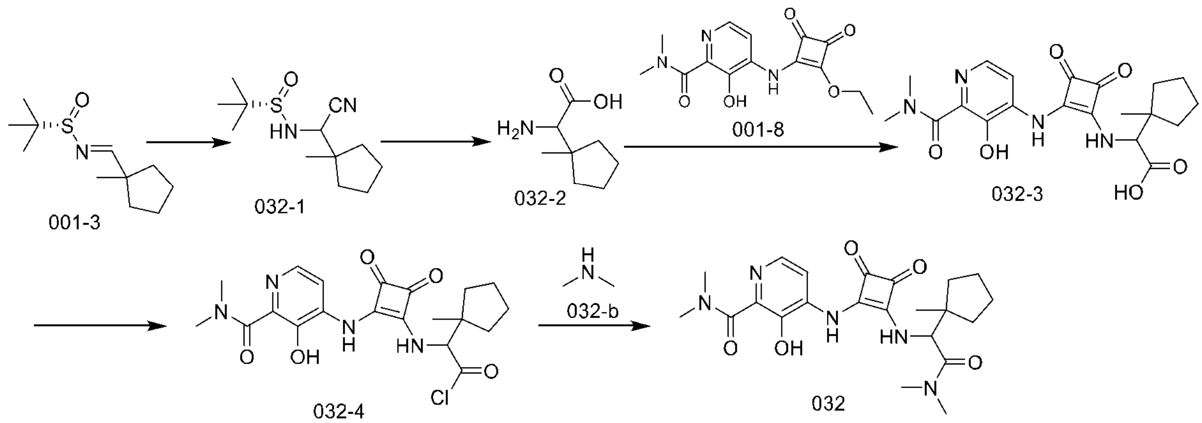


32.1 Synthesis of compound 032-1

001-3 (1 g, 4.64 mmol) was weighed and added to tetrahydrofuran (20 mL), cesium fluoride (2.82 g, 18.6 mmol), and trimethylsilyl cyanide (1.84 g, 18.6 mmol). The reaction mixture was stirred at 40°C for 12 hours. The reaction mixture was directly concentrated under reduced pressure. The residue was purified by silica gel column chromatography (petroleum ether/ethyl acetate = 5/1) to obtain compound 032-1 (1 g, yield: 88.5%).

32.2 Synthesis of compound 032-2

Concentrated hydrochloric acid (3 mL) was added to 032-1 (600 mg, 2.5 mmol), and the reaction mixture was stirred at 100°C for 12 hours. The reaction mixture was directly concentrated to obtain compound 032-2 (450 mg), which was used directly in the next step.

32.3 Synthesis of compound 032-3

032-2 (300 mg, 0.6 mmol) was dissolved in ethanol (10 mL). 001-8 (175 mg, 1.2 mmol) and *N,N*-diisopropylethylamine (255 mg, 2.0 mmol) were added. The reaction mixture was stirred at 55°C overnight. The reaction mixture was directly concentrated under reduced pressure. The residue was purified by silica gel column chromatography to obtain compound 032-3 (330 mg, yield: 51%).

32.4 Synthesis of compound 032-4

032-3 (330 mg, 0.79 mmol) was weighed and dissolved in dichloromethane (10 mL). At 0°C, oxalyl chloride (201 mg, 1.58 mmol) and two drops of N,N-dimethylformamide were added. The reaction mixture was warmed to room temperature and stirred for 1 hour. The reaction mixture was directly concentrated to obtain the target compound 032-4 (330 mg, crude product), which was directly used in the next step.

32.5 Synthesis of compound 032

032-4 (280 mg, 0.12 mmol) was weighed and dissolved in dichloromethane (10 mL). At 0°C, triethylamine (130 mg, 0.23 mmol) and a solution of dimethylamine in tetrahydrofuran (0.07 mL, 0.14 mmol, 2 M) were added. The reaction mixture was warmed to room temperature and stirred for 2 hours. The reaction mixture was concentrated under reduced pressure. The residue was purified by preparative HPLC and lyophilized to obtain compound 032 (11.6 mg, yield: 3.5%).

$^1$H NMR (400 MHz, DMSO-$d_6$): δ 9.09 (s, 1H), 7.92 (d, *J* = 8.0 Hz, 1H), 7.79 (s, 1H), 5.38 (s, 1H), 3.09 (d, *J* = 8.0 Hz, 6H), 3.01 (s, 3H), 2.88 (s, 3H), 1.68-1.60 (m, 6H), 1.32 - 1.24 (m, 2H), 1.01 (s, 3H); LC-MS: $[M+H]^+$ = 444.00.

Example 33. Synthesis of 3-hydroxy-*N,N*-dimethyl-4-((2-((1-(1-methylcyclopentyl)-2-oxopropyl)amino)-3,4-dioxocyclobut-1-en-1-yl)amino)picolinamide (compound 033)

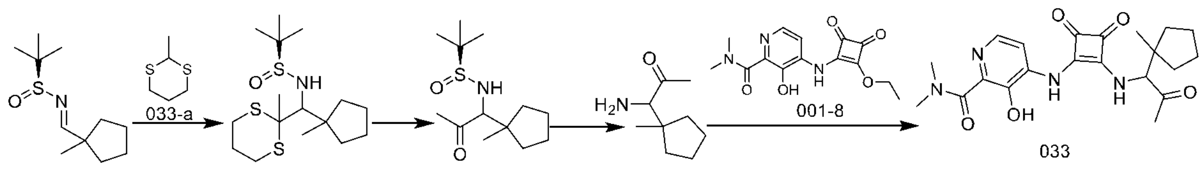


001-3 033-1 033-2 033-3 033

33.1 Synthesis of compound 033-1

At room temperature, 033-a (1.0 g, 4.65 mmol) was added to anhydrous tetrahydrofuran (20 mL). At 0°C, a solution of n-butyllithium in n-hexane (5.2 mL, 8.37 mmol, 1.6 M) was slowly added. The reaction mixture was stirred at 0°C for 0.5 hours. The reaction mixture was cooled to -78°C, and 001-3 (934 mg, 6.98 mmol) was added. The reaction mixture was stirred at -78°C for 0.5 hours. The reaction mixture was quenched with saturated aqueous ammonium chloride solution (10 mL) and diluted with water (30 mL). The mixture was extracted with ethyl acetate (30 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 033-1 (1.4 g, yield: 86.2%). LC-MS: $[M+H]^+$ = 350.05.

33.2 Synthesis of compound 033-2

At 0°C, 033-1 (300 mg, 0.86 mmol), iodine (655 mg, 2.58 mmol), and sodium bicarbonate (614 mg, 7.31 mmol) were added to acetonitrile (15 mL) and water (20 mL). The reaction mixture was stirred at 0°C for 1 hour. A saturated aqueous sodium sulfite solution (20 mL) was added to the reaction mixture, and the mixture was extracted with ethyl acetate (20 mL × 3). The organic phase was washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 8/1 to 5/1) to obtain compound 033-2 (70 mg, yield: 31.4%). LC-MS: $[M+H]^+$ = 260.15.

33.3 Synthesis of compound 033

The synthesis of compound 033 was carried out with reference to the synthesis method for compound 001 in Example 1, except that the starting material 001-9 was replaced with 033-2, to obtain compound 033 (34.2 mg, yield: 65.1%).

$^1$H NMR (400 MHz, DMSO-$d_6$): δ 11.69 (s, 1H), 9.92 (s, 1H), 8.99 (s, 1H), 8.18 - 7.82 (m, 2H), 4.87 (d, *J* = 9.6 Hz, 1H), 3.17 (s, 3H), 3.04 (s, 3H), 2.27 (s, 3H), 1.73 - 1.55 (m, 6H), 1.39 - 1.35 (m, 2H), 0.91 (s, 3H); LC-MS: $[M+H]^+$ = 415.05.

Example 34. Synthesis of 4-((2-((2-cyclopropyl-1-(1-methylcyclopentyl)-2-oxoethyl)amino)-3,4-dioxocyclobut-1-en-1-yl)amino)-3-hydroxy-*N*,*N*-dimethylpicolinamide (compound 034)

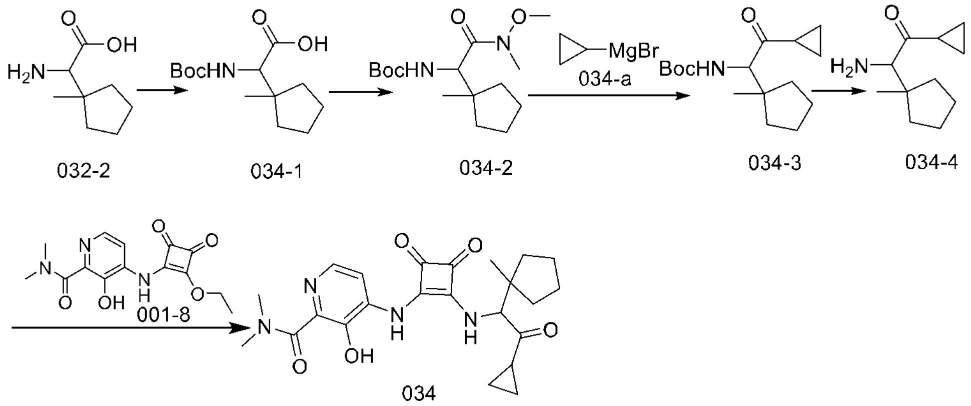

032-2 034-1 034-2 034-3 034-4

034

34.1 Synthesis of compound 034-1

At room temperature, 032-2 (1.4 g, 8.6 mmol), di-tert-butyl dicarbonate (1.9 g, 8.6 mol), and sodium hydroxide (688 mg, 17.2 mmol) were added to 1,4-dioxane (10 mL) and water (10 mL). The reaction was stirred at room temperature overnight. Water (10 mL) was added to the reaction mixture for dilution, and the mixture was extracted with ethyl acetate (20 mL × 2). The organic phases were discarded. The aqueous phase was adjusted to pH = 5-6 with 2 N hydrochloric acid solution and extracted with ethyl acetate (20 mL × 3). The organic phases were combined, washed with saturated brine (20 mL), dried over anhydrous sodium sulfate, filtered, and the filtrate was concentrated under reduced pressure to obtain compound 034-1 (840 mg, crude product). LC-MS: $[M+H\text{-}t\text{-}Bu]^+$= 202.10.

34.2 Synthesis of compound 034-2

At room temperature, 034-1 (540 mg, 2.1 mmol), *N,O*-dimethylhydroxylamine hydrochloride (262 mg, 2.7 mol), propylphosphonic anhydride (2.0 g, 3.2 mmol, 50% solution in ethyl acetate), and triethylamine (857 mg, 8.4 mmol) were added to dichloromethane (10 mL), and the reaction mixture was stirred at room temperature for 0.5 hours. Water (20 mL) was added to the reaction mixture for dilution, and the mixture was extracted with dichloromethane (20 mL × 3). The combined organic phases were washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 10/1 to 5/1) to obtain compound 034-2 (370 mg, yield: 57.1%). LC-MS: $[M+H\text{-}t\text{-}Bu]^+$ = 301.10.

34.3 Synthesis of compound 034-3

At room temperature, 034-2 (370 mg, 1.2 mmol) was dissolved in tetrahydrofuran (5 mL). At 0°C, a solution of 034-a in tetrahydrofuran (12 mL, 12.0 mmol) was added dropwise slowly. After the dropwise addition was completed, the reaction was stirred at room temperature for 3 hours. The reaction mixture was quenched with saturated aqueous ammonium chloride solution (20 mL), diluted with water (30 mL), and extracted with ethyl acetate (30 mL × 3). The organic phases were combined, washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 034-3 (160 mg, yield: 47.5%).

34.4 Synthesis of compound 034-4

At room temperature, 034-3 (160 mg, 0.57 mmol) was dissolved in dichloromethane (2 mL). A solution of hydrogen chloride in 1,4-dioxane (2 mL, 4 M) was slowly added, and the reaction mixture was stirred at room temperature for 0.5 hours. The reaction mixture was directly concentrated to dryness under reduced pressure to obtain compound 034-4 (72 mg, crude product). LC-MS: $[M+H]^+$ = 182.15.

34.5 Synthesis of compound 034

The synthesis of compound 034 was carried out with reference to the synthesis method for compound 001 in Example 1, except that the starting material 001-10 was replaced with 034-4, to obtain compound 034 (56.5 mg, yield: 32.0%).

$^1$H NMR (400 MHz, DMSO-$d_6$): δ 11.68 (s, 1H), 9.95 (s, 1H), 9.06 (s, 1H), 8.13 - 7.84 (m, 2H), 5.12 (d, *J* = 9.6 Hz, 1H), 3.17 (s, 3H), 3.04 (s, 3H), 2.27 - 2.16 (m, 1H), 1.87 - 1.75 (m, 1H), 1.76 - 1.57 (m, 5H), 1.50 - 1.40 (m, 1H), 1.33 - 1.31(m, 1H), 1.08 - 0.97 (m, 3H), 0.96 - 0.86 (m, 4H); LC-MS: $[M+H]^+$ = 441.05.

Example 35. Synthesis of 4-((2-((2-cyclobutyl-1-(1-methylcyclopentyl)-2-oxoethyl)amino)-3,4-dioxocyclobut-1-en-1-yl)amino)-3-hydroxy-*N*,*N*-dimethylpicolinamide (compound 035)

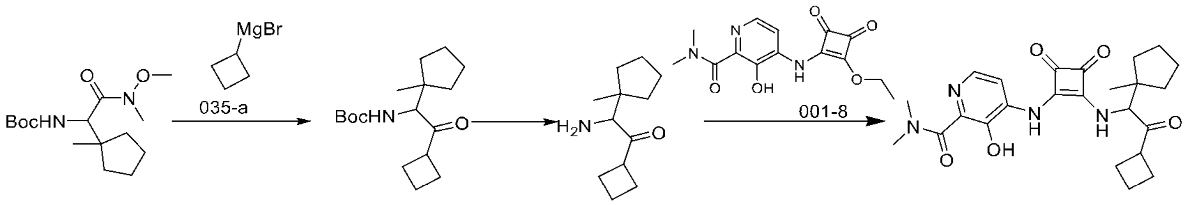


034-2 035-1 035-2 035

The synthesis of compound 035 was carried out with reference to the synthesis method for compound 034 in Example 34, except that the starting material 034-a was replaced with 035-a, to obtain compound 035 (0.9 mg).

$^1$H NMR (400 MHz, DMSO-$d_6$): δ 8.01 (s, 2H), 4.93 (d, *J* = 9.6 Hz, 1H), 3.55 (s, 1H), 3.18 (s, 3H), 3.05 (s, 3H), 2.21-2.14 (m, 2H), 2.00-1.99 (m, 6H), 1.63 (d, *J* = 24.4 Hz, 4H), 1.46 (d, *J* = 6.8 Hz, 2H), 0.92 (s, 3H); LC-MS: $[M+H]^+$ = 455.00.

Example 36. Synthesis of 4-((2-((2-(*tert*-butyl(methyl)amino)-1-(1-methylcyclopentyl)-2-oxoethyl)amino)-3,4-dioxocyclobut-1-en-1-yl)amino)-2-hydroxy-*N*,*N*-dimethylpicolinamide (compound 036)

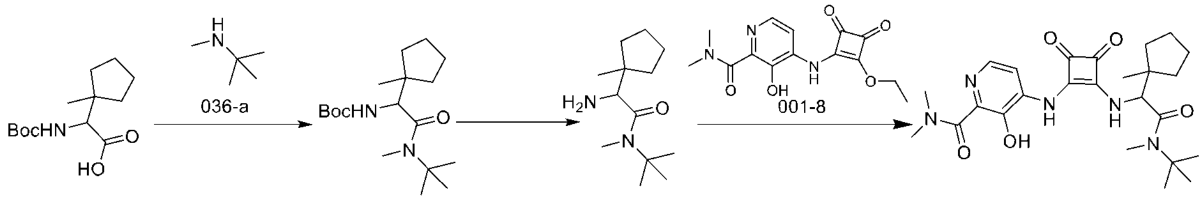


034-1 036-1 036-2 036

36.1 Synthesis of compound 036-1

At room temperature, 036-a (0.35 g, 3.9 mmol), 034-1 (0.5 g, 1.95 mmol), *N,N*-diisopropylethylamine (0.5 g, 3.9 mmol), 2-(7-azabenzotriazol-1-yl)-*N*,*N*,*N'*,*N'*-tetramethyluronium hexafluorophosphate (1.6 g, 4.2 mmol), and *N,N*-dimethylformamide (8 mL) were added to a reaction flask, and the reaction was carried out at 25°C for 2 hours. The reaction mixture was added with water (20 mL) and extracted with ethyl acetate (20 mL × 3). The organic phases were combined, washed with saturated brine (20 mL × 3), dried over anhydrous sodium sulfate, filtered, and the filtrate was concentrated under reduced pressure. The residue was purified by silica gel column chromatography to obtain compound 036-1 (203 mg). LC-MS: $[M+H]^+$ = 327.10.

36.2 Synthesis of compound 036

The synthesis of compound 036 was carried out with reference to the synthesis method for compound 034 in Example 34, except that the starting material 034-3 was replaced with 036-1, to obtain compound 036 (2.0 mg).

$^1$H NMR (400 MHz, DMSO-$d_6$): δ 11.62 (s, 1H), 10.08 (s, 1H), 9.13 (s, 1H), 8.00 (s, 2H), 5.36 (d, *J* =12.0 Hz, 1H), 3.18 (s, 3H), 3.05 (s, 3H), 2.98 (s, 3H), 1.71 - 1.54 (m, 6H), 1.38 (s, 9H), 1.31 - 1.25 (m, 2H), 1.00 (s, 3H); LC-MS: $[M+H]^+$ =

486.05.

Example 37. Synthesis of (*R*)-3-hydroxy-*N,N*-dimethyl-4-((2-(((1-methylcyclopentyl)(phenyl)methyl)amino)-3,4-dioxocyclobut-1-en-1-yl)amino)picolinamide (compound 037)

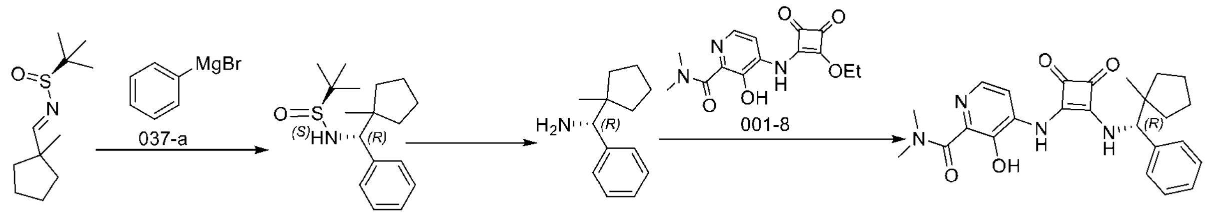


001-3 037-1 037-2 037

37.1 Synthesis of compound 037-1

001-3 (300 mg, 1.39 mmol) was dissolved in tetrahydrofuran (5 mL). The system was purged with nitrogen three times. At 0°C, phenylmagnesium bromide (302 mg, 1.67 mmol) was added dropwise to the reaction mixture. After the dropwise addition was completed, the reaction mixture was warmed to room temperature and reacted for 2 hours. The reaction mixture was poured into an aqueous ammonium chloride solution (20 mL) to quench, extracted with ethyl acetate (15 mL × 3), and the organic phases were combined, washed once with saturated brine (20 mL), then dried over anhydrous sodium sulfate, filtered, and the filtrate was concentrated under reduced pressure. The residue was purified by silica gel column chromatography to obtain compound 037-1 (346 mg, yield: 84.80%). LC-MS: $[M+H]^+$ = 294.05.

37.2 Synthesis of compound 037

The synthesis of compound 037 was carried out with reference to the synthesis method for compound 001 in Example 1, except that the starting material 001-9 was replaced with 037-1, to obtain compound 037 (80.1 mg, chiral purity: 61.2% (HPLC method C)).

$^1$H NMR (400 MHz, DMSO-$d_6$): δ 11.81 (s, 1H), 9.73 (s, 1H), 8.98 (s, 1H), 7.97 (s, 2H), 7.43 - 7.27 (m, 5H), 5.23 (d, *J* = 10.0 Hz, 1H), 3.21 - 3.02 (m, 6H), 1.70 - 1.63 (m, 5H), 1.41 - 1.36 (m, 1H), 1.24 (s, 1H), 1.15 - 1.12 (m, 1H), 0.94 (s, 3H); LC-MS: $[M+H]^+$ = 449.00.

Example 38. Synthesis of (*S*)-3-hydroxy-*N,N*-dimethyl-4-((2-((2-methyl-1-(1-methylcyclopentyl)propyl)amino)-3,4-dioxocyclobut-1-en-1-yl)amino)picolinamide (compound 038)

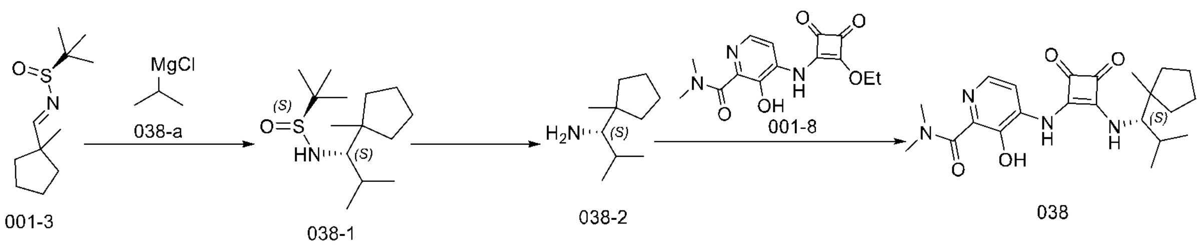


001-3 038-1 038-2 038

38.1 Synthesis of compound 038-1

001-3 (0.5 g, 2.32 mmol) was weighed into a reaction flask, and tetrahydrofuran (5 mL) was added. Under a nitrogen atmosphere at 0°C, isopropylmagnesium chloride (2.5 mL, 6.96 mmol) was added. The reaction was warmed to room temperature and stirred for 2 hours. The reaction mixture was quenched with saturated aqueous ammonium chloride solution (10 mL) and extracted with ethyl acetate (20 mL × 3). The combined organic phases were dried over anhydrous sodium sulfate, filtered, and the filtrate was concentrated under reduced pressure. The residue was purified by silica gel column chromatography to obtain compound 038-1 (330 mg, yield: 83%).

38.2 Synthesis of compound 038

The synthesis of compound 038 was carried out with reference to the synthesis method for compound 001 in Example 1, except that the starting material 001-9 was replaced with 038-1, to obtain compound 038 (56.6 mg, chiral purity: 100.0% (HPLC method D)).

$^1H$ NMR (400 MHz, DMSO-$d_6$): δ 11.79 (s, 1H), 9.76 (s, 1H), 8.31 (d, *J*= 12.0 Hz, 1H), 8.02 (s, 2H), 4.01 - 3.96 (m, 1H), 3.21 (s, 3H), 3.06 (s, 3H), 2.11 - 2.04 (m, 1H), 1.60-1.47 (m, 7H), 1.39-1.30 (m, 2H), 1.00 (s, 3H), 0.92 (dd, *J*= 12.0, 4.0 Hz, 6H); LC-MS: $[M+H]^+$ = 415.05.

Example 39. Synthesis of (*S*)-4-((2-((cyclopropyl(1-methylcyclopentyl)methyl)amino)-3,4-dioxocyclobut-1-en-1-yl)amino)-3-hydroxy-*N*,*N*-dimethylpicolinamide (compound 039)

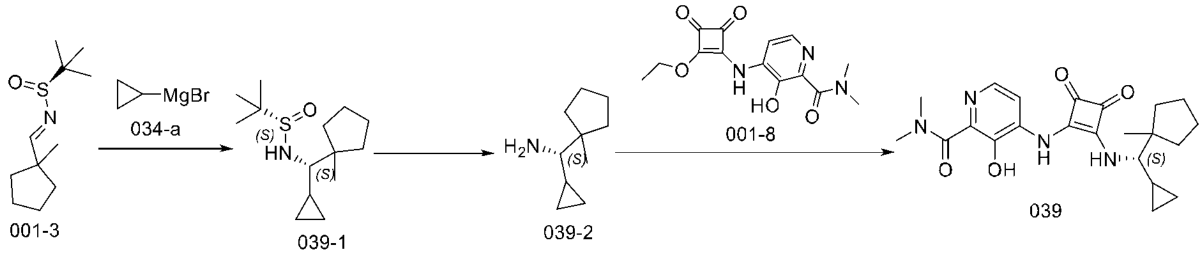


001-3 039-1 039-2 039

39.1 Synthesis of compound 039-1

At room temperature, compound 001-3 (0.3 g, 1.39 mmol) and tetrahydrofuran (6 mL) were added to a reaction flask. The system was purged with nitrogen three times. At -78°C, 034-a (1.0 M, 8.5 mL, 8.5 mmol) was added, and the reaction mixture was stirred at room temperature for 4 hours. The reaction mixture was quenched with aqueous ammonium chloride solution (10 mL) and extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 039-1 (403 mg). LC-MS: $[M+H]^+$ = 258.15.

39.2 Synthesis of compound 039

The synthesis of compound 039 was carried out with reference to the synthesis method for compound 001 in Example 1, except that the starting material 001-9 was replaced with 039-1, to obtain compound 039 (50.5 mg, chiral purity: 99.2% (HPLC method D)).

$^1H$ NMR (400 MHz, DMSO-$d_6$): δ 11.72 (s, 1H), 9.68 (s, 1H), 8.54 (d, *J* = 9.6 Hz, 1H), 8.02 (s, 2H), 3.48 (d, *J* =8.0 Hz, 1H), 3.19 (s, 3H), 3.05 (s, 3H), 1.63-1.60 (m, 6H), 1.41 - 1.36 (m, 1H), 1.32 (d, *J* = 6.0 Hz, 1H), 1.07 (s, 3H), 1.04 (d, *J* = 4.8 Hz, 1H), 0.65-0.62 (m, 1H), 0.48-0.43 (m, 1H), 0.35-0.31 (m, 1H), 0.22-0.18 (m, 1H); LC-MS: $[M+H]^+$ = 413.05.

Example 40. Synthesis of 3-hydroxy-4-((2-((2-hydroxy-2-methyl-1-(1-methylcyclopentyl)propyl)amino)-3,4-dioxocyclobut-1-en-1-yl)amino)-*N*,*N*-dimethylpicolinamide (compound 040)

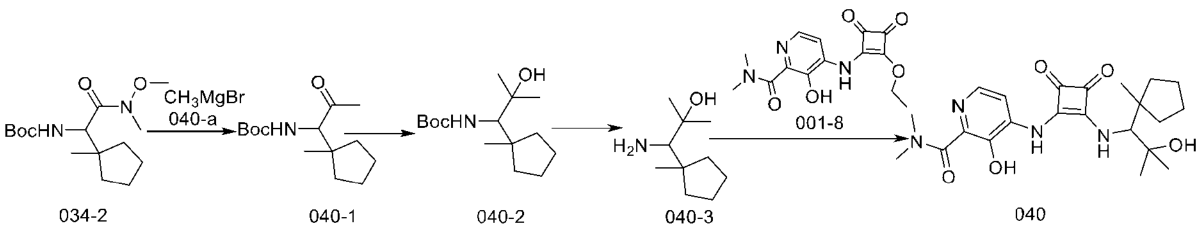


034-2 040-1 040-2 040-3 040

40.1 Synthesis of compound 040-1

At room temperature, 034-2 (300 mg, 1.0 mmol) was dissolved in tetrahydrofuran (5 mL). Under a nitrogen atmosphere at 0°C, a solution of methylmagnesium bromide in tetrahydrofuran (4 mL, 12.0 mmol) was added dropwise slowly. After the dropwise addition was completed, the reaction mixture was stirred at room temperature for 2 hours. The reaction mixture was quenched with saturated aqueous ammonium chloride solution (20 mL), diluted with water (10 mL), and extracted with ethyl acetate (20 mL × 3). The combined organic phases were washed with saturated brine (20 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 040-1 (140 mg, yield: 55.0%). LC-MS: $[M+H-t-Bu]^+$ = 200.10.

40.2 Synthesis of compound 040-2

At room temperature, 040-1 (140 mg, 0.55 mmol) was dissolved in tetrahydrofuran (1 mL). Under a nitrogen atmosphere at -78°C, a solution of methylmagnesium bromide in tetrahydrofuran (0.55 mL, 1.65 mmol) was added dropwise. After the dropwise addition was completed, the reaction mixture was stirred at room temperature for 1 hour. The reaction mixture was quenched with saturated aqueous ammonium chloride solution (10 mL), diluted with water (10 mL), and extracted with ethyl acetate (10 mL × 3). The combined organic phases were washed with saturated brine (10 mL), dried over anhydrous sodium sulfate, filtered, and the filtrate was concentrated under reduced pressure to obtain a crude product. The crude product was purified by silica gel column chromatography (petroleum ether/ethyl acetate = 5/1 to 3/1) to obtain compound 040-2 (100 mg, yield: 67.3%). LC-MS: $[M+H]^+$ = 272.10.

40.3 Synthesis of compound 040

The synthesis of compound 040 was carried out with reference to the synthesis method for compound 034 in Example 34, except that the starting material 034-3 was replaced with 040-2, to obtain compound 040 (56.5 mg, yield: 32.0%).

$^1$H NMR (400 MHz, DMSO-$d_6$): δ 11.73 (s, 1H), 9.95 (s, 1H), 8.78 (s, 1H), 8.22 - 7.86 (m, 2H), 4.68 (s, 1H), 3.95 - 3.84 (m, 1H), 3.18 (s, 3H), 3.04 (s, 3H), 1.73 - 1.51 (m, 4H), 1.42 - 1.40 (m, 4H), 1.27 (s, 3H), 1.13 (s, 3H), 1.07 (s, 3H); LC-MS: $[M+H]^+$ = 431.10.

Example 41. Synthesis of 3-hydroxy-N,N-dimethyl-4-((2-(((1R)-(1-methylcyclopentyl)(tetrahydrofuran-2-yl)methyl)amino)-3,4-dioxocyclobut-1-en-1-yl)amino)picolinamide (compound 041)

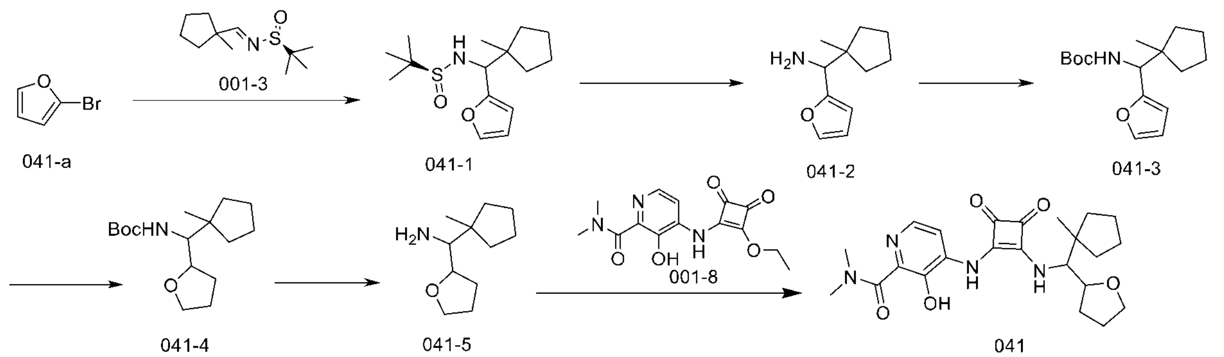

041-a 041-1 041-2 041-3
041-4 041-5 041

41.1 Synthesis of compound 041-1

The synthesis of compound 041-1 was carried out with reference to the synthesis method for compound 001-9 in Example 1, except that the starting material 001-e was replaced with 041-a, to obtain compound 041-1 (380 mg).

41.2 Synthesis of compound 041-2

The synthesis of compound 041-2 was carried out with reference to the synthesis method for compound 001-10 in Example 1, except that the starting material 001-9 was replaced with 041-1, to obtain compound 041-2 (350 mg, crude

product).

41.3 Synthesis of compound 041-3

At room temperature, 041-2 (350 mg, 1.95 mmol), triethylamine (395 mg, 3.9 mmol), and di-*tert*-butyl dicarbonate (511 mg, 2.34 mmol) were dissolved in dichloromethane (5 mL) and stirred at room temperature for 15 hours. The reaction mixture was directly concentrated under reduced pressure. The residue was purified by silica gel column chromatography to obtain compound 041-3 (280 mg). LC-MS: $[M+H-t-Bu]^+$ = 224.10.

41.4 Synthesis of compound 041-4

041-3 (200 mg, 0.72 mmol) was dissolved in methanol (2 mL). The system was purged with hydrogen three times. Wet palladium on carbon (50 mg, 10% Pd content) was added, and the reaction was carried out under a hydrogen atmosphere at 45°C for 3 hours. The reaction mixture was filtered and concentrated under reduced pressure. The residue was purified by silica gel column chromatography to obtain compound 041-4 (80 mg). LC-MS: $[M+H]^+$ = 284.10.

41.5 Synthesis of compound 041-5

At room temperature, 041-4 (40 mg, 0.14 mmol) was dissolved in a solution of hydrogen chloride in 1,4-dioxane (1 mL, 4 M) and stirred at room temperature for 1 hour. The reaction mixture was directly concentrated under reduced pressure to obtain compound 041-5 (25 mg, crude product).

41.6 Synthesis of compound 041

The synthesis of compound 041 was carried out with reference to the synthesis method for compound 001 in Example 1, except that the starting material 001-10 was replaced with 041-5, to obtain compound 041 (18.1 mg).

$^1$H NMR (400 MHz, DMSO-$d_6$): δ 11.75 (s, 1H), 9.67 (s, 1H), 8.40 (d, *J* = 10.4 Hz, 1H), 8.02 (s, 2H), 4.20 - 4.16 (m, 1H), 3.97 (q, *J* = 6.8 Hz, 1H), 3.72 - 3.65 (m, 1H), 3.65 - 3.58 (m, 1H), 3.22 (s, 3H), 3.06 (s, 3H), 2.03 - 1.90 (m, 1H), 1.86 - 1.78 (m, 2H), 1.69 - 1.53 (m, 6H), 1.50 - 1.42 (m, 2H), 1.33 - 1.26 (m, 1H), 0.99 (s, 3H); LC-MS: $[M+H]^+$ = 443.00.

Example 42. Synthesis of (*R*)-3-hydroxy-*N,N*-dimethyl-4-((2-(((3-methyl-5-(trifluoromethyl)pyridin-2-yl)(1-methylcyclopentyl)methyl)amino)-3,4-dioxocyclobut-1-en-1-yl)amino)picolinamide (compound 042)

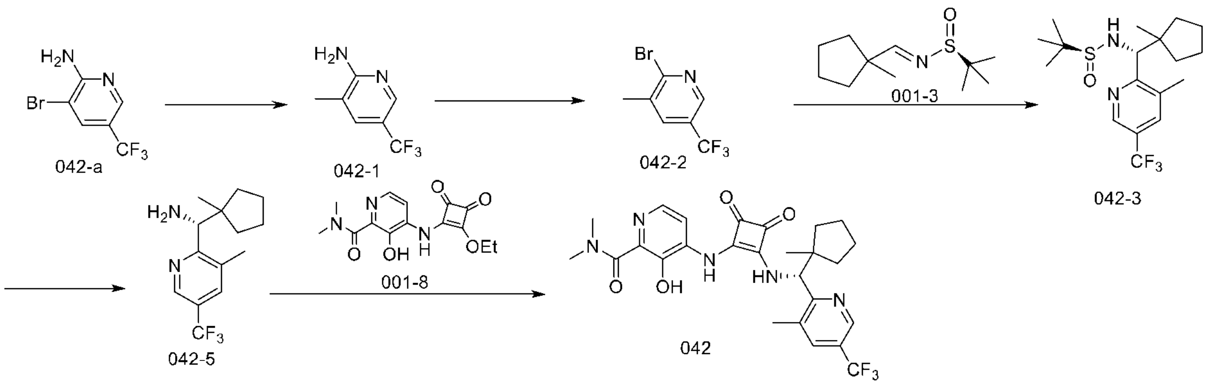


42.1 Synthesis of compound 042-1

042-a (1.5 g, 6.22 mmol) was dissolved in 1,4-dioxane (15 mL). Anhydrous potassium carbonate (2.6 g, 18.67 mmol), trimethylcyclotriboroxane (3.1 g, 24.90 mmol), and [1,1'-bis(diphenylphosphino)ferrocene]palladium(II) dichloride dichloromethane complex (200 mg, 0.25 mmol) were added. The reaction was carried out at 100°C under a nitrogen atmosphere for 5 hours. The reaction mixture was filtered through a pad of diatomite, and the filtrate was concentrated under reduced pressure. The residue was purified by silica gel column chromatography (petroleum ether/ethyl acetate = 2/1) to obtain compound 042-1 (655.0 mg). LC-MS: $[M+H]^+$ = 177.05.

42.2 Synthesis of compound 042-2

042-1 (655 mg, 3.72 mmol) was dissolved in a solution of hydrogen bromide in glacial acetic acid (5 mL, 33%) and water (1 mL). At -15°C, bromine (0.3 mL, 5.95 mmol) and an aqueous sodium nitrite solution (2 mL, 10.04 mmol, 5.02 M) were sequentially added dropwise. The reaction was carried out at room temperature for 3 hours. At -15°C, an aqueous potassium hydroxide solution (8 mL, 2.5 M) was added dropwise slowly. The reaction mixture was stirred at room temperature for 0.5 hours, then extracted with ethyl acetate (10 mL × 3). The organic phases were combined, washed sequentially with saturated aqueous sodium bicarbonate solution (10 mL × 3) and saturated aqueous sodium chloride solution (10 mL × 3), dried over anhydrous sodium sulfate, filtered, and the filtrate was concentrated under reduced pressure. The residue was purified by silica gel column chromatography (petroleum ether/ethyl acetate = 9/1) to obtain compound 042-2 (245.0 mg). LC-MS: $[M+H]^+$ = 239.90.

42.3 Synthesis of compound 042

The synthesis of compound 042 was carried out with reference to the synthesis method for compound 001 in Example 1, except that the starting material 001-e was replaced with 042-2, to obtain compound 042 (58.5 mg, chiral purity: 100% (HPLC method H)).

$^1$H NMR (400 MHz, DMSO-$d_6$): δ 9.37 (s, 1H), 8.86 (s, 1H), 8.12 (s, 1H), 7.97 (s, 2H), 5.83 (d, *J* = 10.0 Hz, 1H), 3.10 (d, *J* = 45.0 Hz, 6H), 1.74 (s, 2H), 1.59 (d, J= 16.8 Hz, 4H), 1.36 (d, *J* = 12.2 Hz, 1H), 1.23 (s, 1H), 1.01 (s, 3H). LC-MS: $[M+H]^+$ = 532.00.

Example 43. Synthesis of (*R*)-4-((2-(((2,6-difluorophenyl)(1-methylcyclopentyl)methyl)amino)-3,4-dioxocyclobut-1-en-1-yl)amino)-3-hydroxy-*N*,*N*-dimethylpicolinamide (compound 043)

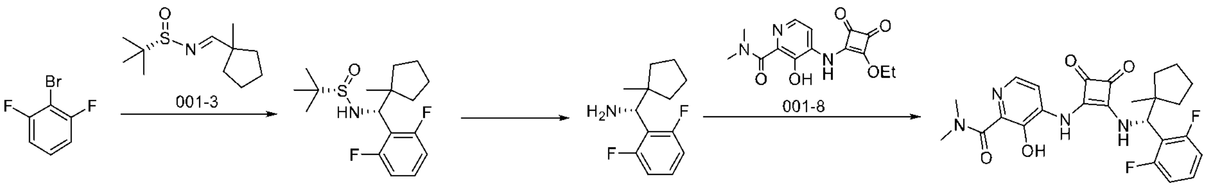


043-a 043-1 043-2 043

The synthesis of compound 043 was carried out with reference to the synthesis method for compound 001 in Example 1, except that the starting material 001-e was replaced with 043-a, to obtain compound 043 (14.3 mg, chiral purity: 73.0% (HPLC method F)).

$^1$H NMR (400 MHz, DMSO-$d_6$): δ 9.01 (s, 1H), 7.75 (d, *J* = 5.1 Hz, 1H), 7.60 (s, 1H), 7.40 (t, J= 7.6 Hz, 1H), 7.10 (t, *J= 9.2* Hz, 2H), 5.76 (d, *J*= 6.0 Hz, 1H), 2.97 (s, 6H), 1.63 (s, 6H), 1.37 (s, 1H), 1.19 (s, 1H), 0.97 (s, 3H). LC-MS: $[M+H]^+$ = 485.10.

Example 44. Synthesis of 3-(((*R*)-(5-fluoropyridin-2-yl)(1-methylcyclopentyl)methyl)amino)-4-((3-hydroxy-2-(3-methyl-3,8-diazabicyclo[3.2.1]octane-8-carbonyl)pyridin-4-yl)amino)cyclobut-3-ene-1,2-dione (compound 044)

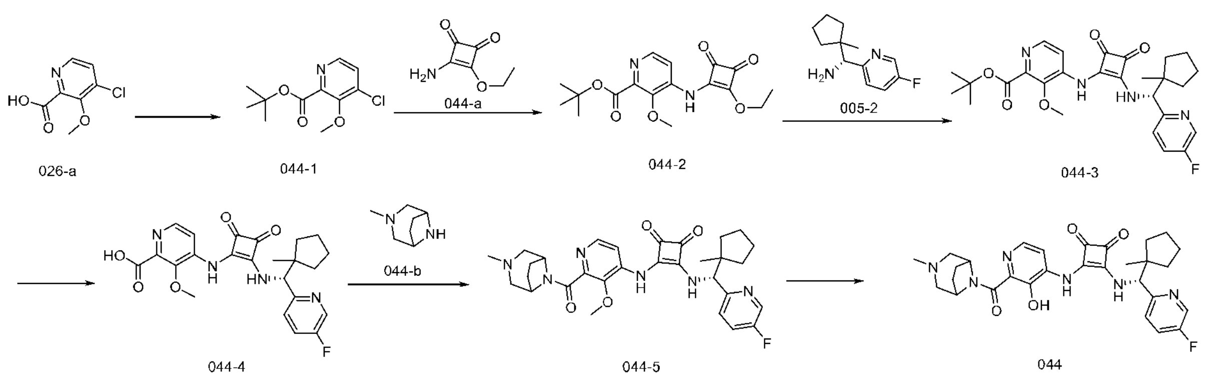


026-a 044-1 044-2 044-3 044-4 044-5 044

44.1 Synthesis of compound 044-1

026-a (3.0 g, 15.99 mmol) was dissolved in tetrahydrofuran (15 mL). Di-*tert*-butyl dicarbonate (6.9 g, 31.61 mmol), 4-dimethylaminopyridine (585 mg, 4.78 mmol), and *tert*-butanol (30 mL) were added. The reaction was carried out at room temperature overnight. The solvent was removed by concentration under reduced pressure, and the residue was purified by silica gel column chromatography (petroleum ether/ethyl acetate = 4/1) to obtain compound 044-1 (2.4 g). LC-MS: $[M+H]^+$ = 244.05.

44.2 Synthesis of compound 044-2

044-1 (2.5 g, 10.26 mmol) was dissolved in tetrahydrofuran (15 mL). Potassium carbonate (3.3 g, 30.78 mmol), 2-di-*tert*-butylphosphino-2',4',6'-triisopropylbiphenyl (500 mg, 1.18 mmol), methanesulfonato(2-di-t-butylphosphino-2',4',6'-tri-i-propyl-1,1'-biphenyl)(2'-amino-1,1'-biphenyl-2-yl)palladium(II) (500 mg, 0.63 mmol), and compound 044-a (6.0 g, 41.04 mmol) were added. The reaction was carried out at 80°C overnight under a nitrogen atmosphere. The reaction mixture was filtered through a pad of diatomite, and the filtrate was concentrated under reduced pressure. The residue was purified by silica gel column chromatography (petroleum ether/ethyl acetate = 3/1) to obtain compound 044-2 (850.0 mg). LC-MS: $[M+H]^+$ = 439.20.

44.3 Synthesis of compound 044-3

044-2 (500 mg, 1.44 mmol) was dissolved in ethanol (10 mL). *N,N*-Diisopropylethylamine (0.75 mL, 4.31 mmol) and 005-2 (575 mg, crude product) were added, and the reaction was carried out at room temperature for 2 hours. The solvent was removed by concentration under reduced pressure, and the residue was purified by silica gel column chromatography (petroleum ether/ethyl acetate = 3/2) to obtain compound 044-3 (570.0 mg). LC-MS: $[M+H]^+$ = 511.20.

44.4 Synthesis of compound 044-4

044-3 (570 mg, 1.12 mmol) was dissolved in dichloromethane (5 mL). Trifluoroacetic acid (5 mL) was added dropwise slowly, and the reaction mixture was stirred at room temperature overnight. The solvent was removed by concentration under reduced pressure to obtain compound 044-4 (600.0 mg, crude product). The crude product was directly used in the next step. LC-MS: $[M+H]^+$ = 455.10.

44.5 Synthesis of compound 044-5

044-4 (450 mg, crude product) was weighed and dissolved in *N,N*-dimethylformamide (5 mL). *N*,*N*,*N*',*N*'-Tetramethyl-*O*-(7-azabenzotriazol-1-yl)uronium hexafluorophosphate (565 mg, 1.49 mmol), triethylamine (301 mg, 2.97 mmol), and compound 044-b (150 mg, 1.19 mmol) were added. The reaction was carried out at room temperature for 3 hours. Water (10 mL) was added to the reaction mixture for dilution, and the mixture was extracted with ethyl acetate (10 mL $\times$ 3). The organic phases were combined, washed with saturated aqueous sodium chloride solution (10 mL $\times$ 3), dried over anhydrous sodium sulfate, filtered, and the filtrate was concentrated under reduced pressure. The residue was purified by silica gel column chromatography (methanol/dichloromethane = 1/12) to obtain compound 044-5 (190.0 mg). LC-MS: $[M+H]^+$ = 563.25.

44.6 Synthesis of compound 044

044-5 (190 mg, 0.34 mmol) was dissolved in dichloromethane (1 mL). Under a nitrogen atmosphere at 0°C, a solution of boron tribromide in dichloromethane (15 mL, 1 M) was added, and the reaction was carried out at room temperature overnight. At 0°C, methanol (5 mL) was added to the reaction mixture to quench the reaction. The reaction mixture was then concentrated under reduced pressure. The residue was purified by preparative HPLC and lyophilized to obtain compound 044 (24.0 mg, chiral purity: 99.5% (HPLC method H)).

$^1$H NMR (400 MHz, DMSO-$d_6$): $\delta$ 13.72 (s, 1H), 10.10 (s, 1H), 9.38 (d, *J* = 10.2 Hz, 1H), 8.65 (d, *J* = 2.8 Hz, 1H), 8.07 (t, *J* = 8.4 Hz, 2H), 7.75 (td, *J* = 8.8, 2.9 Hz, 1H), 7.50 (dd, *J* = 8.6, 4.4 Hz, 1H), 5.83 (s, 1H), 5.38 (d, *J* = 10.0 Hz, 1H), 4.78 (s, 1H), 2.76 (d, *J* = 10.8 Hz, 2H), 2.22 (d, *J* = 10.4 Hz, 2H), 2.18 (s, 3H), 1.91 (s, 3H), 1.76 (dd, *J* = 19.8, 7.8 Hz, 3H), 1.61 (s, 4H), 1.37 - 1.27 (m, 1H), 1.18 - 1.06 (m, 1H), 0.94 (s, 3H). LC-MS: $[M+H]^+$ = 549.15.

Example 45. Synthesis of (*R*)-4-((2-(((6-chloro-3-methylpyridin-2-yl)(1-methylcyclopentyl)methyl)amino)-3,4-dioxocyclobut-1-en-1-yl)amino)-3-hydroxy-*N*,*N*-dimethylpicolinamide (compound 045)

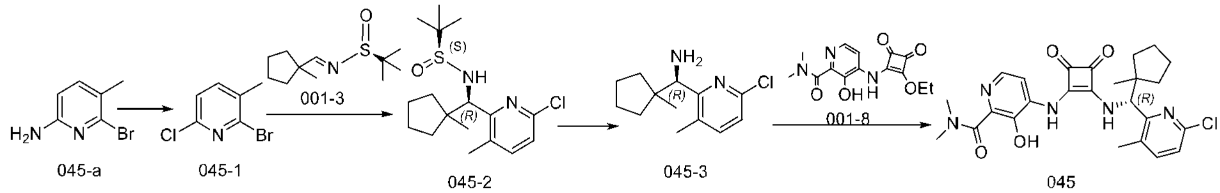

045-a 045-1 045-2 045-3 045

45.1 Synthesis of compound 045-1

045-a (940 mg, 5.03 mmol) was dissolved in concentrated hydrochloric acid (10 mL). At - 20°C, sodium nitrite (690 mg, 10.00 mmol) was added in three portions. After the addition was completed, the temperature was raised to room temperature, and the reaction mixture was stirred for another 1 hour. The reaction mixture was cooled to -10°C, and a 2 M sodium hydroxide solution was slowly added to adjust the pH to 11. After warming to room temperature, water (10 mL) was added for dilution. The mixture was extracted with ethyl acetate (10 mL × 3). The organic phases were combined, washed with saturated aqueous sodium chloride solution (10 mL), dried over anhydrous sodium sulfate, filtered, and the filtrate was concentrated under reduced pressure. The residue was purified by silica gel column chromatography (petroleum ether/ethyl acetate = 9/1) to obtain compound 045-1 (595 mg, yield: 57.6%).

45.2 Synthesis of compound 045

The synthesis of compound 045 was carried out with reference to the synthesis method for compound 001 in Example 1, except that the starting material 001-e was replaced with 045-1, to obtain compound 045 (59.5 mg, yield: 36%, chiral purity: 99.8% (HPLC method H)).

$^1$H NMR (400 MHz, DMSO-$d_6$) δ: 9.18 (d, *J* = 9.6 Hz, 1H), 7.96 (d, *J* = 5.6 Hz, 1H), 7.91 (s, 1H), 7.71 (d, *J* = 8.4 Hz, 1H), 7.36 (d, *J* = 8.4 Hz, 1H), 5.71 (d, *J* = 10.0 Hz, 1H), 3.14 (s, 3H), 3.03 (s, 3H), 2.39 (s, 3H), 1.75-1.62 (m, 2H), 1.64-1.52 (m, 4H), 1.37-1.32 (m, 1H), 1.23-1.17 (m, 1H), 1.00 (s, 3H). LC-MS: [M+H]$^+$ = 498.10.

Example 46. Synthesis of (*S*)-3-hydroxy-*N*,*N*-dimethyl-4-((2-((1-(1-methylcyclopentyl)-2-phenylethyl)amino)-3,4-dioxocyclobut-1-en-1-yl)amino)picolinamide (compound 046)

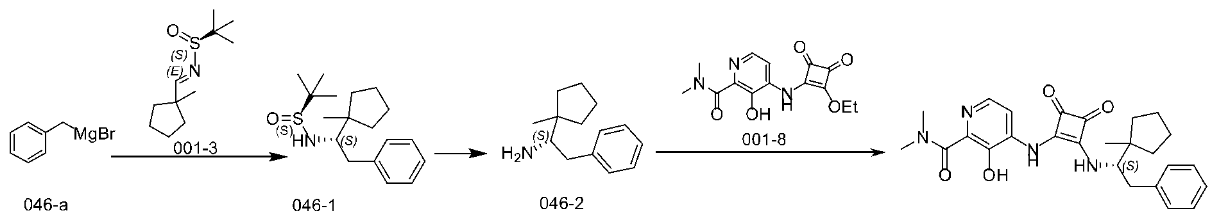

046-a 046-1 046-2 046

46.1 Synthesis of compound 046-1

001-3 (200 mg, 0.93 mmol) was dissolved in dichloromethane (5 mL). The system was purged with nitrogen three times. At -78°C, benzylmagnesium bromide reagent (046-a, 2.79 mL, 2.79 mmol, 1 M) was added dropwise to the reaction mixture. After the dropwise addition was completed, the reaction was carried out at room temperature for 1 hour. The reaction mixture was poured into saturated aqueous ammonium chloride solution (20 mL) to quench, then extracted with ethyl acetate (25 mL × 3). The organic phases were combined, washed once with saturated brine (30 mL), dried over anhydrous sodium sulfate, filtered, and the filtrate was concentrated under reduced pressure. The residue was purified by silica gel column chromatography to obtain compound 046-1 (195 mg, yield: 62.14%). LC-MS: [M+H]$^+$ = 308.15.

46.2 Synthesis of compound 046

The synthesis of compound 046 was carried out with reference to the synthesis method for compound 001 in Example 1, except that the starting material 001-9 was replaced with 046-1, to obtain compound 046. (23 mg, chiral purity: 63.7% (HPLC method D)).

$^1$H NMR (400 MHz, DMSO-*d*$_6$): δ 8.57 (d, *J* = 10.0 Hz, 1H), 7.86 (d, *J* = 5.4 Hz, 1H), 7.76 (s, 1H), 7.24 - 7.14 (m, 4H), 7.11 (t, *J* = 6.8 Hz, 1H), 4.17 (t, *J* = 10.8 Hz, 1H), 3.02 (d, *J* = 16.4 Hz, 6H), 2.95 (s, 1H), 2.56 (d, *J*= 13.8 Hz, 1H), 1.70 - 1.49 (m, 6H), 1.46 - 1.39 (m, 1H), 1.33 - 1.27 (m, 1H), 1.03 (s, 3H). LC-MS: [M+H]$^+$ = 463.15.

Example 47. Synthesis of (*R*)-4-((2-(((5-chloro-3-methylpyridin-2-yl)(1-methylcyclopentyl)methyl)amino)-3,4-dioxocyclobut-1-en-1-yl)amino)-3-hydroxy-*N*,*N*-dimethylpicolinamide (compound 047)

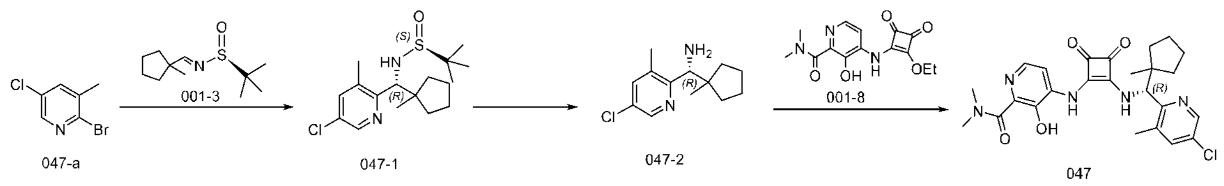


The synthesis of compound 047 was carried out with reference to the synthesis method for compound 001 in Example 1, except that the starting material 001-e was replaced with 047-a, to obtain compound 047 (46.3 mg, chiral purity: 100% (HPLC method P)).

$^1$H NMR (400 MHz, DMSO-*d*$_6$): δ 9.30 (d, *J* = 9.6 Hz, 1H), 8.50 (d, *J* = 1.6 Hz, 1H), 7.96 (d, *J* = 5.2 Hz, 1H), 7.90 (d, *J* = 4.8 Hz, 1H), 7.81 (d, *J* = 1.6 Hz, 1H), 5.70 (d, *J* = 10.0 Hz, 1H), 3.12 (s, 3H), 3.02 (s, 3H), 2.41 (s, 3H), 1.73-1.65 (m, 2H), 1.58-1.49 (m, 4H), 1.33-1.30 (m, 1H), 1.22-1.19 (m, 1H), 0.98 (s, 3H). LCMS: [M+H]$^+$ = 497.95.

Example 48. Synthesis of (R)-4-((2-(((4-chloro-3-methylpyridin-2-yl)(1-methylcyclopentyl)methyl)amino)-3,4-dioxocyclobut-1-en-1-yl)amino)-3-hydroxy-*N*,*N*-dimethylpicolinamide (compound 048)

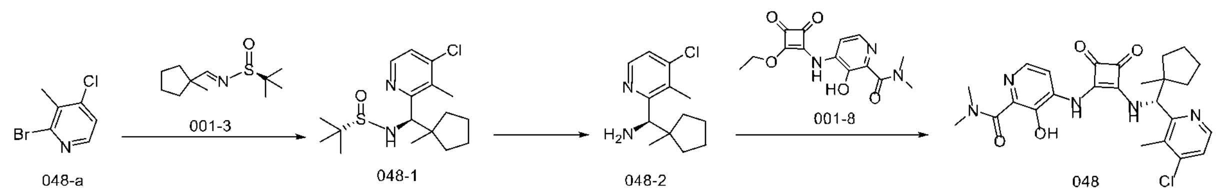


The synthesis of compound 048 was carried out with reference to the synthesis method for compound 001 in Example 1, except that the starting material 001-e was replaced with 048-a, to obtain compound 048 (28.1 mg, chiral purity: 100% (HPLC method H)).

$^1$H NMR (400 MHz, DMSO-*d*$_6$): δ 9.34 (d, *J* = 10.0 Hz, 1H), 8.44 (d, *J* = 5.2 Hz, 1H), 7.96 (d, *J* = 5.2 Hz, 1H), 7.91 (s, 1H), 7.49 (d, *J* = 5.2 Hz, 1H), 5.86 (d, *J* = 10.0 Hz, 1H), 3.13 (s, 3H), 3.03 (s, 3H), 2.45 (s, 3H), 1.78 - 1.66 (m, 2H), 1.63 - 1.48 (m, 4H), 1.36 - 1.28 (m, 1H), 1.20 (d, *J* = 4.4 Hz, 1H), 0.97 (s, 3H).

Example 49. Synthesis of (*R*)-4-((2-(((2-fluoro-5-methylpyrimidin-4-yl)(1-methylcyclopentyl)methyl)amino)-3,4-dioxocyclobut-1-en-1-yl)amino)-3-hydroxy-*N*,*N*-dimethylpicolinamide (compound 049)

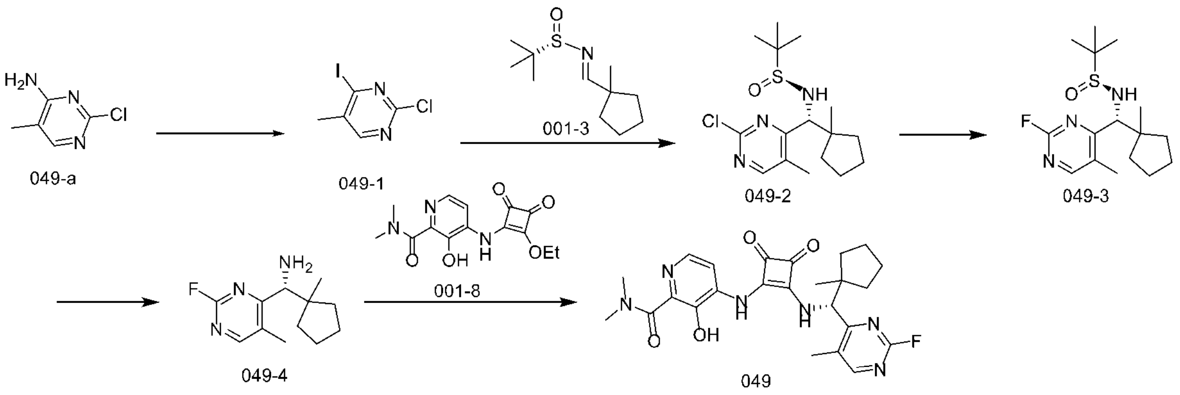


049-a 049-1 049-2 049-3 049-4 049

49.1 Synthesis of compound 049-1

049-a (2.0 g, 13.9 mmol) was weighed into a reaction flask. Acetonitrile (20 mL) and copper(I) iodide (4.0 g, 20.9 mmol) were added. At 0°C, *tert*-butyl nitrite (2.2 g, 20.9 mmol) was slowly added. After the addition was completed, the reaction mixture was heated to 70°C and reacted for 4 hours. The reaction mixture was added with water (50 mL) and extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 049-1 (1.5 g, yield: 42%).

49.2 Synthesis of compound 049-2

At room temperature, 049-1 (0.6 g, 2.36 mmol) and tetrahydrofuran (5 mL) were added to a reaction flask. The system was purged with nitrogen three times. At -78°C, a solution of isopropylmagnesium chloride-lithium chloride complex (3.6 mL, 4.72 mmol, 1.3 M) was added, and the mixture was stirred at -78°C for 1 hour. Subsequently, 001-3 (0.63 g, 2.91 mmol) was added, and the reaction mixture was stirred at -78°C for 3 hours, then warmed to room temperature and stirred for another 12 hours. The reaction mixture was quenched with saturated aqueous ammonium chloride solution (10 mL) and extracted with ethyl acetate (20 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 049-2 (300 mg). LC-MS: $[M+H]^+$ = 344.10.

49.3 Synthesis of compound 049-3

049-2 (0.3 g, 0.872 mmol) was weighed into a reaction flask. Dimethyl sulfoxide (3 mL) and potassium fluoride (1.0 g, 17.45 mmol) were added. The reaction mixture was stirred at 130°C overnight. Then, water (20 mL) was added for dilution, and the mixture was extracted with ethyl acetate (20 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 049-3 (120 mg, yield: 42%). LC-MS: $[M+H]^+$ = 328.15.

49.4 Synthesis of compound 049

The synthesis of compound 049 was carried out with reference to the synthesis method for compound 001 in Example 1, except that the starting material 001-9 was replaced with 049-3, to obtain compound 049 (15.5 mg, yield: 11%, chiral purity: 98.2% (HPLC method H)).

$^1$H NMR (400 MHz, DMSO-$d_6$): δ 10.88 (d, *J* = 672.0 Hz, 1H), 9.26 (s, 1H), 8.67 (s, 1H), 7.97 (s, 2H), 5.69 (d, *J* = 12.0 Hz, 1H), 3.17 (s, 3H), 3.05 (s, 3H), 2.38 (s, 3H), 1.74-1.57 (m, 6H), 1.42 - 1.35 (m, 1H), 1.29 - 1.21 (m, 2H), 1.02 (s, 3H). LC-MS: $[M+H]^+$ = 483.15.

Example 50. Synthesis of (*R*)-4-((2-((cyano(1-methylcyclopentyl)methyl)amino)-3,4-dioxocyclobut-1-en-1-yl)amino)-3-hydroxy-*N*,*N*-dimethylpicolinamide (compound 050)

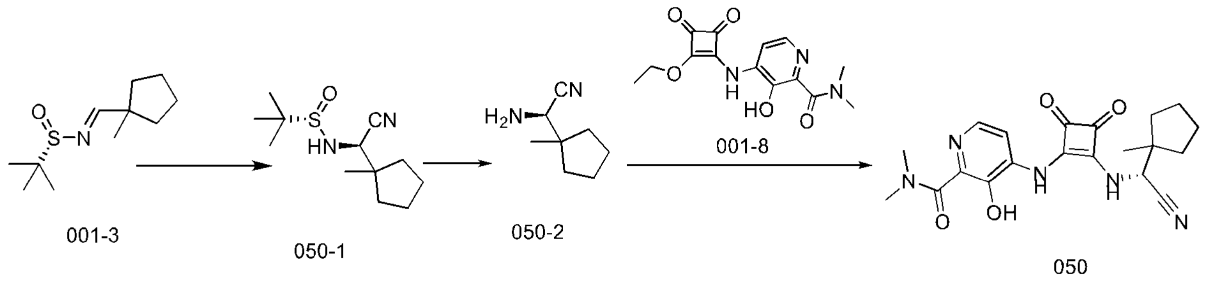

50.1 Synthesis of compound 050-1

001-3 (200 mg, 0.93 mmol) was dissolved in tetrahydrofuran (2 mL). Cesium fluoride (564 mg, 3.71 mmol) and trimethylsilyl cyanide (369 mg, 3.71 mmol) were added, and the reaction was carried out at 40°C for 1 hour. The reaction mixture was poured into water (10 mL) to quench, then extracted with ethyl acetate (15 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 050-1 (210 mg, yield: 83.96%). LC-MS: $[M+H]^+$ = 243.10.

50.2 Synthesis of compound 050

The synthesis of compound 050 was carried out with reference to the synthesis method for compound 001 in Example 1, except that the starting material 001-9 was replaced with 050-1, to obtain compound 050 (68.5 mg, chiral purity: 97.8% (HPLC method D)).

$^1$H NMR (400 MHz, DMSO-$d_6$): δ 9.14 (s, 1H), 7.95 (s, 2H), 5.13 (s, 1H), 3.12 (s, 3H), 3.02 (s, 3H), 1.73 - 1.57 (m, 6H), 1.49 - 1.38 (m, 2H), 1.14 (s, 3H). LC-MS: $[M+H]^+$ = 398.05.

Example 51. Synthesis of (*R*)-4-((2-(((3,6-dimethylpyridin-2-yl)(1-methylcyclopentyl)methyl)amino)-3,4-dioxocyclobut-1-en-1-yl)amino)-3-hydroxy-*N*,*N*-dimethylpicolinamide (compound 051)

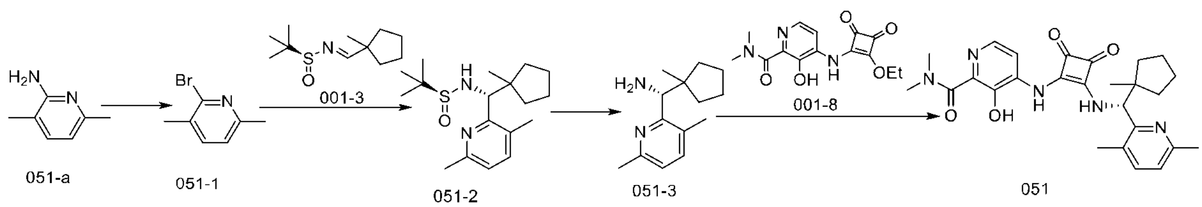

51.1 Synthesis of compound 051-1

051-a (200 mg, 1.64 mmol) was weighed into a reaction flask. A solution of hydrogen bromide in acetic acid (4 mL, 33%) and water (1 mL) was added. At -15°C, a solution of bromine (0.13 mL, 2.62 mmol) and sodium nitrite (305 mg, 4.42 mmol) in water (0.5 mL) was slowly added dropwise to the reaction flask. The reaction was carried out at room temperature for 2 hours. The reaction was quenched with an aqueous solution (15 mL) of potassium hydroxide (551 mg, 9.82 mmol) and stirred at room temperature for 0.5 hours. The mixture was then extracted with ethyl acetate (10 mL × 3). The organic phases were combined, washed sequentially with saturated aqueous sodium bicarbonate solution and saturated brine (10 mL × 3), dried over anhydrous sodium sulfate, filtered, and the filtrate was concentrated under reduced pressure. The residue was purified by silica gel column chromatography (petroleum ether/ethyl acetate = 3/1) to obtain compound 051-1 (100 mg). LC-MS: $[M+H]^+$ = 187.95.

51.1 Synthesis of compound 051

The synthesis of compound 051 was carried out with reference to the synthesis method for compound 001 in Example 1, except that the starting material 001-e was replaced with 051-1, to obtain compound 051 (15.8 mg, chiral purity: 99.7% (HPLC method H)).

$^1$H NMR (400 MHz, DMSO-$d_6$): δ 9.14 (s, 1H), 7.92 (s, 2H), 7.46 (d, *J* = 7.8 Hz, 1H), 7.06 (d, *J* = 7.8 Hz, 1H), 5.69 (d,

*J* = 10.2 Hz, 1H), 3.14 (s, 3H), 3.02 (s, 3H), 2.47 (s, 3H), 2.32 (s, 3H), 1.73 (ddd, *J* = 24.8, 17.3, 7.7 Hz, 2H), 1.54 (dt, *J* = 19.4, 9.3 Hz, 4H), 1.36 - 1.26 (m, 1H), 1.21 - 1.12 (m, 1H), 0.98 (s, 3H). LC-MS: $[M+H]^+$ = 478.05.

Example 52. Synthesis of 3-hydroxy-*N,N*-dimethyl-4-((2-(((1-methylcyclopentyl)(5-methylpyrimidin-4-yl)methyl)amino)-3,4-dioxocyclobut-1-en-1-yl)amino)picolinamide (compound 052)

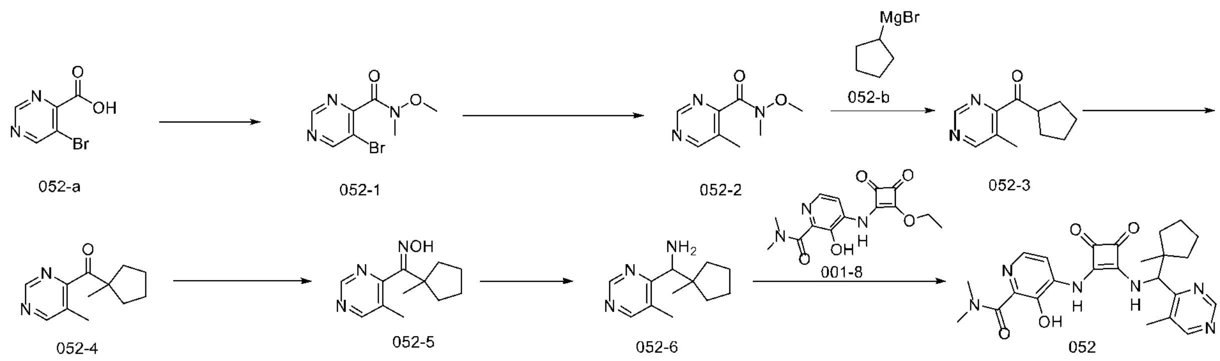


52.1 Synthesis of compound 052-1

At room temperature, 052-a (2.5 g, 12.3 mmol), *N,O*-dimethylhydroxylamine hydrochloride (2.4 g, 24.6 mmol), and *N,N*-diisopropylethylamine (4.8 g, 36.9 mmol) were added to dichloromethane (30 mL). At room temperature, propylphosphonic anhydride (11.8 g, 18.5 mmol, 50% solution in ethyl acetate) was added dropwise slowly. After the dropwise addition was completed, the reaction mixture was stirred at room temperature for 0.5 hours. The reaction mixture was diluted with water (20 mL) and then extracted with dichloromethane (20 mL × 3). The organic phases were combined, dried over anhydrous sodium sulfate, filtered, and the filtrate was concentrated under reduced pressure. The residue was purified by silica gel column chromatography (dichloromethane/methanol = 30/1 to 20/1) to obtain compound 052-1 (2.8 g, yield: 92.7%). LC-MS: $[M+H]^+$ = 247.95.

52.2 Synthesis of compound 052-2

At room temperature, 052-1 (2.8 g, 11.4 mmol), trimethylcyclotriboroxane (5.7 g, 22.8 mmol, 50% solution in tetrahydrofuran), 1,1'-bis(diphenylphosphino)ferrocene palladium(II) dichloride (804 mg, 0.11 mmol), and cesium carbonate (7.4 g, 22.8 mmol) were added to 1,4-dioxane (30 mL) and water (30 mL). After purging with nitrogen three times, the reaction mixture was stirred at 90°C for 4 hours. The reaction mixture was directly concentrated under reduced pressure. The residue was purified by silica gel column chromatography (petroleum ether/ethyl acetate = 3/1 to 1/1) to obtain compound 052-2 (1.8 g, yield: 86.8%). LC-MS: $[M+H]^+$ = 182.10.

52.3 Synthesis of compound 052-3

052-2 (1.8 g, 9.9 mmol) was added to tetrahydrofuran (20 mL). At -78°C, a solution of cyclopropylmagnesium bromide in tetrahydrofuran (10.9 mL, 1 M) was added. After the addition was completed, the reaction mixture was stirred at room temperature for 1 hour. A saturated aqueous ammonium chloride solution (20 mL) was added to the reaction mixture, and the mixture was extracted with ethyl acetate (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 052-3 (550 mg, yield: 29.3%). LC-MS: $[M+H]^+$ = 191.10.

52.4 Synthesis of compound 052-4

052-3 (500 mg, 2.6 mmol) and iodomethane (740 mg, 5.2 mmol) were added to tetrahydrofuran (20 mL). At room temperature, potassium tert-butoxide (580 mg, 5.2 mmol) was added. After the addition was completed, the reaction mixture was stirred at room temperature for 0.5 hours. The reaction mixture was added with water (20 mL) and then extracted with ethyl acetate (20 mL × 3). The organic phases were combined, dried over anhydrous sodium sulfate, filtered, and the filtrate was concentrated under reduced pressure. The residue was purified by silica gel column

chromatography (petroleum ether/ethyl acetate = 5/1 to 3/1) to obtain compound 052-4 (400 mg, yield: 75.4%). LC-MS: $[M+H]^+$ = 205.05.

52.5 Synthesis of compound 052-5

052-4 (180 mg, 0.88 mmol), hydroxylamine hydrochloride (243 mg, 3.52 mmol), and sodium acetate (289 mg, 3.52 mmol) were added to ethanol (4 mL). The reaction was stirred at 80°C for 1 hour. The reaction mixture was diluted with water (10 mL) and then extracted with ethyl acetate (10 mL × 3). The organic phases were combined, dried over anhydrous sodium sulfate, filtered, and the filtrate was concentrated under reduced pressure to obtain compound 052-5 (180 mg, crude product). LC-MS: $[M+H]^+$ = 220.10.

52.6 Synthesis of compound 052-6

052-5 (120 mg, 0.55 mmol) and zinc powder (280 mg, 4.40 mmol) were added to methanol (6 mL). At room temperature, acetic acid (490 mg, 8.20 mmol) was added. After the addition was completed, the reaction was stirred at 50°C for 2 hours. To the reaction mixture, a saturated aqueous sodium bicarbonate solution was added to adjust the pH to 8-9, and then the mixture was extracted with ethyl acetate (10 mL × 3). The organic phases were combined, dried over anhydrous sodium sulfate, filtered, and the filtrate was concentrated under reduced pressure to obtain compound 052-6 (80 mg, crude product). LC-MS: $[M+H]^+$ = 206.15.

52.7 Synthesis of compound 052

052-6 (80 mg, 0.39 mmol), 001-8 (120 mg, 0.39 mmol), and *N,N*-diisopropylethylamine (155 mg, 1.20 mmol) were added to ethanol (5 mL), and the reaction mixture was stirred at room temperature for 2 hours. The reaction mixture was purified by preparative HPLC (basic conditions) and lyophilized to obtain compound 052 (50.5 mg, yield: 28.2%).

$^1$H NMR (600 MHz, DMSO-$d_6$): δ 11.62 (s, 1H), 10.02 (s, 1H), 9.31 (s, 1H), 9.07 (s, 1H), 8.64 (s, 1H), 8.16 - 7.74 (m, 2H), 5.67 (d, *J* = 9.6 Hz, 1H), 3.13 (s, 3H), 3.01 (s, 3H), 2.35 (s, 3H), 1.73 - 1.67 (m, 2H), 1.55 -1.52 (m, 4H), 1.37 - 1.30 (m, 1H), 1.21 (d, *J* = 6.0 Hz, 1H), 0.98 (s, 3H). LC-MS: $[M+H]^+$ = 465.00.

Example 53. Synthesis of (*R*)-4-((2-(((6-chloro-3-fluoropyridin-2-yl)(1-methylcyclopentyl)methyl)amino)-3,4-dioxocyclobut-1-en-1-yl)amino)-3-hydroxy-*N*,*N*-dimethylpicolinamide (compound 053)

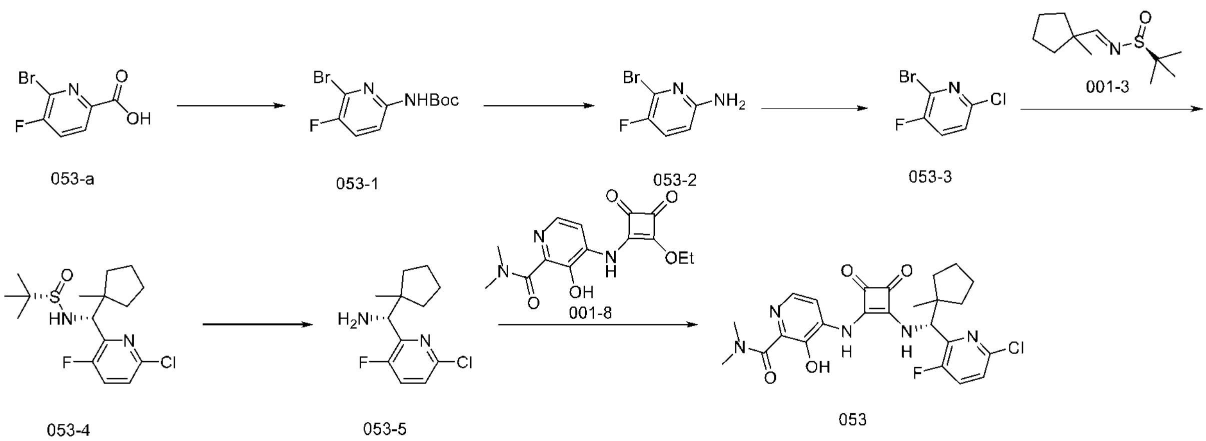


053-a 053-1 053-2 053-3

053-4 053-5 053

53.1 Synthesis of compound 053-1

At room temperature, 053-a (3 g, 13.6 mmol), diphenylphosphoryl azide (4 g, 14.5 mmol), N,N-diisopropylethylamine (2 g, 15.5 mmol), and *tert*-butanol (90 mL) were added to a reaction flask. The system was purged with nitrogen three times, and the reaction was carried out at 100°C for 5 hours. The reaction mixture was directly concentrated under reduced pressure. The residue was purified by silica gel column chromatography to obtain compound 053-1 (3.1 g). LC-MS: $[M+H]^+$ = 236.90.

53.2 Synthesis of compound 053-2

At room temperature, 053-1 (3.1 g, 10.65 mmol) was dissolved in a solution of hydrogen chloride in ethanol (30 mL, 4.0 M) and stirred at room temperature for 2 hours. The reaction mixture was directly concentrated under reduced pressure to obtain compound 053-2 (2.3 g). LC-MS: $[M+H]^+$ = 192.95.

53.3 Synthesis of compound 053-3

At room temperature, 053-2 (2.3 g, 12.0 mmol) was dissolved in concentrated hydrochloric acid (25 mL). At -20°C, sodium nitrite (1.5 g, 21.7 mmol) was slowly added, and the reaction mixture was stirred at room temperature for 2 hours. The reaction mixture was adjusted to pH = 11 with 10.0 M sodium hydroxide solution and extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 053-3 (1.0 g). LC-MS: $[M+H]^+$ = 211.85.

53.4 Synthesis of compound 053

The synthesis of compound 053 was carried out with reference to the synthesis method for compound 001 in Example 1, except that the starting material 001-e was replaced with 053-3, to obtain compound 053 (23.1 mg, chiral purity: 96.4% (HPLC method H)).

$^1$H NMR (400 MHz, DMSO-*d*$_6$): δ 11.72 (s, 1H), 10.06 (s, 1H), 9.23 (s, 1H), 7.96 - 7.87 (m, 3H), 7.58 (dd, *J* = 4.0, 4.0 Hz, 1H), 5.76 (d, *J* = 12.0 Hz, 1H), 3.17 (s, 3H), 3.05 (s, 3H), 1.74 -1.71 (m, 1H), 1.66 -1.57 (m, 5H), 1.36 - 1.28 (m, 1H), 1.22-1.16 (m, 1H), 0.99 (s, 3H). LC-MS: $[M+H]^+$ = 501.95.

Example 54. Synthesis of (*R*)-3-hydroxy-*N*,*N*-dimethyl-4-((2-(((1-methylcyclopentyl)(4-methylpyrimidin-5-yl)methyl) amino)-3,4-dioxocyclobut-1-en-1-yl)amino)picolinamide (compound 054)

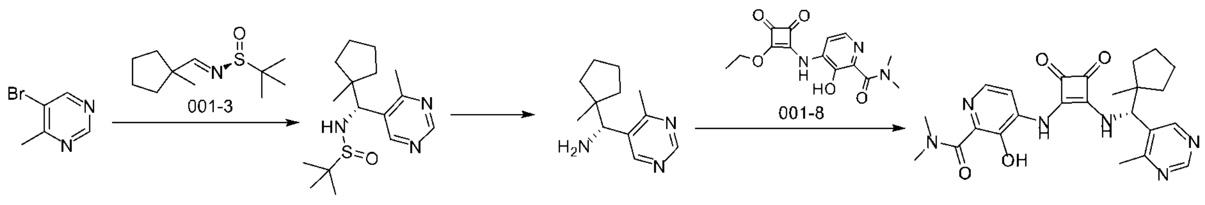


054-a 054-1 054-2 054

The synthesis of compound 054 was carried out with reference to the synthesis method for compound 001 in Example 1, except that the starting material 001-e was replaced with 054-a, to obtain compound 054 (11.8 mg, chiral purity: 100.0% (HPLC method L)).

$^1$H NMR (400 MHz, DMSO-*d*$_6$): δ 11.77 (s, 1H), 9.69 (s, 1H), 8.95 (s, 1H), 8.66 (s, 1H), 7.99 (s, 1H), 7.94 (s, 1H), 5.54 (d, *J* = 8.8 Hz, 1H), 3.16 (s, 3H), 3.05 (s, 3H), 2.62 (s, 3H), 1.71 - 1.57 (m, 6H), 1.52 - 1.44 (m, 1H), 1.27 (s, 1H), 1.02 (s, 3H). LC-MS: $[M+H]^+$ = 465.00.

Example 55. Synthesis of (*R*)-3-hydroxy-*N*,*N*-dimethyl-4-((2-(((1-methylcyclopentyl)(4-methylpyridin-3-yl)methyl)amino)-3,4-dioxocyclobut-1-en-1-yl)amino)picolinamide (compound 055)

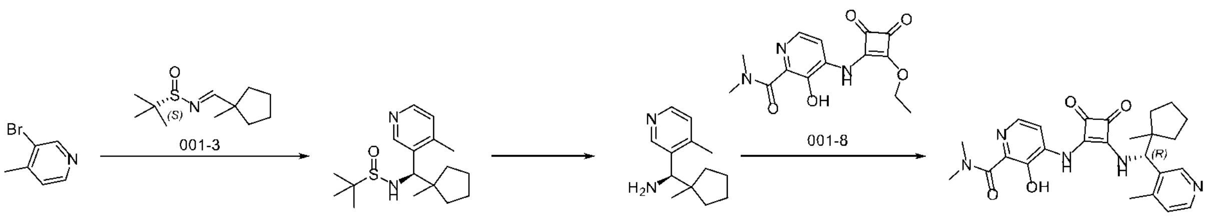


055-a 055-1 055-2 055

The synthesis of compound 055 was carried out with reference to the synthesis method for compound 001 in Example 1, except that the starting material 001-e was replaced with 055-a, to obtain compound 055 (289 mg, yield: 63.8%, chiral purity: 68.8% (HPLC method H)).

$^1$H NMR (400 MHz, DMSO-$d_6$): δ 9.003 (s, 1H), 8.535 (s, 1H), 8.331 (s, 1H), 7.963-7.906 (m , 2H),7.228-7.215 (d, *J= 7.2* Hz, 1H), 5.610-5.586 (d, *J*= 7.6 Hz, 1H), 3.139 (s, 3H), 3.035 (s, 3H), 2.429 (s, 3H), 1.631-1.594 (m, 6H), 1.451 - 1.432 (m, 1H), 1.268 - 1.240 (m, 1H), 0.996 (s, 3H). LC-MS: [M+H]$^+$ = 464.05.

Example 56. Synthesis of (*R*)-3-hydroxy-*N*,*N*-dimethyl-4-((2-(((1-methylcyclopentyl)(3-methylpyridin-4-yl)methyl)amino)-3,4-dioxocyclobut-1-en-1-yl)amino)picolinamide (compound 056)

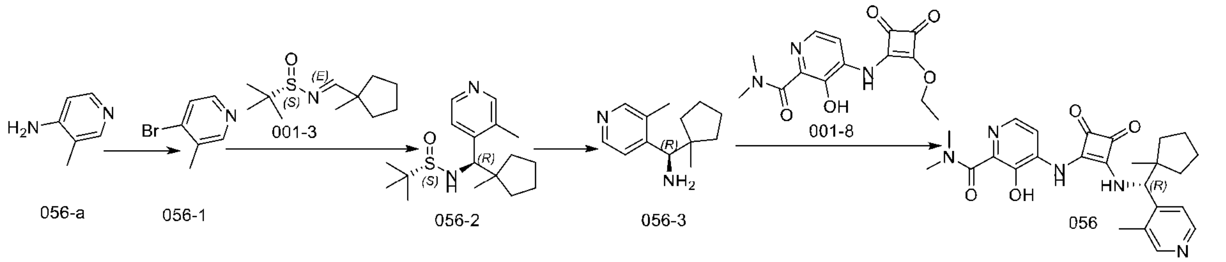


56.1 Synthesis of compound 056-1

056-a (1.0 g, 9.25 mmol) was weighed and dissolved in hydrobromic acid (20 mL, 48%). At -20°C, a solution of sodium nitrite (1.3 g, 18.50 mmol) in water (10 mL) was slowly added. After the dropwise addition was completed, the reaction was carried out at -20°C for 1 hour. Then, $CuBr_2$ (4.1 g, 18.50 mmol) was added. After the addition was completed, the reaction mixture was warmed to room temperature and reacted for another 3 hours. At 0°C, a 50% aqueous sodium hydroxide solution was slowly added to adjust the pH to 8-9, and the mixture was extracted with ethyl acetate (20 mL × 3). The organic phases were combined, washed once with saturated brine (30 mL), dried over anhydrous sodium sulfate, filtered, and the filtrate was concentrated under reduced pressure. The residue was purified by silica gel column chromatography to obtain compound 056-1 (1.0 g, yield: 62.8%).

56.2 Synthesis of compound 056

The synthesis of compound 056 was carried out with reference to the synthesis method for compound 001 in Example 1, except that the starting material 001-e was replaced with 056-1, to obtain compound 056 (31 mg, yield: 40%, chiral purity: 100.0% (HPLC method H)).

$^1$H NMR (400 MHz, DMSO-$d_6$): δ 11.84 (s, 1H), 9.77 (s, 1H), 8.94 (s, 1H), 8.43 (d, J= 4.0 Hz, 1H), 8.39 (s, 1H), 7.93 (s, 2H), 7.25 (d, *J= 4.0* Hz, 1H), 5.53 (d, *J*= 8.0 Hz, 1H), 3.08 (d, *J= 40.0* Hz, 6H), 2.39 (s, 3H), 1.60 (m, 6H), 1.42 (m, 1H), 1.25 (m, 1H), 0.97 (s, 3H). LC-MS: [M+H]$^+$ = 464.05.

Example 57. Synthesis of (*R*)-3-hydroxy-*N*,*N*-dimethyl-4-((2-(((1-methylcyclopentyl)(2-methylpyridin-3-yl)methyl)amino)-3,4-dioxocyclobut-1-en-1-yl)amino)picolinamide (compound 057)

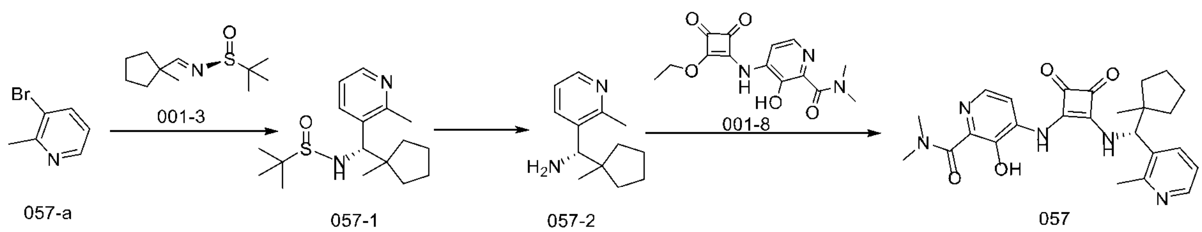


The synthesis of compound 057 was carried out with reference to the synthesis method for compound 001 in Example 1, except that the starting material 001-e was replaced with 057-a, to obtain compound 057 (59.8 mg, chiral purity: 62.3% (HPLC method H)).

$^1$H NMR (400 MHz, DMSO-$d_6$): δ 11.83 (s, 1H), 9.71 (s, 1H), 8.91 (s, 1H), 8.38 (dd, J= 4.8, 1.2 Hz, 1H), 7.96 (s, 2H), 7.68 (d, *J*= 7.8 Hz, 1H), 7.31 (dd, *J*= *7.8,* 4.8 Hz, 1H), 5.59 (d, *J*= *9.6* Hz, 1H), 3.16 (s, 3H), 3.05 (s, 3H), 2.62 (s, 3H), 1.69 - 1.55 (m, 6H), 1.48 - 1.40 (m, 1H), 1.26 (d, *J*= 7.6 Hz, 1H), 0.99 (s, 3H). LC-MS: [M+H]$^+$ = 464.00.

Example 58. Synthesis of (*R*)-3-hydroxy-*N*-methyl-4-((2-(((1-methylcyclopentyl)(3-methylpyridin-2-yl)methyl)amino)-3,4-dioxocyclobut-1-en-1-yl)amino)picolinamide (compound 058)

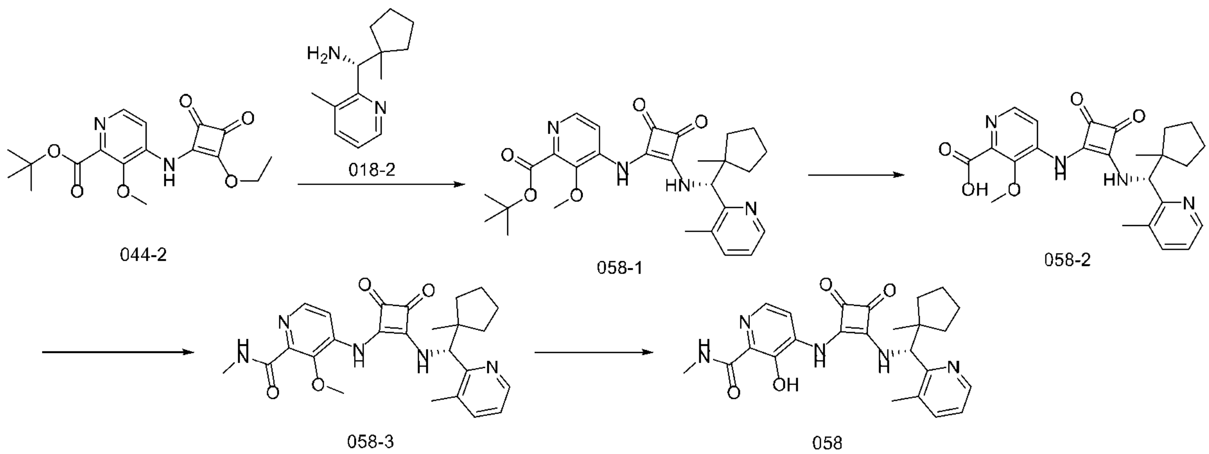


58.1 Synthesis of compound 058-1

044-2 (450 mg, 1.29 mmol) was weighed into a reaction flask. Ethanol (10 mL), *N,N*-diisopropylethylamine (500 mg, 3.87 mmol), and 018-2 (370 mg, 1.81 mmol) were added sequentially. The reaction mixture was stirred at room temperature for 2 hours and then directly concentrated under reduced pressure. The residue was purified by silica gel column chromatography (petroleum ether/ethyl acetate = 2/1) to obtain compound 058-1 (900 mg). LC-MS: [M+H]$^+$ = 507.05.

58.2 Synthesis of compound 058-2

058-1 (800 mg, 1.5 mmol) was weighed into a reaction flask and dissolved in dichloromethane (10 mL). Trifluoroacetic acid (10 mL) was added dropwise slowly, and the reaction mixture was stirred at room temperature overnight. Then, the reaction mixture was directly concentrated under reduced pressure to obtain compound 058-2 (1.6 g, crude product), which was directly used in the next step. LC-MS: [M+H]$^+$ = 450.95.

58.3 Synthesis of compound 058-3

058-2 (1.0 g, crude product) was weighed into a reaction flask. *N,N*-Dimethylformamide (10 mL), *N*,*N*,*N*',*N*'-tetramethyl-*O*-(7-azabenzotriazol-1-yl)uronium hexafluorophosphate (506 mg, 1.33 mmol), triethylamine (450 mg, 4.44 mmol), and methylamine hydrochloride (89.92 mg, 1.33 mmol) were added sequentially. The reaction was carried out at room temperature for 2 hours. Water (10 mL) was added to the reaction mixture for dilution, and the mixture was extracted with ethyl acetate (10 mL × 3). The organic phases were combined, washed with saturated aqueous sodium chloride solution (10 mL × 3), dried over anhydrous sodium sulfate, and filtered. The filtrate was concentrated under reduced pressure, and the residue was purified by silica gel column chromatography (petroleum ether/ethyl acetate = 1/1) to obtain 058-3 (140 mg). LC-MS: [M+H]$^+$ = 464.00.

58.4 Synthesis of compound 058

058-3 (140 mg, 0.18 mmol) was weighed into a reaction flask. The air in the flask was replaced with nitrogen. At 0°C, a solution of boron tribromide in dichloromethane (8 mL, 1.0 M) was added. The reaction was carried out at room temperature overnight. The reaction was quenched with methanol (4 mL) in an ice bath. The reaction mixture was concentrated under reduced pressure. The residue was purified by preparative HPLC (basic conditions) and lyophilized to obtain compound 058 (26.7 mg, chiral purity: 100% (HPLC method L)).

$^1$H NMR (400 MHz, DMSO-$d_6$): δ 10.29 (s, 1H), 9.51 (s, 2H), 8.48 (d, *J* = 4.6 Hz, 1H), 8.12 (d, *J*= 5.4 Hz, 1H), 7.62 (d, *J*= 7.8 Hz, 2H), 7.25 (dd, *J* = 7.4, 4.8 Hz, 1H), 5.72 (d, *J*= 9.8 Hz, 1H), 2.85 (d, *J*= 3.8 Hz, 3H), 2.38 (s, 3H), 1.71 (dd, *J* = 16.8, 6.3 Hz, 2H), 1.54 (dd, *J* = 17.4, 9.6 Hz, 4H), 1.37 - 1.25 (m, 1H), 1.20 (d, J= 12.4 Hz, 1H), 0.99 (s, 3H). LC-MS: $[M+H]^+$ = 450.00.

Example 59. Synthesis of (*R*)-3-((3-hydroxy-2-(morpholine-4-carbonyl)pyridin-4-yl)amino)-4-(((1-methylcyclopentyl)(3-methylpyridin-2-yl)methyl)amino)cyclobut-3-ene-1,2-dione (compound 059)

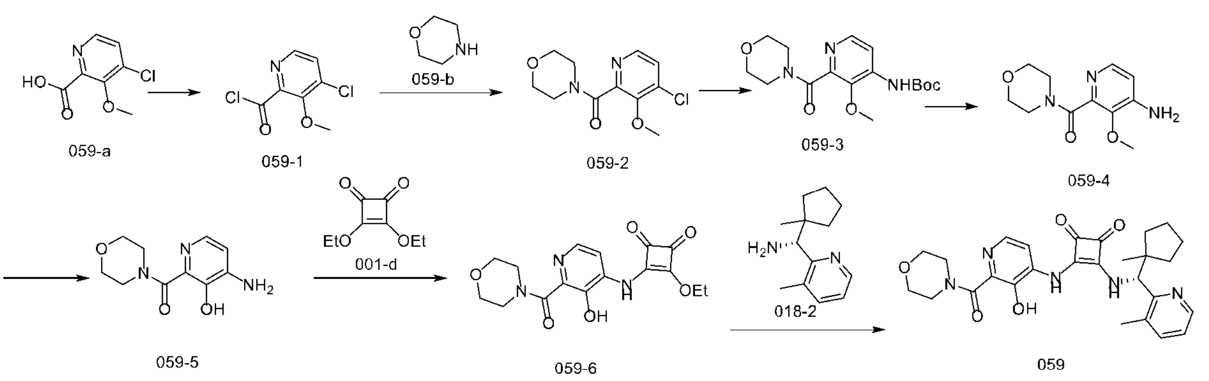


59.1 Synthesis of compound 059-2

059-a (2.0 g, 10.66 mmol) was weighed into a reaction flask, and dichloromethane (30 mL) was added. At 0°C, oxalyl chloride (2.7 g, 21.32 mmol) was added dropwise to the reaction system. After the dropwise addition was completed, the temperature was raised to ambient temperature, and stirring was continued for 2 hours. The reaction mixture was directly concentrated under reduced pressure to obtain 059-1 (2.0 g, crude product). The crude product was directly used in the next step without purification.

059-1 (2.0 g, crude product) was dissolved in dichloromethane (20 mL). At 0°C, triethylamine (2.95 g, 29.1 mmol) and 059-b (1.1 g, 11.65 mmol) were added sequentially, maintaining the internal temperature below 5°C during the addition. After the addition was completed, the reaction mixture was warmed to room temperature and stirred for another 12 hours. Water (50 mL) was added for dilution, and the mixture was extracted with dichloromethane (30 mL × 2). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 5/1 to 1/1) to obtain compound 059-2 (2.5 g, yield: 100%). LC-MS: $[M+H]^+$ = 257.00.

59.2 Synthesis of compound 059-3

059-2 (1.5 g, 5.84 mmol) was added to a reaction flask, followed by the addition of 1,4-dioxane (30 mL), *tert*-butyl carbamate (1.0 g, 8.77 mmol), cesium carbonate (5.7 g, 17.53 mmol), palladium acetate (135 mg, 0.60 mmol), and 2-dicyclohexylphosphino-2',4',6'-triisopropylbiphenyl (286 mg, 0.60 mmol). Under a nitrogen atmosphere, the reaction mixture was stirred at 95°C for 16 hours. The reaction mixture was directly filtered, and the filtrate was concentrated under reduced pressure. The residue was purified by silica gel column chromatography (petroleum ether/ethyl acetate = 1/1) to obtain compound 059-3 (2.2 g). LC-MS: $[M+H]^+$ = 338.05.

59.3 Synthesis of compound 059-4

059-3 (1.5 g, 4.45 mmol) was dissolved in dichloromethane (20 mL). A solution of hydrogen chloride in 1,4-dioxane (3 mL, 4 M) was added, and the reaction was carried out at 20°C for 4 hours. The reaction mixture was directly concentrated under reduced pressure to obtain compound 059-4 (1.5 g crude product). The crude product was directly used in the next step.

59.4 Synthesis of compound 059-5

059-4 (1.0 g, 4.21 mmol) was weighed into a reaction flask. Tetrabutylammonium iodide (1.56 g, 4.21 mmol) was added. Under a nitrogen atmosphere at 0°C, boron tribromide (12.6 mL, 12.6 mmol) was added dropwise slowly,

maintaining the internal temperature at 0°C during the dropwise addition. After the dropwise addition was completed, the reaction mixture was warmed to room temperature and reacted overnight. A saturated aqueous sodium bicarbonate solution (30 mL) was slowly added dropwise to quench the reaction. The mixture was extracted with dichloromethane (50 mL × 2). The organic phases were discarded. The aqueous phase was concentrated under reduced pressure. The residue was purified by preparative HPLC (basic) to obtain compound 059-5 (600 mg, yield: 63.7%). LC-MS: $[M+H]^+$ = 224.05.

59.5 Synthesis of compound 059-6

059-5 (400 mg, 1.79 mmol) was weighed into a reaction flask, followed by the addition of 001-d (305 mg, 1.79 mmol), ethanol (2 mL), and N,N-diisopropylethylamine (695 mg, 5.38 mmol). The reaction mixture was stirred at 55°C for 2 hours and then directly concentrated under reduced pressure. The residue was purified by silica gel column chromatography (dichloromethane/methanol = 20/1) to obtain compound 059-6 (0.2 g, yield: 32%).

59.6 Synthesis of compound 059

059-6 (200 mg, 0.58 mmol) and 018-2 (118 mg, 0.58 mmol) were weighed into a reaction flask. Ethanol (10 mL) and N, N-diisopropylethylamine (224 mg, 1.73 mmol) were added, and the reaction was carried out at room temperature for 2 hours. The reaction mixture was then purified by preparative HPLC (basic conditions) and lyophilized to obtain compound 059 (30 mg, yield: 10%, chiral purity: 100.0% (HPLC method L)).

$^1$H NMR (400 MHz, DMSO-$d_6$): δ 9.39 (d, *J* = 8.0 Hz, 1H), 8.46 (d, *J* = 4.0 Hz, 1H), 8.00 (d, *J* = 8.0 Hz, 1H), 7.90 (s, 1H), 7.61 (d, *J* = 8.0 Hz, 1H), 7.25-7.22 (m, 1H), 5.73 (d, *J* = 8.0 Hz, 1H), 3.63 (d, *J* = 20.0 Hz, 8H), 2.38 (s, 3H), 1.79 - 1.66 (m, 2H), 1.62 - 1.47 (m, 4H), 1.33 - 1.26 (m, 1H), 1.21-1.17 (m, 1H), 0.98 (s, 3H). LC-MS: $[M+H]^+$ = 506.05.

Example 60. Synthesis of (*R*)-3-(((3,6-difluoropyridin-2-yl)(1-methylcyclopentyl)methyl)amino)-4-((3-hydroxy-2-(morpholine-4-carbonyl)pyridin-4-yl)amino)cyclobut-3-ene-1,2-dione (compound 060)

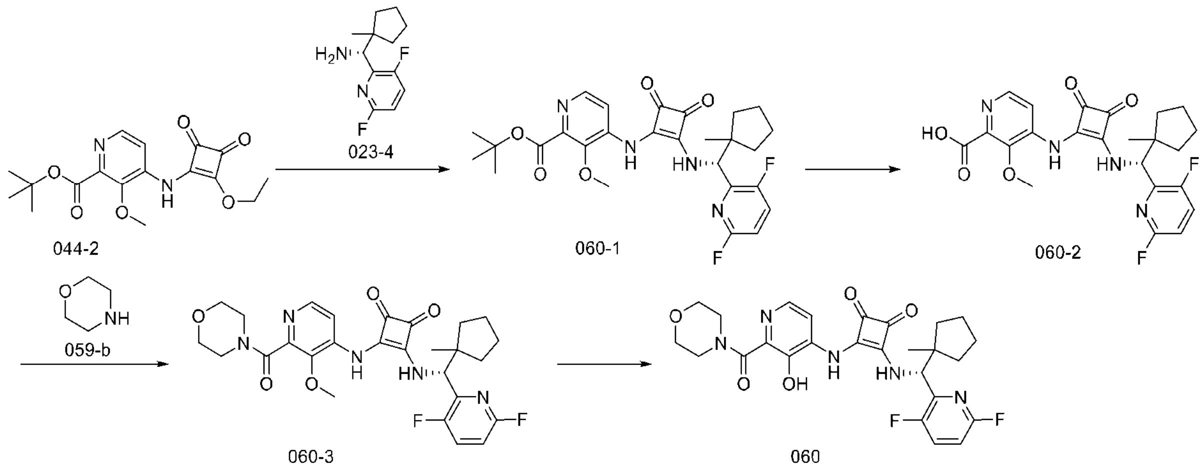


044-2 060-1 060-2 060-3 060

60.1 Synthesis of compound 060-2

The synthesis of compound 060-2 was carried out with reference to the synthesis method for 058-2 in Example 58, except that the starting material 018-2 was replaced with 023-4, to obtain compound 060-2 (0.3 g, crude product).

60.2 Synthesis of compound 060-3

060-2 (0.3 g, crude product) was weighed into a reaction flask. *N,N*-Dimethylformamide (5 mL), 059-b (66 mg, 0.73 mmol), triethylamine (193 mg, 1.90 mmol), and *N,N,N',N'*-tetramethyl-*O*-(7-azabenzotriazol-1-yl)uronium hexafluorophosphate (480 mg, 1.27 mmol) were added. The resulting reaction mixture was stirred at 20°C for 1 hour. Water (20 mL) was added for dilution, and then the mixture was extracted with ethyl acetate (30 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 060-3 (280 mg, yield: 81%). LC-MS: $[M+H]^+$ = 542.00.

60.3 Synthesis of compound 060

060-3 (50 mg, 0.10 mmol) was weighed into a reaction flask. Tetrabutylammonium iodide (35 mg, 0.10 mmol) was added. Under a nitrogen atmosphere at 0°C, boron tribromide (0.3 mL, 0.3 mmol) was added dropwise slowly, maintaining the internal temperature at 0°C during the dropwise addition. After the dropwise addition was completed, the reaction mixture was warmed to room temperature and reacted overnight. A saturated aqueous sodium bicarbonate solution (3 mL) was slowly added dropwise to quench the reaction. The mixture was extracted with dichloromethane (50 mL × 2). The organic phases were discarded. The aqueous phase was concentrated under reduced pressure. The residue was purified by preparative HPLC (basic conditions) and lyophilized to obtain compound 060 (11 mg, yield: 22.5%, chiral purity: 97.2% (HPLC method Q)).

$^1$H NMR (400 MHz, DMSO-*d*$_6$): δ 9.34 (d, *J* = 8.0 Hz, 1H), 8.04-7.98 (m, 2H), 7.89 (s, 1H), 7.27 (d, *J* = 8.0 Hz, 1H), 5.73 (d, *J* = 8.0 Hz, 1H), 3.66 (s, 8H), 1.73 (d, *J* = 12.0Hz, 1H), 1.61 (s, 5H), 1.34 - 1.28 (m, 1H), 1.09 (d, *J* = 4.0 Hz, 1H), 0.99 (s, 3H). LC-MS: [M+H]$^+$ = 527.90.

Example 61. Synthesis of (*R*)-4-((2-(((3,5-dimethylpyridin-2-yl)(1-methylcyclopentyl)methyl)amino)-3,4-dioxocyclobut-1-en-1-yl)amino)-3-hydroxy-*N*,*N*-dimethylpicolinamide (compound 061)

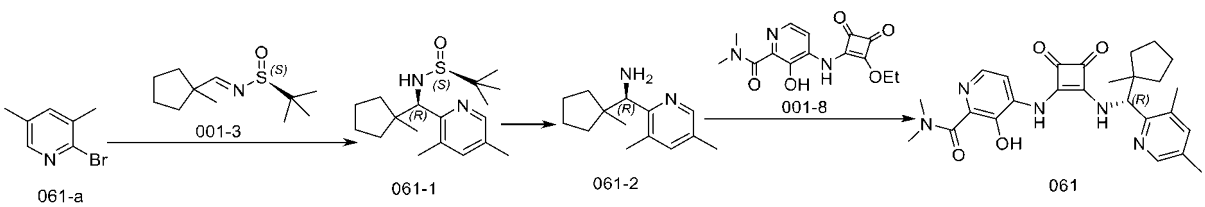


061-a 061-1 061-2 061

The synthesis of compound 061 was carried out with reference to the synthesis method for compound 001 in Example 1, except that the starting material 001-e was replaced with 061-a, to obtain compound 061 (80.1 mg, total yield: 56%, chiral purity: 100% (HPLC method H)).

$^1$H NMR (400 MHz, DMSO-*d*$_6$): δ 11.69 (bs, 1H), 10.07 (bs, 1H), 9.34 (d, *J*= 9.2 Hz, 1H), 8.32 (s, 1H), 7.99 (d, *J*= 2.8 Hz, 1H), 7.94 (bs, 1H), 7.44 (s, 1H), 5.69 (d, *J*= 10.0 Hz, 1H), 3.17 (s, 3H), 3.04 (s, 3H), 2.35 (s, 3H), 2.26 (s, 3H), 1.79-1.67 (m, 2H), 1.59-1.47 (m, 4H), 1.33-1.29 (m, 1H), 1.21-1.15 (m, 1H), 0.99 (s, 3H). LCMS: [M+H]$^+$ = 478.05.

Example 62. Synthesis of 4-((2-(((4,4-difluoro-2-methyltetrahydrofuran-2-yl)(3,6-difluoropyridin-2-yl)methyl)amino)-3,4-dioxocyclobut-1-en-1-yl)amino)-3-hydroxy-*N*,*N*-dimethylpicolinamide (compound 062)

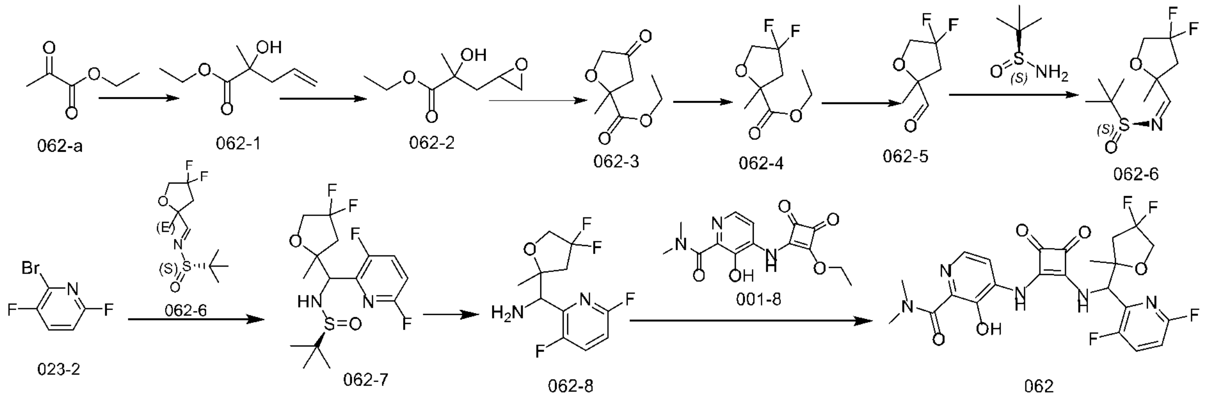


062-a 062-1 062-2 062-3 062-4 062-5 062-6

023-2 062-7 062-8 062

62.1 Synthesis of compound 061-1

062-a (10.0 g, 86.12 mmol) was weighed into a reaction flask, and dichloromethane (100 mL) was added. Under a nitrogen atmosphere at -78°C, titanium tetrachloride (21.23 g, 112 mmol) was slowly added, and the mixture was stirred at these conditions for 30 minutes. Allyltrimethylsilane (12.8 g, 112 mmol) was added dropwise slowly, and the reaction

mixture was stirred at -78°C for 2 hours. At 0°C, a saturated aqueous sodium carbonate solution (100 mL) was added. The mixture was extracted with dichloromethane (100 mL × 3). The organic phases were combined, washed with saturated brine (100 mL), dried over anhydrous sodium sulfate, filtered, and the filtrate was concentrated under reduced pressure. The residue was purified by silica gel column chromatography (petroleum ether/ethyl acetate = 5/1 to 1/1) to obtain compound 062-1 (13 g, yield: 95%). LC-MS: $[M+H]^+$ = 159.15.

62.2 Synthesis of compound 062-2

062-1 (10.0 g, 63.21 mmol) was weighed into a reaction flask, and dichloromethane (100 mL) was added. At 0°C, *m*-chloroperoxybenzoic acid (19.25 g, 94.85 mmol) was added. The resulting reaction mixture was warmed to 35°C and stirred for 16 hours. The reaction mixture was cooled to 0°C, and a saturated aqueous sodium sulfite solution (50 mL) was added. The mixture was extracted with dichloromethane (100 mL × 3). The organic phases were combined, washed with saturated sodium carbonate (50 mL), dried over anhydrous sodium sulfate, filtered, and the filtrate was concentrated under reduced pressure. The residue was purified by silica gel column chromatography to obtain compound 062-2 (8.8 g, yield: 80%).

62.3 Synthesis of compound 062-3

062-2 (8.0 g, 45.92 mmol) was weighed into a reaction flask. Tetrahydrofuran (50 mL) and magnesium bromide (1.27 g, 6.9 mmol) were added, and the reaction mixture was stirred at 80°C for 16 hours. Water (100 mL) was added, and the mixture was extracted with dichloromethane (100 mL × 3). The combined organic phases were concentrated under reduced pressure, and then dichloromethane (50 mL) was added. At 0°C, silica gel (8.4 g, 137.6 mmol) was added, followed by the portionwise addition of pyridinium chlorochromate (14.8 g, 68.9 mmol). After the addition was completed, the reaction mixture was stirred at room temperature for 1 hour and then at 35°C for 16 hours. The reaction mixture was filtered, and the filtrate was dried over anhydrous sodium sulfate and concentrated under reduced pressure. The residue was purified by silica gel column chromatography to obtain compound 062-3 (3.2 g, yield: 40.5%).

$^1$H NMR (400 MHz, $CDCl_3$): δ 4.22 (q, *J* = 8.0 Hz, 2H), 4.13 (d, *J* = 4.0 Hz, 2H), 2.86 (d, *J* = 16.0 Hz, 1H), 2.42 (d, *J* = 20.0 Hz, 1H), 1.65 (s, 3H), 1.29 (t, *J* = 4.0 Hz, 3H).

62.4 Synthesis of compound 062-4

062-3 (2.0 g, 11.62 mmol) was weighed into a reaction flask, and diethylaminosulfur trifluoride (3.74 g, 23.23 mmol) was added. The reaction mixture was stirred at room temperature for 16 hours. Dichloromethane (20 mL) was added, and the mixture was cooled to 0°C. The reaction was quenched with saturated aqueous sodium bicarbonate solution (20 mL). The mixture was extracted with dichloromethane (100 mL × 3). The organic phases were combined, dried over anhydrous sodium sulfate, and concentrated under reduced pressure. The residue was purified by silica gel column chromatography (petroleum ether/ethyl acetate = 6/1 to 3/1) to obtain compound 062-4 (1.4 g, yield: 62%).

62.5 Synthesis of compound 062-5

062-4 (1.3 g, 6.69 mmol) was weighed into a reaction flask, and dichloromethane (20 mL) was added. Under a nitrogen atmosphere, the temperature was lowered to -78°C, and diisobutylaluminum hydride (10 mL, 10.04 mmol, 1 N) was added dropwise. After the dropwise addition was completed, the reaction mixture was stirred at this temperature for 2 hours. The reaction was quenched with 100 mL of saturated aqueous sodium potassium tartrate solution. The mixture was extracted with dichloromethane (30 mL × 3). The organic phases were combined, dried over anhydrous sodium sulfate, filtered, and the filtrate was concentrated under reduced pressure to obtain compound 062-5 (0.9 g, yield: 90%), which was directly used in the next step.

62.6 Synthesis of compound 062-6

062-5 (0.5 g, 3.33 mmol) was weighed into a reaction flask. Dichloromethane (10 mL), (*S*)-tert-butylsulfinamide (605.5 mg, 5.0 mmol), and tetraethyl titanate (1.52 g, 6.66 mmol) were added. The reaction mixture was stirred at 25°C for 12 hours. The reaction mixture was poured into ice water and extracted with dichloromethane (30 mL × 3). The organic phases were combined and dried over anhydrous sodium sulfate. The filtrate was concentrated under reduced pressure to obtain a residue, which was purified by silica gel column chromatography (petroleum ether/ethyl acetate = 3/1 to 1/1) to obtain compound 062-6 (490 mg, yield: 58%). LC-MS: $[M+H]^+$ = 254.05.

62.7 Synthesis of compound 062-7

At room temperature, 023-2 (0.3 g, 1.55 mmol) and tetrahydrofuran (5 mL) were added to a reaction flask. The system was purged with nitrogen three times. At 0°C, a solution of isopropylmagnesium chloride-lithium chloride complex (2.4 mL, 3.1 mmol, 1.3 M) was added, and the reaction mixture was stirred at room temperature for 0.5 hours. The reaction system was cooled to 0°C, and 062-6 (0.3 g, 1.55 mmol) was added. After the addition was completed, the temperature was raised to room temperature, and stirring was continued for 1 hour. The reaction mixture was quenched with saturated aqueous ammonium chloride solution (10 mL) and extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 062-7 (300 mg, yield: 52%).

62.8 Synthesis of compound 062

The synthesis of compound 062 was carried out with reference to the synthesis method for compound 001 in Example 1, except that the starting material 001-9 was replaced with 062-7, to obtain compound 062 (61.7 mg, yield: 35.8%).

$^1$H NMR (400 MHz, DMSO-$d_6$) δ: 9.37 (s, 1H), 8.03 (q, *J* = 8.0 Hz, 1H), 7.88 (s, 1H), 7.75 (s, 1H), 7.33-7.29 (m, 1H), 5.94 (d, *J* = 20.0 Hz, 1H), 4.15 - 3.91 (m, 2H), 3.88-3.79 (m, 1H), 3.03 (d, *J* = 20.0 Hz, 6H), 2.86 - 2.66 (m, 1H), 2.42 - 2.29 (m, 1H), 1.32 (d, *J* = 48.0 Hz, 3H). LC-MS: $[M+H]^+$ = 524.05.

Example 63. Synthesis of (*R*)-6-chloro-2-hydroxy-*N,N*-dimethyl-3-((2-(((1-methylcyclopentyl)(3-methylpyridin-2-yl)methyl)amino)-3,4-dioxocyclobut-1-en-1-yl)amino)benzamide (compound 063)

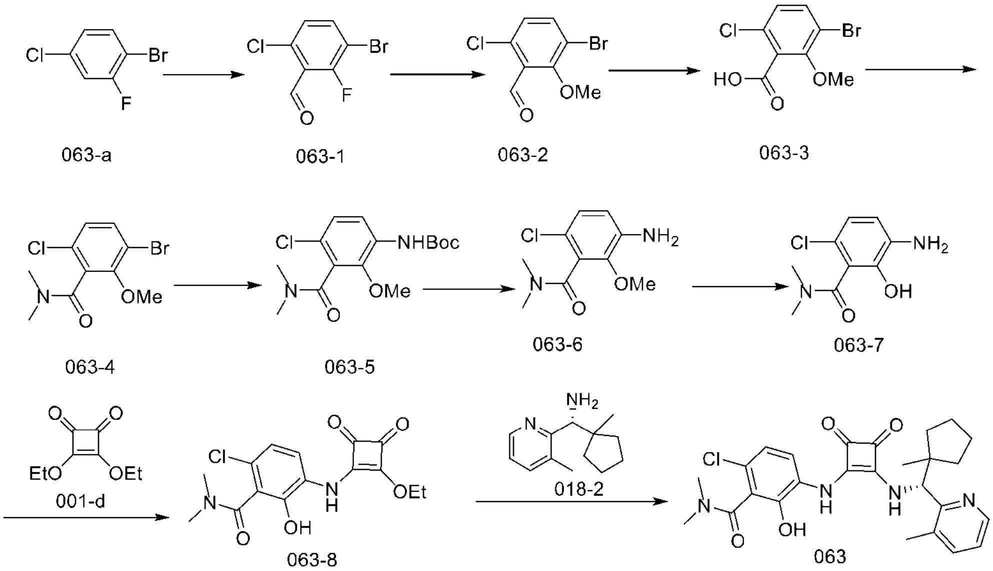

063-a
063-1
063-2
063-3
063-4
063-5
063-6
063-7
001-d
063-8
018-2
063

63.1 Synthesis of compound 063-1

063-a (6.0 g, 28.6 mmol) was weighed and dissolved in tetrahydrofuran (20 mL). The system was purged with nitrogen three times. At -78°C, lithium diisopropylamide (42.9 mL, 42.9 mmol, 1 M) was added, and the reaction was carried out at -78°C for 1 hour. Then, at -78°C, *N,N*-dimethylformamide (4.2 g, 57.2 mmol) was added to the system. After the addition was completed, the temperature was slowly raised to room temperature, and the reaction was carried out for another 2 hours. The reaction mixture was quenched with saturated ammonium chloride aqueous solution (80 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 063-1 (6.0 g, yield: 88.2%).

63.2 Synthesis of compound 063-2

063-1 (6.0 g, 25.2 mmol) was weighed and dissolved in methanol (30 mL). Sodium methoxide (1.6 g, 30.3 mmol) was added, and the reaction was carried out at 60°C for 4 hours. The reaction mixture was cooled to room temperature, quenched with water (80 mL), and extracted with ethyl acetate (50 mL × 3). The organic phase was dried over anhydrous sodium sulfate, filtered, and the filtrate was concentrated under reduced pressure. The residue was purified by silica gel column chromatography to obtain compound 063-2 (3.0 g, yield: 47.59%).

63.3 Synthesis of compound 063-3

063-2 (1.0 g, 4.21 mmol) was weighed into a reaction flask. Acetone (5 mL) and Jones reagent (842 mg, 8.42 mmol) were added. The reaction mixture was stirred at 25°C for 6 hours. The reaction mixture was poured into water (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 063-3 (1.0 g, yield: 94.3%).

63.4 Synthesis of compound 063-4

063-3 (1.0 g, 3.8 mmol) was weighed and dissolved in dichloromethane (10 mL). At 0°C, oxalyl chloride (800 mg, 6.3 mmol) and *N,N*-dimethylformamide (0.1 mL) were added, and the reaction was carried out at this temperature for 1 hour. The reaction mixture was concentrated, and dichloromethane (10 mL) was added again. At 0°C, triethylamine (1.2 g, 12.6 mmol) and a solution of dimethylamine in tetrahydrofuran (8.4 mL, 8.4 mmol, 1 M) were added. After the addition was completed, the reaction mixture was warmed to 25°C and reacted for 2 hours. The reaction mixture was quenched with water (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 063-4 (1.0 g, yield: 90.9%). LCMS: $[M+H]^+$ = 292.1.

63.5 Synthesis of compound 063-5

Compound 063-4 (1.0 g, 3.4 mmol) was weighed and dissolved in 1,4-dioxane (20 mL). Under a nitrogen atmosphere, *tert*-butyl carbamate (800 mg, 6.8 mmol), potassium carbonate (944 mg, 6.8 mmol), tris(dibenzylideneacetone)dipalladium (100 mg, 10wt%), and 2-dicyclohexylphosphino-2',4',6'-triisopropylbiphenyl (100 mg, 10wt%) were sequentially added. The reaction was carried out at 100°C for 4 hours. The reaction mixture was cooled to room temperature, filtered, and the filtrate was concentrated under reduced pressure. The residue was purified by silica gel column chromatography to obtain compound 063-5 (900 mg, yield: 79.6%).

63.6 Synthesis of compound 063-6 hydrochloride

063-5 (900 mg, 2.74 mmol) was weighed and dissolved in a solution of hydrogen chloride in 1,4-dioxane (5 mL, 4 M). The reaction mixture was stirred at 25°C for 1 hour, and then concentrated under reduced pressure to obtain 063-6 hydrochloride (600 mg, crude product).

63.7 Synthesis of compound 063-7

063-6 hydrochloride (600 mg, 2.62 mmol) was dissolved in dichloromethane (1 mL). At 0°C, boron tribromide (10 mL, 10 mmol, 1 M) was added. After the addition was completed, the reaction mixture was warmed to 25°C and reacted for 1 hour. The reaction mixture was quenched with methanol (5 mL) and then concentrated under reduced pressure to obtain the crude product of compound 063-7 (400 mg). LCMS: $[M+H]^+$ = 215.1.

63.8 Synthesis of compound 063-8

063-7 (400 mg, 1.86 mmol) was weighed into a reaction flask. Ethanol (5 mL), triethylamine (377 mg, 0.88 mmol), and 001-d (317 mg, 0.63 mmol) were added sequentially. The reaction mixture was reacted at 50°C for 2 hours, then concentrated under reduced pressure. The residue was purified by silica gel column chromatography to obtain compound 063-8 (300 mg, yield: 47.5%).

63.9 Synthesis of compound 063

063-8 (150 mg, 0.44 mmol) was dissolved in ethanol (2 mL). *N,N*-Diisopropylethylamine (113 mg, 0.88 mmol) and 018-2 (90 mg, 0.44 mmol) were added, and the reaction mixture was reacted at room temperature for 2 hours. Then, the reaction mixture was purified by preparative HPLC and lyophilized to obtain compound 063 (18.8 mg, yield: 8.54%, chiral purity: 99.9% (HPLC method H)).

$^1$H NMR (400 MHz, DMSO-$d_6$): δ 9.03 (d, *J* =8.0 Hz, 1H), 8.93 (d, J=8.0 Hz, 1H), 8.43-8.40 (m, 1H), 7.66-7.58 (m, 2H), 7.22-7.20 (m, 1H), 6.82 (d, *J* =8.0 Hz, 1H), 5.74-6.70 (m, 1H), 2.97 (d, *J* =16.0 Hz, 3H), 2.74 (d, *J* =12.0 Hz, 3H), 2.74 (d, *J* =12.0 Hz, 3H), 2.38 (s, 3H), 1.56-1.52 (m, 4H), 1.31-1.28 (m, 1H), 1.19-1.16 (m, 1H), 0.97 (s, 3H). LCMS: $[M+H]^+$ = 497.1.

Example 64. Synthesis of (*R*)-6-chloro-2-hydroxy-*N*-methyl-3-((2-(((1-methylcyclopentyl)(3-methylpyridin-2-yl)methyl) amino)-3,4-dioxocyclobut-1-en-1-yl)amino)benzamide (compound 064)

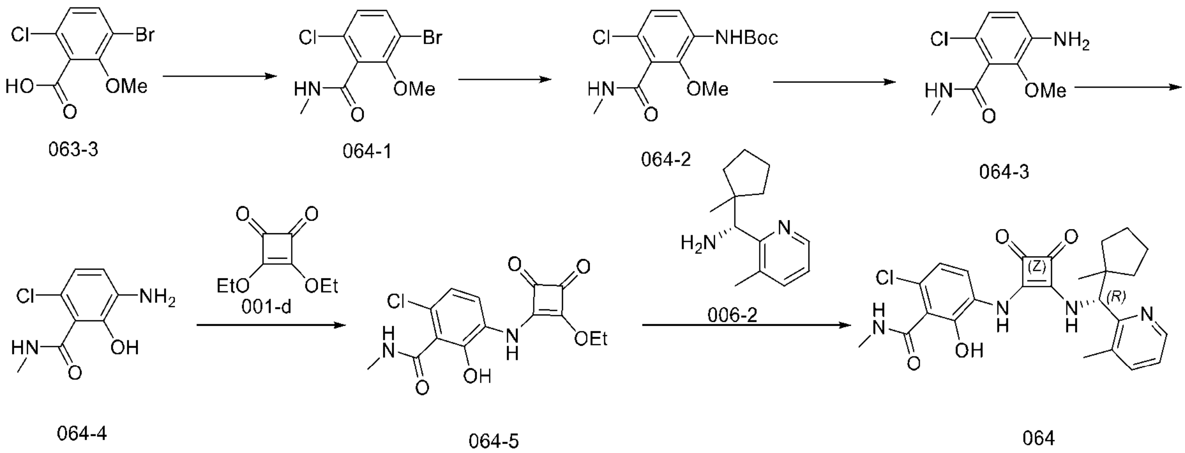


64.1 Synthesis of compound 064-1

063-3 (1.8 g, 6.78 mmol) was weighed and dissolved in *N,N*-dimethylformamide (10 mL). Triethylamine (2.1 g, 20.3 mmol), 2-(7-azabenzotriazol-1-yl)-*N,N,N',N'*-tetramethyluronium hexafluorophosphate (3.4 g, 8.8 mmol), and methylamine hydrochloride (549 mg, 8.14 mmol) were added. The reaction was carried out at 25°C for 2 hours. The reaction mixture was diluted with water (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 064-1 (1.1 g, yield: 58.5%).

64.2 Synthesis of compound 064

The synthesis of compound 064 was carried out with reference to the synthesis method for compound 063 in Example 63, except that the starting material 063-4 was replaced with 064-1, to obtain compound 064 (14.3 mg, yield: 9.6%, chiral purity: 98.5% (HPLC method H)).

$^1$H NMR (400 MHz, DMSO-$d_6$): δ 9.66 (s, 2H), 9.06 (d, *J* = 8.0 Hz, 1H), 8.70 (s, 1H), 8.47 (d, *J* = 4.0 Hz, 1H), 7.62 (t, *J* = 8.0 Hz, 2H), 7.25-7.23 (m, 1H), 6.81 (d, *J* = 8.0 Hz, 1H), 5.73 (d, *J* = 8.0 Hz, 1H), 2.75 (s, 3H), 2.38 (s, 3H), 1.75-1.69 (m, 2H), 1.57-1.50 (m, 4H), 1.32-1.29 (m, 1H), 1.21-1.17 (m, 1H), 0.98 (s, 3H). LCMS: $[M+H]^+$ = 483.1.

Example 65. Synthesis of (*R*)-4-((2-(((5-fluoro-3-methylpyridin-2-yl)(1-methylcyclopentyl)methyl)amino)-3,4-dioxocyclobut-1-en-1-yl)amino)-3-hydroxy-*N*,*N*-dimethylpicolinamide (compound 065)

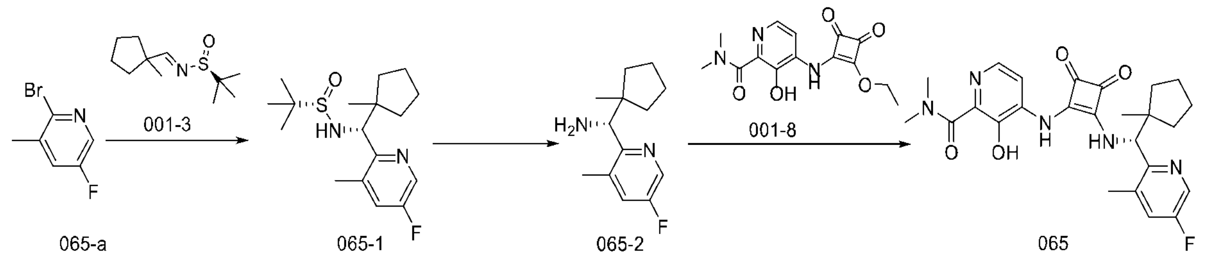


065-a 065-1 065-2 065

The synthesis of compound 065 was carried out with reference to the synthesis method for compound 001 in Example 1, except that the starting material 001-e was replaced with 065-a, to obtain compound 065 (43.0 mg, yield: 27.5%, chiral purity: 98.5% (HPLC method P)).

$^1$H NMR (400 MHz, DMSO-$d_6$): δ 9.42 (d, *J* = 12.0 Hz, 1H), 8.36 (s, 1H), 7.96 (d, *J* = 4.0 Hz, 1H), 7.92 (s, 1H), 7.60 (d, *J* = 12.0 Hz, 1H), 5.71 (d, *J* = 8.0 Hz, 1H), 3.15 (s, 3H), 3.03 (s, 3H), 2.32 (s, 3H),1.70-1.56 (m, 6H), 1.28-1.24 (m, 1H), 1.16-1.08 (m, 1H), 0.95 (s, 3H). LCMS: [M+H]$^+$ = 482.1.

Example 66. Synthesis of (*R*)-4-((2-(((5-fluoro-3-methylpyridin-2-yl)(1-methylcyclopentyl)methyl)amino)-3,4-dioxocyclobut-1-en-1-yl)amino)-3-hydroxy-*N*-isopropyl-*N*-methylpicolinamide (compound 066)

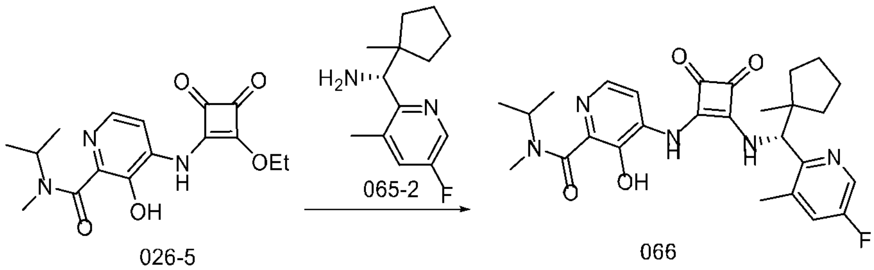


026-5 066

At room temperature, 065-2 (100 mg, 0.45 mmol), 026-5 (150 mg, 0.45 mmol), and *N,N*-diisopropylethylamine (175 mg, 1.35 mmol) were dissolved in ethanol (5 mL). The reaction mixture was stirred at room temperature for 1 hour. Then, the reaction mixture was purified by preparative HPLC (basic conditions) and lyophilized to obtain compound 066 (92.6 mg, yield: 40%, chiral purity: 96.0% (HPLC method D)).

$^1$H NMR (400 MHz, DMSO-$d_6$): δ 10.97-10.13 (m, 1H), 9.31 (d, *J* = 8.0 Hz, 1H), 8.47 (s, 1H), 7.92 (s, 2H), 7.64-7.61 (m, 1H), 5.72 (d, *J* = 8.0 Hz, 1H), 4.46 (d, *J* = 252.0 Hz, 1H), 2.87 (s, 3H), 2.42 (s, 3H), 1.75-1.67 (m, 2H), 1.65 - 1.46 (m, 4H), 1.36-1.30 (m, 1H), 1.22 - 1.08 (m, 7H), 0.98 (s, 3H). LC-MS: [M+H]$^+$ = 510.00.

Example 67. Synthesis of 4-((2-(((5,6-difluoro-3-methylpyridin-2-yl)(1-methylcyclopentyl)methyl)amino)-3,4-dioxocyclobut-1-en-1-yl)amino)-3-hydroxy-*N*,*N*-dimethylpicolinamide (compound 067)

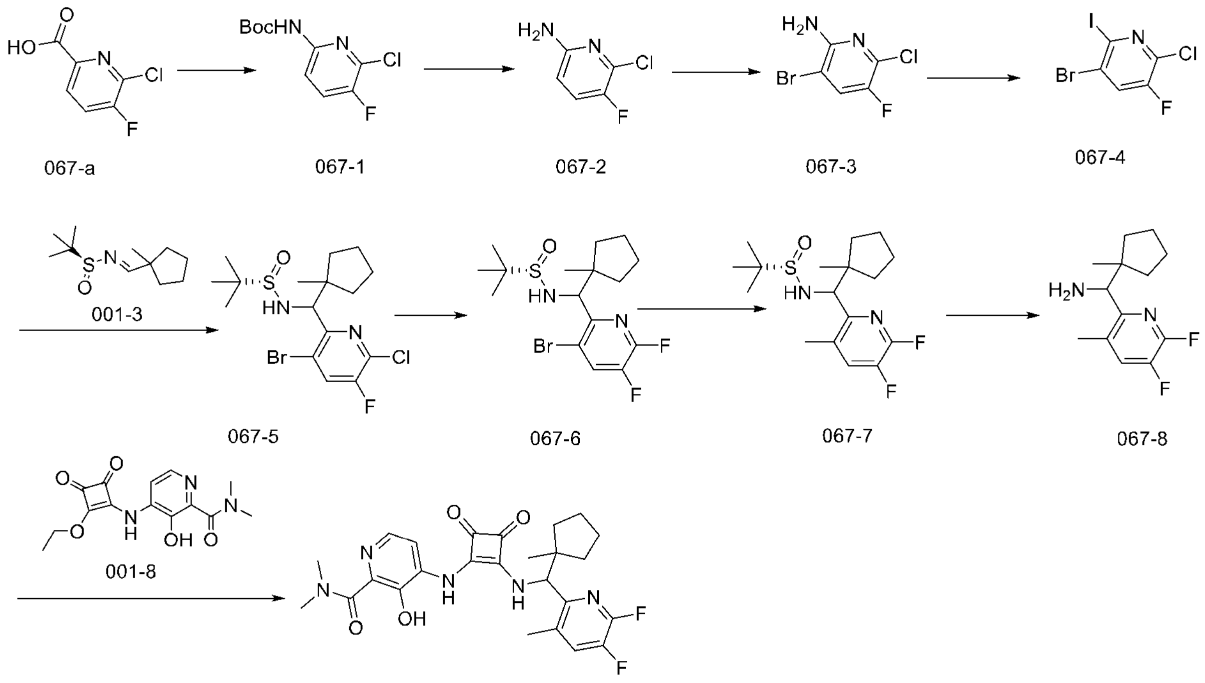

067-a 067-1 067-2 067-3 067-4

001-3

067-5 067-6 067-7 067-8

001-8

067

67.1 Synthesis of compound 067-1

067-a (5 g, 28.50 mmol) was weighed into a reaction flask, and *tert*-butanol (80 mL) was added. Under a nitrogen atmosphere, triethylamine (4.53 mL, 31.35 mmol) and diphenylphosphoryl azide (6.8 mL, 31.35 mmol) were added. The reaction mixture was stirred at 80°C for 12 hours. The reaction mixture was concentrated under reduced pressure. The residue was purified by silica gel column chromatography to obtain compound 067-1 (4.5 g, yield: 62%). LC-MS: $[M+H-56]^+$ = 191.00.

67.2 Synthesis of compound 067-2

067-1 (4 g, 16.22 mmol) was weighed into a reaction flask. Dichloromethane (10 mL) and a solution of hydrogen chloride in 1,4-dioxane (5 mL) were added, and the reaction mixture was stirred at room temperature for 5 hours. The reaction mixture was concentrated under reduced pressure to obtain compound 067-2 (2.8 g, crude product).

67.3 Synthesis of compound 067-3

067-2 (2.5 g, 17.06 mmol) was weighed into a reaction flask, and dichloromethane (10 mL), N,N-dimethylformamide (5 mL), potassium carbonate (4.72 g, 34.12 mmol), and N-bromosuccinimide (3.64 g, 20.47 mmol) were added. The reaction mixture was stirred at room temperature for 1 hour. Saturated aqueous sodium sulfite solution (25 mL) and water (25 mL) were added, and the mixture was extracted with ethyl acetate (60 mL × 3). The organic phases were combined, washed with saturated brine (60 mL), dried over anhydrous sodium sulfate, filtered, and the filtrate was directly concentrated under reduced pressure. The residue was purified by silica gel column chromatography to obtain compound 067-3 (3.0 g, crude product). LC-MS: $[M+H-56]^+$ = 225.00.

67.4 Synthesis of compound 067-4

067-3 (1 g, 4.44 mmol) was weighed into a reaction flask. Acetonitrile (30 mL), copper(I) iodide (1.27 g, 6.66 mmol), elemental iodine (2.25 g, 8.88 mmol), and tert-butyl nitrite (686 mg, 6.66 mmol) were added. The reaction mixture was stirred under reflux at 60°C for 4 hours. The reaction mixture was added with water (50 mL) and extracted with ethyl acetate (60 mL × 3). The organic phases were combined, washed with saturated brine (100 mL), dried over anhydrous sodium sulfate, filtered, and the filtrate was directly concentrated under reduced pressure. The residue was purified by silica gel column chromatography to obtain compound 067-4 (800 mg, yield: 53.7%).

67.5 Synthesis of compound 067-5

067-4 (0.6 g, 1.78 mmol) and toluene (5 mL) were added to a reaction flask. The system was purged with nitrogen three times. At -78°C, n-butyllithium (0.7 mL, 1.78 mmol, 2.5 M) was added dropwise, and the mixture was stirred for 0.5 hours. 001-3 (346 mg, 1.61 mmol) was added, and the reaction mixture was stirred at -78°C for another 0.5 hours. Then, the reaction mixture was warmed to room temperature and stirred for 12 hours. The reaction mixture was quenched with ammonium chloride solution (10 mL) and extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 067-5 (350 mg, yield: 46.0%). LC-MS: $[M+H]^+$ = 427.00.

67.6 Synthesis of compound 067-6

067-5 (0.3 g, 0.705 mmol) was weighed into a reaction flask. Dimethyl sulfoxide (5 mL), potassium fluoride (123 mg, 2.11 mmol), and cesium fluoride (321 mg, 2.11 mmol) were added. The reaction mixture was stirred at 100°C for 16 hours. Water (30 mL) was added, and the mixture was extracted with ethyl acetate (60 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 067-6 (260 mg, yield: 90%). LC-MS: $[M+H]^+$ = 409.00.

67.7 Synthesis of compound 067-7

067-6 (0.24 g, 0.586 mmol) was weighed into a reaction flask. 1,4-Dioxane (5 mL), cesium carbonate (328 mg, 1.17 mmol), methylboronic acid (53 mg, 0.879 mmol), and [1,1'-bis(diphenylphosphino)ferrocene]palladium(II) dichloride (42.6 mg, 0.0586 mmol) were added. Under a nitrogen atmosphere, the reaction mixture was stirred at 95°C for 5 hours. Water (30 mL) was added, and the mixture was extracted with ethyl acetate (60 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 067-7 (110 mg, yield: 54%). LC-MS: $[M+H]^+$ = 345.10.

67.8 Synthesis of compound 067

The synthesis of compound 067 was carried out with reference to the synthesis method for compound 001 in Example 1, except that the starting material 001-9 was replaced with 067-7, to obtain compound 067 (30.1 mg, yield: 20.6%). LC-MS: $[M+H]^+$ = 500.05.

$^1$H NMR (400 MHz, DMSO-$d_6$) δ 8.01 - 7.77 (m, 3H), 5.66 (d, J= 8.0 Hz, 1H), 3.14 (s, 3H), 3.03 (s, 3H), 2.41 (s, 3H), 1.73 - 1.52 (m, 6H), 1.39 - 1.30 (m, 1H), 1.22 (m, 1H), 0.99 (s, 3H).

Example 68. Synthesis of 4-((2-(((S)-(6-chloro-3-fluoropyridin-2-yl)((S)-4,4-difluoro-2-methyltetrahydrofuran-2-yl) methyl)amino)-3,4-dioxocyclobut-1-en-1-yl)amino)-3-hydroxy-*N*,*N*-dimethylpicolinamide (068A or 068B) and 4-((2-(((S)-(6-chloro-3-fluoropyridin-2-yl)((R)-4,4-difluoro-2-methyltetrahydrofuran-2-yl)methyl)amino)-3,4-dioxocyclobut-1-en-1-yl)amino)-3-hydroxy-N,N-dimethylpicolinamide (068B or 068A)

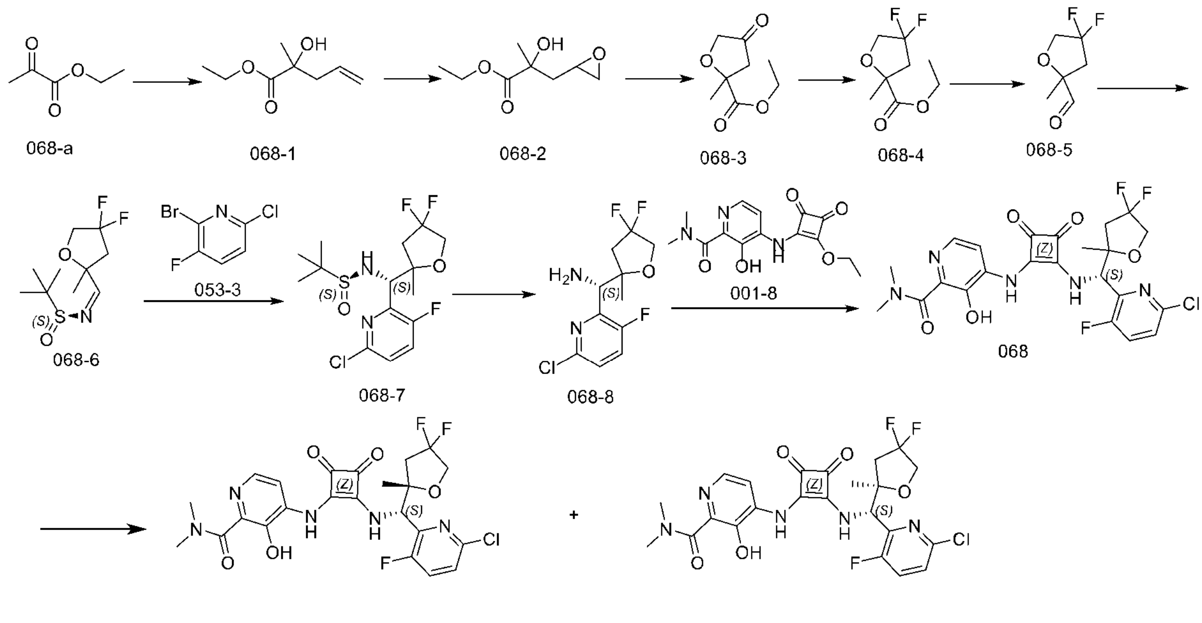


068A or 068B

068B or 068A

68.1 Synthesis of compound 068-1

068-a (10.0 g, 86.12 mmol) was weighed into a reaction flask, and dichloromethane (100 mL) was added. Under a nitrogen atmosphere at -78°C, titanium tetrachloride (21.23 g, 112 mmol) was slowly added, and the mixture was stirred at these conditions for 30 minutes. Then, allyltrimethylsilane (12.8 g, 112 mmol) was slowly added dropwise, and the reaction mixture was stirred at -78°C for 2 hours. At 0°C, a saturated aqueous sodium carbonate solution (100 mL) was added. The resulting mixture was extracted with dichloromethane (100 mL × 3). The organic phases were combined, washed with saturated brine (100 mL), dried over anhydrous sodium sulfate, filtered, and the filtrate was concentrated under reduced pressure. The residue was purified by silica gel column chromatography (petroleum ether/ethyl acetate = 5/1 to 1/1) to obtain compound 068-1 (13 g, yield: 95%). LC-MS: $[M+H]^+$ = 159.15.

68.2 Synthesis of compound 068-2

068-1 (10.0 g, 63.21 mmol) was weighed into a reaction flask, and dichloromethane (100 mL) was added. At 0°C, m-chloroperoxybenzoic acid (19.25 g, 94.85 mmol) was added. Then, the temperature was raised to 35°C, and the reaction was stirred for 16 hours. The reaction mixture was cooled to 0°C, and a saturated aqueous sodium sulfite solution (50 mL) was added. The mixture was extracted with dichloromethane (100 mL × 3). The organic phases were combined, washed with saturated aqueous sodium carbonate solution (50 mL), dried over anhydrous sodium sulfate, filtered, and the filtrate was concentrated under reduced pressure. The residue was purified by silica gel column chromatography to obtain compound 068-2 (8.8 g, yield: 80%).

68.3 Synthesis of compound 068-3

068-2 (8.0 g, 45.92 mmol) was weighed into a reaction flask. Tetrahydrofuran (50 mL) and magnesium bromide (1.27 g, 6.9 mmol) were added, and the reaction was stirred at 80°C for 16 hours. Water (100 mL) was added, and the resulting mixture was extracted with dichloromethane (100 mL × 3). The organic phases were concentrated under reduced pressure, and dichloromethane (50 mL) was added again. At 0°C, silica gel (8.4 g, 137.6 mmol) was added, followed by the portionwise addition of pyridinium chlorochromate (14.8 g, 68.9 mmol). The reaction mixture was warmed to room temperature and stirred for 1 hour, then stirred at 35°C for 16 hours. The reaction mixture was filtered, and the filtrate was dried over anhydrous sodium sulfate and concentrated under reduced pressure. The residue was purified by silica gel column chromatography to obtain compound 068-3 (3.2 g, yield: 40.5%).

$^1H$ NMR (400 MHz, $CDCl_3$): δ 4.22 (q, *J*= 8.0 Hz, 2H), 4.13 (d, *J*= 4.0 Hz, 2H), 2.86 (d, *J*= 16.0 Hz, 1H), 2.42 (d, *J*= 20.0 Hz, 1H), 1.65 (s, 3H), 1.29 (t, *J*= *4.0* Hz, 3H).

68.4 Synthesis of compound 068-4

068-3 (2.0 g, 11.62 mmol) was weighed into a reaction flask. Diethylaminosulfur trifluoride (3.74 g, 23.23 mmol) was added, and the reaction was stirred at room temperature for 16 hours. Dichloromethane (20 mL) was added, and the mixture was cooled to 0°C. Saturated aqueous sodium bicarbonate solution (20 mL) was added to quench the reaction. The resulting mixture was extracted with dichloromethane (100 mL × 3). The organic phases were combined, dried over anhydrous sodium sulfate, filtered, and the filtrate was concentrated under reduced pressure. The residue was purified by silica gel column chromatography (petroleum ether/ethyl acetate = 6/1 to 3/1) to obtain compound 068-4 (1.4 g, yield: 62%).

68.5 Synthesis of compound 068-5

068-4 (1.3 g, 6.69 mmol) was weighed into a reaction flask, and dichloromethane (20 mL) was added. Under a nitrogen atmosphere, the temperature was lowered to -78°C, and diisobutylaluminum hydride (10 mL, 1 N, 10.04 mmol) was added dropwise. The reaction was stirred at -78°C for 2 hours. The reaction mixture was quenched with a saturated aqueous potassium sodium tartrate solution (100 mL). The resulting mixture was extracted with dichloromethane (30 mL × 3). The organic phases were combined, dried over anhydrous sodium sulfate, filtered, and the filtrate was concentrated under reduced pressure to obtain compound 068-5 (0.9 g, yield: 90%), which was directly used in the next step.

68.6 Synthesis of compound 068-6

068-5 (0.5 g, 3.33 mmol) was weighed into a reaction flask. Dichloromethane (10 mL), (S)-tert-butylsulfinamide (605.5 mg, 5.0 mmol), and tetraethyl titanate (1.52 g, 6.66 mmol) were added. The reaction was stirred at 25°C for 12 hours. The reaction mixture was poured into ice water. The resulting mixture was extracted with dichloromethane (30 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 068-6 (490 mg, yield: 58%). LC-MS: $[M+H]^+$ = 254.05.

68.7 Synthesis of compound 068-7

At room temperature, 053-3 (0.3 g, 1.43 mmol) and tetrahydrofuran (5 mL) were added to a reaction flask. The system was purged with nitrogen three times. At 0°C, a solution of isopropylmagnesium chloride-lithium chloride complex (1.43 mL, 1.3 M, 1.85 mmol) was added, and the reaction was stirred at room temperature for 0.5 hours. At 0°C, 068-6 (0.29 g, 1.14 mmol) was added, and the reaction was stirred at room temperature for 1 hour. The reaction mixture was quenched with aqueous ammonium chloride solution (10 mL) and extracted with ethyl acetate (20 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 068-7 (300 mg, yield: 66%). LC-MS: $[M+H]^+$ = 385.00.

68.8 Synthesis of compound 068-8

068-7 (300 mg, 0.78 mmol) was weighed into a reaction flask. Dichloromethane (1 mL) and a solution of hydrogen chloride in 1,4-dioxane (1 mL, 4.0 M) were added, and the reaction mixture was stirred at room temperature for 0.5 hours. The reaction mixture was directly concentrated under reduced pressure. Compound 068-8 (200 mg, crude product) was obtained.

68.9 Synthesis of compound 068

At room temperature, 068-8 (180 mg, 0.641 mmol), 001-8 (196 mg, 0.64 mmol), and *N,N*-diisopropylethylamine (250 mg, 1.92 mmol) were dissolved in ethanol (2 mL), and the reaction mixture was stirred at room temperature for 1 hour. The reaction mixture was purified by HPLC (basic conditions) and lyophilized to obtain compound 068 (222.3 mg, yield: 64%). LC-MS: $[M+H]^+$ = 540.00.

$^1$H NMR (400 MHz, DMSO-$d_6$) δ 10.83 (d, *J* = 628.0 Hz, 1H), 9.37 (s, 1H), 7.97-7.91 (m, 3H), 7.69-7.62 (m, 1H), 5.97 (q, *J* =8.0 Hz, 1H), 4.13 - 3.83 (m, 2H), 3.16 (s, 3H), 3.05 (s, 3H), 2.91-2.66 (m, 1H), 2.46-2.33 (m, 1H), 1.34 (d, *J= 40.0* Hz, 3H).

68.10 Synthesis of compounds 068A and 068B

Compound 068 was resolved using a chiral column to obtain two isomers, 068A (9.1 mg, yield: 8%) and 068B (14.1 mg, yield: 12.7%).

Characterization data for compound 068A:

Chiral purity: 99.9%, $T_R$ = 11.468 min, HPLC method: CHIRALPAK AD-H, 4.6 * 250 mm 5 μm, 15% ethanol/0.1% diethylamine/n-hexane, 0.8 mL/min, 30°C.
$^1$H NMR (400 MHz, DMSO-$d_6$) δ 9.35 (s, 1H), 8.04 - 7.85 (m, 3H), 7.66-7.63 (m, 1H), 5.99 (d, *J= 8.0* Hz, 1H), 4.14-4.05 (m, 1H), 4.01 - 3.91 (m, 1H), 3.16 (s, 3H), 3.05 (s, 3H), 2.90 - 2.79 (m, 1H), 2.46 - 2.33 (m, 1H), 1.29 (s, 3H);

Characterization data for compound 068B:
Chiral purity: 99.9%, $T_R$ = 16.691 min, HPLC method: CHIRALPAK AD-H, 4.6 * 250 mm 5 μm, 15% ethanol/0.1% diethylamine/n-hexane, 0.8 mL/min, 30°C.

$^1$H NMR (400 MHz, DMSO-$d_6$) δ 9.37 (s, 1H), 8.05 - 7.83 (m, 3H), 7.67-7.64 (m, 1H), 5.95 (d, *J* = 8.8 Hz, 1H), 4.10-4.01 (m, 1H), 3.90-3.80 (m, 1H), 3.16 (s, 3H), 3.05 (s, 3H), 2.80 - 2.69 (m, 1H), 2.46 - 2.35 (m, 1H), 1.39 (s, 3H).

Example 69. Synthesis of (*R*)-4-((2-(((5-fluoro-3-methylpyridin-2-yl)(1-methylcyclopentyl)methyl)amino)-3,4-dioxocyclobut-1-en-1-yl)amino)-3-hydroxy-*N*-methyl-*N*-(2,2,2-trifluoroethyl)picolinamide (compound 069)

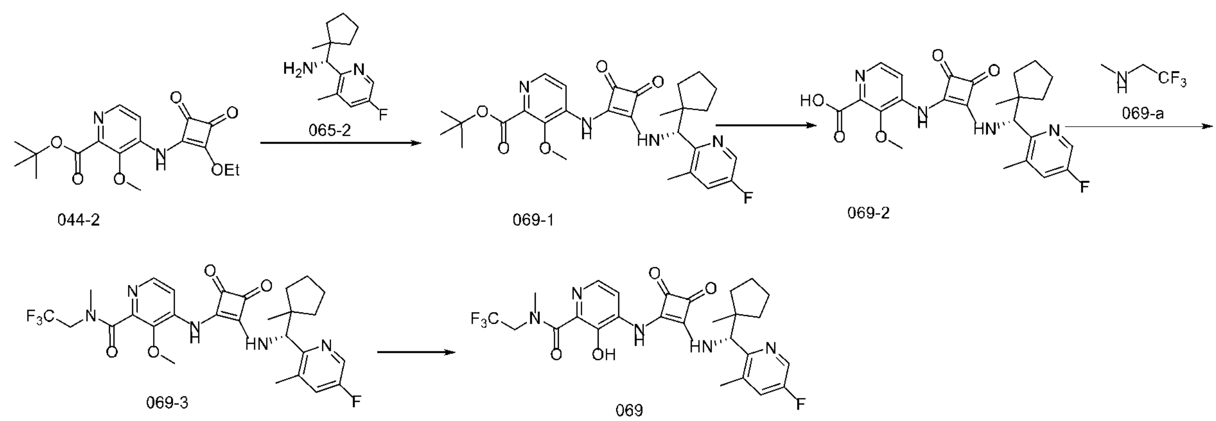


69.1 Synthesis of compound 069-2

The synthesis of compound 069-2 was carried out with reference to the synthesis method for compound 044-4 in Example 44, except that the starting material 005-2 was replaced with 065-2, to obtain compound 069-2.

69.2 Synthesis of compound 069-3

069-2 (600 mg, 1.06 mmol) was dissolved in N,N-dimethylformamide (6 mL). Triethylamine (428 mg, 4.24 mmol), propylphosphonic anhydride (1.0 g, 1.59 mmol), and N-methyl-2,2,2-trifluoroethylamine (158 mg, 1.06 mmol) were added sequentially. The reaction was carried out at room temperature for 1 hour. The reaction mixture was added with water (10 mL) and extracted with ethyl acetate (10 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 069-3 (600 mg). LC-MS: $[M+H]^+$ = 564.22.

69.3 Synthesis of compound 069

At room temperature, 069-3 (300 mg, 0.53 mmol) was dissolved in dichloromethane (3 mL). The mixture was cooled to 0°C using an ice-water bath, and a solution of boron tribromide in dichloromethane (1 M, 4.3 mL, 4.26 mmol) was added. The reaction mixture was warmed to room temperature and reacted for 3 hours. The reaction mixture was quenched with methanol (10 mL), purified by HPLC (basic conditions), and lyophilized to obtain compound 069 (21.0 mg, chiral purity: 98.8% (HPLC method: CHIRALPAK AD-H, 4.6 * 250 mm 5 μm, 20% isopropanol/n-hexane, 0.8 mL/min,

30°C)). LC-MS: $[M+H]^+$ = 550.20.

$^1$H NMR (400 MHz, DMSO-$d_6$) δ 9.41 - 9.24 (m, 1H), 8.46 (d, *J* = 4.8 Hz, 1H), 7.98 (dd, *J* = 18.4, 5.2 Hz, 1H), 7.86 (s, 1H), 7.65 - 7.59 (m, 1H), 5.72 (d, *J* = 10.0 Hz, 1H), 4.79 (d, *J* = 9.2 Hz, 1H), 4.35 (q, *J* = 9.2 Hz, 1H), 3.10 (d, *J* = 24.8 Hz, 3H), 2.43 (s, 3H), 1.70 (dd, *J* = 15.2, 8.4 Hz, 2H), 1.55 (dd, *J* = 19.2, 10.4 Hz, 4H), 1.35 - 1.29 (m, 1H), 1.20 (d, *J* = 4.8 Hz, 1H), 0.98 (s, 3H).

Example 70. Synthesis of (*R*)-4-((2-(((5-fluoropyridin-2-yl)(1-methylcyclopentyl)methyl)amino)-3,4-dioxocyclobut-1-en-1-yl)amino)-3-hydroxy-N-methyl-N-(2,2,2-trifluoroethyl)picolinamide (compound 070)

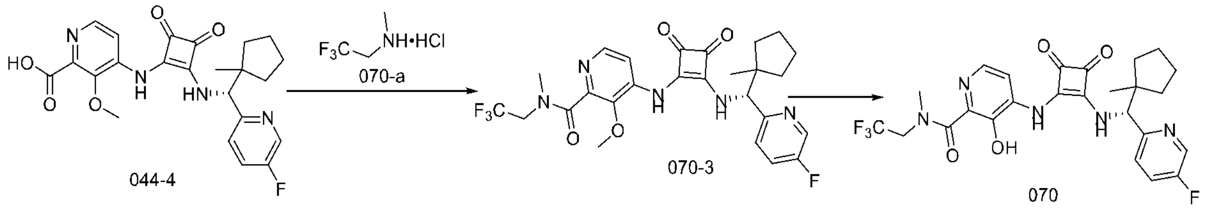


044-4

070-3

070

The synthesis of compound 070 was carried out with reference to the synthesis method for compound 069 in Example 69, except that the starting material 069-2 was replaced with 044-4 and 069-a was replaced with 070-a, to obtain compound 070 (4.8 mg, chiral purity: 100% (HPLC method: CHIRALPAK AD-H, 4.6 * 250 mm 5 μm, 15% ethanol/0.1% diethylamine/n-hexane, 0.8 mL/min, 30°C)). LC-MS: $[M+H]^+$ = 536.10.

$^1$H NMR (400 MHz, DMSO-$d_6$) δ 9.36 (s, 1H), 8.64 (d, *J* = 2.8 Hz, 1H), 8.02 (s, 1H), 7.74 (td, *J* = 8.8, 2.8 Hz, 1H), 7.50 (dd, *J* = 8.8, 4.4 Hz, 1H), 5.38 (d, *J* = 10.0 Hz, 1H), 5.07 - 4.33 (m, 2H), 3.14 (d, *J* = 18.0 Hz, 3H), 1.73 (d, *J* = 7.2 Hz, 2H), 1.60 (s, 4H), 1.17 (s, 2H), 0.93 (s, 3H).

Example 71. Synthesis of (*R*)-*N*-(2,2-difluoroethyl)-4-((2-(((5-fluoropyridin-2-yl)(1-methylcyclopentyl)methyl)amino)-3,4-dioxocyclobut-1-en-1-yl)amino)-3-hydroxy-N-methylpicolinamide (compound 071)

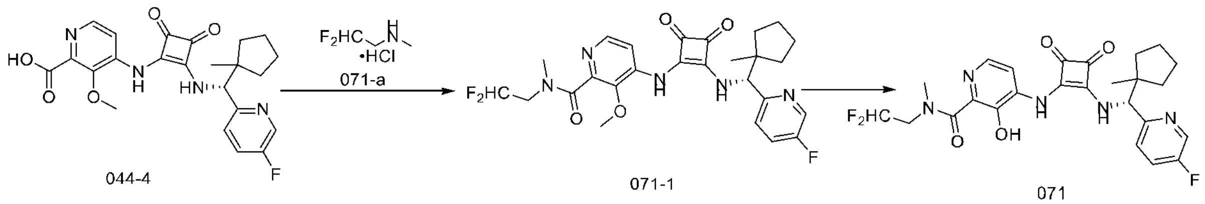


044-4

071-1

071

The synthesis of compound 071 was carried out with reference to the synthesis method for compound 069 in Example 69, except that the starting material 069-2 was replaced with 044-4 and 069-a was replaced with 071-a, to obtain compound 071 (18.7 mg, chiral purity: 100% (HPLC method: CHIRALPAK AD-H, 4.6 * 250 mm 5 μm, 15% ethanol/0.1% diethylamine/n-hexane, 0.8 mL/min, 30°C)). LC-MS: $[M+H]^+$ = 518.15.

$^1$H NMR (400 MHz, DMSO-$d_6$) δ 9.38 (s, 1H), 8.61 (s, 1H), 7.98 (dd, *J* = 13.6, 5.2 Hz, 1H), 7.91 (s, 1H), 7.72 (td, *J* = 8.8, 2.8 Hz, 1H), 7.47 (dd, *J* = 8.8, 4.4 Hz, 1H), 6.28 (td, *J* = 55.6, 20.0 Hz, 1H), 5.36 (d, *J* = 10.0 Hz, 1H), 4.21 - 3.85 (m, 2H), 3.11 (d, *J* = 8.0 Hz, 3H), 1.71 (dd, *J* = 18.4, 11.2 Hz, 2H), 1.58 (s, 4H), 1.16 - 1.06 (m, 2H), 0.91 (s, 3H).

Example 72. Synthesis of (*R*)-4-((2-(((5-fluoropyridin-2-yl)(1-methylcyclopentyl)methyl)amino)-3,4-dioxocyclobut-1-en-1-yl)amino)-3-hydroxy-*N*-isopropyl-*N*-methylpicolinamide (compound 072)

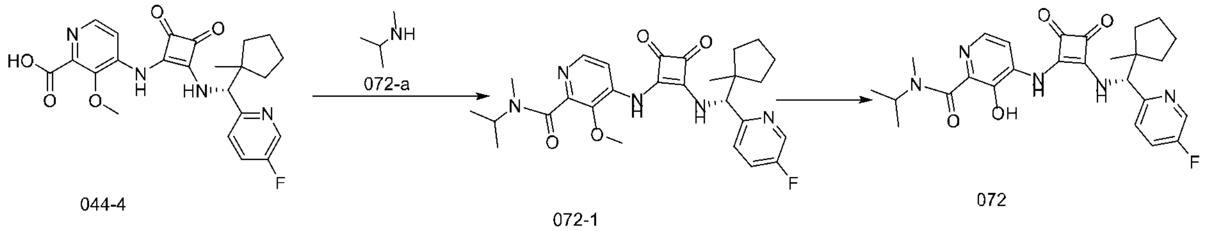


044-4 072-1 072

The synthesis of compound 072 was carried out with reference to the synthesis method for compound 069 in Example 69, except that the starting material 069-2 was replaced with 044-4 and 069-a was replaced with 072-a, to obtain compound 072 (9.5 mg, chiral purity: 92.7% (HPLC method: CHIRALPAK AD-H (4.6 * 250 mm 5 μm), 15% ethanol/5% isopropanol/n-hexane, 0.8 mL/min, 30°C)). LC-MS: $[M+H]^+$ = 496.20.

$^1H$ NMR (400 MHz, DMSO-$d_6$) δ 9.38 (s, 1H), 8.61 (s, 1H), 7.91 (s, 2H), 7.72 (dd, *J* = 14.8, 8.4 Hz, 1H), 7.48 (dd, *J* = 8.0, 4.0 Hz, 1H), 5.38 (d, *J* = 9.6 Hz, 1H), 4.46 (d, *J* = 255.6 Hz, 1H), 2.87 (s, 3H), 1.78 - 1.68 (m, 2H), 1.59 (s, 4H), 1.30 (d, *J* = 10.0 Hz, 1H), 1.13 (s, 7H), 0.92 (s, 3H).

Example 73. Synthesis of (*R*)-3-((2-(3,3-difluoropyrrolidine-1-carbonyl)-3-hydroxypyridin-4-yl)amino)-4-(((5-fluoropyridin-2-yl)(1-methylcyclopentyl)methyl)amino)cyclobut-3-ene-1,2-dione (compound 073)

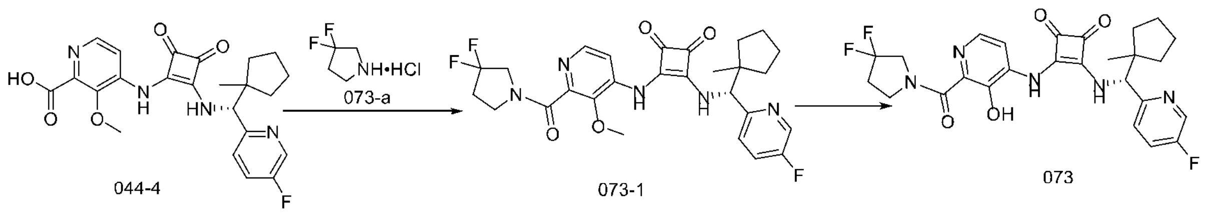


044-4 073-1 073

The synthesis of compound 073 was carried out with reference to the synthesis method for compound 069 in Example 69, except that the starting material 069-2 was replaced with 044-4 and 069-a was replaced with 073-a, to obtain compound 073 (23.2 mg, chiral purity: 93.3% (HPLC method: CHIRALPAK AD-H, 4.6 * 250 mm 5 μm, 15% isopropanol/n-hexane, 0.8 mL/min, 30°C)). LC-MS: $[M+H]^+$ = 530.10.

$^1H$ NMR (400 MHz, DMSO-$d_6$) δ 10.14 (s, 1H), 9.40 (d, *J* = 8.0 Hz, 1H), 8.64 (d, *J* = 2.8 Hz, 1H), 8.08 (s, 2H), 7.75 (td, *J* = 8.8, 2.8 Hz, 1H), 7.51 (dd, *J* = 8.8, 4.4 Hz, 1H), 5.38 (d, *J* = 10.0 Hz, 1H), 4.47 (dd, *J* = 55.6, 43.6 Hz, 2H), 3.93 (dt, *J* = 14.8, 10.4 Hz, 2H), 1.79 - 1.69 (m, 2H), 1.60 (s, 4H), 1.29 (dd, *J* = 21.2, 15.6 Hz, 2H), 1.22 - 1.07 (m, 2H), 0.93 (s, 3H).

Example 74. Synthesis of (R)-3-hydroxy-N,N-dimethyl-4-((2-(((1-methylcyclobutyl)(3-methylpyridin-2-yl)methyl)amino)-3,4-dioxocyclobut-1-en-1-yl)amino)picolinamide (compound 074)

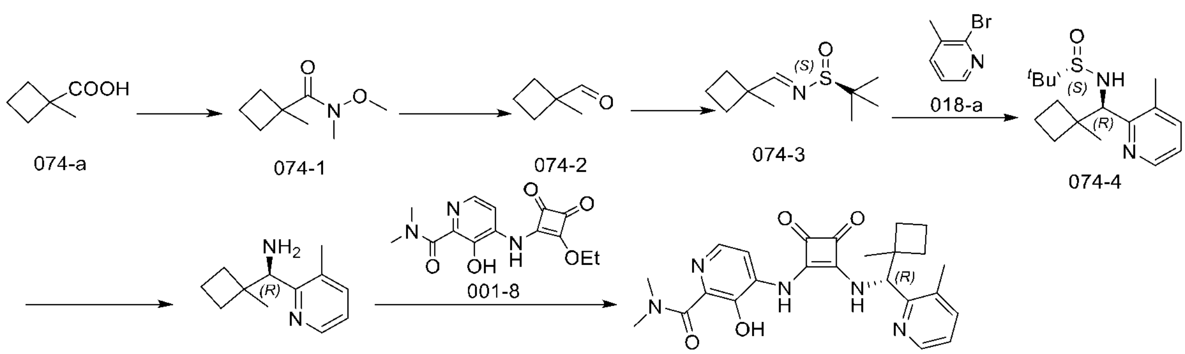


074-a 074-1 074-2 074-3 074-4 074-5 074

74.1 Synthesis of compound 074-1

074-a (2 g, 17.52 mmol), dimethylhydroxylamine hydrochloride (1.88 g, 19.27 mmol), and triethylamine (3.5 g, 34.6 mmol) were dissolved in N,N-dimethylformamide (30 mL). At room temperature, *N*,*N*,*N'*,*N'*-tetramethyl-*O*-(7-azabenzotriazol-1-yl)uronium hexafluorophosphate (9.9 g, 26.28 mmol) was added, and the reaction mixture was stirred for 30 minutes. Then, water (300 mL) was added to dilute the reaction mixture, and the mixture was extracted with ethyl acetate (100 mL × 3). The organic phase was dried over anhydrous sodium sulfate, filtered, and the filtrate was concentrated under reduced pressure. The residue was purified by silica gel column chromatography (petroleum ether/ethyl acetate = 4/1) to obtain compound 074-1 (2.2 g, 14 mmol).

74.2 Synthesis of compound 074-2

A three-necked flask was purged with nitrogen, and 074-1 (865 mg, 5.5 mmol) and dichloromethane (19 mL) were added. The temperature was lowered to -78°C, and diisobutylaluminum hydride (1 M, 8.25 mL, 8.25 mmol) was added. After stirring for 30 minutes, water (20 mL) was added, and the mixture was warmed to room temperature. Sodium hydroxide solution (2 M, 100 mL) was added, and stirring was continued for 30 minutes. The mixture was extracted with dichloromethane (60 mL × 3). The organic phase was dried over anhydrous sodium sulfate, filtered, and the filtrate was concentrated under reduced pressure to obtain a 30 mL dichloromethane solution containing 074-2, which was directly used in the next step.

74.3 Synthesis of compound 074-3

(*S*)-*tert*-Butanesulfinamide (1.0 g, 8.25 mmol) was added to the solution obtained in step 74.2. The system was purged with nitrogen, and tetraethyl titanate (1.26 g, 5.50 mmol) was added. The reaction mixture was stirred at room temperature for 2 hours. Saturated aqueous ammonium chloride solution (100 mL) was added, and the mixture was stirred for 30 minutes, then filtered. The filtrate was extracted with dichloromethane (60 mL × 3). The organic phase was dried over anhydrous sodium sulfate, filtered, and the filtrate was concentrated under reduced pressure. The residue was purified by silica gel column chromatography (petroleum ether/ethyl acetate = 8/1) to obtain compound 074-3 (185 mg, 0.92 mmol). LC-MS: $[M+H]^+$ = 202.15.

74.4 Synthesis of compound 074-4

A three-necked flask was purged with nitrogen. 018-a (348 mg, 2 mmol) and tetrahydrofuran (4 mL) were added. The temperature was lowered to -78°C, and n-butyllithium (2.5 M, 0.74 mL, 1.84 mmol) was added. After stirring for 30 minutes, 074-3 (185 mg, 0.92 mmol) was added, and the reaction was carried out at -78°C for another 1 hour. The reaction mixture was quenched with saturated aqueous ammonium chloride solution (30 mL) and extracted with ethyl acetate (20 mL × 3). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 074-4 (190 mg, 0.645 mmol). LC-MS: $[M+H]^+$ = 295.10.

74.5 Synthesis of compound 074-5

074-4 (160 mg, 0.54 mmol) was dissolved in 1,4-dioxane (1.5 mL). A solution of hydrogen chloride in 1,4-dioxane (4 M, 0.5 mL) was added, and the reaction mixture was stirred at room temperature for 30 minutes. The reaction mixture was concentrated under reduced pressure to obtain compound 074-5 (crude product), which was directly used in the next step. LC-MS: $[M+H]^+$ = 191.15.

74.6 Synthesis of compound 074

To the crude product obtained in step 074-5, ethanol (2 mL), N,N-diisopropylethylamine (113 μL, 0.65 mmol), and 001-8 (131 mg, 0.43 mmol) were added sequentially. The reaction was carried out at room temperature for 2 hours. The reaction mixture was concentrated under reduced pressure, and the residue was purified by preparative HPLC to obtain compound 074 (53.1 mg, 118 μmol, chiral purity: 98.8% (HPLC method: CHIRALPAK AD-H, 4.6 * 250 mm 5 μm, 15% ethanol/n-hexane, 0.8 mL/min, 30°C)). LC-MS: $[M+H]^+$ = 450.10.

$^1$H NMR (400 MHz, DMSO-$d_6$) δ 11.66 (s, 1H), 10.10 (s, 1H), 9.37 (d, *J* = 8.4 Hz, 1H), 8.48 (d, *J* = 4.0 Hz, 1H), 8.00 (d, *J* = 4.4 Hz, 1H), 7.95 (s, 1H), 7.65 (d, *J* = 7.2 Hz, 1H), 7.28 - 7.25 (m, 1H), 5.69 (d, *J* = 9.6 Hz, 1H), 3.17 (s, 3H), 3.04 (s, 3H), 2.38 (s, 3H), 2.25 - 2.14 (m, 2H), 1.93 - 1.86 (m, 1H), 1.68 - 1.61 (m, 1H), 1.58 - 1.49 (m, 2H), 1.18 (s, 3H).

Example 75. Synthesis of 3-hydroxy-*N,N*-dimethyl-4-((2-(((3-methylpyridin-2-yl)(1-methylspiro[2.2]pentan-1-yl)methyl)amino)-3,4-dioxocyclobutan-1-yl)amino)picolinamide (compound 075)

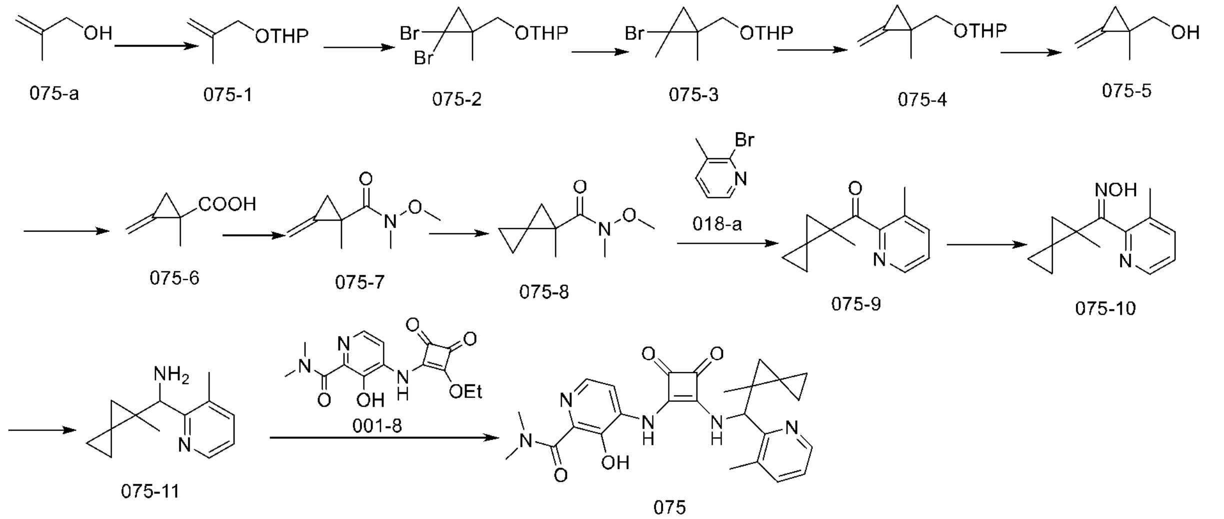

075-a 075-1 075-2 075-3 075-4 075-5
075-6 075-7 075-8 075-9 075-10
075-11 075

75.1 Synthesis of compound 075-1

075-a (21 mL, 0.25 mol) was dissolved in 3,4-dihydro-2*H*-pyran (22.8 mL, 0.25 mol). Concentrated hydrochloric acid (12 M, 250 μL, 3 mmol) was added, and the reaction mixture was stirred at room temperature for 2 hours. The reaction mixture was poured into a saturated aqueous sodium bicarbonate solution (200 mL) and extracted with ethyl acetate (100 mL × 3). The organic phase was dried over anhydrous sodium sulfate, filtered, and the filtrate was concentrated under reduced pressure to obtain compound 075-1 (37.37 g, 0.239 mol).

75.2 Synthesis of compound 075-2

075-1 (37.37 g, 0.239 mol) was dissolved in dichloromethane (68 mL). Cetrimonium bromide (4.036 g, 12 mmol), triethylamine (2 mL, 14.35 mmol), and bromoform (121 g, 0.478 mol) were added sequentially. The temperature was lowered to -20°C, and an aqueous sodium hydroxide solution (1 g/mL, 153 mL, 3.827 mol) was added. The reaction mixture was transferred to room temperature and stirred for 2 hours. Ice water (500 mL) and ethyl acetate (400 mL) were added. The mixture was filtered, and the phases were separated to obtain the organic phase. The aqueous phase was extracted with ethyl acetate (200 mL × 2). The organic phases were combined, dried over anhydrous sodium sulfate, filtered, and the filtrate was concentrated under reduced pressure. The residue was purified by silica gel column chromatography (petroleum ether/ethyl acetate = 97/3) to obtain compound 075-2 (62.17 g, 189.5 mmol).

75.3 Synthesis of compound 075-3

A three-necked flask was purged with nitrogen, and 075-2 (16.4 g, 50 mmol) and tetrahydrofuran (150 mL) were added. The temperature was lowered to -95°C, and n-butyllithium (2.5 M, 20 mL, 50 mmol) was added. After stirring for 30 minutes, iodomethane (9.34 mL, 150 mmol) was added, and the reaction was carried out at -95°C for another 30 minutes. The reaction mixture was then transferred to room temperature and stirred for another 1 hour. The reaction was quenched with saturated aqueous ammonium chloride solution (200 mL) and extracted with ethyl acetate (100 mL × 3). The organic phase was dried over anhydrous sodium sulfate, filtered, and the filtrate was concentrated under reduced pressure. The residue was purified by silica gel column chromatography (petroleum ether/ethyl acetate = 96/4) to obtain compound 075-3 (12 g, 45.6 mmol).

75.4 Synthesis of compound 075-4

075-3 (12 g, 45.6 mmol) was dissolved in dimethyl sulfoxide (100 mL). At room temperature, potassium tert-butoxide (6.654 g, 59.3 mmol) was added, and the reaction mixture was stirred for 4 hours. The reaction was then quenched with saturated aqueous ammonium chloride solution (100 mL) and diluted with water (500 mL). The mixture was

extracted with petroleum ether (200 mL × 3). The combined organic phases were dried over anhydrous sodium sulfate, filtered, and the filtrate was concentrated under reduced pressure. The residue was purified by silica gel column chromatography (petroleum ether/ethyl acetate = 20/1) to obtain compound 075-4 (7.62 g, 41.8 mmol).

75.5 Synthesis of compound 075-5

075-4 (4.975 g, 27.3 mmol) was dissolved in methanol (100 mL). At room temperature, *p*-toluenesulfonic acid (1.01 g, 5.87 mmol) was added, and the reaction mixture was stirred for 2 hours. The reaction mixture was poured into a saturated aqueous sodium bicarbonate solution (200 mL) and extracted with dichloromethane (80 mL × 3). The organic phase was dried over anhydrous sodium sulfate, filtered, and the filtrate was concentrated under reduced pressure to obtain compound 075-5 (crude product), which was directly used in the next step.

75.6 Synthesis of compound 075-6

The crude product of 075-5 was dissolved in acetone (50 mL), and the temperature was lowered to 0°C. Jones reagent (60 mL) was slowly added, and the mixture was warmed to room temperature and stirred for 3 hours. Isopropanol (30 mL) was added to quench the reaction. The reaction mixture was diluted with water (500 mL) and extracted with ethyl acetate (80 mL × 3). The organic phase was dried over anhydrous sodium sulfate, filtered, and the filtrate was concentrated under reduced pressure. The residue was purified by silica gel column chromatography (petroleum ether/ethyl acetate = 4/1) to obtain compound 075-6 (1.893 g, 16.88 mmol).

75.7 Synthesis of compound 075-7

075-6 (1.833 g, 16.35 mmol) was dissolved in *N,N*-dimethylformamide (41 mL). *N,O*-Dimethylhydroxylamine hydrochloride (2.393 g, 24.53 mmol) and *N,N*-diisopropylethylamine (8.54 mL, 49 mmol) were added sequentially. After cooling to 0°C, *N,N,N',N'*-tetramethyl-O-(7-azabenzotriazol-1-yl)uronium hexafluorophosphate (11.19 g, 29.43 mmol) was added. The reaction mixture was transferred to room temperature and reacted for 2 hours. Water (500 mL) was added to dilute the reaction mixture, and the mixture was extracted with ethyl acetate (100 mL × 3). The organic phase was dried over anhydrous sodium sulfate, filtered, and the filtrate was concentrated under reduced pressure. The residue was purified by silica gel column chromatography (petroleum ether/ethyl acetate = 4/1) to obtain compound 075-7 (1.96 g, 12.63 mmol). LC-MS: $[M+H]^+$ = 156.10.

75.8 Synthesis of compound 075-8

A three-necked flask was purged with nitrogen, and dichloromethane (20 mL) and diethylzinc (1 M, 50.52 mL, 50.52 mmol) were added. After cooling to 0°C, trifluoroacetic acid (1.934 mL, 25.26 mmol) was added, and the mixture was stirred for 30 minutes. Then, diiodomethane (8.14 mL, 101 mmol) was added, and the mixture was stirred for another 30 minutes. Subsequently, 075-7 (1.96 g, 12.63 mmol) was added, and the reaction mixture was warmed to room temperature and stirred for 4 hours. The reaction was quenched with saturated aqueous ammonium chloride solution (100 mL), and the mixture was diluted with water (200 mL). The mixture was extracted with ethyl acetate (100 mL × 3). The organic phase was dried over anhydrous sodium sulfate, filtered, and the filtrate was concentrated under reduced pressure. The residue was purified by silica gel column chromatography (petroleum ether/ethyl acetate = 4/1) to obtain compound 075-8 (1.753 g, 10.36 mmol). LC-MS: $[M+H]^+$ = 170.10.

75.9 Synthesis of compound 075-9

A three-necked flask was purged with nitrogen, and 018-a (1.032 g, 6 mmol) and tetrahydrofuran (12 mL) were added. The temperature was lowered to -78°C, and *n*-butyllithium (2.5 M, 2.1 mL, 5.25 mmol) was added. After stirring for 30 minutes, 075-8 (507 mg, 3 mmol) was added. The reaction mixture was warmed to room temperature and stirred for another 1 hour. The reaction was quenched with saturated aqueous ammonium chloride solution (20 mL), and the reaction mixture was diluted with water (80 mL). The mixture was extracted with ethyl acetate (50 mL × 3). The organic phase was dried over anhydrous sodium sulfate, filtered, and the filtrate was concentrated under reduced pressure. The residue was purified by silica gel column chromatography (petroleum ether/ethyl acetate = 10/1) to obtain compound 075-9 (490 mg, 2.43 mmol). LC-MS: $[M+H]^+$ = 202.10.

75.10 Synthesis of compound 075-10

075-9 (490 g, 2.43 mmol), hydroxylamine hydrochloride (678 mg, 9.76 mmol), and sodium acetate (500 mg, 6.1

mmol) were dissolved in ethanol (10.8 mL) and refluxed for 2 hours. The reaction mixture was cooled to room temperature again, and the reaction mixture was diluted with water (80 mL) and extracted with ethyl acetate (50 mL × 3). The organic phase was dried over anhydrous sodium sulfate, filtered, and the filtrate was concentrated under reduced pressure to obtain compound 075-10 (612 mg crude product, 2.43 mmol), which was directly used in the next step. LC-MS: $[M+H]^+$ = 217.10.

75.11 Synthesis of compound 075-11

075-10 (612 mg crude product, 2.43 mmol) was dissolved in hydrochloric acid (2 M solution in water, 20 mL). At room temperature, zinc powder (1.588 g, 24.3 mmol) was added, and the reaction was carried out for 2 hours. The reaction mixture was poured into a saturated aqueous sodium carbonate solution (200 mL). Ethyl acetate (80 mL) was added, and the mixture was filtered. The filtrate was separated, and the aqueous phase was re-extracted with ethyl acetate (80 mL × 2). The organic phases were combined, dried over anhydrous sodium sulfate, filtered, and the filtrate was concentrated under reduced pressure to obtain compound 075-11 (411 mg crude product), which was directly used in the next step. LC-MS: $[M+H]^+$ = 203.10.

75.12 Synthesis of compound 075

075-11 (101 mg, 0.5 mmol) and *N,N*-diisopropylethylamine (131 μL, 0.75 mmol) were dissolved in ethanol (2 mL). At room temperature, 001-8 (153 mg, 0.5 mmol) was added, and the reaction was carried out for 2 hours. The reaction mixture was concentrated under reduced pressure, and the residue was purified by preparative liquid chromatography to obtain compound 075 (177.7 mg). LC-MS: $[M+H]^+$ = 462.10.

$^1$H NMR (400 MHz, DMSO-$d_6$) δ 9.34-9.27 (m, 1H), 8.48-8.44 (m, 1H), 8.00-7.97 (m, 1H), 7.93 (s, 1H), 7.62 (d, *J* = 7.2 Hz, 1H), 7.26-7.22 (m, 1H), 5.66 (dd, *J*1 = 34.8 Hz, $J_2$ = 9.6 Hz, 1H), 3.15 (s, 3H), 3.03 (s, 3H), 2.35 (d, *J* = 6.0 Hz, 3H), 1.19 (d, *J* = 3.6 Hz, 0.5H), 1.15 (d, *J* = 6.4 Hz, 3H), 0.75-0.72 (m, 0.5H), 0.70-0.58 (m, 3H), 0.57-0.50 (m, 2H).

Example 76. Synthesis of (*R*)-4-((2-(((2-fluorophenyl)(1-methylcyclopentyl)methyl)amino)-3,4-dioxocyclobut-1-en-1-yl)amino)-3-hydroxy-*N,N*-dimethylpicolinamide (compound 076)

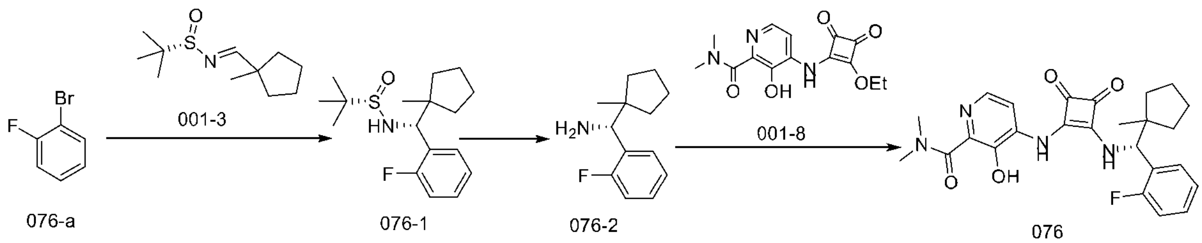


076-a 076-1 076-2 076

The synthesis of compound 076 was carried out with reference to the synthesis method for compound 001 in Example 1, except that the starting material 001-e was replaced with 076-a, to obtain compound 076 (39.8 mg, chiral purity: 100% (HPLC method: CHIRALPAK AD-H, 4.6 * 250 mm 5 μm, 15% ethanol/n-hexane, 0.8 mL/min, 30°C)). LC-MS: $[M+H]^+$ = 467.15.

$^1$H NMR (400 MHz, DMSO-$d_6$) δ 9.78 (s, 1H), 8.95 (s, 1H), 7.93 (s, 2H), 7.43 - 7.28 (m, 2H), 7.29 - 7.14 (m, 2H), 5.59 (d, *J* = 8.0 Hz, 1H), 3.09 (d, *J* = 44.0 Hz, 6H), 1.62 (d, *J* = 4.0 Hz, 6H), 1.38 (d, *J* = 8.0 Hz, 1H), 1.15 (t, *J* = 20.0 Hz, 1H), 0.96 (s, 3H).

Example 77. Synthesis of (*R*)-4-((2-((bicyclo[1.1.1]pentan-1-yl(3-methylpyridin-2-yl)methyl)amino)-3,4-dioxocyclobut-1-en-1-yl)amino)-3-hydroxy-*N,N*-dimethylpicolinamide (compound 077)

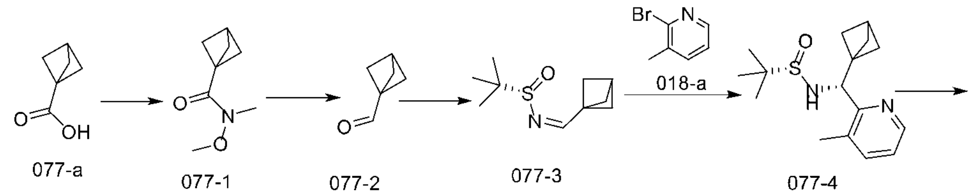


077-a 077-1 077-2 077-3 077-4

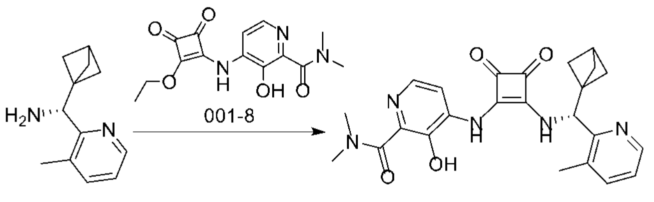


077-5 077

The synthesis of compound 077 was carried out with reference to the synthesis method for compound 074 in Example 74, except that the starting material 074-a was replaced with 077-a, to obtain compound 077 (83.0 mg, yield: 34.9%, chiral purity: 100% (HPLC method: CHIRALPAK AD-H, 4.6 * 250 mm 5 μm, 15% ethanol/n-hexane, 0.8 mL/min, 30°C)). LC-MS: $[M+H]^+$ = 448.1.

$^1$H NMR (400 MHz, DMSO-$d_6$) δ 9.44 (s, 2H), 8.44 (d, *J* = 4.0 Hz, 1H), 7.91 (d, *J* = 4.0 Hz, 1H), 7.80 (s, 1H), 7.63 (d, *J* = 4.0 Hz, 1H),7.25-7.23 (m, 1H), 5.78 (s, 1H), 3.09 (s, 3H), 3.01 (s, 3H), 2.49-2.45 (m, 4H), 2.33 (s, 2H), 1.64-1.59 (m, 4H).

Example 78. Synthesis of (*R*)-6-chloro-2-hydroxy-*N,N*-dimethyl-3-((2-(((1-methylcyclopentyl)(3-methylpyrazin-2-yl) methyl)amino)-3,4-dioxocyclobut-1-en-1-yl)amino)benzamide (compound 078)

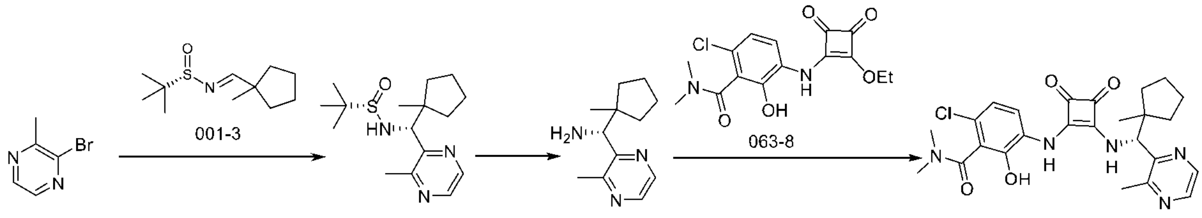


078-a 078-1 078-2 078

The synthesis of compound 078 was carried out with reference to the synthesis method for compound 001 in Example 1, except that the starting material 001-e was replaced with 078-a and 001-8 was replaced with 063-8, to obtain compound 078 (13.1 mg, chiral purity: 100% (HPLC method: CHIRALPAK AD-H, 4.6 * 250 mm 5 μm, 20% ethanol/n-hexane, 0.8 mL/min, 30°C)). LC-MS: $[M+H]^+$ = 498.10.

$^1$H NMR (400 MHz, DMSO-$d_6$) δ 9.01 (d, *J* = 8.0 Hz, 1H), 8.94 (d, *J* = 8.0 Hz, 1H), 8.55 - 8.41 (m, 2H), 7.64 (d, *J* = *20.0* Hz, 1H), 6.84 (d, *J* = 8.0 Hz, 1H), 5.74 (d, *J* = 12.0 Hz, 1H), 2.97 (d, *J* = 8.0 Hz, 3H), 2.75 (d, *J* =12.0 Hz, 3H), 2.62 (s, 3H), 1.69 (dd, *J* = 8.0, 8.0 Hz, 2H), 1.55 (dd, *J* = 16.0, 8.0 Hz, 4H), 1.32 (d, *J* = 12.0 Hz, 1H), 1.25 - 1.17 (m, 1H), 0.97 (s, 3H).

Example 79. Synthesis of (*R*)-4-((2-(((3,3-difluoro-1-methylcyclobutyl)(3-methylpyridin-2-yl)methyl)amino)-3,4-dioxocyclobut-1-en-1-yl)amino)-3-hydroxy-*N,N*-dimethylpicolinamide (compound 079)

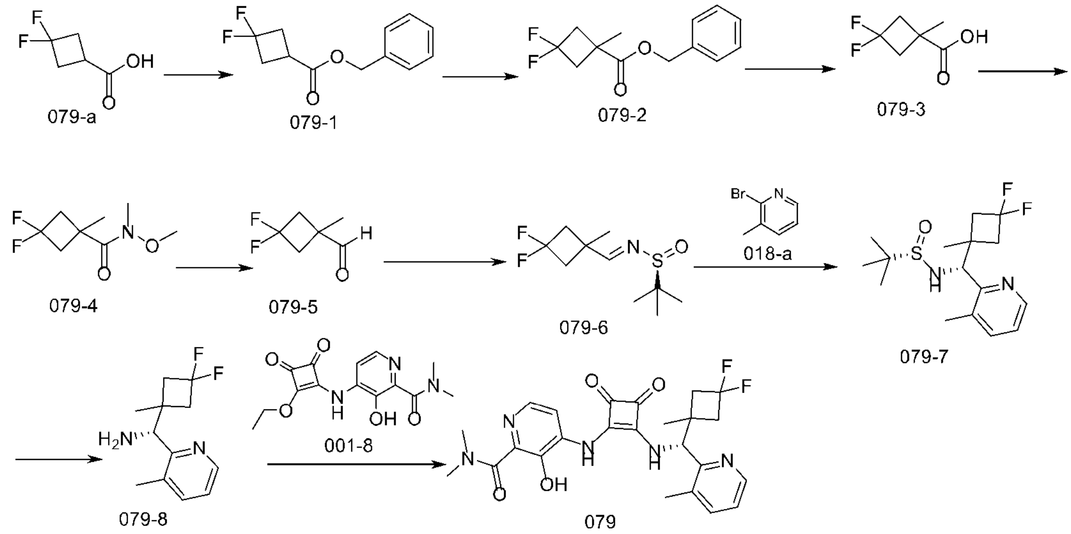


079-a 079-1 079-2 079-3 079-4 079-5 079-6 079-7 079-8 079

79.1 Synthesis of compound 079-1

079-a (3.0 g, 22.04 mmol) was weighed into a reaction flask. *N,N*-Dimethylformamide (30 mL), benzyl bromide (3.76 g, 22.04 mmol), and cesium carbonate (10.77 g, 33.06 mmol) were added, and the reaction was carried out at 25°C for 2 hours. The reaction mixture was quenched with water (100 mL). The aqueous phase was extracted with ethyl acetate (50 mL × 3). The organic phases were retained, dried over anhydrous sodium sulfate, and concentrated under reduced pressure. The residue was purified by silica gel column chromatography (petroleum ether/ethyl acetate = 40/1) to obtain compound 079-1 (3.2 g, yield: 65.1%).

79.2 Synthesis of compound 079-2

079-1 (3.2 g, 14.15 mmol) was weighed into a reaction flask, and tetrahydrofuran (30 mL) was added. Under a nitrogen atmosphere at -78°C, potassium bis(trimethylsilyl)amide (1 M, 21.2 mL, 21.2 mmol) was added. After reacting for 2 hours, iodomethane (4.02 g, 28.29 mmol) was added. The reaction mixture was warmed to 25°C and reacted for 24 hours. The reaction mixture was quenched with saturated ammonium chloride (100 mL). The aqueous phase was extracted with ethyl acetate (50 mL × 3). The organic phases were retained, dried over anhydrous sodium sulfate, and concentrated under reduced pressure. The residue was purified by silica gel column chromatography (petroleum ether/ethyl acetate = 40/1) to obtain compound 079-2 (1.7 g, yield: 50.0%).

79.3 Synthesis of compound 079-3

079-2 (1.7 g, 7.08 mmol) was weighed into a reaction flask. Methanol (10 mL), water (10 mL), and sodium hydroxide (1.42 g, 35.3 mmol) were added sequentially, and the reaction was carried out at 25°C for 2 hours. The reaction mixture was adjusted to pH = 1 with 1 M hydrochloric acid aqueous solution. The aqueous phase was extracted with ethyl acetate (50 mL × 3). The organic phases were retained, dried over anhydrous sodium sulfate, and concentrated under reduced pressure to obtain compound 079-3 (1.0 g, crude product).

79.4 Synthesis of compound 079-4

079-3 (1.0 g, 6.66 mmol) was weighed into a reaction flask, and N,N-dimethylformamide (5 mL) was added. Subsequently, 2-(7-azabenzotriazol-1-yl)-*N,N N',N'*-tetramethyluronium hexafluorophosphate (3.03 g, 7.99 mmol), triethylamine (1.35 g, 13.3 mmol), and dimethylhydroxylamine hydrochloride (0.97 g, 10.0 mmol) were added sequentially. The reaction was carried out at 25°C for 2 hours. The reaction mixture was quenched with water. The aqueous phase was extracted with ethyl acetate (30 mL × 3). The organic phases were retained, dried over anhydrous sodium sulfate, and concentrated under reduced pressure. The residue was purified by silica gel column chromatography (petroleum ether/ethyl acetate = 5/1) to obtain compound 079-4 (700 mg, yield: 54.3%). LC-MS: $[M+H]^+$ = 194.1.

79.5 Synthesis of compound 079-5

079-4 (700 mg, 3.62 mmol) was weighed into a reaction flask, and dichloromethane (10 mL) was added. At -78°C,

diisobutylaluminum hydride (1 M, 4.35 mL, 4.35 mmol) was added, and the reaction was carried out for 2 hours. The reaction was quenched with 1 M hydrochloric acid solution, extracted with dichloromethane, and the organic phase was concentrated under reduced pressure to obtain compound 079-5 (300 mg, crude product).

79.6 Synthesis of compound 079-6

079-5 (300 mg, 2.24 mmol) was weighed into a reaction flask, and dichloromethane (10 mL) was added. Tetraethyl titanate (1.9 g, 8.32 mmol) and (*S*)-*tert*-butylsulfinamide (406 mg, 5.43 mmol) were added sequentially. The reaction was carried out at 25°C for 2 hours. The reaction mixture was quenched with water, filtered, and the aqueous phase was extracted with ethyl acetate (30 mL × 3). The organic phases were retained, dried, and concentrated under reduced pressure. The residue was purified by silica gel column chromatography (petroleum ether/ethyl acetate = 3/1) to obtain compound 079-6 (120 mg, yield: 22.6%).

79.7 Synthesis of compound 079

The synthesis of compound 079 was carried out with reference to the synthesis method for compound 074 in Example 74, except that the starting material 074-3 was replaced with 079-6, to obtain compound 079 (34.0 mg, yield: 39.6%, chiral purity: 99.0% (HPLC method: CHIRALPAK AD-H, 4.6 * 250 mm 5 μm, 15% ethanol/n-hexane, 0.8 mL/min, 30°C)). LC-MS: $[M+H]^+$ = 486.1.

$^1$H NMR (400 MHz, DMSO-$d_6$) δ 9.37 (s, 1H), 8.41 (d, *J* = 4.0 Hz, 1H), 7.81 (d, *J* = 4.0 Hz, 1H), 7.64 (d, *J* = 4.0 Hz, 1H), 7.55 (s, 1H), 7.26-7.23 (m, 1H), 5.73 (s, 1H), 2.95 (s, 6H), 2.86-2.76 (m, 2H), 2.36 (s, 3H), 2.29-2.20 (m, 2H), 1.21 (s, 3H).

Example 80. Synthesis of 6-chloro-3-((2-(((4,4-difluoro-2-methyltetrahydrofuran-2-yl)(3-methylpyridin-2-yl)methyl)amino)-3,4-dioxocyclobut-1-en-1-yl)amino)-2-hydroxy-N, N-dimethylbenzamide (compound 080)

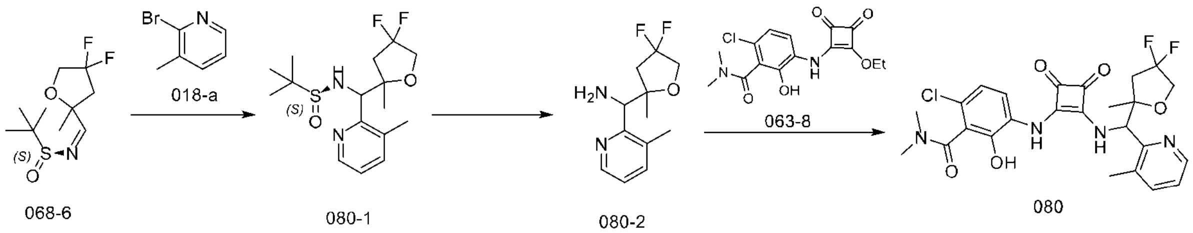


068-6 080-1 080-2 080

The synthesis of compound 080 was carried out with reference to the synthesis method for compound 068 in Example 68, except that the starting material 053-3 was replaced with 018-a, to obtain compound 080 (15.7 mg). LC-MS: $[M+H]^+$ = 535.00.

$^1$H NMR (400 MHz, DMSO-$d_6$) δ 9.13 (d, *J* = 9.4 Hz, 1H), 9.06 - 8.84 (m, 1H), 8.41 (s, 1H), 7.74 - 7.56 (m, 2H), 7.30 - 7.15 (m, 1H), 6.76 (s, 1H), 5.90 (dd, *J* = 19.4, 9.8 Hz, 1H), 4.09 - 3.81 (m, 2H), 3.09 - 2.99 (m, 1H), 2.98 - 2.85 (m, 3H), 2.74 (dd, *J* = 18.4, 4.4 Hz, 3H), 2.38 (d, *J* = 4.2 Hz, 3H), 2.21 (d, *J* = 18.6 Hz, 1H), 1.47 - 1.18 (m, 3H).

Example 81. Synthesis of 6-chloro-3-((2-(((1*S*)-(4-fluoro-2-methyltetrahydrofuran-2-yl)(3-methylpyridin-2-yl)methyl) amino)-3,4-dioxocyclobut-l-en-1-yl)amino)-2-hydroxy-*N,N*-dimethylbenzamide (compound 081)

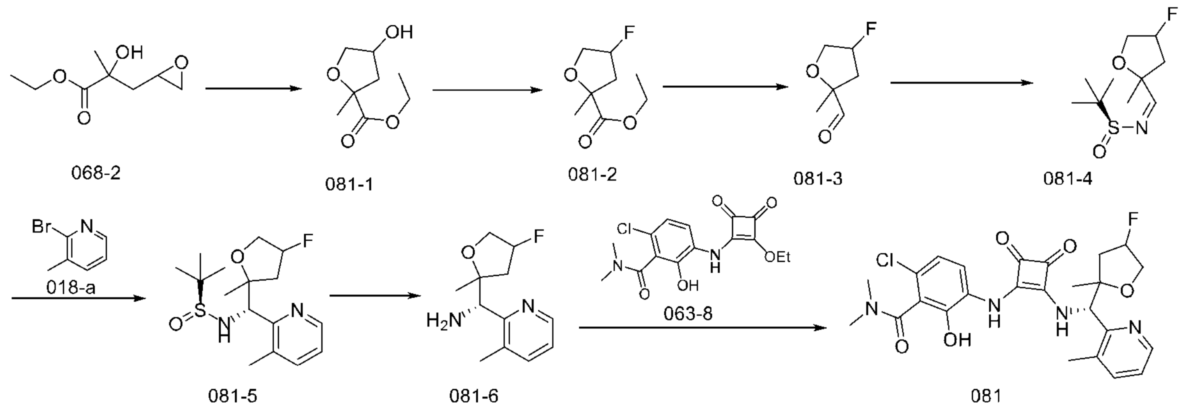

068-2 081-1 081-2 081-3 081-4
081-5 081-6 081

81.1 Synthesis of compound 081-1

068-2 (4.80 g, 27.56 mmol) was dissolved in tetrahydrofuran (50 mL). Magnesium bromide (0.76 g, 27.56 mmol) was added, and the reaction mixture was reacted for 16 hours at 80°C. The reaction mixture was filtered through a sand core funnel, and the filtrate was collected. The filtrate was then concentrated under reduced pressure. The residue was purified by silica gel column chromatography to obtain compound 081-1 (1.62 g, yield: 33.75%).

81.2 Synthesis of compound 081-2

081-1 (1.62 g, 9.30 mmol) was dissolved in dichloromethane (90 mL). Pyridine (1.33 mL, 16.74 mmol) was added, followed by the slow dropwise addition of diethylaminosulfur trifluoride (4.53 mL, 35.34 mmol). The reaction mixture was reacted at 25°C for 2 hours. The reaction mixture was slowly poured into a 5% aqueous sodium bicarbonate solution (40 mL) and extracted with dichloromethane (80 mL × 3). The organic phases were combined, washed with saturated brine (80 mL), dried over anhydrous sodium sulfate, filtered, and the filtrate was concentrated under reduced pressure. The residue was purified by silica gel column chromatography to obtain compound 081-2 (536 mg, yield: 32.71%).

81.3 Synthesis of compound 081-3

At room temperature, 081-2 (536 mg, 3.04 mmol) was dissolved in dichloromethane (10 mL). Under a nitrogen atmosphere at -78°C, diisobutylaluminum hydride (4.56 mL, 4.56 mmol, 1 M) was added, and the reaction was carried out at -78°C for 3 hours. The reaction mixture was quenched with water (2 mL), followed by the addition of a saturated aqueous sodium potassium tartrate solution (50 mL) to clarify the reaction mixture. The mixture was extracted with dichloromethane (50 mL × 3). The organic phases were combined, washed with saturated brine (50 mL), dried over anhydrous sodium sulfate, filtered, and the filtrate was concentrated under reduced pressure to obtain compound 081-3 (401 mg, crude product).

81.4 Synthesis of compound 081-4

At room temperature, 081-3 (401 mg, crude product) was dissolved in dichloromethane (12 mL). Tetraethyl titanate (1.38 g, 6.06 mmol) and (*S*)-*tert*-butylsulfinamide (0.55 g, 4.55 mmol) were added, and the reaction was carried out at room temperature for 16 hours. The reaction mixture was quenched with water (10 mL) and extracted with ethyl acetate (20 mL × 3). The organic phase was directly concentrated under reduced pressure. The residue was purified by silica gel column chromatography to obtain compound 081-4 (290 mg).

81.5 Synthesis of compound 081-5

At room temperature, 018-a (210 mg, 1.22 mmol) and tetrahydrofuran (6 mL) were added to a reaction flask. The system was purged with nitrogen three times. At -78°C, n-butyllithium (0.58 mL, 1.46 mmol, 2.5 M) was added, and the reaction was carried out at this temperature for 0.5 hours. 081-4 (287 mg, 1.22 mmol) was dissolved in tetrahydrofuran (0.5 mL), added dropwise to the reaction mixture, and reacted at -78°C for 1 hour. The reaction mixture was poured into saturated aqueous ammonium chloride solution (10 mL) to quench, then extracted with ethyl acetate (10 mL × 3). The organic phases were combined, washed with saturated brine (20 mL), dried over anhydrous sodium sulfate, filtered, and the filtrate was concentrated under reduced pressure. The residue was purified by silica gel column chromatography to

obtain compound 081-5 (220 mg, yield: 49.43%). LC-MS: $[M+H]^+$ = 329.10.

81.6 Synthesis of compound 081-6

At room temperature, 081-5 (200 mg, 0.60 mmol) was dissolved in a solution of hydrogen chloride in 1,4-dioxane (6 mL, 1 M), and the reaction mixture was stirred for 0.5 hours. The reaction mixture was concentrated under reduced pressure to obtain compound 081-6 (140 mg, crude product).

81.7 Synthesis of compound 081

081-6 (70 mg, 0.31 mmol) was dissolved in ethanol (3 mL). 063-8 (106 mg, 0.31 mmol) and *N,N*-diisopropylethylamine (201 mg, 1.56 mmol) were added, and the reaction mixture was stirred at room temperature for 6 hours. The reaction mixture was purified by HPLC (basic conditions) and lyophilized to obtain compound 081 (6.2 mg, chiral purity: 99.6% (HPLC method: CHIRALPAK AD-H, 4.6 * 250 mm 5 μm, 20% ethanol/n-hexane, 0.8 mL/min, 30°C)). LC-MS: $[M+H]^+$ = 517.05.

$^1$H NMR (400 MHz, DMSO-$d_6$) δ 7.83 (d, *J* = 8.2 Hz, 1H), 7.73 (d, *J*= 8.2 Hz, 1H), 7.47 (s, 1H), 4.83 (d, *J* = 4.8 Hz, 2H), 4.06 (s, 3H), 3.88 (t, *J* = 6.2 Hz, 2H), 2.93 (q, *J* = 7.6 Hz, 2H), 2.52 (d, *J* = 1.8 Hz, 1H), 2.46 (s, 2H), 1.55 - 1.46 (m, 1H), 1.24 (t, *J* = *7.6* Hz, 3H), 1.05 (dd, *J* = 6.6, 4.4 Hz, 2H), 0.95 (dd, *J* = 6.8, 4.4 Hz, 2H), 0.83 (t, *J* = 7.2 Hz, 3H).

Example 82. Synthesis of 3-hydroxy-N,N-dimethyl-4-((2-(((1S)-(3-methylpyridin-2-yl)(2-methyltetrahydrofuran-2-yl)methyl)amino)-3,4-dioxocyclobut-1-en-1-yl)amino)picolinamide (compound 082)

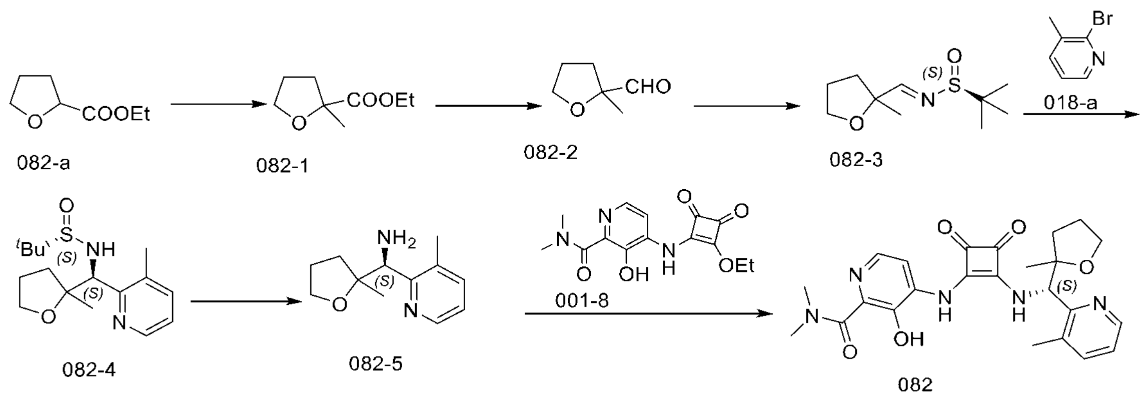

082-a 082-1 082-2 082-3 082-4 082-5 082

82.1 Synthesis of compound 082-1

A three-necked flask was purged with nitrogen, and diisopropylamine (7 mL, 50 mmol) and tetrahydrofuran (22 mL) were added. The temperature was lowered to -78°C, and n-butyllithium (2.5 M, 21 mL, 52.5 mmol) was added. After stirring for 30 minutes, the mixture was warmed to room temperature to obtain a freshly prepared 1 M solution of lithium diisopropylamide.

A three-necked flask was purged with nitrogen, and 082-a (3.57 g, 24.76 mmol) and tetrahydrofuran (60 mL) were added. The temperature was lowered to -78°C, and freshly prepared lithium diisopropylamide solution (1 M, 37.14 mL, 37.14 mmol) was added. After stirring for 30 minutes, iodomethane (2.77 mL, 44.57 mmol) was added. The reaction mixture was transferred to room temperature and stirred for another 2 hours. The reaction was quenched with saturated aqueous ammonium chloride solution (200 mL) and extracted with ethyl acetate (80 mL × 3). The organic phase was dried over anhydrous sodium sulfate, filtered, and the filtrate was concentrated under reduced pressure. The residue was purified by silica gel column chromatography (petroleum ether/ethyl acetate = 10/1) to obtain compound 082-1 (2.43 g, 15.36 mmol).

82.2 Synthesis of compound 082-2

A three-necked flask was purged with nitrogen, and 082-1 (2.43 g, 15.36 mmol) and dichloromethane (35 mL) were added. The temperature was lowered to -78°C, and diisobutylaluminum hydride (1 M, 23 mL, 23 mmol) was added. The mixture was stirred for 30 minutes. At -78°C, water (20 mL) was added, and the temperature was warmed back to room

temperature. Aqueous sodium hydroxide solution (2 M, 100 mL) was added, and stirring was continued for 30 minutes. The mixture was extracted with dichloromethane (60 mL × 3). The organic phase was dried over anhydrous sodium sulfate, filtered, and the filtrate was concentrated under reduced pressure to obtain a 50 mL dichloromethane solution containing 082-2, which was directly used in the next step.

82.3 Synthesis of compound 082-3

To the dichloromethane solution obtained in step 82.2, (S)-tert-butanesulfinamide (2.788 g, 23 mmol) was added. The system was purged with nitrogen, and tetraethyl titanate (3.22 mL, 15.36 mmol) was added. The reaction mixture was stirred at room temperature for 2 hours. Saturated aqueous ammonium chloride solution (100 mL) was added, and the mixture was filtered. The filtrate was extracted with dichloromethane (60 mL × 3). The organic phase was dried over anhydrous sodium sulfate, filtered, and the filtrate was concentrated under reduced pressure. The residue was purified by silica gel column chromatography (petroleum ether/ethyl acetate = 4/1) to obtain compound 082-3 (581 mg, 2.67 mmol). LC-MS: $[M+H]^+$ = 218.10.

82.4 Synthesis of compound 082

The synthesis of compound 082 was carried out with reference to the synthesis method for compound 074 in Example 74, except that the starting material 074-3 was replaced with 082-3, to obtain compound 082 (25.4 mg, chiral purity: 78.3% (HPLC method: CHIRALPAK AD-H, 4.6 * 250 mm 5 μm, 10% ethanol/n-hexane, 0.8 mL/min, 30°C)). LC-MS: $[M+H]^+$ = 466.10.

$^1$H NMR (400 MHz, DMSO-$d_6$) δ 9.40 (d, *J* = 10.0 Hz, 1H), 8.43 (t, *J* = 4.8 Hz, 1H), 7.94 (d, *J* = 5.2 Hz, 1H), 7.84 (s, 1H), 7.62 (d, *J* = 7.6 Hz, 1H), 7.26-7.22 (m, 1H), 5.80 (d, *J* = 9.6 Hz, 1H), 3.73 (t, *J* = 6.4 Hz, 1.5H), 3.66-3.60 (m, 0.5H), 3.09 (s, 3H), 3.01 (s, 3H), 2.38 (s, 3H), 2.28-2.21 (m, 1H), 1.96-1.87 (m, 1.4H), 1.77-1.70 (m, 0.3H), 1.65-1.59 (m, 0.7H), 1.53-1.46 (m, 0.3H), 1.39-1.35 (m, 0.3H), 1.30 (s, 1H), 1.13 (s, 2H).

Example 83. Synthesis of 6-chloro-2-hydroxy-*N,N*-dimethyl-3-((2-(((3-methylpyridin-2-yl)(3-methyltetrahydrofuran-3-yl)methyl)amino)-3,4-dioxocyclobut-1-en-1-yl)amino)benzamide (compound 083)

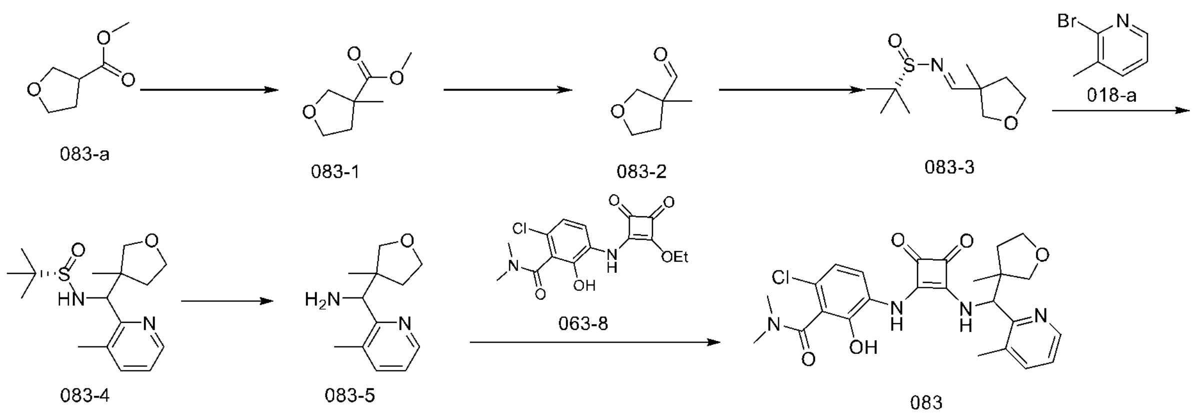

083-a
083-1
083-2
083-3
083-4
083-5
083

83.1 Synthesis of compound 083-1

Diisopropylamine (3.03 g, 29.97 mmol) was dissolved in super-dry tetrahydrofuran (20 mL). The system was purged with nitrogen three times. At -78°C, a solution of n-butyllithium in n-hexane (11.06 mL, 2.5 M) was added dropwise slowly. The reaction mixture was slowly warmed to room temperature and reacted for 1 hour. The temperature was then lowered to -78°C, and a solution of 083-a (3.0 g, 23.05 mmol) in tetrahydrofuran (5 mL) was slowly added to the reaction flask. The reaction was carried out at this temperature for another 1 hour. Iodomethane (1.58 mL, 25.36 mmol) was slowly added dropwise to the reaction flask, and the reaction was carried out at room temperature for 2 hours. The reaction mixture was quenched with saturated aqueous ammonium chloride solution and extracted with ethyl acetate (20 mL). The organic phase was separated, and the aqueous phase was washed with ethyl acetate (20 mL × 3). The organic phases were combined, washed with saturated brine (50 mL × 3), dried over anhydrous sodium sulfate, filtered, and the filtrate was concentrated under reduced pressure. The residue was purified by silica gel column chromatography (petroleum ether/ethyl acetate = 10/1) to obtain compound 083-1 (900.0 mg, yield: 27.1%).

83.2 Synthesis of compound 083-2

083-1 (900 mg, 6.24 mmol) was dissolved in dichloromethane (4 mL). The system was purged with nitrogen three times. At -78°C, a solution of diisobutylaluminum hydride in n-hexane (9.3 mL, 1.0 M) was slowly added, and the reaction was carried out at this temperature for 4 hours. The reaction mixture was quenched with saturated aqueous ammonium chloride solution and extracted with dichloromethane (10 mL). The organic phase was separated. The aqueous phase was washed with dichloromethane (8 mL × 3). The organic phases were combined, washed with saturated brine (15 mL × 3), dried over anhydrous sodium sulfate, filtered, and the filtrate was concentrated under reduced pressure to obtain compound 083-2 (680.0 mg, crude product), which was directly used in the next step.

83.3 Synthesis of compound 083

The synthesis of compound 083 was carried out with reference to the synthesis method for compound 074 in Example 74, except that the starting material 074-2 was replaced with 083-2, to obtain compound 083 (7.3 mg). LC-MS: $[M+H]^+$ = 499.05.

$^1H$ NMR (400 MHz, DMSO-$d_6$) δ 9.99 (s, 1H), 9.68 (s, 1H), 9.18 - 8.76 (m, 1H), 8.46 (s, 1H), 7.64 (s, 2H), 7.26 (s, 1H), 6.92 (s, 1H), 5.92 - 5.62 (m, 1H), 4.00 - 3.64 (m, 2H), 3.55 (d, *J* = 8.7 Hz, 1H), 2.98 (s, 3H), 2.76 (s, 3H), 2.38 (s, 3H), 2.03 (m, 2H), 1.52 (d, *J*= 70.4 Hz, 1H), 1.05 (d, *J*= *40.0* Hz, 2H).

Example 84. Synthesis of (*R*)-3-hydroxy-*N*-methyl-*N*-(methyl-$d_3$)-4-((2-(((1-methylcyclopentyl)(3-methylpyridin-2-yl)methyl)amino)-3,4-dioxocyclobut-1-en-1-yl)amino)picolinamide (compound 084)

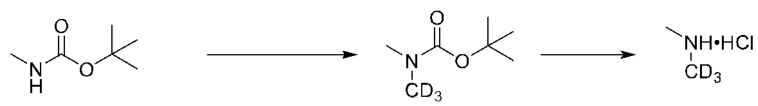

084-a 084-1 084-2

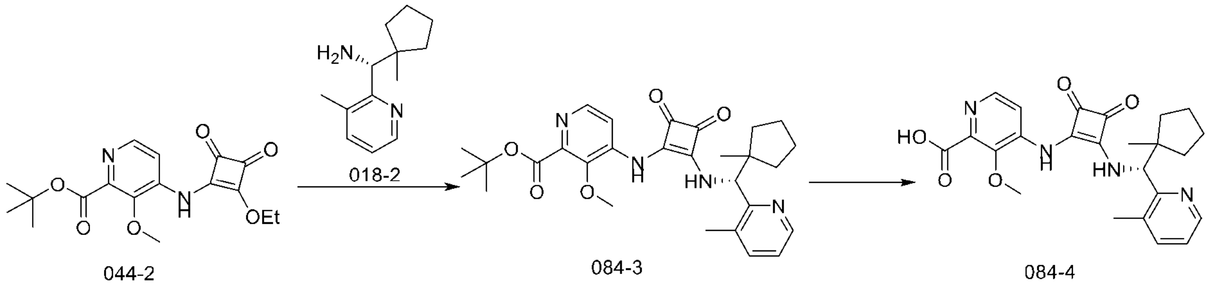


044-2 084-3 084-4

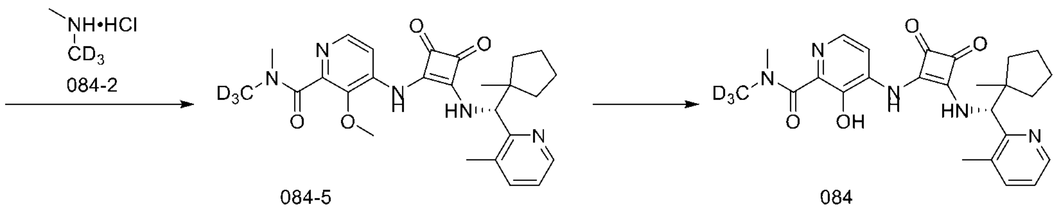


084-5 084

84.1 Synthesis of compound 084-1

084-a (500.0 mg, 3.81 mmol) was dissolved in *N,N*-dimethylformamide (10 mL). The system was purged with nitrogen three times. At 0°C, sodium hydride (457.36 mg, 11.44 mmol, 60%) was slowly added, followed by the addition of deuterated iodomethane. The reaction mixture was stirred at 0°C for 20 minutes and then warmed to room temperature and reacted for 1 hour. At 0°C, the reaction was quenched with water, and ethyl acetate (10 mL) was added for extraction. The organic phase was separated, and the aqueous phase was further extracted with ethyl acetate (10 mL × 3). The organic phases were combined, washed with saturated brine (20 mL × 3), dried over anhydrous sodium sulfate, filtered, and the filtrate was concentrated under reduced pressure. The residue was purified by silica gel column chromatography (petroleum ether/ethyl acetate = 9/1) to obtain compound 084-1 (550.0 mg, yield: 97%).

84.2 Synthesis of compound 084-2

084-1 (550.0 mg, 3.71 mmol) was dissolved in a solution of hydrogen chloride in ethyl acetate (10 mL, 2.0 M). The reaction was carried out at room temperature for 3 hours. The solvent was removed by concentration under reduced pressure to obtain compound 084-2 (120.0 mg, crude product), which was directly used in the next step.

84.3 Synthesis of compound 084

The synthesis of compound 084 was carried out with reference to the synthesis method for compound 044 in Example 44, except that the starting material 005-2 was replaced with 018-2 and 044-b was replaced with 084-2, to obtain compound 084 (37.5 mg, chiral purity: 100% (HPLC method: CHIRALPAK AD-H, 4.6 * 250 mm 5 μm, 15% ethanol/n-hexane, 0.8 mL/min, 30°C)). LC-MS: $[M+H]^+$ = 467.15.

$^1H$ NMR (400 MHz, DMSO-$d_6$) δ 11.68 (s, 1H), 10.03 (s, 1H), 9.30 (s, 1H), 8.47 (d, *J* = 4.0 Hz, 1H), 7.96 (s, 2H), 7.62 (d, *J* = 4.0 Hz, 2H), 7.24 (dd, *J* = 8.0, 4.0 Hz, 1H), 5.72 (d, *J* = 12.0 Hz, 1H), 3.09 (d, *J* = 56.0 Hz, 3H), 2.38 (s, 3H), 1.81 - 1.64 (m, 2H), 1.63 - 1.44 (m, 4H), 1.35 - 1.25 (m, 1H), 1.18 (d, *J* = 4.9 Hz, 1H), 0.98 (s, 3H).

Example 85. Synthesis of 4-((2-(((6-chloro-3-fluoropyridin-2-yl)(3-methylbicyclo[3.1.0]hexan-3-yl)methyl)amino)-3,4-dioxocyclobut-1-en-1-yl)amino)-3-hydroxy-N,N-dimethylpicolinamide (compound 085)

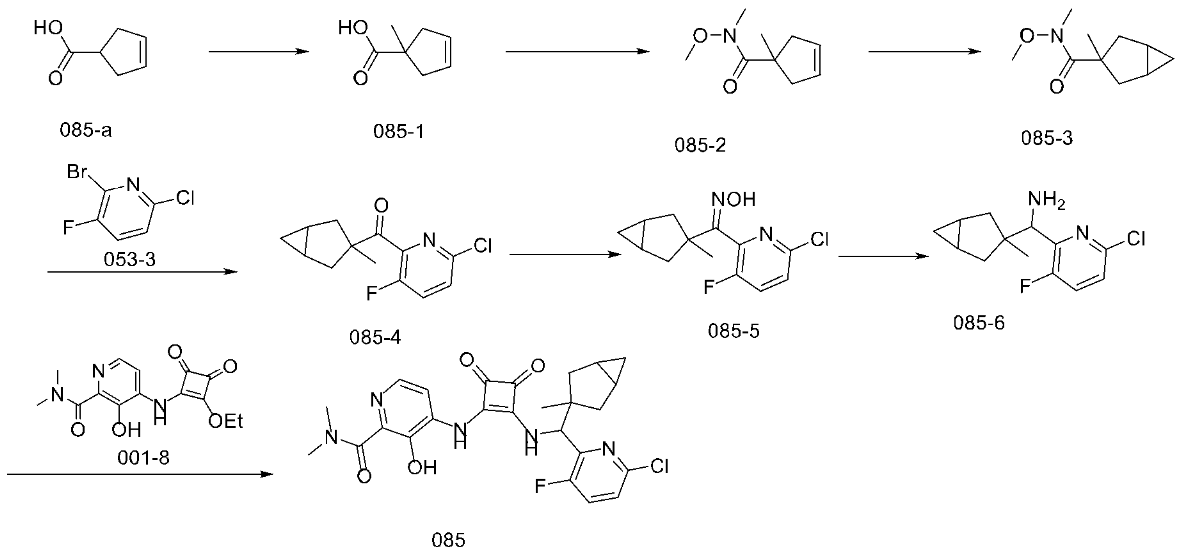

085-a
085-1
085-2
085-3
085-4
085-5
085-6
085

85.1 Synthesis of compound 085-1

Diisopropylamine (4.15 g, 41.02 mmol) was dissolved in tetrahydrofuran (40 mL). The system was purged with nitrogen three times. At -78°C, a solution of n-butyllithium in n-hexane (27 mL, 43.20 mmol, 1.6 M) was added dropwise slowly. After the addition was completed, the reaction mixture was warmed to room temperature and reacted for 1 hour. The system was cooled to -30°C, and a solution of 085-a (2 g, 17.84 mmol) in tetrahydrofuran (5 mL) was added dropwise to the reaction system. After the addition was completed, the reaction was warmed to room temperature and carried out for 3 hours. Then, the system was cooled again to -30°C, and iodomethane (3.8 g, 26.76 mmol) was added dropwise. After the addition was completed, the reaction was warmed to room temperature and carried out for 15 hours. The reaction mixture was quenched with saturated aqueous ammonium chloride solution (50 mL), then extracted with ethyl acetate (40 mL × 3). The organic phases were combined, washed with saturated brine (50 mL), dried over anhydrous sodium sulfate, filtered, and the filtrate was concentrated under reduced pressure. The residue was purified by silica gel column chromatography to obtain compound 085-1 (800 mg, yield: 35.6%).

85.2 Synthesis of compound 085-2

085-1 (800 mg, 6.34 mmol) was dissolved in dichloromethane (10 mL). At 0°C, oxalyl chloride (1.2 g, 9.51 mmol) was added dropwise slowly. After the addition was completed, the reaction mixture was warmed to room temperature and

reacted for 1 hour. The reaction mixture was concentrated under reduced pressure. The residue was dissolved in dichloromethane (10 mL). At 0°C, triethylamine (1.9 g, 19.02 mmol) and dimethylhydroxylamine hydrochloride (930 mg, 9.51 mmol) were slowly added. The reaction was carried out at room temperature for 1 hour. The reaction mixture was concentrated under reduced pressure, and the residue was purified by silica gel column chromatography to obtain compound 085-2 (580 mg).

85.3 Synthesis of compound 085-3

085-2 (580 mg, 3.43 mmol) was dissolved in dichloromethane (20 mL). The system was purged with nitrogen three times. At 0°C, diethylzinc (2.1 g, 17.14 mmol) was added, and the mixture was stirred at 0°C for 30 minutes. Subsequently, diiodomethane (9.2 g, 34.27 mmol) was added. The reaction mixture was maintained at 0°C and stirred for 30 minutes, then warmed naturally to room temperature and reacted for 2 hours. The reaction mixture was quenched with saturated aqueous ammonium chloride solution (30 mL), then extracted with ethyl acetate (40 mL × 3). The organic phases were combined, washed with saturated brine (20 mL), dried over anhydrous sodium sulfate, filtered, and the filtrate was concentrated under reduced pressure. The residue was purified by HPLC (acidic conditions) to obtain compound 085-3 (440 mg). LC-MS: $[M+H]^+$ = 184.10.

85.4 Synthesis of compound 085-4

At room temperature, 053-3 (500 mg, 2.38 mmol) was dissolved in anhydrous tetrahydrofuran (10 mL). The system was purged with nitrogen three times. After cooling to 0°C and stirring for 10 minutes, a solution of isopropylmagnesium chloride-lithium chloride complex in tetrahydrofuran (3.66 mL, 1.3 M, 4.75 mmol) was added. The reaction mixture was warmed to room temperature and stirred for 30 minutes. After cooling again to 0°C, 085-3 (435 mg, 2.38 mmol) was dissolved in anhydrous tetrahydrofuran (0.5 mL) and added to the above reaction system. The reaction mixture was warmed to room temperature and stirred for 1 hour. Saturated aqueous ammonium chloride solution (30 mL) was added, and the mixture was extracted with ethyl acetate (30 mL × 3). The organic phases were combined, washed with saturated brine (35 mL), dried over anhydrous sodium sulfate, filtered, and the filtrate was concentrated under reduced pressure. The residue was purified by silica gel column chromatography (ethyl acetate/petroleum ether = 1/20) to obtain compound 085-4 (320 mg). LC-MS: $[M+H]^+$ = 254.05.

85.5 Synthesis of compound 085-5

At room temperature, 085-4 (300 mg, 1.18 mmol) was dissolved in ethanol (5 mL). Hydroxylamine hydrochloride (493 mg, 7.09 mmol) and sodium acetate (582 mg, 7.09 mmol) were then added. The reaction mixture was heated to 80°C and reacted for 2 hours. The reaction mixture was diluted with water (20 mL) and extracted with dichloromethane (20 mL × 3). The organic phases were combined, washed with saturated brine (30 mL), dried over anhydrous sodium sulfate, filtered, and the filtrate was concentrated under reduced pressure to obtain compound 085-5 (195 mg, crude product). LC-MS: $[M+H]^+$ = 269.05.

85.6 Synthesis of compound 085-6

At room temperature, 085-5 (180 mg, 0.67 mmol) and zinc powder (263 mg, 4.02 mmol) were dissolved in a mixture of methanol (3 mL) and hydrochloric acid (4.5 mL, 4 N). The reaction was carried out at room temperature for 4 hours. The pH was adjusted to 8 with saturated aqueous sodium bicarbonate solution. Water (15 mL) was added, and the mixture was extracted with ethyl acetate (20 mL × 3). The organic phases were combined, washed with saturated brine (20 mL), dried over anhydrous sodium sulfate, filtered, and the filtrate was concentrated under reduced pressure to obtain compound 085-6 (165 mg, crude product). LC-MS: $[M+H]^+$= 255.10.

85.7 Synthesis of compound 085

085-6 (90 mg, 0.35 mmol) was dissolved in ethanol (2 mL). Then, 001-8 (108 mg, 0.35 mmol) and *N,N*-diisopropylethylamine (226 mg, 1.75 mmol) were added, and the reaction mixture was stirred at room temperature for 2 hours. The reaction mixture was purified by HPLC (basic conditions) and lyophilized to obtain compound 085 (27.9 mg). LC-MS: $[M+H]^+$ = 514.10.

$^1$H NMR (400 MHz, DMSO-$d_6$) δ 9.19 (d, *J* = 9.4 Hz, 1H), 7.92 (ddd, *J* = 14.4, 13.4, 5.56 Hz, 3H), 7.59 (dd, *J* = 8.8, 3.4 Hz, 1H), 5.69 (d, *J* = 9.8 Hz, 1H), 3.16 (s, 3H), 3.05 (s, 3H), 1.63 (dd, *J* = 13.2, 5.6 Hz, 1H), 1.51 (dd, *J* = 15.8, 8.2 Hz, 2H), 1.35 (d, *J* = 3.6 Hz, 2H), 1.26 - 1.17 (m, 1H), 1.07 (d, *J* = 35.2 Hz, 3H), 0.85 (td, *J* = 8.0, 4.4 Hz, 1H), 0.03 (dt, *J* = 8.6, 4.4 Hz, 1H).

Example 86. Synthesis of (*R*)-3-hydroxy-*N,N*-bis(methyl-$d_3$)-4-((2-(((1-methylcyclopentyl)(3-methylpyridin-2-yl)methyl)amino)-3,4-dioxocyclobut-1-en-1-yl)amino)picolinamide (compound 086)

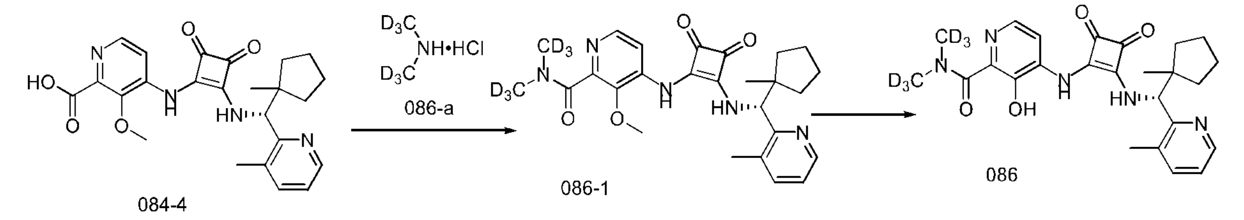


The synthesis of compound 086 was carried out with reference to the synthesis method for compound 084 in Example 84, except that the starting material 084-2 was replaced with 086-a, to obtain compound 086 (33.5 mg, yield: 46.2%, chiral purity: 100% (HPLC method: CHIRALPAK AD-H, 4.6 * 250 mm 5 μm, 15% ethanol/n-hexane, 0.8 mL/min, 30°C)). LC-MS: $[M+H]^+$ = 470.1.

$^1$H NMR (400 MHz, DMSO-$d_6$) δ 9.33 (d, *J* = 12.0 Hz, 1H), 8.44 (d, *J* = 4.0 Hz, 1H), 7.95 (d, *J* = 4.0 Hz, 1H), 7.89 (s, 1H), 7.61 (d, *J* = 8.0 Hz, 1H), 7.24-7.21 (m, 1H), 5.72 (d, *J* = 8.0 Hz, 1H), 2.37 (s, 3H), 1.74-1.71 (m, 2H), 1.56-1.49 (m, 4H), 1.32-1.29 (m, 1H), 1.21-1.17 (m, 1H), 0.97 (s, 3H).

Example 87. Synthesis of 3-((2-((((*S*)-4,4-difluoro-2-methyltetrahydrofuran-2-yl)(3-methylpyridin-2-yl)methyl)amino)-3,4-dioxocyclobut-1-en-1-yl)amino)-6-fluoro-2-hydroxy-*N,N*-dimethylbenzamide (087A or 087B) and 3-((2-((((*R*)-4,4-difluoro-2-methyltetrahydrofuran-2-yl)(3-methylpyridin-2-yl)methyl)amino)-3,4-dioxocyclobut-1-en-1-yl)amino)-6-fluoro-2-hydroxy-*N,N*-dimethylbenzamide (087B or 087A)

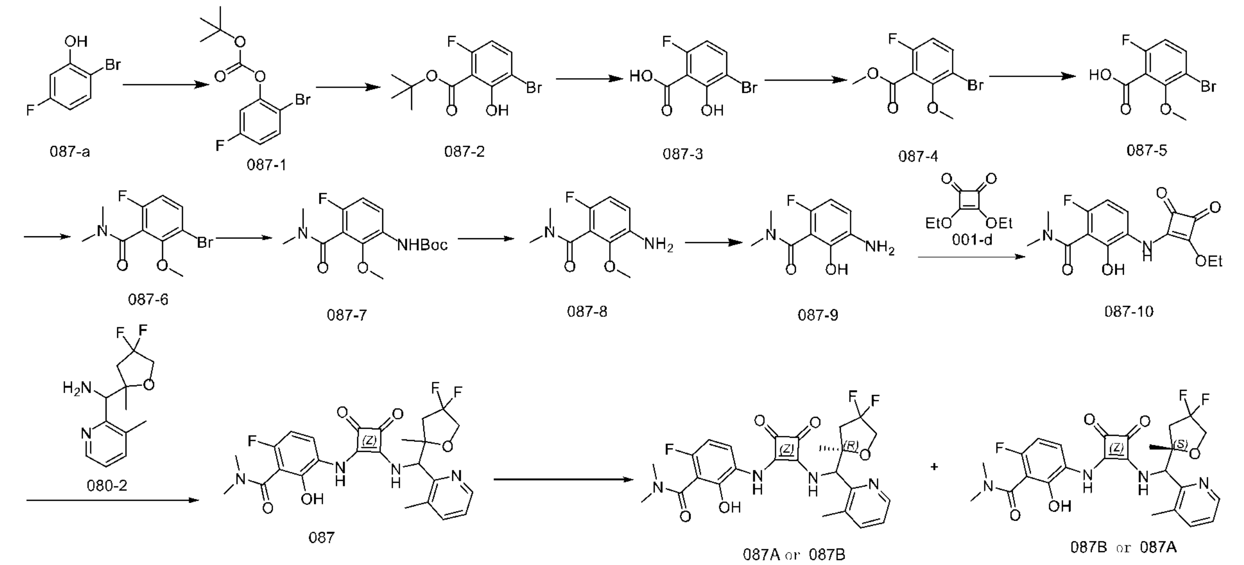


87.1 Synthesis of compound 087-1

087-a (10 g, 52.36 mmol) was weighed into a reaction flask. Dichloromethane (150 mL), 4-dimethylaminopyridine (640 mg, 5.24 mmol), and di-*tert*-butyl dicarbonate (17.14 g, 78.53 mmol) were added. The reaction mixture was stirred at room temperature for 12 hours. The reaction mixture was concentrated under reduced pressure. The residue was purified by silica gel column chromatography to obtain compound 087-1 (15 g, yield: 98%).

87.2 Synthesis of compound 087-2

087-1 (7 g, 24.05 mmol) was weighed into a reaction flask, and dichloromethane (100 mL) was added. Under a nitrogen atmosphere, the temperature was lowered to -78°C. Freshly prepared lithium diisopropylamide (36.1 mL, 1.0 M,

36.07 mmol) was added dropwise slowly, and the reaction mixture was stirred at -78°C for 1.5 hours. The reaction mixture was slowly warmed to room temperature, and the reaction was quenched with 2 N hydrochloric acid (50 mL), then extracted with ethyl acetate (60 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 087-2 (5.5 g, yield: 78.6%).

87.3 Synthesis of compound 087-3

087-2 (5 g, 17.18 mmol) was weighed into a reaction flask. Dichloromethane (10 mL) and trifluoroacetic acid (10 mL) were added, and the reaction mixture was stirred at room temperature for 12 hours. The reaction mixture was filtered, and the filter cake was eluted with dichloromethane (60 mL × 2). The filter cake was dried to obtain compound 087-3 (2.5 g, crude product).

87.4 Synthesis of compound 087-4

087-3 (2 g, 8.51 mmol) was weighed into a reaction flask. *N,N*-Dimethylformamide (30 mL), potassium carbonate (3.62 g, 25.53 mmol), and iodomethane (5.88 g, 42.55 mmol) were added. The reaction mixture was stirred at 60°C for 4 hours. The reaction mixture was added with water (50 mL) and extracted with ethyl acetate (60 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 087-4 (2.0 g, yield: 78.6%). LC-MS: $[M+H]^+$ = 264.95.

87.5 Synthesis of compound 087-5

087-4 (2 g, 7.6 mmol) was weighed into a reaction flask. Ethanol (15 mL), water (5 mL), and sodium hydroxide (912 mg, 22.81 mmol) were added. The reaction mixture was stirred at 80°C for 2 hours. The reaction mixture was cooled to room temperature, and 2 N hydrochloric acid (30 mL) was added to adjust the pH to 2-3. The mixture was extracted with ethyl acetate (60 mL × 3). The organic phases were combined, washed with saturated brine (100 mL), dried over anhydrous sodium sulfate, filtered, and the filtrate was concentrated under reduced pressure to obtain compound 087-5 (1.8 g, yield: 95%).

87.6 Synthesis of compound 087

The synthesis of compound 087 was carried out with reference to the synthesis method for compound 063 in Example 63, except that the starting material 063-3 was replaced with 087-5 and 018-2 was replaced with 080-2, to obtain compound 087 (200 mg, yield: 83%). LC-MS: $[M+H]^+$= 519.05.

87.7 Synthesis of compounds 087A and 087B

087 was resolved into isomers using a chiral column to obtain 087A (52.4 mg, yield: 52.4%) and 087B (33.7 mg, yield: 33.7%).

Compound 087A:

Chiral purity: 96.3%, $T_R$ = 13.805 min, (HPLC method: CHIRALPAK AD-H, 4.6 * 250 mm 5 μm, 15% ethanol/n-hexane, 0.8 mL/min, 30°C);
$^1$H NMR (400 MHz, DMSO-$d_6$) δ 9.03 (d, *J* = 8.0 Hz, 1H), 8.46 (d, *J* = 4.0 Hz, 1H), 7.66 (t, *J* = 12.0 Hz, 2H), 7.29 (dd, *J* = 16.0, 8.0 Hz, 1H), 6.70 (t, *J* = 8.0 Hz, 1H), 5.92 (d, *J* = 8.0 Hz, 1H), 3.97-3.89 (m, 1H), 3.11-3.03 (m, 1H), 2.98 (s, 3H), 2.85 (s, 3H), 2.52 (s, 1H), 2.41 (s, 3H), 2.34 - 2.14 (m, 1H), 1.48 (s, 3H);

Compound 087B:

Chiral purity: 98.7%, $T_R$ = 21.050 min (HPLC method: CHIRALPAK AD-H, 4.6 * 250 mm 5 μm, 15% ethanol/*n*-hexane, 0.8 mL/min, 30°C);
$^1$H NMR (400 MHz, DMSO-$d_6$) δ 9.85 (d, *J* = 224 Hz, 1H), 9.02 (d, *J* = 8.0 Hz, 1H), 8.49 (d, *J= 4.0* Hz, 1H), 7.68-7.64 (m, 2H), 7.32-7.29 (m, 1H), 6.76 (t, *J* = 8.0 Hz, 1H), 5.93 (d, *J* = 8.0 Hz, 1H), 4.11 - 3.90 (m, 2H), 3.09 - 2.93 (m, 4H), 2.83 (s, 3H), 2.41 (s, 3H), 2.37-2.28 (m, 1H), 1.22 (s, 3H).

Example 88. Synthesis of (R)-6-chloro-3-((2-(((3,3-difluoro-1-methylcyclobutyl)(3-methylpyridin-2-yl)methyl)amino)-3,4-dioxocyclobut-l-en-1-yl)amino)-2-hydroxy-N,N-dimethylbenzamide (compound 088)

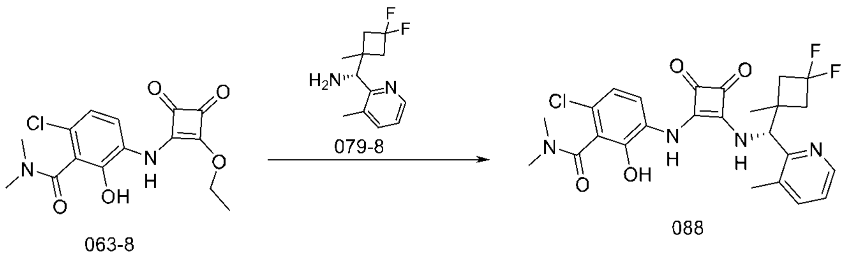


063-8

088

The synthesis of compound 088 was carried out with reference to the synthesis method for compound 079 in Example 79, except that the starting material 001-8 was replaced with 063-8, to obtain compound 088 (12.0 mg, yield: 8.7%, chiral purity: 100% (HPLC method: CHIRALPAK AD-H, 4.6 * 250mm 5 μm, 20% ethanol/n-hexane, 0.8 mL/min, 30°C)). LC-MS: $[M+H]^{+}$= 519.1.

$^{1}H$ NMR (400 MHz, DMSO-$d_6$) δ 9.02 (dd, $J_1$ = 12.0 Hz, $J_2$ = 28.0 Hz, 1H), 8.45 (s, 1H), 7.67-7.63 (m, 2H), 7.29-7.24 (m, 1H), 6.86 (s, 1H), 5.07 (d, $J_1$ = 8.0 Hz, 1H), 3.31 (s, 3H), 3.10-2.97 (m, 5H), 2.36 (s, 3H), 2.30-2.25 (m, 2H), 0.97 (d, $J_1$ =8.0 Hz, 3H).

Example 89. Synthesis of (*R*)-3-((2-(((3,3-difluoro-1-methylcyclobutyl)(3-methylpyridin-2-yl)methyl)amino)-3,4-dioxocyclobut-1-en-1-yl)amino)-6-fluoro-2-hydroxy-*N,N*-dimethylbenzamide (compound 089)

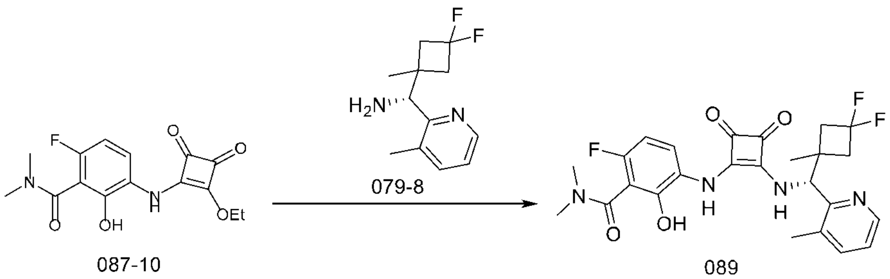


087-10

089

The synthesis of compound 089 was carried out with reference to the synthesis method for compound 079 in Example 79, except that the starting material 001-8 was replaced with 087-10, to obtain compound 089 (53.6 mg, yield: 47.7%, chiral purity: 100% (HPLC method: CHIRALPAK AD-H, 4.6 * 250mm 5 μm, 15% ethanol/n-hexane, 0.8 mL/min, 30°C)). LC-MS: $[M+H]^{+}$ = 503.1.

$^{1}H$ NMR (400 MHz, DMSO-$d_6$) δ 8.95 (d, *J* = 8.0 Hz, 1H), 8.42 (s, d, *J* = 4.0 Hz, 1H) 1H), 7.65-7.61 (m, 2H), 7.26-7.23 (m, 1H), 6.59 (s, 1H), 5.74 (d, $J_1$=*8.0* Hz, 1H), 3.32 (s, 3H), 2.05-2.68 (m, 5H), 2.35 (s, 3H), 2.30-2.19 (m, 2H), 1.20 (s, 3H).

Example 90. Synthesis of (*R*)-6-fluoro-2-hydroxy-*N*-isopropyl-*N*-methyl-3-((2-(((1-methylcyclopentyl)(3-methylpyrazin-2-yl)methyl)amino)-3,4-dioxocyclobut-1-en-1-yl)amino)benzamide (compound 090)

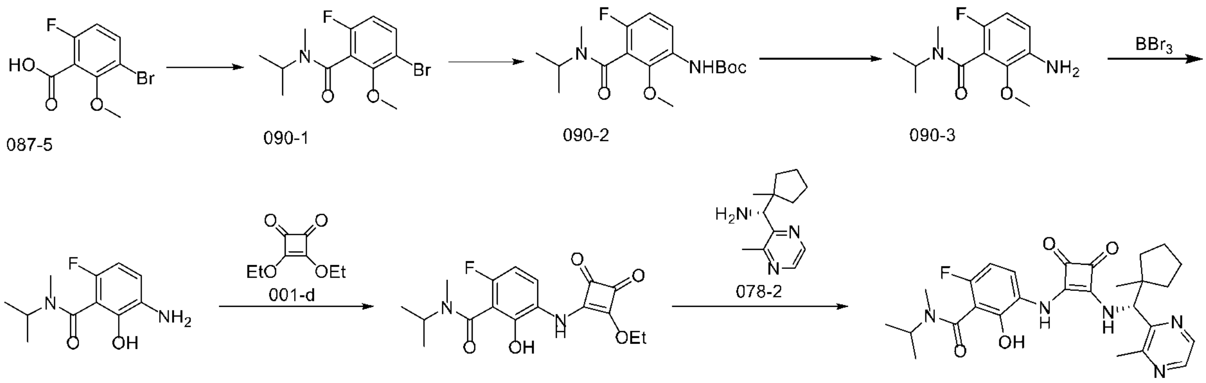


087-5 090-1 090-2 090-3

090-4 090-5 090

90.1 Synthesis of compound 090-1

087-5 (1.5 g, 6.02 mmol) was dissolved in N,N-dimethylformamide (20 mL). *N,N*-Diisopropylethylamine (2.34 g, 18.07 mmol), *N,N,N',N'*-tetramethyl-O-(7-azabenzotriazol-1-yl)uronium hexafluorophosphate (3.44 g, 9.03 mmol), and N-isopropylmethylamine (528.64 mg, 7.23 mmol) were added. The reaction was carried out at room temperature for 2 hours. Water (20 mL) and ethyl acetate (25 mL) were added for extraction. The organic phase was separated, and the aqueous phase was further extracted with ethyl acetate (20 mL × 3). The organic phases were combined, washed with saturated brine (30 mL × 3), dried over anhydrous sodium sulfate, filtered, and the filtrate was concentrated under reduced pressure. The residue was purified by silica gel column chromatography (petroleum ether/ethyl acetate = 1/3) to obtain compound 090-1 (1.2 g, yield: 65.5%). LC-MS: $[M+H]^+$ = 303.95.

90.2 Synthesis of compound 090

The synthesis of compound 090 was carried out with reference to the synthesis method for compound 063 in Example 63, except that the starting material 063-4 was replaced with 090-1 and 018-2 was replaced with 078-2, to obtain compound 090 (64.08 mg, chiral purity: 100% (HPLC method: CHIRALPAK AD-H, 4.6 * 250 mm 5 μm, 20% isopropanol/n-hexane, 0.8 mL/min, 30°C)). LC-MS: $[M+H]^+$ = 510.15.

$^1$H NMR (400 MHz, DMSO-$d_6$) δ10.12 (s, 1H), 9.57 (s, 1H), 8.91 (d, *J* = 8.0 Hz, 1H), 8.55 (s, 1H), 8.50 (d, *J* = 4.0 Hz, 1H),7.67 - 7.51 (m, 1H), 6.74 (m, 1H), 5.75 (d, *J* = 8.0 Hz, 1H), 4.26 (d, *J* = 440.0 Hz, 1H), 2.86 (s, 3H), 2.64 (s, 3H), 1.79 - 1.67 (m, 2H), 1.58 (m, 4H), 1.39 - 1.28 (m, 1H), 1.28 - 1.15 (m, 1H), 1.09 (d, *J* = 28.0 Hz, 6H), 1.00 (s, 3H).

Example 91. Synthesis of (*R*)-3-hydroxy-N,N-dimethyl-4-((2-(((1-methylcyclopentyl)(3-methylpyrazin-2-yl)methyl)amino)-3,4-dioxocyclobut-1-en-1-yl)amino)picolinamide (compound 091)

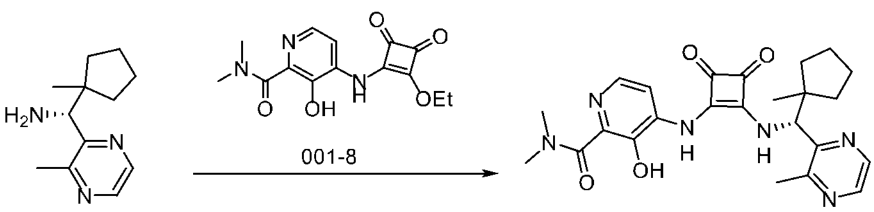


078-2 091

The synthesis of compound 091 was carried out with reference to the synthesis method for compound 001 in Example 1, except that the starting material 001-10 was replaced with 078-2, to obtain compound 091 (84.7 mg, chiral purity: 83.8% (HPLC method: CHIRALPAK AD-H, 4.6 * 250 mm 5 μm, 15% ethanol/n-hexane, 0.8 mL/min, 30°C)). LC-MS: $[M+H]^+$ = 465.10.

$^1$H NMR (400 MHz, DMSO-$d_6$) δ 10.02 (s, 1H), 9.31 (s, 1H), 8.54 (d, *J* = 8.0 Hz, 1H), 8.48 (d, *J* = 4.0 Hz, 1H), 7.96 (d, *J* = 4.0 Hz, 2H), 5.74 (d, *J* = 8.0 Hz, 2H), 3.09 (d, *J* = 52.0 Hz, 6H), 2.62 (s, 3H), 1.77 - 1.65 (m, 2H), 1.64 - 1.49 (m, 4H), 1.36 - 1.27 (m, 1H), 1.24 - 1.15 (m, 1H), 0.97 (s, 3H).

Example 92. Synthesis of (*R*)-4-((2-(((3-cyclopropylpyridin-2-yl)(1-methylcyclopentyl)methyl)amino)-3,4-dioxocyclobut-1-en-1-yl)amino)-3-hydroxy-*N,N*-dimethylpicolinamide (compound 092)

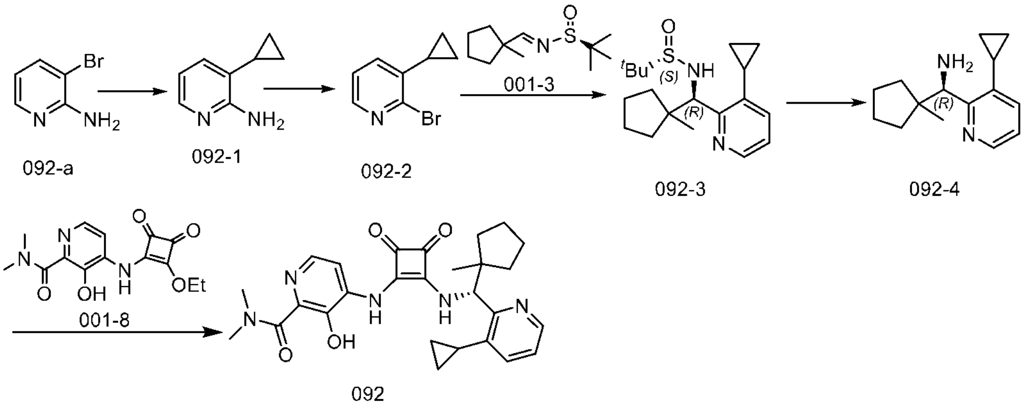


92.1 Synthesis of compound 092-1

092-a (1.038 g, 6.0 mmol) was weighed into a reaction flask. Toluene (24 mL), water (2.4 mL), cyclopropylboronic acid (670 mg, 7.8 mmol), potassium phosphate (3.821 g, 18.0 mmol), tricyclohexylphosphine (670 mg, 7.8 mmol), and palladium acetate (136 mg, 0.6 mmol) were added. Under a nitrogen atmosphere, the mixture was stirred at 110°C for 2 hours. Water (25 mL) was added, and the mixture was extracted with ethyl acetate (20 mL × 3). The organic phases were combined, washed with saturated brine (50 mL), dried over anhydrous sodium sulfate, filtered, and the filtrate was directly concentrated under reduced pressure. The residue was purified by silica gel column chromatography to obtain compound 092-1 (0.805 g, yield: 100%). LC-MS: $[M+H]^+$ = 135.15.

92.2 Synthesis of compound 092-2

092-1 (0.742 g, 5.0 mmol) was weighed into a reaction flask. Dichloromethane (20 mL), copper(II) bromide (0.558 g, 2.5 mmol), and *tert*-butyl nitrite (0.654 mL, 5.5 mmol) were added. The reaction was carried out at room temperature for 2 hours. Water (50 mL) was added, and the mixture was extracted with ethyl acetate (60 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 092-2 (566 mg, yield: 51.4%). LC-MS: $[M+H]^+$ = 200.00.

92.3 Synthesis of compound 092

The synthesis of compound 092 was carried out with reference to the synthesis method for compound 001 in Example 1, except that the starting material 001-e was replaced with 092-2, to obtain compound 092 (8 mg, yield: 12.7%, chiral purity: 100% (HPLC method: CHIRALPAK AD-H, 4.6 * 250 mm 5 μm, 20% isopropanol/n-hexane, 0.8 mL/min, 30°C)).

$^1$H NMR (400 MHz, DMSO-$d_6$) δ 9.40 (d, *J* = 8.0 Hz, 1H), 8.46 (d, *J* = 4.0 Hz, 1H), 7.99-7.94 (m, 2H), 7.40-7.38 (m, 1H), 7.27-7.24 (m, 1H), 6.13 (d, *J* = 12.0 Hz, 1H), 3.16 (s, 3H), 3.04 (s, 3H), 2.09 - 1.81 (m, 1H), 1.79 - 1.61 (m, 2H), 1.59 - 1.52 (m, 4H), 1.35 -1.33 (m, 1H), 1.23 -1.22 (m, 1H), 1.06 -1.01 (m, 5H), 0.72 -0.65 (m, 2H).

Example 93. Synthesis of 4-((2-(((*S*)-((*S*)-4,4-difluoro-2-methyltetrahydrofuran-2-yl)(5-fluoro-3-methylpyridin-2-yl)methyl)amino)-3,4-dioxocyclobut-1-en-1-yl)amino)-3-hydroxy-*N,N*-dimethylpicolinamide (093A or 093B) and 4-((2-(((*S*)-((*R*)-4,4-difluoro-2-methyltetrahydrofuran-2-yl)(5-fluoro-3-methylpyridin-2-yl)methyl)amino)-3,4-dioxocyclobut-1-en-1-yl)amino)-3-hydroxy-N,N-dimethylpicolinamide (093B or 093A)

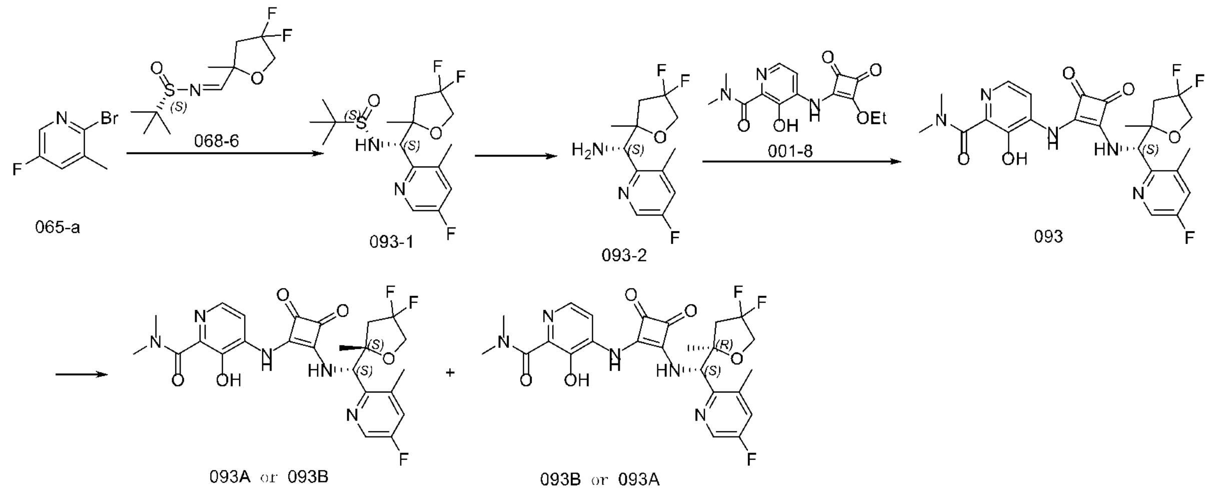


065-a 093-1 093-2 093

093A or 093B + 093B or 093A

93.1 Synthesis of compound 093

The synthesis of compound 093 was carried out with reference to the synthesis method for compound 001 in Example 1, except that the starting material 001-e was replaced with 065-a and 001-3 was replaced with 068-6, to obtain compound 093 (141.0 mg). LC-MS: $[M+H]^+$ = 520.10.

$^1H$ NMR (400 MHz, DMSO-$d_6$) δ 11.68 (s, 1H), 9.96 (s, 1H), 9.35 (s, 1H), 8.50 (dd, *J* = 4.0, 4.0 Hz, 1H), 7.96 (d, *J= 4.0* Hz, 2H), 7.67 (dt, *J* = 4.0, 4.0 Hz, 1H), 5.88 (dd, *J=4.0,* 4,0 Hz, 1H), 3.96 (dt, *J* = 24.0, 12.0 Hz, 1H), 3.41 (dd, *J* = 12.0, 8.0 Hz, 1H), 3.14 (s, 3H), 3.02 (s, 3H), 3.01 - 2.80 (m, 1H), 2.42 (s, 3H), 2.29 (ddd, *J* = 28.0, 24.0, 16.0 Hz, 1H), 1.32 (d, *J* = 96.0 Hz, 3H).

93.2 Synthesis of compounds 093A and 093B

Compound 093 was subjected to chiral resolution to obtain compound 093A (28.2 mg) and compound 093B (38.2 mg).

Characterization data for 093A are as follows:

Chiral purity: 100%, $T_R$ = 10.938 min (HPLC method: CHIRALPAK AD-H, 4.6 * 250 mm 5 μm, 15% ethanol/n-hexane, 0.8 mL/min, 30°C).
$^1H$ NMR (400 MHz, DMSO-$d_6$) δ 9.44 (d, *J* = 9.0 Hz, 1H), 8.53 (d, *J* = 2.0 Hz, 1H), 8.02 (d, *J* = 4.0 Hz, 1H), 7.95 (s, 1H), 7.74 (dd, *J* = 8.0, 4.0 Hz, 1H), 5.95 (d, *J* = 8.0 Hz, 1H), 4.01 (dd, *J* = 12.0, 8.0 Hz, 1H), 3.50 - 3.36 (m, 1H), 3.11 (d, *J* = 32.0 Hz, 6H), 2.98 (d, *J* = 20.0 Hz, 1H), 2.50 (s, 3H), 2.30 (dd, *J* = 12.0, 4.0 Hz,1H), 1.53 (s, 3H);

Characterization data for 093B are as follows:
Chiral purity: 97.7%, $T_R$ = 15.786 min (HPLC method: CHIRALPAK AD-H, 4.6 * 250 mm 5 μm, 15% ethanol/n-hexane, 0.8 mL/min, 30°C).

$^1H$ NMR (400 MHz, DMSO-$d_6$) δ 9.33 (d, *J* = 4.0 Hz, 1H), 8.45 (s, 1H), 7.95 (d, *J* = 8.0 Hz, 1H), 7.88 (s, 1H), 7.70 - 7.58 (m, 1H), 5.88 (d, *J* = 12.0 Hz, 1H), 4.07 - 3.86 (m, 2H), 3.05 (d, *J* = 40.0 Hz, 6H), 2.92 (d, *J* = 16.0 Hz, 1H), 2.41 (s, 3H), 2.31 (td, *J* = 16.0, 16.0 Hz, 1H), 1.19 (s, 3H).

Example 94. Synthesis of (*R*)-4-((2-(((3-fluoro-6-(fluoromethyl)pyridin-2-yl)(1-methylcyclopentyl)methyl)amino)-3,4-dioxocyclobut-1-en-1-yl)amino)-3-hydroxy-*N,N*-dimethylpicolinamide (compound 094)

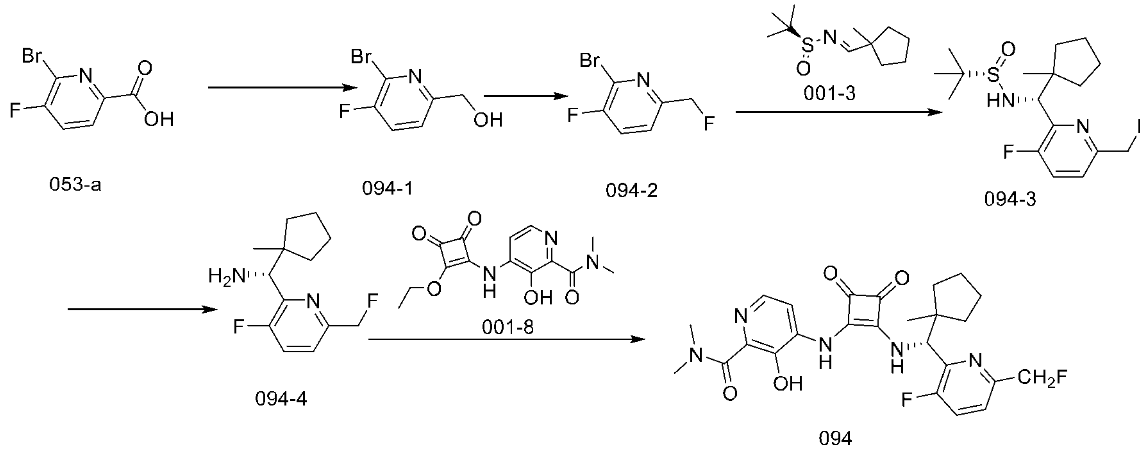


053-a 094-1 094-2 094-3

094-4 094

94.1 Synthesis of compound 094-1

053-a (2 g, 9.09 mmol) was dissolved in tetrahydrofuran (20 mL). The mixture was cooled to 0°C using an ice-water bath, and a solution of borane in tetrahydrofuran (1 M, 27 mL, 27.27 mmol) was slowly added. After the addition was completed, the reaction mixture was warmed to 80°C and reacted for 2 hours. The reaction mixture was quenched with methanol (20 mL) and then concentrated under reduced pressure. The residue was purified by silica gel column chromatography to obtain compound 094-1 (1.2 g). LC-MS: $[M+H]^+$ = 205.95.

94.2 Synthesis of compound 094-2

094-1 (1.2 g, 5.82 mmol) was dissolved in dichloromethane (10 mL). The temperature was lowered to -78°C, and diethylaminosulfur trifluoride (2.8 g, 17.47 mmol) was added dropwise slowly. The reaction mixture was stirred at -78°C for 1 hour, then warmed to room temperature and reacted for 3 hours. The reaction mixture was added with water (10 mL) and extracted with dichloromethane (10 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 094-2 (400 mg). LC-MS: $[M+H]^+$ = 207.95.

94.3 Synthesis of compound 094-3

094-2 (400 mg, 1.92 mmol) was dissolved in tetrahydrofuran (4 mL). The mixture was cooled to 0°C in an ice-water bath, and a solution of isopropylmagnesium chloride-lithium chloride complex in tetrahydrofuran (1.3 M, 1.9 mL, 2.5 mmol) was added dropwise slowly. The reaction mixture was warmed to room temperature and reacted for 1 hour. The reaction mixture was again cooled to 0°C in an ice-water bath, and a solution of 001-3 (373 mg, 1.73 mmol) in tetrahydrofuran (2 mL) was added dropwise slowly. After the addition was completed, the reaction mixture was warmed to room temperature and reacted for 1 hour. The reaction mixture was quenched with saturated aqueous ammonium chloride solution (10 mL), then extracted with ethyl acetate (10 mL × 3). The organic phases were combined, washed with saturated brine (10 mL), dried over anhydrous sodium sulfate, filtered, and the filtrate was concentrated under reduced pressure. The residue was purified by silica gel column chromatography to obtain compound 094-3 (140 mg). LC-MS: $[M+H]^+$=345.10.

94.4 Synthesis of compound 094-4

094-3 (140 mg, 0.42 mmol) was dissolved in a solution of hydrogen chloride in 1,4-dioxane (4 M, 1 mL). The reaction was carried out at room temperature for 1 hour. The reaction mixture was concentrated under reduced pressure. The residue was purified by silica gel column chromatography to obtain compound 094-4 (110 mg, crude product). LC-MS: $[M+H]^+$ = 241.15.

94.5 Synthesis of compound 094

094-4 (110 mg, 0.46 mmol) was dissolved in ethanol (1 mL). N,N-Diisopropylethylamine (118 mg, 0.91 mmol) and 001-8 (139 mg, 0.46 mmol) were added, and the reaction was carried out at room temperature for 1 hour. The reaction mixture was purified by HPLC (basic conditions) and lyophilized to obtain compound 094 (28.4 mg, chiral purity: 95.3%

(HPLC method: CHIRALPAKAD-H, 4.6 * 250 mm 5 μm, 15% ethanol/n-hexane, 0.8 mL/min, 30°C)). LC-MS: $[M+H]^+$ = 500.15.

$^1H$ NMR (400 MHz, DMSO-$d_6$) δ 11.59 (s, 1H), 10.03 (s, 1H), 9.30 (d, *J* = 8.4 Hz, 1H), 7.93 (s, 2H), 7.84 (t, *J* = 9.2 Hz, 1H), 7.57 (dd, *J* = 8.4, 3.6 Hz, 1H), 5.84 (d, *J* = 10.0 Hz, 1H), 5.52 (d, *J* = 46.8 Hz, 2H), 3.16 (s, 3H), 3.04 (s, 3H), 1.77 - 1.71 (m, 1H), 1.68 - 1.56 (m, 5H), 1.36 - 1.28 (m, 1H), 1.21 - 1.15 (m, 1H), 1.00 (s, 3H).

Example 95. Synthesis of 3-((2-(((4,4-difluoro-2-methyltetrahydrofuran-2-yl)(3-methylpyridin-2-yl)methyl)amino)-3,4-dioxocyclobut-1-en-1-yl)amino)-2-hydroxy-*N,N*-dimethylbenzamide (compound 095)

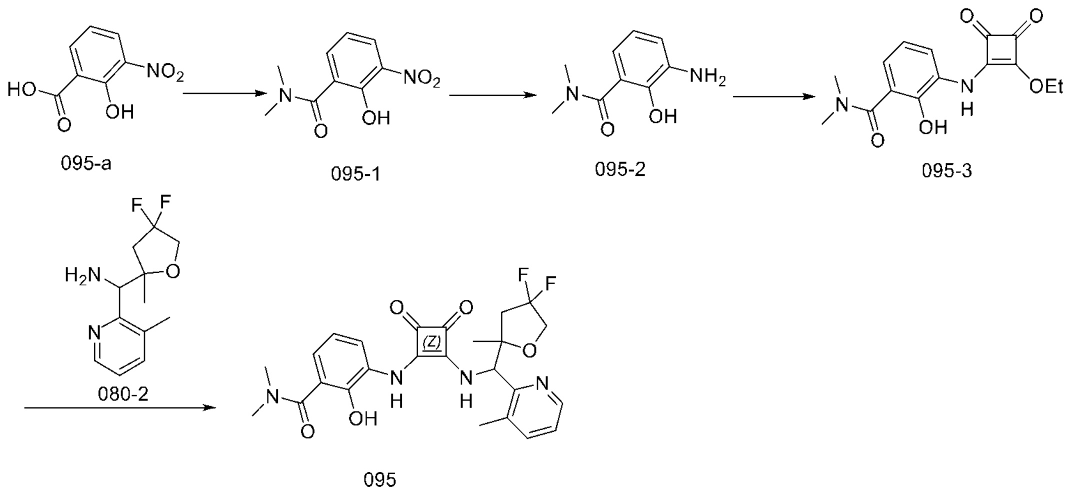


095-a

095-1

095-2

095-3

095

95.1 Synthesis of compound 095-1

095-a (300.0 mg, 1.64 mmol) was dissolved in dichloromethane (10 mL). The system was purged with nitrogen three times. At 0°C, oxalyl chloride (374.27 mg, 2.95 mmol) was added dropwise slowly, followed by the addition of 2 drops of *N,N*-dimethylformamide. The reaction was carried out at room temperature for 1 hour. The reaction mixture was concentrated under reduced pressure to remove the solvent. The residue was dissolved in dichloromethane (10 mL). Triethylamine (0.68 mL, 4.91 mmol) and dimethylamine (147.72 mg, 3.28 mmol) were added, and the reaction was carried out at room temperature for 1 hour. The reaction mixture was concentrated under reduced pressure. The residue was purified by silica gel column chromatography (petroleum ether/ethyl acetate = 1/1) to obtain compound 095-1 (180.0 mg, yield: 52.27%). LC-MS: $[M+H]^+$ = 211.05.

95.2 Synthesis of compound 095-2

095-1 (160.0 mg, 0.76 mmol) was dissolved in ethanol (5 mL). Palladium on carbon (90.0 mg, 10%) was added, and the system was purged with hydrogen three times. The reaction was carried out at room temperature for 3 hours. The reaction mixture was filtered, and the filtrate was concentrated under reduced pressure to obtain compound 095-2 (120.0 mg, crude product), which was directly used in the next step. LC-MS: $[M+H]^+$ = 181.10.

95.3 Synthesis of compound 095-3

095-2 (115.0 mg, crude product) was dissolved in ethanol (5 mL). *N,N*-Diisopropylethylamine (247.44 mg, 1.91 mmol) and diethyl squarate (108.59 mg, 0.64 mmol) were added, and the reaction was carried out at room temperature for 2 hours. The solvent was removed by concentration under reduced pressure, and the residue was purified by silica gel column chromatography (petroleum ether/ethyl acetate = 1/1) to obtain compound 095-3 (158.0 mg). LC-MS: $[M+H]^+$ = 305.05.

95.4 Synthesis of compound 095

095-3 (158.0 mg, 0.52 mmol) was dissolved in ethanol (5 mL). *N,N*-Diisopropylethylamine (201.32 mg, 1.56 mmol) and 080-2 (125.79 mg, crude product) were added, and the reaction was carried out at room temperature overnight.

The reaction mixture was purified by preparative HPLC to obtain compound 095 (139.6 mg). LC-MS: $[M+H]^+$ = 501.10.

$^1$H NMR (400 MHz, DMSO-$d_6$) δ 9.91 (s, 1H), 9.64 (s, 1H), 9.11 (t, *J* = 8.0 Hz, 1H), 8.49 (t, *J* = 4.0 Hz, 1H), 7.77 - 7.55 (m, 2H), 7.37 - 7.15 (m, 1H), 6.89 - 6.75 (m, 2H), 5.92 (dd, *J* = 8.0, 4.0 Hz, 1H), 4.10 - 3.86 (m, 2H), 3.05 (dd, *J* = 16.0, 4.0 Hz, 1H), 2.92 (s, 6H), 2.40 (s, 3H), 2.36 - 2.14 (m, 1H), 1.34 (d, *J* = 104.0 Hz, 3H).

Example 96. Synthesis of (*R*)-4-((2-(((3,3-difluoro-1-methylcyclobutyl)(5-fluoro-3-methylpyridin-2-yl)methyl)amino)-3,4-dioxocyclobut-1-en-1-yl)amino)-3-hydroxy-*N,N*-dimethylpicolinamide (compound 096)

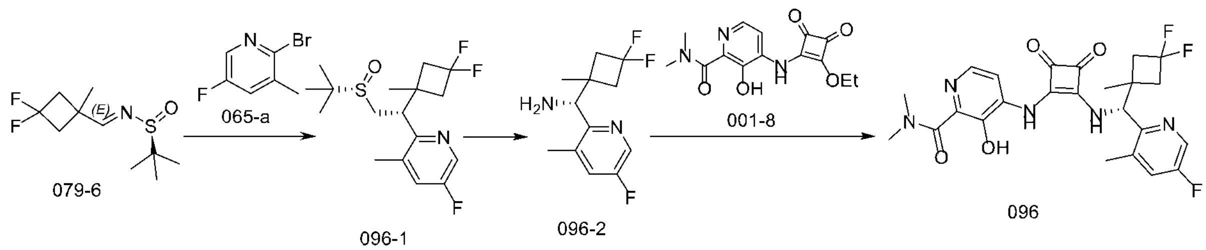


The synthesis of compound 096 was carried out with reference to the synthesis method for compound 074 in Example 74, except that the starting material 074-3 was replaced with 079-6 and 018-a was replaced with 065-a, to obtain compound 096 (57.4 mg, yield: 42.5%, chiral purity: 98.5% (HPLC method: CHIRALPAK AD-H, 4.6 * 250 mm 5 μm, 20% isopropanol/n-hexane, 0.8 mL/min, 30°C)). LC-MS: $[M+H]^+$ = 504.15.

$^1$H NMR (400 MHz, DMSO-$d_6$) δ 11.68 (s, 1H), 9.97 (s, 1H), 9.29 (s, 1H), 8.53 (s, 1H), 8.00 (s, 2H), 7.70 (d, *J* = 9.7 Hz, 1H), 5.75 (d, *J* = 9.7 Hz, 1H), 3.17 (s, 3H), 3.05 (s, 3H), 2.80 (q, *J* = 15.6 Hz, 2H), 2.41 (s, 3H), 2.36 - 2.23 (m, 2H), 1.23 (s, 3H).

Example 97. Synthesis of 4-((2-(((1*S*)-(4,4-difluoro-2-methyltetrahydrofuran-2-yl)(6-(difluoromethyl)-3-fluoropyridin-2-yl)methyl)amino)-3,4-dioxocyclobut-1-en-1-yl)amino)-3-hydroxy-*N,N*-dimethylpicolinamide (compound 097)

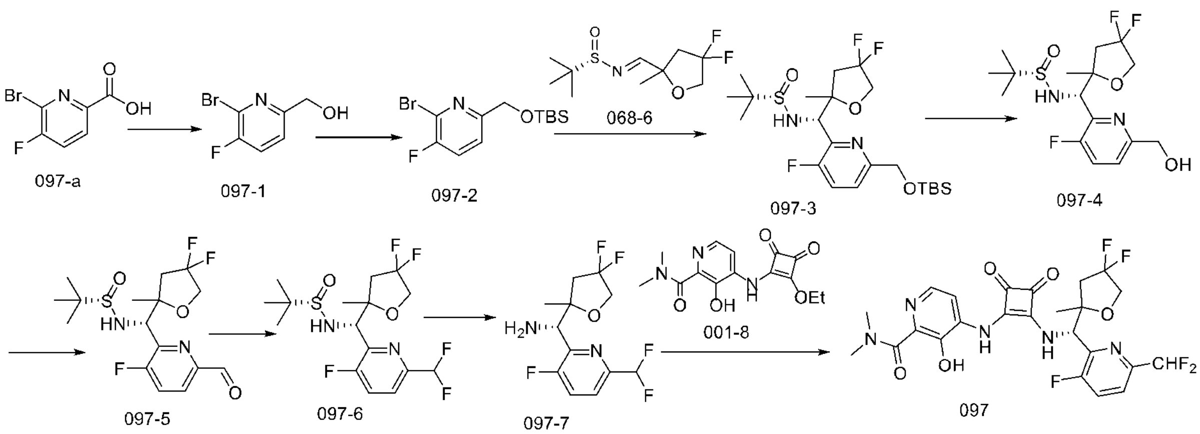


97.1 Synthesis of compound 097-1

097-a (2.0 g, 9.09 mmol) was dissolved in tetrahydrofuran (5 mL). At 0°C, a solution of borane in tetrahydrofuran (1 M, 27.2 mL, 27.2 mmol) was added, and the reaction was carried out at 85°C for 2 hours. The reaction mixture was quenched with saturated aqueous sodium bicarbonate solution. The aqueous phase was extracted with ethyl acetate (30 mL × 3). The organic phases were retained, dried, and concentrated under reduced pressure to obtain compound 097-1 (2.0 g, crude product). LC-MS: $[M+H]^+$ = 205.95.

97.2 Synthesis of compound 097-2

097-1 (2.0 g, 0.13 mmol) was dissolved in dichloromethane (20 mL). Imidazole (793 mg, 11.65 mmol) and *tert*-butyldimethylchlorosilane (1.76 g, 11.65 mmol) were added. The reaction was carried out at 25°C for 2 hours. The reaction mixture was quenched with an aqueous solution (30 mL). The aqueous phase was extracted with dichloromethane (30 mL × 3). The organic phases were retained, dried over anhydrous sodium sulfate, and concentrated under reduced pressure. The residue was purified by silica gel column chromatography (petroleum ether/ethyl acetate = 10/1) to obtain compound 097-2 (1.2 g, yield: 38.6%).

97.3 Synthesis of compound 097-3

097-2 (1.0 g, 3.12 mmol) was dissolved in tetrahydrofuran (10 mL). At -78°C, *n*-butyllithium (2.5 M, 1.5 mL, 3.75 mmol) was added. After the reaction was carried out for 1 hour, 068-6 (790.9 mg, 3.12 mmol) was added, and the reaction was carried out at -78°C for another 2 hours. The reaction mixture was quenched with saturated ammonium chloride solution (30 mL). The aqueous phase was extracted with ethyl acetate (30 mL × 3). The organic phases were retained, dried over anhydrous sodium sulfate, and concentrated under reduced pressure. The residue was purified by silica gel column chromatography (petroleum ether/ethyl acetate = 2/1) to obtain compound 097-3 (700 mg, yield: 45.3%).

97.4 Synthesis of compound 097-4

097-3 (600 mg, 1.21 mmol) was dissolved in tetrahydrofuran (2 mL). A solution of tetrabutylammonium fluoride in tetrahydrofuran (1 M, 5.0 mL, 5.0 mmol) was added, and the reaction was carried out at 25°C for 2 hours. The reaction mixture was quenched with water (30 mL). The aqueous phase was extracted with ethyl acetate (30 mL × 3). The organic phases were retained, dried over anhydrous sodium sulfate, and concentrated under reduced pressure. The residue was separated by silica gel column chromatography (petroleum ether/ethyl acetate = 2/1) to obtain compound 097-4 (400 mg, yield: 86.9%).

97.5 Synthesis of compound 097-5

Oxalyl chloride (186.8 mg, 1.47 mmol) was dissolved in dichloromethane (2 mL). At -78°C, dimethyl sulfoxide (115 mg, 1.47 mmol) was added, and the reaction was carried out for 15 minutes. Then, 097-4 (280 mg, 0.73 mmol) was added. After 0.5 hours, the reaction mixture was warmed to 25°C and reacted for 2 hours. The reaction mixture was quenched with water (30 mL). The aqueous phase was extracted with ethyl acetate (30 mL × 3). The organic phases were retained, dried over anhydrous sodium sulfate, and concentrated under reduced pressure. The residue was separated by silica gel column chromatography (petroleum ether/ethyl acetate = 3/1) to obtain compound 097-5 (200 mg, yield: 71.8%).

97.6 Synthesis of compound 097-6

097-5 (200 mg, 0.52 mmol) was dissolved in dichloromethane (2 mL). Diethylaminosulfur trifluoride (100.5 mg, 0.62 mmol) was added, and the reaction was carried out at 25°C for 2 hours. The reaction mixture was quenched with saturated aqueous sodium bicarbonate solution (30 mL). The aqueous phase was extracted with ethyl acetate (30 mL × 3). The organic phases were retained, dried over anhydrous sodium sulfate, and concentrated under reduced pressure. The residue was purified by silica gel column chromatography (petroleum ether/ethyl acetate = 5/1) to obtain compound 097-6 (80 mg, yield: 37.8%).

97.7 Synthesis of compound 097-7

097-6 (80 mg, 0.19 mmol) was dissolved in a solution of hydrogen chloride in ethyl acetate (1 M, 2 mL), and the reaction was carried out at 25°C for 1 hour. The reaction mixture was concentrated under reduced pressure to obtain compound 097-7 (50 mg, crude product).

97.8 Synthesis of compound 097

097-7 (50.0 mg, 0.16 mmol) was dissolved in ethanol (2 mL). Triethylamine (34.1 mg, 0.33 mmol) and 001-8 (51.5 mg, 0.11 mmol) were added, and the reaction was carried out at room temperature for 2 hours. The reaction mixture was separated by preparative HPLC to obtain compound 097 (16.0 mg, yield: 17.1%, chiral purity: 88.7% (HPLC method: CHIRALPAK AD-H, 4.6 * 250 mm 5 μm, 20% ethanol/n-hexane, 0.8 mL/min, 30°C)). LC-MS: $[M+H]^+$ = 556.1.

$^1H$ NMR (400 MHz, DMSO-$d_6$) δ 9.32 (s, 2H), 7.99-7.79 (m, 4H), 7.03 (t, *J* = 52.0 Hz, 1H), 6.06 (s, 1H), 4.11-4.89 (m, 2H), 3.12 (s, 3H), 3.02 (s, 3H), 2.93-2.85 (m, 1H), 2.38-2.36 (m, 1H), 1.26 (s, 3H).

Example 98. Synthesis of 4-((2-((((4,4-difluoro-2-methyltetrahydrofuran-2-yl)(3-fluoro-6-(trifluoromethyl)pyridin-2-yl) methyl)amino)-3,4-dioxocyclobut-1-en-1-yl)amino)-3-hydroxy-*N,N*-dimethylpicolinamide (compound 098)

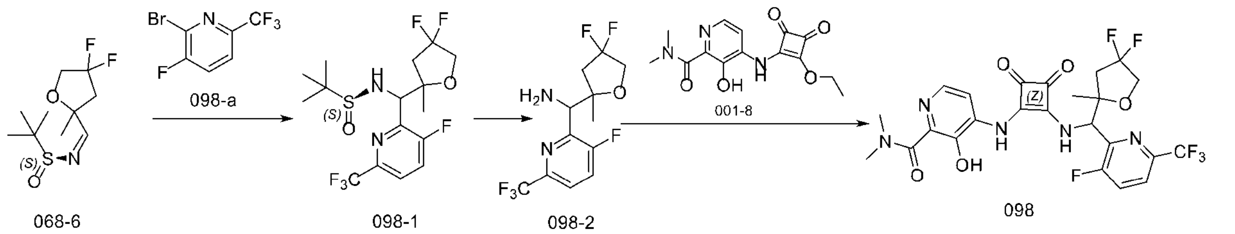


The synthesis of compound 098 was carried out with reference to the synthesis method for compound 068 in Example 68, except that the starting material 053-3 was replaced with 098-a, to obtain compound 098 (28.6 mg). LC-MS: $[M+H]^+$ = 574.00.

$^1H$ NMR (400 MHz, DMSO-$d_6$) δ 9.28 (s, 1H), 8.14 - 8.03 (m, 2H), 7.92 (dd, *J* = 8.8, 5.8 Hz, 2H), 6.10 (dd, *J* = 14.8, 5.8 Hz, 1H), 4.14 - 4.01 (m, 1H), 3.95 - 3.68 (m, 1H), 3.13 (s, 3H), 3.04 (s, 3H), 2.91 - 2.80 (m, 1H), 2.39 (td, *J* = 16.6, 8.6 Hz, 1H), 1.38 (d, *J* = 58.2 Hz, 3H).

Example 99. Synthesis of (*R*)-4-((2-(((6-bromo-3-methylpyridin-2-yl)(1-methylcyclopentyl)methyl)amino)-3,4-dioxocyclobut-1-en-1-yl)amino)-3-hydroxy-*N,N*-dimethylpicolinamide (compound 099)

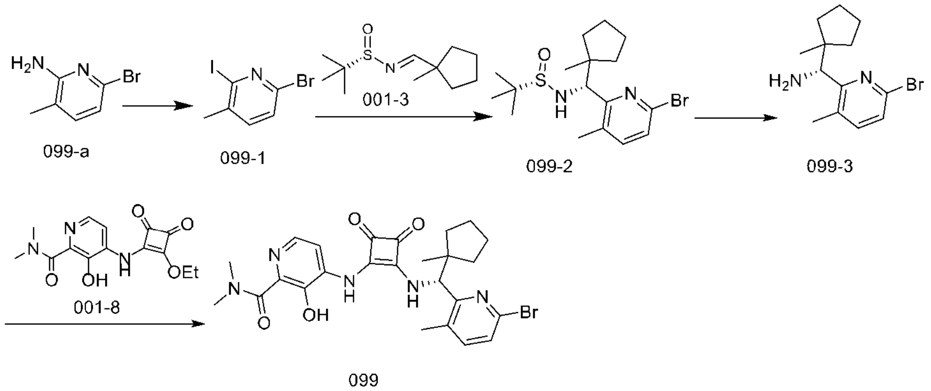


99.1 Synthesis of compound 099-1

099-a (2.50 g, 13.37 mmol), copper(I) iodide (3.82 g, 20.05 mmol), diiodomethane (28.64 g, 106.93 mmol), and *tert*-butyl nitrite (5.51 g, 53.46 mmol) were dissolved in tetrahydrofuran (50 mL). The system was purged with nitrogen three times. The reaction mixture was reacted at 70°C for 4 hours. The reaction mixture was poured into water (50 mL) and extracted with ethyl acetate (60 mL × 3). The organic phases were combined, washed with saturated brine (60 mL), dried over anhydrous sodium sulfate, filtered, and the filtrate was concentrated under reduced pressure. The residue was purified by HPLC and lyophilized to obtain compound 099-1 (1.72 g, yield: 43.2%).

99.2 Synthesis of compound 099

The synthesis of compound 099 was carried out with reference to the synthesis method for compound 001 in Example 1, except that the starting material 001-e was replaced with 099-1, to obtain compound 099 (59.7 mg, chiral purity: 94.1% (HPLC method: CHIRALPAK AD-H, 4.6 * 250 mm 5 μm, 15% ethanol/n-hexane, 0.8 mL/min, 30°C)). LC-MS: $[M+H]^+$ = 542.05.

$^1H$ NMR (400 MHz, DMSO-$d_6$) δ 9.12 (d, *J* = 8.6 Hz, 1H), 7.93 (t, *J* = 8.0 Hz, 2H), 7.58 (d, *J* = 8.2 Hz, 1H), 7.47 (d, *J* = 8.2 Hz, 1H), 5.68 (d, *J* = 10.0 Hz, 1H), 3.14 (s, 3H), 3.02 (s, 3H), 2.35 (s, 3H), 1.73 - 1.64 (m, 2H), 1.55 (dd, *J* = 18.0, 9.8 Hz, 4H), 1.36 - 1.30 (m, 1H), 1.24 - 1.16 (m, 1H), 0.98 (s, 3H).

Example 100. Synthesis of (*R*)-4-((2-(((4-fluoro-3-methylpyridin-2-yl)(1-methylcyclopentyl)methyl)amino)-3,4-dioxocyclobut-1-en-1-yl)amino)-3-hydroxy-*N,N*-dimethylpicolinamide (compound 100)

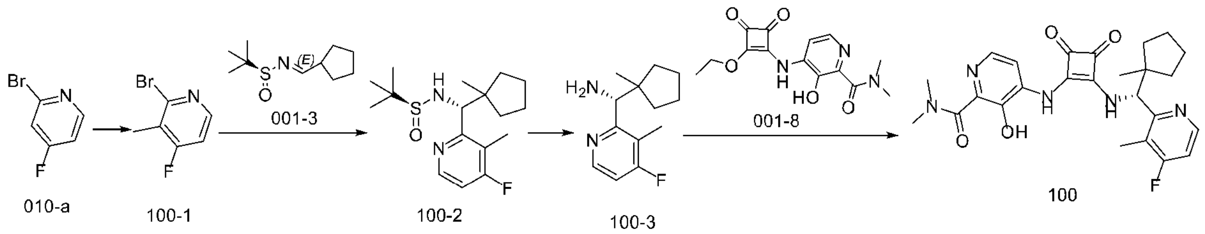


100.1 Synthesis of compound 100-1

Super-dry tetrahydrofuran (20 mL) and diisopropylamine (1.72 g, 17.05 mmol) were added to a three-necked flask. The system was purged with nitrogen three times. At -78°C, a solution of *n*-butyllithium (5.91 mL, 2.5 M) was slowly added. The reaction mixture was slowly warmed to room temperature and reacted for 1 hour. At -78°C, 010-a (2.0 g, 11.36 mmol) was slowly added, and the reaction was carried out at this temperature for another 1 hour. Iodomethane (1.77 g, 12.50 mmol) was then slowly added, and the reaction was carried out at -78°C for another 1 hour. Saturated aqueous ammonium chloride solution was added to quench the reaction, followed by extraction with ethyl acetate (20 mL × 3). The organic phases were combined, washed with saturated brine (20 mL × 3), dried over anhydrous sodium sulfate, filtered, and the filtrate was concentrated under reduced pressure. The residue was purified by silica gel column chromatography (petroleum ether/ethyl acetate = 3/1) to obtain compound 100-1 (780.0 mg, yield: 36%). LC-MS: $[M+H]^+$ = 189.95.

100.2 Synthesis of compound 100

The synthesis of compound 100 was carried out with reference to the synthesis method for compound 001 in Example 1, except that the starting material 001-e was replaced with 100-1, to obtain compound 100 (44.7 mg, chiral purity: 99.6% (HPLC method: CHIRALPAK AD-H, 4.6 * 250 mm 5 μm, 20% ethanol/n-hexane, 0.8 mL/min, 30°C)). LC-MS: $[M+H]^+$ = 482.10.

$^1$H NMR (400 MHz, DMSO-$d_6$) δ 9.34 (d, *J* = 8.0 Hz, 1H), 8.50 (dd, *J* = 4.0, 4.0 Hz, 1H), 7.95 (t, *J* = 20.0 Hz, 2H), 7.24 (dd, *J* = 8.0, 4.0 Hz, 1H), 5.77 (d, *J* = 10.0 Hz, 1H), 3.09 (d, *J* = 48.0 Hz, 6H), 2.30 (s, 3H), 1.71 (dd, J= 8.0, 4.0 Hz, 2H), 1.63 - 1.48 (m, 4H), 1.37 - 1.28 (m, 1H), 1.26 - 1.14 (m, 1H), 0.97 (s, 3H).

Example 101. Synthesis of 4-((2-(((5-fluoro-3-methylpyridin-2-yl)(3-methylbicyclo[3.1.0]hexan-3-yl)methyl)amino)-3,4-dioxocyclobut-1-en-1-yl)amino)-3-hydroxy-*N,N*-dimethylpicolinamide (compound 101)

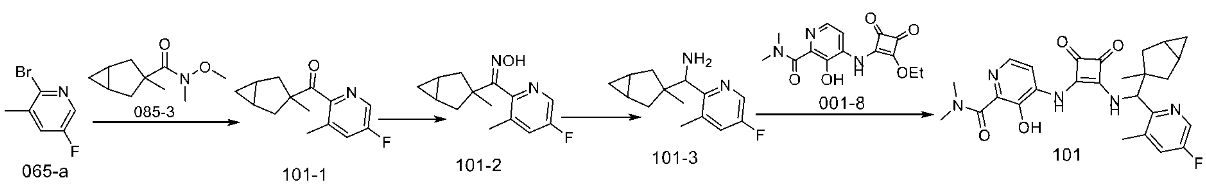


The synthesis of compound 101 was carried out with reference to the synthesis method for compound 085 in Example 85, except that the starting material 053-3 was replaced with 065-a, to obtain compound 101 (40.5 mg). LC-MS: $[M+H]^+$ = 494.20.

$^1$H NMR (400 MHz, DMSO-$d_6$) δ 9.19 (dd, *J* = 33.8, 9.8 Hz, 1H), 8.39 (t, *J* = 5.2 Hz, 1H), 7.90 (d, *J* = 5.4 Hz, 1H), 7.78 (d, *J* = 5.0 Hz, 1H), 7.59 (dd, *J* = 9.8, 2.6 Hz, 1H), 5.63 (t, *J* = 18.4 Hz, 1H), 2.99 (d, *J* = 13.8 Hz, 6H), 2.41 (d, *J* = 19.6 Hz, 3H), 1.54 (ddd, *J* = 32.6, 16.6, 9.8 Hz, 4H), 1.29 - 1.23 (m, 2H), 1.02 (d, *J= 42.4* Hz, 3H), 0.76 (td, *J*= 8.0, 4.4 Hz, 1H), -0.10 (d, *J*= 3.8 Hz, 1H).

Example 102. Synthesis of (R)-N-(4-((2-(((5-fluoro-3-methylpyridin-2-yl)(1-methylcyclopentyl)methyl)amino)-3,4-dioxocyclobut-1-en-1-yl)amino)-3-hydroxypyridin-2-yl)-N-methylacetamide (compound 102)

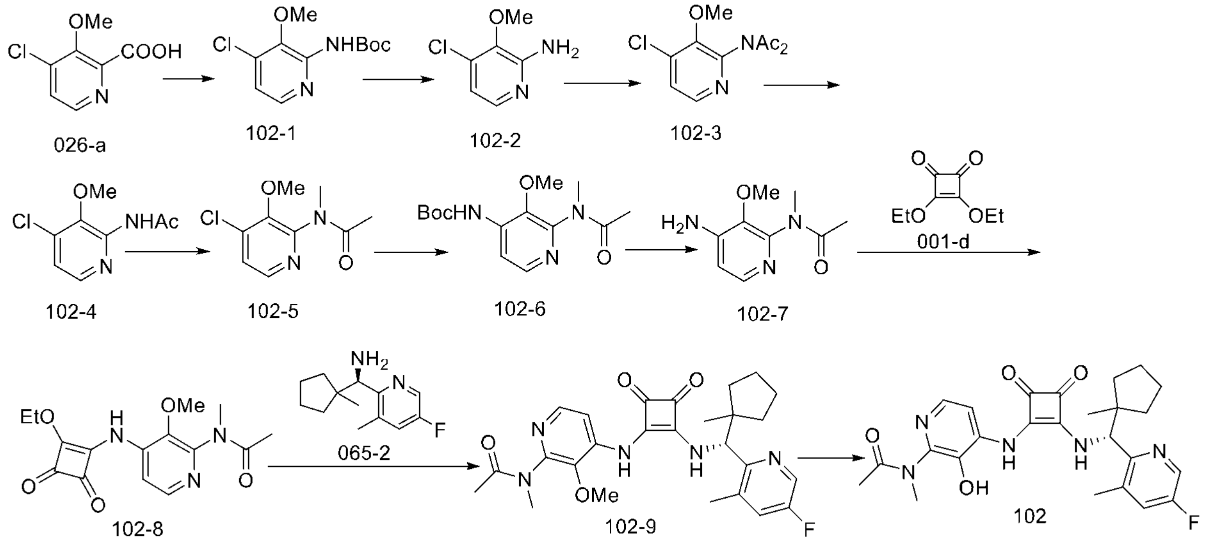


026-a 102-1 102-2 102-3 102-4 102-5 102-6 102-7 102-8 102-9 102

102.1 Synthesis of compound 102-1

At room temperature, 026-a (2.251 g, 12 mmol) was dissolved in *tert-butanol* (30 mL). Diphenylphosphoryl azide (3.142 mL, 15.6 mmol) and triethylamine (2.5 mL, 18 mmol) were added. The reaction mixture was refluxed at 85°C for 4 hours. The reaction mixture was concentrated under reduced pressure. The residue was diluted with water (200 mL) and extracted with ethyl acetate (80 mL × 3). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 102-1 (2.762 g, 10.7 mmol). LC-MS: $[M+H]^+$ = 259.05.

102.2 Synthesis of compound 102-2

102-1 (2.36 g, 9.12 mmol) was dissolved in 1,4-dioxane (12 mL). A solution of hydrogen chloride in 1,4-dioxane (12 mL, 4 M) was added, and the reaction was carried out at room temperature for 4 hours. The reaction mixture was concentrated under reduced pressure to obtain compound 102-2 (1.5 g), which was directly used in the next step. LC-MS: $[M+H]^+$ = 159.10.

102.3 Synthesis of compound 102-3

102-2 (1.5 g) was dissolved in dichloromethane (24 mL). Triethylamine (6.34 mL, 45.6 mmol) was added. The temperature was lowered to 0°C, and acetyl chloride (1.56 mL, 21.9 mmol) was added dropwise slowly. The reaction mixture was warmed to room temperature and reacted for 2 hours. The reaction mixture was cooled to 0°C, and methanol (5 mL) was slowly added dropwise to quench the reaction. The mixture was then transferred to room temperature and stirred for another 30 minutes. The reaction mixture was concentrated under reduced pressure. The residue was diluted with water (200 mL) and extracted with ethyl acetate (80 mL × 3). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 102-3 (1.556 g, 6.4 mmol). LC-MS: $[M+H]^+$ = 243.05.

102.4 Synthesis of compound 102-4

102-3 (1.556 g, 6.41 mmol) was dissolved in tetrahydrofuran (20 mL). Ammonia (1 mL, 30% solution in water) was added, and the reaction mixture was stirred at room temperature for 6 hours. The reaction mixture was concentrated under reduced pressure. The residue was purified by silica gel column chromatography (petroleum ether/ethyl acetate = 1/3) to obtain compound 102-4 (838 mg, 4.2 mmol). LC-MS: $[M+H]^+$ = 201.05.

102.5 Synthesis of compound 102-5

102-4 (838 mg, 4.18 mmol) was dissolved in *N,N*-dimethylformamide (16.7 mL). The system was purged with nitrogen three times. After the temperature was lowered to 0°C, sodium hydride (60% purity, 200 mg, 5 mmol) was added, and the mixture was stirred for 30 minutes. The reaction mixture was then transferred to room temperature, and iodomethane (390 μL, 6.27 mmol) was added. The reaction was carried out for another 2 hours. Saturated aqueous ammonium chloride solution (10 mL) was added to quench the reaction, and the mixture was extracted with ethyl acetate (50 mL × 3). The organic phase was dried over anhydrous sodium sulfate, filtered, and the filtrate was concentrated under reduced pressure. The residue was purified by silica gel column chromatography (petroleum ether/ethyl acetate = 1/2) to obtain compound 102-5 (826 mg, 3.85 mmol). LC-MS: $[M+H]^+$ = 215.05.

102.6 Synthesis of compound 102-6

102-5 (692 mg, 3.22 mmol), tert-butyl carbamate (754 mg, 6.44 mmol), potassium carbonate (890 mg, 6.44 mmol), tris(dibenzylideneacetone)dipalladium (295 mg, 0.322 mmol), and 2-dicyclohexylphosphino-2',4',6'-triisopropylbiphenyl (307 mg, 0.644 mmol) were dissolved in 1,4-dioxane (13 mL). The system was purged with nitrogen three times and refluxed at 110°C for 8 hours. The reaction mixture was filtered, and the filtrate was concentrated under reduced pressure. The residue was diluted with water (200 mL) and extracted with ethyl acetate (60 mL × 3). The organic phase was dried over anhydrous sodium sulfate, filtered, and the filtrate was concentrated under reduced pressure. The residue was purified by silica gel column chromatography (petroleum ether/ethyl acetate = 1/3) to obtain compound 102-6 (902 mg, 3.05 mmol). LC-MS: $[M+H]^+$ = 296.10.

102.7 Synthesis of compound 102-7

102-6 (871 mg, 2.95 mmol) was dissolved in a solution of hydrogen chloride in 1,4-dioxane (14 mL, 4 M). The reaction was carried out at room temperature for 4 hours. The reaction mixture was concentrated under reduced pressure. To the residue, saturated aqueous sodium carbonate solution was added to adjust the pH to 10. The mixture was extracted with dichloromethane (50 mL × 3). The organic phase was dried over anhydrous sodium sulfate, filtered, and the filtrate was concentrated under reduced pressure. The residue was purified by silica gel column chromatography (dichloromethane/methanol = 16/1) to obtain compound 102-7 (273 mg, 1.4 mmol). LC-MS: $[M+H]^+$ = 196.10.

102.8 Synthesis of compound 102-8

102-7 (249 mg, 1.275 mmol) was dissolved in N,Ndimethylformamide (5 mL). The system was purged with nitrogen three times. After cooling to 0°C, sodium hydride (60% purity, 76.5 mg, 1.91 mmol) was added, and the reaction mixture was stirred for 30 minutes. The reaction mixture was then transferred to room temperature, and diethyl squarate (377 μL, 2.55 mmol) was added. The reaction was carried out for another 2 hours. Saturated aqueous ammonium chloride solution (10 mL) was added to quench the reaction. The mixture was extracted with dichloromethane (50 mL × 4). The organic phase was dried over anhydrous sodium sulfate, filtered, and the filtrate was concentrated under reduced pressure. The residue was purified by silica gel column chromatography (dichloromethane/methanol = 16/1) to obtain compound 102-8 (218 mg, 0.68 mmol). LC-MS: $[M+H]^+$ = 320.05.

102.9 Synthesis of compound 102-9

102-8 (202 mg, 0.63 mmol) and *N,N*-diisopropylethylamine (274 μL, 1.58 mmol) were dissolved in ethanol (3 mL). At room temperature, 065-2 (93 mg, 0.63 mmol) was added, and the reaction was carried out for 2 hours. The reaction mixture was concentrated under reduced pressure. The residue was purified by silica gel column chromatography (dichloromethane/ethyl acetate = 1/3) to obtain compound 102-9 (111 mg, 0.224 mmol). LC-MS: $[M+H]^+$ = 496.20.

102.10 Synthesis of compound 102

102-9 (106 mg, 0.21 mmol) was dissolved in boron tribromide (1 M, 1.26 mL, 1.26 mmol), and the reaction was carried out at room temperature for 8 hours. The temperature was lowered to 0°C, and methanol (0.5 mL) was slowly added dropwise to quench the reaction. After the addition was completed, the reaction mixture was transferred to room temperature and stirred for another 10 minutes. The reaction mixture was concentrated under reduced pressure, and the residue was purified by preparative HPLC to obtain compound 102 (39.4 mg, 81.8 μmol, chiral purity: 100% (HPLC method: CHIRALPAK AD-H, 4.6 * 250 mm 5 μm, 20% isopropanol/n-hexane, 0.8 mL/min, 30°C)). LC-MS: $[M+H]^+$ = 482.15.

$^1$H NMR (400 MHz, DMSO-$d_6$) δ 10.02 (bs, 1H), 9.29 (d, *J* = 9.6 Hz, 1H), 8.49 (d, *J* = 2.4 Hz, 1H), 7.93 (d, *J*= 5.2 Hz, 1H), 7.85 (s, 1H), 7.64 (dd, $J_1$ = 10.0 Hz, $J_2$ = 2.8 Hz, 1H), 5.72 (d, *J*= 10.0 Hz, 1H), 3.05 (s, 3H), 2.43 (s, 3H), 1.74 (s, 3H), 1.65-1.49 (m, 5H), 1.35-1.30 (m, 1H), 1.28-1.17 (m, 2H), 0.99 (s, 3H).

Example 103. Synthesis of (*R*)-2-hydroxy-*N,N*,6-trimethyl-3-((2-(((1-methylcyclopentyl)(3-methylpyridin-2-yl)methyl) amino)-3,4-dioxocyclobut-1-en-1-yl)amino)benzamide (compound 103)

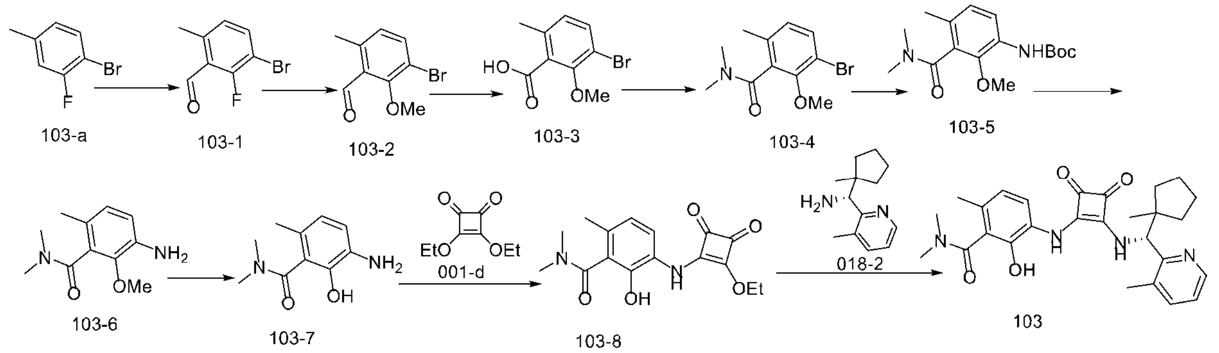


The synthesis of compound 103 was carried out with reference to the synthesis method for compound 063 in Example 63, except that the starting material 063-a was replaced with 103-a, to obtain compound 103 (60.5 mg, yield: 36.7%, chiral purity: 100% (HPLC method: CHIRALPAK AD-H, 4.6 * 250 mm 5 μm, 20% ethanol/n-hexane, 0.8 mL/min, 30°C)). LC-MS: $[M+H]^+$ = 477.1.

$^1$H NMR (400 MHz, DMSO-$d_6$) δ 9.60 (s, 1H), 9.16 (s, 1H), 8.89 (s, 1H), 8.44(s, 1H), 7.60 (d, *J* = 8.0 Hz, 1H), 7.45 (d, *J* = 8.0 Hz, 1H), 7.22 (s, 1H), 6.67 (s, 1H), 5.72 (d, *J* = 8.0 Hz, 1H), 2.98 (s, 3H), 2.73 (s, 3H), 2.38 (s, 3H), 2.03 (s, 3H), 1.75-1.50 (m, 6H), 1.32-1.17 (m, 2H), 0.98 (s, 3H).

Example 104. Synthesis of (*R*)-6-fluoro-2-hydroxy-*N,N*-dimethyl-3-((2-(((1-methylcyclopentyl)(3-methylpyridin-2-yl) methyl)amino)-3,4-dioxocyclobut-1-en-1-yl)amino)benzamide (compound 104)

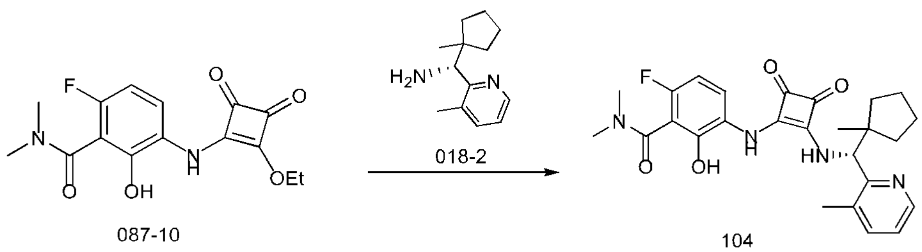


The synthesis of compound 104 was carried out with reference to the synthesis method for compound 001 in Example 1, except that the starting material 001-10 was replaced with 018-2 and 001-8 was replaced with 087-10, to obtain compound 104 (287.4 mg, yield: 64.3%, chiral purity: 100% (HPLC method: CHIRALPAK AD-H, 4.6 * 250 mm 5 μm, 15% ethanol/n-hexane, 0.8 mL/min, 30°C)). LC-MS: $[M+H]^+$ = 481.15.

$^1$H NMR (400 MHz, DMSO-$d_6$) δ 10.09 (s, 1H), 9.63 (s, 1H), 8.93 (d, J= 12.0 Hz, 1H), 8.46 (d, *J= 4.0* Hz, 1H), 7.64-7.60 (m, 2H), 7.25-7.22 (m, 1H), 6.74 (t, *J* = 12.0 Hz, 1H), 5.73 (d, *J* = 12.0 Hz, 1H), 2.99 (s, 3H), 2.82 (s, 3H), 2.39 (s, 3H), 1.80-1.69 (m, 2H), 1.66 - 1.45 (m, 4H), 1.35 - 1.25 (m, 1H), 1.23-1.18 (m, 1H), 0.99 (s, 3H).

Example 105. Synthesis of (R)-3-hydroxy-*N,N*-dimethyl-4-((2-(((3-(methyl-$d_3$)pyridin-2-yl)(1-methylcyclopentyl)methyl) amino)-3,4-dioxocyclobut-1-en-1-yl)amino)picolinamide (compound 105)

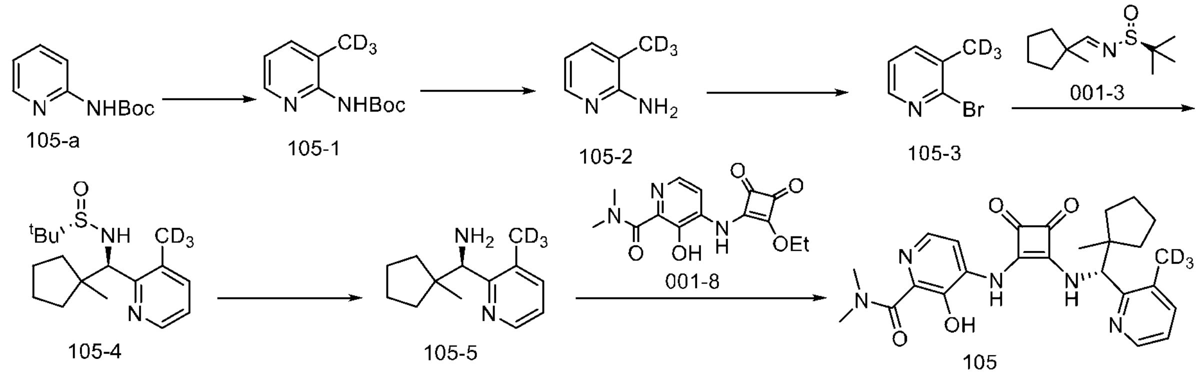


105.1 Synthesis of compound 105-1

A three-necked flask was purged with nitrogen, and 105-a (486 mg, 2.5 mmol), tetrahydrofuran (10 mL), and *N,N,N',N'*-tetramethylethylenediamine (937 μL, 6.25 mmol) were added. The temperature was lowered to -78°C, and *n*-butyllithium (2.5 M, 3 mL, 7.5 mmol) was added. The reaction mixture was warmed to room temperature and stirred for 20 minutes. Then, deuterated iodomethane (233 μL, 3.75 mmol) was added, and the stirring was continued for 2 hours. Saturated aqueous ammonium chloride solution (100 mL) was added to quench the reaction, and the mixture was extracted with ethyl acetate (60 mL × 3). The organic phase was dried over anhydrous sodium sulfate, filtered, and the filtrate was concentrated under reduced pressure. The residue was purified by silica gel column chromatography (petroleum ether/ethyl acetate = 3/1) to obtain compound 105-1 (172 mg, 0.814 mmol). LC-MS: $[M+H]^+$ = 212.15.

105.2 Synthesis of compound 105-2

105-1 (172 mg, 0.814 mmol) was dissolved in 1,4-dioxane (2 mL). A solution of hydrogen chloride in 1,4-dioxane (4 M, 2 mL) was added, and the reaction was carried out at room temperature for 2 hours. The reaction mixture was concentrated under reduced pressure to obtain compound 105-2 (166 mg crude product, 0.814 mmol), which was directly used in the next step. LC-MS: $[M+H]^+$ = 112.20.

105.3 Synthesis of compound 105-3

105-2 (166 mg, crude product) was dissolved in hydrobromic acid (48% solution in water, 2.75 mL). Liquid bromine (110 μL, 2.16 mmol) was added, and the temperature was lowered to -15°C. Sodium nitrite (226 mg, 3.27 mmol) was added, and the reaction mixture was warmed to room temperature and reacted for another 2 hours. At 0°C, the reaction mixture was poured into a mixture of saturated sodium sulfite (25 mL) and saturated sodium carbonate (40 mL). After stirring for 30 minutes, the mixture was extracted with ethyl acetate (20 mL × 3). The organic phase was dried over anhydrous sodium sulfate, filtered, and the filtrate was concentrated under reduced pressure. The residue was purified by silica gel column chromatography (petroleum ether/ethyl acetate = 10/1) to obtain compound 105-3 (45 mg, 0.257 mmol). LC-MS: $[M+H]^+$ = 175.00.

105.4 Synthesis of compound 105

The synthesis of compound 105 was carried out with reference to the synthesis method for compound 001 in Example 1, except that the starting material 001-e was replaced with 105-3, to obtain compound 105 (32.4 mg, 69.44 μmol, chiral purity: 100% (HPLC method: CHIRALPAK AD-H, 4.6 * 250 mm 5 μm, 20% ethanol/n-hexane, 0.8 mL/min, 30°C)). LC-MS: $[M+H]^+$ = 467.10.

$^1H$ NMR (400 MHz, DMSO-$d_6$) δ 9.35 (d, J= 10.0 Hz, 1H), 8.47 (d, J= 4.0 Hz, 1H), 7.97 (d, *J*= 5.2 Hz, 1H), 7.90 (s, 1H), 7.63 (d, *J* = 8.8 Hz, 1H), 7.25 (dd, $J_1$ = 7.2 Hz, $J_2$ = 4.4 Hz, 1H), 5.73 (d, *J= 10.0* Hz, 1H), 3.14 (s, 3H), 3.03 (s, 3H), 1.81 - 1.69 (m, 2H), 1.63 - 1.46 (m, 4H), 1.35 - 1.29 (m, 1H), 1.23 - 1.17 (m, 1H), 1.00 (s, 3H).

Example 106. Synthesis of 4-((2-(((6-bromo-3-chloropyridin-2-yl)(1-methylcyclopentyl)methyl)amino)-3,4-dioxocyclobut-1-en-1-yl)amino)-3-hydroxy-N,N-dimethylpicolinamide (compound 106)

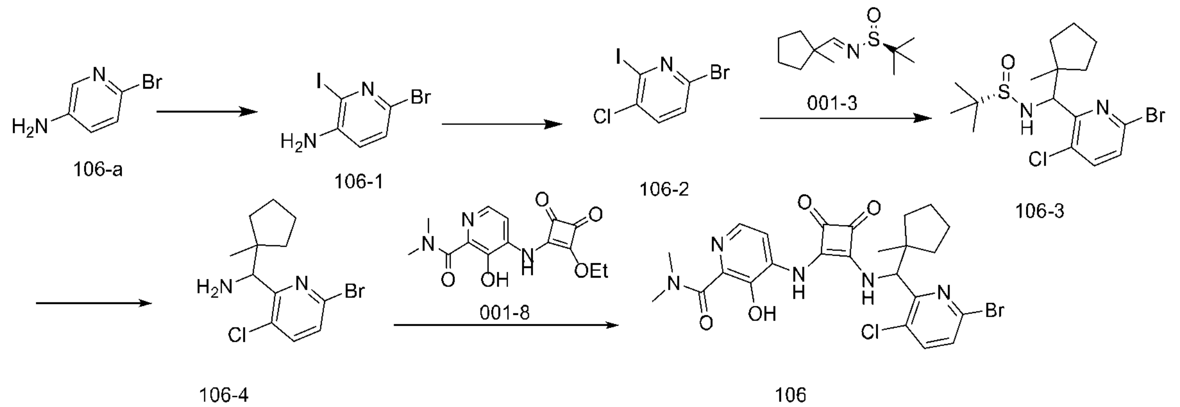


106.1 Synthesis of compound 106-1

106-a (3.0 g, 17.34 mmol) was weighed into a reaction flask. N,N-Dimethylformamide (30 mL) and N-iodosuccinimide (4.68 g, 20.81 mmol) were added, and the reaction mixture was stirred at room temperature for 12 hours. The reaction mixture was added with water (50 mL) and extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 106-1 (3.0 g, yield: 57.9%). LC-MS: $[M+H]^+$ = 298.85.

106.2 Synthesis of compound 106-2

106-1 (2.0 g, 17.34 mmol) was weighed into a reaction flask. Acetonitrile (20 mL), copper(II) chloride (3.6 g, 26.8 mmol), and isoamyl nitrite (4.72 g, 40.2 mmol) were added. The reaction mixture was stirred at room temperature for 4 hours. The reaction mixture was added with water (50 mL) and extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 106-2 (0.9 g, yield: 42%). LC-MS: $[M+H]^+$ = 319.75.

106.3 Synthesis of compound 106

The synthesis of compound 106 was carried out with reference to the synthesis method for compound 001 in Example 1, except that the starting material 001-e was replaced with 106-2, to obtain compound 106 (29.2 mg, yield: 15.7%). LC-MS: $[M+H]^+$ = 564.00.

$^1$H NMR (400 MHz, DMSO-$d_6$) δ 9.17 (d, J= 8.0 Hz, 1H), 7.95-7.91 (m, 3H), 7.66 (d, *J* = 8.0 Hz, 1H), 6.01 (d, *J*= *12.0* Hz, 1H), 3.16 (s, 3H), 3.04 (s, 3H), 1.79 - 1.67 (m, 2H), 1.59 (s, 4H), 1.35-1.31 (m, 1H), 1.27 - 1.17 (m, 1H), 1.02 (s, 3H).

Example 107. Synthesis of (R)-4-((2-(((6-(difluoromethyl)pyridin-2-yl)(1-methylcyclopentyl)methyl)amino)-3,4-dioxocyclobut-1-en-1-yl)amino)-3-hydroxy-N,N-dimethylpicolinamide (compound 107)

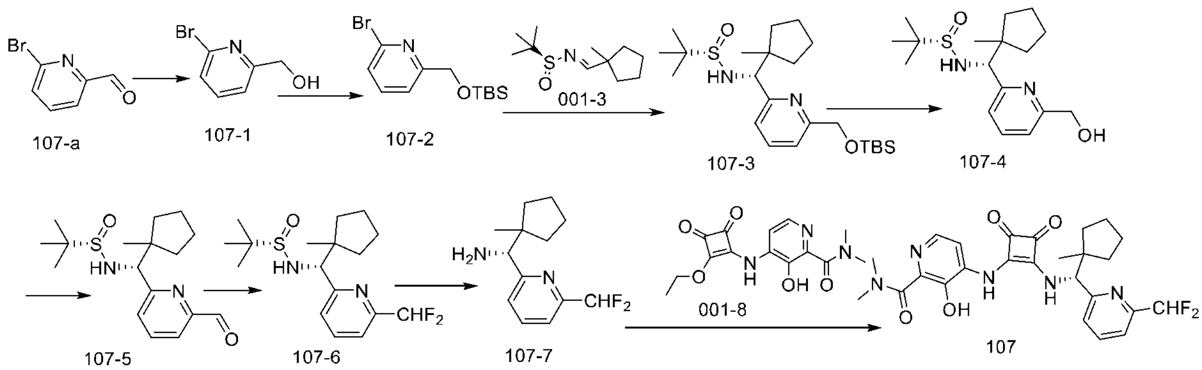

107.1 Synthesis of compound 107-1

107-a (2.00 g, 10.75 mmol) was dissolved in methanol (30 mL). The reaction mixture was cooled to 0°C, and sodium borohydride (488 mg, 12.90 mmol) was slowly added. The reaction mixture was then warmed to 25°C and reacted at this temperature for 1 hour. The reaction mixture was poured into water (20 mL) to quench the reaction, then extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 107-1 (1.96 g, yield: 96.95%).

107.2 Synthesis of compound 107-2

Imidazole (777 mg, 11.42 mmol) and tert-butyldimethylchlorosilane (1.73 g, 11.42 mmol) were dissolved in dichloromethane (21 mL). The reaction mixture was cooled to 0°C, and a solution of 107-1 (1.95 g, 10.37 mmol) in dichloromethane (11 mL) was slowly added to the reaction mixture. The reaction mixture was stirred at room temperature for 1 hour. The reaction mixture was directly concentrated under reduced pressure. The residue was purified by silica gel column chromatography to obtain compound 107-2 (2.94 g, yield: 93.78%).

107.3 Synthesis of compound 107-3

107-2 (1.00 g, 3.37 mmol) was dissolved in tetrahydrofuran (20 mL). The system was purged with nitrogen three times. The reaction mixture was cooled to -78°C, and n-butyllithium (1.59 mL, 3.97 mmol) was added dropwise. The reaction was carried out for 0.5 hours. 001-3 (712 mg, 3.31 mmol) was dissolved in tetrahydrofuran (0.3 mL), and the resulting solution was added dropwise to the reaction mixture. The reaction mixture was reacted at -78°C for 1 hour. The reaction mixture was poured into saturated aqueous ammonium chloride solution (30 mL) to quench the reaction, and extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 107-3 (990 mg, yield: 68.21%). LC-MS: $[M+H]^+$ = 439.20.

107.4 Synthesis of compound 107-4

107-3 (990 mg, 2.26 mmol) was dissolved in tetrahydrofuran (13 mL). The reaction mixture was cooled to 0°C, and tetrabutylammonium fluoride (6.78 mL, 6.78 mmol) was slowly added. The reaction mixture was then warmed to 25°C and reacted at this temperature for 2 hours. The reaction mixture was poured into water (30 mL) to quench the reaction, then extracted with ethyl acetate (30 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 107-4 (550 mg, yield: 75.12%).

107.5 Synthesis of compound 107-5

Oxalyl chloride (0.18 mL, 2.10 mmol) was dissolved in anhydrous dichloromethane (5 mL). The system was purged with nitrogen, and the reaction mixture was cooled to -78°C. Dimethyl sulfoxide (0.30 mL, 4.20 mmol) was slowly added, and the mixture was stirred at this temperature for 0.2 hours. 107-4 (340 mg, 1.05 mmol) was dissolved in anhydrous dichloromethane (5 mL), and the resulting solution was added dropwise to the reaction mixture. The reaction was carried out at -78°C for 1 hour. N,N-Diisopropylethylamine (1.36 mL, 8.40 mmol) was slowly added to the reaction mixture. The reaction mixture was warmed to 0°C and reacted at this temperature for 0.5 hours. The reaction mixture was poured into water (20 mL) to quench the reaction, then extracted with dichloromethane (20 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 107-5 (290 mg, yield: 85.83%).

107.6 Synthesis of compound 107-6

107-5 (290 mg, 0.89 mmol) was dissolved in anhydrous dichloromethane (4 mL). The system was purged with nitrogen three times. The reaction mixture was cooled to 0°C, and diethylaminosulfur trifluoride (188 mg, 1.17 mmol) was added dropwise slowly. The reaction mixture was stirred at 0°C for 2 hours. Methanol (2 mL) was slowly added dropwise to the reaction mixture to quench the reaction. The mixture was then concentrated under reduced pressure. The residue was purified by silica gel column chromatography to obtain compound 107-6 (180 mg, yield: 58.11%). LC-MS: $[M+H]^+$ = 345.10.

107.7 Synthesis of compound 107-7

At room temperature, 107-6 (170 mg, 0.49 mmol) was dissolved in a solution of hydrogen chloride in ethanol (7 mL, 1 M). The reaction mixture was stirred at room temperature for 1 hour and then directly concentrated under reduced pressure to obtain compound 107-7 (110 mg, crude product). LC-MS: $[M+H]^+$ = 241.15.

107.8 Synthesis of compound 107

107-7 (110 mg, 0.45 mmol) was dissolved in ethanol (3 mL). 001-8 (140 mg, 0.45 mmol) and *N,N*-diisopropylethylamine (290 mg, 2.25 mmol) were added, and the reaction mixture was stirred at room temperature for 1 hour. The reaction mixture was purified by HPLC (basic conditions) and lyophilized to obtain compound 107 (89.4 mg, chiral purity: 99.8% (HPLC method: CHIRALPAK AD-H, 4.6 * 250 mm 5 μm, 15% isopropanol/n-hexane, 0.8 mL/min, 30°C)). LC-MS: $[M+H]^+$ = 500.15.

$^1$H NMR (400 MHz, DMSO-$d_6$) δ 9.23 (d, *J* = 9.8 Hz, 1H), 7.95 (t, *J*= 7.8 Hz, 1H), 7.88 (d, *J= 5.4 Hz,* 1H), 7.82 (d, *J* = 4.8 Hz, 1H), 7.58 (d, *J* = 7.8 Hz, 1H), 7.51 (d, *J* = 7.8 Hz, 1H), 6.99 (d, *J* = 55.0 Hz, 1H), 5.40 (d, *J* = 9.4 Hz, 1H), 3.01 (d, *J* = 21.8 Hz, 6H), 1.79 - 1.63 (m, 2H), 1.55 (s, 4H), 1.34 - 1.25 (m, 1H), 1.12 - 1.04 (m, 1H), 0.89 (s, 3H).

Example 108. Synthesis of (*R*)-4-((2-(((6-(fluoromethyl)pyridin-2-yl)(1-methylcyclopentyl)methyl)amino)-3,4-dioxocyclobut-1-en-1-yl)amino)-3-hydroxy-*N,*N-dimethylpicolinamide (compound 108)

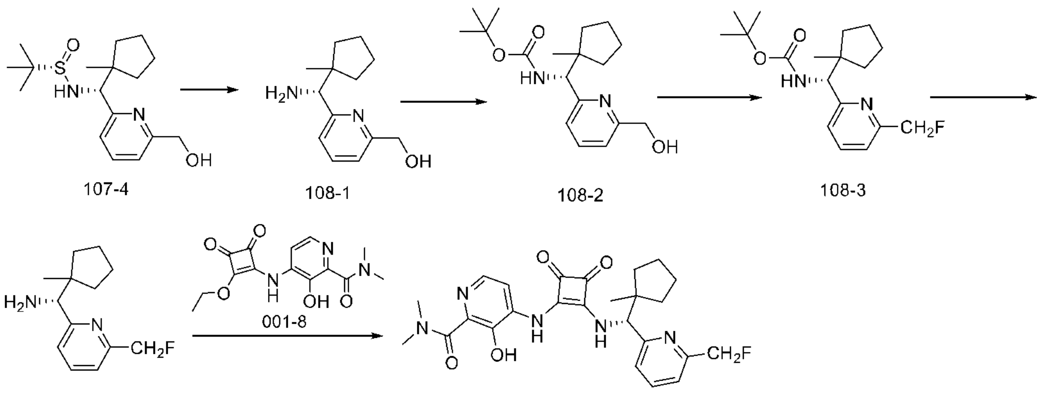
107-4 108-1 108-2 108-3
108-4 001-8 108

108.1 Synthesis of compound 108-1

At room temperature, 107-4 (490 mg, 1.51 mmol) was dissolved in a solution of hydrogen chloride in 1,4-dioxane (10 mL, 1 M). The reaction mixture was stirred at room temperature for 1 hour and then directly concentrated under reduced pressure to obtain compound 108-1 (329 mg, crude product). LC-MS: $[M+H]^+$ = 221.15.

108.2 Synthesis of compound 108-2

108-1 (329 mg, 1.49 mmol) was dissolved in dichloromethane (11 mL). Triethylamine (0.41 mL, 2.98 mmol) and di-*tert*-butyl dicarbonate (325 mg, 1.49 mmol) were added. The reaction mixture was stirred at 25°C for 1 hour. The reaction mixture was directly concentrated under reduced pressure. The residue was purified by silica gel column chromatography to obtain compound 108-2 (340 mg, yield: 71.05%). LC-MS: $[M+H]^+$ = 321.15.

108.3 Synthesis of compound 108-3

108-2 (290 mg, 0.90 mmol) was dissolved in anhydrous dichloromethane (5 mL). The system was purged with nitrogen three times. The reaction mixture was cooled to -78°C, and diethylaminosulfur trifluoride (289 mg, 1.80 mmol) was added dropwise slowly. The reaction was carried out at -78°C for 1 hour. The reaction mixture was poured into saturated aqueous sodium bicarbonate solution (10 mL) to quench the reaction, then extracted with ethyl acetate (20 mL × 3). The organic phases were combined, washed with saturated brine (30 mL), dried over anhydrous sodium sulfate, filtered, and the filtrate was concentrated under reduced pressure. The residue was purified by silica gel column chromatography to

obtain compound 108-3 (38 mg, yield: 13.02%). LC-MS: $[M+H]^+$ = 323.15.

108.4 Synthesis of compound 108-4

At room temperature, 108-3 (33 mg, 0.10 mmol) was dissolved in a solution of hydrogen chloride in 1,4-dioxane (2 mL, 0.5 M). The reaction mixture was stirred at room temperature for 1 hour, and then directly concentrated under reduced pressure to obtain compound 108-4 (35 mg, crude product). LC-MS: $[M+H]^+$ = 223.15.

108.5 Synthesis of compound 108

108-4 (35 mg, 0.15 mmol) was dissolved in ethanol (2 mL). 001-8 (48 mg, 0.15 mmol) and N,N-diisopropylethylamine (96 mg, 0.75 mmol) were added, and the reaction mixture was stirred at room temperature for 1 hour. The reaction mixture was purified by HPLC (basic conditions) and lyophilized to obtain compound 108 (17.3 mg, chiral purity: 100% (HPLC method: CHIRALPAK AD-H, 4.6 * 250 mm 5 μm, 10% ethanol/5% IPA/n-hexane, 0.8 mL/min, 30°C)). LC-MS: $[M+H]^+$ = 482.15.

$^1H$ NMR (400 MHz, DMSO-$d_6$) δ 9.26 (s, 1H), 7.90 (dd, *J* = 19.8, 11.8 Hz, 3H), 7.44 (d, *J*= 7.8 Hz, 1H), 7.37 (d, *J*= 7.8 Hz, 1H), 5.58 (s, 1H), 5.46 (s, 1H), 5.39 (d, *J* = 10.2 Hz, 1H), 3.10 (d, *J*= 44.0 Hz, 6H), 1.79 - 1.70 (m, 2H), 1.60 (s, 4H), 1.32 (d, *J*= 10.2 Hz, 1H), 1.15 - 1.09 (m, 1H), 0.95 (s, 3H).

Example 109. Synthesis of (R)-4-((2-(((6-(difluoromethyl)-3-fluoropyridin-2-yl)(1-methylcyclopentyl)methyl)amino)-3,4-dioxocyclobut-1-en-1-yl)amino)-3-hydroxy-N,N-dimethylpicolinamide (compound 109)

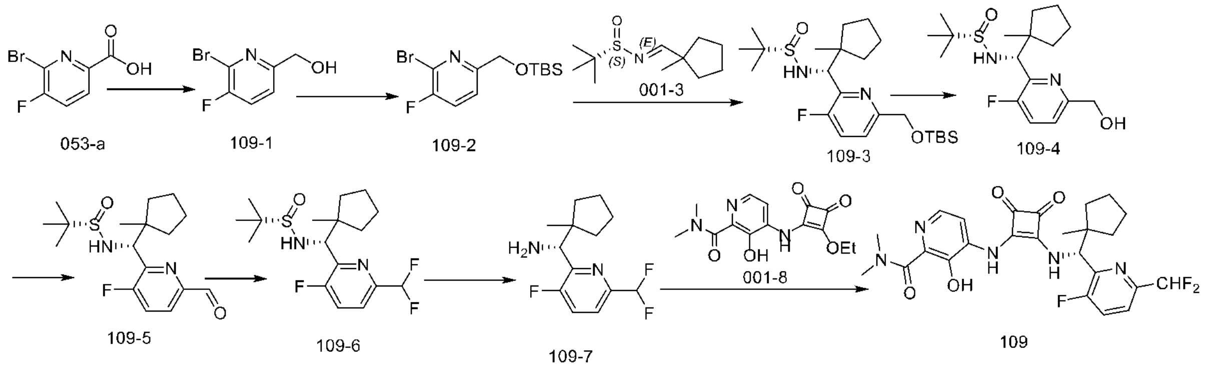


109.1 Synthesis of compound 109-1

053-a (2.0 g, 9.09 mmol) was dissolved in tetrahydrofuran (5 mL). At 0°C, a solution of borane in tetrahydrofuran (1 M, 27.2 mL, 27.2 mmol) was added. The reaction mixture was warmed to 85°C and reacted for 2 hours. The reaction mixture was quenched with saturated aqueous sodium bicarbonate solution. The aqueous phase was extracted with ethyl acetate (30 mL × 3). The organic phases were retained, dried, and concentrated under reduced pressure to obtain compound 109-1 (2.0 g, crude product). LC-MS: $[M+H]^+$= 207.1.

109.2 Synthesis of compound 109

The synthesis of compound 109 was carried out with reference to the synthesis method for compound 107 in Example 107, except that the starting material 107-1 was replaced with 109-1, to obtain compound 109 (16.2 mg, yield: 26.95%, chiral purity: 96.5% (HPLC method: CHIRALPAK AD-H, 4.6 * 250mm 5 μm, 15% ethanol/n-hexane, 0.8 mL/min, 30°C)). LC-MS: $[M+H]^+$= 518.1.

$^1H$ NMR (400 MHz, DMSO-$d_6$) δ 9.23 (s, 1H), 7.94-7.73 (m, 4H), 6.99 (t, *J*= 52.0 Hz, 1H), 5.84 (d, *J*= 8.0 Hz, 1H), 3.11 (s, 3H), 3.01 (s, 3H), 1.73-1.62 (m, 6H), 1.33-1.20 (m, 1H), 1.17-1.16 (m, 1H), 0.98 (s, 3H).

Example 110. Synthesis of 4-((2-(((S)-3-cyclobutyl-1-((S)-4,4-difluoro-2-methyltetrahydrofuran-2-yl)prop-2-yn-1-yl) amino)-3,4-dioxocyclobut-1-en-1-yl)amino)-3-hydroxy-N,N-dimethylpicolinamide (110A or 110B or 110C or 110D) and 4-((2-(((S)-3-cyclobutyl-1-((R)-4,4-difluoro-2-methyltetrahydrofuran-2-yl)prop-2-yn-1-yl)amino)-3,4-dioxocyclobut-1-en-1-yl)amino)-3-hydroxy-N,N-dimethylpicolinamide (110B or 110A or 110C or 110D) and 4-((2-(((R)-3-cyclobutyl-1-((S)-4,4-difluoro-2-methyltetrahydrofuran-2-yl)prop-2-yn-1-yl)amino)-3,4-dioxocyclobut-1-en-1-yl)amino)-3-hydroxy-N,N-dimethylpicolinamide (110C or 110A or 110B or 110D) and 4-((2-(((R)-3-cyclobutyl-1-((*R*)-4,4-difluoro-2-methyltetrahydrofuran-2-yl)prop-2-yn-1-yl)amino)-3,4-dioxocyclobut-1-en-1-yl)amino)-3-hydroxy-*N,N*-dimethylpicolinamide (110D or 110A or 110B or 110C)

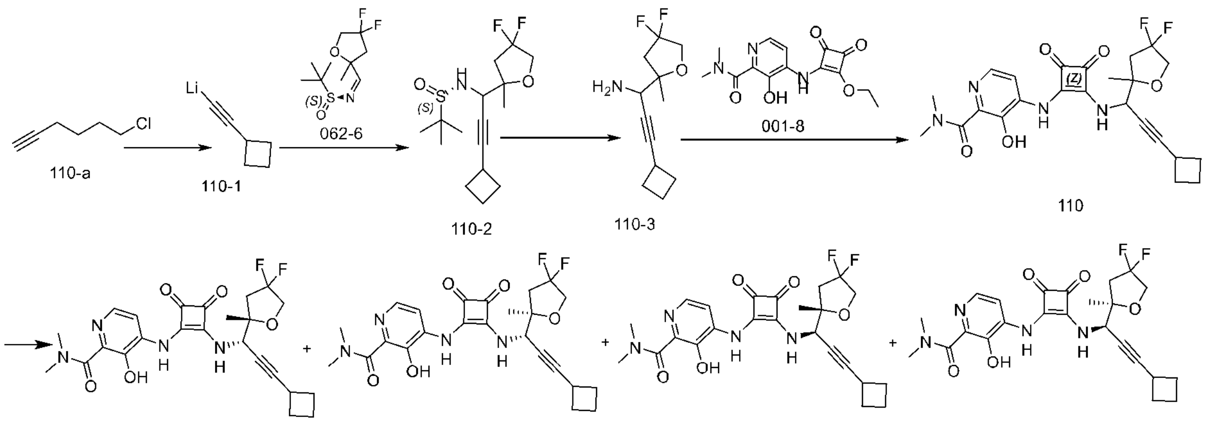


110A or 110B or 110C or 110D    110B or 110A or 110C or 110D    110C or 110A or 110B or 110D    110D or 110A or 110B or 110C

110.1 Synthesis of compound 110-1

110-a (0.2 g, 1.72 mmol) was weighed into a reaction flask. Tetrahydrofuran (10 mL) was added. Under a nitrogen atmosphere at -78°C, *n*-butyllithium (1.44 mL, 2.5 N, 3.6 mmol) was added dropwise. The temperature was raised to 35°C, and the mixture was stirred for 16 hours to obtain a solution of compound 110-1 (150 mg) in tetrahydrofuran.

110.2 Synthesis of compound 110-2

062-6 (0.2 g, 0.789 mmol) was weighed into a reaction flask, and tetrahydrofuran (5 mL) was added. Under a nitrogen atmosphere at -78°C, a solution of 110-1 in tetrahydrofuran (102 mg, 1.18 mmol) was added dropwise. The reaction was carried out at -78°C for 0.5 hours. Water (3 mL) was slowly added to quench the reaction, and the mixture was extracted with ethyl acetate (30 mL × 3). The organic phases were combined and dried over anhydrous sodium sulfate, and the filtrate was concentrated under reduced pressure to obtain a residue. The residue was purified by silica gel column chromatography (petroleum ether/ethyl acetate = 2/1 to 1/1) to obtain compound 110-2 (150 mg, yield: 57%).

110.3 Synthesis of compound 110

The synthesis of compound 110 was carried out with reference to the synthesis method for compound 001 in Example 1, except that the starting material 001-9 was replaced with 110-2, to obtain compound 110 (30.2 mg, yield: 18.7%). LC-MS: $[M+H]^+$ = 488.95.

$^1$H NMR (400 MHz, DMSO-$d_6$) δ 7.76 (s, 1H), 7.29 (d, *J* = 4.0 Hz, 1H), 5.13 (d, *J*= 12.0 Hz, 1H), 4.03 (s, 2H), 3.04 (s, 3H), 2.97 (s, 3H), 2.80-2.60 (m, 1H), 2.50 - 2.30 (m, 2H), 2.20 (s, 2H), 2.10 (s, 2H), 1.92 - 1.78 (m, 2H), 1.34 (s, 3H).

110.4 Synthesis of compounds 110A, 110B, 110C, and 110D

Compound 110 (320 mg, 0.66 mmol) was subjected to chiral resolution to obtain compound 110A (peak 1, 17.5 mg), compound 110B (peak 2, 78.2 mg), compound 110C (peak 3, 73.5 mg), and compound 110D (peak 4, 33.9 mg).

HPLC method: CHIRALPAK AD-H, 4.6 * 250 mm, 5 μm, 15% isopropanol/n-hexane, 0.8 mL/min, 30°C.

110A:

Chiral purity: 99.7%, $T_R$ = 8.710 min;

$^1$H NMR (400 MHz, DMSO-$d_6$) δ 9.11 (s, 1H), 7.99 (d, *J* = 5.2 Hz, 1H), 7.93 (s, 1H), 5.12 (d, *J*= 6.4 Hz, 1H), 4.13 - 4.03 (m, 2H), 3.11 *(d, J*= 14.4 Hz, 3H), 3.03 (s, 3H), 2.61 (d, *J* = 15.6 Hz, 1H), 2.49 - 2.39 (m, 2H), 2.24 (dd, *J*= 16.4, 8.5 Hz, 2H), 2.04 (dt, *J*= 20.0, 10.1 Hz, 2H), 1.87 (ddd, *J*= 19.2, 16.8, 10.3 Hz, 2H), 1.40 (s, 3H).

110B:

Chiral purity: 96.9%, $T_R$ = 9.354 min;
$^1$H NMR (400 MHz, DMSO-$d_6$) δ 9.14 (s, 1H), 7.98 (t, J= 14.4 Hz, 2H), 5.06 (d, *J*= 6.2 Hz, 1H), 4.10 (dt, J= 25.6, 9.4 Hz, 2H), 3.15 - 3.09 (m, 3H), 3.04 (s, 3H), 2.77 - 2.66 (m, 1H), 2.49 - 2.41 (m, 2H), 2.23 (dd, *J* = 16.0, 7.9 Hz, 2H), 2.08 - 1.97 (m, 2H), 1.95 - 1.80 (m, 2H), 1.34 (s, 3H).

110C:

Chiral purity: 90.6%, $T_R$ = 11.334 min;
$^1$H NMR (400 MHz, DMSO-$d_6$) δ 9.16 (s, 1H), 8.02 (d, *J*= 5.2 Hz, 1H), 7.96 (s, 1H), 5.07 (d, *J*= 8.4 Hz, 1H), 4.25 - 3.98 (m, 2H), 3.13 (d, *J*= 15.2 Hz, 3H), 3.04 (s, 3H), 2.73 (dt, *J*= 19.2, 15.1 Hz, 1H), 2.46 (d, *J = 4.8* Hz, 2H), 2.28 - 2.17 (m, 2H), 2.08 - 1.96 (m, 2H), 1.95 - 1.80 (m, 2H), 1.34 (s, 3H).

110D:

Chiral purity: 94.8%, $T_R$ = 15.859 min;
$^1$H NMR (400 MHz, DMSO-$d_6$) δ 9.11 (s, 1H), 8.00 (d, *J* = 5.2 Hz, 1H), 7.95 (s, 1H), 5.12 (d, *J* = 7.6 Hz, 1H), 4.14 - 4.03 (m, 2H), 3.13 (d, *J = 9.6* Hz, 3H), 3.04 (s, 3H), 2.61 (d, *J* = 15.6 Hz, 1H), 2.44 (dd, *J* = 14.4, 10.1 Hz, 2H), 2.28 - 2.20 (m, 2H), 2.03 (dd, *J* = 18.8, 8.9 Hz, 2H), 1.94 - 1.81 (m, 2H), 1.40 (s, 3H).

Example 111. Synthesis of 4-((2-((1-(3,3-difluoro-1-methylcyclopentyl)but-2-yn-1-yl)amino)-3,4-dioxocyclobut-1-en-1-yl)amino)-3-hydroxy-*N,N*- dimethylpicolinamide (compound 111)

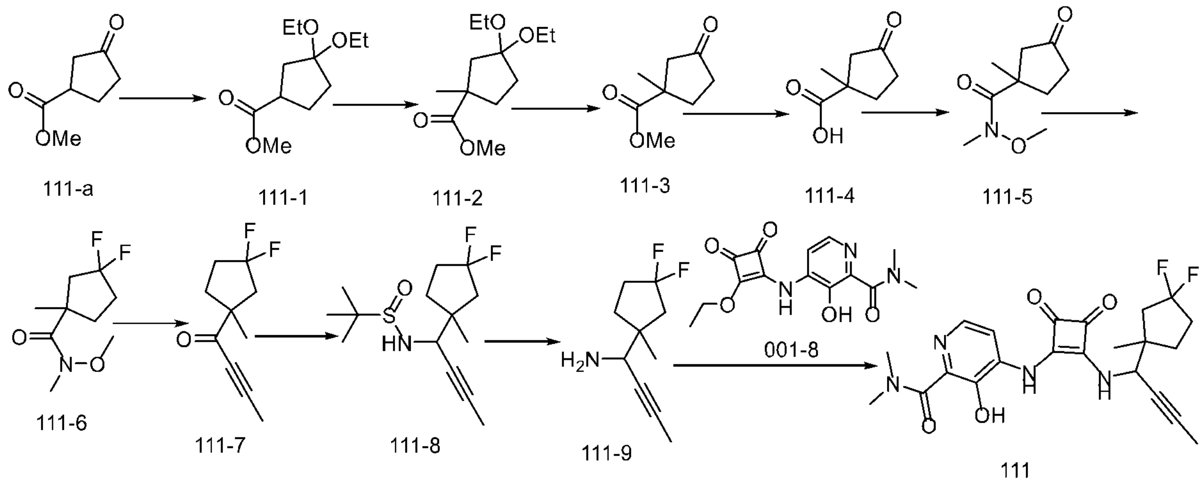


111-a 111-1 111-2 111-3 111-4 111-5

111-6 111-7 111-8 111-9 111

111.1 Synthesis of compound 111-1

111-a (8.0 g, 56.28 mmol) was dissolved in ethanol (50 mL). Triethyl orthoformate (20.85 g, 140.69 mmol) and *p*-toluenesulfonic acid (1.45 g, 8.44 mmol) were added, and the reaction was carried out at room temperature for 1 hour. A dilute aqueous sodium bicarbonate solution (100 mL) was added dropwise to quench the reaction. Ethyl acetate (80 mL) was added for extraction, and the organic phase was separated. The aqueous phase was extracted with ethyl acetate (100 mL × 3). The organic phases were combined, washed with saturated brine (150 mL × 3), dried over anhydrous sodium sulfate, filtered, and the filtrate was concentrated under reduced pressure. The residue was purified by silica gel column chromatography to obtain compound 111-1 (12.0 g, yield: 98%).

111.2 Synthesis of compound 111-2

Diisopropylamine (7.3 g, 72.13 mmol) was dissolved in super-dry tetrahydrofuran (50 mL). The system was purged with nitrogen three times. At -78°C, a solution of n-butyllithium in n-hexane (26.63 mL, 2.5 M) was added dropwise slowly. The reaction mixture was warmed to room temperature and reacted for 1 hour. Then, the temperature was lowered to -78°C, and a solution of 111-1 (12.0 g, 55.48 mmol) in tetrahydrofuran (10 mL) was slowly added to the reaction flask. The reaction was continued at this temperature for 1 hour. Subsequently, iodomethane (11.81 g, 83.23 mmol) was slowly added, and the reaction was carried out at room temperature for 3 hours. The reaction was quenched with saturated aqueous ammonium chloride solution (100 mL) and extracted with ethyl acetate (100 mL). The organic phase was separated, and the aqueous phase was further extracted with ethyl acetate (100 mL × 3). The organic phases were combined, washed with saturated brine (100 mL × 3), dried over anhydrous sodium sulfate, filtered, and the filtrate was concentrated under reduced pressure. The residue was purified by silica gel column chromatography to obtain compound 111-2 (6.0 g, yield: 47.0%).

111.3 Synthesis of compound 111-3

111-2 (5.0 g, 21.72 mmol) was dissolved in a mixed solvent of trifluoroacetic acid/dichloromethane (40 mL, 1/10 (v/v)). The reaction was carried out at room temperature for 1 hour. The reaction was quenched with water, and ethyl acetate (60 mL) was added for extraction. The organic phase was separated, and the aqueous phase was further extracted with ethyl acetate (60 mL × 3). The organic phases were combined, washed with saturated brine (100 mL × 3), dried over anhydrous sodium sulfate, filtered, and the filtrate was concentrated under reduced pressure to obtain compound 111-3 (3.4 g, crude product), which was directly used in the next step.

111.4 Synthesis of compound 111-4

111-3 (3.4 g, crude product) was dissolved in a mixed solvent of ethanol/water (30 mL, 3/1 (v/v)). Sodium hydroxide (3.48 g, 87.08 mmol) was added, and the reaction was carried out at room temperature for 1 hour. Water (30 mL) and ethyl acetate (50 mL) were added for extraction, and the organic phase was separated. The aqueous phase was extracted with ethyl acetate (80 mL × 2), and the organic phases were discarded. The aqueous phase was then adjusted to pH = 2-3 with hydrochloric acid solution (1 N), and extracted with ethyl acetate (50 mL × 3). The organic phases were combined, washed with saturated brine (50 mL × 3), dried over anhydrous sodium sulfate, filtered, and the filtrate was concentrated under reduced pressure. The residue was purified by silica gel column chromatography to obtain compound 111-4 (3.2 g, yield: 97%).

111.5 Synthesis of compound 111-5

111-4 (3.2 g, 22.51 mmol) was dissolved in dichloromethane (20 mL). The temperature was lowered to 0°C, and under a nitrogen atmosphere, oxalyl chloride (2.86 mL, 33.77 mmol) was added dropwise slowly. Then, one drop of *N,N*-dimethylformamide was added dropwise. After the addition was completed, the reaction mixture was warmed to room temperature and reacted for 1 hour. The solvent was removed by concentration under reduced pressure. The residue was redissolved in dichloromethane (20 mL), and the temperature was lowered to 0°C. Triethylamine (9.39 mL, 67.53 mmol) and dimethylhydroxylamine hydrochloride (3.29 g, 33.77 mmol) were slowly added. The reaction was carried out at room temperature for 3 hours. The solvent was removed by concentration under reduced pressure. The residue was purified by silica gel column chromatography to obtain compound 111-5 (1.75 g, yield: 42%).

111.6 Synthesis of compound 111-6

111-5 (1.75 g, 9.45 mmol) was dissolved in dichloromethane (15 mL). Diethylaminosulfur trifluoride (6.24 mL, 47.24 mmol) and trifluoroacetic acid (0.15 mL, 1.89 mmol) were added, and the reaction was carried out at room temperature overnight. The reaction was quenched with saturated aqueous sodium bicarbonate solution. Dichloromethane (15 mL) was added for extraction, and the organic phase was separated. The aqueous phase was further extracted with dichloromethane (15 mL × 3). The organic phases were combined, washed with saturated brine (25 mL × 3), dried over anhydrous sodium sulfate, filtered, and the filtrate was concentrated under reduced pressure. The residue was purified by silica gel column chromatography to obtain compound 111-6 (1.3 g, yield: 66%).

111.7 Synthesis of compound 111-7

111-6 (1.2 g, 5.79 mmol) was dissolved in super-dry tetrahydrofuran (10 mL). The system was purged with

nitrogen three times. At 0°C, 1-propynylmagnesium bromide (18 mL, 9.0 mmol) was slowly added. The reaction was carried out at room temperature for 4 hours. At 0°C, saturated aqueous ammonium chloride solution was added to quench the reaction. Ethyl acetate (15 mL) was added for extraction, and the organic phase was separated. The aqueous phase was further extracted with ethyl acetate (15 mL × 3). The organic phases were combined, washed with saturated brine (20 mL × 3), dried over anhydrous sodium sulfate, filtered, and the filtrate was concentrated under reduced pressure. The residue was purified by silica gel column chromatography to obtain compound 111-7 (0.65 g, yield: 60%).

111.8 Synthesis of compound 111-8

111-7 (100.0 mg, 0.54 mmol) was dissolved in tetrahydrofuran (10 mL). Tetraethyl titanate (245.01 mg, 1.07 mmol) and tert-butanesulfinamide (97.63 mg, 0.81 mmol) were added, and the reaction was carried out at 60°C for 4 hours. At -30°C, sodium borohydride (121.91 mg, 3.22 mmol) was slowly added, and the reaction was carried out at room temperature for 0.5 hours. At 0°C, methanol (5 mL) was added to quench the reaction. Ethyl acetate (10 mL) and water (15 mL) were added, and the mixture was filtered. The filtrate was extracted, and the aqueous phase was further extracted with ethyl acetate (15 mL × 3). The organic phases were combined, washed with saturated brine (15 mL × 3), dried over anhydrous sodium sulfate, filtered, and the filtrate was concentrated under reduced pressure to obtain 111-8 (65.0 mg, yield: 41.53%).

111.9 Synthesis of compound 111-9

111-8 (65.0 mg, crude product) was dissolved in a solution of hydrogen chloride in 1,4-dioxane (4 mL, 4.0 M). The reaction was carried out at room temperature for 0.5 hours. The reaction mixture was concentrated under reduced pressure to obtain compound 111-9 (45 mg, crude product). The crude product was directly used in the next step. LC-MS: $[M+H]^+$ = 188.10.

111.10 Synthesis of compound 111

111-9 (45.0 mg, crude product) was dissolved in ethanol (5 mL). *N,N*-Diisopropylethylamine (93.19 mg, 0.72 mmol) and 001-8 (58.70 mg, 0.19 mmol) were added, and the reaction was carried out at room temperature overnight. The reaction mixture was purified by preparative HPLC to obtain compound 111 (14.2 mg). LC-MS: $[M+H]^+$ = 447.10.

$^1$H NMR (400 MHz, DMSO-$d_6$) δ 7.88 (d, *J* = 8.0 Hz, 1H), 7.72 (s, 1H), 4.90 (d, *J* = *4.0* Hz, 1H), 2.99 (d, *J* = *12.0* Hz, 6H), 2.21 (dt, *J* = 28.0, 13.0 Hz, 3H), 2.04 - 1.90 (m, 1H), 1.84 (s, 3H), 1.56 (dd, *J* = 20.0, 7.7 Hz, 1H), 1.16 (d, *J* = 8.0 Hz, 3H), 1.13 - 0.81 (m, 1H).

Example 112. Synthesis of 4-((2-((1-(4,4-difluoro-2-methyltetrahydrofuran-2-yl)but-2-yn-1-yl)amino)-3,4-dioxocyclobut-1-en-1-yl)amino)-3-hydroxy-N,N-dimethylpicolinamide (compound 112)

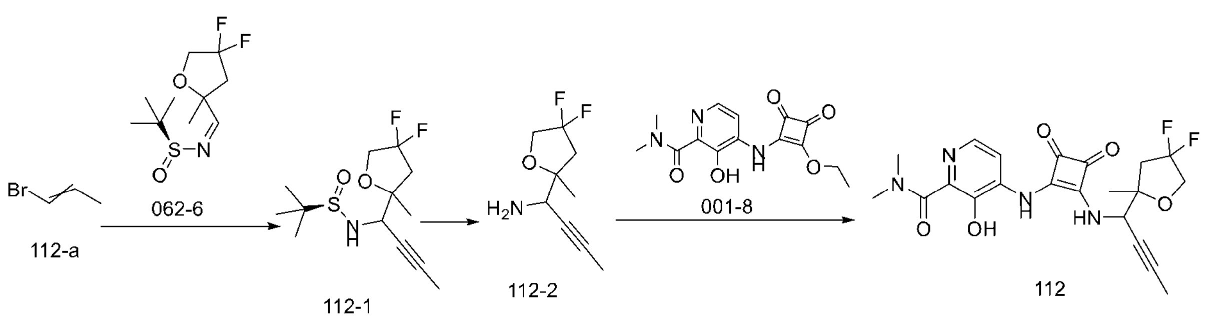


112-a 112-1 112-2 112

112.1 Synthesis of compound 112-1

112-a (120 mg, 0.99 mmol) was dissolved in tetrahydrofuran (2.5 mL). The system was purged with nitrogen three times. The reaction mixture was cooled to -78°C, and n-butyllithium (0.87 mL, 2.18 mmol) was added dropwise. The reaction was carried out at -78°C for 2 hours. 062-6 (200 mg, 0.79 mmol) was dissolved in tetrahydrofuran (0.3 mL), and the resulting solution was added dropwise to the reaction mixture. The reaction mixture was reacted at -78°C for 1 hour. The reaction mixture was poured into a saturated aqueous ammonium chloride solution (10 mL) to quench the reaction, and then extracted with ethyl acetate (10 mL × 3). The organic phases were combined, washed with saturated brine (20 mL),

dried over anhydrous sodium sulfate, filtered, and the filtrate was concentrated under reduced pressure. The residue was purified by silica gel column chromatography to obtain compound 112-1 (100 mg, yield: 34.36%). LC-MS: $[M+H]^+$ = 294.05.

112.2 Synthesis of compound 112-2

At room temperature, 112-1 (100 mg, 0.34 mmol) was dissolved in a solution of hydrogen chloride in 1,4-dioxane (4 mL, 0.5 M). The reaction mixture was stirred at room temperature for 0.2 hours, and then directly concentrated under reduced pressure to obtain compound 112-2 (100 mg, crude product). LC-MS: $[M+H]^+$ = 190.10.

112.3 Synthesis of compound 112

112-2 (58 mg, 0.30 mmol) was dissolved in ethanol (2 mL). Then, 001-8 (84 mg, 0.27 mmol) and *N,N-diisopropylethylamine* (194 mg, 1.5 mmol) were added, and the reaction mixture was stirred at room temperature for 6 hours. The reaction mixture was purified by HPLC (basic conditions) and lyophilized to obtain compound 112 (18.0 mg). LC-MS: $[M+H]^+$ = 449.05.

$^1H$ NMR (400 MHz, DMSO-$d_6$) δ 9.13 (s, 1H), 7.95 (t, *J* = 4.8 Hz, 1H), 7.86 (s, 1H), 5.05 (d, *J* = 22.4 Hz, 1H), 4.05 (dt, *J* = 20.0, 7.8 Hz, 2H), 3.04 (d, *J* = 28.6 Hz, 6H), 2.76 - 2.57 (m, 1H), 2.46 - 2.36 (m, 1H), 1.85 *(d, J*= 1.8 Hz, 3H), 1.35 *(d, J*= 18.4 Hz, 3H).

Example 113. Synthesis of (R)-3-hydroxy-N, N-dimethyl-4-((2-((1-(1-methylcyclopentyl)but-2-yn-1-yl)amino)-3,4-dioxocyclobut-1-en-1-yl)amino)picolinamide (compound 113)

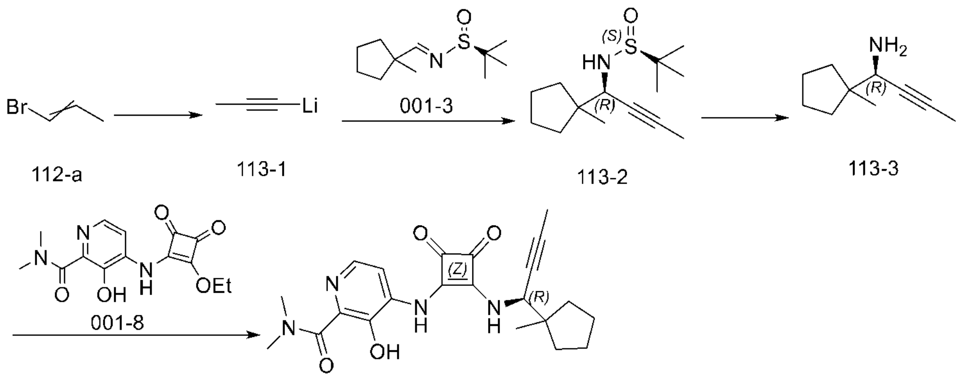


112-a 113-1 113-2 113-3

113

113.1 Synthesis of compound 113-1

112-a (72 μL, 0.84 mmol) was dissolved in tetrahydrofuran (2.8 mL). The system was purged with nitrogen three times. At -78°C, a solution of n-butyllithium in n-hexane (1.1 mL, 1.68 mmol, 1.6 M) was added. After stirring for 30 minutes, a solution of compound 113-1 in tetrahydrofuran (approximately 4 mL, 0.84 mmol) was obtained and directly used in the next step.

113.2 Synthesis of compound 113-2

001-3 (129 mg, 0.60 mmol) was dissolved in tetrahydrofuran (3 mL). The system was purged with nitrogen three times. At -78°C, a mixed solution of 113-1 in n-hexane and tetrahydrofuran (approximately 4 mL, 0.84 mmol) was added. After the addition was completed, the reaction was stirred at -78°C for 1 hour. At -78°C, the reaction mixture was quenched with saturated aqueous ammonium chloride solution (10 mL). After warming to room temperature, water (5 mL) was added for dilution, and the mixture was extracted with ethyl acetate (10 mL × 3). The organic phases were combined, washed with saturated aqueous sodium chloride solution (10 mL), dried over anhydrous sodium sulfate, filtered, and the filtrate was concentrated under reduced pressure. The residue was purified by silica gel column chromatography (petroleum ether/ethyl acetate = 3/2) to obtain compound 113-2 (92 mg, yield: 60%).

113.3 Synthesis of compound 113-3 hydrochloride

113-2 (80 mg, 0.31 mmol) was dissolved in 1,4-dioxane solution (2.3 mL). A solution of hydrogen chloride in 1,4-dioxane (0.75 mL, 4 M) was added, and the reaction mixture was stirred at room temperature for 20 minutes. The reaction mixture was directly concentrated under reduced pressure to obtain compound 113-3 hydrochloride (60 mg, crude product), which was used directly in the next step.

113.4 Synthesis of compound 113

113-3 hydrochloride (60 mg, crude product) was dissolved in ethanol (3 mL). *N,N*-Diisopropylethylamine (164 μL, 0.94 mmol) and 001-8 (96 mg, 0.31 mmol) were added, and the reaction mixture was stirred at room temperature overnight. The reaction mixture was concentrated under reduced pressure. The residue was purified by preparative HPLC to obtain compound 113 (73.2 mg, overall yield over two steps: 57%, chiral purity: 96.7% (HPLC method: CHIRALPAK AD-H, 4.6 * 250 mm 5 μm, 15% isopropanol/0.1% diethylamine/n-hexane, 0.8 mL/min, 30°C)). LCMS: $[M+H]^+$ = 411.00.

$^1H$ NMR (400 MHz, DMSO-$d_6$) δ 11.63 (bs, 1H), 9.70 (bs, 1H), 8.92 (s, 1H), 8.02 (d, *J* = 4.8 Hz, 1H), 7.97 (bs, 1H), 4.85-4.82 (m, 1H), 3.16 (s, 3H), 3.05 (s, 3H), 1.86 (d, *J*= 2.4 Hz, 3H), 1.64-1.58 (m, 6H), 1.40-1.29 (m, 2H), 1.07 (s, 3H).

Example 114. Synthesis of (R)-6-chloro-2-hydroxy-N, N-dimethyl-3-((2-((1-(1-methylcyclopentyl)but-2-yn-1-yl)amino)-3,4-dioxocyclobut-1-en-1-yl)amino)benzamide (compound 114)

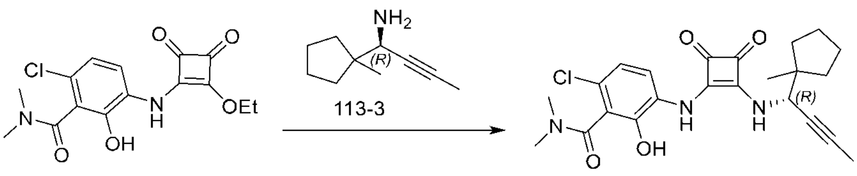


063-8

114

114.1 Synthesis of compound 114

063-8 (100 mg, 0.29 mmol) was dissolved in ethanol (2 mL). Triethylamine (60 mg, 0.59 mmol) and 113-3 hydrochloride (45 mg, 0.29 mmol) were added, and the reaction was carried out at room temperature for 1 hour. The reaction mixture was concentrated under reduced pressure. The residue was separated by preparative HPLC to obtain compound 114 (13.0 mg, yield: 9.9%, chiral purity: 91.5% (HPLC method: CHIRALPAK AD-H, 4.6 * 250 mm 5 μm, 10% isopropanol/10% ethanol/n-hexane, 0.8 mL/min, 30°C)). LCMS: $[M+H]^+$ = 444.1.

$^1H$ NMR (400 MHz, DMSO-$d_6$) δ 8.7 (s, 1H), 7.59 (s, 2H), 6.30 (s, 2H), 4.83 (s, 1H), 2.90 (d, *J*= *12.0* Hz, 3H), 2.80 (d, *J*= 8.0 Hz, 3H), 1.79 (s, 3H), 1.57 (s, 6H), 1.25-1.21 (m, 2H), 1.00 (d, *J*= 8.0 Hz, 1H).

Example 115. Synthesis of (R)-4-((2-((3-cyclopropyl-1-(1-methylcyclopentyl)prop-2-yn-1-yl)amino)-3,4-dioxocyclobut-1-en-1-yl)amino)-3-hydroxy-N,N-dimethylpicolinamide (compound 115)

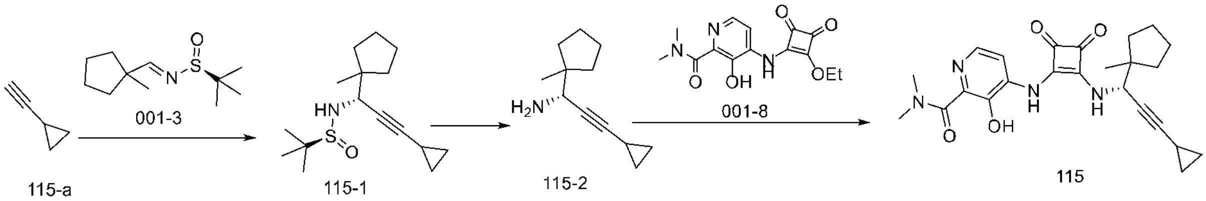


115-a 115-1 115-2 115

The synthesis of compound 115 was carried out with reference to the synthesis method for compound 113 in Example 113, except that the starting material 113-1 was replaced with 115-a, to obtain compound 115 (44.3 mg, chiral purity: 94.2% (HPLC method: CHIRALPAK AD-H, 4.6 * 250 mm 5 μm, 20% isopropanol/n-hexane, 0.8 mL/min, 30°C)). LC-MS: $[M+H]^+$ = 437.05.

$^1H$ NMR (400 MHz, DMSO-$d_6$) δ 7.60 (d, *J*= 4.4 Hz, 1H), 7.15 (d, *J*= 4.8 Hz, 1H), 4.82 (s, 1H), 2.90 (s, 3H), 2.81 (s, 3H), 1.74 (s, 1H), 1.58 (s, 6H), 1.35 - 1.27 (m, 2H), 1.02 (s, 3H), 0.80 - 0.72 (m, 2H), 0.61 - 0.53 (m, 2H).

Example 116. Synthesis of (*R*)-4-((2-((3-cyclobutyl-1-(1-methylcyclopentyl)prop-2-yn-1-yl)amino)-3,4-dioxocyclobut-1-en-1-yl)amino)-3-hydroxy-*N,N*-dimethylpicolinamide (compound 116)

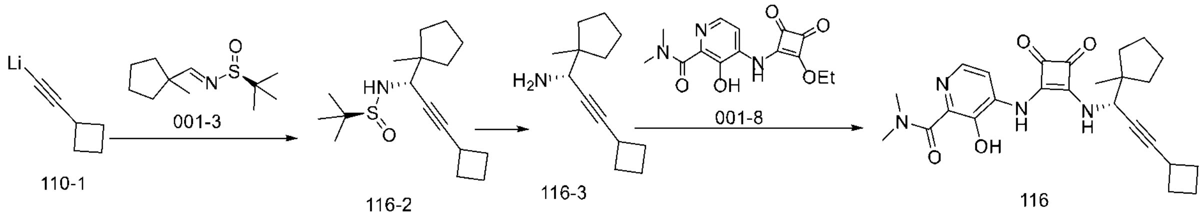


110-1 116-2 116-3 116

116.1 Synthesis of compound 116-2

At room temperature, 110-a (100 mg, 0.86 mmol) was added to tetrahydrofuran (2 mL). The system was purged with nitrogen three times. After the reaction mixture was cooled to -78°C, *n*-butyllithium (0.86 mL, 2.2 mmol, 2.5 M solution in tetrahydrofuran) was added dropwise slowly. The reaction mixture was stirred at 30°C for 16 hours. The reaction mixture was cooled again to -78°C, and a solution of 001-3 (100 mg, 0.47 mmol) in tetrahydrofuran (0.5 mL) was added. The reaction mixture was stirred at 20°C for another 1 hour. The reaction mixture was quenched with saturated aqueous ammonium chloride solution (10 mL), and then extracted with ethyl acetate (10 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 116-2 (65 mg, yield: 25.6%). LC-MS: $[M-104]^+$= 192.15.

116.1 Synthesis of compound 116

The synthesis of compound 116 was carried out with reference to the synthesis method for compound 001 in Example 1, except that the starting material 001-9 was replaced with 116-2, to obtain compound 116 (24.3 mg, yield: 24.5%, chiral purity: 93.8% (HPLC method: CHIRALPAK AD-H, 4.6 * 250 mm 5 μm, 20% isopropanol/n-hexane, 0.8 mL/min, 30°C)). LC-MS: $[M+H]^+$ = 451.05.

$^1H$ NMR (400 MHz, DMSO-$d_6$) δ 11.62 (s, 1H), 9.66 (s, 1H), 8.90 (s, 1H), 8.00 (s, 2H), 4.90 - 4.76 (m, 1H), 3.18 - 3.00 (m, 7H), 2.27 - 2.16 (m, 2H), 2.00 (dt, *J* = 8.8, 5.6 Hz, 2H), 1.92 - 1.75 (m, 2H), 1.60 (t, *J = 12.8* Hz, 6H), 1.39 - 1.28 (m, 2H), 1.06 (s, 3H).

Example 117. Synthesis of (*R*)-4-((2-((3-(azetidin-3-yl)-1-(1-methylcyclopentyl)prop-2-yn-1-yl)amino)-3,4-dioxocyclobut-1-en-1-yl)amino)-3-hydroxy-*N,N*-dimethylpicolinamide (compound 117)

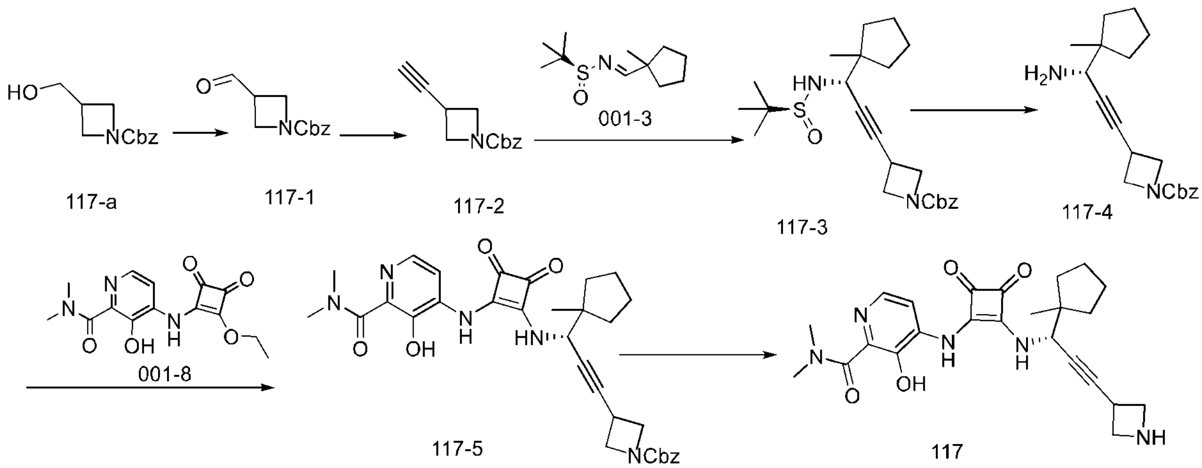


117-a 117-1 117-2 117-3 117-4

117-5 117

117.1 Synthesis of compound 117-1

117-a (2.0 g, 9.05 mmol) was dissolved in dichloromethane (30 mL). Dess-Martin periodinane (5.8 g, 13.57 mmol) was slowly added, and the reaction mixture was stirred at room temperature for 5 hours. The reaction mixture was poured into an aqueous sodium thiosulfate solution (30 mL) to quench the reaction, filtered, and the filtrate was collected. Saturated aqueous sodium bicarbonate solution was added to the filtrate to adjust the pH to neutral. The mixture was extracted with dichloromethane (30 mL × 3). The organic phases were combined, washed with saturated brine (50 mL), dried over anhydrous sodium sulfate, filtered, and the filtrate was concentrated under reduced pressure. The residue was purified by silica gel column chromatography to obtain compound 117-1 (950 mg, yield: 28.76%).

117.2 Synthesis of compound 117-2

117-1 (950 mg, 4.30 mmol) was dissolved in methanol (8 mL). Anhydrous potassium carbonate (1.2 g, 8.60 mmol) was added, and the system was purged with nitrogen three times. Dimethyl (1-diazo-2-oxopropyl)phosphonate (999 mg, 5.20 mmol) was added dropwise to the reaction mixture, and the reaction was carried out at 25°C for 16 hours. The reaction mixture was directly concentrated under reduced pressure, and 5% aqueous sodium bicarbonate solution (10 mL) was added. The mixture was extracted with ethyl acetate (20 mL × 3). The organic phases were combined, washed with saturated brine (30 mL), dried over anhydrous sodium sulfate, filtered, and the filtrate was concentrated under reduced pressure. The residue was purified by silica gel column chromatography to obtain compound 117-2 (380 mg, yield: 40.74%). LC-MS: $[M+H]^+$ = 216.05.

117.3 Synthesis of compound 117-5

The synthesis of compound 117-5 was carried out with reference to the synthesis method for compound 113 in Example 113, except that the starting material 113-1 was replaced with 117-2, to obtain compound 117-5 (377 mg, yield: 84.05%). LC-MS: $[M+H]^+$= 586.05.

117.4 Synthesis of compound 117

At room temperature, 117-5 (200 mg, 0.34 mmol) was dissolved in dichloromethane (3 mL) and trifluoroacetic acid (6 mL), and the reaction mixture was stirred at 25°C for 16 hours. The reaction mixture was purified by preparative HPLC (basic conditions) and lyophilized to obtain compound 117 (30.4 mg, chiral purity: 100% (HPLC method: CHIRALPAK IC, 4.6 * 250 mm 5 μm, 15% isopropanol/45% ethanol/0.1% diethylamine/n-hexane, 0.8 mL/min, 30°C)). LC-MS: $[M+H]^+$ = 452.05.
$^1$H NMR (400 MHz, DMSO-$d_6$) δ 8.99 (s, 2H), 7.90 (d, *J* = 5.0 Hz, 1H), 7.78 (s, 1H), 4.89 (s, 1H), 3.66 (s, 2H), 3.52 (d, *J* = 6.8 Hz, 3H), 3.00 (d, *J* = 13.2 Hz, 6H), 1.61 (s, 6H), 1.31 (dd, *J* = 14.2, 8.8 Hz, 2H), 1.05 (s, 3H).

Example 118. Synthesis of (*R*)-3-hydroxy-*N,N*-dimethyl-4-((2-((1-(1-methylcyclopentyl)-3-(pyridin-2-yl)prop-2-yn-1-yl) amino)-3,4-dioxocyclobut-1-en-1-yl)amino)picolinamide (compound 118)

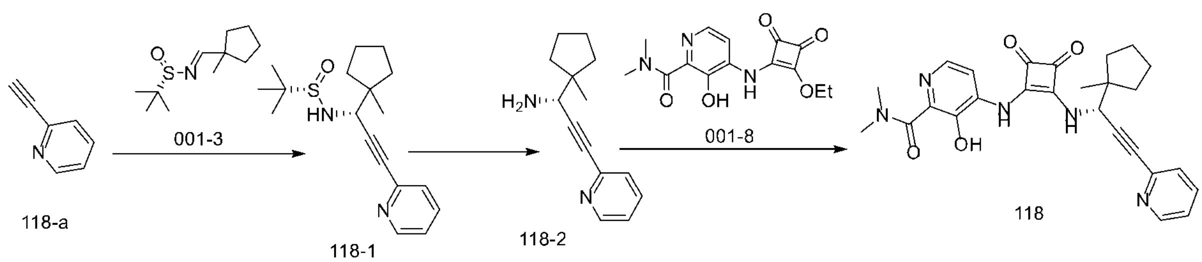


The synthesis of compound 118 was carried out with reference to the synthesis method for compound 113 in Example 113, except that the starting material 113-1 was replaced with 118-a, to obtain compound 118 (73.1 mg, chiral purity: 100% (HPLC method: CHIRALPAK IC, 4.6 * 250 mm 5 μm, 15% isopropanol/45% ethanol/0.1% diethylamine/n-hexane, 0.8 mL/min, 30°C)). LC-MS: $[M+H]^+$ = 474.00.
'H NMR (400 MHz, DMSO-$d_6$) δ 8.55 - 8.51 (m, 1H), 7.91 (d, *J* = 5.2 Hz, 1H), 7.79 (td, *J* = 7.7, 1.8 Hz, 2H), 7.50 (d, *J* = 7.8 Hz, 1H), 7.40 - 7.34 (m, 1H), 5.12 (s, 1H), 2.99 (d, *J* = 20.6 Hz, 6H), 1.66 (d, *J* = 13.4 Hz, 6H), 1.45 - 1.33 (m, 2H), 1.12 (s, 3H).

Example 119. Synthesis of (*R*)-3-hydroxy-*N,N*-dimethyl-4-((2-((3-(1-methyl-1*H*-pyrazol-3-yl)-1-(1-methylcyclopentyl)prop-2-yn-1-yl)amino)-3,4-dioxocyclobut-1-en-1-yl)amino)picolinamide (compound 119)

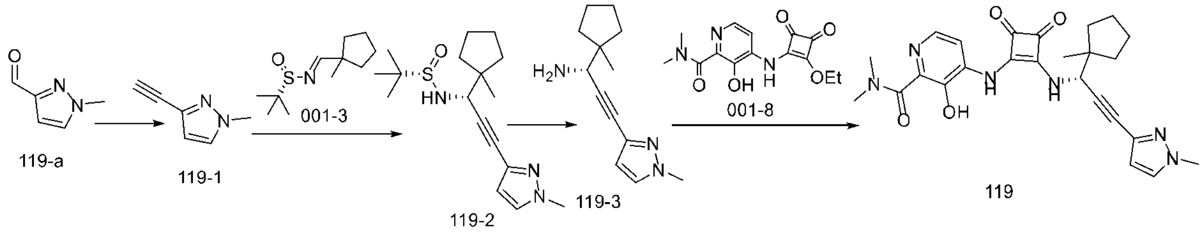


119.1 Synthesis of compound 119-1

119-a (200 mg, 1.82 mmol) was dissolved in methanol (5 mL). Dimethyl (1-diazo-2-oxopropyl)phosphonate (419 mg, 2.18 mmol) and cesium carbonate (710 mg, 2.18 mmol) were added, and the reaction was carried out at room temperature for 3 hours. The reaction mixture was filtered, and the filtrate was concentrated under reduced pressure. The residue was purified by silica gel column chromatography (petroleum ether/ethyl acetate = 3/1) to obtain compound 119-1 (100.0 mg). LC-MS: $[M+H]^+$ = 107.15.

119.2 Synthesis of compound 119

The synthesis of compound 119 was carried out with reference to the synthesis method for compound 113 in Example 113, except that the starting material 113-1 was replaced with 119-1, to obtain compound 119 (45.2 mg, chiral purity: 81.0% (HPLC method: CHIRALPAK AD-H, 4.6 * 250 mm, 5 μm, 15% ethanol/n-hexane, 0.8 mL/min, 30°C)). LC-MS: $[M+H]^+$ = 477.15.

$^1$H NMR (400 MHz, DMSO-$d_6$) δ 9.08 (d, *J*= 8.6 Hz, 1H), 7.99 (d, *J*= 5.4 Hz, 1H), 7.91 (s, 1H), 7.71 (d, *J= 2.2* Hz, 1H), 6.41 (d, *J*= 2.2 Hz, 1H), 5.07 (d, *J*= 9.2 Hz, 1H), 3.80 (d, *J*= 8.2 Hz, 3H), 3.06 (d, *J= 39.0* Hz, 6H), 1.74 - 1.55 (m, 6H), 1.43 - 1.29 (m, 2H), 1.10 (d, J= 7.2 Hz, 3H).

Example 120. Synthesis of (*S*)-3-hydroxy-*N,N*-dimethyl-4-((2-((1-(1-methylcyclopentyl)but-3-yn-1-yl)amino)-3,4-dioxocyclobut-1-en-1-yl)amino)picolinamide (compound 120)

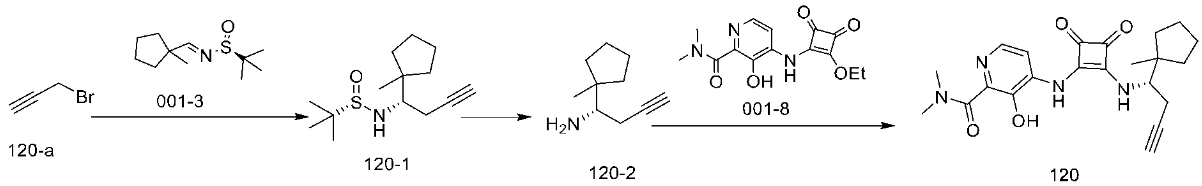


120.1 Synthesis of compound 120-1

120-a (328 mg, 2.76 mmol) and Zn (185 mg, 2.76 mmol) were weighed into a reaction flask. The system was purged with nitrogen three times, and tetrahydrofuran (3 mL) was added. At 25°C, a solution of 001-3 (100 mg, 0.46 mmol) in tetrahydrofuran (0.3 mL) was added dropwise to the reaction mixture. The temperature was heated to 66°C, and the reaction was carried out for 16 hours. The reaction mixture was poured into water (10 mL) to quench the reaction, then extracted with ethyl acetate (15 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 120-1 (66 mg, yield: 43.53%). LC-MS: $[M+H]^+$ = 256.10.

120.2 Synthesis of compound 120

The synthesis of compound 120 was carried out with reference to the synthesis method for compound 113 in Example 113, except that the starting material 113-2 was replaced with 120-1, to obtain compound 120 (28.7 mg, chiral

purity: 96.0% (HPLC method: CHIRALPAK AD-H, 4.6 * 250 mm 5 μm, 15% ethanol/n-hexane, 0.8 mL/min, 30°C)). LC-MS: $[M+H]^+$ = 411.00.

$^1H$ NMR (400 MHz, DMSO-$d_6$) δ 11.72 (s, 1H), 9.65 (s, 1H), 8.57 (d, *J*= 9.8 Hz, 1H), 8.02 (s, 2H), 4.20 (t, *J*= *9.0* Hz, 1H), 3.19 (s, 3H), 3.05 (s, 3H), 2.87 (s, 1H), 2.59 (d, *J*= 16.8 Hz, 1H), 2.44 - 2.36 (m, 1H), 1.61 (s, 4H), 1.51 - 1.44 (m, 2H), 1.32 (dd, *J*= 18.4, 6.6 Hz, 2H), 0.96 (s, 3H).

Example 121. Synthesis of 4-((2-(((6-fluoro-3-methylpyridin-2-yl)(3-methylbicyclo[3.1.0]hexan-3-yl)methyl)amino)-3,4-dioxocyclobut-1-en-1-yl)amino)-3-hydroxy-*N,N*-dimethylpicolinamide (compound 121)

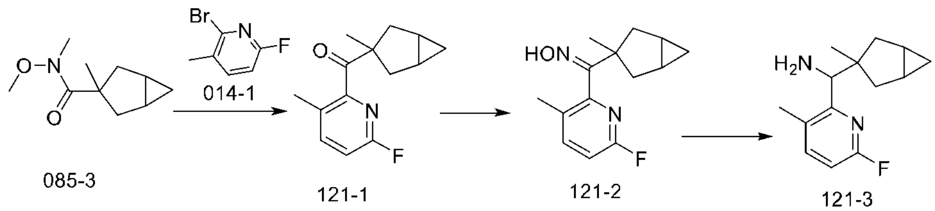


085-3 121-1 121-2 121-3

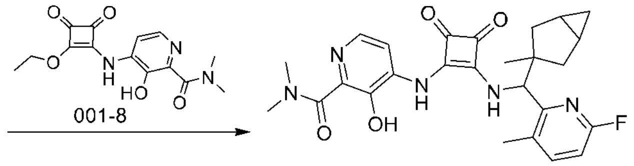


121

The synthesis of compound 121 was carried out with reference to the synthesis method for compound 085 in Example 85, except that the starting material 053-3 was replaced with 014-1, to obtain compound 121 (13.0 mg). LC-MS: $[M+H]^+$ = 494.05.

$^1H$ NMR (400 MHz, DMSO-*$d_6$*) δ 9.14 (d, *J* = 8.8 Hz, 2H), 7.95 (d, *J* = 4.8 Hz, 1H), 7.87 (s, 1H), 7.82 (t, *J* = 8.0 Hz, 1H), 7.03 (dd, *J* = 8.4, 3.2 Hz, 1H), 5.64 (d, *J* = 10.0 Hz, 1H), 3.14 (s, 3H), 3.03 (s, 3H), 2.45 - 2.29 (m, 3H), 2.02 - 1.92 (m, 1H), 1.62 (dd, *J* = 13.2, 6.0 Hz, 1H), 1.55 - 1.43 (m, 2H), 1.37 - 1.27 (m, 2H), 1.15 - 0.94 (m, 3H), 0.86 - 0.78 (m, 1H), -0.05 (d, *J* = 4.0 Hz, 1H).

Example 122. Synthesis of 3-hydroxy-*N,N*-dimethyl-4-((2-(((3-methylbicyclo[3.1.0]hexan-3-yl)(3-methylpyridin-2-yl)methyl)amino)-3,4-dioxocyclobut-1-en-1-yl)amino)picolinamide (compound 122)

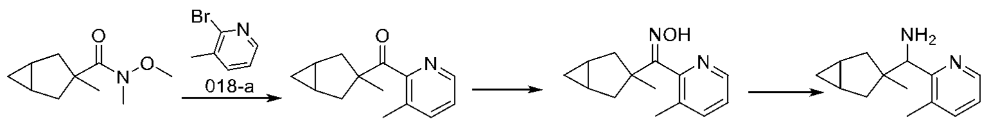


085-3 122-1 122-2 122-3

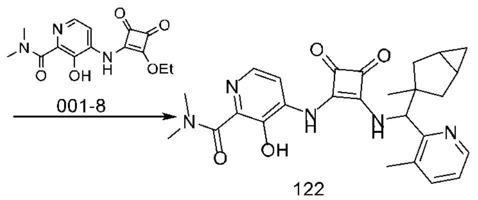


122

The synthesis of compound 122 was carried out with reference to the synthesis method for compound 085 in Example 85, except that the starting material 053-3 was replaced with 018-a, to obtain compound 122 (38.1 mg). LC-MS: $[M+H]^+$ = 476.00.

$^1H$ NMR (400 MHz, DMSO-*$d_6$*) δ 9.16 (d, *J* = 9.4 Hz, 1H), 8.37 (d, *J* = 4.2 Hz, 1H), 7.81 (d, *J* = 5.0 Hz, 1H), 7.57 (d, *J* = 7.2 Hz, 2H), 7.17 (dd, *J* = 7.4, 4.8 Hz, 1H), 5.62 (d, *J* = 9.4 Hz, 1H), 2.91 (d, *J* = 8.9 Hz, 6H), 2.35 (s, 3H), 1.57 (t, *J* = 9.8 Hz, 2H), 1.51 - 1.42 (m, 2H), 1.23 (s, 2H), 1.07 (s, 3H), 0.79 - 0.69 (m, 1H), -0.12 (d, *J* = 3.6 Hz, 1H).

Example 123. Synthesis of 3-hydroxy-*N*,*N*-dimethyl-4-((2-(((2-methylbicyclo[3.1.0]hexan-2-yl)(3-methylpyridin-2-yl)methyl)amino)-3,4-dioxocyclobut-1-en-1-yl)amino)picolinamide (compound 123)

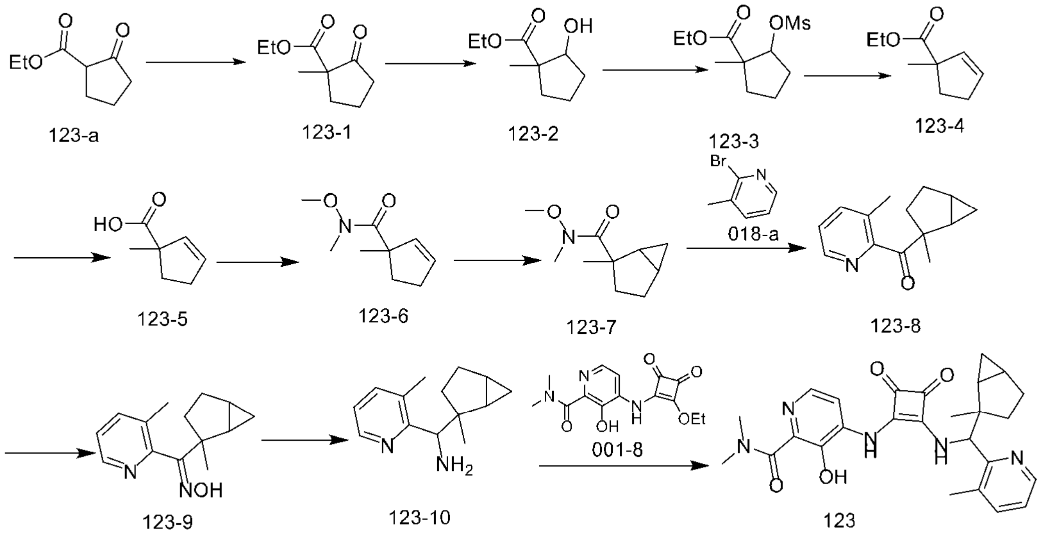

123-a 123-1 123-2 123-3 123-4
123-5 123-6 123-7 123-8
123-9 123-10 123

123.1 Synthesis of compound 123-1

123-a (8.0 g, 51.24 mmol) was weighed into a reaction flask. Acetone (100 mL) and potassium carbonate (21.2 g, 153.7 mmol) were added, followed by the slow addition of iodomethane (14.5 g, 102.5 mmol). The reaction mixture was stirred at 70°C for 3 hours. The reaction mixture was diluted with water (100 mL) and extracted with ethyl acetate (100 mL × 3). The organic phases were combined, washed with saturated brine (50 mL), dried over anhydrous sodium sulfate, filtered, and the filtrate was concentrated under reduced pressure. The residue was purified by silica gel column chromatography (petroleum ether/ethyl acetate = 8/1 to 1/1) to obtain compound 123-1 (7.5 g, yield: 86%).

123.2 Synthesis of compound 123-2

123-1 (6.0 g, 35.3 mmol) was weighed into a reaction flask, and methanol (40 mL) was added. At 0°C, sodium borohydride (0.8 g, 56.42 mmol) was slowly added. The reaction mixture was stirred at 0°C for 2 hours. Saturated aqueous ammonium chloride solution (30 mL) was slowly added. The mixture was extracted with ethyl acetate (60 mL × 3). The organic phases were combined, washed with saturated brine (100 mL), dried over anhydrous sodium sulfate, filtered, and the filtrate was concentrated under reduced pressure to obtain compound 123-2 (5.5 g).

123.3 Synthesis of compound 123-3

123-2 (4.0 g, 23.24 mmol) was weighed and dissolved in dichloromethane (30 mL). Under a nitrogen atmosphere at 0°C, triethylamine (3.5 g, 69.6 mmol) and methanesulfonyl chloride (2.0 g, 34.8 mmol) were added dropwise sequentially. The reaction was carried out at 0°C for 1 hour. The reaction mixture was diluted with water (50 mL) and extracted with dichloromethane (100 mL × 3). The organic phases were combined, washed with saturated brine (50 mL), dried over anhydrous sodium sulfate, filtered, and the filtrate was concentrated under reduced pressure to obtain compound 123-3 (5.2 g, crude product), which was directly used in the next step.

123.4 Synthesis of compound 123-4

123-3 (4.0 g, 16.0 mmol) was weighed and dissolved in N,N-dimethylformamide (30 mL). 1,8-Diazabicyclo[5.4.0]undec-7-ene (7.3 g, 48.0 mmol) was added dropwise, and the reaction was carried out at 150°C for 4 hours. The reaction mixture was cooled to room temperature, diluted with water (100 mL), and extracted with ethyl acetate (100 mL × 3). The organic phases were combined, washed with saturated brine (50 mL), dried over anhydrous sodium sulfate, filtered, and the filtrate was concentrated under reduced pressure. The residue was purified by silica gel column chromatography (petroleum ether/ethyl acetate = 8/1 to 1/1) to obtain compound 123-4 (2.4 g, yield: 100%).

123.5 Synthesis of compound 123-5

123-4 (2.0 g, 13.0 mmol) was weighed into a reaction flask. Ethanol (10 mL), water (5 mL), and sodium hydroxide (780 mg, 19.5 mmol) were added, and the reaction was carried out at 80°C for 2 hours. 1 N hydrochloric acid solution was added to adjust the pH to 4-5. The mixture was extracted with ethyl acetate (20 mL × 3). The combined organic phases were washed with saturated brine (50 mL), dried over anhydrous sodium sulfate, filtered, and the filtrate was concentrated under reduced pressure to obtain compound 123-5 (2.0 g, crude product), which was directly used in the next step.

123.6 Synthesis of compound 123-6

123-5 (2.0 g, 15.8 mmol) was weighed and dissolved in dichloromethane (20 mL). At 0°C, oxalyl chloride (3.0 g, 24.0 mmol) and 5 drops of N,N-dimethylformamide were added dropwise slowly. After the addition was completed, the reaction mixture was warmed to room temperature and reacted for 1 hour. The reaction mixture was concentrated under reduced pressure. The residue was dissolved in dichloromethane (20 mL). At 0°C, triethylamine (4.6 g, 45.6 mmol) and dimethylhydroxylamine hydrochloride (2.2 g, 22.8 mmol) were slowly added. The reaction was carried out at room temperature for 1 hour. The reaction mixture was concentrated under reduced pressure. The residue was purified by silica gel column chromatography to obtain compound 123-6 (2.1 g, yield: 78%).

123.7 Synthesis of compound 123-7

123-6 (2.0 g, 11.83 mmol) was weighed and dissolved in dichloromethane (20 mL). The system was purged with nitrogen three times. At 0°C, diethylzinc (59.2 mL, 118.3 mmol) was added, and the mixture was stirred for 30 minutes. Diiodomethane (31.7 g, 59.2 mmol) was added and stirred at 0°C for another 30 minutes. The reaction mixture was then warmed to room temperature and reacted for 2 hours. The reaction mixture was quenched with saturated aqueous ammonium chloride solution (30 mL), then extracted with ethyl acetate (40 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 123-7 (1.2 g, yield: 55.3%). LC-MS: $[M+H]^+$ = 184.10.

123.8 Synthesis of compound 123-8

018-a (500 mg, 2.91 mmol) was dissolved in diethyl ether (10 mL). The system was purged with nitrogen three times. At -78°C, a solution of n-butyllithium in n-hexane (1.8 mL, 2.13 mmol, 1.6 M) was added dropwise slowly. The reaction was maintained at -78°C for 15 minutes. Then, a solution of 123-7 (480 mg, 2.62 mmol) in diethyl ether (5 mL) was added dropwise to the reaction system. After the addition was completed, the reaction was maintained at -78°C and reacted for another 15 minutes. A saturated aqueous ammonium chloride solution (10 mL) was slowly added to quench the reaction. The mixture was extracted with ethyl acetate (30 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 123-8 (300 mg, yield: 51.1%). LC-MS: $[M+H]^+$ = 216.10.

123.9 Synthesis of compound 123-9

At room temperature, 123-8 (290 mg, 1.35 mmol) was dissolved in ethanol (10 mL). Then, hydroxylamine hydrochloride (140 mg, 2.02 mmol) and sodium acetate (221 mg, 2.7 mmol) were added. The reaction mixture was heated to 90°C and reacted for 2 hours. Water (20 mL) was added for dilution, and the mixture was extracted with dichloromethane (30 mL × 3). The organic phases were combined, washed with saturated brine (10 mL), dried over anhydrous sodium sulfate, filtered, and the filtrate was concentrated under reduced pressure to obtain compound 123-9 (250 mg, crude product). LC-MS: $[M+H]^+$ = 231.10.

123.10 Synthesis of compound 123-10

At room temperature, 123-9 (200 mg, 0.868 mmol) and zinc powder (341 mg, 5.21 mmol) were dissolved in hydrochloric acid (5 mL, 2 N), and the reaction was carried out at room temperature for 2 hours. A saturated aqueous sodium bicarbonate solution was added to adjust the pH to 8. Water (10 mL) was added for dilution, and the mixture was extracted with ethyl acetate (10 mL × 3). The organic phases were combined, washed with saturated brine (10 mL), dried over anhydrous sodium sulfate, filtered, and the filtrate was concentrated under reduced pressure to obtain compound 123-10 (180 mg, crude product). LC-MS: $[M+H]^+$ = 217.15.

123.11 Synthesis of compound 123

At room temperature, 123-10 (200 mg, 0.92 mmol), 001-8 (282 mg, 0.92 mmol), and *N,N*-diisopropylethylamine (240 mg, 1.85 mmol) were dissolved in ethanol (10 mL), and the reaction mixture was stirred at room temperature for 1 hour. The reaction mixture was purified by preparative HPLC (basic conditions) and lyophilized to obtain compound 123 (150.2 mg). LC-MS: $[M+H]^+$ = 475.95.

$^1$H NMR (400 MHz, DMSO-$d_6$) $\delta$ 10.07 (s, 1H), 9.45-9.35 (m, 1H), 8.47-8.43 (m, 1H), 7.98-7.92 (m, 2H), 7.64 - 7.50 (m, 1H), 7.27 - 7.16 (m, 1H), 5.83-5.75 (m, 1H), 3.16 (s, 3H), 3.02 (s, 3H), 2.42 (d, *J*= 8.0 Hz, 3H), 1.74-1.32 (m, 3H), 1.27 - 0.79 (m, 6H), 0.60 - 0.27 (m, 1H), 0.04 - -0.47 (m, 1H).

Example 124. Synthesis of 4-((2-((bicyclo[3.1.0]hexan-1-yl(3-methylpyridin-2-yl)methyl)amino)-3,4-dioxocyclobut-1-en-1-yl)amino)-3-hydroxy-*N*,*N*-dimethylpicolinamide (compound 124)

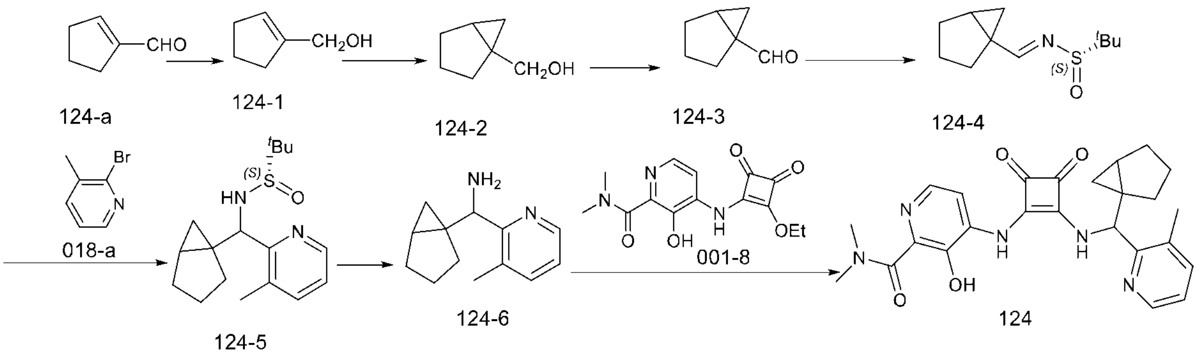

124-a, 124-1, 124-2, 124-3, 124-4, 018-a, 124-5, 124-6, 001-8, 124

124.1 Synthesis of compound 124-1

124-a (2.2 mL, 25.0 mmol) was dissolved in methanol (25 mL). At 0°C, sodium borohydride (345 mg, 7.5 mmol) was added, and the reaction mixture was stirred for 30 minutes. Then, the reaction mixture was warmed to room temperature and stirred for another 1 hour. The reaction mixture was directly concentrated under reduced pressure. The residue was purified by silica gel column chromatography (petroleum ether/ethyl acetate = 4/1) to obtain compound 124-1 (1.864 g, yield: 76%).

124.2 Synthesis of compound 124-2

124-1 (1.8 g, 17.93 mmol) and diiodomethane (2.9 mL, 35.86 mmol) were dissolved in anhydrous dichloromethane (45 mL). The system was purged with nitrogen three times. At 0°C, a solution of diethylzinc in n-hexane (26.9 mL, 26.9 mmol, 1 M) was added, and the reaction mixture was stirred at 0°C for 1 hour. At 0°C, the reaction mixture was quenched with saturated aqueous ammonium chloride solution (30 mL). The mixture was extracted with ethyl acetate (30 mL × 3). The organic phases were combined, washed with saturated sodium chloride solution (20 mL), dried over anhydrous sodium sulfate, filtered, and the filtrate was concentrated under reduced pressure. The residue was purified by silica gel column chromatography (petroleum ether/ethyl acetate = 4/1) to obtain compound 124-2 (4.371 g, crude product), which was directly used in the next step.

124.3 Synthesis of compound 124-3

124-2 (4.1 g, crude product) and silica gel (100-200 mesh, 11.0 g, 1.5 times the mass of pyridinium chlorochromate) and dichloromethane (42 mL) were added to a reaction flask. Pyridinium chlorochromate (1.8 g each time, total 7.2 g, 34 mmol) was then added in four portions, and the reaction mixture was stirred at room temperature for 1 hour. The reaction mixture was filtered, and the filtrate was concentrated under reduced pressure. The residue was purified by silica gel column chromatography (petroleum ether/ethyl acetate = 8/1) to obtain compound 124-3 (410 mg, yield: 21.9%).

124.4 Synthesis of compound 124-4

124-3 (355 mg, 3.22 mmol), anhydrous dichloromethane (10 mL), (S)-tert-butanesulfinamide (470 mg, 3.87

mmol), and tetraethyl titanate (1.35 mL, 6.44 mmol) were sequentially added to a reaction flask, and the reaction mixture was stirred at room temperature for 1 hour. At room temperature, water (10 mL) was added to the reaction mixture to quench the reaction. After stirring for 1 hour, the mixture was extracted with ethyl acetate (15 mL × 3). The organic phases were combined, washed with saturated aqueous sodium chloride solution (15 mL), dried over anhydrous sodium sulfate, filtered, and the filtrate was concentrated under reduced pressure. The residue was purified by silica gel column chromatography (petroleum ether/ethyl acetate = 4/1) to obtain compound 124-4 (381 mg, yield: 55.6%).

124.5 Synthesis of compound 124-5

018-a (1.1 mL, 10.0 mmol) was dissolved in anhydrous tetrahydrofuran (3 mL). The system was purged with nitrogen three times. At -78°C, a solution of n-butyllithium in n-hexane (5 mL, 12.0 mmol, 2.4 M) was slowly added. After stirring at -78°C for 1 hour, a brown-black solution (approximately 1 M) was obtained and directly used in the next step.

124-4 (300 mg, 1.4 mmol) was dissolved in anhydrous tetrahydrofuran (5 mL). The system was purged with nitrogen three times. At -78°C, the aforementioned brown-black solution (2.8 mL, approximately 2.8 mmol) was added dropwise to the reaction system, and stirring was continued for 1 hour. At -78°C, the reaction mixture was quenched with saturated aqueous ammonium chloride solution (5 mL). Water (10 mL) was added for dilution, and the mixture was extracted with ethyl acetate (10 mL × 3). The organic phases were combined, washed with saturated brine (10 mL), dried over anhydrous sodium sulfate, filtered, and the filtrate was concentrated under reduced pressure. The residue was purified by silica gel column chromatography (petroleum ether/ethyl acetate = 1/1) to obtain compound 124-5 (132 mg, 0.43 mmol, yield: 30.8%).

124.6 Synthesis of compound 124-6 hydrochloride

124-5 (100 mg, 0.33 mmol) was dissolved in 1,4-dioxane (0.9 mL). A solution of hydrogen chloride in ethyl acetate (0.3 mL, 4 M) was added, and the reaction mixture was stirred at room temperature for 1 hour. The reaction mixture was directly concentrated to dryness under reduced pressure to obtain compound 124-6 hydrochloride (90 mg, crude product), which was directly used in the next step. LC-MS: $[M+H]^+$ = 203.10.

124.7 Synthesis of compound 124

124-6 hydrochloride (90 mg, crude product) was dissolved in ethanol (2 mL). *N,N*-Diisopropylethylamine (284 μL, 1.63 mmol) and 001-8 (80 mg, 0.26 mmol) were added sequentially to a reaction flask, and the reaction mixture was stirred at room temperature for 2 hours. The reaction mixture was directly concentrated under reduced pressure. The residue was purified by preparative HPLC to obtain compound 124 (63.4 mg, overall yield over two steps: 42%). LCMS: $[M+H]^+$ = 461.95.

$^1H$ NMR (400 MHz, DMSO-$d_6$) δ 9.45 (dd, $J_1$ = 36.0 Hz, $J_2$ = 8.0 Hz, 1H), 8.44 (t, $J$ = 5.2 Hz, 1H), 7.99 (d, $J$ = 4.4 Hz, 1H), 7.94 (bs, 1H), 7.64 (d, $J$ = 7.6 Hz, 1H), 7.25 (t, $J$ = 5.2 Hz, 1H), 5.87 (dd, $J_1$ = 39.2 Hz, $J_2$ = 9.2 Hz, 1H), 3.16 (s, 3H), 3.04 (s, 3H), 2.34 (d, $J$ = 9.6 Hz, 3H), 1.83-1.48 (m, 5H), 1.35 (t, $J$ = 4.4 Hz, 0.5H), 1.23-1.10 (m, 2H), 0.83-0.79 (m, 0.5H), 0.55 (t, $J$ = 4.8 Hz, 0.5H), 0.45-0.4 (m, 1H), 0.13 (t, $J$ = 5.2 Hz, 0.5H).

Example 125. Synthesis of 4-((2-(((*R*)-((*S*)-3,3-difluoro-1-methylcyclopentyl)(*o*-tolyl)methyl)amino)-3,4-dioxocyclobut-1-en-1-yl)amino)-3-hydroxy-*N*,*N*-dimethylpicolinamide (125A or 125B or 125C or 125D) or 4-((2-(((*R*)-(*R*)-3,3-difluoro-1-methylcyclopentyl)(3-methylpyridin-2-yl)methyl)amino)-3,4-dioxocyclobut-1-en-1-yl)amino)-3-hydroxy-*N*,*N*-dimethylpicolinamide (125B or 125A or 125C or 125D) or 4-((2-(((*S*)-(*S*)-3,3-difluoro-1-methylcyclopentyl)(3-methylpyridin-2-yl)methyl)amino)-3,4-dioxocyclobut-1-en-1-yl)amino)-3-hydroxy-N,N-dimethylpicolinamide (125C or 125A or 125B or 125D) or 4-((2-(((S)-((R)-3,3-difluoro-1-methylcyclopentyl)(3-methylpyridin-2-yl)methyl)amino)-3,4-dioxocyclobut-1-en-1-yl)amino)-3-hydroxy-N,N-dimethylpicolinamide (125D or 125A or 125B or 125C)

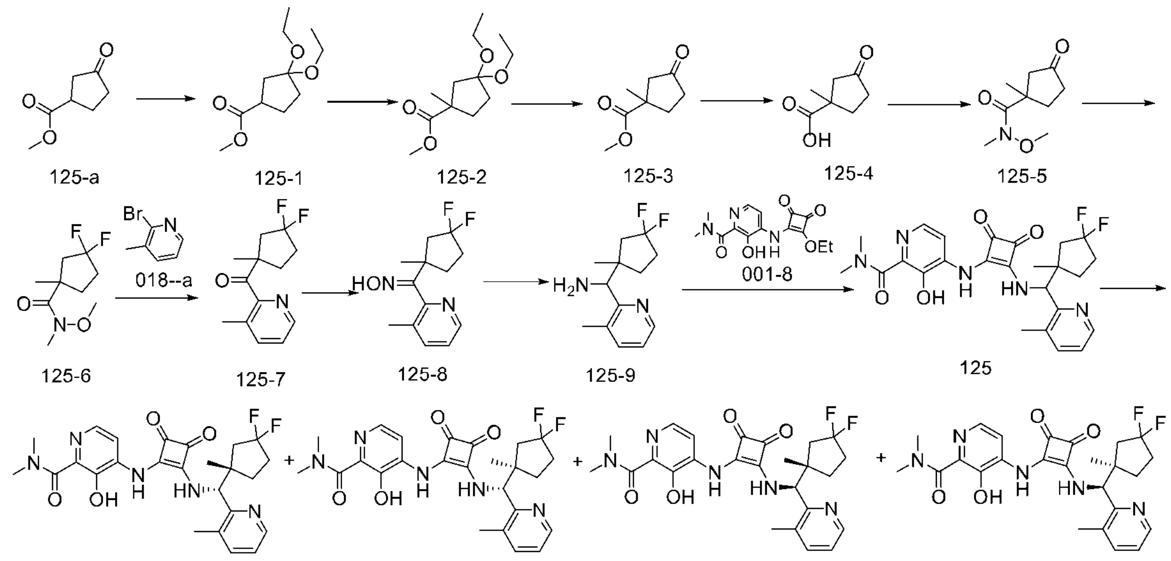


125-a 125-1 125-2 125-3 125-4 125-5

125-6 125-7 125-8 125-9 125

125A or 125B or 125C or 125D 125B or 125A or 125C or 125D 125C or 125A or 125B or 125D 125D or 125A or 125B or 125C

125.1 Synthesis of compound 125-1

125-a (5 g, 32.01 mmol), triethyl orthoformate (17.7 g, 80.04 mmol), and p-toluenesulfonic acid (460 mg, 4.8 mmol) were dissolved in ethanol (50 mL). The reaction was carried out at room temperature for 16 hours. The reaction was quenched with saturated aqueous sodium bicarbonate solution (100 mL) and extracted with ethyl acetate (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 125-1 (7.2 g, purity 95%).

125.2 Synthesis of compound 125-2

Diisopropylamine (3.5 g, 34.4 mmol) was dissolved in tetrahydrofuran (50 mL). The system was purged with nitrogen three times. The temperature was lowered to -78°C using a dry ice/ethyl acetate bath. Then, a solution of n-butyllithium in n-hexane (14.9 mL, 2.5 M, 37.27 mmol) was added dropwise slowly. After the addition was completed, the reaction mixture was warmed to room temperature and reacted for 1 hour. The temperature was lowered to -30°C. 125-1 (6.2 g, 28.67 mmol) was dissolved in tetrahydrofuran (10 mL) and added to the reaction system. The reaction mixture was warmed to room temperature and reacted for 1 hour. The temperature was lowered to - 30°C. Iodomethane (5.3 g, 37.27 mmol) was added dropwise to the system. The reaction mixture was warmed to room temperature and reacted for 3 hours. The reaction was quenched with saturated aqueous ammonium chloride solution (100 mL) and extracted with ethyl acetate (80 mL × 3). The organic phases were combined, washed with saturated brine (100 mL), dried over anhydrous sodium sulfate, filtered, and the filtrate was concentrated under reduced pressure. The residue was purified by silica gel column chromatography to obtain compound 125-2 (4.1 g).

125.3 Synthesis of compound 125-3

125-2 (4.1 g, 17.8 mmol) was dissolved in a mixed solvent of trifluoroacetic acid/dichloromethane (40 mL, 1/10 (v/v)). The reaction was carried out at room temperature for 1 hour. The reaction was quenched with water (50 mL) and extracted with ethyl acetate (60 mL × 3). The organic phases were combined, dried over anhydrous sodium sulfate, filtered, and the filtrate was concentrated under reduced pressure to obtain compound 125-3 (4.1 g, crude product).

125.4 Synthesis of compound 125-4

125-3 (2.7 g, 17.29 mmol) and sodium hydroxide (3.5 g, 86.44 mmol) were dissolved in a mixed solvent of ethanol/water (30 mL, 3/1 (v/v)). The reaction was carried out at room temperature for 1 hour. Water (50 mL) was added, and the mixture was extracted with ethyl acetate (50 mL × 3). The aqueous phase was collected and adjusted to pH = 2 with 1 N hydrochloric acid, then extracted again with ethyl acetate (50 mL × 3). The organic phases were combined, dried over

anhydrous sodium sulfate, filtered, and the filtrate was concentrated under reduced pressure. The residue was purified by silica gel column chromatography to obtain compound 125-4 (2.1 g).

125.5 Synthesis of compound 125-5

125-4 (1.7 g, 11.96 mmol) was dissolved in dichloromethane (20 mL), and the temperature was lowered to 0°C. Then, oxalyl chloride (2.3 g, 17.94 mmol) was added dropwise slowly. The reaction mixture was warmed to room temperature and reacted for 1 hour. The reaction mixture was concentrated under reduced pressure. The residue was dissolved in dichloromethane (20 mL), and the temperature was lowered to 0°C. Then, triethylamine (3.6 g, 35.88 mmol) and dimethylhydroxylamine hydrochloride (1.8 g, 17.94 mmol) were slowly added. The reaction was carried out at room temperature for 1 hour. The reaction mixture was concentrated under reduced pressure. The residue was purified by silica gel column chromatography to obtain compound 125-5 (1.0 g).

125.6 Synthesis of compound 125-6

125-5 (700 mg, 3.78 mmol), diethylaminosulfur trifluoride (3.1 g, 18.9 mmol), and trifluoroacetic acid (74 mg, 0.75 mmol) were dissolved in dichloromethane (20 mL). The reaction was carried out at room temperature for 15 hours. Additional diethylaminosulfur trifluoride (3.1 g, 18.9 mmol) was added, and the reaction was carried out at room temperature for another 10 hours. The reaction was quenched with water (20 mL) and extracted with dichloromethane (20 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 125-6 (300 mg).

125.7 Synthesis of compound 125-7

018-a (207 mg, 1.21 mmol) was dissolved in tetrahydrofuran (3 mL). The system was purged with nitrogen three times. The temperature was lowered to -78°C, and a solution of *n*-butyllithium in n-hexane (0.53 mL, 1.33 mmol) was added dropwise slowly. The reaction was carried out for 15 minutes. 125-6 (250 mg, 1.21 mmol) was dissolved in tetrahydrofuran (2 mL), and the resulting solution was added dropwise to the reaction system. The reaction was carried out at -78°C for another 15 minutes. A saturated aqueous ammonium chloride solution (20 mL) was slowly added to quench the reaction. The mixture was extracted with ethyl acetate (20 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 125-7 (170 mg). LC-MS: $[M+H]^+$ = 240.05.

125.8 Synthesis of compound 125-8

At room temperature, 125-7 (170 mg, 0.71 mmol) was dissolved in ethanol (4 mL). The temperature was lowered to 0°C, and hydroxylamine hydrochloride (247 mg, 3.55 mmol) and sodium acetate (373 mg, 3.55 mmol) were added. The reaction mixture was warmed to 80°C and reacted for 2 hours. Water (10 mL) was added, and the mixture was extracted with ethyl acetate (10 mL × 3). The organic phases were combined, washed with saturated brine (10 mL), dried over anhydrous sodium sulfate, filtered, and the filtrate was concentrated under reduced pressure to obtain compound 125-8 (150 mg, crude product). LC-MS: $[M+H]^+$ = 255.05.

125.9 Synthesis of compound 125-9

At room temperature, 125-8 (150 mg, 0.59 mmol) and zinc powder (385 mg, 5.9 mmol) were dissolved in hydrochloric acid (2 mL, 2 N). The reaction was carried out at room temperature for 1 hour. The pH was adjusted to 8-9 with saturated aqueous sodium bicarbonate solution. Water (10 mL) was added, and the mixture was extracted with ethyl acetate (10 mL × 3). The organic phases were combined, washed with saturated brine (10 mL), dried over anhydrous sodium sulfate, filtered, and the filtrate was concentrated under reduced pressure to obtain compound 125-9 (100 mg, crude product).

125.10 Synthesis of compound 125

At room temperature, 125-9 (50 mg, 0.21 mmol), 001-8 (97 mg, 0.32 mmol), and *N,N*-diisopropylethylamine (83 mg, 0.64 mmol) were dissolved in ethanol (1 mL). The reaction mixture was stirred at room temperature for 0.5 hours. The reaction mixture was purified by HPLC (basic conditions) and then lyophilized to obtain compound 125 (32.3 mg). LC-MS:

$[M+H]^+$ = 479.05.

$^1$H NMR (400 MHz, DMSO-$d_6$) δ 11.66 (s, 1H), 10.00 (s, 1H), 9.30 (s, 1H), 8.50 (t, *J* = 3.6 Hz, 1H), 7.97 (s, 2H), 7.66 (d, *J*= 6.4 Hz, 1H), 7.29 (dt, *J* = 8.4, 4.4 Hz, 1H), 5.72 (dd, *J* = 10.0, 2.4 Hz, 1H), 3.09 (d, *J* = 50.8 Hz, 6H), 2.38 (s, 3H), 2.32 - 1.73 (m, 5H), 1.61 - 1.47 (m, 1H), 1.09 (d, *J* = 50.8 Hz, 3H).

125.11 Synthesis of compounds 125A, 125B, 125C, and 125D

Compound 125 was subjected to chiral resolution to obtain compound 125A (90.1 mg), 125B (109.4 mg), 125C (103.2 mg), and 125D (84.4 mg).

HPLC method: CHIRALPAKAD-H, 4.6 * 250 mm 5 μm, 20% ethanol/n-hexane, 0.8 mL/min, 30°C.

125A:

Chiral purity: 100%, $T_R$ = 10.397 min;

$^1$H NMR (400 MHz, DMSO-$d_6$) δ 9.33 (d, *J* = 8.8 Hz, 1H), 8.50 (d, *J* = 4.0 Hz, 1H), 7.99 (d, *J* = 4.8 Hz, 1H), 7.94 (s, 1H), 7.67 (d, *J* = 7.6 Hz, 1H), 7.34 - 7.24 (m, 1H), 5.73 (d, *J* = 9.8 Hz, 1H), 3.16 (s, 3H), 3.04 (s, 3H), 2.57 (d, *J* = 18.2 Hz, 1H), 2.39 (s, 3H), 2.23 - 2.02 (m, 3H), 1.92 (td, *J* = 15.2, 5.2 Hz, 1H), 1.55 - 1.46 (m, 1H), 1.04 (s, 3H).

125B:

Chiral purity: 100%, $T_R$ = 9.427 min;

$^1$H NMR (400 MHz, DMSO-$d_6$) δ 9.33 (d, *J* = 8.8 Hz, 1H), 8.51 (d, *J* = 4.2 Hz, 1H), 7.98 (t, *J* = 10.6 Hz, 2H), 7.67 (d, *J* = 7.6 Hz, 1H), 7.30 (dd, *J* = 7.6, 4.8 Hz, 1H), 5.74 (d, *J* = 9.8 Hz, 1H), 3.16 (s, 3H), 3.04 (s, 3H), 2.66 - 2.53 (m, 1H), 2.40 (s, 3H), 2.26 - 2.11 (m, 1H), 2.00 (dd, *J* = 17.2, 8.2 Hz, 1H), 1.88 (ddd, *J* = 23.0, 14.0, 8.4 Hz, 2H), 1.65 - 1.55 (m, 1H), 1.16 (s, 3H).

125C:

Chiral purity: 100%, $T_R$ = 8.380 min;

$^1$H NMR (400 MHz, DMSO-$d_6$) δ 9.33 (d, *J* = 8.2 Hz, 1H), 8.51 (d, *J* = 4.2 Hz, 1H), 7.97 (t, *J* = 10.2 Hz, 2H), 7.68 (d, *J* = 7.8 Hz, 1H), 7.30 (dd, *J* = 7.4, 4.8 Hz, 1H), 5.74 (d, *J* = 9.8 Hz, 1H), 3.16 (s, 3H), 3.04 (s, 3H), 2.65 - 2.53 (m, 1H), 2.40 (s, 3H), 2.26 - 2.12 (m, 1H), 1.99 (d, *J* = 12.8 Hz, 1H), 1.96 - 1.81 (m, 2H), 1.59 (t, *J* = 8.8 Hz, 1H), 1.16 (s, 3H).

125D:

Chiral purity: 100%, $T_R$ = 8.140 min;

$^1$H NMR (400 MHz, DMSO-$d_6$) δ 9.33 (d, *J* = 9.8 Hz, 1H), 8.50 (d, *J* = 4.2 Hz, 1H), 7.99 (d, *J* = 5.4 Hz, 1H), 7.94 (s, 1H), 7.67 (d, *J* = 7.6 Hz, 1H), 7.29 (dd, *J* = 7.6, 4.8 Hz, 1H), 5.73 (d, *J* = 9.8 Hz, 1H), 3.15 (s, 3H), 3.04 (s, 3H), 2.57 (d, *J* = 18.4 Hz, 1H), 2.39 (s, 3H), 2.26 - 2.04 (m, 3H), 1.97 - 1.87 (m, 1H), 1.50 (t, *J* = 8.2 Hz, 1H), 1.04 (s, 3H).

Example 126. Synthesis of 4-((2-(((3,6-difluoropyridin-2-yl)(3-methylbicyclo[3.1.0]hexan-3-yl)methyl)amino)-3,4-dioxocyclobut-1-en-1-yl)amino)-3-hydroxy-*N*,*N*-dimethylpicolinamide (compound 126)

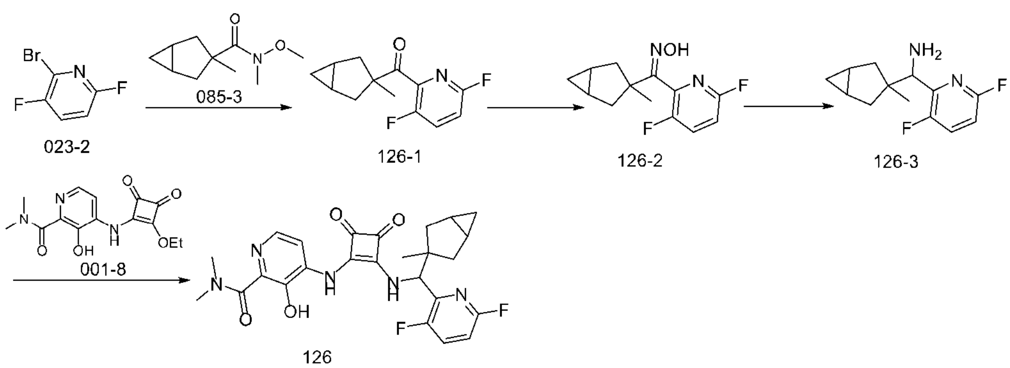


023-2 126-1 126-2 126-3

126

The synthesis of compound 126 was carried out with reference to the synthesis method for compound 085 in

Example 85, except that the starting material 053-3 was replaced with 023-2, to obtain compound 126 (34.9 mg). LC-MS: $[M+H]^+$ = 497.90.

$^1$H NMR (400 MHz, DMSO-$d_6$) δ 11.70 (s, 1H), 10.01 (s, 1H), 9.17 (dd, *J* = 27.6, 10.0 Hz, 1H), 7.98 (dt, *J* = 15.6, 7.0 Hz, 3H), 7.29 - 7.21 (m, 1H), 5.68 (dd, *J* = 40.6, 10.2 Hz, 1H), 3.18 (s, 3H), 3.04 (s, 3H), 1.60 (dd, *J* = 13.0, 5.6 Hz, 1H), 1.56 - 1.37 (m, 3H), 1.32 (d, *J* = 3.6 Hz, 2H), 1.04 (d, *J* = 37.8 Hz, 3H), 0.82 (td, *J* = 8.0, 4.4 Hz, 1H), 0.01 (dd, *J* = 7.8, 3.8 Hz, 1H).

Example 127. Synthesis of 4-((2-(((3-fluoro-1-methylcyclopentyl)(3-methylpyridin-2-yl)methyl)amino)-3,4-dioxocyclobut-1-en-1-yl)amino)-3-hydroxy-*N*,*N*-dimethylpicolinamide (compound 127) hydrochloride

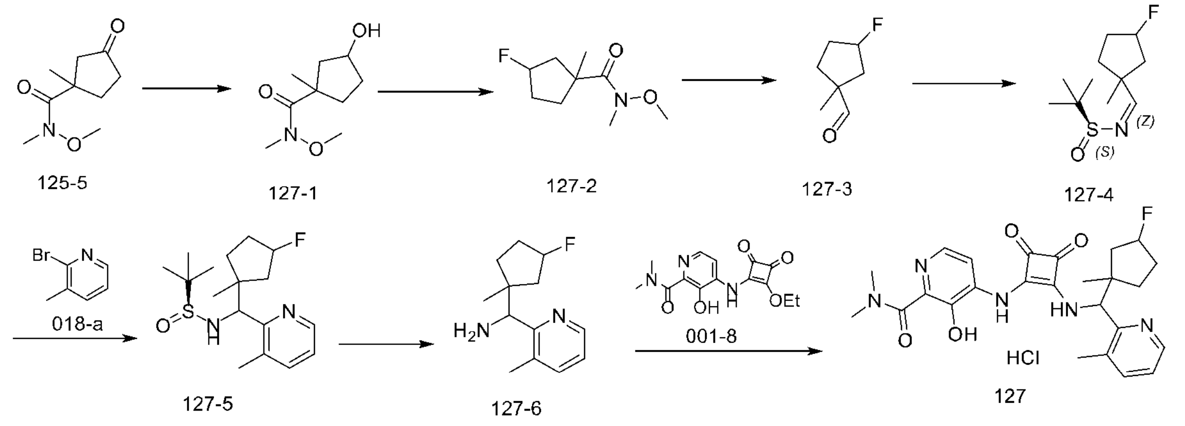


125-5 127-1 127-2 127-3 127-4 127-5 127-6 127

127.1 Synthesis of compound 127-1

125-5 (2.19 g, 11.82 mmol) was dissolved in ethanol (40 mL). The reaction mixture was cooled to 0°C, and then sodium borohydride (537 mg, 14.19 mmol) was slowly added. The reaction mixture was warmed to room temperature and reacted for 1 hour. The reaction mixture was poured into a saturated aqueous ammonium chloride solution (60 mL) to quench the reaction, and then extracted with dichloromethane (60 mL × 3). The organic phases were combined, washed with saturated brine (60 mL), dried over anhydrous sodium sulfate, filtered, and the filtrate was concentrated under reduced pressure. The residue was purified by silica gel column chromatography to obtain compound 127-1 (1.75 g, yield: 79.1%).

127.2 Synthesis of compound 127-2

127-1 (1.75 g, 9.34 mmol) was dissolved in anhydrous dichloromethane (90 mL). Pyridine (1.33 mL, 16.81 mmol) was added, followed by slow dropwise addition of diethylaminosulfur trifluoride (4.53 mL, 35.49 mmol). The reaction mixture was reacted at 25°C for 2 hours. The reaction mixture was slowly poured into a 5% aqueous sodium bicarbonate solution (60 mL) and extracted with dichloromethane (60 mL × 3). The organic phases were combined, washed with saturated brine (60 mL), dried over anhydrous sodium sulfate, filtered, and the filtrate was concentrated under reduced pressure. The residue was purified by silica gel column chromatography to obtain compound 127-2 (757 mg, yield: 42.8%).

127.3 Synthesis of compound 127-3

At room temperature, 127-2 (757 mg, 4.00 mmol) was dissolved in dichloromethane (13 mL). Under a nitrogen atmosphere at -78°C, diisobutylaluminum hydride (6.00 mL, 6.00 mmol, 1 M) was added, and the reaction was carried out at -78°C for 3 hours. Water (3 mL) was added to quench the reaction, followed by the addition of a saturated aqueous potassium sodium tartrate solution (100 mL) to clarify the reaction mixture. The mixture was extracted with dichloromethane (100 mL × 3). The organic phases were combined, washed with saturated brine (100 mL), dried over anhydrous sodium sulfate, filtered, and the filtrate was concentrated under reduced pressure to obtain compound 127-3 (520 mg, crude product).

127.4 Synthesis of compound 127-4

At room temperature, 127-3 (520 mg, crude product) was dissolved in dichloromethane (15 mL). Tetraethyl

titanate (1.83 g, 8.00 mmol) and (S)-tert-butylsulfinamide (726 mg, 5.99 mmol) were added, and the reaction was carried out at room temperature for 16 hours. The reaction mixture was quenched with water (10 mL) and extracted with ethyl acetate (30 mL × 3). The organic phase was directly concentrated under reduced pressure. The residue was purified by silica gel column chromatography to obtain compound 127-4 (220 mg, yield over two steps: 23.60%).

127.5 Synthesis of compound 127-5

At room temperature, 018-a (140 mg, 0.81 mmol) and tetrahydrofuran (5 mL) were added to a reaction flask. The system was purged with nitrogen three times. At -78°C, n-butyllithium (0.39 mL, 2.5 M, 0.98 mmol) was added, and the reaction was carried out at -78°C for 0.5 hours. 127-4 (189 mg, 0.81 mmol) was dissolved in tetrahydrofuran (0.5 mL) and the resulting solution was added dropwise to the reaction mixture. The reaction was carried out at -78°C for 1 hour. The reaction mixture was poured into a saturated aqueous ammonium chloride solution (20 mL) to quench the reaction, and then extracted with ethyl acetate (20 mL × 3). The organic phases were combined, washed with saturated brine (20 mL), dried over anhydrous sodium sulfate, filtered, and the filtrate was concentrated under reduced pressure. The residue was purified by silica gel column chromatography to obtain compound 127-5 (110 mg, yield: 34.8%). LC-MS: $[M+H]^+$ = 327.10.

127.6 Synthesis of compound 127-6

At room temperature, 127-5 (100 mg, 0.30 mmol) was dissolved in a solution of hydrogen chloride in 1,4-dioxane (6 mL, 0.5 M) and stirred at room temperature for 0.5 hours. The reaction mixture was directly concentrated under reduced pressure to obtain compound 127-6 (70 mg, crude product).

127.7 Synthesis of compound 127 hydrochloride

127-6 (70 mg, 0.31 mmol) was dissolved in ethanol (3 mL). 001-8 (95 mg, 0.31 mmol) and N,N-diisopropylethylamine (201 mg, 1.56 mmol) were added, and the reaction mixture was stirred at room temperature for 3 hours. The reaction mixture was purified by HPLC (acidic conditions), and then lyophilized to obtain compound 127 hydrochloride (28.0 mg). LC-MS: $[M+H]^+$ = 482.10.

$^1$H NMR (400 MHz, DMSO-$d_6$) δ 10.85 - 10.68 (m, 1H), 9.60 (dd, *J* = 15.6, 9.8 Hz, 1H), 8.49 (d, *J* = 4.2 Hz, 1H), 8.38 - 8.23 (m, 2H), 7.66 (d, *J* = 7.6 Hz, 1H), 7.33 - 7.23 (m, 1H), 5.72 (dd, *J* = 27.0, 9.8 Hz, 1H), 5.13 (d, *J* = 54.8 Hz, 1H), 2.97 (d, *J* = 25.8 Hz, 6H), 2.38 (s, 3H), 2.16 - 2.04 (m, 1H), 1.96 - 1.61 (m, 4H), 1.48 (dd, *J* = 19.4, 14.4 Hz, 1H), 1.13 - 0.91 (m, 3H).

Example 128. Synthesis of 4-((2-(((*S*)-((*R*)-4,4-difluoro-2-methyltetrahydrofuran-2-yl)(3-methylpyridin-2-yl)methyl)amino)-3,4-dioxocyclobut-1-en-1-yl)amino)-3-hydroxy-*N*,*N*-dimethylpicolinamide (compounds 128A and 128B) and 4-((2-(((*S*)-((*S*)-4,4-difluoro-2-methyltetrahydrofuran-2-yl)(3-methylpyridin-2-yl)methyl)amino)-3,4-dioxocyclobut-1-en-1-yl)amino)-3-hydroxy-*N*,*N*-dimethylpicolinamide (compounds 128B and 128A)

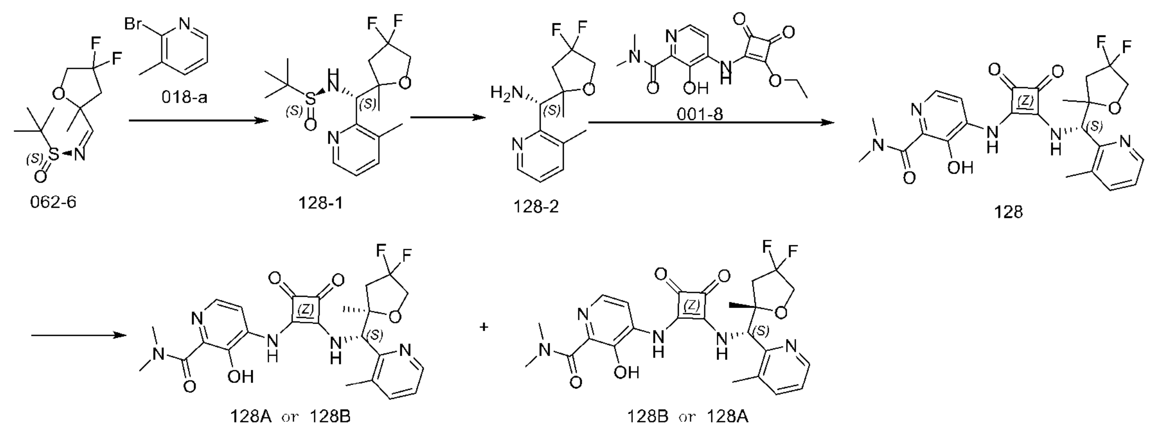


062-6 128-1 128-2 128

128A or 128B + 128B or 128A

128.1 Synthesis of compound 128

The synthesis of compound 128 was carried out with reference to the synthesis method for compound 062 in Example 62, except that the starting material 023-2 was replaced with 018-a, to obtain compound 128 (137.7 mg, yield:

51%).

128.2 Synthesis of compounds 128A and 128B

The racemate was resolved by chiral column chromatography to obtain 128A (41 mg) and 128B (30.2 mg). LC-MS: $[M+H]^+$ = 502.10.

HPLC method: CHIRALPAK AD-H, 4.6 * 250 mm 5 μm, 20% isopropanol/n-hexane, 0.8 mL/min, 30°C.

Characterization data for 128A are as follows:

Chiral purity: 99.1%, $T_R$ = 9.645 min;
$^1$H NMR (400 MHz, DMSO-$d_6$) δ 10.82 (d, *J* = 644.0 Hz, 1H), 9.43 (s, 1H), 8.52 (d, *J* = 4.0 Hz, 1H), 7.98 (s, 2H), 7.69 (d, *J* = 8.0 Hz, 1H), 7.35-7.31 (m, 1H), 5.92 (d, *J* = 8.0 Hz, 1H), 3.99-3.91 (m, 1H), 3.37 (s, 1H), 3.17 (s, 3H), 3.05 (s, 3H), 2.52 (s, 1H), 2.41 (s, 3H), 2.34 - 2.21 (m, 1H), 1.48 (s, 3H).

Characterization data for 128B are as follows:

Chiral purity: 97.8%, $T_R$ = 14.299 min;
$^1$H NMR (400 MHz, DMSO-$d_6$) δ 10.79 (d, *J* = 672.0 Hz, 1H), 9.35 (s, 1H), 8.48 (d, *J* = 4.0 Hz, 1H), 7.98 (s, 2H), 7.66 (d, *J* = 4.0 Hz, 1H), 7.30 (q, *J* = 4.0, 1H), 5.91 (d, *J* = 12.0 Hz, 1H), 4.07 - 3.90 (m, 2H), 3.16 (s, 3H), 3.02 (s, 3H), 2.50 (s, 1H), 2.39 (s, 3H), 2.39-2.25 (m, 1H), 1.20 (s, 3H).

Example 129. Synthesis of (*R*)-4-((2-(((3,6-difluoropyridin-2-yl)(5-methylspiro[2.3]hexan-5-yl)methyl)amino)-3,4-dioxocyclobutan-1-yl)amino)-3-hydroxy-*N*,*N*-dimethylpicolinamide (compound 129)

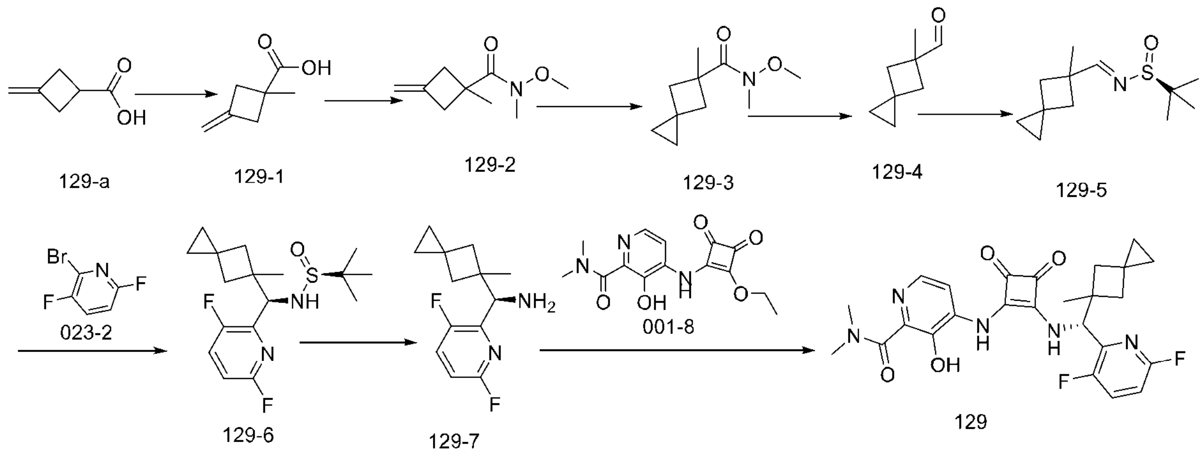

129-a 129-1 129-2 129-3 129-4 129-5

023-2 129-6 129-7 001-8 129

129.1 Synthesis of compound 129-1

Diisopropylamine (6.8 g, 66.9 mmol) was weighed into a reaction flask, and tetrahydrofuran (20 mL) was added. After purging with nitrogen three times, n-butyllithium (26.8 mL, 66.9 mmol, 2.5 M) was added at -78°C. The reaction mixture was warmed to 25°C and reacted for 2 hours. The reaction mixture was cooled to -78°C, and 129-a (3.0 g, 26.8 mmol) was added. The temperature was then warmed to 25°C, and the reaction was carried out for 2 hours. The reaction system was again cooled to -78°C, and iodomethane (5.7 g, 40.0 mmol) was added. The temperature was warmed to 25°C, and the reaction was carried out for another 4 hours. The reaction mixture was quenched with saturated aqueous ammonium chloride solution (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 129-1 (3.2 g, yield: 94.9%).

129.2 Synthesis of compound 129-2

129-1 (3.2 g, 25.3 mmol) was weighed and dissolved in *N,N*-dimethylformamide (50 mL). Triethylamine (5.1 g, 50.7 mmol), 2-(7-azabenzotriazol-1-yl)-*N*,*N*,*N'*,*N'*-tetramethyluronium hexafluorophosphate (10.6 g, 27.9 mmol), and

dimethylhydroxylamine hydrochloride (2.47 g, 25.3 mmol) were added sequentially. The reaction was carried out at 25°C for 3 hours. The reaction mixture was diluted with water (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 129-2 (3.1 g, yield: 72.2%). LCMS: $[M+H]^+$ = 170.1.

129.3 Synthesis of compound 129-3

Diethylzinc (17.7 mL, 17.7 mmol, 1 M) was dissolved in dichloromethane (15 mL). At 0°C, trifluoroacetic acid (1.0 g, 8.85 mmol) was slowly added, and the reaction was maintained at 0°C and carried out for 15 minutes. Then, diiodomethane (9.5 g, 35.4 mmol) was slowly added, and the reaction was carried out at 0°C for another 30 minutes. 129-2 (1.5 g, 8.85 mmol) was added, and the reaction was carried out at room temperature for 6 hours. The reaction mixture was quenched with saturated ammonium chloride aqueous solution (50 mL) and extracted with ethyl acetate (50 mL × 3). The organic phase was dried over anhydrous sodium sulfate, filtered, and the filtrate was concentrated under reduced pressure. The residue was purified by silica gel column chromatography to obtain compound 129-3 (1.2 g, yield: 74.1%). LCMS: $[M+H]^+$ = 184.1.

129.4 Synthesis of compound 129

The synthesis of compound 129 was carried out with reference to the synthesis method for compound 074 in Example 74, except that the starting material 074-1 was replaced with 129-3 and 018-a was replaced with 023-2, to obtain compound 129 (9.8 mg, yield: 10.4%, chiral purity: 95.0% (HPLC method: CHIRALPAK AD-H, 4.6 * 250 mm 5 μm, 15% ethanol/n-hexane, 0.8 mL/min, 30°C)). LCMS: $[M+H]^+$ = 498.1.

$^1$H NMR (400 MHz, DMSO-$d_6$) δ 9.25 (s, 2H), 8.01-7.95 (m,1H), 7.80 (d, *J* = 4.0 Hz, 1H), 7.59 (d, *J* = 4.0 Hz, 1H),7.24-7.20 (m,1H), 5.83 (s,1H), 3.59 (t, *J* = 4.0 Hz, 3H), 2.96 (d, *J* = 8.0 Hz, 3H), 2.27 (t, *J* = 12.0 Hz, 1H), 1.75-1.72 (m, 6H), 1.24-1.22 (m, 1H), 2.96 (d, *J* = 4.0 Hz, 1H), 0.39-0.35(m, 1H), 0.26-0.22 (m, 1H).

Example 130. Synthesis of (R)-6-fluoro-2-hydroxy-N,N-dimethyl-3-((2-(((1-methylcyclopentyl)(3-methylpyrazin-2-yl) methyl)amino)-3,4-dioxocyclobut-1-en-1-yl)amino)benzamide (compound 130)

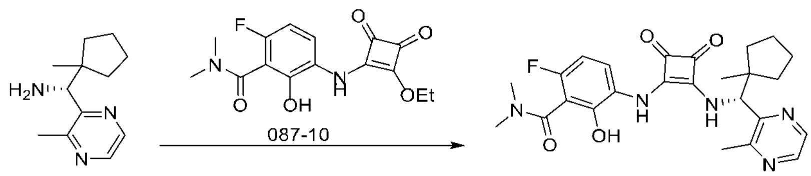


078-2 130

The synthesis of compound 130 was carried out with reference to the synthesis method for compound 001 in Example 1, except that the starting material 001-10 was replaced with 078-2 and 001-8 was replaced with 087-10, to obtain compound 130 (112.4 mg, yield: 41.7%, chiral purity: 100% (HPLC method: CHIRALPAK AD-H, 4.6 * 250 mm 5 μm, 25% isopropanol/n-hexane, 0.8 mL/min, 30°C)). LC-MS: $[M+H]^+$ = 482.10.

$^1$H NMR (400 MHz, DMSO-$d_6$) δ 8.92 (d, *J* = 8.0 Hz, 1H), 8.47 - 8.44 (m, 2H), 7.65-7.61 (m, 1H), 6.56 (t, *J* = 8.0 Hz, 1H), 5.74 (d, *J* = 8.0 Hz, 1H), 2.96 (s, 3H), 2.81 (s, 3H), 2.63 (s, 3H), 1.72 - 1.69 (m, 2H), 1.58-1.52 (m, 4H), 1.33 - 1.30 (m, 1H), 1.22-1.18 (m, 1H), 0.97 (s, 3H).

Example 131. Synthesis of 4-((2-(((4,4-difluoro-2-methyltetrahydrofuran-2-yl)(3-methylpyridin-2-yl)methyl)amino)-3,4-dioxocyclobut-1-en-1-yl)amino)-3-hydroxy-N-isopropyl-N-methylpicolinamide (compound 131)

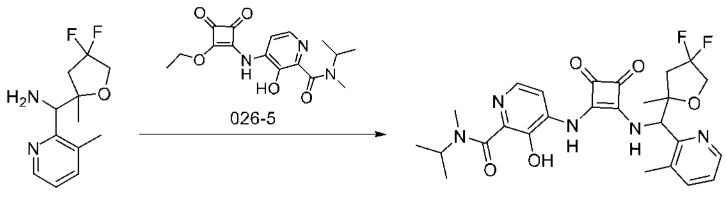

128-2 131

The synthesis of compound 131 was carried out with reference to the synthesis method for compound 001 in Example 1, except that the starting material 001-10 was replaced with 128-2 and 001-8 was replaced with 026-5, to obtain compound 131 (57.2 mg). LC-MS: $[M+H]^+$ = 530.15.

$^1H$ NMR (400 MHz, DMSO-$d_6$) δ 9.41 (s, 1H), 8.49 (d, *J* = 3.8 Hz, 1H), 7.94 (s, 2H), 7.68 (d, *J* = 7.6 Hz, 1H), 7.35 - 7.28 (m, 1H), 5.93 (d, *J* = 9.8 Hz, 1H), 4.47 (d, *J* = 248.9 Hz, 1H), 4.09 - 3.86 (m, 2H), 3.11 - 2.99 (m, 1H), 2.87 (s, 3H), 2.41 (s, 3H), 2.36 - 2.25 (m, 1H), 1.48 (s, 3H), 1.13 (s, 6H).

<Biological activity test assay>

1. Calcium flux activity screening assay for compounds of the present disclosure

1.1 Screening of human CCR6 calcium flux activity

The activity of the compounds was evaluated using HEK293 cells stably expressing human CCR6 via the FLIPR platform. A 384-well black cell culture plate (Greiner, 781946) was used, with a seeding density of 20,000 cells/well, and incubated overnight at 37°C in a 5% $CO_2$ incubator. The culture medium in the cell plate wells was removed, and 20 μL of Fluo-4 Direct™ Calcium Assay Buffer (Invitrogen, F10471, Component C), 20 μL of 2× Fluo-4 Direct™ Calcium Assay Reagent (Invitrogen, F10471, Component A) working solution, and 10 μL of 6× test compound working solution were sequentially added to each well. The cell plate was incubated in a 37 °C, 5% $CO_2$ incubator for 50 min, followed by a 10 min incubation at room temperature. Additionally, a 6×$EC_{80}$ human MIP-3 alpha/CCL20 (MCE, HY-P7262) working solution was prepared. After the incubation was completed, 10 μL/well of the 6×$EC_{80}$ human MIP-3 alpha/CCL20 working solution was transferred to the cell plate using a FLIPR instrument, and the fluorescence signal changes at an excitation wavelength of 494 nm and an emission wavelength of 516 nm were recorded over 90 seconds. The difference between the maximum fluorescence signal ($RFU_{Max}$) and the minimum fluorescence signal ($RFU_{Min}$) was used as the output signal. The compound inhibition rate and the log value of the compound concentration were calculated, and a four-parameter fitting analysis was performed using GraphPad Prism 8 software to obtain the $IC_{50}$ value of the test compound.

1.2 Screening of human CXCR2 calcium flux activity

The activity of the compounds was evaluated using HEK293 cells stably expressing human CXCR2 via the FLIPR platform. A 384-well black cell culture plate (Greiner, 781946) was used, with a seeding density of 20,000 cells/well, and incubated overnight at 37°C in a 5% $CO_2$ incubator. The culture medium in the cell plate wells was removed, and 20 μL of Fluo-4 Direct™ Calcium Assay Buffer (Invitrogen, F10471, Component C), 20 μL of 2× Fluo-4 Direct™ Calcium Assay Reagent (Invitrogen, F10471, Component A) working solution, and 10 μL of 6× test compound working solution were sequentially added to each well. The cell plate was incubated in a 37 °C, 5% $CO_2$ incubator for 50 min, followed by a 10 min incubation at room temperature. Additionally, a 6×$EC_{80}$ human IL-8 (Sigma, SRP3311) working solution was prepared. After the incubation was completed, 10 μL/well of the 6×$EC_{80}$ human IL-8 working solution was transferred to the corresponding cell wells using a FLIPR instrument, and the fluorescence signal changes at an excitation wavelength of 494 nm and an emission wavelength of 516 nm were recorded over 130 s. The difference between the maximum fluorescence signal ($RFU_{Max}$) and the minimum fluorescence signal ($RFU_{Min}$) was used as the output signal. The compound inhibition rate and the log value of the compound concentration were calculated, and a four-parameter fitting analysis was performed using GraphPad Prism 8 software to obtain the $IC_{50}$ value of the test compound.

Table 1. Inhibitory activity data of compounds against CCR6 and CXCR2

| Example | Compound number | CCR6 inhibitory activity, $IC_{50}$ (nM) | CXCR2 inhibitory activity, $IC_{50}$ (nM) | CCR6 selectivity |
|---|---|---|---|---|
| 1 | 001 | 2.4 | - | - |
| 2 | 002 | 7.0 | - | - |

(continued)

| Example | Compound number | CCR6 inhibitory activity, $IC_{50}$ (nM) | CXCR2 inhibitory activity, $IC_{50}$ (nM) | CCR6 selectivity |
|---|---|---|---|---|
| 3 | 003 | 22.1 | 2209 | 100.0 |
| 4 | 004 | 12.3 | - | - |
| 5 | 005 | 10.9 | 1254 | 115.0 |
| 6 | 006 | 74.0 | - | - |
| 8 | 008 | 150.9 | - | - |
| 9 | 009 | 8.0 | 2427 | 303.4 |
| 11 | 011 | 22.5 | - | - |
| 12 | 012 | 9.0 | - | - |
| 13 | 013 | 19.3 | - | - |
| 14 | 014 | 14.3 | 6741 | 471.4 |
| 15 | 015 | 160.5 | - | - |
| 16 | 016 | 55.8 | 27613 | 494.9 |
| 17 | 017 | 10.0 | - | - |
| 18 | 018 | 11.0 | 91998 | 8363.5 |
| 19 | 019 | 5.0 | 1821 | 364.2 |
| 21 | 021 | 6.4 | - | - |
| 22 | 022 | 7.4 | - | - |
| 23 | 023 | 0.9 | 204.3 | 227.0 |
| 24 | 024 | 47.4 | - | - |
| 25 | 025 | 2.8 | 790.8 | 282.4 |
| 26 | 026 | 0.7 | - | - |
| 27 | 027 | 1372 | - | - |
| 29 | 029 | 160.6 | - | - |
| 34 | 034 | 887.3 | - | - |
| 35 | 035 | 820.5 | - | - |
| 37 | 037 | 95.8 | - | - |
| 38 | 038 | 112.8 | - | - |
| 48 | 048 | 60.8 | - | - |
| 51 | 051 | 12.7 | 31357 | 2469.1 |
| 52 | 052 | 195.6 | - | - |
| 53 | 053 | 1.5 | 1745 | 1163.3 |
| 59 | 059 | 13.6 | - | - |
| 60 | 060 | 1.9 | 393.3 | 207.0 |
| 61 | 061 | 151.6 | - | - |
| 62 | 062 | 32.9 | - | - |
| 63 | 063 | 11.7 | 15336 | 1310.8 |
| 64 | 064 | 6.5 | 11246 | 1730.2 |
| 65 | 065 | 3.9 | - | - |
| 66 | 066 | 19.0 | 47932 | 2522.7 |

(continued)

| Example | Compound number | CCR6 inhibitory activity, $IC_{50}$ (nM) | CXCR2 inhibitory activity, $IC_{50}$ (nM) | CCR6 selectivity |
|---|---|---|---|---|
| 68B | 068B | 5.3 | 7633 | 1440.2 |
| 77 | 077 | 17.6 | - | - |
| 78 | 078 | 56.2 | - | - |
| 79 | 079 | 17.3 | - | - |
| 85 | 085 | 38.0 | - | - |
| 93A | 093A | 17.1 | > 100000 | > 5848.0 |
| 94 | 094 | 1.1 | 192.2 | 174.73 |
| 103 | 103 | 73.6 | - | - |
| 104 | 104 | 4.0 | - | - |
| 107 | 107 | 3.8 | 744.7 | 196.0 |
| 108 | 108 | 3.9 | 739.6 | 189.6 |
| 109 | 109 | 2.4 | 133.2 | *55.5* |
| 110 | 110A | 9.3 | 297.2 | 32.0 |
| 110 | 110B | 53.6 | - | - |
| 111 | 111 | 105.9 | - | - |
| 112 | 112 | 164.6 | - | - |
| 113 | 113 | 49.8 | 1479.0 | 29.7 |
| 114 | 114 | 55.3 | 1450.0 | 26.2 |
| 115 | 115 | 47.6 | 527.4 | 11.1 |
| 116 | 116 | 28.9 | 379.4 | 13.1 |
| 118 | 118 | 182.7 | - | - |
| 121 | 121 | 86.2 | - | - |
| 122 | 122 | 181.5 | - | - |
| 123 | 123 | 265.4 | - | - |
| 125 | 125A | 16.0 | 60932.0 | 3808.3 |

Experimental conclusion: The compounds of the present disclosure exhibit excellent inhibitory activity against CCR6 and demonstrate good selectivity relative to CXCR2.

2. ***In vivo* pharmacokinetic study of compounds of the present disclosure**

2.1 Experimental instruments

| Name | Fixed asset number | Model/specification | Manufacturer |
|---|---|---|---|
| Electronic analytical balance | GG1206363 | MS105Du | Mettler-Toledo |
| Electronic scale | GG1206426 | YP1001 | Shanghai Yoke Instrument |
| High speed refrigerated centrifuge | GG1206084 | STR16 | Thermo Fisher Scientific |
| Magnetic stirrer | GG1206485 | R05 | Germany IKA |

2.2 Experimental preparation

2.2.1 Rats: SD rats, SPF grade, 6 rats per compound, male, body weight range 180-200 g.

2.2.2 Housing conditions: The animals were housed in an SPF-grade animal facility with free access to food and water, three per cage, under a 12/12-hour light/dark cycle (7:00 am/7:00 pm), at a temperature of 23 ± 3°C.

2.2.3 Drug preparation: The compound was precisely weighed, mixed with the required volume of 1% Tween 80, vortexed for 2 min, and then sonicated for 5 min until no visible particles remained. The mixture was subsequently stirred on a magnetic stirrer for 20 min, and the middle layer of the drug solution was collected for administration. For the IV group, the compound was accurately weighed, dissolved in the required volume of 50% PEG400, sonicated for 10 min, and vortexed until clear before administration.

2.3 Dosing regimen

The rats were randomly divided into 2 groups based on body weight, with 3 rats per compound per group. Group 1 was administered a single oral gavage dose (fasted), and Group 2 was administered a single tail vein bolus injection. Blood collection time points: for the po group, blood was collected at 0.167 h, 0.5 h, 1 h, 2 h, 3 h, 4 h, 6 h, and 8 h; for the iv group, blood was collected at 0.033 h, 0.167 h, 0.5 h, 1 h, 2 h, 4 h, 6 h, and 8 h.

2.4 Sample collection and preparation

After administration to the rats, blood was collected from the orbital venous plexus according to the sampling time points. Approximately 0.2-0.3 mL of blood was collected at each time point into anticoagulant EP tubes (containing 4 μL of EDTA-K2, 375 mg/mL, dried at 50°C). The tubes were slowly inverted up and down three times and stored in an ice box (for no more than 30 minutes). Centrifugation was performed at 4°C at 3000 × g for 10 minutes, and the supernatant was transferred to labeled EP tubes for bioanalytical testing. If testing could not be performed on the same day, the samples were stored at -80°C until analysis (care was taken to avoid multiple freeze/thaw cycles).

2.5 Sample analysis and data analysis

The data were analyzed using WinNonlin via a non-compartmental model to obtain PK parameters ($C_{max}$, $AUC_{0-last}$, $AUC_{inf}$, $T_{1/2}$, *etc.,* selected based on different routes of administration). For specific data, please refer to Table 2.

Table 2. PK experimental results of compounds in SD rats

| Example number | Compound number | Dosage mg/kg | Administration mode | $C_{max}$ (ng/mL) | $T_{1/2}$ (hr) | $AUC_{last}$ (ng/mL * hr) | Oral bioavailability F (%) |
|---|---|---|---|---|---|---|---|
| 18 | 018 | 1<br>1 | PO<br>IV | 920<br>2243 | 4.30<br>- | 1709<br>1159 | 49.1<br>- |
| 23 | 023 | 1<br>1 | PO<br>IV | 335<br>4127 | 2.08<br>- | 616<br>2018 | 30.5<br>- |
| 53 | 053 | 1<br>1 | PO<br>IV | 380<br>3890 | 2.33<br>- | 596<br>1798 | 33.1<br>- |
| 68 | 068 | 1<br>1 | PO<br>IV | 244<br>4213 | 1.47<br>1.43 | 347<br>1697 | 20.5<br>- |
| | 068B | 1<br>1 | PO<br>IV | 291<br>3633 | 1.53<br>0.25 | 298<br>1272 | 23.4<br>- |
| 77 | 077 | 1<br>1 | PO<br>IV | 158<br>2280 | -<br>- | 272<br>683 | 39.8<br>- |
| 79 | 079 | 1<br>1 | PO<br>IV | 566<br>3673 | 2.01<br>- | 499<br>1175 | 42.5<br>- |
| 89 | 089 | 1<br>1 | PO<br>IV | 96.4<br>4303 | 2.61<br>- | 163<br>772 | 21.1<br>- |

(continued)

| Example number | Compound number | Dosage mg/kg | Administration mode | $C_{max}$ (ng/mL) | $T_{1/2}$ (hr) | $AUC_{last}$ (ng/mL * hr) | Oral bioavailability F (%) |
|---|---|---|---|---|---|---|---|
| 93 | 093A | 1<br>1 | PO<br>IV | 271<br>4840 | 1.10<br>0.24 | 295<br>1245 | 23.7<br>- |
| | 093B | 1<br>1 | PO<br>IV | 285<br>1890 | 3.83<br>- | 370<br>716 | 51.6<br>- |
| 94 | 094 | 1<br>1 | PO<br>IV | 208<br>4457 | 2.18<br>0.37 | 276<br>1207 | 22.8<br>- |
| 98 | 098 | 1<br>1 | PO<br>IV | 102<br>2735 | 2.74<br>- | 136<br>630 | 21.6<br>- |
| 109 | 109 | 1<br>1 | PO<br>IV | 264<br>3140 | 2.07<br>0.50 | 485<br>786 | 61.8<br>- |
| 110 | 110 | 1<br>1 | PO<br>IV | 268<br>3783 | 0.96<br>0.29 | 444<br>739 | 60.2<br>- |
| | 110A | 1<br>1 | PO<br>IV | 311<br>1863 | 0.93<br>0.32 | 397<br>580 | 68.6<br>- |
| 125 | 125B | 1<br>1 | PO<br>IV | 822<br>3047 | 1.37<br>- | 2036<br>1495 | 136<br>- |
| 126 | 126 | 1<br>1 | PO<br>IV | 266<br>3900 | -<br>- | 417<br>1553 | 26.9<br>- |
| 128 | 128B | 1<br>1 | PO<br>IV | 182<br>3267 | -<br>- | 334<br>910 | 36.7<br>- |
| 130 | 130 | 1<br>1 | PO<br>IV | 158<br>5527 | 2.79<br>0.22 | 181<br>752 | 24.0<br>- |

Experimental conclusion: The compounds of the present disclosure can achieve higher *in vivo* exposure and higher oral bioavailability at lower doses, and have excellent overall pharmacokinetic properties.

***3. In vitro* liver microsome metabolic stability assay of compounds of the present disclosure**

Experimental method:

3.1 Preparation before experiment

3.1.1 Preparation of standard solution:

Preparation of working solutions for test compounds and standards: An appropriate amount of the compound was accurately weighed and dissolved in a corresponding volume of suitable solvent to prepare 1 mM stock solutions of the test compounds and standards. The stock solutions were then diluted with appropriate solvent to obtain 100 μM working solutions of the test compounds, ensuring that the DMSO content in the working solutions remained below 10%.

3.1.2 Preparation of internal standard working solution:

The internal standard compound testosterone (or tolbutamide) and its corresponding solvent, internal standard concentration (200 ng/mL), were selected based on the properties of the test compound.

3.1.3 Preparation of 0.1 mol/L potassium phosphate buffer (pH 7.4)

Solution A: 0.1 mol/L $K_2HPO_4$ (2.28 g $K_2HPO_4 \cdot 3H_2O$ was dissolved in 100 mL of double-distilled water);
Solution B: 0.1 mol/L $KH_2PO_4$ (1.36 g $KH_2PO_4$ was dissolved in 100 mL of double-distilled water);
80.2 mL of Solution A and 19.8 mL of Solution B were mixed thoroughly, and the pH was adjusted to 7.4.

3.1.4 Preparation of 5 mmol/L glycine buffer

37.5 mg of glycine was weighed, dissolved in 100 mL of ultrapure water, and adjusted to pH 8.0 with NaOH.

3.1.5 Preparation of 500 U/mL glucose-6-phosphate dehydrogenase

1.25 mg of glucose-6-phosphate dehydrogenase (400 U/mg) was weighed (the specific amount weighed was calculated based on the activity of the purchased glucose-6-phosphate dehydrogenase), dissolved in 1 mL of glycine buffer to a concentration of 500 U/mL, aliquoted into 20 μL per tube, and stored at -20°C for future use.

3.1.6 Preparation of cofactor solution

7.83 mg of NADP, 0.06 mL of D-glucose-6-phosphate (260 mg/mL), and 10.17 mg of $MgCl_2 \cdot 6H_2O$ were precisely weighed.

The mixture was dissolved in 1 mL of pure water. The cofactor ratio composition: NADP 10 mM, D-glucose-6-phosphate 60 mM, $MgCl_2 \cdot 6H_2O$ 50 mM.

3.2 Experimental section

3.2.1 Incubation experiment

The reaction system (taking a 500 μL system as an example) was composed of the following solution ratios: 416.5 μL phosphate buffer, 5 μL compound working solution, 25 μL liver microsomes, 3.5 μL glucose-6-phosphate dehydrogenase, and 50 μL cofactor solution. The total incubation volume could be adjusted according to experimental requirements.

416.5 μL of phosphate buffer was taken, 25 μL of liver microsomes, 3.5 μL of glucose-6-phosphate dehydrogenase, and 50 μL of cofactor solution were added, vortexed twice to mix, and preincubated in a 37°C water bath for 5 minutes. Then, 5 μL of the test compound working solution was added, vortexed twice to mix, each time for 2-3 seconds, and placed in a 37°C water bath to initiate the reaction, with two parallel samples set. At 0.5 min, 1 min, 2 min, 5 min, 15 min, and 30 min after the reaction, 50 μL of the mixture was taken and added to centrifuge tubes containing 200 μL of 0°C pre-cooled internal standard working solution to terminate the reaction, vortexed for 1 minute to mix, and centrifuged at 13,000 rpm for 10 minutes in a 4°C pre-cooled high-speed centrifuge. The supernatant was collected for analysis. After the compound was added and vortex-mixed, 50 μL of the mixture was rapidly transferred to a centrifuge tube containing 200 μL of a pre-cooled (0°C) internal standard working solution to terminate the reaction, vortex-mixed for 1 minute, and used as the relative zero-point sample (relative to the standard group). The sample was then placed into a 37°C water bath to initiate the reaction, and timing was started.

3.3 Sample analysis

The sample analysis of the test compound was performed using the liquid chromatography-tandem mass spectrometry (LCMS/MS) method. The semi-quantitative determination was performed using the ratio of the analyte peak area to the internal standard peak area. The retention times of the analyte and internal standard, chromatogram acquisition, and chromatogram integration were processed using Analyst software.

3.4 Data analysis

The *in vitro* elimination rate constant k of the test compound and the control compound was determined by converting the ratio of the peak area of the test compound to the internal standard into the remaining rate, and $T_{1/2}$ was then calculated according to the following formula.

$$T_{1/2} = \frac{Ln2}{k} = \frac{0.693}{k}$$

Table 3. Results of liver microsomal metabolism stability experiments of compounds

| Example number | Compound number | Liver microsome $T_{1-2}$ (min) | | | |
|---|---|---|---|---|---|
| | | Human | Rat | Mouse | Beagle dog |
| 2 | 002 | 281.53 | 85.28 | 1127.82 | 202.32 |
| 3 | 003 | 511.95 | 93.67 | - | - |
| 4 | 004 | 672.88 | 90.26 | 6368.42 | 320.98 |
| 5 | 005 | 431.17 | 101.29 | 1188.6 | 254.48 |
| 9 | 009 | 225.37 | 62.30 | 178.84 | 89.42 |
| 13 | 013 | 345.84 | 54.23 | - | 252.63 |
| 17 | 017 | 1176.24 | 324.15 | 1273.80 | 848.60 |
| 22 | 022 | 271.15 | 116.86 | 1925.01 | 271.86 |
| 62 | 062 | 1048.05 | 801.17 | 228.33 | 1250.74 |
| 63 | 063 | 296.47 | 20.71 | ≥120 | 202.25 |
| 65 | 065 | 197.68 | 39.70 | ≥120 | 200.21 |
| 68 | 068 | 413.13 | 239.35 | - | 381.53 |
| 68 | 068B | 995.19 | 570.62 | 65.23 | 199.34 |
| 70 | 070 | 201.75 | 34.22 | - | 37.22 |
| 71 | 071 | 262.47 | 52.81 | - | 54.52 |
| 78 | 078 | 1014.44 | 53.62 | - | 1869.75 |
| 80 | 080 | 539.06 | 109.92 | - | 1205.26 |
| 81 | 081 | 713.96 | 403.32 | - | 669.81 |
| 82 | 082 | 261.96 | 290.01 | - | 275.16 |
| 83 | 083 | 412.77 | 290.38 | - | 741.46 |
| 85 | 085 | 226.52 | 56.96 | 54.79 | 204.66 |
| 87 | 087A | 511.03 | 120.46 | - | 431.98 |
| 87 | 087B | 369.68 | 571.31 | - | 567.51 |
| 88 | 088 | 423.69 | 39.67 | - | 753.15 |
| 89 | 089 | 331.10 | 88.93 | - | 258.52 |
| 90 | 090 | 209.60 | 50.16 | - | 355.30 |
| 91 | 091 | 717.09 | 72.62 | - | 338.24 |
| 93 | 093A | 359.51 | 110.15 | - | 104.27 |
| 97 | 097 | 724.74 | 230.78 | - | 209.62 |
| 98 | 098 | 208.30 | 195.59 | - | 102.59 |
| 108 | 108 | 458.54 | 124.28 | - | 63.57 |
| 110 | 110 | 324.84 | 97.61 | 1846.01 | 369.92 |
| 110 | 110A | 641.85 | 292.82 | 1597.63 | 358.15 |
| 111 | 111 | 548.28 | 266.64 | - | 982.5 |
| 112 | 112 | - | 560.45 | - | - |
| 113 | 113 | 577.09 | 124.51 | - | 520.59 |
| 114 | 114 | 392.83 | 120.25 | 361.28 | 489.6 |
| 115 | 115 | 247.27 | 114.18 | 1889.54 | 1110.44 |

(continued)

| Example number | Compound number | Liver microsome $T_{1-2}$ (min) | | | |
|---|---|---|---|---|---|
| | | Human | Rat | Mouse | Beagle dog |
| 125 | 125C | 343.09 | 32.18 | 550.74 | 222.01 |
| 125 | 125D | 572.07 | 83.07 | 19686.4 | 220.11 |
| 126 | 126 | 531.62 | 58.22 | ≥120 | 331.52 |
| 128 | 128A | 310.89 | 78.14 | - | 160.31 |
| 128 | 128B | 158.82 | 216.24 | - | 222.45 |
| 130 | 130 | - | 109.49 | - | 2270.89 |

Experimental conclusion: The compounds of the present disclosure are metabolized at a slow rate in human liver microsomes and are relatively stable.

The above is an exemplary description of the embodiment of the technical solution of the present disclosure. It should be understood that the scope of protection of the present disclosure is not limited to the above-described embodiments. Any modifications, equivalent replacements, improvements, *etc.,* made by those skilled in the art within the spirit and principles of the present disclosure shall be included within the scope of protection of the claims of the present disclosure.

## Claims

**1.** A compound represented by formula (I), a racemate thereof, a stereoisomer thereof, a tautomer thereof, an N-oxide thereof, or a pharmaceutically acceptable salt of any of the foregoing:

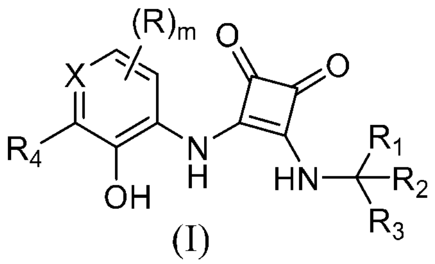

(I)

wherein

$R_1$ is H or $C_{1-6}$ alkyl;
$R_2$ is $C_{4-8}$ cycloalkyl or 5- to 6-membered heterocycloalkyl, wherein the $C_{4-8}$ cycloalkyl and 5- to 6-membered heterocycloalkyl are optionally substituted by 1, 2, 3, or 4 $R_{2-1}$;
each $R_{2-1}$ is the same or different and is independently halogen, deuterium, $C_{1-6}$ alkyl, or $C_{1-6}$ haloalkyl;
$R_3$ is 6- to 9-membered heteroaryl, $-(CR_fR_g)_n$-5- to 9-membered heteroaryl, CN, $C_{3-6}$ alkyl, $C_{2-6}$ alkynyl, $C_{3-6}$ cycloalkyl, $-C(=O)R_a$, $-C(=O)NR_bR_c$, $-NR_dC(=O)R_e$, 4- to 10-membered heterocyclyl, $-(CR_hR_i)_p$-4- to 6-membered heterocycloalkyl, $C_{6-10}$ aryl, or $-(CR_jR_k)_q$-$C_{6-10}$ aryl, wherein the 6- to 9-membered heteroaryl, $-(CR_fR_g)_n$-5- to 9-membered heteroaryl, $C_{3-6}$ alkyl, $C_{3-6}$ cycloalkyl, $-C(=O)R_a$, $-C(=O)NR_bR_c$, $-NR_dC(=O)R_e$, 4- to 10-membered heterocyclyl, $-(CR_hR_i)_p$-4- to 6-membered heterocycloalkyl, $C_{6-10}$ aryl, and $-(CR_jR_k)_q$-$C_{6-10}$ aryl are optionally substituted by 1, 2, 3, or 4 $R_{3-1}$, wherein the $C_{2-6}$ alkynyl is optionally substituted by 1, 2, 3, or 4 $R_{3-2}$;
each $R_{3-1}$ is the same or different and is independently H, CN, OH, oxo (=O), halogen, $C_{3-6}$ cycloalkyl, or $C_{1-6}$ alkyl, wherein the $C_{3-6}$ cycloalkyl and $C_{1-6}$ alkyl are optionally substituted by 1, 2, 3, or 4 substituents independently selected from the group consisting of halogen and deuterium;
each $R_{3-2}$ is the same or different and is independently H, OH, halogen, $C_{1-6}$ alkyl, $C_{3-6}$ cycloalkyl, 4- to 6-membered heterocycloalkyl, or 4- to 9-membered heteroaryl, wherein the $C_{1-6}$ alkyl, $C_{3-6}$ cycloalkyl, 4- to 6-membered heterocycloalkyl, and 4- to 9-membered heteroaryl are optionally substituted by 1, 2, 3, or 4 substituents independently selected from the group consisting of halogen and $C_{1-6}$ alkyl;

$R_a$ is $C_{1-6}$ alkyl or $C_{3-6}$ cycloalkyl;
$R_b$, $R_c$, $R_d$, and $R_e$ are each independently H or $C_{1-6}$ alkyl;
$R_f$, $R_g$, $R_h$, $R_i$, $R_j$, and $R_k$ are each independently H, halogen, or $C_{1-6}$ alkyl;
n, p, and q are each independently 1, 2, or 3;
m is 0, 1, or 2;
each R is the same or different and is independently H, CN, halogen, $C_{1-6}$ haloalkyl, or $C_{1-6}$ alkyl;
X is $CR_5$ or N;
$R_5$ is H, CN, halogen, $C_{1-6}$ haloalkyl, or $C_{1-6}$ alkyl;
$R_4$ is $-C(=O)NR_mR_n$, $-NR_xC(=O)R_y$, $-S(=O)(=NR_v)R_w$, or $-C(=O)R_z$;
$R_m$ and $R_n$ are each independently H, $C_{1-6}$ alkyl, or $C_{1-6}$ haloalkyl, wherein the $C_{1-6}$ alkyl is optionally substituted by 1, 2, or more deuterium atoms;
$R_x$ is H or $C_{1-6}$ alkyl;
$R_y$ is $C_{1-6}$ alkyl;
$R_z$ is $C_{1-6}$ alkyl, $C_{1-6}$ haloalkyl, or 4- to 10-membered heterocycloalkyl, wherein the 4- to 10-membered heterocycloalkyl is optionally substituted by 1, 2, 3, or 4 $R_{z-1}$;
each $R_{z-1}$ is the same or different and is independently halogen, oxo (=O), or $C_{1-6}$ alkyl;
$R_v$ and $R_w$ are each independently H or $C_{1-6}$ alkyl.

**2.** The compound, the racemate thereof, the stereoisomer thereof, the tautomer thereof, the N-oxide thereof, or the pharmaceutically acceptable salt of any of the foregoing according to claim 1, wherein $R_1$ is H or methyl;

preferably, $R_2$ is $C_{4-6}$ cycloalkyl or 5- to 6-membered heterocycloalkyl, wherein the $C_{4-6}$ cycloalkyl and 5- to 6-membered heterocycloalkyl are optionally substituted by 1, 2, 3, or 4 substituents selected from the group consisting of halogen, deuterium, and $C_{1-3}$ alkyl; more preferably, the $C_{4-6}$ cycloalkyl is $C_{5-6}$ cycloalkyl; preferably, $R_2$ is cyclobutyl, cyclopentyl, spiro[2.2]pentyl, bicyclo[1.1.1]pentyl, bicyclo[3.1.0]hexyl, spiro[2.3]hexyl, bicyclo[2.1.1]hexyl, cyclohexyl, or tetrahydrofuranyl, wherein the cyclobutyl, cyclopentyl, spiro[2.2]pentyl, bicyclo[1.1.1]pentyl, bicyclo[3.1.0]hexyl, spiro[2.3]hexyl, bicyclo[2.1.1]hexyl, cyclohexyl, and tetrahydrofuranyl are optionally substituted by 1, 2, 3, or 4 substituents selected from the group consisting of F, Cl, deuterium, $-CH_2F$, $-CHF_2$, $-CF_3$, and methyl;
preferably, $R_2$ is

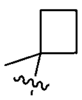, 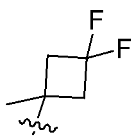, 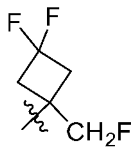, 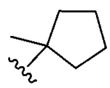, 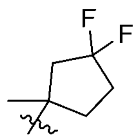, 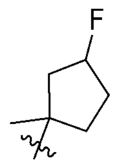,

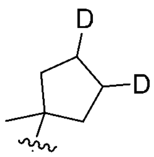, 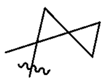, 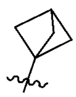, 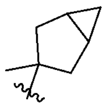, 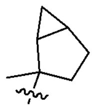, 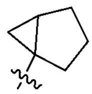, 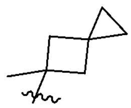, 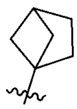, 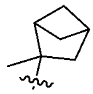,

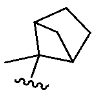, 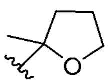

(such as

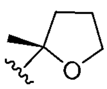 or 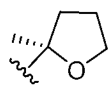), 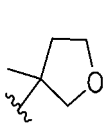, 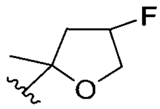, or 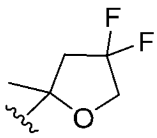

(such as

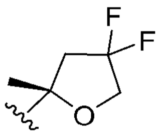 or 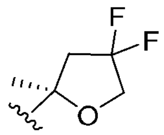

).

**3.** The compound, the racemate thereof, the stereoisomer thereof, the tautomer thereof, the N-oxide thereof, or the pharmaceutically acceptable salt of any of the foregoing according to claim 1 or 2, wherein $R_3$ is 6-membered heteroaryl, $-(CR_fR_g)_n$-5- to 6-membered heteroaryl, CN, $C_{3-6}$ alkyl, $C_{2-4}$ alkynyl, $C_{3-5}$ cycloalkyl, $-C(=O)R_a$, $-C(=O)NR_bR_c$, $-NR_dC(=O)R_e$, 4- to 8-membered heterocyclyl, - $(CR_hR_i)_p$-4- to 6-membered heterocycloalkyl, phenyl, or $-(CR_jR_k)_q$-phenyl, wherein the 6-membered heteroaryl, $-(CR_fR_g)_n$-5- to 6-membered heteroaryl, $C_{3-6}$ alkyl, $C_{3-5}$ cycloalkyl, $-C(=O)R_a$, - $C(=O)NR_bR_c$, $-NR_dC(=O)R_e$, 4- to 8-membered heterocyclyl, $-(CR_hR_i)_p$-4- to 6-membered heterocycloalkyl, phenyl, and $-(CR_jR_k)_q$-phenyl are optionally substituted by 1, 2, 3, or 4 $R_{3-1}$, wherein the $C_{2-4}$ alkynyl is optionally substituted by 1, 2, 3, or 4 $R_{3-2}$;

preferably, $R_a$ is methyl, ethyl, *n*-propyl, isopropyl, *n*-butyl, isobutyl, *sec*-butyl, *tert*-butyl, cyclopropyl, or cyclobutyl;
preferably, $R_b$, $R_c$, $R_d$, and $R_e$ are each independently **H,** methyl, ethyl, *n*-propyl, isopropyl, *n*-butyl, isobutyl, *sec*-butyl, or *tert*-butyl;
preferably, $R_f$, $R_g$, $R_h$, $R_i$, $R_j$, and $R_k$ are each independently **H,** F, or methyl;
preferably, $R_3$ is pyridinyl, pyrimidinyl, pyrazinyl, pyridazinyl, $-CH(CH_3)$-pyridinyl, $-CH(CH_3)$-oxazolyl, CN, n-propyl, isopropyl, n-butyl, tert-butyl, ethynyl, 1-propynyl, 2-propynyl, 2-butynyl, 3-butynyl, cyclopropyl, cyclobutyl, $-C(=O)C_{1-4}$ alkyl, $-C(=O)C_{3-4}$ cycloalkyl, $-C(=O)N(C_{1-3}$ alkyl)($C_{1-4}$ alkyl), $-NHC(=O)C_{1-3}$ alkyl, oxetanyl, azetidinyl, pyrrolidinyl, tetrahydrofuranyl,

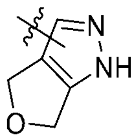,

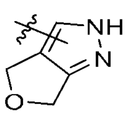, 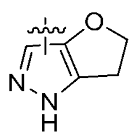, 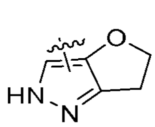, 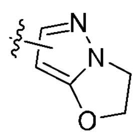, 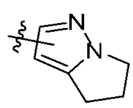, 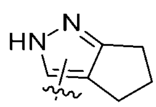, 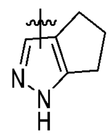,

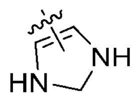,

$-CH(CH_3)$-azetidinyl, phenyl, or $-CH_2$-phenyl, wherein the pyridinyl, pyrimidinyl, pyrazinyl, pyridazinyl, $-CH(CH_3)$-pyridinyl, $-CH(CH_3)$-oxazolyl, n-propyl, isopropyl, n-butyl, *tert*-butyl, cyclopropyl, cyclobutyl, $-C(=O)C_{1-4}$ alkyl, $-C(=O)C_{3-4}$ cycloalkyl, $-C(=O)N(C_{1-3}$ alkyl)($C_{1-4}$ alkyl), $-NHC(=O)C_{1-3}$ alkyl, oxetanyl, azetidinyl, pyrrolidinyl, tetrahydrofuranyl,

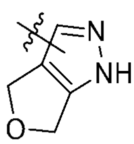,

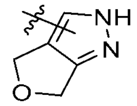, 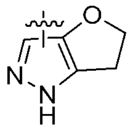, 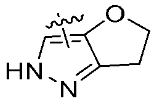, 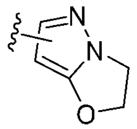, 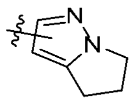, 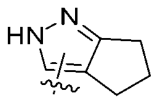, 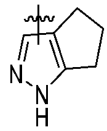,

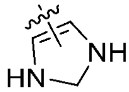,

-CH($CH_3$)-azetidinyl, phenyl, and -$CH_2$-phenyl are optionally substituted by 1, 2, 3, or 4 $R_{3-1}$, wherein the ethynyl, 1-propynyl, 2-propynyl, 2-butynyl, and 3-butynyl are optionally substituted by 1, 2, 3, or 4 $R_{3-2}$;

preferably, each $R_{3-1}$ is the same or different and is independently H, CN, OH, oxo (=O), F, Cl, Br, methyl, ethyl, cyclopropyl, -$CH_2$F, -$CHF_2$, -$CF_3$, or -$CD_3$;

preferably, each $R_{3-2}$ is the same or different and is independently $C_{3-5}$ cycloalkyl, 4- to 5-membered heterocycloalkyl, or 5- to 6-membered heteroaryl, wherein the $C_{3-5}$ cycloalkyl, 4- to 5-membered heterocycloalkyl, and 5- to 6-membered heteroaryl are optionally substituted by 1, 2, 3, or 4 substituents independently selected from the group consisting of halogen and $C_{1-3}$ alkyl;

preferably, each $R_{3-2}$ is the same or different and is independently cyclopropyl, cyclobutyl, oxetanyl, azetidinyl, pyridyl, pyrazolyl, or bicyclo[1.1.1]pentyl, wherein the cyclopropyl, cyclobutyl, oxetanyl, azetidinyl, pyridyl, pyrazolyl, and bicyclo[1.1.1]pentyl are optionally substituted by 1, 2, 3, or 4 substituents independently selected from the group consisting of F and methyl;

preferably, each $R_{3-2}$ is the same or different and is independently

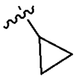, 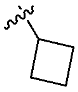, 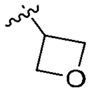,

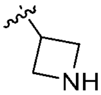, 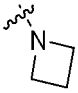, 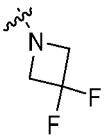, 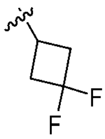, 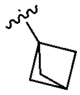, 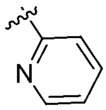, or 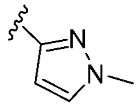;

preferably, $R_3$ is

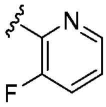, 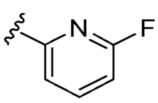, 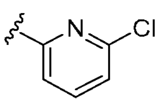, 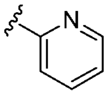, 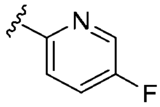, 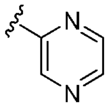,

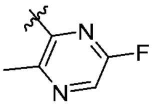, 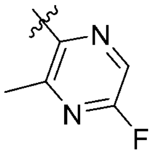, 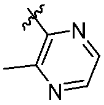, 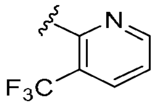, 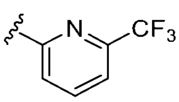, 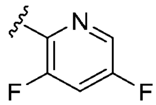,

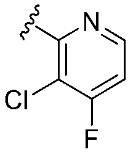, 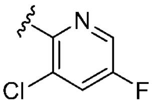, 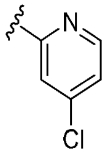, 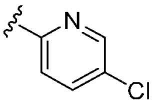, 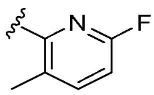, 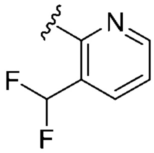, 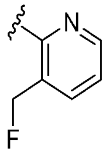,

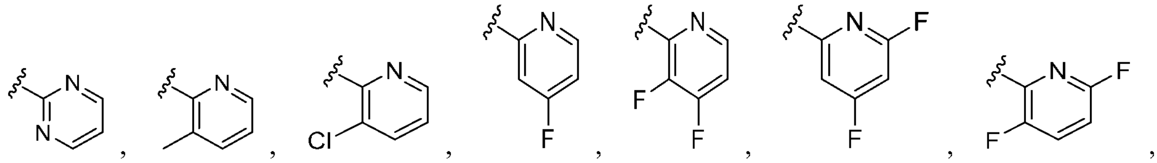

, , , , , , ,

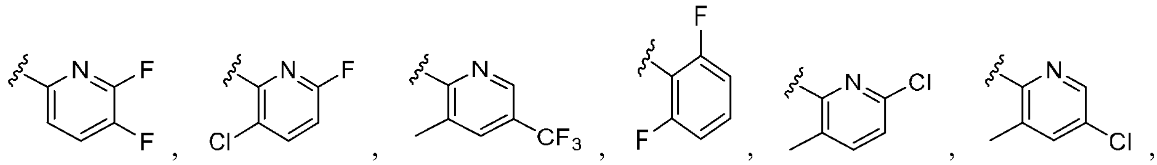

, , , , , , ,

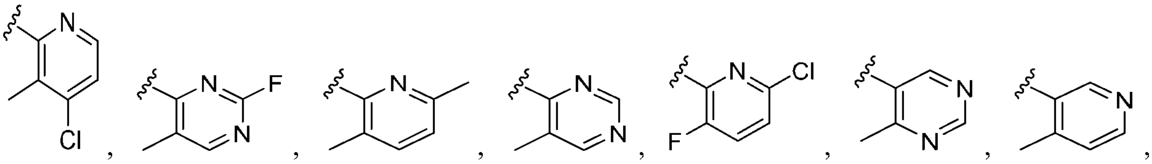

, , , , , , ,

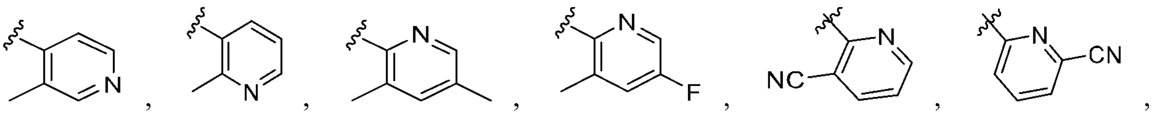

, , , , , ,

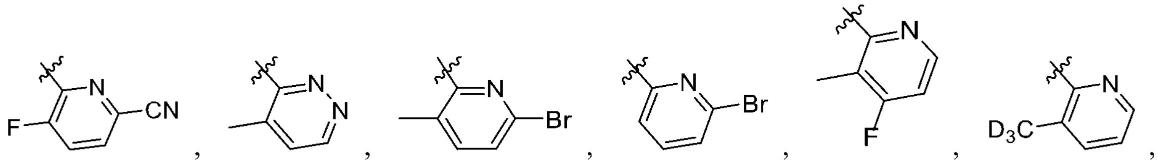

, , , , , ,

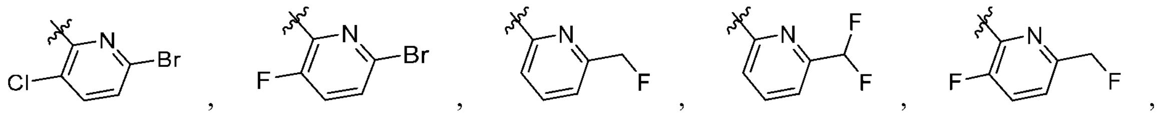

, , , , ,

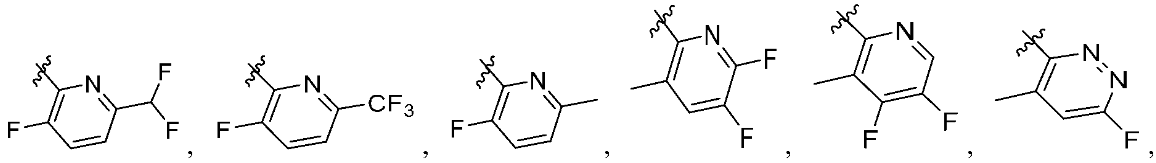

, , , , , ,

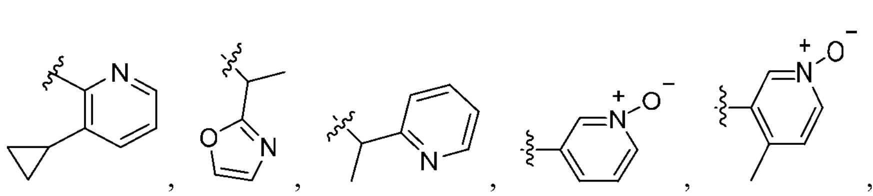

, , , , ,

CN, isopropyl, *tert*-butyl, cyclopropyl,

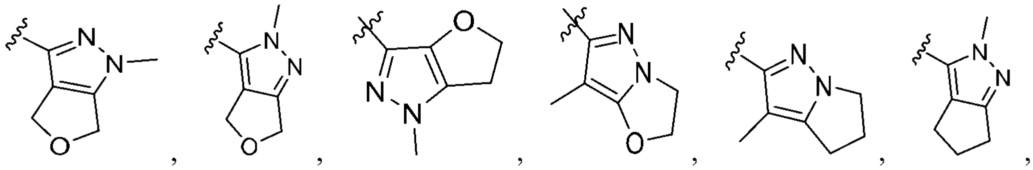

, , , , , ,

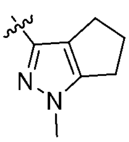 , 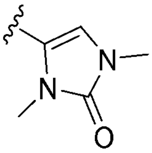 , 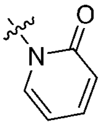 , 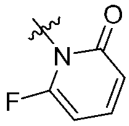 , 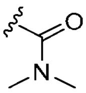 , 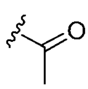 , 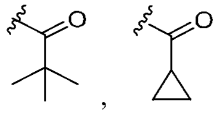 ,

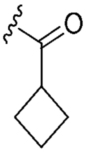 , 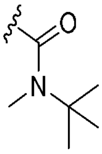 , 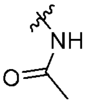 , 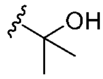 , 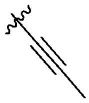 , 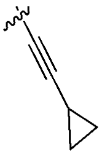 , 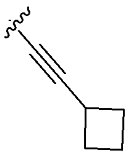 , 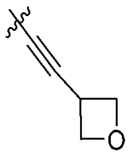 ,

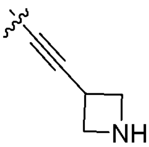 , 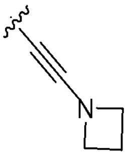 , 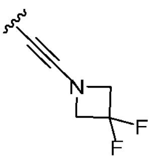 , 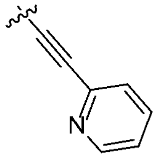 , 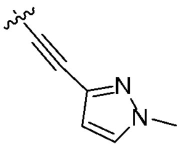 , 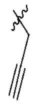 , 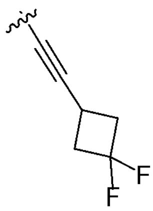 ,

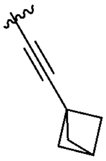 , 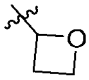 , 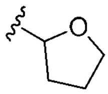 , 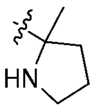 , 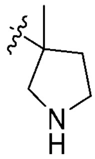 , 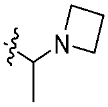 , 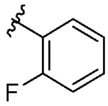 ,

phenyl, or benzyl;
preferably, the

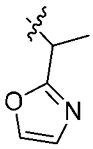 , 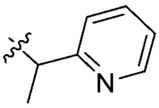 , 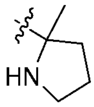 , 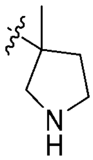 , or 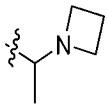 is 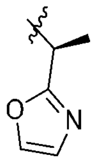 ,

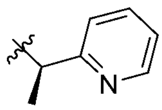 , 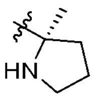 , 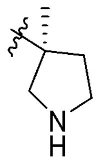 , or 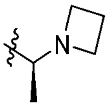 .

**4.** The compound, the racemate thereof, the stereoisomer thereof, the tautomer thereof, the N-oxide thereof, or the pharmaceutically acceptable salt of any of the foregoing according to any one of claims 1 to 3, wherein $R_m$ and $R_n$ are each independently H, methyl, ethyl, $CD_3$, *n*-propyl, isopropyl, - $CH_2CH_2F$, $-CH_2CHF_2$, or $-CH_2CF_3$;

preferably, $R_x$ is H or methyl; $R_y$ is methyl or ethyl;
preferably, $R_v$ is H, methyl, or ethyl; $R_w$ is methyl or ethyl;
preferably, $R_z$ is $C_{1-3}$ alkyl, $C_{1-3}$ haloalkyl, or 5- to 8-membered heterocycloalkyl, wherein the 5-to 8-membered heterocycloalkyl is optionally substituted by 1, 2, 3, or 4 $R_{z-1}$; more preferably, the 5- to 8-membered heterocycloalkyl contains 1, 2, or 3 N and 0, 1, or 2 O or S;
preferably, $R_z$ is

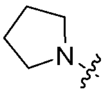, 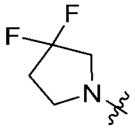, 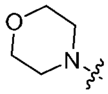, 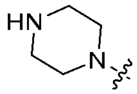, 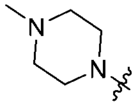, 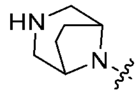,

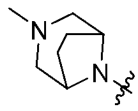, 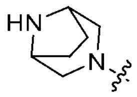, or 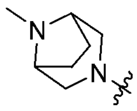;

preferably, each $R_{z-1}$ is the same or different and is independently F, Cl, oxo (=O), methyl, or ethyl;
preferably, $R_4$ is

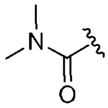, 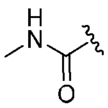, 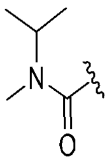, 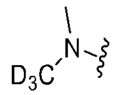, 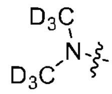, 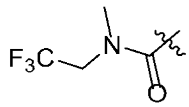,

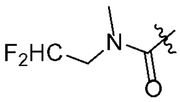, 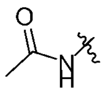, 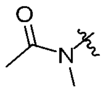, 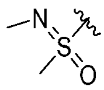, 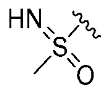, 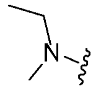, 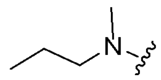,

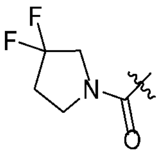, 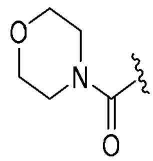, 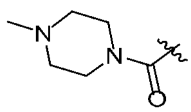, 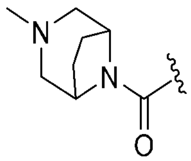, or 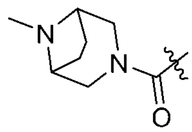;

preferably, m is 0 or 1;
preferably, each R is the same or different and is independently H, CN, F, Cl, $-CH_3$, or $-CH_2F$;
preferably, $R_5$ is H, CN, F, Cl, $-CH_3$, or $-CH_2F$;
preferably, X is CH, C(CN), CF, C(Cl), $C(CH_3)$, $C(CH_2F)$, or N.

**5.** The compound, the racemate thereof, the stereoisomer thereof, the tautomer thereof, the N-oxide thereof, or the pharmaceutically acceptable salt of any of the foregoing according to any one of claims 1 to 4, wherein the compound represented by formula (I) has a structure represented by the following formula (I-a) or (I-b):

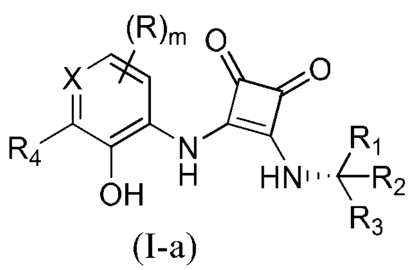
(I-a)
or
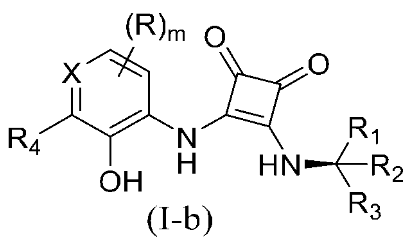
(I-b)
,

wherein X, m, R, $R_1$, $R_2$, $R_3$, and $R_4$ each independently have the definition as described in any one of claims 1 to 4;
preferably, the compound represented by formula (I) has a structure represented by any one of the following formulas (I-1) to (I-3):

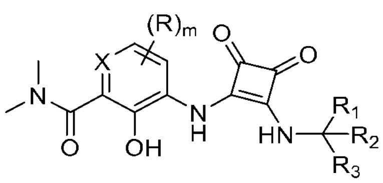
(I-1) ,
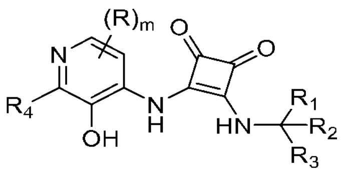
(I-2) , or
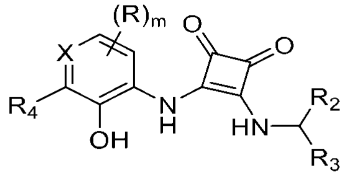
(I-3) ,

wherein X, m, R, $R_1$, $R_2$, $R_3$, and $R_4$ each independently have the definition as described in any one of claims 1 to 4;

preferably, the compound represented by formula (I) has a structure represented by any one of the following formulas (II-1) to (II-3) or formula (II):

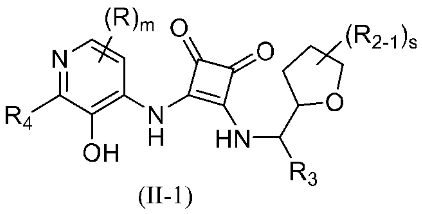
(II-1) ,
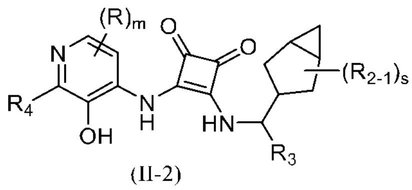
(II-2) ,

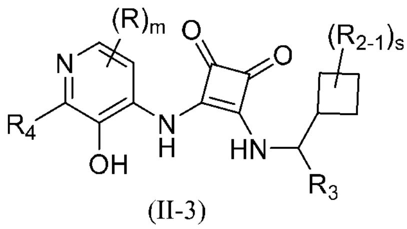
(II-3) , or
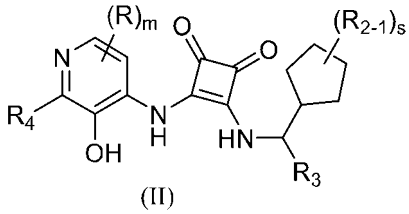
(II) ,

wherein m, R, $R_{2-1}$, $R_3$, and $R_4$ each independently have the definition as described in any one of claims 1 to 4, and s is 0, 1, 2, or 3;

preferably, the compound represented by formula (I) has a structure represented by any one of the following formulas (II-1a) to (II-3b) or formulas (II-a) to (II-b):

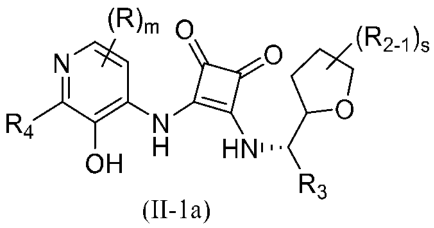
(II-1a) ,
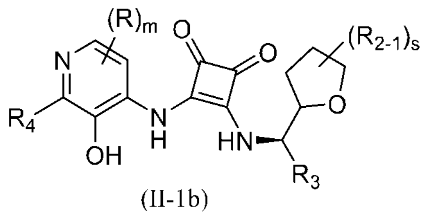
(II-1b) ,

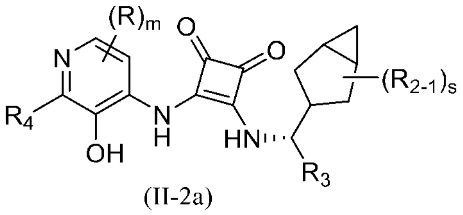
(II-2a) ,
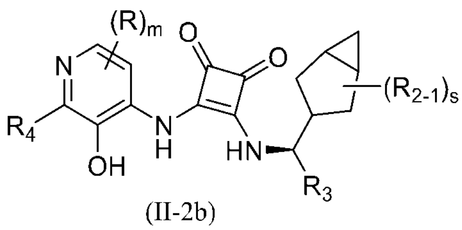
(II-2b) ,

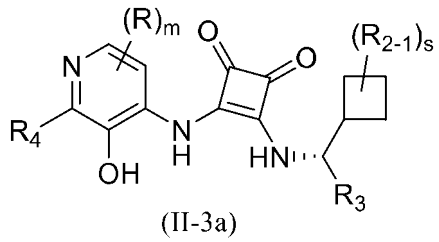
(II-3a) ,

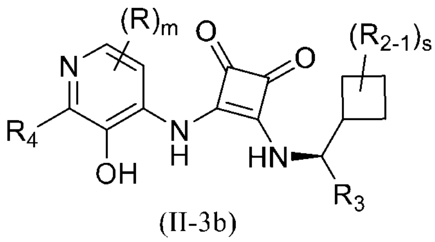
(II-3b) ,

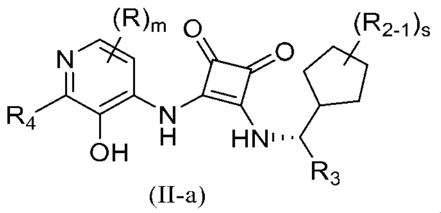
(II-a) , or

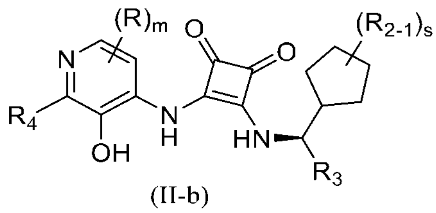
(II-b) ,

wherein m, R, $R_{2-1}$, $R_3$, and $R_4$ each independently have the definition as described in any one of claims 1 to 4, and s is 0, 1, 2, or 3;

preferably, the compound represented by formula (I) has a structure represented by any one of the following formulas (III-1) to (III-3) or formula (III):

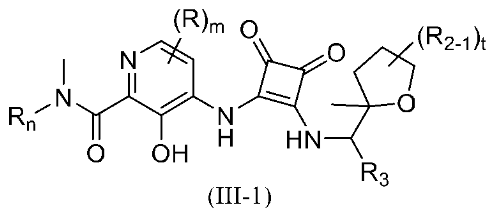
(III-1) ,

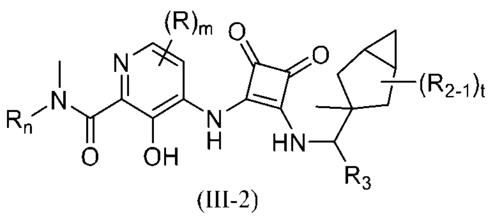
(III-2) ,

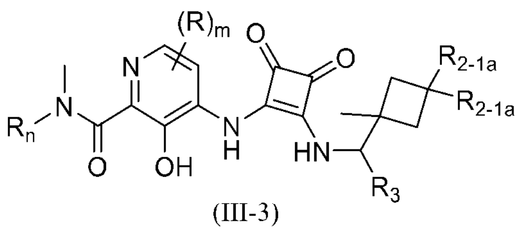
(III-3) , or

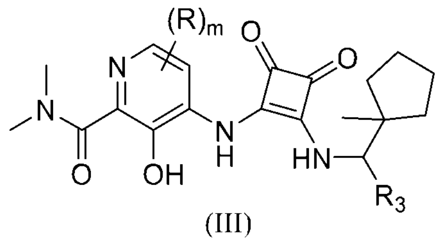
(III) ,

wherein t is 0, 1, or 2; each $R_{2-1a}$ is the same or different and is independently $R_{2-1}$, or two $R_{2-1a}$ together with the carbon atom to which they are attached form a cyclopropyl or a cyclobutyl; m, $R_{2-1}$, $R_n$, R, and $R_3$ each independently have the definition as described in any one of claims 1 to 4;

preferably, the compound represented by formula (I) has a structure represented by the following formulas (III-1a) to (III-3b) or formulas (III-a) to (III-b):

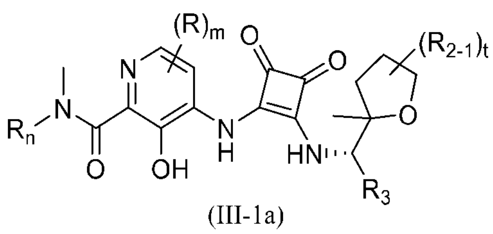
(III-1a) ,

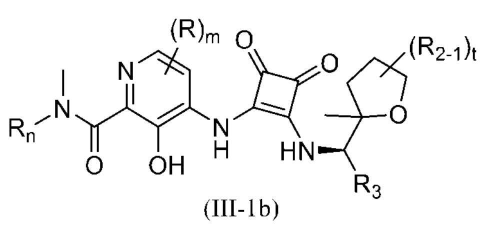
(III-1b) ,

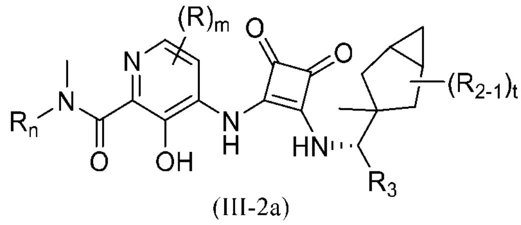
(III-2a) ,

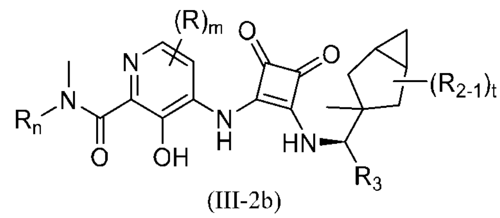
(III-2b) ,

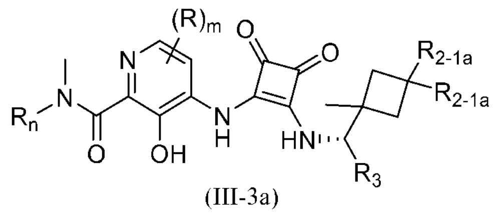
(III-3a) ,

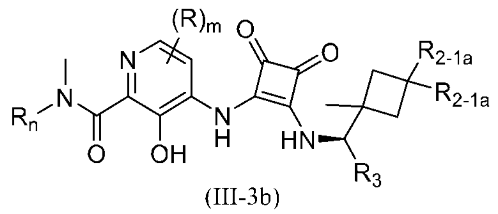
(III-3b) ,

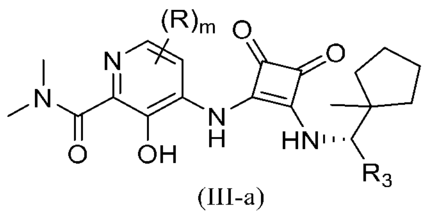
(III-a) , or

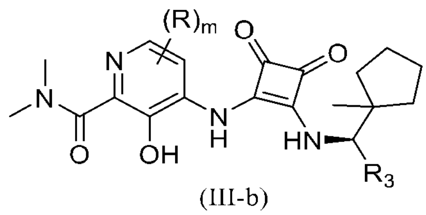
(III-b) ;

wherein t is 0, 1, or 2; each $R_{2-1a}$ is the same or different and is independently $R_{2-1}$, or two $R_{2-1a}$ together with the carbon atom to which they are attached form a cyclopropyl or a cyclobutyl; m, $R_{2-1}$, $R_n$, R, and $R_3$ each independently have the definition as described in any one of claims 1 to 4.

**6.** The compound, the racemate thereof, the stereoisomer thereof, the tautomer thereof, the N-oxide thereof, or the pharmaceutically acceptable salt of any of the foregoing according to any one of claims 1 to 5, wherein the compound represented by formula (I) has a structure as shown below:

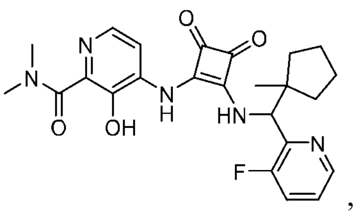
,
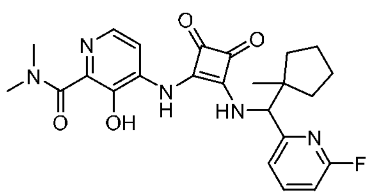
,
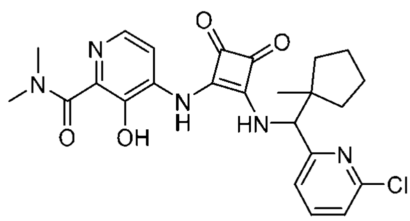
,
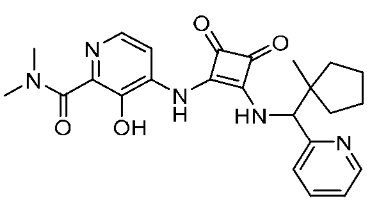
,
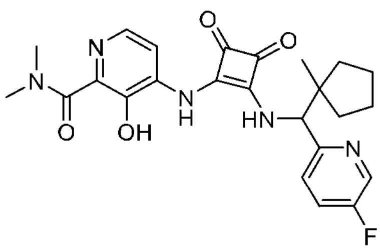
,
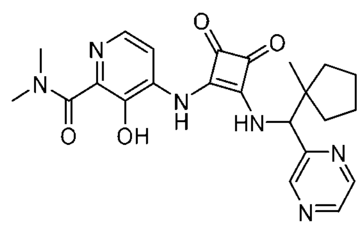
,

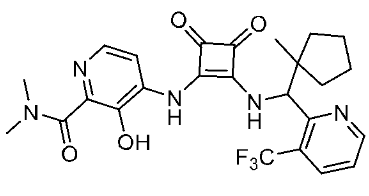,
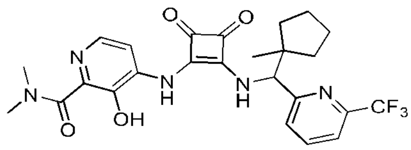,
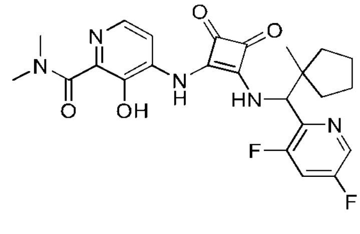,

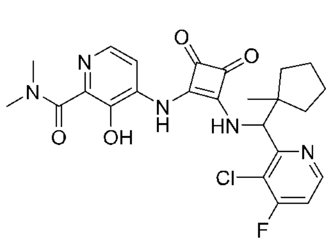,
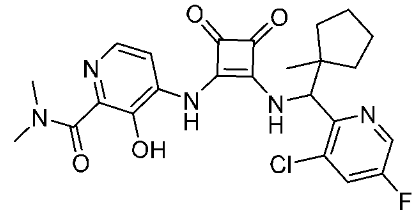,
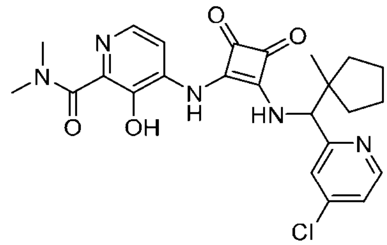,

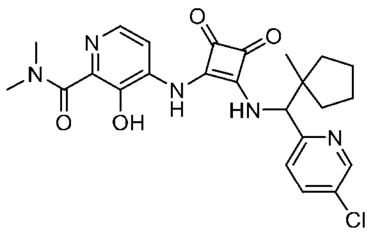,
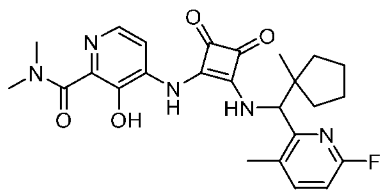,
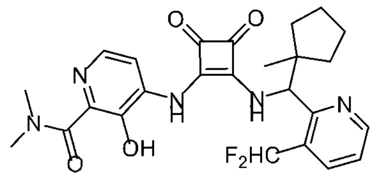,

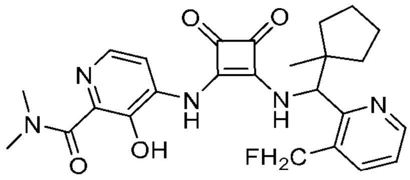,
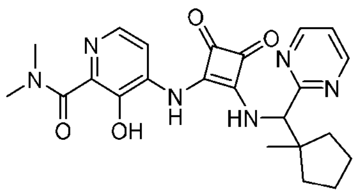,
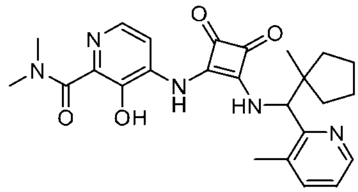,

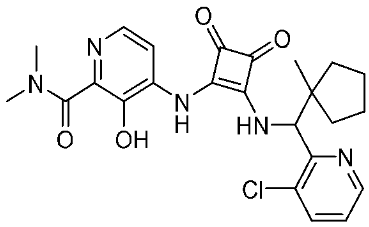,
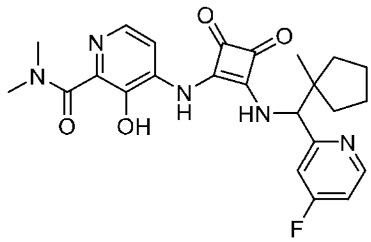,
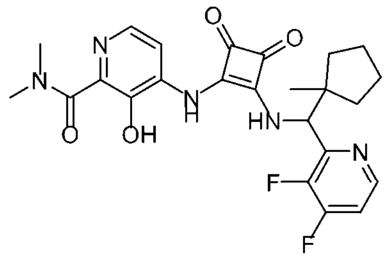,

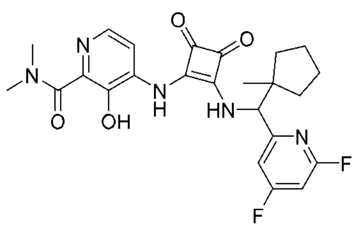,
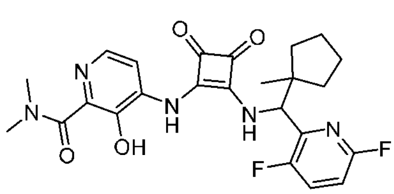,
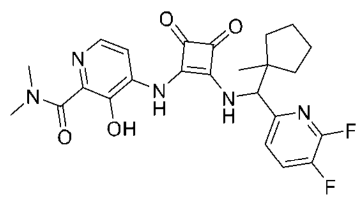,

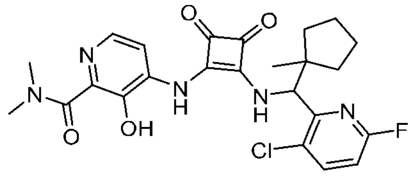,
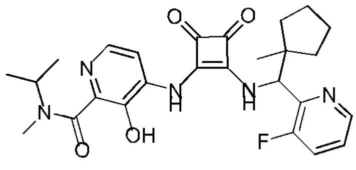,
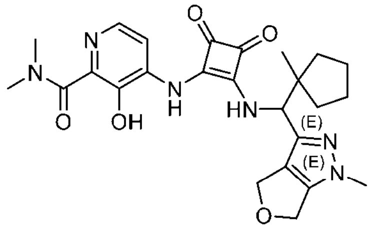,
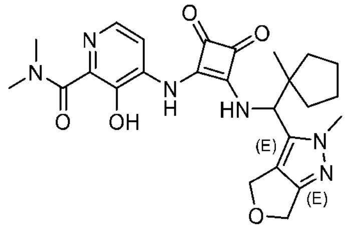,
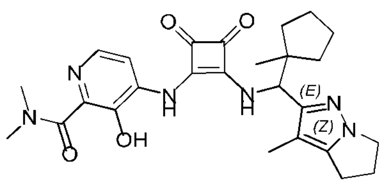,
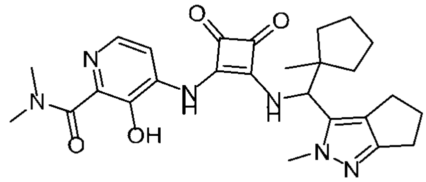,
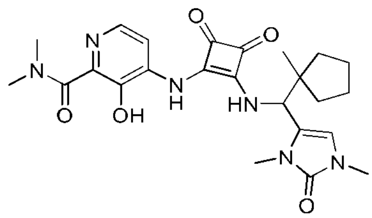,
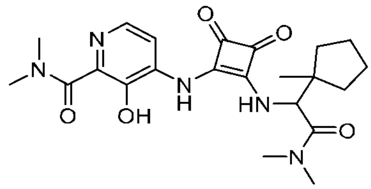,
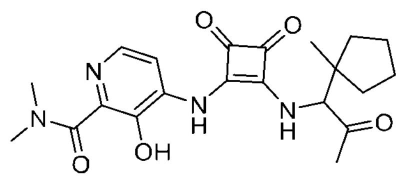,
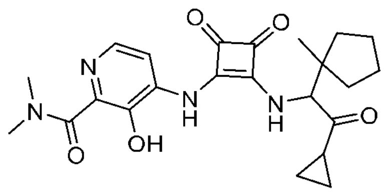,
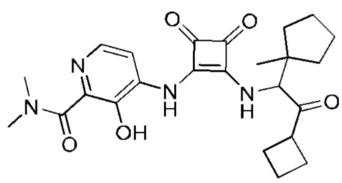,
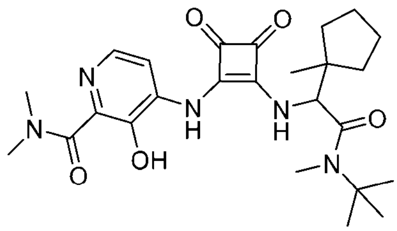,
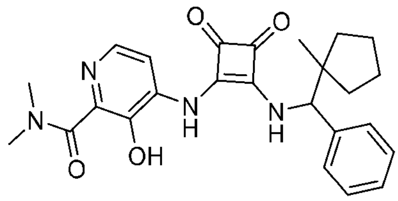,
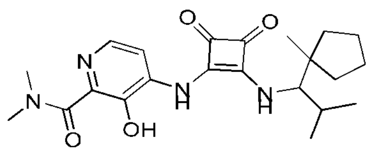,
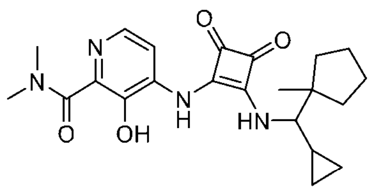,
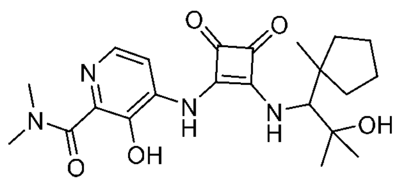,
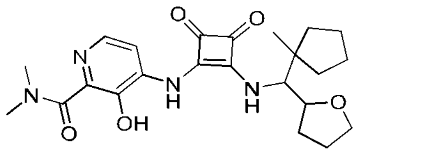,

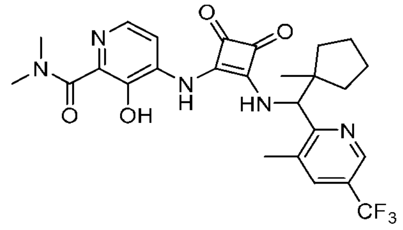,
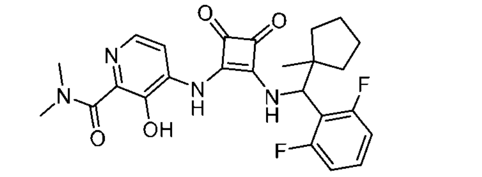,

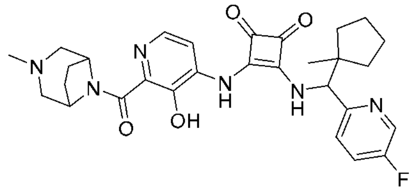,
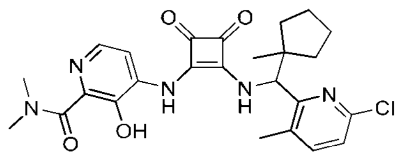,

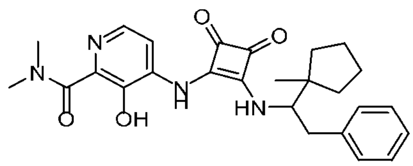,
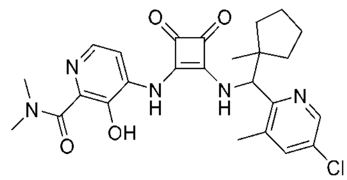,
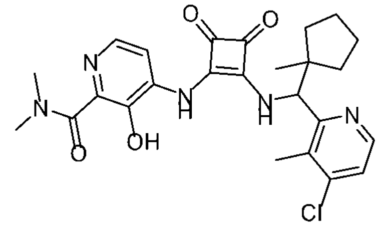,

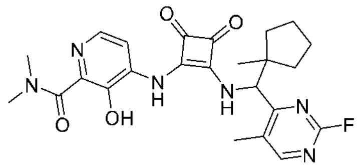,
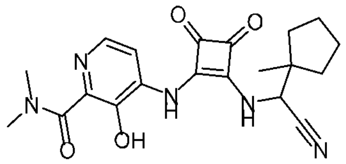,
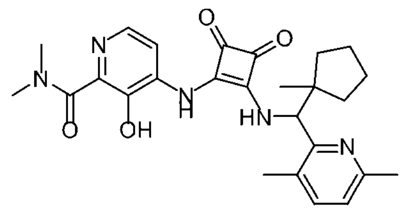,

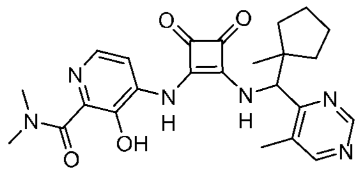,
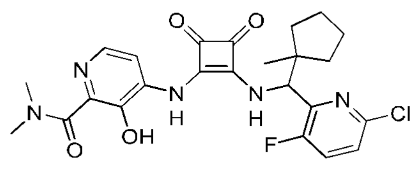,
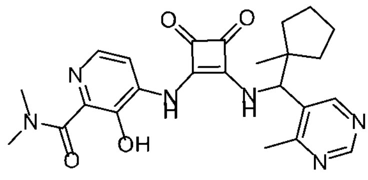,

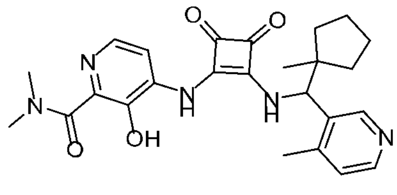,
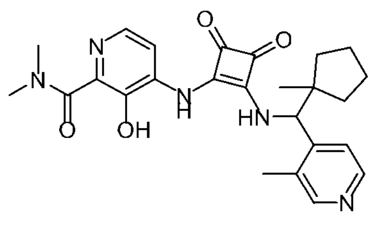,
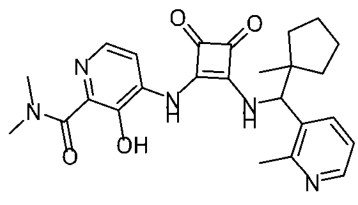,

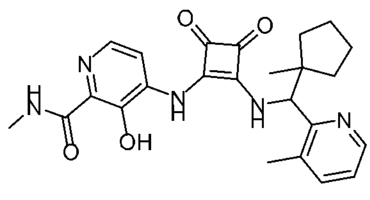,
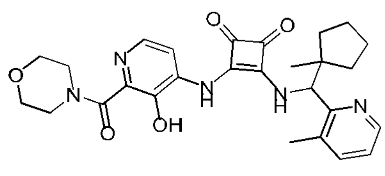,
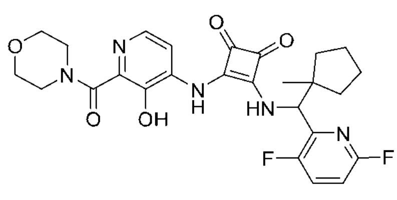,

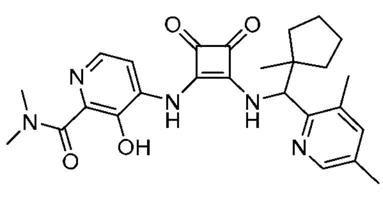,
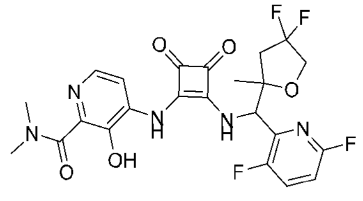,
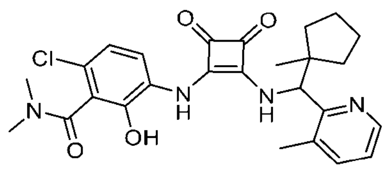,

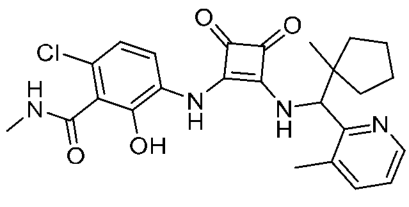,
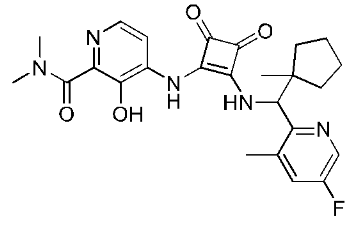,
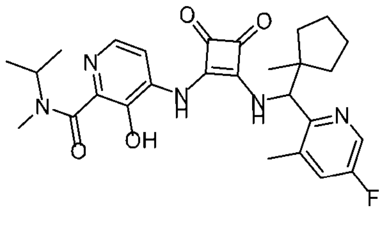,

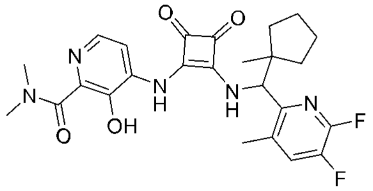,
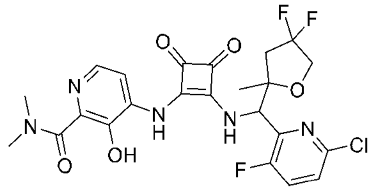,
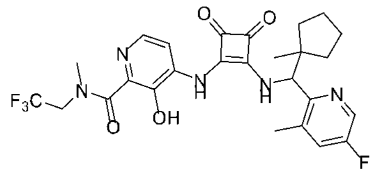,

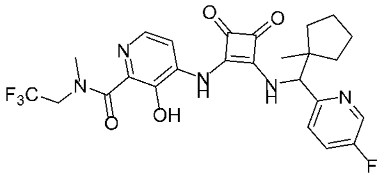,
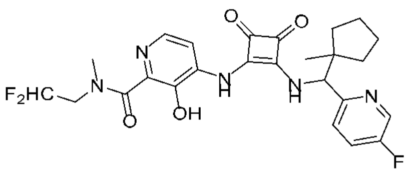,
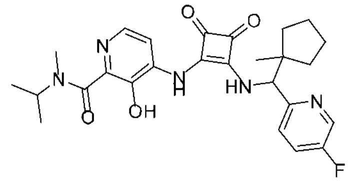,

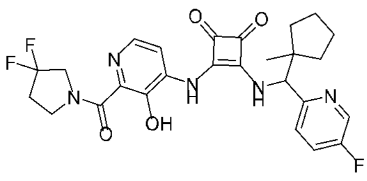,
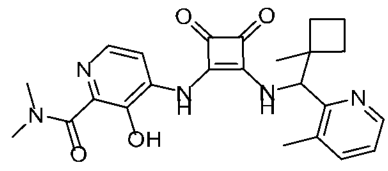,
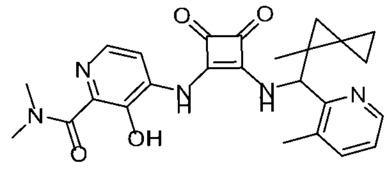,

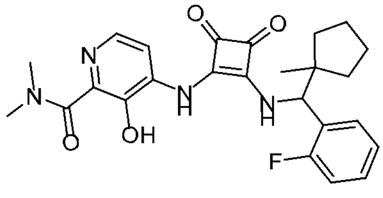,
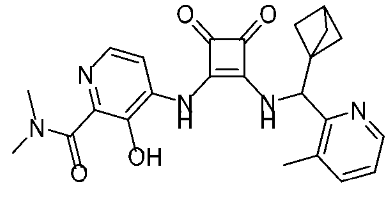,
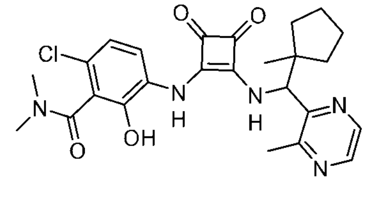,

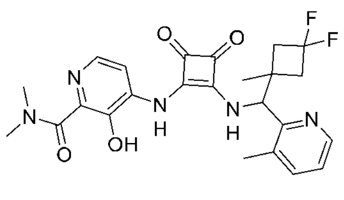,
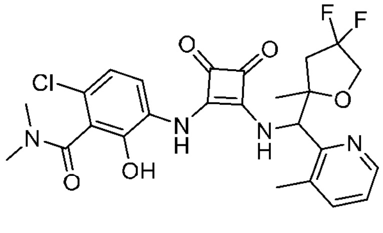,
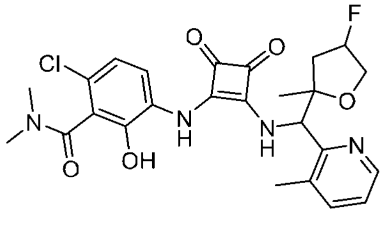,

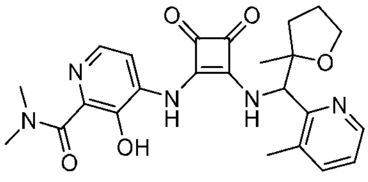,
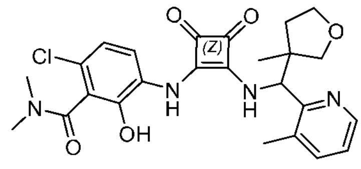,
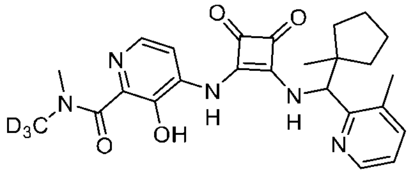,

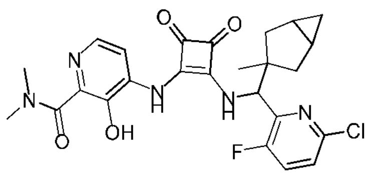,
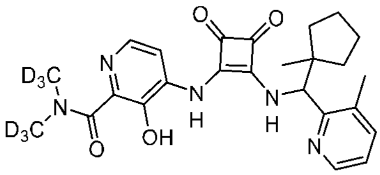,
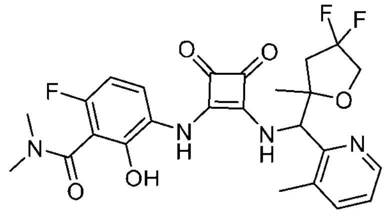,

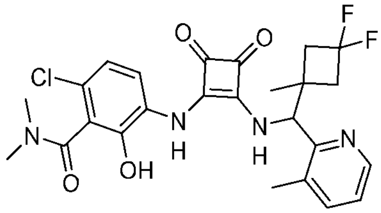,
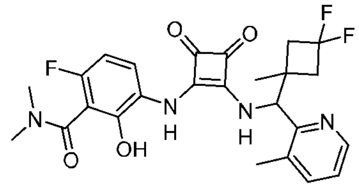,
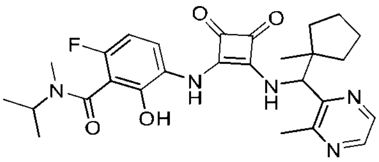,

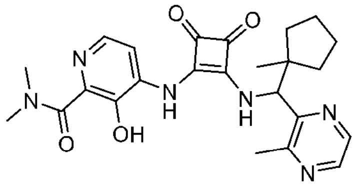,
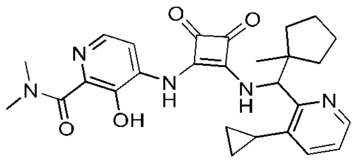,
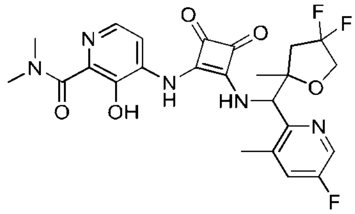,

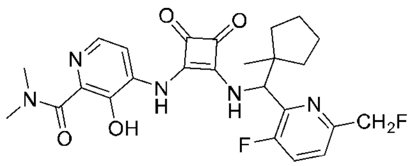,
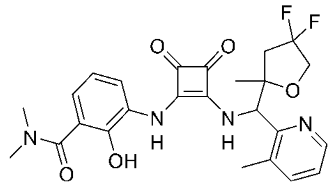,
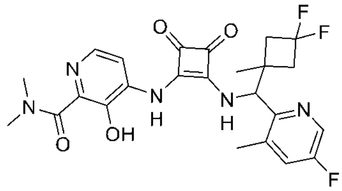,

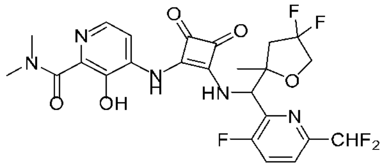,
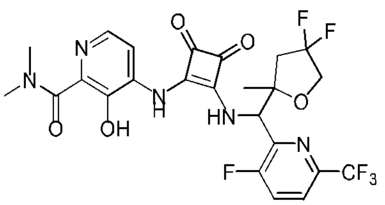,
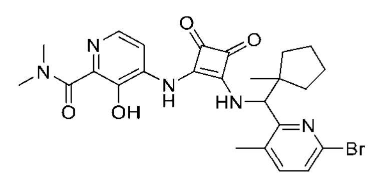,

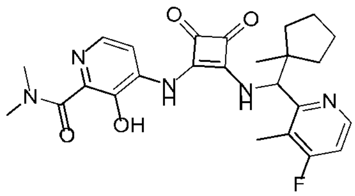,
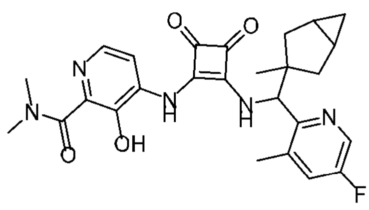,
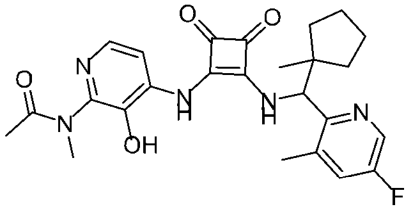,

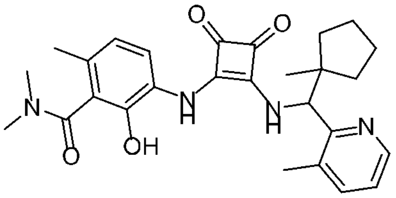,
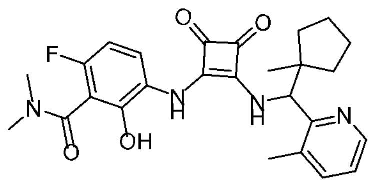,
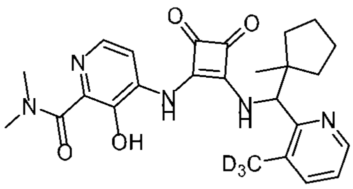,

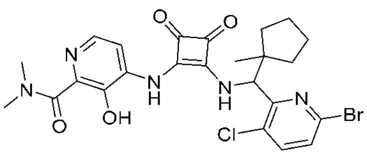,
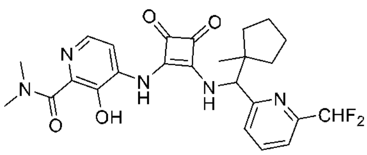,
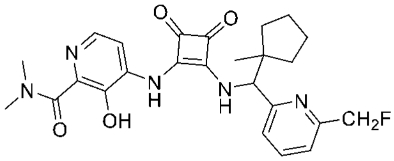,

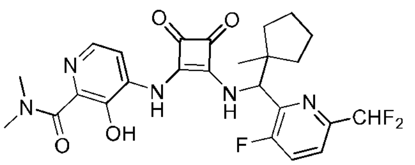,
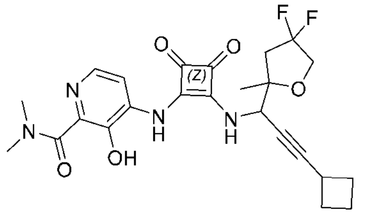,
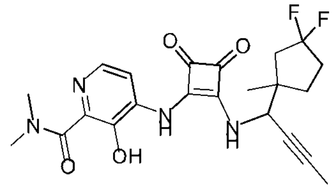,

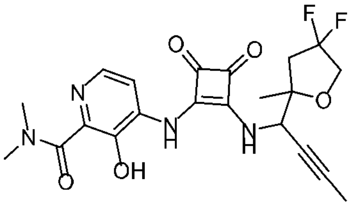,
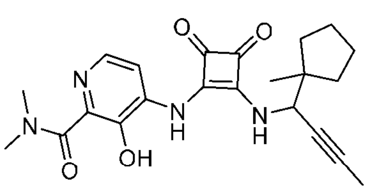,
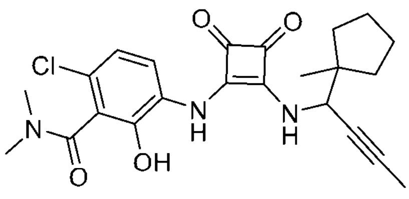,

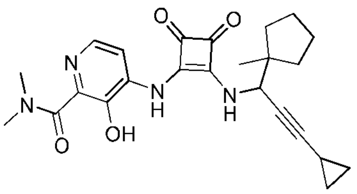,
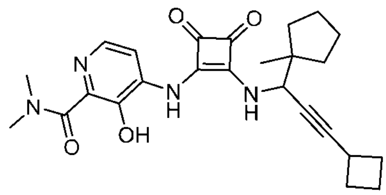,

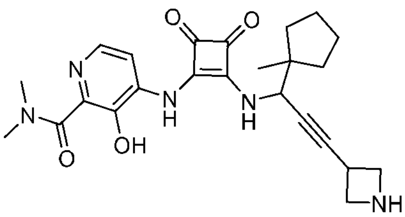,
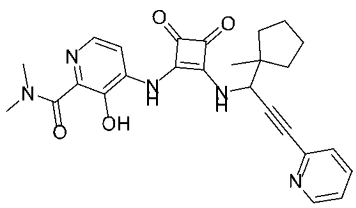,

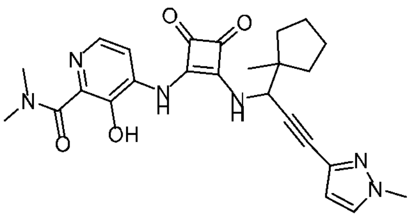,
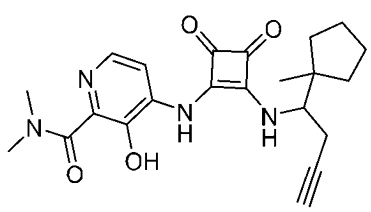,

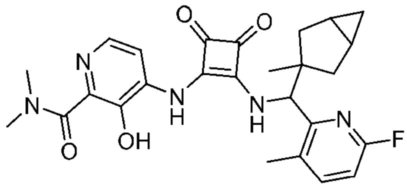,
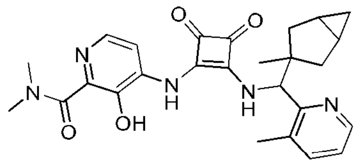,
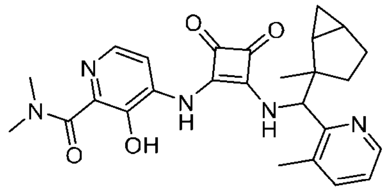,
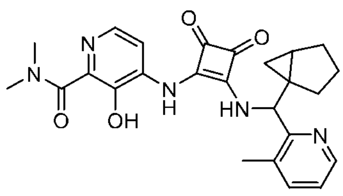,
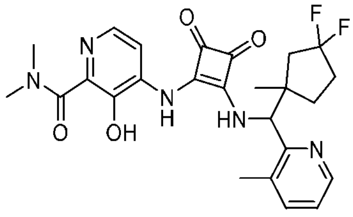,
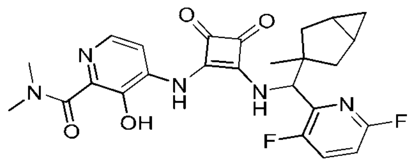,
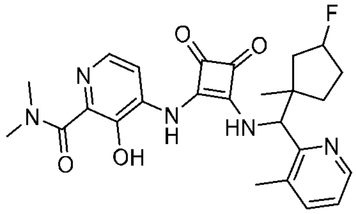,
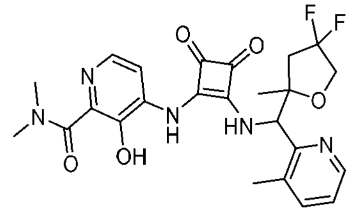,
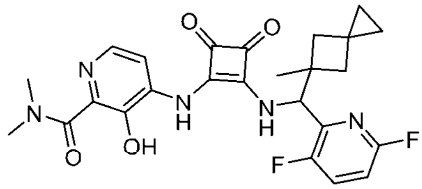,
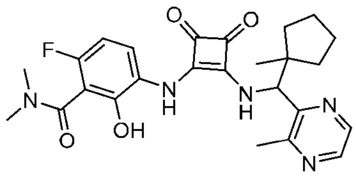,
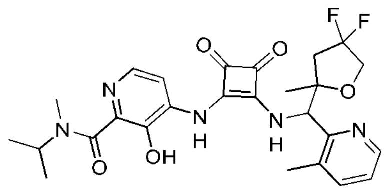,
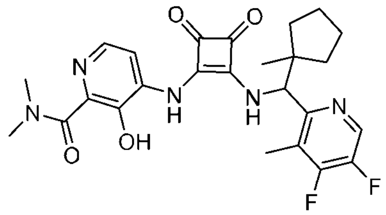,
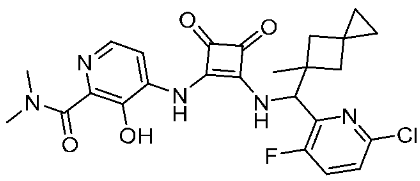,
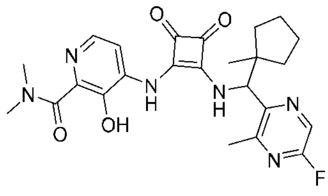,
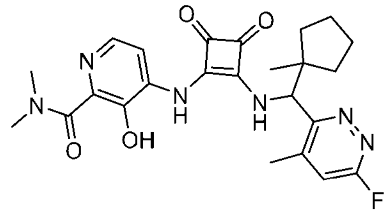,
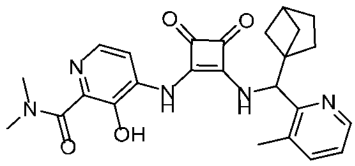,
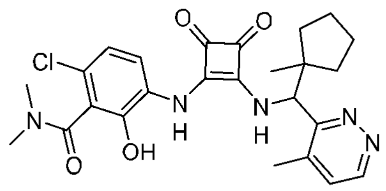,
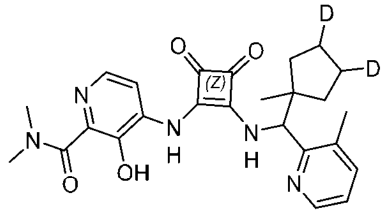,
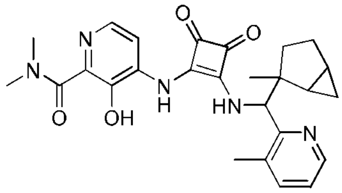,
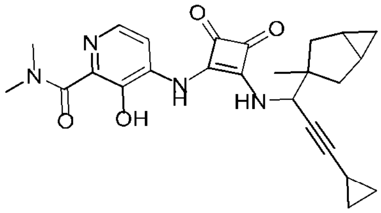,
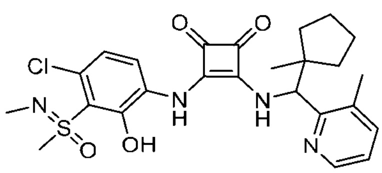,

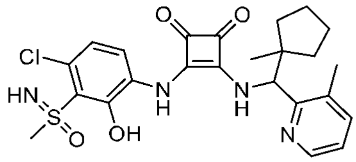, 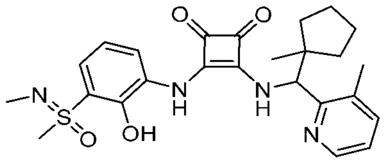, 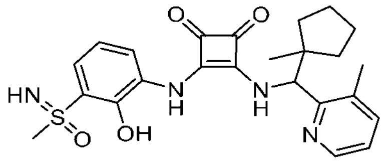,

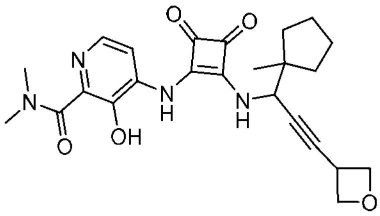, 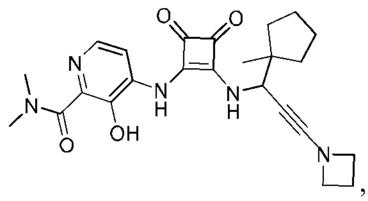, 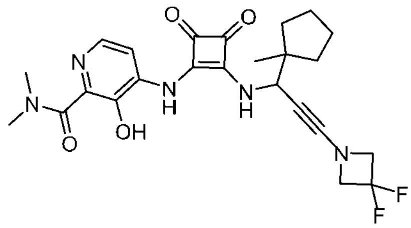,

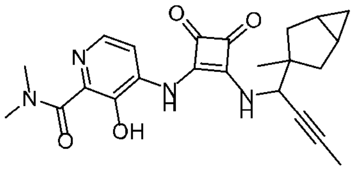, 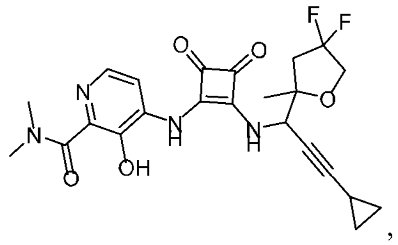, 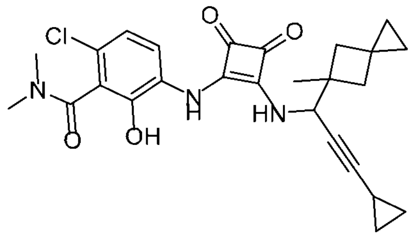,

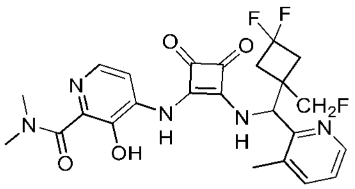, 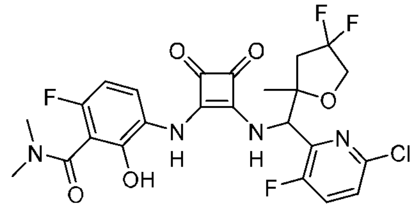, 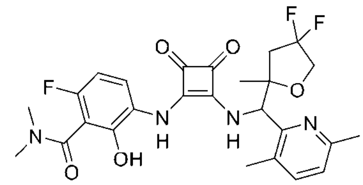,

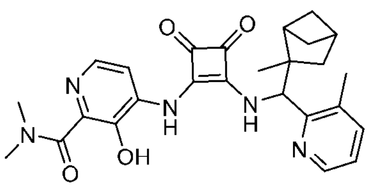, 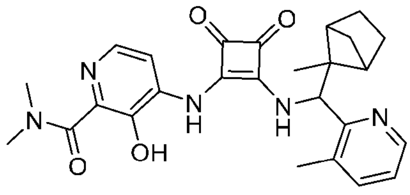, 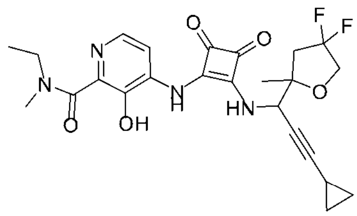,

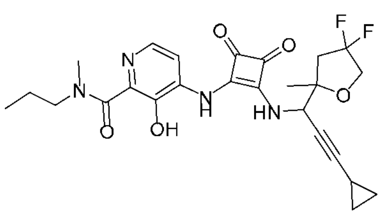, 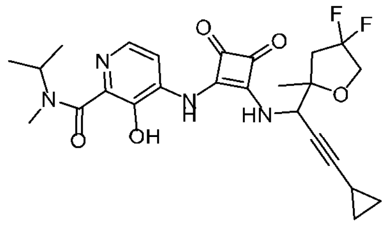, 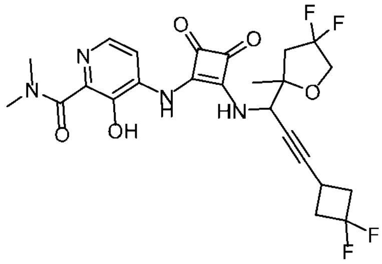,

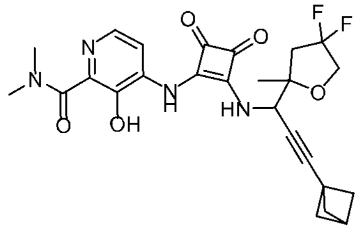,
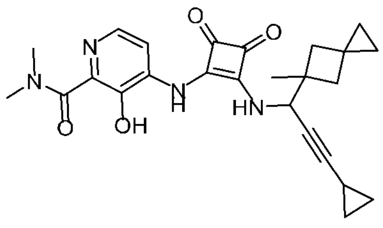,
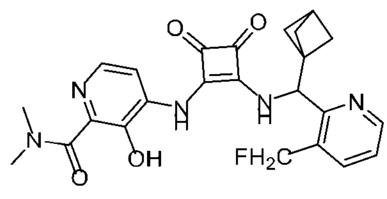,

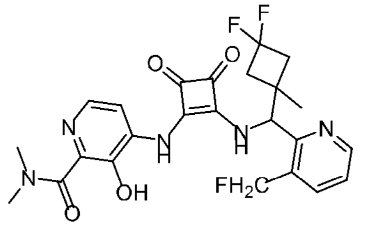,
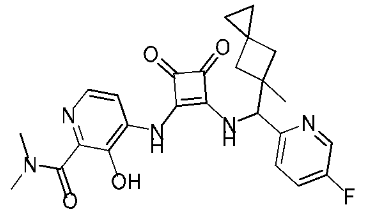,
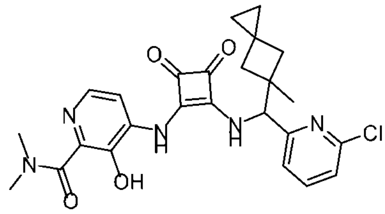,

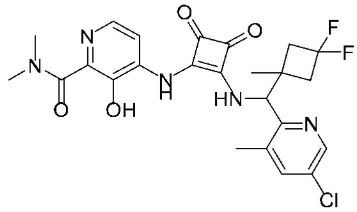,
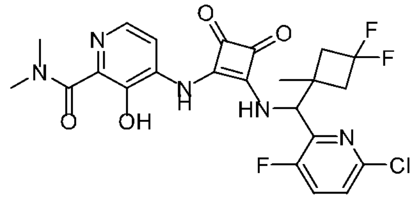,

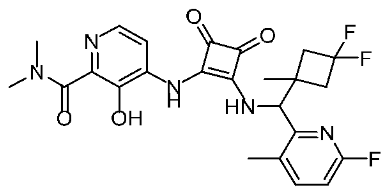,
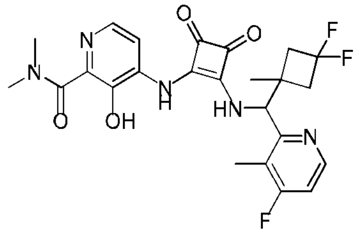,
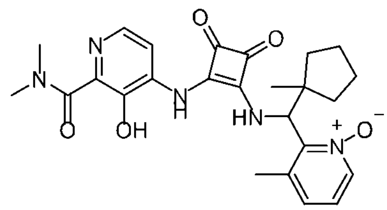,

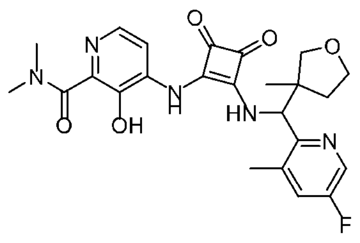,
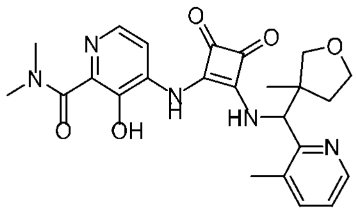,
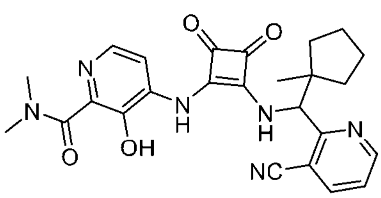,

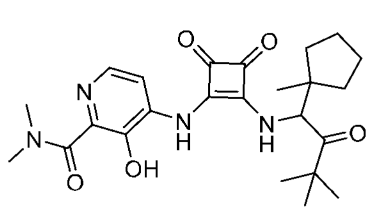,
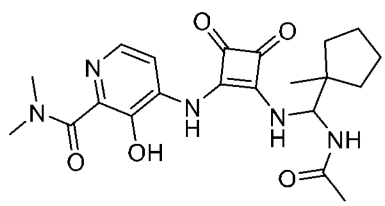,
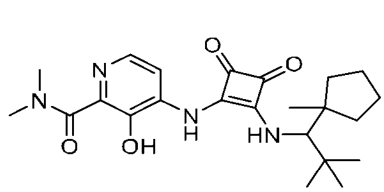,

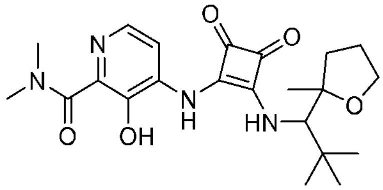,
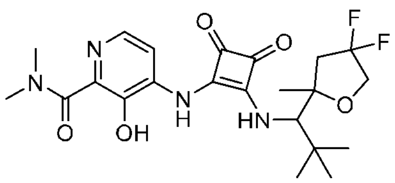,
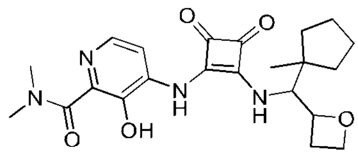,

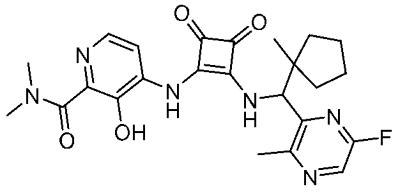,
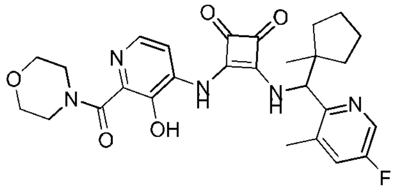,
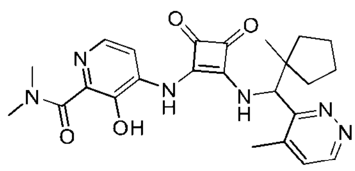,

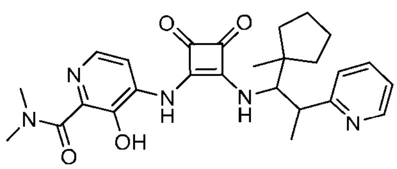,
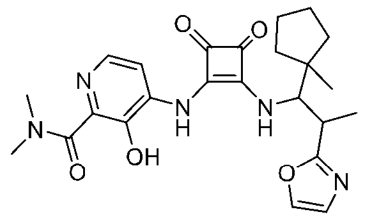,
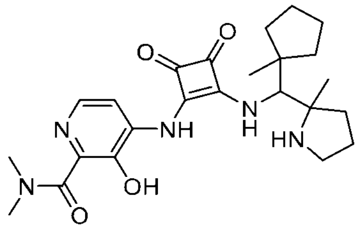,

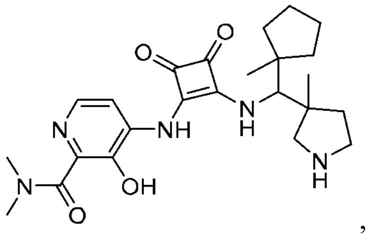,
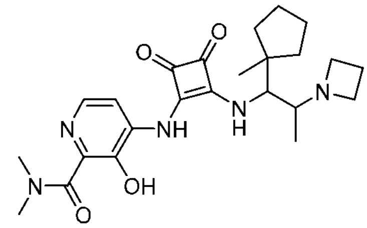,
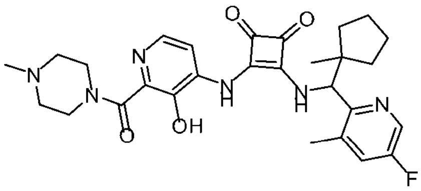,

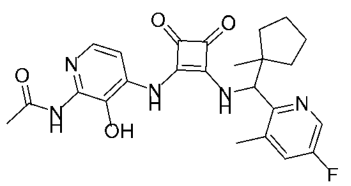,
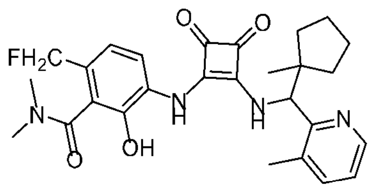,
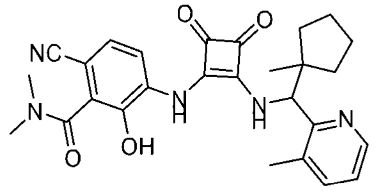,

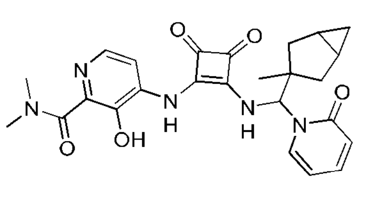,
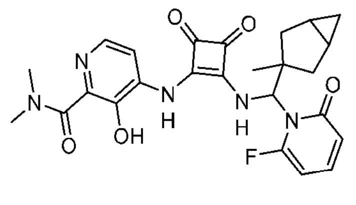,
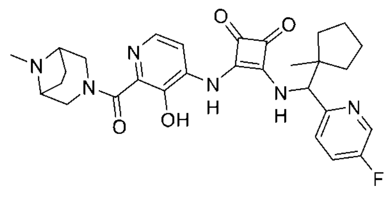,

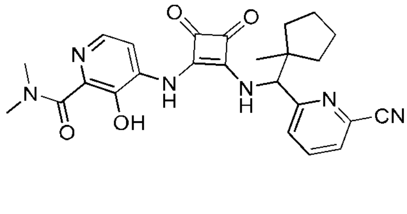,
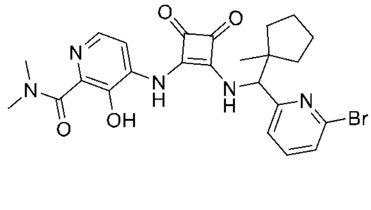,
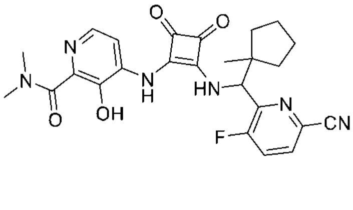,

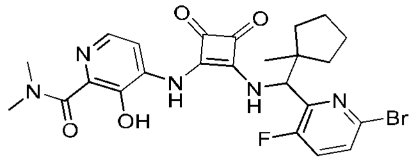, 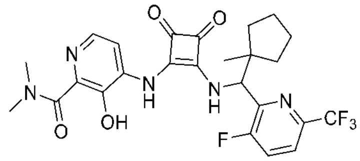, 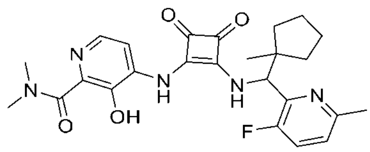,

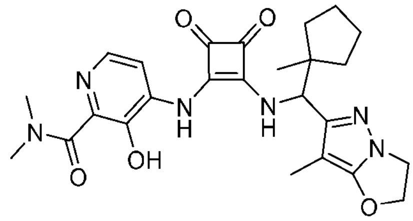, 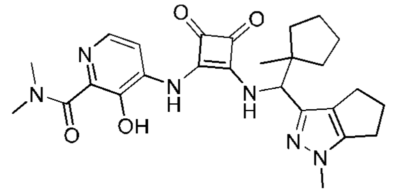,

or

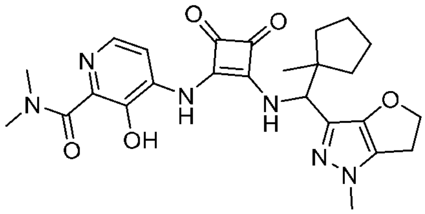;

preferably, the compound represented by formula (I) has a structure as shown below:

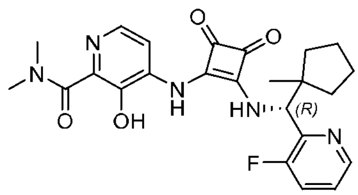, 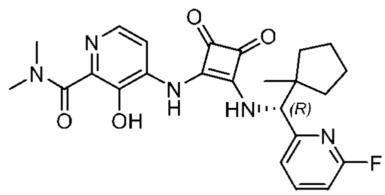,

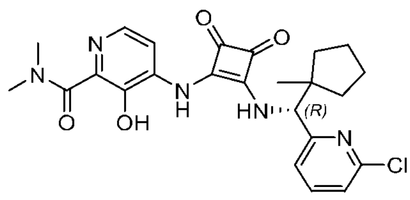, 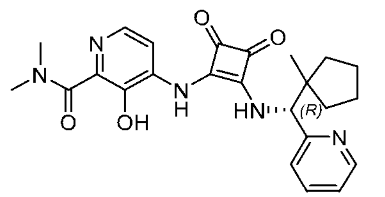,

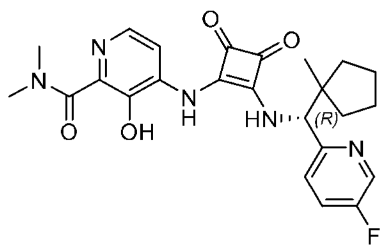, 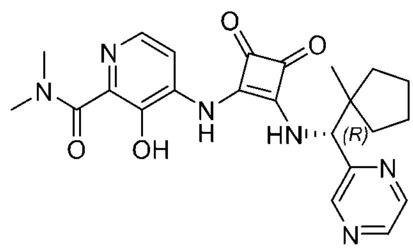,

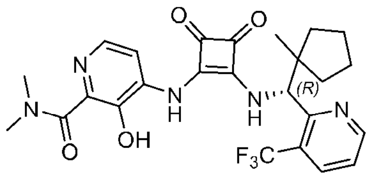,
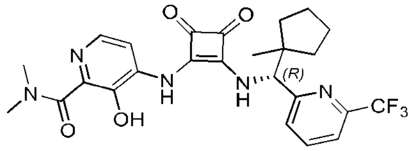,
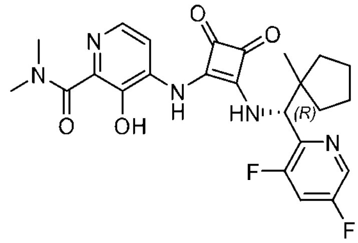,

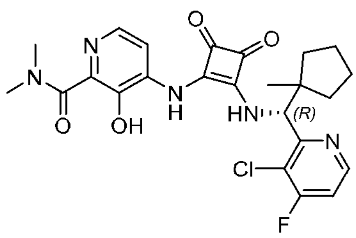,
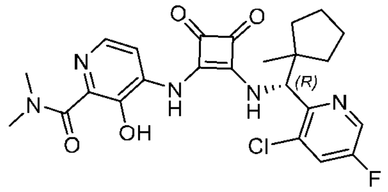,
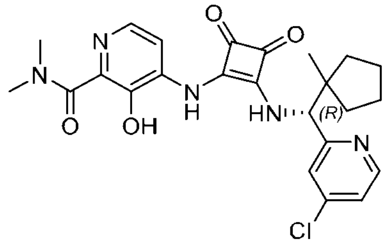,

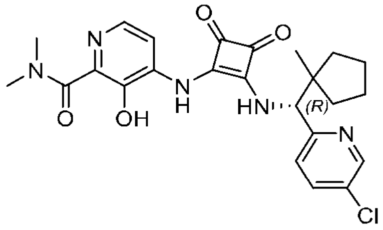,
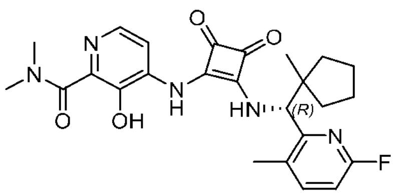,
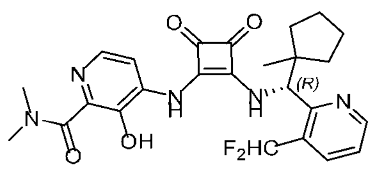,

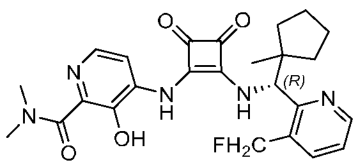,
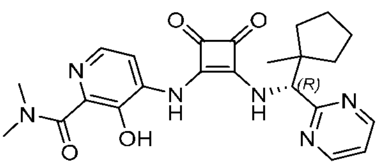,
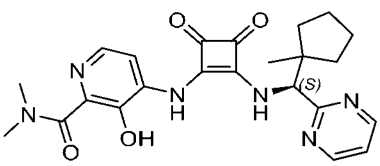,

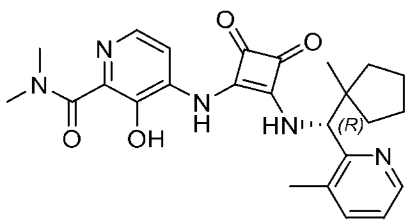,
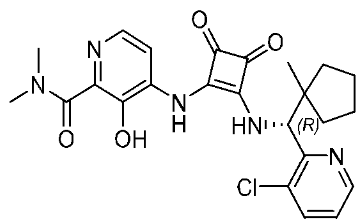,
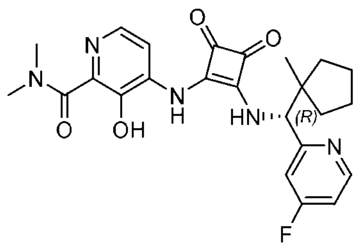,

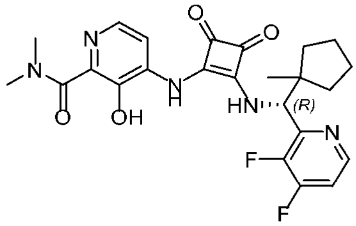,
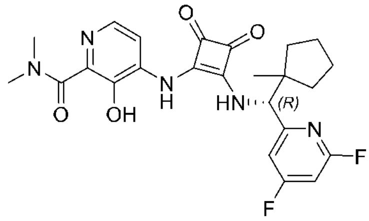,
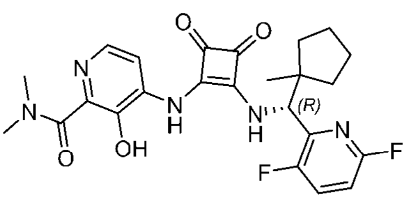,

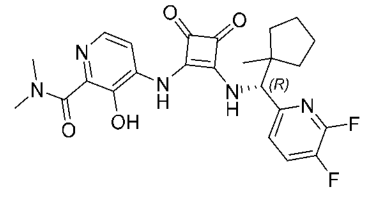 , 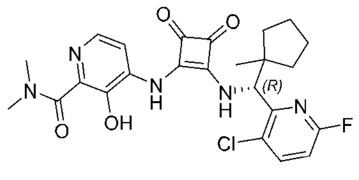 , 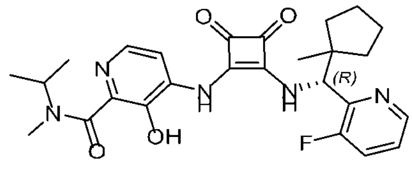 ,

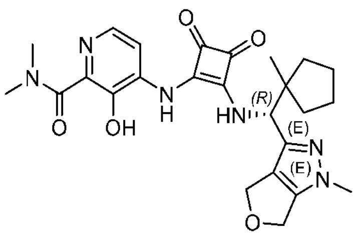 , 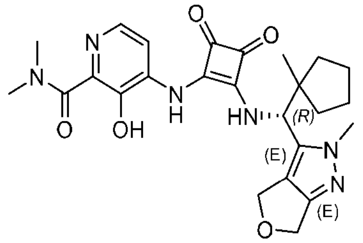 , 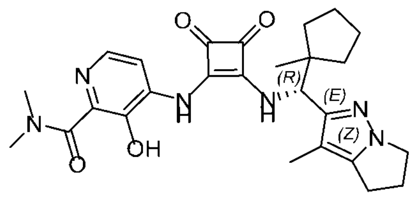 ,

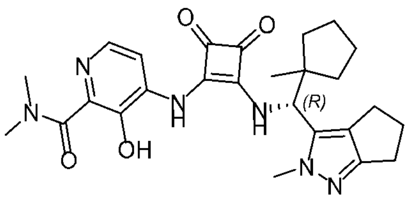 , 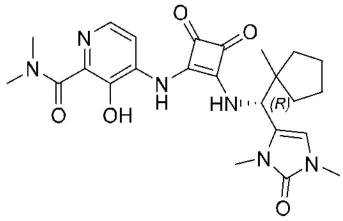 , 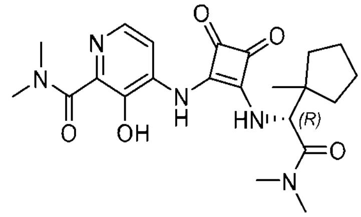 ,

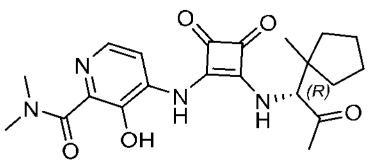 , 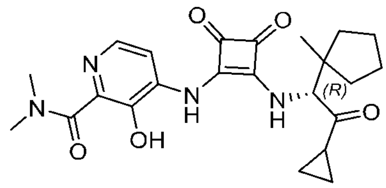 , 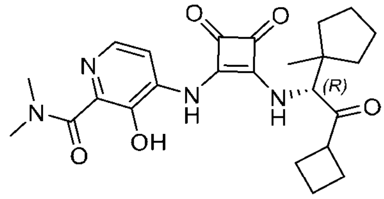 ,

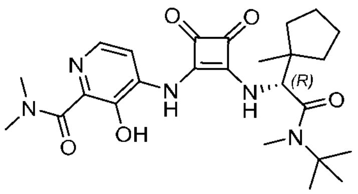 , 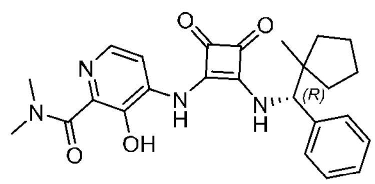 , 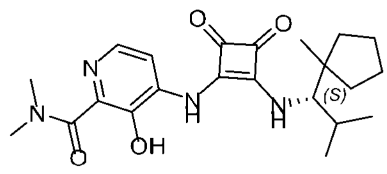 ,

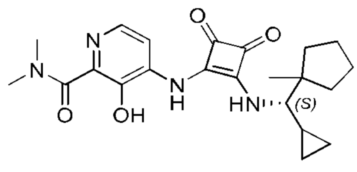 , 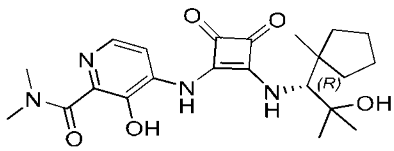 ,

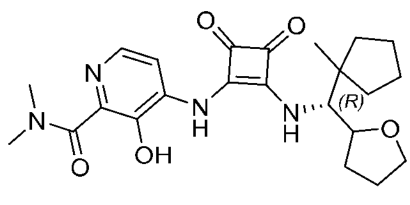 , 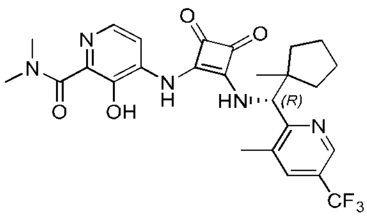 , 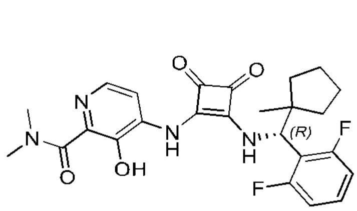 ,

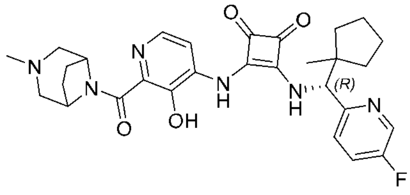,
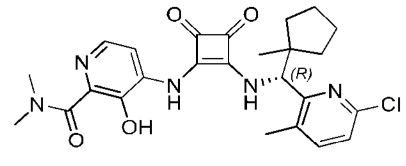,

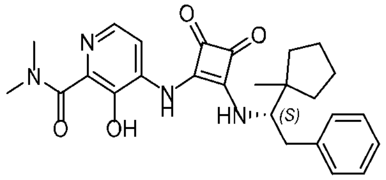,
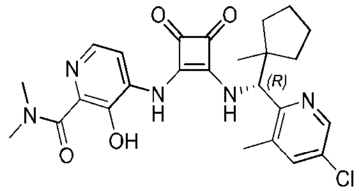,
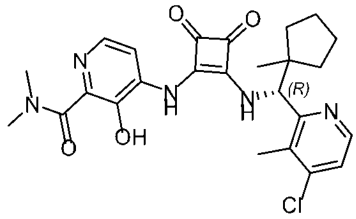,

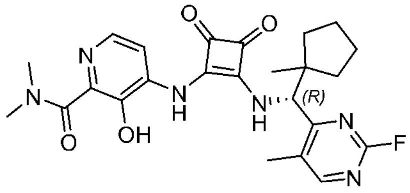,
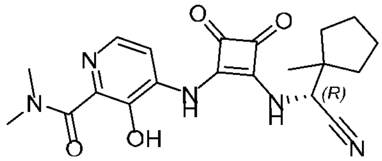,
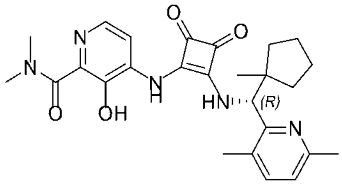,

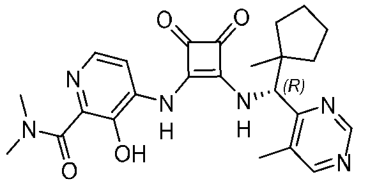,
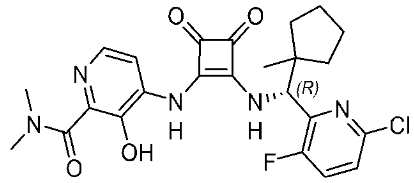,

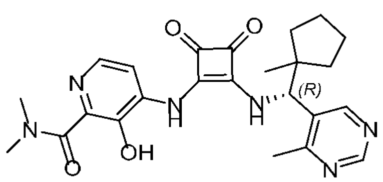,
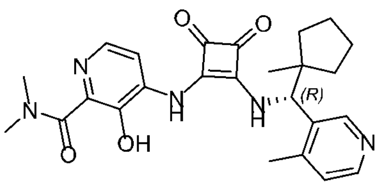,
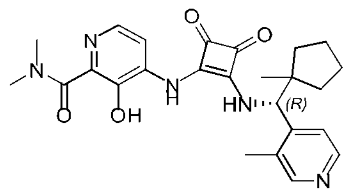,

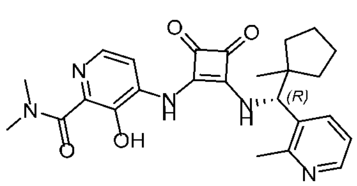,
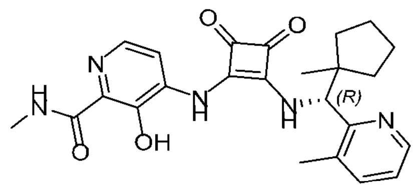,

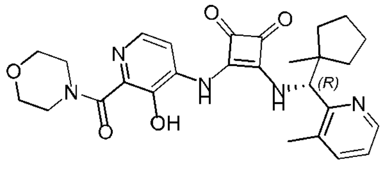,
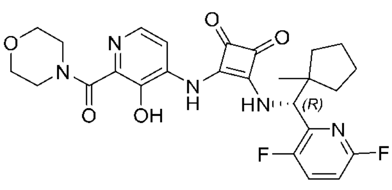,
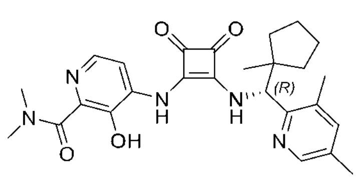,

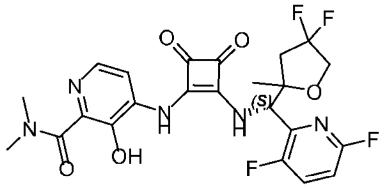, 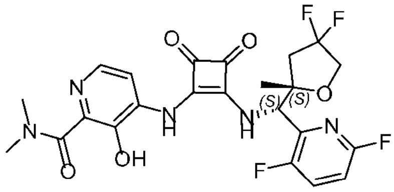, 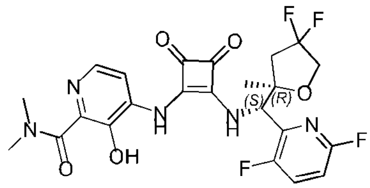,

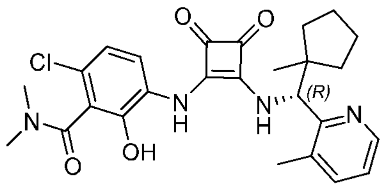, 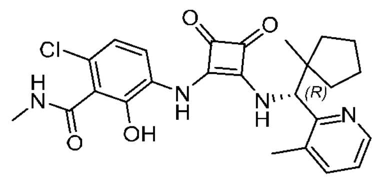, 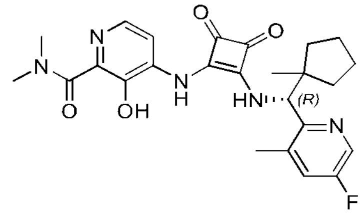,

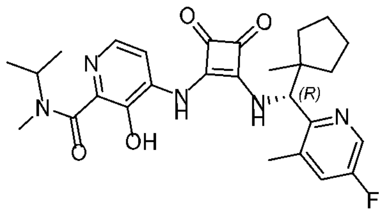, 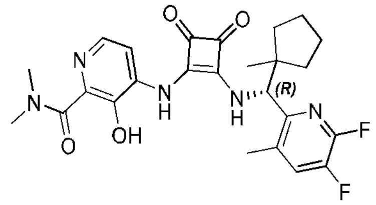, 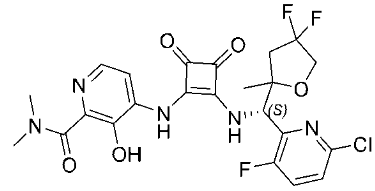,

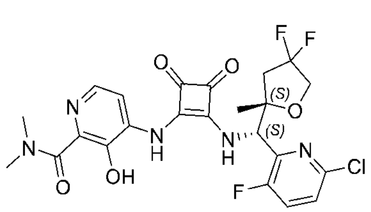, 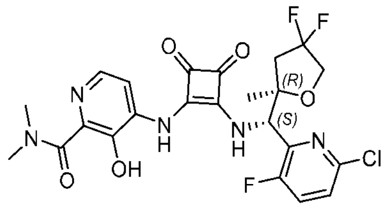, 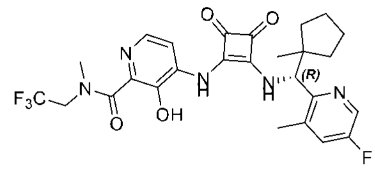,

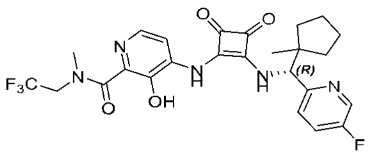, 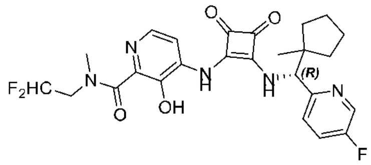, 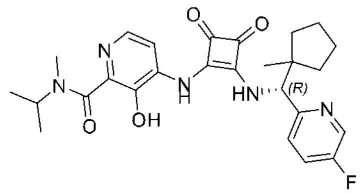,

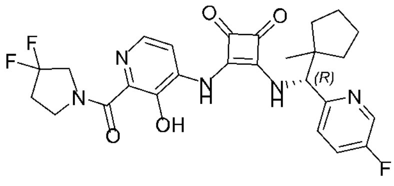, 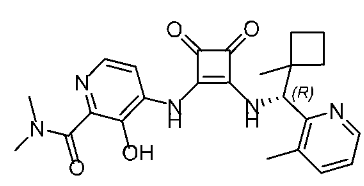, 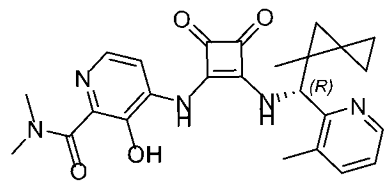,

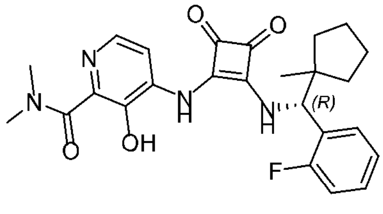, 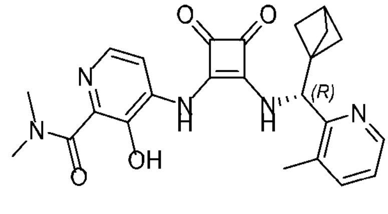, 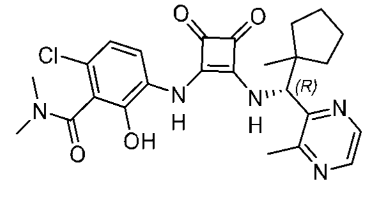,

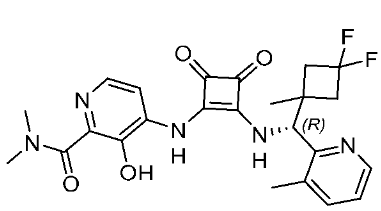,
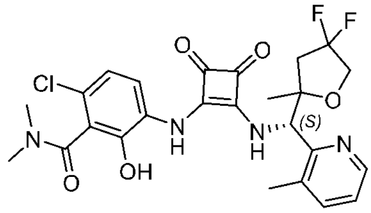,
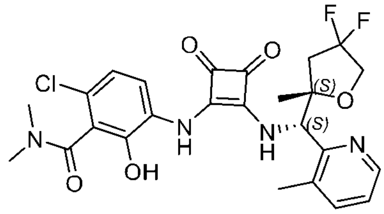,

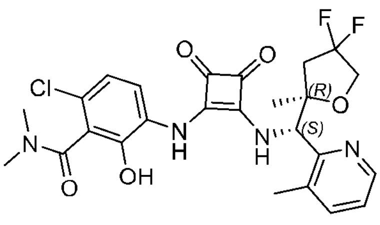,
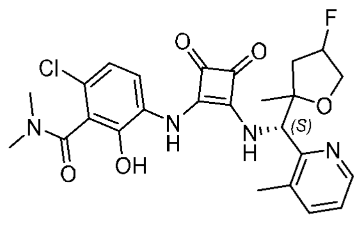,
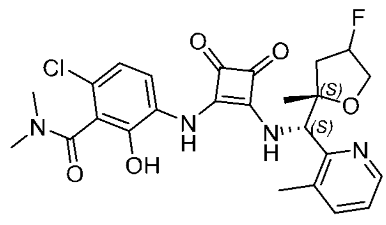,

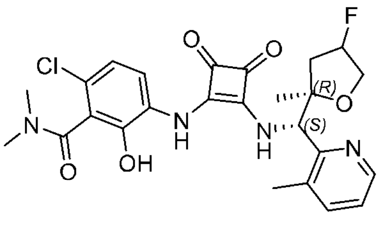,
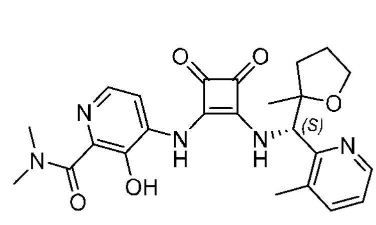,
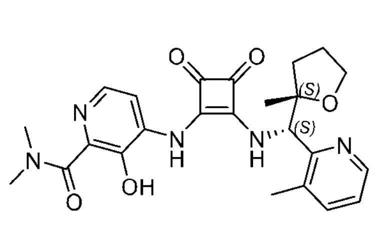,

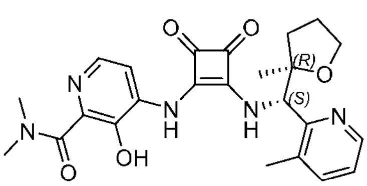,
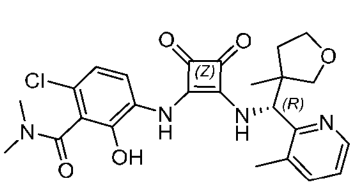,
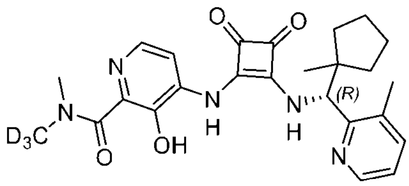,

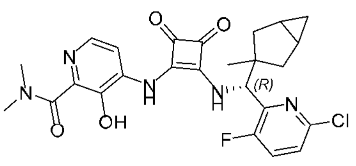,
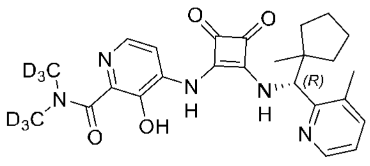,
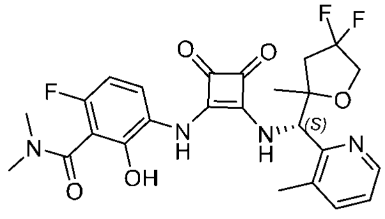,

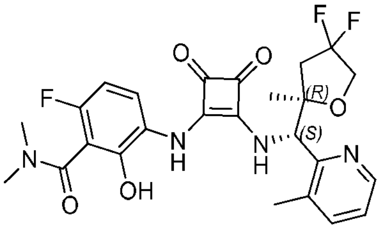,
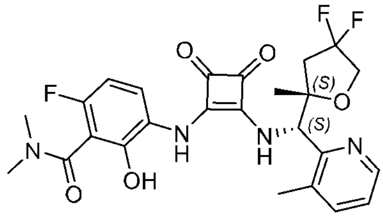,
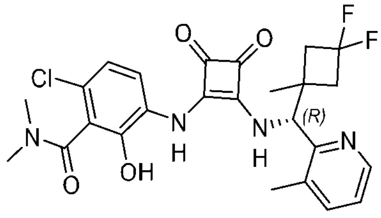,

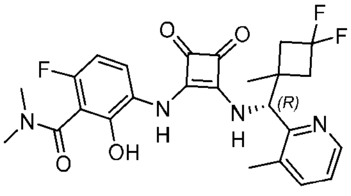,
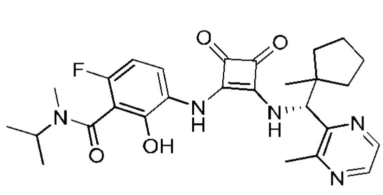,
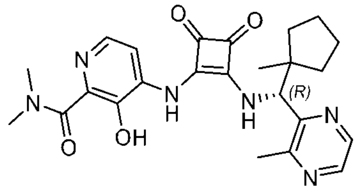,

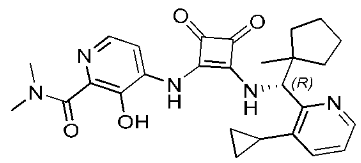 , 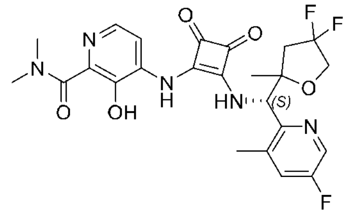 , 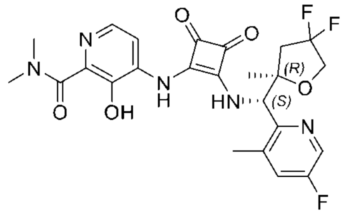 ,

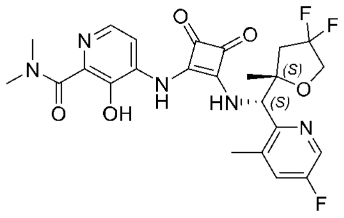 , 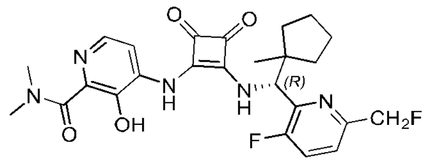 , 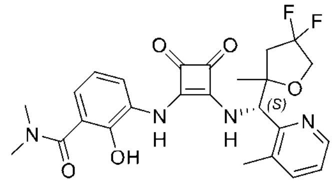 ,

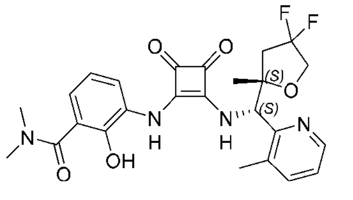 , 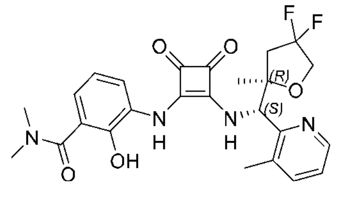 , 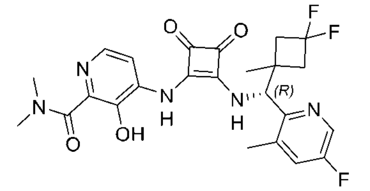 ,

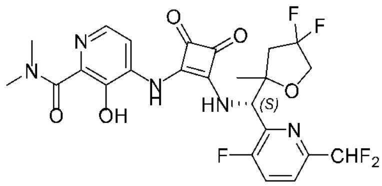 , 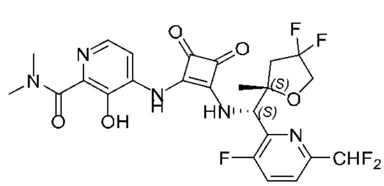 , 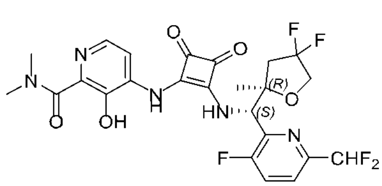 ,

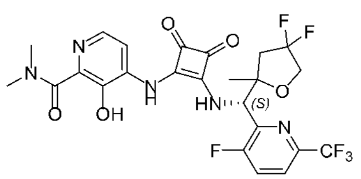 , 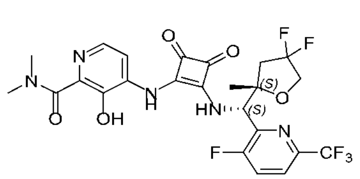 , 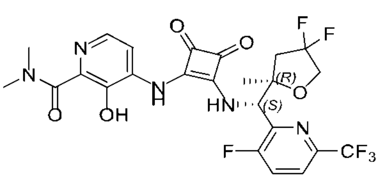 ,

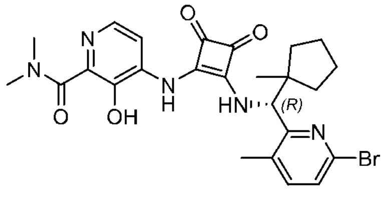 , 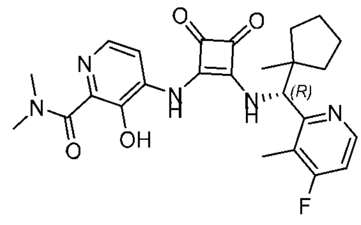 , 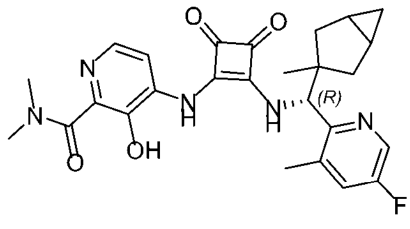 ,

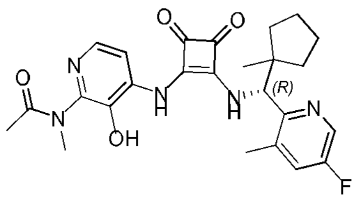 , 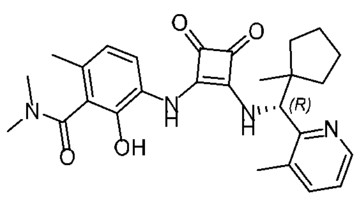 , 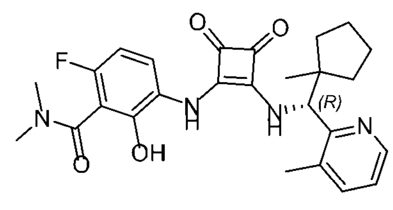 ,

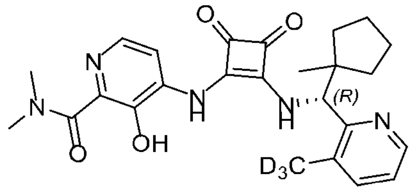
,
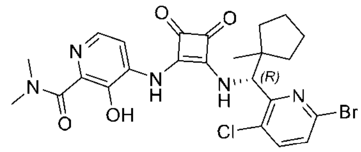
,
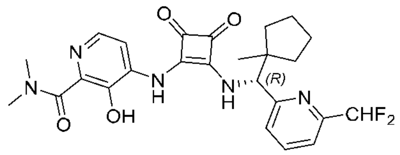
,
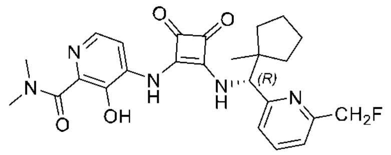
,
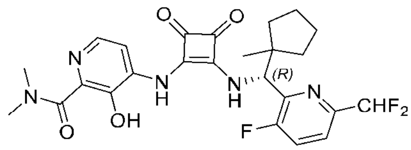
,
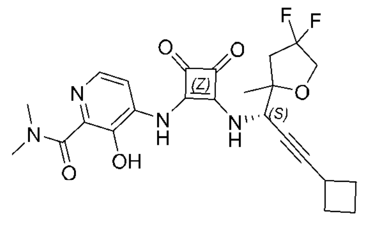
,
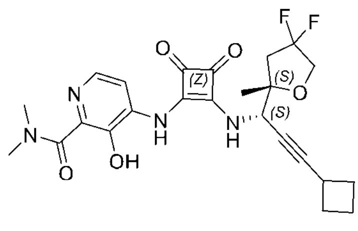
,
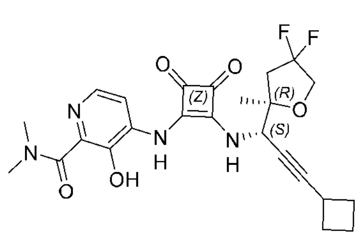
,
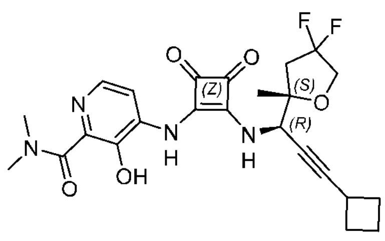
,
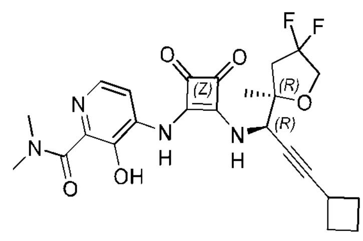
,
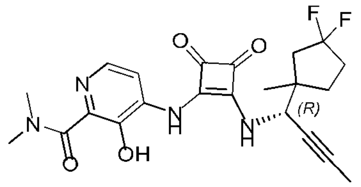
,
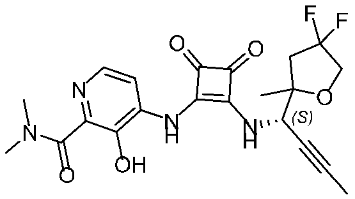
,
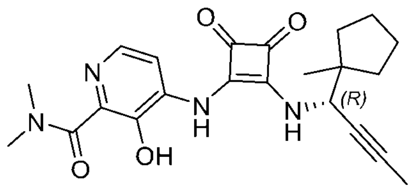
,
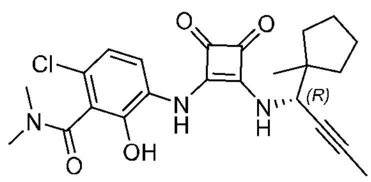
,
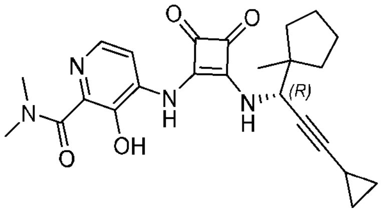
,
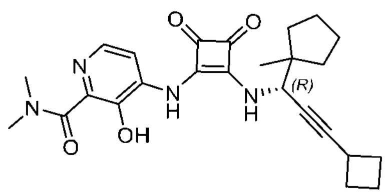
,
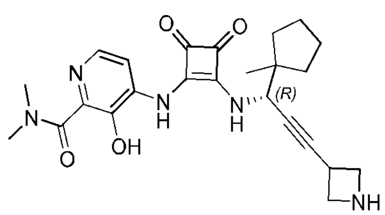
,
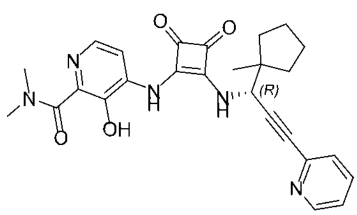
,

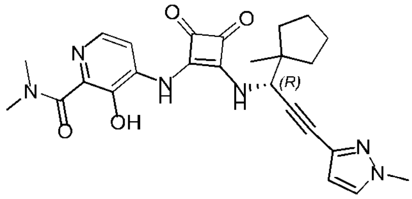
,
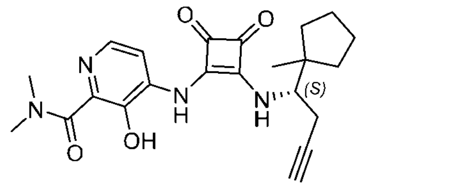
,
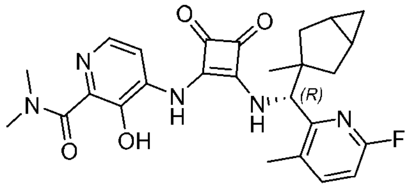
,
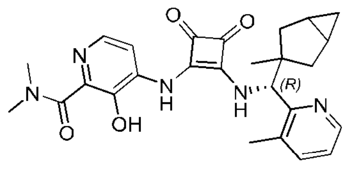
,
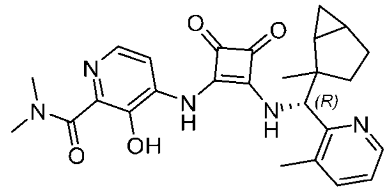
,
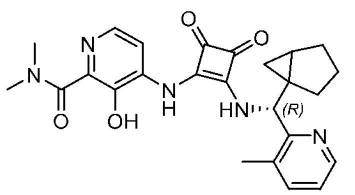
,
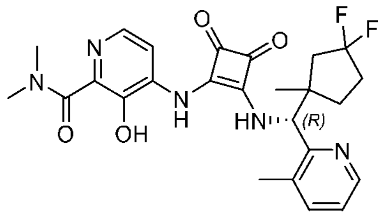
,
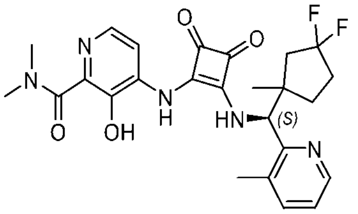
,
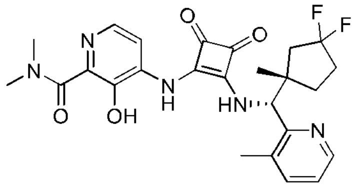
,
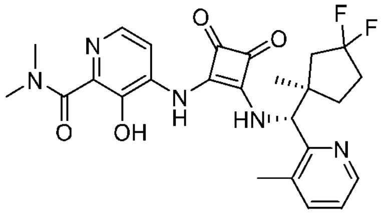
,
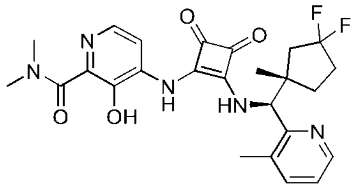
,
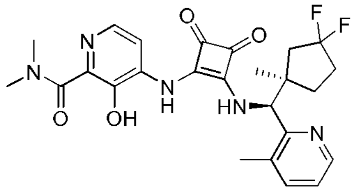
,
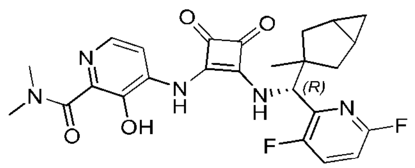
,
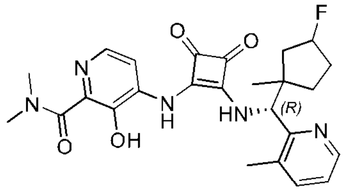
,
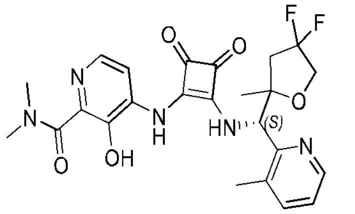
,
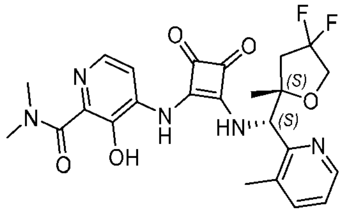
,
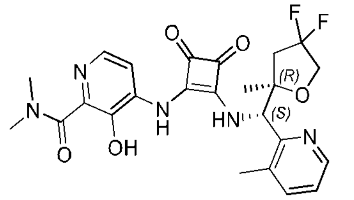
,
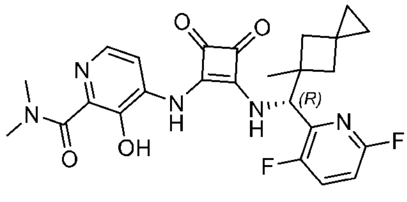
,
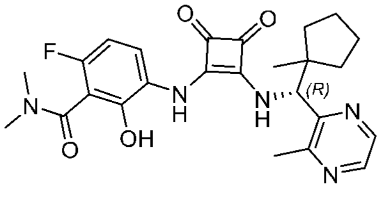
,
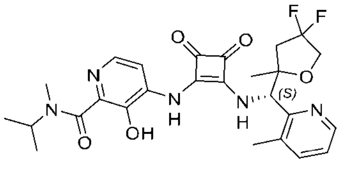
,

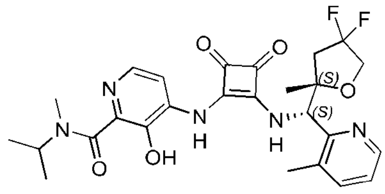, 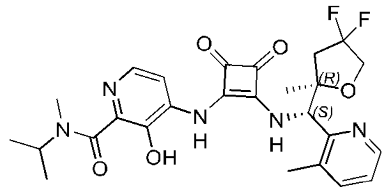, 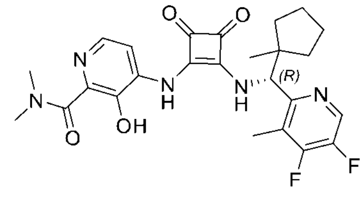,

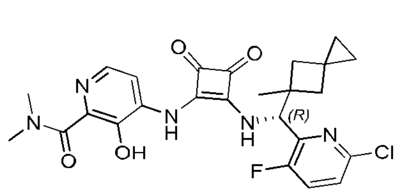, 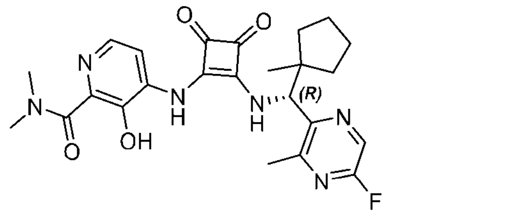,

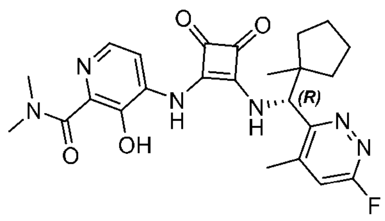, 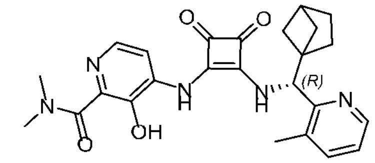, 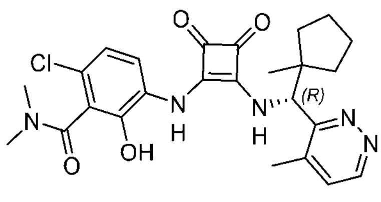,

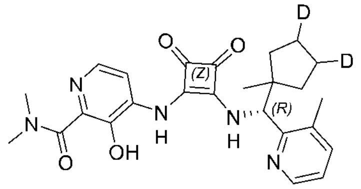, 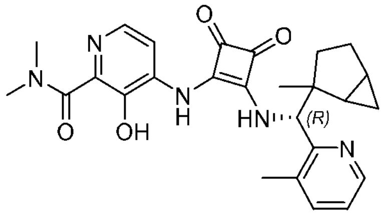, 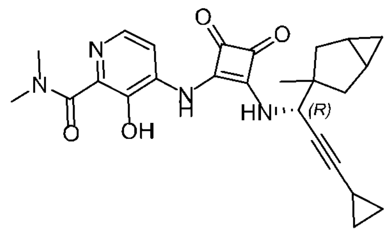,

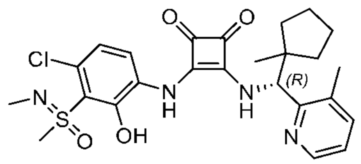, 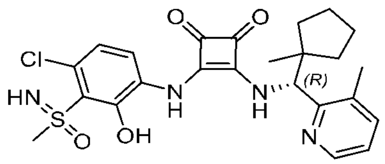, 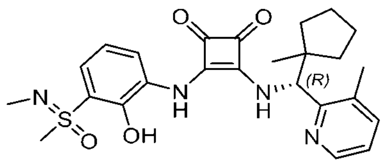,

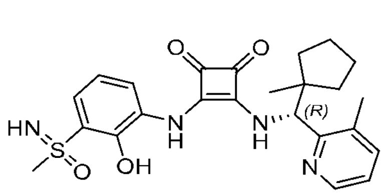, 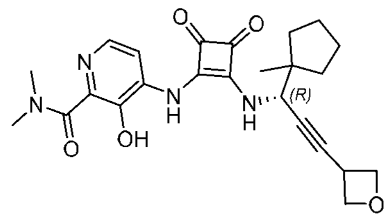, 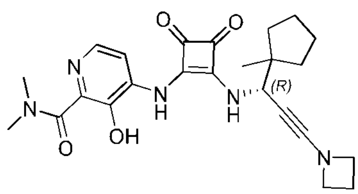,

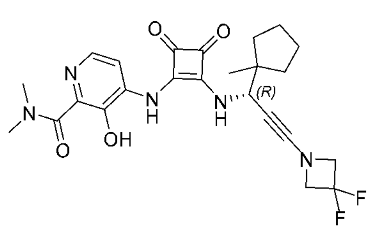, 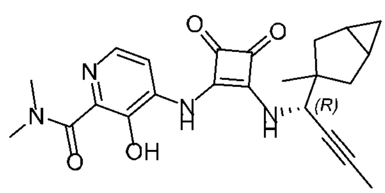, 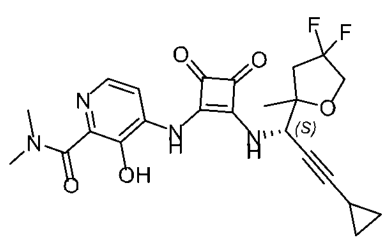,

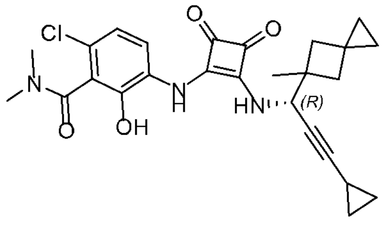, 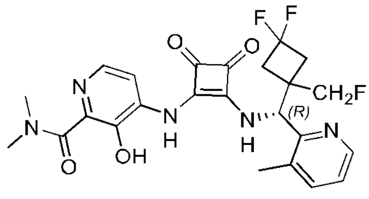, 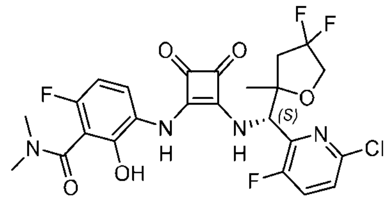,

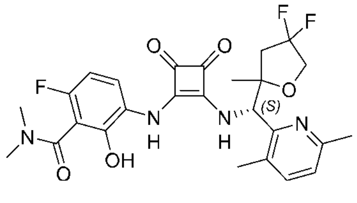, 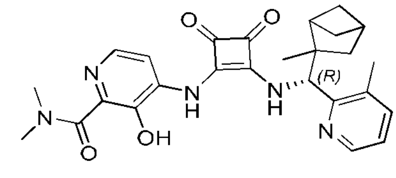, 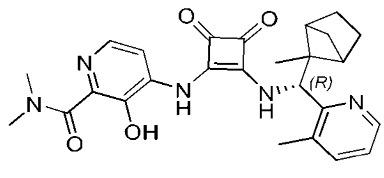,

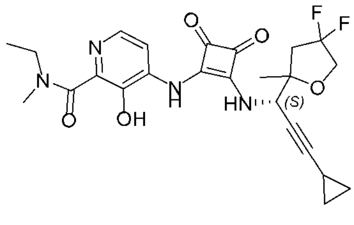, 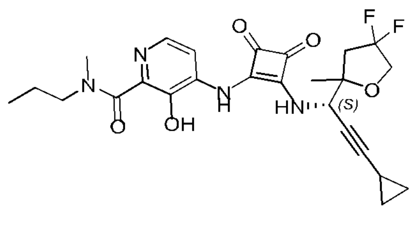, 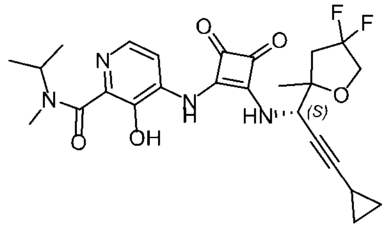,

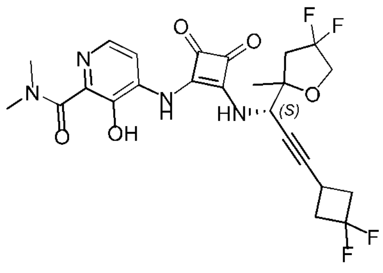, 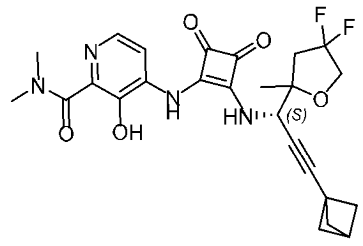, 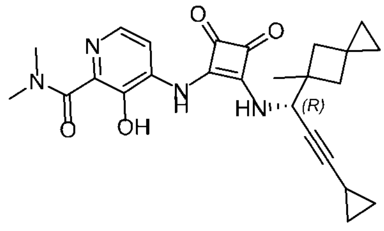,

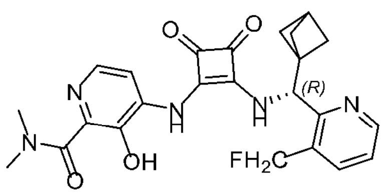, 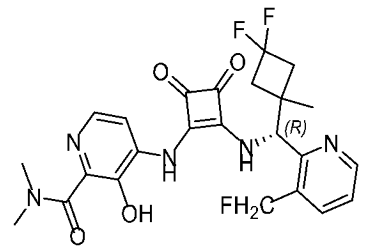, 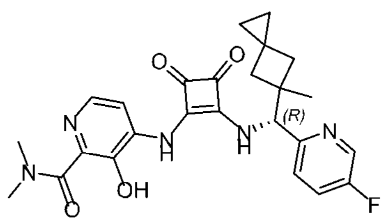,

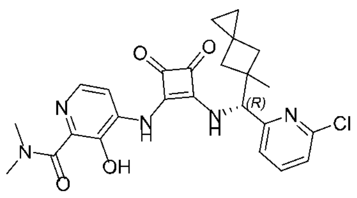, 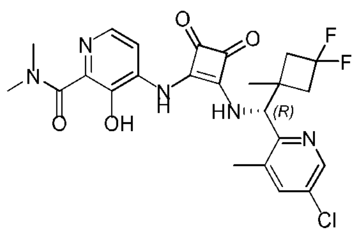, 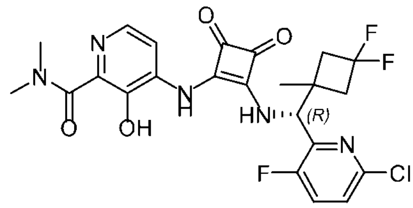,

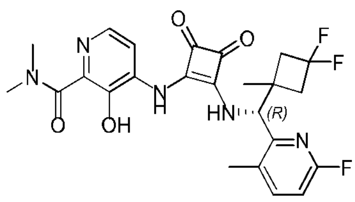,
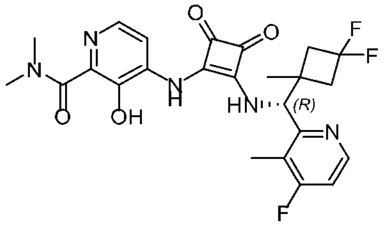,
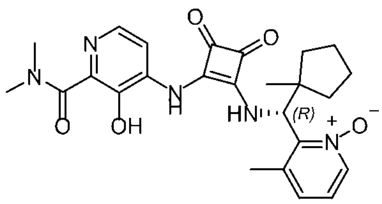,

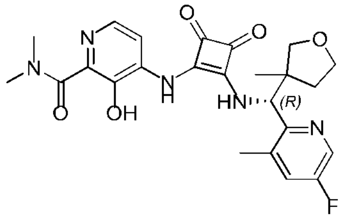,
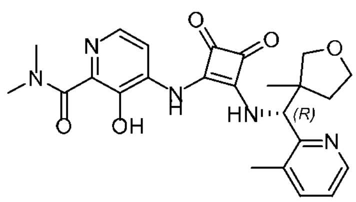,
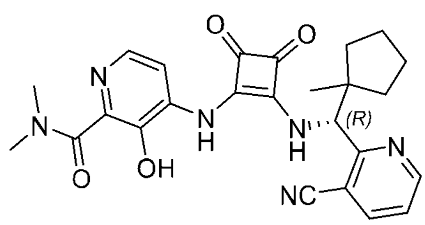,

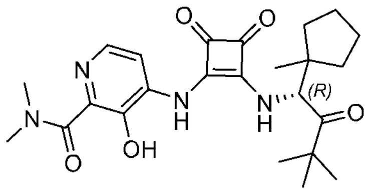,
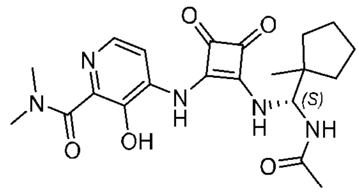,
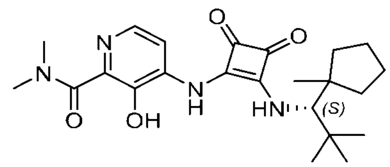,

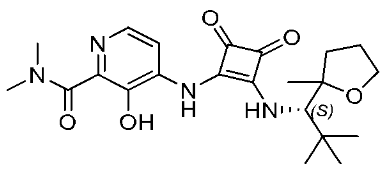,
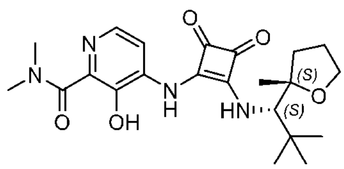,
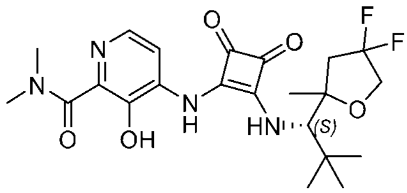,

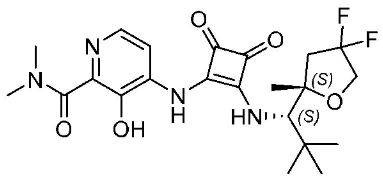,
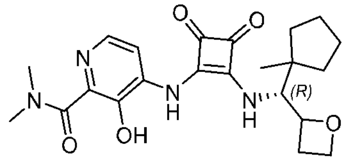,
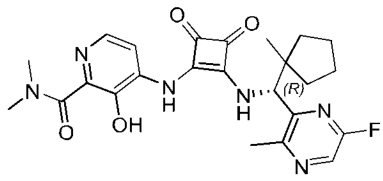,

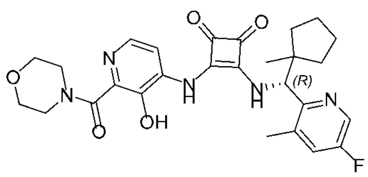,
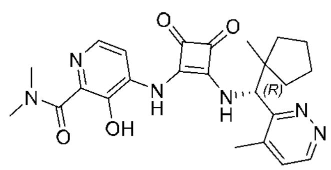,
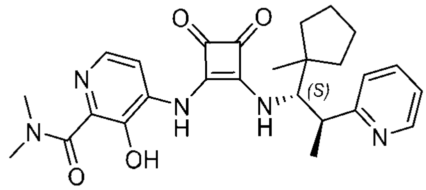,

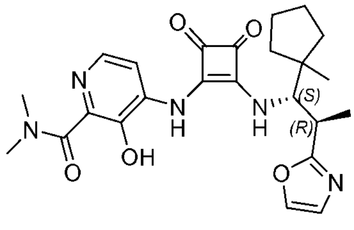,
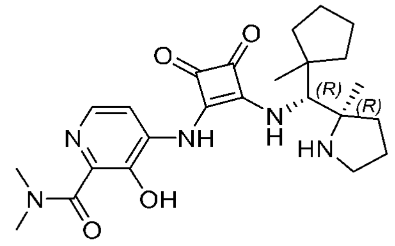,
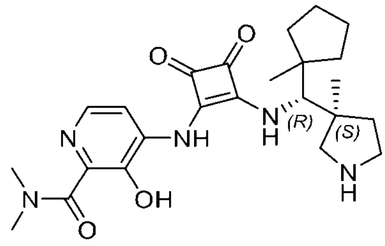,

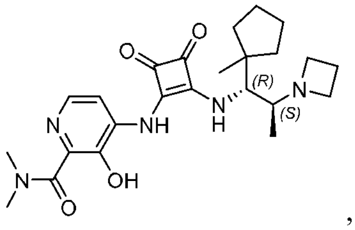,
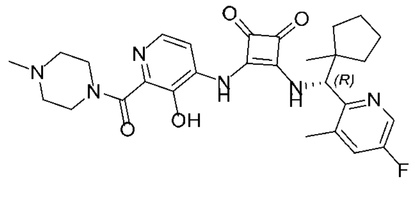,
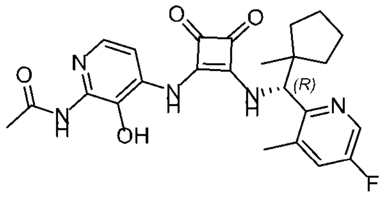,
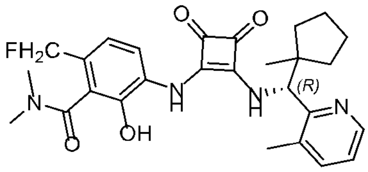,
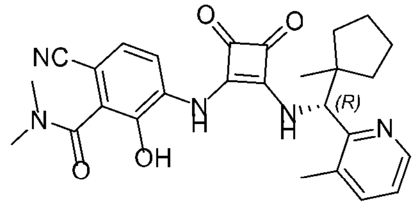,
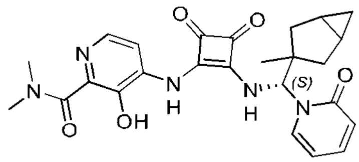,
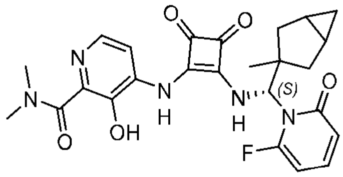,
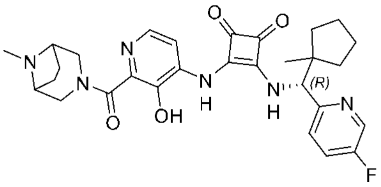,
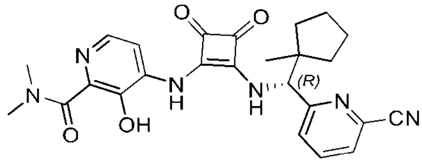,
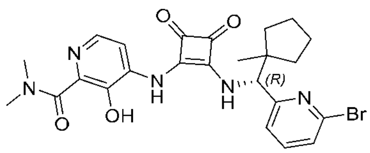,
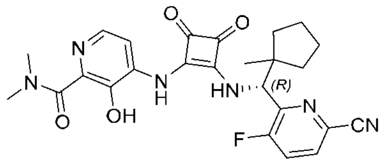,
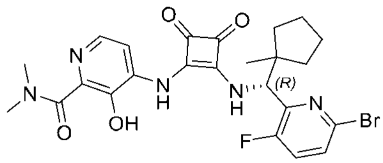,
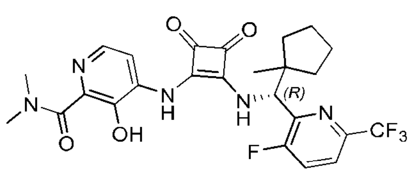,
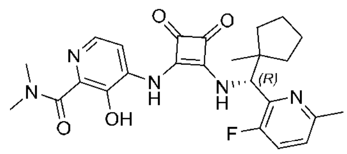,
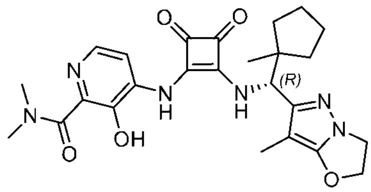,
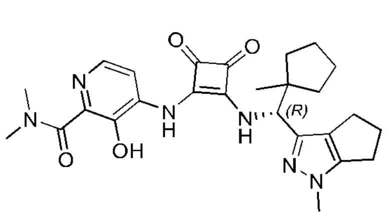, or
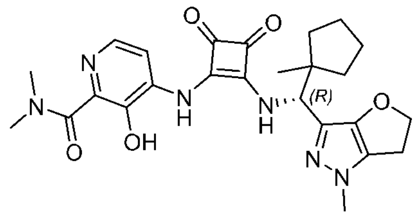.

7. A method for preparing the compound, the racemate thereof, the stereoisomer thereof, the tautomer thereof, the N-oxide thereof, or the pharmaceutically acceptable salt of any of the foregoing as defined in any one of claims 1 to 6, comprising the following step:

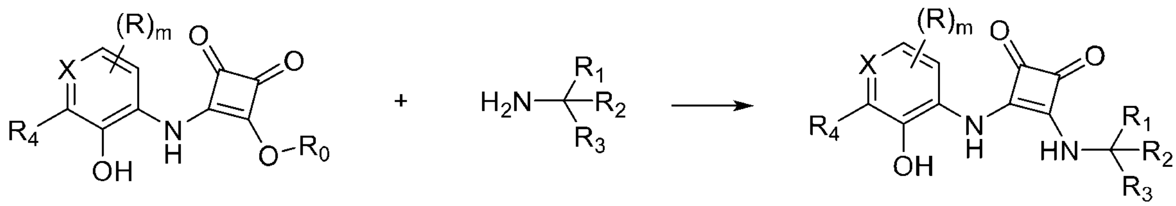

1 2 (I) ,

reacting compound 1 with compound 2 to obtain the compound represented by formula (I); wherein $R_1$, $R_2$, $R_3$, $R_4$, R, X, and m each independently have the definition as described in any one of claims 1 to 6, and $R_0$ is $C_{1-6}$ alkyl.

**8.** A compound represented by formula 1 or formula 2:

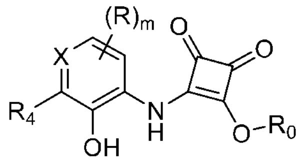

1

or

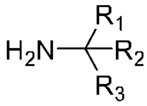

2 ,

wherein $R_0$, $R_1$, $R_2$, $R_3$, $R_4$, R, X, and m each independently have the definition as described in any one of claims 1 to 7;

preferably, the compound represented by formula 2 is selected from the group consisting of the following compounds:

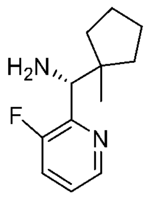

001-10 ,

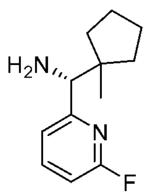

002-2 ,

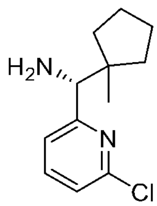

003-2 ,

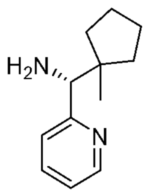

004-2 ,

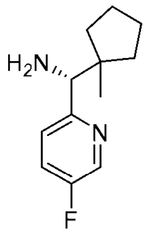

005-2 ,

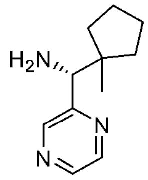

006-2 ,

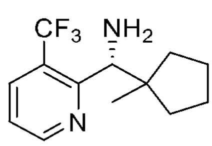

007-2 ,

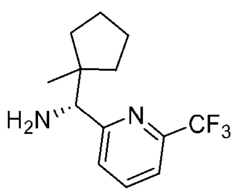

008-2 ,

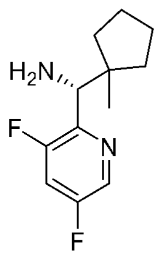

009-4 ,

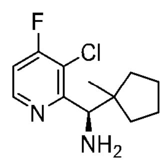

010-3 ,

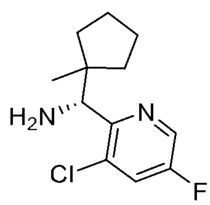

011-2 ,

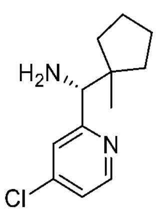
012-2 ,
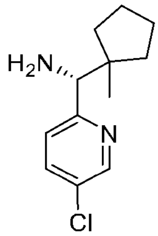
013-2 ,
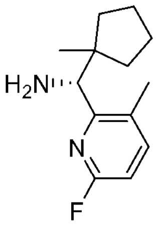
014-3 ,
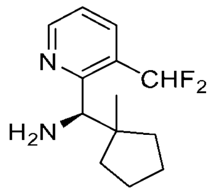
015-3 ,
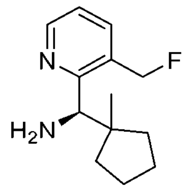
016-6 ,

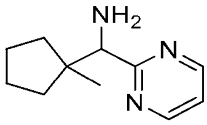
017-2 ,
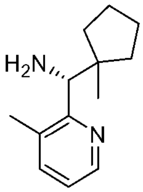
018-2 ,
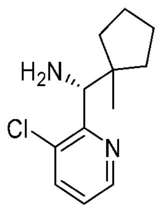
019-2 ,
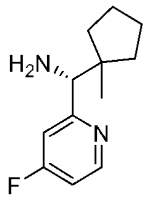
020-2 ,
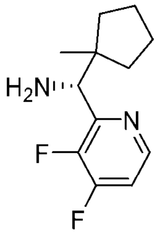
021-8 ,
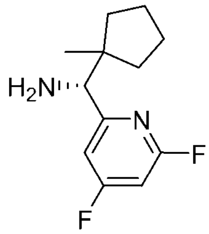
022-6 ,

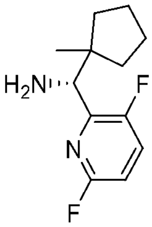
023-4 ,
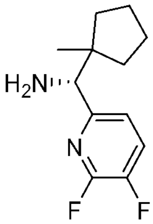
024-4 ,
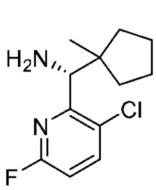
025-3 ,
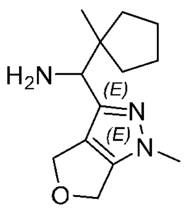
027-7 ,
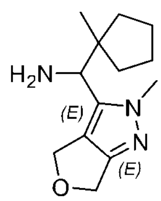
028-4 ,
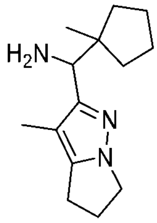
029-9 ,

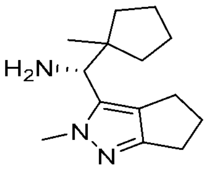
030-4 ,
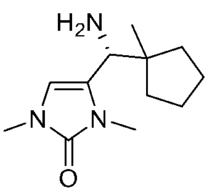
031-8 ,
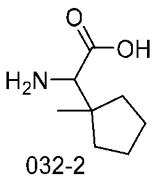
032-2 ,
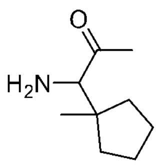
033-3 ,
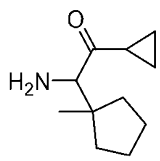
034-4 ,

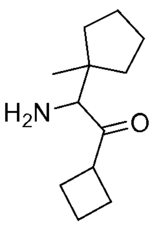
035-2 ,
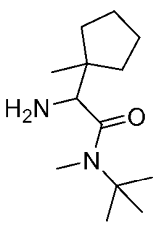
036-2 ,
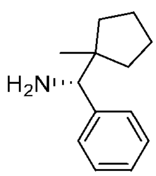
037-2 ,
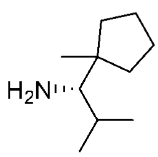
038-2 ,
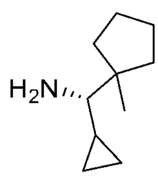
039-2 ,
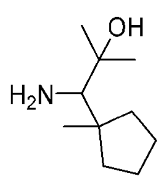
040-3 ,

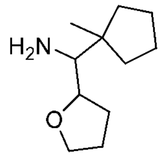
041-5 ,
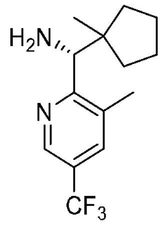
042-5 ,
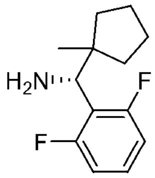
043-2 ,
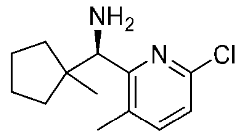
045-3 ,
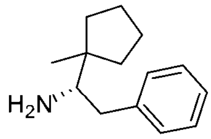
046-2 ,

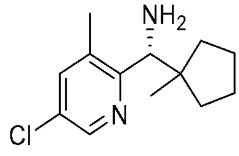
047-2 ,
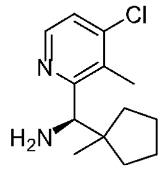
048-2 ,
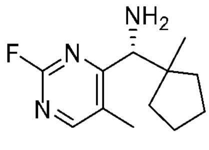
049-4 ,
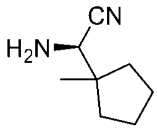
050-2 ,
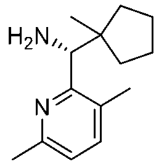
051-3 ,

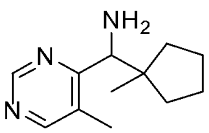
052-6 ,
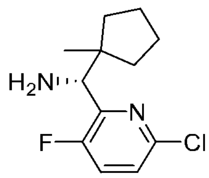
053-5 ,
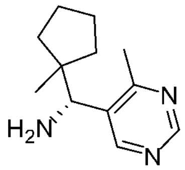
054-2 ,
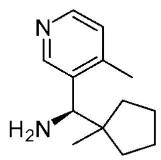
055-2 ,
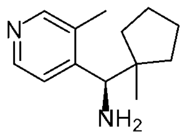
056-3 ,

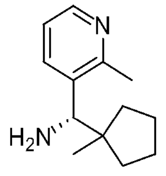
057-2 ,
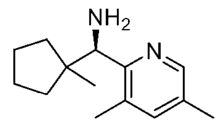
061-2 ,
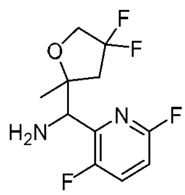
062-8 ,
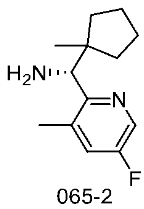
065-2 ,
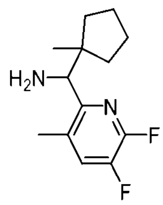
067-8 ,
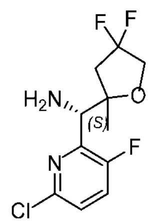
068-8 ,

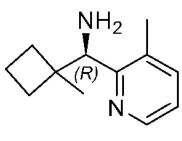
074-5 ,
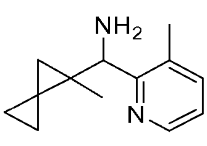
075-11 ,
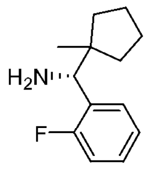
076-2 ,
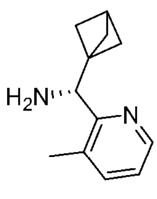
077-5 ,
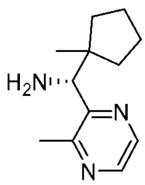
078-2 ,
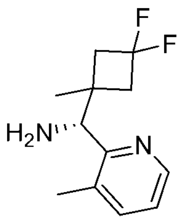
079-8 ,

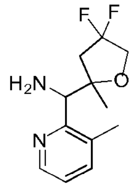
080-2 ,
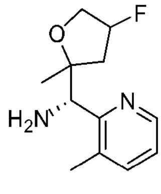
081-6 ,
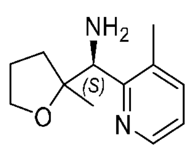
082-5 ,
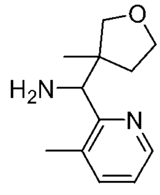
083-5 ,
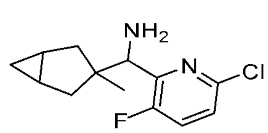
085-6 ,

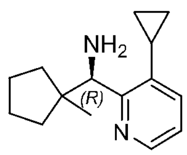
092-4 ,
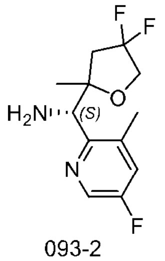
093-2 ,
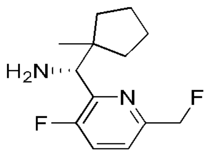
094-4 ,
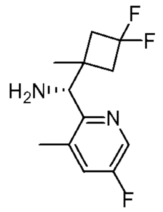
096-2 ,
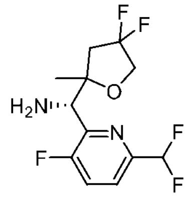
097-7 ,
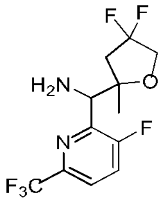
098-2 ,
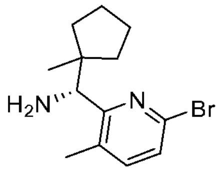
099-3 ,
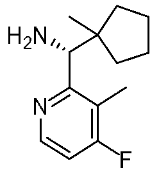
100-3 , or
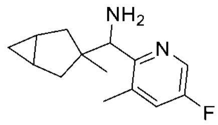
101-3 .

**9.** A pharmaceutical composition comprising a therapeutically effective amount of the compound, the racemate thereof, the stereoisomer thereof, the tautomer thereof, the N-oxide thereof, or the pharmaceutically acceptable salt of any of the foregoing as defined in any one of claims 1 to 6.

**10.** Use of the compound, the racemate thereof, the stereoisomer thereof, the tautomer thereof, the N-oxide thereof, or the pharmaceutically acceptable salt of any of the foregoing as defined in any one of claims 1 to 6, or the pharmaceutical composition as defined in claim 9, in the manufacture of a medicament;

preferably, the medicament is for diagnosing, preventing, and/or treating a CCR6 receptor-mediated disease or disorder;
preferably, the medicament is a CCR6 antagonist;
preferably, the disease or disorder is an allergic disease, psoriasis, contact hypersensitivity, Sjögren's syndrome, dry eye disease, or multiple sclerosis;
preferably, the disease or disorder is rheumatoid arthritis, juvenile arthritis, juvenile rheumatoid arthritis, systemic-onset rheumatoid arthritis, pauciarticular rheumatoid arthritis, pauciarticular juvenile rheumatoid arthritis, polyarticular rheumatoid arthritis, enteropathic arthritis, juvenile Reiter's syndrome, ankylosing spondylitis, juvenile ankylosing spondylitis, SEA syndrome, reactive arthritis (reactive arthropathy), psoriatic arthropathy, juvenile enteropathic arthritis, polymyalgia rheumatica, enteropathic spondylitis, juvenile idiopathic arthritis (JIA), juvenile psoriatic arthritis, juvenile rheumatoid arthritis, systemic-onset juvenile rheumatoid arthritis, giant cell arteritis, or secondary osteoarthritis caused by an inflammatory disease;
preferably, the disease or disorder is inflammatory bowel disease (IBD), Crohn's disease, or ulcerative colitis.

## INTERNATIONAL SEARCH REPORT

International application No.
**PCT/CN2024/090046**

### A. CLASSIFICATION OF SUBJECT MATTER

C07D213/81(2006.01)i；C07D213/02(2006.01)i；C07D401/12(2006.01)i；C07D405/14(2006.01)i；C07C237/30(2006.01)i；A61K31/435(2006.01)i；A61K31/4025(2006.01)i；A61P37/00(2006.01)i；A61P1/00(2006.01)i；A61P19/02(2006.01)i

According to International Patent Classification (IPC) or to both national classification and IPC

### B. FIELDS SEARCHED

Minimum documentation searched (classification system followed by classification symbols)

IPC:C07D,C07C,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of science, Registry(STN), Caplus(STN): CCR6, 拮抗剂, 趋化因子, 二氧代环丁烯, dioxocyclobutenyl+, inhibitor, 根据权利要求1式(I)化学式进行结构检索, structural search according to chemical formula (I) of claim 1

### C. DOCUMENTS CONSIDERED TO BE RELEVANT

| Category* | Citation of document, with indication, where appropriate, of the relevant passages | Relevant to claim No. |
|---|---|---|
| X | WO 2004011418 A1 (SCHERING CORPORATION et al.) 05 February 2004 (2004-02-05)<br>description, embodiment 2061 | 1-5, 7-8 |
| X | CN 103906738 A (GALDERMA RESEARCH & DEVELOPMENT) 02 July 2014 (2014-07-02)<br>claim 1, and description, paragraphs [0013] and [0021]-[0024], and embodiments 1, 8 and 21 | 1-10 |
| X | WO 2013061002 A1 (GALDERMA RESEARCH & DEVELOPMENT) 02 May 2013 (2013-05-02)<br>claim 1, and description, paragraphs [0011] and [0015]-[0018], and embodiment 1 | 1-10 |
| X | WO 2016102877 A1 (GALDERMA RESEARCH & DEVELOPMENT) 30 June 2016 (2016-06-30)<br>description, embodiment 5 | 8 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 July 2024** | **30 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/090046**

**C. DOCUMENTS CONSIDERED TO BE RELEVANT**

| Category* | Citation of document, with indication, where appropriate, of the relevant passages | Relevant to claim No. |
|---|---|---|
| X | REGISTRY. "2639551-86-1 et al." *STN*, 30 April 2021 (2021-04-30), entire document | 8 |
| X | REGISTRY. "2382309-86-4 et al." *STN*, 25 November 2019 (2019-11-25), entire document | 8 |
| A | CN 112672791 A (PFIZER INC.) 16 April 2021 (2021-04-16) claim 1, and description, paragraphs [0039]-[0048] and [0554], and embodiments 1-42 | 1-10 |
| A | WO 2010063802 A1 (NOVARTIS AG) 10 June 2010 (2010-06-10) claim 1, and description, embodiments 1-40 | 1-10 |

Form PCT/ISA/210 (second sheet) (July 2022)

**INTERNATIONAL SEARCH REPORT**

International application No.
**PCT/CN2024/090046**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **8**
because they relate to parts of the international application that do not comply with the prescribed requirements to such an extent that no meaningful international search can be carried out, specifically:

General formula 2 of claim 8 covers a large number of compounds, and thus no effective search can be performed. The present search report is provided on the basis of a preferred compound of the compounds of formula 2 in claim 8.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/090046**

| Patent document cited in search report | | | Publication date (day/month/year) | Patent family member(s) | | | Publication date (day/month/year) |
|---|---|---|---|---|---|---|---|
| WO | 2004011418 | A1 | 05 February 2004 | US | 2007021494 | A1 | 25 January 2007 |
| | | | | US | 7964646 | B2 | 21 June 2011 |
| | | | | MXPA | 05001274 | A | 08 September 2005 |
| | | | | AU | 2003259302 | A1 | 16 February 2004 |
| | | | | AU | 2003259302 | B2 | 26 June 2008 |
| | | | | EP | 1539678 | A1 | 15 June 2005 |
| | | | | EP | 1539678 | B1 | 05 September 2012 |
| | | | | US | 2011213029 | A1 | 01 September 2011 |
| | | | | US | 2009306079 | A1 | 10 December 2009 |
| | | | | US | 7947720 | B2 | 24 May 2011 |
| | | | | IL | 166427 | A0 | 15 January 2006 |
| | | | | RU | 2005105692 | A | 10 December 2005 |
| | | | | BR | 0313109 | A | 21 June 2005 |
| | | | | PL | 374876 | A1 | 14 November 2005 |
| | | | | TW | 200716520 | A | 01 May 2007 |
| | | | | US | 2004106794 | A1 | 03 June 2004 |
| | | | | JP | 2005534684 | A | 17 November 2005 |
| | | | | CA | 2496676 | A1 | 05 February 2004 |
| | | | | CA | 2496676 | C | 14 June 2011 |
| | | | | NZ | 538521 | A | 29 February 2008 |
| | | | | NO | 20051036 | L | 20 April 2005 |
| | | | | KR | 20050026060 | A | 14 March 2005 |
| CN | 103906738 | A | 02 July 2014 | PL | 2771329 | T3 | 30 September 2016 |
| | | | | DK | 2771329 | T3 | 06 June 2016 |
| | | | | RS | 54775 | B1 | 31 October 2016 |
| | | | | SMT | 201600151 | B | 01 July 2016 |
| | | | | JP | 2014530894 | A | 20 November 2014 |
| | | | | JP | 6068486 | B2 | 25 January 2017 |
| | | | | US | 2014296254 | A1 | 02 October 2014 |
| | | | | US | 9388149 | B2 | 12 July 2016 |
| | | | | MX | 2014003958 | A | 27 August 2014 |
| | | | | MX | 342924 | B | 19 October 2016 |
| | | | | PT | 2771329 | E | 17 June 2016 |
| | | | | CY | 1117705 | T1 | 17 May 2017 |
| | | | | AU | 2012328198 | A1 | 03 April 2014 |
| | | | | ZA | 201401678 | B | 29 April 2015 |
| | | | | BR | 112014009889 | A2 | 25 April 2017 |
| | | | | WO | 2013061004 | A1 | 02 May 2013 |
| | | | | WO | 2013061004 | A8 | 17 April 2014 |
| | | | | IN | CHN2014A | | 19 June 2015 |
| | | | | FR | 2981935 | A1 | 03 May 2013 |
| | | | | FR | 2981935 | B1 | 07 August 2015 |
| | | | | RU | 2014121390 | A | 10 December 2015 |
| | | | | HUE | 029128 | T2 | 28 February 2017 |
| | | | | HRP | 20160608 | T1 | 01 July 2016 |
| | | | | US | 2016362403 | A1 | 15 December 2016 |
| | | | | US | 9580412 | B2 | 28 February 2017 |
| | | | | ES | 2569053 | T3 | 06 May 2016 |
| | | | | SI | 2771329 | T1 | 29 July 2016 |
| | | | | EP | 2771329 | A1 | 03 September 2014 |

Form PCT/ISA/210 (patent family annex) (July 2022)

**INTERNATIONAL SEARCH REPORT**
**Information on patent family members**

International application No.
**PCT/CN2024/090046**

| Patent document cited in search report | Publication date (day/month/year) | Patent family member(s) | Publication date (day/month/year) |
|---|---|---|---|
| | | EP 2771329 B1 | 16 March 2016 |
| | | CA 2848263 A1 | 02 May 2013 |
| | | KR 20140090168 A | 16 July 2014 |
| WO 2013061002 A1 | 02 May 2013 | CA 2852404 A1 | 02 May 2013 |
| | | ES 2578782 T3 | 01 August 2016 |
| | | US 2015087675 A1 | 26 March 2015 |
| | | US 9120772 B2 | 01 September 2015 |
| | | JP 2014530893 A | 20 November 2014 |
| | | EP 2771336 A1 | 03 September 2014 |
| | | EP 2771336 B1 | 27 April 2016 |
| | | FR 2981936 A1 | 03 May 2013 |
| | | FR 2981936 B1 | 20 December 2013 |
| | | US 2016166562 A1 | 16 June 2016 |
| | | US 9415039 B2 | 16 August 2016 |
| WO 2016102877 A1 | 30 June 2016 | FR 3030515 A1 | 24 June 2016 |
| | | FR 3030515 B1 | 20 January 2017 |
| | | US 2019225598 A1 | 25 July 2019 |
| | | US 10647706 B2 | 12 May 2020 |
| | | US 2017349575 A1 | 07 December 2017 |
| | | US 10287278 B2 | 14 May 2019 |
| | | EP 3237384 A1 | 01 November 2017 |
| | | EP 3237384 B1 | 30 January 2019 |
| | | CA 2971707 A1 | 30 June 2016 |
| | | CA 2971707 C | 25 July 2023 |
| CN 112672791 A | 16 April 2021 | NZ 773473 A | 28 July 2023 |
| | | UY 38379 A | 30 April 2020 |
| | | CL 2021000683 A1 | 15 October 2021 |
| | | ECSP 21018584 A | 29 April 2021 |
| | | MX 2021003318 A | 14 May 2021 |
| | | CO 2021003391 A2 | 08 April 2021 |
| | | AU 2019344107 A1 | 08 April 2021 |
| | | AU 2019344107 B2 | 15 September 2022 |
| | | TW 202024056 A | 01 July 2020 |
| | | TWI 754172 B | 01 February 2022 |
| | | DOP 2021000045 A | 30 April 2021 |
| | | WO 2020058869 A1 | 26 March 2020 |
| | | US 2020095239 A1 | 26 March 2020 |
| | | US 10975065 B2 | 13 April 2021 |
| | | MA 53643 A | 29 December 2021 |
| | | GEP 20237476 B | 27 March 2023 |
| | | PE 20211070 A1 | 09 June 2021 |
| | | CA 3055805 A1 | 21 March 2020 |
| | | CR 20210146 A | 20 May 2021 |
| | | SG 11202101827 RA | 29 April 2021 |
| | | KR 20230175326 A | 29 December 2023 |
| | | PH 12021550554 A1 | 21 February 2022 |
| | | BR 112021003956 A2 | 25 May 2021 |
| | | IL 281634 A | 31 May 2021 |
| | | CU 20210016 A7 | 12 October 2021 |
| | | NI 202100012 A | 22 June 2021 |

Form PCT/ISA/210 (patent family annex) (July 2022)

**INTERNATIONAL SEARCH REPORT**
**Information on patent family members**

International application No.
**PCT/CN2024/090046**

| Patent document cited in search report | Publication date (day/month/year) | Patent family member(s) | Publication date (day/month/year) |
|---|---|---|---|
| | | EP 3852879 A1 | 28 July 2021 |
| | | US 2021206757 A1 | 08 July 2021 |
| | | US 11708360 B2 | 25 July 2023 |
| | | ZA 202101169 B | 27 July 2022 |
| | | AR 116464 A1 | 12 May 2021 |
| | | JP 2021532166 A | 25 November 2021 |
| | | JP 6989729 B2 | 05 January 2022 |
| | | KR 20210046717 A | 28 April 2021 |
| | | KR 102615234 B1 | 19 December 2023 |
| | | EA 202190586 A1 | 05 July 2021 |
| WO 2010063802 A1 | 10 June 2010 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310481783
- CN 202410172844
- CN 202410467079
- CN 202410479410